# EUROPEAN PATENT APPLICATION

(11) **EP 4 434 997 A2**
(43) Date of publication of application: **25.09.2024**
(21) Application number: 24159919.0
(22) Date of filing: 30.10.2015
(51) Int. Cl.: C07K 14/435

(54) **DELIVERY OF NEGATIVELY CHARGED PROTEINS USING CATIONIC LIPIDS**

(30) Priority: 30.10.2014 US 201414529010
(62) Divisional of application: 15797506.1
(71) Applicant: President And Fellows Of Harvard College, Cambridge, Massachusetts 02138 (US)
(72) Inventor: LIU, David R., Lexington, MA 02420 (US); THOMPSON, David B., Brookline, MA 02446 (US); ZURIS, John Anthony, Cambridge, MA 02142 (US)
(74) Representative: Atkins, James Gordon John

(57) **Abstract**

Compositions, methods, strategies, kits, and systems for the delivery of negatively charged proteins, protein complexes, and fusion proteins, using cationic polymers or lipids are provided. Delivery of proteins into cells can be effected *in vivo*, *ex vivo*, or *in vitro.* Proteins that can be delivered using the compositions, methods, strategies, kits, and systems provided herein include, without limitation, enzymes, transcription factors, genome editing proteins, Cas9 proteins, TALEs, TALENs, nucleases, binding proteins (e.g., ligands, receptors, antibodies, antibody fragments; nucleic acid binding proteins, etc.), structural proteins, and therapeutic proteins (*e.g*., tumor suppressor proteins, therapeutic enzymes, growth factors, growth factor receptors, transcription factors, proteases, *etc*.), as well as variants and fusions of such proteins.

## Description

### RELATED APPLICATION

This application is claims priority to U.S. patent application, U.S.S.N. 14/529,010, filed October 30, 2014, which is a continuation-in-part of and claims priority under 35 U.S.C. § 120 to U.S. patent application, U.S.S.N. 14/462,189, filed August 18, 2014, to U.S. patent application, U.S. S.N. 14/462,163, filed August 18, 2014, and to International Application, PCT/US2014/05424735, filed September 5, 2014, which claims priority under U.S.C. § 365(c) to U.S. patent application, U.S.S.N. 14/462,189, filed August 18, 2014, and to U.S. patent application, U.S.S.N. 14/462,163, filed August 18, 2014, and also claims priority under 35 U.S.C. § 119(e) to U.S. provisional patent application, U.S.S.N. 61/874,746, filed September 6, 2013, the entire contents of each of which are incorporated herein by reference.

### BACKGROUND OF THE INVENTION

Macromolecular delivery into mammalian cells is an attractive approach for cell manipulation, as it would allow modulation of gene expression and modification of the genome, which, in turn, would open new avenues for research and enable the therapeutic targeting of molecules currently viewed as "undruggable" by small molecules. In particular, recombinant nucleases targeting genes or alleles associated with disease have great potential as therapeutic agents. The current methods of macromolecular delivery include viral delivery of nucleic acid molecules, receptor-mediated delivery of nucleic acids or proteins, and the use of protein fusions with cell-penetrating peptides such as TAT, Arg9, or Penetratin for the delivery of proteins. Each of these delivery systems offers benefits for particular applications; in most cases, however, questions regarding efficacy, cytotoxicity, and ease of preparation remain. Easily prepared reagents capable of effectively delivering macromolecules (*e.g*., functional effector proteins) to a variety of cell lines without significant cytotoxicity or other adverse side effect remain of considerable concern.

Most proteins do not spontaneously enter mammalian cells and are thus naturally limited in their use as research tools and their potential as therapeutic agents. Techniques for the delivery of proteins into mammalian cells have been developed recently to address intracellular targets. These techniques include the use of lipid-based reagents (Zelphati et al., J. Biol. Chem. 276, 35103-35110, 2001), nanoparticles (Hasadsri et al., J. Biol. Chem., 2009), vault ribonucleoprotein particles (Lai et al., ACS Nano 3, 691-699, 2009); genetic or chemical fusion to receptor ligands (Gabel et al., J. Cell Biol. 103, 1817-1827, 1986; Rizk et al., Proc. Natl. Acad. Sci. U.S.A. 106, 11011-11015, 2009); and fusion to cell-penetrating peptides (Wadia et al., Curr. Protein Pept. Sci. 4, 97-104, 2003; Zhou et al., Cell Stem Cell 4, 381-384, 2009). Perhaps the most common method for protein delivery is genetic fusion to protein transduction domains (PTDs) including the HIV-1 transactivator of transcription (Tat) peptide and polyarginine peptides. These cationic PTDs promote association with negatively charged cell-surface structures and subsequent endocytosis of exogenous proteins. Both Tat and polyarginine have been used to deliver a variety of macromolecules into cells both *in vitro* and *in vivo* (Wadia et al., Curr. Protein Pept. Sci. 4, 97-104, 2003; Zhou et al., Cell Stem Cell 4, 381-384, 2009; Myou et al., J. Immunol. 169, 2670-2676, 2002; Bae et al., Clin. Exp. Immunol. 157, 128-138, 2009; Schwarze et al., Science 285, 1569-1572, 1999). Despite these advances, intracellular targets remain difficult to affect using exogenous proteins, and even modest success can require toxic concentrations of the respective transduction agent due to the low efficiency with which proteins are functionally delivered into cells (Zhou et al., Cell Stem Cell 4, 381-384, 2009; Wang et al., Nat. Biotechnol. 26, 901-908, 2008). Therefore, there remains a need for better delivery systems for getting functional effector proteins into cells to target intracellular biomolecules.

### SUMMARY OF THE INVENTION

The present disclosure provides systems, compositions, preparations, kits, and related methods for delivering proteins into cells using cationic polymers or cationic lipids. In some embodiments, the proteins to be delivered are negatively charged proteins, also referred to herein as anionic proteins, such as, for example, naturally occurring or engineered negatively charged proteins. In some embodiments, the proteins to be delivered are associated with a negatively charged protein, *e.g.,* via covalent or non-covalent interactions, to form a protein complex. In some such embodiments, the complex comprising the protein to be delivered associated with the negatively charged proteins, *e.g.*, a supernegatively charged protein or a naturally occurring negatively charged protein, has a net negative charge. In some embodiments, the proteins to be delivered bind a nucleic acid thus forming a protein:nucleic acid complex having a net negative charge.

Some aspects of this disclosure are based on the discovery that negatively charged proteins can be associated with cationic polymers or cationic lipids and that such protein:lipid complexes are efficiently delivered into cells. This technology can be applied to naturally negatively charged proteins (*e.g*., the proteins listed in tables 3-6, Sirt1 (-59, 86 kDa), PPARg (-13, 54 kDa), PRDM16 (-23, 140 kDa), PGC1a (-15, 91 kDa), TP53BP1 (-148, 213 kDa), Utrophin (-142, 394 kDa), Dystrophin (-89, 426 kDa), Bik (-17, 18 kDa), IκBα (-29, 35 kDa), Von Hippel-Lindau disease tumor suppressor (-18, 24 kDa), E3 ubiquitin ligases, metal-binding proteins, VP64 transcriptional activators, the anionic 3xFLAG peptide tag, and fusions thereof), to engineered supernegatively charged proteins (*e.g*., supernegatively charged GFP or streptavidin variants), to proteins that bind to nucleic acids and form negatively charged protein:nucleic acid complexes (*e.g*., Cas9 proteins, and variants and fusions thereof), or to protein fusions in which a protein to be delivered is associated with a negatively charged protein, *e.g.,* a supernegatively charged protein as disclosed herein.

For example, in some embodiments, systems, compositions, preparations, kits, and related methods are provided for delivering proteins to cells, for example, functional effector proteins, such as, *e.g.,* enzymes (*e.g.,* oxidoreductases, transferases, hydrolases, lyases, isomerases, or ligases); transcriptional activators, transcriptional repressors, genome editing proteins, Cas9 proteins, TALEs, TALENs, nucleases, binding proteins (*e.g.,* ligands, receptors, antibodies, antibody fragments; nucleic acid binding proteins, *etc*.); structural proteins; therapeutic proteins (*e.g*., tumor suppressor proteins, therapeutic enzymes, growth factors, growth factor receptors, transcription factors, proteases, *etc.*)*,* as well as variants and fusions thereof. Additional suitable proteins that can be delivered to cells according to the inventive concepts disclosed herein will be apparent to the skilled artisan based on the present disclosure, and the disclosure is not limited in this respect. In some embodiments, the protein to be delivered is a functional effector protein, for example, an enzyme, a tumor suppressor protein, or a protein that binds a nucleic acid, and is delivered into cells using a supercharged protein (*e.g.,* a negatively charged supercharged protein, also referred to herein as a supernegatively charged protein), and a cationic polymer or a cationic lipid. As described in greater detail herein, fusing or associating proteins to be delivered to a cell, for example, functional effector proteins (*e.g*., enzymes, tumor suppressor proteins, proteins that bind a nucleic acid, nucleases, transcriptional activators/repressors, Cas9 proteins including variants and fusions thereof, *etc.*) with charged proteins, *e.g.,* super positively or supernegatively charged proteins, allows for delivery of the proteins to the interior of cells, for example, to affect gene expression or genomic modifications

While delivery of proteins has proven effective for extracellular targets, their use to address intracellular targets is comparatively undeveloped due to the inability of most proteins to spontaneously enter mammalian cells. Enabling exogenous proteins to access intracellular targets is most commonly achieved by delivery of their encoding DNA sequences through chemical transfection, electroporation, or viral delivery. The introduction of exogenous DNA into cells, however, raises the possibility of permanent recombination into the genome, potential disruption of endogenous genes, and long-term exposure to the encoded agent. For some research or therapeutic applications, including genome editing applications that seek to effect a one-time, permanent modification of genomic DNA, the functional delivery of non-replicable protein agents may offer improved safety or broader applicability. Further, while the delivery of proteins using cationic compounds such as lipids and polymers has remained technically challenging and in many cases induces cellular toxicity, it was surprisingly found, using the compositions and methods provided herein, that proteins (*e.g.,* functional effector proteins as described herein) can be delivered to cells with no or minimal toxicity with significant improvements in efficiency. For example, as described in Example 7, delivery of Cas9:gRNA complexes with cationic lipids is highly efficient (up to 80% modification of cultured human cells from a single treatment) and also induces higher genome modification specificity compared with plasmid transfection, typically resulting in >10-fold higher on-target: off-target DNA modification ratios in human cells.

Accordingly, in some aspects, the present disclosure provides systems, strategies, reagents, and methods for the delivery of charged proteins, such as, for example, naturally occurring negatively charged proteins, engineered supernegatively charged proteins, proteins that bind nucleic acids, or are associated, covalently or non-covalently, with a negatively charged protein, into cells using cationic lipids or cationic polymers. In some embodiments, the proteins to be delivered are functional effector proteins, for example, enzymes, tumor suppressor proteins, proteins that bind a nucleic acid, nucleases, transcriptional activators/repressors, Cas9 proteins including variants and fusions thereof, *etc.*

In some embodiments, a negatively charged protein, *e.g.,* a naturally occurring negatively charged protein or an engineered supernegatively charged protein, is delivered to a cell or associated with a protein to be delivered in order to deliver the latter protein into the cell. In some embodiments, the supercharged protein has been engineered to exhibit an increase in its overall surface charge as compared to the corresponding unmodified protein. In some embodiments, the supercharged protein has been engineered to exhibit a decrease in its overall surface charge as compared to the corresponding unmodified protein. In other embodiments, the supercharged protein used in the context of this disclosure is a naturally occurring supercharged protein. In embodiments, in which the supercharged protein is associated with the protein to be delivered, the supercharged protein may be associated with the protein to be delivered through covalent or non-covalent interactions. In some embodiments, a protein that binds to a nucleic acid is delivered to a cell. In such embodiments, the protein:nucleic acid complex typically has a net negative charge. For example, without wishing to be bound by any particular theory, a Cas9 protein, variant, or fusion protein associated with a gRNA has net negative charged facilitating association with a cationic polymer or cationic lipid or with a positively supercharged protein. In certain embodiments, the negatively charged protein, protein complex, or protein: nucleic acid complex, is further associated with a cationic polymer or cationic lipid for delivery into a cell.

Examples of suitable engineered or naturally occurring supercharged proteins are described in Tables 3-6 of this application, and in international PCT patent application PCT/US07/70254, filed June 1, 2007, published as WO 2007/143574 on December 13, 2007; in international PCT application PCT/US09/041984, filed on April 28, 2009, published as WO 2009/134808 on November 5, 2009; and in international PCT application PCT/US10/001250, filed on April 28, 2010, published as WO 2010/129023 on November 11, 2010; the entire contents of each of which are incorporated herein by reference. In some embodiments, the negatively charged protein is Sirt1 (-59, 86 kDa), PPARg (-13, 54 kDa), PRDM16 (-23, 140 kDa), PGC1a (-15, 91 kDa), TP53BP1 (-148, 213 kDa), Utrophin (-142, 394 kDa), Dystrophin (-89, 426 kDa), Bik (-17, 18 kDa), IκBα. (-29, 35 kDa), Von Hippel-Lindau disease tumor suppressor (-18, 24 kDa), an E3 ubiquitin ligase, or a metal-binding protein. Further examples of supercharged proteins for use in delivering nucleases to cells are described herein.

Examples of suitable functional effector proteins, for example, nucleases and RNA-programmable effector proteins, such as Cas9 proteins, for delivery using the inventive methods, compositions, and systems are described in U.S. Provisional Patent Application, U.S.S.N. 61/868,846, filed August 22, 2013, entitled "Engineered Transcription Activator-Like Effector (TALE) Domains and Uses Thereof," U.S. Provisional Patent Application, U.S.S.N. 61/874,609, filed September 6, 2013, entitled "Cas9 Variants and Uses Thereof," U.S. Provisional Patent Application, U.S.S.N. 61/874,682, filed September 6, 2013, entitled "Switchable Cas9 Nucleases and Uses Thereof," U.S. Non-provisional Application, U.S.S.N. 14/320,519, filed June 20, 2014, entitled "Engineered Transcription Activator-Like Effector (TALE) Domains and Uses Thereof," U.S. Non-provisional Application, U.S.S.N. 14/320,498, filed June 30, 2014, entitled "Cas9-FokI Fusion Proteins And Uses Thereof," U.S. Non-provisional Application, U.S.S.N. 14/320,467, filed June 30, 2014, entitled "Cas9-Recombinase Fusion Proteins And Uses Thereof," U.S. Non-provisional Application, U.S.S.N. 14/326,329, filed July 8, 2014, entitled "Switchable gRNAs Comprising Aptamers," U.S. Non-provisional Application, U.S.S.N. 14/326,340, filed July 8, 2014, entitled "mRNA-Sensing Switchable gRNAs," U.S. Non-provisional Application, U.S.S.N. 14/326,361, filed July 8, 2014, entitled "Extended DNA-Sensing gRNAs," U.S. Non-provisional Application, U.S.S.N. 14/325,815, filed July 8, 2014, entitled "Fusions Of Cas9 Domains And Nucleic Acid-Editing Domains," U.S. Non-provisional Application, U.S. S.N. 14/326,109, filed July 8, 2014, entitled "Methods For Nucleic Acid Editing," U.S. Non-provisional Application, U.S. S.N. 14/326,140, filed July 8, 2014, entitled "Methods For Correcting PI3K Point Mutations," U.S. Non-provisional Application, U.S.S.N. 14/326,269, filed July 9, 2014, entitled "Methods For Correcting Presenilin Point Mutations," U.S. Non-provisional Application, U.S.S.N. 14/326,290, filed July 8, 2014, entitled "Methods For Correcting α-Antitrypsin Point Mutations," U.S. Non-provisional Application, U.S.S.N. 14/326,318, filed July 8, 2014, entitled "Methods For Correcting Von Willebrand Factor Point Mutations," U.S. Non-provisional Application, U.S.S.N. 14/326,303, filed July 8, 2014, entitled "Methods For Correcting Caspase-9 Point Mutations," and U.S. Provisional Application, U.S.S.N. 62/030,943, entitled "Cas9 Proteins Including Ligand-Dependent Inteins," the entire contents of each of which are incorporated herein by reference.

In some embodiments, the supercharged protein, engineered or naturally occurring, is positively charged. In other embodiments, for example those involving delivery of certain effector proteins using cationic lipids and/or cationic polymers, the supercharged protein is negatively charged. In certain embodiments, a superpositively or supernegatively charged protein is non-covalently associated with a protein to be delivered, for example, an effector protein. Alternatively, a superpositively or supernegatively charged protein may be covalently bound to the protein to be delivered, for example, an effector protein. In some embodiments, the effector protein is fused to a supercharged protein. In certain embodiments, the resulting fusion protein comprises a linker, *e.g.,* a cleavable linker, between the supercharged protein and the effector protein.

Some aspects of this disclosure provide compositions comprising a supercharged protein, *e.g.,* a supernegatively charged protein, associated with a protein to be delivered, *e.g.,* a functional effector protein (*e.g.,* enzymes (*e.g.,* oxidoreductases, transferases, hydrolases, lyases, isomerases, or ligases); transcriptional activators, transcriptional repressors, genome editing proteins, Cas9 proteins, TALEs, TALENs, nucleases, binding proteins (*e.g.,* ligands, receptors, antibodies, antibody fragments; nucleic acid binding proteins, *etc*.); structural proteins; therapeutic proteins (*e.g*., tumor suppressor proteins, therapeutic enzymes, growth factors, growth factor receptors, transcription factors, proteases, *etc.*)*,* as well as variants and fusions thereof). In some embodiments, the composition further comprises a cationic lipid. In some embodiments, the composition further comprises a cationic polymer. In some embodiments, the composition further comprises a buffer or excipient. In some embodiments, the supercharged protein has an overall positive charge that is greater than its corresponding unmodified protein and is in a quantity sufficient for and is formulated for delivery to and penetration into a cell. In other embodiments, for example those involving delivery of certain proteins using cationic lipids and/or cationic polymers, the supercharged protein has an overall negative charge that is greater than its corresponding unmodified protein. In some embodiments, the functional effector protein is a site-specific enzyme, *e.g.,* a nuclease, Cas9 protein, recombinase, *etc.* In some embodiments, the Cas9 protein is a wild type Cas9 protein, a Cas9 nickase, or comprises a nuclease inactivated (dCas9) protein. In some embodiments, the Cas9 protein is a fusion protein comprising dCas9. In some embodiments, the fusion protein comprises a transcriptional activator (*e.g.,* VP64), a transcriptional repressor (*e.g.,* KRAB, SID) a nuclease domain (*e.g.,* FokI), a recombinase domain (*e.g.,* Hin, Gin, or Tn3), a deaminase (*e.g.,* a cytidine deaminase or an adenosine deaminase) or an epigenetic modifier domain (*e.g.,* TET1). In some embodiments involving nucleases, the nuclease is a TALE nuclease, a Cas9 nuclease, a Cas9 nickase, or a zinc finger nuclease. In some embodiments, the nuclease specifically binds and cleaves a nucleic acid sequence. In some embodiments, the targeted nucleic acid sequence is a sequence of a gene that is a therapeutic target, for example a gene that is desirable to inactivate in the treatment of a disease. In some embodiments, the targeted nucleic acid sequence is a PRDM16, PPAPγ, VEGF-A, Oct-4, PI3K, presenilin, α-antitrypsin, von willebrand factor, or caspase-9 gene sequence.

In some embodiments, the functional effector protein is a transcription factor. In some embodiments, the functional effector protein is a TALE transcriptional activator or repressor. In some embodiments, the transcription factor, transcriptional activator, or transcriptional repressor specifically binds and activates or represses a gene. In some embodiments, the gene is a therapeutic target. In some embodiments, the functional effector protein is a TALE effector. In some embodiments, the supercharged protein is covalently bound to the functional effector protein, thus forming a fusion protein. In some embodiments, the supercharged protein is associated with the functional effector protein via a linker. In some embodiments, the linker is a cleavable linker. In some embodiments, the linker is a UV-cleavable linker or a linker that is cleaved by a lysosomal enzyme. In some embodiments, the supercharged protein is non-covalently associated with the functional effector protein, thus forming a complex. In some embodiments, the supercharged protein has an overall net positive charge. In other embodiments the supercharged protein has an overall net negative charge, and the protein(s) are associated with a cationic lipid. In other embodiments the supercharged protein has an overall net negative charge, and the protein(s) are associated with a cationic polymer. In some embodiments, the overall net positive charge is between about +5 and about +40, or the overall net negative charge is between about -5 and about -50. In some embodiments, the supercharged protein is more positively charged or is more negatively charged at physiological pH than its corresponding unmodified protein. In some embodiments, the corresponding unmodified protein is a naturally occurring protein. In some embodiments, the supercharged protein is at least +5 more positively or is at least -5 more negatively charged at physiological pH than its corresponding unmodified protein. In some embodiments, the supercharged protein is a fluorescent protein. In some embodiments, the supercharged protein is green fluorescent protein (GFP). In some embodiments, the supercharged protein is a superpositively charged GFP. In some embodiments, the supercharged protein is a superpositively charged GFP (+36 GFP) comprising at least 20 contiguous amino acid residues of the sequence:

GGASKGERLFRGKVPILVELKGDVNGHKFSVRGKGKGDATRGKLTLKFICTTGKLPVP
WPTLVTTLTYGVQCFSRYPKHMKRHDFFKSAMPKGYVQERTISFKKDGKYKTRAEVKF

EGRTLVNRIKLKGRDFKEKGNILGHKLRYNFNSHKVYITADKRKNGIKAKFKIRHNVKD
GSVQLADHYQQNTPIGRGPVLLPRNHYLSTRSKLSKDPKEKRDHMVLLEFVTAAGIKHG
RDERYK ( SEQ ID NO: 1).

In some embodiments, the supercharged protein comprises the amino acid sequence set forth in SEQ ID NO: 1. In some embodiments, the supercharged protein consists of the amino acid sequence set forth in SEQ ID NO: 1. In some embodiments, the composition is a pharmaceutical composition. In some embodiments, the composition comprises a pharmaceutically acceptable excipient. In some embodiments, the composition is formulated for administration to a subject and comprises the supercharged protein and the functional effector protein in an amount effective for delivery to at least one cell of the subject. In some embodiments, the composition comprises the supercharged protein and the functional effector protein in an amount effective for inducing a measurable therapeutic effect after administration to a subject.

Some aspects of the disclosure provide compositions comprising a protein to be delivered associated with a nucleic acid, *e.g.,* with an RNA or DNA, and a cationic lipid or a cationic polymer. It was surprisingly found that when a nucleic acid-binding protein, such as, for example, a Cas9 protein, is associated with a nucleic acid, the complex can be encapsulated by cationic lipids and effectively delivered to cells. In some embodiments, the net charge of the protein and the associated nucleic acid is negative. In some embodiments, the protein alone, *i.e.,* without the associated nucleic acid, is not negatively charged or cannot efficiently be associated with or encapsulated into a cationic polymer or a cationic lipid. In some embodiments, the composition comprises a protein to be delivered associated with a negatively supercharged protein (*e.g.,* a supernegatively charged GFP or a supernegatively charged streptavidin) and a cationic lipid or cationic polymer, which also provides for effective delivery to a cell. In some embodiments, the association between the protein to be delivered and the supernegatively charged protein is covalent. For example, in some embodiments, the protein to be delivered and the supernegatively charged protein form a fusion protein. In other embodiments, the association is non-covalent. For example, in some embodiments, the protein to be delivered is conjugated to a first binding agent (*e.g*., biotin), and the supernegatively charged protein is conjugated to a second binding agent (*e.g*., streptavidin) that binds the first binding agent. It will be appreciated that the disclosure is not limited to biotin:streptavidin. Additional suitable methods and binding agents (*e.g.,* additional ligand/receptor pairs, or antibody/antigen pairs) will be apparent to the skilled artisan based on the instant disclosure. In some embodiments, the protein to be delivered is associated with the supernegatively charged protein via a linker, for example, via a cleavable linker. In some embodiments, the linker is cleaved under conditions present in endosomes of the cell into which the protein is to be delivered, thus facilitating endosomal escape of the protein.

Some aspects of the disclosure provide compositions comprising a Cas9 protein associated with a gRNA and a cationic lipid. It was surprisingly found that when a Cas9 protein is associated with a gRNA, the complex can be encapsulated by cationic lipids and effectively delivered to cells. This may be accomplished with or without a supercharged protein. In some embodiments, the composition comprises a Cas9 protein associated with a negatively supercharged protein (*e.g.,* supernegatively charged GFP) and a cationic lipid, which also provides for successful delivery to a cell. In some embodiments, the composition exhibits low toxicity when delivered to a population of cells, for example, wherein at least 60%, at least 65%, at least 70%, at least 75%, at least 80%, at least 85%, at least 90%, at least 95%, or at least 99% of the cells are viable following administration of the composition. In some embodiments, the Cas9 protein is a wild type Cas9 protein, a Cas9 nickase, or comprises a nuclease inactivated (dCas9) protein. In some embodiments, the Cas9 protein is a fusion protein comprising dCas9. In some embodiments, the fusion protein comprises a transcriptional activator (*e.g.,* VP64), a transcriptional repressor (*e.g.,* KRAB, SID) a nuclease domain (*e.g.,* FokI), a recombinase domain (*e.g.,* Hin, Gin, or Tn3), a deaminase (*e.g.,* a cytidine deaminase or an adenosine deaminase) or an epigenetic modifier domain (*e.g.,* TET1).

Other aspects of the disclosure provide compositions comprising a Cas9 protein associated with a gRNA and a cationic polymer. As with cationic lipids, when a Cas9 protein is associated with a gRNA, the complex can associate with cationic polymers and be effectively delivered to cells. This may be accomplished with or without a supercharged protein. In some embodiments, the composition comprises a Cas9 protein associated with a negatively supercharged protein (*e.g.,* supernegatively charged GFP) and a cationic polymer, which also provides for successful delivery to a cell. In some embodiments, the composition exhibits low toxicity when delivered to a population of cells, for example, wherein at least 60%, at least 65%, at least 70%, at least 75%, at least 80%, at least 85%, at least 90%, at least 95%, or at least 99% of the cells are viable following administration of the composition. In some embodiments, the Cas9 protein is a wild type Cas9 protein, a Cas9 nickase, or comprises a nuclease inactivated (dCas9) protein. In some embodiments, the Cas9 protein is a fusion protein comprising dCas9. In some embodiments, the fusion protein comprises a transcriptional activator (*e.g*., VP64), a transcriptional repressor (*e.g.,* KRAB, SID) a nuclease domain (*e.g.,* FokI), a recombinase domain (*e.g.,* Hin, Gin, or Tn3), a deaminase (*e.g.,* a cytidine deaminase or an adenosine deaminase) or an epigenetic modifier domain (*e.g*., LSD1, TET1).

Some aspects of this disclosure provide methods for administering a composition provided herein to a subject. In some embodiments, the method comprises administering a composition described herein to a subject, for example, a composition comprising a protein to be delivered and a cationic polymer or cationic lipid. In some embodiments, the subject is susceptible to, is suffering from, or is displaying one or more symptoms of a disease, disorder, or condition. In some embodiments, the composition is administered to the subject in an amount sufficient and under suitable conditions for at least one sign or symptom to be ameliorated as a result of the administration. In some embodiments, the protein to be delivered is a protein implicated or known to be involved in a disease, disorder, or condition, for example, a protein listed in any of Tables 4-6. In some embodiments, the step of administering is performed under conditions sufficient for the functional effector protein to penetrate a cell of the subject. In some embodiments, the disease, disorder, or condition is associated with abnormally elevated levels of an mRNA, a protein, or combination thereof. For example, in some embodiments, the disease, disorder, or condition is associated with abnormally low levels or reduced activity of the protein to be delivered, wherein the protein to be delivered is a protein listed in any of Tables 3-6. In some embodiments, the composition comprises a nuclease that specifically binds and cleaves a genomic sequence, for example, a normal or a pathogenic allele; a gene associated with susceptibility to, or onset or progression of, a disease; a gene encoding a pathogenic RNA or protein; or a gene encoding an RNA or protein that is expressed at abnormally high levels in diseased cells or tissue. In some embodiments, the step of administering comprises a route of administration selected from the group consisting of oral, intravenous, intramuscular, intraarterial, subcutaneous, intraventricular, topical, inhalational, and mucosal delivery.

Some aspects of this disclosure provide methods for introducing a protein to be delivered into a cell. In some embodiments, the method comprises contacting the cell with a composition described herein, *e.g.,* with a composition comprising the protein to be delivered and a cationic polymer or cationic lipid, under conditions suitable for the protein to enter the cell, thereby introducing the protein into the cell. In some embodiments, the protein to be delivered is a negatively charged protein, for example, a naturally negatively charged protein or an engineered supernegatively charged protein. In some embodiments, the protein to be delivered is associated with a nucleic acid. In some embodiments, the protein to be delivered is associated with a negatively charged protein. The association may be covalent or non-covalent.

For example, in some embodiments, the method comprises contacting the cell with a composition comprising a Cas9 protein and a cationic lipid and/or cationic polymer under conditions suitable for the Cas9 protein to enter the cell, thereby introducing the Cas9 protein into the cell. In some embodiments, the Cas9 protein enters the nucleus of the cell, for example the Cas9 protein is directed to the nucleus by including a nuclear localization signal (NLS) in the protein. In some embodiments, the method further comprises confirming that the functional effector protein (*e.g.,* including Cas9) has penetrated the cell. In some embodiments, the cell is in a subject, and the contacting is done *in vivo.* In some embodiments, the subject is diagnosed with having or being at risk of developing a disease associated with an abnormal expression level of a gene, and wherein the functional effector protein (*e.g.,* including Cas9) modulates the expression level of the gene. In some embodiments, the method further comprises detecting a change in the level of expression of the gene or detecting a therapeutic response in the subject. In some embodiments, the cell is a somatic cell. In some embodiments, the cell is contacted with the composition or the pharmaceutical composition in an amount, for a time, and under conditions sufficient to induce programming of the cell to a desired cell fate. In some embodiments, the method further comprises using the programmed cell in a cell replacement therapeutic approach. In some embodiments, the cell is a cell carrying a genomic allele associated with a disease and the functional effector protein specifically targets the allele. In some embodiments, the cell is contacted *ex vivo* and re-administered to the subject after successful targeting of the undesired allele by the functional effector protein.

Some aspects of this disclosure provide kits comprising a composition as described herein, for example, a composition comprising a supercharged protein associated with a functional effector protein. In some embodiments, the kits comprises a Cas9 protein and a supercharged protein. In some embodiments, the kits comprises a Cas9 protein and a cationic lipid. In some embodiments, the kits comprises a Cas9 protein and a cationic polymer. In some embodiments, the kit further comprises instructions for using the components included in the kit.

These and other aspects and embodiments of the invention, as well as various advantages and utilities will be more apparent with respect to the drawings and detailed description of the invention.

### DEFINITIONS

As used herein and in the claims, the singular forms "a," "an," and "the" include the singular and the plural reference unless the context clearly indicates otherwise. Thus, for example, a reference to "an agent" includes a single agent and a plurality of agents.

The term "associated with" as used herein in the context of two or more moieties (*e.g.*, proteins or protein domains) refers to the fact that the moieties are physically associated with or connected to one another, either directly or via one or more additional moieties that serve as a linking agent, to form a structure that is sufficiently stable so that the moieties remain physically associated under the conditions in which the structure is used, *e.g.,* under physiological conditions. Supercharged proteins may be associated with functional effector proteins (*e.g.,* enzymes (*e.g.,* oxidoreductases, transferases, hydrolases, lyases, isomerases, or ligases); transcriptional activators, transcriptional repressors, genome editing proteins, Cas9 proteins, TALEs, TALENs, nucleases, binding proteins (*e.g.,* ligands, receptors, antibodies, antibody fragments; nucleic acid binding proteins, *etc.*); structural proteins; therapeutic proteins (*e.g*., tumor suppressor proteins, therapeutic enzymes, growth factors, growth factor receptors, transcription factors, proteases, *etc.*)*,* as well as variants and fusions thereof) through non-covalent interactions (*e.g.,* electrostatic interactions). In certain embodiments, a supercharged protein may be associated with a functional effector protein through electrostatic interactions to form a complex. In some embodiments, a sufficient number of weaker interactions can provide sufficient stability for moieties to remain physically associated under a variety of different conditions. In certain embodiments, a supercharged protein is associated with a functional effector protein via a covalent bond (*e.g.,* an amide bond). In some embodiments, a functional effector protein is associated with a supercharged protein directly by a peptide bond, or indirectly via a linker.

The term "Cas9" or "Cas9 nuclease" refers to an RNA-guided nuclease comprising a Cas9 protein, or a fragment thereof (*e.g.,* a protein comprising an active or inactive DNA cleavage domain of Cas9 or a partially inactive DNA cleavage domain (*e.g.,* a Cas9 "nickase"), and/or the gRNA binding domain of Cas9). In some embodiments, the term "Cas9" refers to a fusion protein comprising Cas9 or a fragment thereof.

In some embodiments, Cas9 refers to Cas9 from: *Corynebacterium ulcerans* (NCBI Refs: NC_015683.1, NC_017317.1); *Corynebacterium diphtheria* (NCBI Refs: NC_016782.1, NC_016786.1); *Spiroplasma syrphidicola* (NCBI Ref: NC_021284.1); *Prevotella intermedia* (NCBI Ref: NC_017861.1); *Spiroplasma taiwanense* (NCBI Ref: NC_021846.1); *Streptococcus iniae* (NCBI Ref: NC_021314.1); *Belliella baltica* (NCBI Ref: NC_018010.1); *Psychroflexus torquisI* (NCBI Ref: NC_018721.1); *Streptococcus thermophilus* (NCBI Ref: YP_820832.1); *Listeria innocua* (NCBI Ref: NP_472073.1); *Campylobacter jejuni* (NCBI Ref: YP_002344900.1); or *Neisseria. meningitidis* (NCBI Ref: YP_002342100.1).

The term "cationic lipid" refers to a lipid which has a cationic, or positive, charge at physiologic pH. Cationic lipids can take a variety of forms including, but not limited to, liposomes or micelles. Cationic lipids useful for certain aspects of the present disclosure are known in the art, and, generally comprise both polar and non-polar domains, bind to polyanions, such as nucleic acid molecules or negatively supercharged proteins, and are typically known to facilitate the delivery of nucleic acids into cells. Examples of useful cationic lipids include polyethylenimine, polyamidoamine (PAMAM) starburst dendrimers, Lipofectin (a combination of DOTMA and DOPE), Lipofectase, LIPOFECTAMINE^{®} (*e.g.,* LIPOFECTAMINE^{®} 2000, LIPOFECTAMINE^{®} 3000, LIPOFECTAMINE^{®} RNAiMAX, LIPOFECTAMINE^{®} LTX), SAINT-RED (Synvolux Therapeutics, Groningen Netherlands), DOPE, Cytofectin (Gilead Sciences, Foster City, Calif), and Eufectins (JBL, San Luis Obispo, Calif.). Exemplary cationic liposomes can be made from N-[1-(2,3-dioleoloxy)-propyl]-N,N,N-trimethylammonium chloride (DOTMA), N-[1 -(2,3-dioleoloxy)-propyl]-N,N,N-trimethylammonium methylsulfate (DOTAP), 3β-[N-(N',N'-dimethylaminoethane)carbamoyl]cholesterol (DC-Chol), 2,3,-dioleyloxy-N-[2(sperminecarboxamido)ethyl]-N,N-dimethyl-1-propanaminium trifluoroacetate (DOSPA), 1,2-dimyristyloxypropyl-3-dimethyl-hydroxyethyl ammonium bromide; and dimethyldioctadecylammonium bromide (DDAB). Cationic lipids have been used in the art to deliver nucleic acid molecules to cells (see, *e.g.,* U.S. Pat. Nos. 5,855,910; 5,851,548; 5,830,430; 5,780,053; 5,767,099; 8,569,256; 8,691,750; 8,748,667; 8,758,810; 8,759,104; 8,771,728; Lewis et al. 1996. Proc. Natl. Acad. Sci. USA 93:3176; Hope et al. 1998. Molecular Membrane Biology 15:1). In addition, other lipid compositions are also known in the art and include, *e.g.,* those taught in U.S. Pat. No. 4,235,871; U.S. Pat. Nos. 4,501,728; 4,837,028; 4,737,323.

The term "cationic polymer," as used herein, refers to a polymer having a net positive charge. Cationic polymers are well known in the art, and include those described in Samal et al., Cationic polymers and their therapeutic potential. Chem Soc Rev. 2012 Nov 7;41(21):7147-94; in published U.S. patent applications U.S. 2014/0141487 A1, U.S. 2014/0141094 A1, U.S. 2014/0044793 A1, U.S. 2014/0018404 A1, U.S. 2014/0005269 A1, and U.S. 2013/0344117 A1; and in U.S. Pat. Nos. 8,709,466; 8,728,526; 8,759,103; and 8,790,664; the entire contents of each are incorporated herein by reference. Exemplary cationic polymers include, but are not limited to, polyallylamine (PAH); polyethyleneimine (PEI); poly(L-lysine) (PLL); poly(L-arginine) (PLA); polyvinylamine homo- or copolymer; a poly(vinylbenzyl-tri-C₁-C₄-alkylammonium salt); a polymer of an aliphatic or araliphatic dihalide and an aliphatic N,N,N',N'-tetra-C₁-C₄-alkyl-alkylenediamine; a poly(vinylpyridin) or poly(vinylpyridinium salt); a poly(N,N-diallyl-N,N-di-C₁-C₄-alkyl-ammoniumhalide); a homo- or copolymer of a quaternized di-C₁-C₄-alkyl-aminoethyl acrylate or methacrylate; POLYQUAD^{™}; a polyaminoamide; and the like.

The term "click chemistry" refers to a chemical philosophy introduced by K. Barry Sharpless of The Scripps Research Institute, describing chemistry tailored to generate covalent bonds quickly and reliably by joining small units comprising reactive groups together. Click chemistry does not refer to a specific reaction, but to a concept including reactions that mimic reactions found in nature. See, *e.g.,* Kolb, Finn and Sharpless Angewandte Chemie International Edition (2001) 40: 2004-2021; Evans, Australian Journal of Chemistry (2007) 60: 384-395, and Joerg Lahann, Click Chemistry for Biotechnology and Materials Science, 2009, John Wiley & Sons Ltd, ISBN 978-0-470-69970-6, the entire contents of each of which are incorporated herein by reference. In some embodiments, click chemistry reactions are modular, wide in scope, give high chemical yields, generate inoffensive byproducts, are stereospecific, exhibit a large thermodynamic driving force > 84 kJ/mol to favor a reaction with a single reaction product, and/or can be carried out under physiological conditions. A distinct exothermic reaction makes a reactant "spring loaded". In some embodiments, a click chemistry reaction exhibits high atom economy, can be carried out under simple reaction conditions, use readily available starting materials and reagents, uses no toxic solvents or use a solvent that is benign or easily removed (preferably water), and/or provides simple product isolation by non-chromatographic methods (crystallization or distillation).

The term "deaminase" refers to an enzyme that catalyzes a deamination reaction. In some embodiments, the deaminase is a cytidine deaminase, catalyzing the hydrolytic deamination of cytidine or deoxycytidine to uracil or deoxyuracil, respectively.

The term "effective amount," as used herein, refers to an amount of a biologically active agent that is sufficient to elicit a desired biological response. For example, in some embodiments, an effective amount of a functional effector protein (*e.g.,* nucleases, transcriptional activators/repressors, recombinases, Cas9 proteins including variants and fusions thereof, *etc.*) may refer to the amount of the protein that is sufficient to induce a detectable effect (*e.g.,* cleavage of a target site, modification of a target site, modulation of gene expression, *etc.*)*.* Such an effect may be detected in a suitable assay, *e.g.,* in a cell-free assay, or in a target cell, tissue, or subject organism. As will be appreciated by the skilled artisan, the effective amount of an agent, *e.g.,* a functional effector protein, may vary depending on various factors as, for example, on the desired biological response, the specific allele to be targeted, the genome, target site, cell, or tissue being targeted, and the supercharged protein being used.

The term "effector protein" refers to a protein that modulates a biological function of a cell when introduced into the cell, *e.g.,* a modification of a nucleic acid molecule in the cell (such as a cleavage, deamination, recombination, *etc.*)*,* or a modulation (*e.g.,* increases or decreases) the expression or the expression level of a gene in the cell.

The term "engineered," as used herein refers to a protein molecule, complex, substance, or entity that has been designed, produced, prepared, synthesized, and/or manufactured by a human. Accordingly, an engineered product is a product that does not occur in nature. In some embodiments, an engineered protein or composition, *e.g.,* an engineered supercharged protein associated with a functional effector protein, such as a nuclease, Cas9 protein (including variants and fusions thereof) is a supercharged protein that has been designed to meet particular requirements or to have particular desired features, *e.g.,* to have a specified net charge, to specifically bind and/or cleave or modify a target sequence of interest, to have a specific minimal or maximal cleavage or enzymatic activity, and/or to have a specific stability.

The term "epigenetic modifier," as used herein, refers to a protein or catalytic domain thereof having enzymatic activity that results in the epigenetic modification of DNA, for example chromosomal DNA. Epigenetic modifications include, but are not limited to DNA methylation and demethylation; histone modifications including methylation and demethylation (*e.g.,* mono-, di- and tri-methylation), histone acetylation and deacetylation, as well we histone ubiquitylation, phosphorylation, and sumoylation.

The term "functional protein" refers to a protein that is in a form in which it exhibits a property and/or activity by which it is characterized.

The term "fusion protein" refers to a protein comprising a plurality of heterologous proteins, protein domains, or peptides, *e.g.,* a supercharged protein and a functional effector protein, associated with each other via a peptide linkage, thus forming a single amino acid sequence. In certain embodiments, a fusion protein is encoded by a gene.

The term "gene" has its meaning as understood in the art. It will be appreciated by those of ordinary skill in the art that the term "gene" may include gene regulatory sequences (*e.g.,* promoters, enhancers, *etc.*) and/or intron sequences. It will further be appreciated that definitions of gene include references to nucleic acids that do not encode proteins but rather encode functional RNA molecules such as RNAi agents, ribozymes, tRNAs, *etc.* For the purpose of clarity it should be noted that, as used in the present application, the term "gene" generally refers to a portion of a nucleic acid that encodes a protein; the term may optionally encompass regulatory sequences, as will be clear from context to those of ordinary skill in the art. This definition is not intended to exclude application of the term "gene" to non-protein-coding expression units but rather to clarify that, in most cases, the term as used in this document refers to a protein-coding nucleic acid.

The term "isolated" refers to a molecule, complex, substance, or entity that has been (1) separated from at least some of the components with which it was associated when initially produced (whether in nature or in an experimental setting), and/or (2) produced, prepared, synthesized, and/or manufactured by a human. Isolated substances and/or entities may be separated from at least about 10%, about 20%, about 30%, about 40%, about 50%, about 60%, about 70%, about 80%, about 90%, or more of the other components with which they were initially associated. In some embodiments, isolated agents are more than about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components.

The term "linker," as used herein, refers to a chemical group or a molecule linking two molecules or moieties, *e.g.,* a supercharged protein and a nuclease. Typically, the linker is positioned between, or flanked by, two groups, molecules, or other moieties and connected to each one via a covalent bond, thus connecting the two. In some embodiments, the linker comprises an amino acid or a plurality of amino acids (*e.g.,* a peptide or protein). In some embodiments, the linker is an organic molecule, group, polymer, or chemical moiety. In some embodiments, the linker is a cleavable linker, *e.g.,* the linker comprises a bond that can be cleaved upon exposure to a cleaving activity, such as UV light or a hydrolytic enzyme, such as a lysosomal protease. In some embodiments, the linker is any stretch of amino acids having at least 1, at least 2, at least 3, at least 4, at least 5, at least 6, at least 7, at least 8, at least 9, at least 10, at least 15, at least 20, at least 25, at least 30, at least 40, at least 50, or more amino acids. In some embodiments, the peptide linker comprises repeats of the tri-peptide Gly-Gly-Ser, *e.g.,* comprising the sequence (GGS)ₙ, wherein n represents at least 1, 2, 3, 4, 5, 6, 7, 8, 9, 10, or more repeats. In some embodiments, the linker comprises the sequence (GGS)₆ (SEQ ID NO:2). In some embodiments, the peptide linker is the 16 residue "XTEN" linker, or a variant thereof (See, *e.g.,* Schellenberger et al. A recombinant polypeptide extends the in vivo half-life of peptides and proteins in a tunable manner. Nat. Biotechnol. 27, 1186-1190 (2009)). In some embodiments, the XTEN linker comprises the sequence SGSETPGTSESATPES (SEQ ID NO:3), SGSETPGTSESA (SEQ ID NO:4), or SGSETPGTSESATPEGGSGGS (SEQ ID NO:5). In some embodiments, the peptide linker is one or more selected from VPFLLEPDNINGKTC (SEQ ID NO:6), GSAGSAAGSGEF (SEQ ID NO:7), SIVAQLSRPDPA (SEQ ID NO:8), MKIIEQLPSA (SEQ ID NO:9), VRHKLKRVGS (SEQ ID NO:10), GHGTGSTGSGSS (SEQ ID NO: 11), MSRPDPA (SEQ ID NO: 12); or GGSM (SEQ ID NO:13).

The term "nuclease," as used herein, refers to an agent, for example, a protein or a small molecule, capable of cleaving a phosphodiester bond connecting nucleotide residues in a nucleic acid molecule. In some embodiments, a nuclease is a protein, e.g., an enzyme that can bind a nucleic acid molecule and cleave a phosphodiester bond connecting nucleotide residues within the nucleic acid molecule. A nuclease may be an endonuclease, cleaving a phosphodiester bond within a polynucleotide chain, or an exonuclease, cleaving a phosphodiester bond at the end of the polynucleotide chain. In some embodiments, a nuclease is a site-specific nuclease, binding and/or cleaving a specific phosphodiester bond within a specific nucleotide sequence, which is also referred to herein as the "recognition sequence," the "nuclease target site," or the "target site." In some embodiments, a nuclease recognizes a single stranded target site, while in other embodiments, a nuclease recognizes a double-stranded target site, for example, a double-stranded DNA target site. The target sites of many naturally occurring nucleases, for example, many naturally occurring DNA restriction nucleases, are well known to those of skill in the art. In many cases, a DNA nuclease, such as EcoRI, HindIII, or BamHI, recognize a palindromic, double-stranded DNA target site of 4 to 10 base pairs in length, and cut each of the two DNA strands at a specific position within the target site. Some endonucleases cut a double-stranded nucleic acid target site symmetrically, *i.e.,* cutting both strands at the same position so that the ends comprise base-paired nucleotides, also referred to herein as blunt ends. Other endonucleases cut a double-stranded nucleic acid target site asymmetrically, *i.e.,* cutting each strand at a different position so that the ends comprise unpaired nucleotides. Unpaired nucleotides at the end of a double-stranded DNA molecule are also referred to as "overhangs," *e.g.,* as "5'-overhang" or as "3'-overhang," depending on whether the unpaired nucleotide(s) form(s) the 5' or the 3' end of the respective DNA strand. Double-stranded DNA molecule ends ending with unpaired nucleotide(s) are also referred to as sticky ends, as they can "stick to" other double-stranded DNA molecule ends comprising complementary unpaired nucleotide(s). A nuclease protein typically comprises a "binding domain" that mediates the interaction of the protein with the nucleic acid substrate, and a "cleavage domain" that catalyzes the cleavage of the phosphodiester bond within the nucleic acid backbone. In some embodiments, a nuclease protein can bind and cleave a nucleic acid molecule in a monomeric form, while, in other embodiments, a nuclease protein has to dimerize or multimerize in order to cleave a target nucleic acid molecule. Binding domains and cleavage domains of naturally occurring nucleases, as well as modular binding domains and cleavage domains that can be combined to create nucleases that bind specific target sites, are well known to those of skill in the art. For example, transcriptional activator like elements can be used as binding domains to specifically bind a desired target site, and fused or conjugated to a cleavage domain, for example, the cleavage domain of FokI, to create an engineered nuclease cleaving the desired target site.

The term "nucleic acid" and the term "nucleic acid molecule," as used interchangeably herein, refer to a compound comprising a nucleoside, a nucleotide, or a polymer of nucleotides. Typically, polymeric nucleic acids, *e.g.,* nucleic acid molecules comprising three or more nucleotides are linear molecules, in which adjacent nucleotides are linked to each other via a phosphodiester linkage. In some embodiments, "nucleic acid" refers to individual nucleic acid residues (*e.g.* nucleotides and/or nucleosides). In some embodiments, "nucleic acid" refers to an oligonucleotide chain comprising three or more individual nucleotide residues. As used herein, the terms "oligonucleotide" and "polynucleotide" can be used interchangeably to refer to a polymer of nucleotides (*e.g.,* a string of at least three nucleotides). In some embodiments, "nucleic acid" encompasses RNA as well as single and/or double-stranded DNA. Nucleic acids may be naturally occurring, for example, in the context of a genome, a transcript, an mRNA, tRNA, rRNA, siRNA, snRNA, a plasmid, cosmid, chromosome, chromatid, or other naturally occurring nucleic acid molecule. On the other hand, a nucleic acid molecule may be a non-naturally occurring molecule, *e.g.,* a recombinant DNA or RNA, an artificial chromosome, an engineered genome, or fragment thereof, or a synthetic DNA, RNA, DNA/RNA hybrid, or including non-naturally occurring nucleotides or nucleosides. Furthermore, the terms "nucleic acid," "DNA," "RNA," and/or similar terms include nucleic acid analogs, *i.e.* analogs having other than a phosphodiester backbone. Nucleic acids can be purified from natural sources, produced using recombinant expression systems and optionally purified, chemically synthesized, *etc.* Where appropriate, *e.g.,* in the case of chemically synthesized molecules, nucleic acids can comprise nucleoside analogs such as analogs having chemically modified bases or sugars, and backbone modifications. A nucleic acid sequence is presented in the 5' to 3' direction unless otherwise indicated. In some embodiments, a nucleic acid is or comprises natural nucleosides (*e.g.* adenosine, thymidine, guanosine, cytidine, uridine, deoxyadenosine, deoxythymidine, deoxyguanosine, and deoxycytidine); nucleoside analogs (*e.g.,* 2-aminoadenosine, 2-thiothymidine, inosine, pyrrolo-pyrimidine, 3-methyl adenosine, 5-methylcytidine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g.,* methylated bases); intercalated bases; modified sugars (*e.g.,* 2'-fluororibose, ribose, 2'-deoxyribose, arabinose, and hexose); and/or modified phosphate groups (*e.g.,* phosphorothioates and 5'-*N*-phosphoramidite linkages).

The term "pharmaceutical composition," as used herein, refers to a composition that can be administrated to a subject, for example, in the context of treatment of a disease or disorder. In some embodiments, a pharmaceutical composition comprises an active ingredient, *e.g.,* a supercharged protein associated with a functional effector protein, such as a nuclease, or a nucleic acid encoding a supercharged protein and a functional effector protein, *e.g.,* in the form of a fusion protein, and a pharmaceutically acceptable excipient.

The term "physiological pH" as used herein refers to a pH value that is found in a normal, non-pathologic cell or subject. In some embodiments, physiological pH is between pH 5 - 8. In some embodiments, physiological pH is pH 7-7.5, for example, pH 7.0, pH 7.1, pH 7.2, pH 7.3, pH 7.4, or pH 7.5. In some embodiments, physiological pH is pH 6.5-7.5. In some embodiments, physiological pH is pH 5, pH 5.5, pH 6, pH 6.5, pH 7, pH 7.5, or pH 8.

The term "prevention" or "prevent" refer to the prophylactic treatment of a subject who is at risk of developing a disease, disorder, or condition (*e.g.,* at an elevated risk as compared to a control subject, or a control group of subject, or at an elevated risk as compared to the average risk of an age-matched and/or gender-matched subject), resulting in a decrease in the probability that the subject will develop the disease, disorder, or condition (as compared to the probability without prevention), and/or to the inhibition of further advancement of an already established disorder.

The term "proliferative disease," as used herein, refers to any disease in which cell or tissue homeostasis is disturbed in that a cell or cell population exhibits an abnormally elevated proliferation rate. Proliferative diseases include hyperproliferative diseases, such as pre-neoplastic hyperplastic conditions and neoplastic diseases. Neoplastic diseases are characterized by an abnormal proliferation of cells and include both benign and malignant neoplasias. Malignant neoplasms are also referred to as cancers.

The term "protein" is interchangeably used herein with the terms "peptide" and "polypeptide" and refers to a polymer of amino acid residues linked together by peptide (amide) bonds. The terms refer to a protein, peptide, or polypeptide of any size, structure, or function. Typically, a protein, peptide, or polypeptide will be at least three amino acids long. A protein, peptide, or polypeptide may refer to an individual protein or a collection of proteins. One or more of the amino acids in a protein, peptide, or polypeptide may be modified, for example, by the addition of a chemical entity such as a carbohydrate group, a hydroxyl group, a phosphate group, a farnesyl group, an isofarnesyl group, a fatty acid group, a linker for conjugation, functionalization, or other modification, *etc.* A protein, peptide, or polypeptide may also be a single molecule or may be a multi-molecular complex. A protein, peptide, or polypeptide may be just a fragment of a naturally occurring protein or peptide. A protein, peptide, or polypeptide may be naturally occurring, recombinant, or synthetic, or any combination thereof. A protein may comprise different domains, for example, a TALE effector protein may comprise a nucleic acid binding domain and an effector domain, *e.g.,* a nucleic acid cleavage domain or a transcriptional activator or repressor domain. In some embodiments, a protein comprises a proteinaceous part, *e.g.,* an amino acid sequence constituting a nucleic acid binding domain, and an organic compound, *e.g.,* a compound that can act as a nucleic acid cleavage agent.

The term "protein to be delivered," also sometimes referred to herein as "effector protein" or "functional effector protein," refers to a protein that is to be delivered to a target cell. The protein may be any type of protein, including, for example, enzymes (*e.g.,* oxidoreductases, transferases, hydrolases, lyases, isomerases, or ligases); transcriptional activators, transcriptional repressors, genome editing proteins, Cas9 proteins, TALEs, TALENs, nucleases, binding proteins (*e.g.,* ligands, receptors, antibodies, antibody fragments; nucleic acid binding proteins, *etc.*)*;* structural proteins; therapeutic proteins (*e.g.,* tumor suppressor proteins, therapeutic enzymes, growth factors, growth factor receptors, transcription factors, proteases, *etc.*)*,* fluorescent proteins, as well as variants and fusions thereof.

The term "RNA-programmable nuclease," and "RNA-guided nuclease" are used interchangeably herein and refer to a nuclease that forms a complex with (*e.g.,* binds or associates with) one or more RNA molecule that is not a target for cleavage. In some embodiments, an RNA-programmable nuclease, when in a complex with an RNA, may be referred to as a nuclease:RNA complex. RNA-programmable nucleases include Cas9. Typically, the bound RNA(s) is referred to as a guide RNA (gRNA). gRNAs can exist as a complex of two or more RNAs, or as a single RNA molecule. gRNAs that exist as a single RNA molecule may be referred to as single-guide RNAs (sgRNAs), though "gRNA" is used interchangeably to refer to guide RNAs that exist as either single molecules or as a complex of two or more molecules. Typically, gRNAs that exist as single RNA species comprise two domains: (1) a domain that shares homology to a target nucleic acid (*e.g.,* and directs binding of a Cas9 complex to the target); and (2) a domain that binds a Cas9 protein. The gRNA comprises a nucleotide sequence that complements a target site, which mediates binding of the nuclease/RNA complex to said target site and providing the sequence specificity of the nuclease:RNA complex.

The term "recombinase," as used herein, refers to a site-specific enzyme that mediates the recombination of DNA between recombinase recognition sequences, which results in the excision, integration, inversion, or exchange (*e.g.,* translocation) of DNA fragments between the recombinase recognition sequences. Recombinases can be classified into two distinct families: serine recombinases (*e.g.,* resolvases and invertases) and tyrosine recombinases (*e.g.,* integrases). Examples of serine recombinases include, without limitation, Hin, Gin, Tn3, β-six, CinH, ParA, γδ, Bxb1, φC31, TP901, TG1, ϕBT1, R4, ϕRV1, ϕFC1, MR11, A118, U153, and gp29. Examples of tyrosine recombinases include, without limitation, Cre, FLP, R, Lambda, HK101, HK022, and pSAM2. The serine and tyrosine recombinase names stem from the conserved nucleophilic amino acid residue that the recombinase uses to attack the DNA and which becomes covalently linked to the DNA during strand exchange. Recombinases have numerous applications, including the creation of gene knockouts/knock-ins and gene therapy applications. See, *e.g.,* Brown et al., "Serine recombinases as tools for genome engineering." Methods. 2011;53(4):372-9; Hirano et al., "Site-specific recombinases as tools for heterologous gene integration." Appl. Microbiol. Biotechnol. 2011; 92(2):227-39; Chavez and Calos, "Therapeutic applications of the ΦC31 integrase system." Curr. Gene Ther. 2011;11(5):375-81; Turan and Bode, "Site-specific recombinases: from tag-and-target- to tag-and-exchange-based genomic modifications." FASEB J. 2011; 25(12):4088-107; Venken and Bellen, "Genome-wide manipulations of Drosophila melanogaster with transposons, Flp recombinase, and ΦC31 integrase." Methods Mol. Biol. 2012; 859:203-28; Murphy, "Phage recombinases and their applications." Adv. Virus Res. 2012; 83:367-414; Zhang et al., "Conditional gene manipulation: Cre-ating a new biological era." J. Zhejiang Univ. Sci. B. 2012; 13(7):511-24; Karpenshif and Bernstein, "From yeast to mammals: recent advances in genetic control of homologous recombination." DNA Repair (Amst). 2012; 1;11(10):781-8; the entire contents of each are hereby incorporated by reference in their entirety. The recombinases provided herein are not meant to be exclusive examples of recombinases that can be used in embodiments of the invention. The methods and compositions of the invention can be expanded by mining databases for new orthogonal recombinases or designing synthetic recombinases with defined DNA specificities (See, *e.g.,* Groth et al., "Phage integrases: biology and applications." J. Mol. Biol. 2004; 335, 667-678; Gordley et al., "Synthesis of programmable integrases." Proc. Natl. Acad. Sci. USA. 2009; 106, 5053-5058; the entire contents of each are hereby incorporated by reference in their entirety). Other examples of recombinases that are useful in the methods and compositions described herein are known to those of skill in the art, and any new recombinase that is discovered or generated is expected to be able to be used in the different embodiments of the invention. In some embodiments, a recombinase (or catalytic domain thereof) is fused to a Cas9 protein (*e.g.,* dCas9).

The term "recombine," or "recombination," in the context of a nucleic acid modification (*e.g.,* a genomic modification), is used to refer to the process by which two or more nucleic acid molecules, or two or more regions of a single nucleic acid molecule, are modified by the action of a recombinase protein. Recombination can result in, *inter alia,* the insertion, inversion, excision, or translocation of a nucleic acid sequence, *e.g.,* in or between one or more nucleic acid molecules.

The term "subject," as used herein, refers to an individual organism. In some embodiments, the subject is a human of either sex at any stage of development. In some embodiments, the subject is a non-human mammal. In some embodiments, the subject is a non-human primate. In some embodiments, the subject is a rodent. In some embodiments, the subject is a laboratory animal, for example, a mouse, a rat, a gerbil, a guinea pig, a fish, a frog, or a fly. In some embodiments, the subject is a farm animal, for example, a sheep, a goat, a pig, or a cattle. In some embodiments, the subject is a companion animal, for example, a cat or a dog. In some embodiments, the subject is a vertebrate, an amphibian, a reptile, a fish, an insect, a fly, or a nematode. In some embodiments, the subject is genetically engineered, *e.g.,* a genetically engineered non-human subject.

The term "supercharge" refers to any modification of a protein that results in the increase or decrease of the overall net charge of the protein. Modifications include, but are not limited to, alterations in amino acid sequence or addition of charged moieties (*e.g.,* carboxylic acid groups, phosphate groups, sulfate groups, amino groups). Supercharging also refers to the association of an agent with a charged protein, naturally occurring or modified, to form a complex with increased or decreased charge relative to the agent alone.

The term "target site," as used herein in the context of functional effector proteins that bind a nucleic acid molecule, such as nucleases and transcriptional activators or repressors, refers to a sequence within a nucleic acid molecule that is bound and acted upon by the effector protein, *e.g.,* cleaved by the nuclease or transcriptionally activated or repressed by the transcriptional activator or repressor, respectively. A target site may be single-stranded or double-stranded. In the context of RNA-guided (*e.g.,* RNA-programmable) nucleases (*e.g.,* Cas9), a target site typically comprises a nucleotide sequence that is complementary to the gRNA of the RNA-programmable nuclease, and a protospacer adjacent motif (PAM) at the 3' end adjacent to the gRNA-complementary sequence. For the RNA-guided nuclease Cas9 (or variants or fusions comprising having gRNA binding activity), the target site may be, in some embodiments, 20 base pairs plus a 3 base pair PAM (*e.g.,* NNN, wherein N represents any nucleotide). Typically, the first nucleotide of a PAM can be any nucleotide, while the two downstream nucleotides are specified depending on the specific RNA-guided nuclease. Exemplary target sites for RNA-guided nucleases, such as Cas9, are known to those of skill in the art and include, without limitation, NNG, NGN, NAG, and NGG, wherein N represents any nucleotide. In addition, Cas9 nucleases from different species (*e.g., S. thermophilus* instead of *S. pyogenes*) recognizes a PAM that comprises the sequence NGGNG. Additional PAM sequences are known, including, but not limited to, NNAGAAW and NAAR (see, *e.g*., Esvelt and Wang, *Molecular Systems Biology,* 9:641 (2013), the entire contents of which are incorporated herein by reference). For example, the target site of an RNA-guided nuclease, such as, *e.g.,* Cas9, may comprise the structure [NZ]-[PAM], where each N is, independently, any nucleotide, and Z is an integer between 1 and 50, inclusive. In some embodiments, Z is at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50. In some embodiments, Z is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48,49, or 50. In some embodiments, Z is 20. In some embodiments, "target site" may also refer to a sequence within a nucleic acid molecule that is bound but not cleaved by a nuclease. For example, certain embodiments described herein provide proteins comprising an inactive (or inactivated) Cas9 DNA cleavage domain. Such proteins (*e.g.,* when also including a Cas9 RNA binding domain) are able to bind the target site specified by the gRNA, however because the DNA cleavage site is inactivated, the target site is not cleaved by the particular protein. However, such proteins as described herein are typically conjugated, fused, or bound by another protein (*e.g.,* a nuclease, transcriptional activator, recombinase, deaminase, *etc.*) or molecule that mediates modification of the nucleic acid molecule. In some embodiments, the sequence actually cleaved will depend on the protein (*e.g.,* nuclease) or molecule that mediates cleavage of the nucleic acid molecule, and in some cases, for example, will relate to the proximity or distance from which the inactivated Cas9 protein(s) is/are bound. In the context of nucleases that dimerize, for example, dimers of a protein comprising an inactive Cas9 (or a Cas9 RNA binding domain) and a DNA cleavage domain (*e.g.,* FokI cleavage domain or an active Cas9 cleavage domain), a target sites typically comprises a left-half site (bound by one protein), a right-half site (bound by the second protein), and a spacer sequence between the half sites in which the cut is made. In some embodiments, either the left-half site or the right half-site (and not the spacer sequence) is cut. This structure ([left-half site]-[spacer sequence]-[right-half site]) is referred to herein as an LSR structure. In some embodiments, the left-half site and/or the right-half site correspond to an RNA-guided target site (*e.g.,* a Cas9 target site). In some embodiments, either or both half-sites are shorter or longer than, *e.g.,* a typical region targeted by Cas9, for example shorter or longer than 20 nucleotides. In some embodiments, the left and right half sites comprise different nucleic acid sequences. In some embodiments, the target site is a sequence comprising three (3) RNA-guided nuclease target site sequences, for example, three sequences corresponding to Cas9 target site sequences, in which the first and second, and second and third Cas9 target site sequences are separated by a spacer sequence. In some embodiments, the spacer sequence is at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, at least 50, at least 60, at least 70, at least 80, at least 90, at least 100, at least 125, at least 150, at least 175, at least 200, or at least 250 bp long.

The terms "transcriptional activator" and "transcriptional repressor" refer to an agent such as a protein (*e.g.,* a transcription factor or fragment thereof), that binds a target nucleic acid sequence and causes an increase or decrease of the level of expression of a gene product associated with the target nucleic acid sequence, respectively. For example, if the target nucleic acid sequence is located within a regulatory region of a gene, a transcriptional activator causes an increase of the level of expression of a gene product encoded by the gene (conversely, a transcriptional repressor causes a decrease of the level of expression of a gene product encoded by the gene). The gene product can be an RNA transcribed from the gene (*e.g.,* an mRNA) or a polypeptide translated from an mRNA transcribed from the gene. Typically an increase or decrease in the level of an mRNA results in an or decrease increase in the level of a polypeptide translated therefrom. The level of expression may be determined using standard techniques for measuring mRNA or protein.

The term "Transcriptional Activator-Like Effector," (TALE) as used herein, refers to effector proteins comprising a DNA binding domain, which contains a highly conserved 33-34 amino acid sequence comprising a highly variable two-amino acid motif (Repeat Variable Diresidue, RVD). The RVD motif determines binding specificity to a nucleic acid sequence, and can be engineered according to methods well known to those of skill in the art to specifically bind a desired DNA sequence (see, *e.g.,* Miller, Jeffrey; et.al. (February 2011). "A TALE nuclease architecture for efficient genome editing". Nature Biotechnology 29 (2): 143-8; Zhang, Feng; et.al. (February 2011). "Efficient construction of sequence-specific TAL effectors for modulating mammalian transcription". Nature Biotechnology 29 (2): 149-53; Geiβler, R.; Scholze, H.; Hahn, S.; Streubel, J.; Bonas, U.; Behrens, S. E.; Boch, J. (2011), Shiu, Shin-Han. ed. "Transcriptional Activators of Human Genes with Programmable DNA-Specificity". PLoS ONE 6 (5): e19509; Boch, Jens (February 2011). "TALEs of genome targeting". Nature Biotechnology 29 (2): 135-6; Boch, Jens; et.al. (December 2009). "Breaking the Code of DNA Binding Specificity of TAL-Type III Effectors". Science 326 (5959): 1509-12; and Moscou, Matthew J.; Adam J. Bogdanove (December 2009). "A Simple Cipher Governs DNA Recognition by TAL Effectors". Science 326 (5959): 1501; the entire contents of each of which are incorporated herein by reference). The simple relationship between amino acid sequence and DNA recognition has allowed for the engineering of specific DNA binding domains by selecting a combination of repeat segments containing the appropriate RVDs. TALE effector proteins include, without limitation, TALE nucleases (TALENs) and TALE transcriptional activators and repressors.

The term "Transcriptional Activator-Like Element Nuclease," (TALEN) as used herein, refers to an artificial nuclease comprising a transcriptional activator like effector DNA binding domain to a DNA cleavage domain, for example, a FokI domain. A number of modular assembly schemes for generating engineered TALE constructs have been reported (Zhang, Feng; et.al. (February 2011). "Efficient construction of sequence-specific TAL effectors for modulating mammalian transcription". Nature Biotechnology 29 (2): 149-53; Geiβler, R.; Scholze, H.; Hahn, S.; Streubel, J.; Bonas, U.; Behrens, S. E.; Boch, J. (2011), Shiu, Shin-Han. ed. "Transcriptional Activators of Human Genes with Programmable DNA-Specificity". PLoS ONE 6 (5): e19509; Cermak, T.; Doyle, E. L.; Christian, M.; Wang, L.; Zhang, Y.; Schmidt, C.; Baller, J. A.; Somia, N. V. et al. (2011). "Efficient design and assembly of custom TALEN and other TAL effector-based constructs for DNA targeting". Nucleic Acids Research*;* Morbitzer, R.; Elsaesser, J.; Hausner, J.; Lahaye, T. (2011). "Assembly of custom TALE-type DNA binding domains by modular cloning". Nucleic Acids Research*;* Li, T.; Huang, S.; Zhao, X.; Wright, D. A.; Carpenter, S.; Spalding, M. H.; Weeks, D. P.; Yang, B. (2011). "Modularly assembled designer TAL effector nucleases for targeted gene knockout and gene replacement in eukaryotes". Nucleic Acids Research*:,* Weber, E.; Gruetzner, R.; Werner, S.; Engler, C.; Marillonnet, S. (2011). Bendahmane, Mohammed. ed. "Assembly of Designer TAL Effectors by Golden Gate Cloning". PLoS ONE 6 (5): e19722; each of which is incorporated herein by reference).

The term "transcriptional repressor" refers to a transcription factor, *e.g.,* a protein, that binds a target nucleic acid sequence and causes a reduction of the level of expression of a gene product associated with the target nucleic acid sequence. For example, if the target nucleic acid sequence is located within a regulatory region of a gene, a transcriptional repressor causes a reduction of the level of expression of a gene product encoded by the gene. The gene product can be an RNA transcribed from the gene (*e.g.,* an mRNA) or a polypeptide translated from an mRNA transcribed from the gene. Typically a reduction in the level of an mRNA results in a reduction in the level of a polypeptide translated therefrom. The level of expression may be determined using standard techniques for measuring mRNA or protein.

The term "zinc finger nuclease," as used herein, refers to a nuclease comprising a nucleic acid cleavage domain conjugated to a binding domain that comprises a zinc finger array. In some embodiments, the cleavage domain is the cleavage domain of the type II restriction endonuclease FokI. Zinc finger nucleases can be designed to target virtually any desired sequence in a given nucleic acid molecule for cleavage, and the possibility to design zinc finger binding domains to bind unique sites in the context of complex genomes allows for targeted cleavage of a single genomic site in living cells, for example, to achieve a targeted genomic alteration of therapeutic value. Targeting a double-strand break to a desired genomic locus can be used to introduce frame-shift mutations into the coding sequence of a gene due to the error-prone nature of the non-homologous DNA repair pathway. Zinc finger nucleases can be generated to target a site of interest by methods well known to those of skill in the art. For example, zinc finger binding domains with a desired specificity can be designed by combining individual zinc finger motifs of known specificity. The structure of the zinc finger protein Zif268 bound to DNA has informed much of the work in this field and the concept of obtaining zinc fingers for each of the 64 possible base pair triplets and then mixing and matching these modular zinc fingers to design proteins with any desired sequence specificity has been described (Pavletich NP, Pabo CO (May 1991). "Zinc finger-DNA recognition: crystal structure of a Zif268-DNA complex at 2.1 A". Science 252 (5007): 809-17, the entire contents of which are incorporated herein). In some embodiments, separate zinc fingers that each recognizes a 3 base pair DNA sequence are combined to generate 3-, 4-, 5-, or 6-finger arrays that recognize target sites ranging from 9 base pairs to 18 base pairs in length. In some embodiments, longer arrays are contemplated. In other embodiments, 2-finger modules recognizing 6-8 nucleotides are combined to generate 4-, 6-, or 8- zinc finger arrays. In some embodiments, bacterial or phage display is employed to develop a zinc finger domain that recognizes a desired nucleic acid sequence, for example, a desired nuclease target site of 3-30 bp in length. Zinc finger nucleases, in some embodiments, comprise a zinc finger binding domain and a cleavage domain fused or otherwise conjugated to each other via a linker, for example, a polypeptide linker. The length of the linker determines the distance of the cut from the nucleic acid sequence bound by the zinc finger domain. If a shorter linker is used, the cleavage domain will cut the nucleic acid closer to the bound nucleic acid sequence, while a longer linker will result in a greater distance between the cut and the bound nucleic acid sequence. In some embodiments, the cleavage domain of a zinc finger nuclease has to dimerize in order to cut a bound nucleic acid. In some such embodiments, the dimer is a heterodimer of two monomers, each of which comprise a different zinc finger binding domain. For example, in some embodiments, the dimer may comprise one monomer comprising zinc finger domain A conjugated to a FokI cleavage domain, and one monomer comprising zinc finger domain B conjugated to a FokI cleavage domain. In this nonlimiting example, zinc finger domain A binds a nucleic acid sequence on one side of the target site, zinc finger domain B binds a nucleic acid sequence on the other side of the target site, and the dimerize FokI domain cuts the nucleic acid in between the zinc finger domain binding sites.

The term "zinc finger," as used herein, refers to a small nucleic acid-binding protein structural motif characterized by a fold and the coordination of one or more zinc ions that stabilize the fold. Zinc fingers encompass a wide variety of differing protein structures (see, *e.g.,* Klug A, Rhodes D (1987). "Zinc fingers: a novel protein fold for nucleic acid recognition". Cold Spring Harb. Symp. Quant. Biol. 52: 473-82, the entire contents of which are incorporated herein by reference). Zinc fingers can be designed to bind a specific sequence of nucleotides, and zinc finger arrays comprising fusions of a series of zinc fingers, can be designed to bind virtually any desired target sequence. Such zinc finger arrays can form a binding domain of a protein, for example, of a nuclease, *e.g.,* if conjugated to a nucleic acid cleavage domain. Different types of zinc finger motifs are known to those of skill in the art, including, but not limited to, Cys₂His₂, Gag knuckle, Treble clef, Zinc ribbon, Zn₂/Cys₆, and TAZ2 domain-like motifs (see, e.g., Krishna SS, Majumdar I, Grishin NV (January 2003). "Structural classification of zinc fingers: survey and summary". Nucleic Acids Res. 31 (2): 532-50). Typically, a single zinc finger motif binds 3 or 4 nucleotides of a nucleic acid molecule. Accordingly, a zinc finger domain comprising 2 zinc finger motifs may bind 6-8 nucleotides, a zinc finger domain comprising 3 zinc finger motifs may bind 9-12 nucleotides, a zinc finger domain comprising 4 zinc finger motifs may bind 12-16 nucleotides, and so forth. Any suitable protein engineering technique can be employed to alter the DNA-binding specificity of zinc fingers and/or design novel zinc finger fusions to bind virtually any desired target sequence from 3 - 30 nucleotides in length (see, *e.g.,* Pabo CO, Peisach E, Grant RA (2001). "Design and selection of novel cys2His2 Zinc finger proteins". Annual Review of Biochemistry 70: 313-340; Jamieson AC, Miller JC, Pabo CO (2003). "Drug discovery with engineered zinc-finger proteins". Nature Reviews Drug Discovery 2 (5): 361-368; and Liu Q, Segal DJ, Ghiara JB, Barbas CF (May 1997). "Design of polydactyl zinc-finger proteins for unique addressing within complex genomes". Proc. Natl. Acad. Sci. U.S.A. 94 (11); the entire contents of each of which are incorporated herein by reference). Fusions between engineered zinc finger arrays and protein domains that cleave a nucleic acid can be used to generate a "zinc finger nuclease." A zinc finger nuclease typically comprises a zinc finger domain that binds a specific target site within a nucleic acid molecule, and a nucleic acid cleavage domain that cuts the nucleic acid molecule within or in proximity to the target site bound by the binding domain. Typical engineered zinc finger nucleases comprise a binding domain having between 3 and 6 individual zinc finger motifs and binding target sites ranging from 9 base pairs to 18 base pairs in length. Longer target sites are particularly attractive in situations where it is desired to bind and cleave a target site that is unique in a given genome.

The terms "treatment," "treat," and "treating," refer to a clinical intervention aimed to reverse, alleviate, delay the onset of, or inhibit the progress of a disease or disorder, or one or more symptoms thereof, as described herein. As used herein, the terms "treatment," "treat," and "treating" refer to a clinical intervention aimed to reverse, alleviate, delay the onset of, or inhibit the progress of a disease or disorder, or one or more symptoms thereof, as described herein. In some embodiments, treatment may be administered after one or more symptoms have developed and/or after a disease has been diagnosed. In other embodiments, treatment may be administered in the absence of symptoms, *e.g*., to prevent or delay onset of a symptom or inhibit onset or progression of a disease. For example, treatment may be administered to a susceptible individual prior to the onset of symptoms (*e.g*., in light of a history of symptoms and/or in light of genetic or other susceptibility factors). Treatment may also be continued after symptoms have resolved, for example, to prevent or delay their recurrence.

### BRIEF DESCRIPTION OF THE DRAWINGS

*Figure 1**.* Schematic of macromolecular delivery into mammalian cells.
*Figure 2**.* Programming adipocyte cell fate: the switch from White Adipose Tissue (WAT) to Brown Adipose Tissue (BAT).
*Figure 3**.* Using supercharged delivery platforms to deliver TALE activators programmed to target PPARγ or PRDM16.
*Figure 4**.* Schematic of a fusion protein comprising a +36 GFP fusion, an 18.5 mer TALE domain, and a VP64 activation domain.
*Figure 5**.* Expression and purification of the +36 GFP-TALE activator-fusion protein.
*Figure* 6. Testing for activation of fat cell regulator genes upon delivery of +36 GFP PPARγ and PRDM16 TALE activator fusion proteins.
*Figure* 7. Delivery efficacy of +36 GFP TALE activator fusion proteins at different concentrations.
*Figure 8**.* Comparison of delivery efficacy of two different +36 GFP-PRDM16 TALE fusion proteins in NIH 3T3 cells.
*Figure 9**.* PPARγ gene expression after delivery of PPARγ-TALE activator fusion and comparison to various controls.
*Figure 10**.* PRDM16 gene expression after delivery of RDM16-TALE activator fusion and comparison to various controls.
*Figure 11**.* Moderate TALE activity is observed in the presence of serum.
*Figure 12**.* Validation of viral delivery of PPARγ followed by 7-day treatment with adipogenesis cocktail.
*Figure 13**.* Schematic of an assay for programming fibroblasts into WAT and BAT.
*Figure 14**.* Adipocyte formation observed upon treatment with +36 GFP TALE activator fusion protein.
*Figure 15**.* Staining of various treatments after 7 days with LipidTOX red shows formation of adipocytes after viral delivery as well as after delivery of supercharged PPARγ TALE activator fusion protein.
*Figure 16*. Staining of various treatments after 7 days with LipidTOX red shows formation of adipocytes after viral delivery as well as after delivery of supercharged PPARγ TALE activator fusion protein.
*Figure 17*. Expression of WAT biomarker genes after viral delivery as well as after delivery of supercharged PPARγ TALE activator fusion protein.
*Figure 18**.* Delivery of supercharged PRDM16 TALE activator fusion proteins to induce brown-fat adipocytes *in vivo.* Robust adipocyte formation was observed after viral delivery of PPARγ and PRDM16 and also after delivery of supercharged TALE activator protein fusions.
*Figure 19**.* Comparison of TALE/TALE, viral/TALE, and viral/viral-induced expression of brown fat markers by expression of PPARγ and PRDM16.
*Figure 20**.* RT-qPCR assessments are consistent with fat cell differentiation observed by LipidTOX staining.
*Figure 21**.* Delivery of functional TALE activator fusion proteins as complexes with +36 GFP improves TALE activator activity after delivery.
*Figure 22**.* PRDM16 gene expression after TALE activator fusion delivery either as a fusion (+36GFP PRDM16 TALE-3) or a complex (+36GFP + PRDM16 TALE-3) with +36GFP. Delivery of complexes tends to increase TALE activator activity.
*Figure 23**.* Effect of Aurein peptide fusion to +36GFP on PRDM16 gene expression after TALE activator fusion delivery (either as a fusion or a complex with +36GFP).
*Figure 24**.* PRDM16 gene expression after TALE activator fusion delivery either as a fusion (+36GFP PRDM16 TALE-3) or a complex (+36GFP + PRDM16 TALE-3) with Lipofectamine LTX.
*Figure 25**.* Delivery of supercharged fusion proteins or complexes with Cas9 into mammalian cells. (GGS)9-T-ALAL-PKKKRKV corresponds to SEQ ID NO: 251.
*Figure 26**.* Purification of wild-type Cas9 protein and Cas9 fusion proteins with +36GFP and Aurein-GGS9.

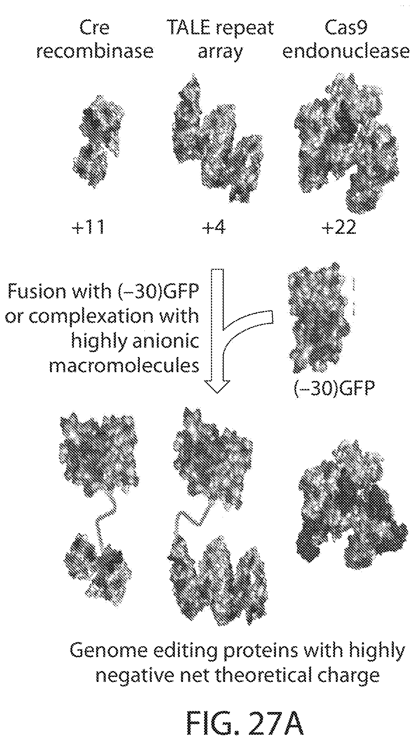

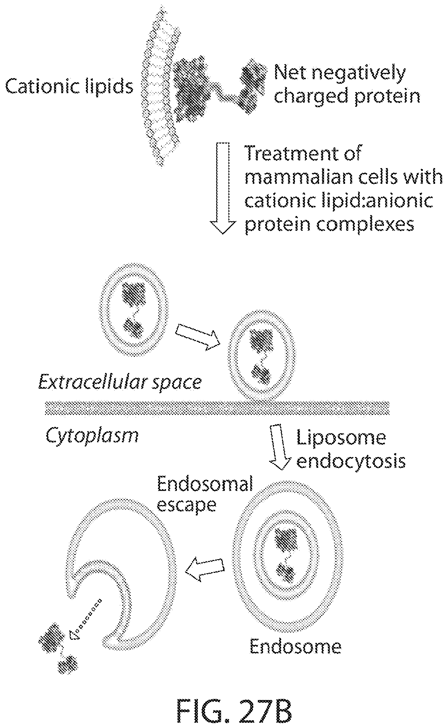

*Figure 27A-B.* A strategy for delivering proteins into mammalian cells by fusion or non-covalent association with polyanionic macromolecules and encapsulation with cationic lipids is shown. Figure 27(A) shows that recombinases, transcriptional-activator-like effector (TALE) proteins, and Cas9 endonucleases bind nucleic acids and are natively cationic (net theoretical charges are shown in black) and are not efficiently encapsulated by cationic lipids. These proteins can be rendered highly anionic, however, by fusion to either a supernegatively charged protein such as (-30)GFP, or by complexation with polyanionic nucleic acids. Figure 27(B) shows a schematic representing that cationic lipids commonly used to transfect DNA and RNA encapsulate the resulting highly anionic proteins or protein:nucleic acid complexes, mediating their delivery into mammalian cells.

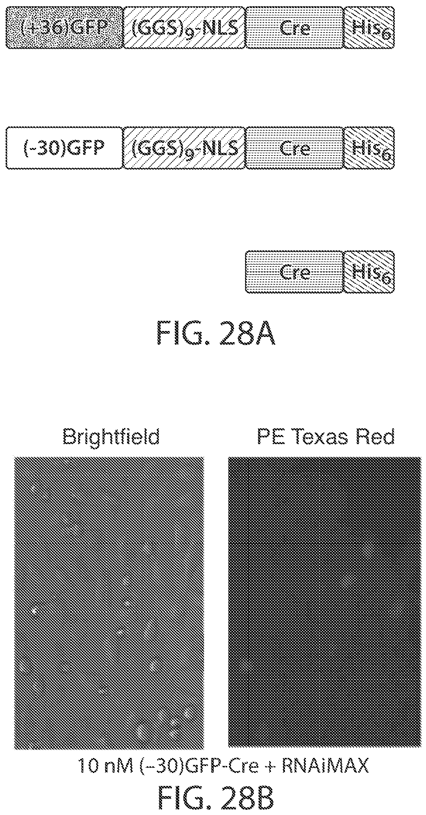

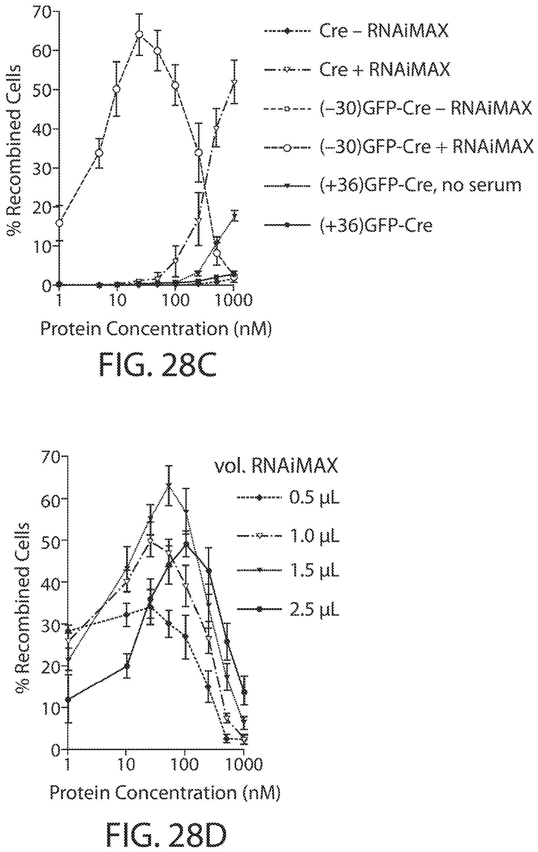

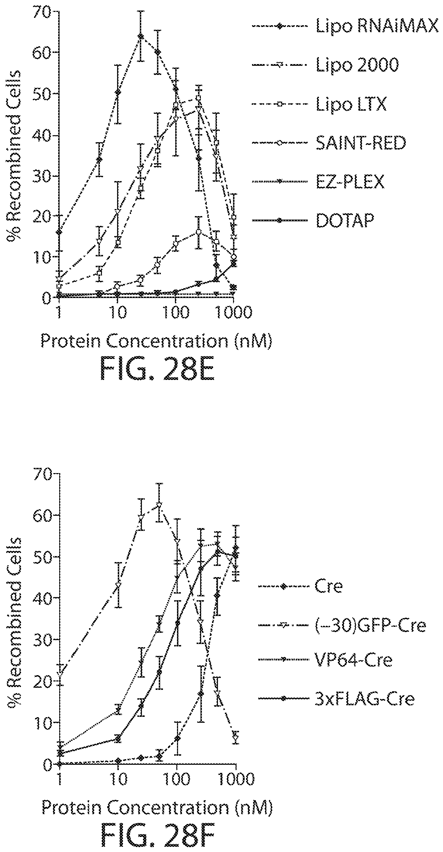

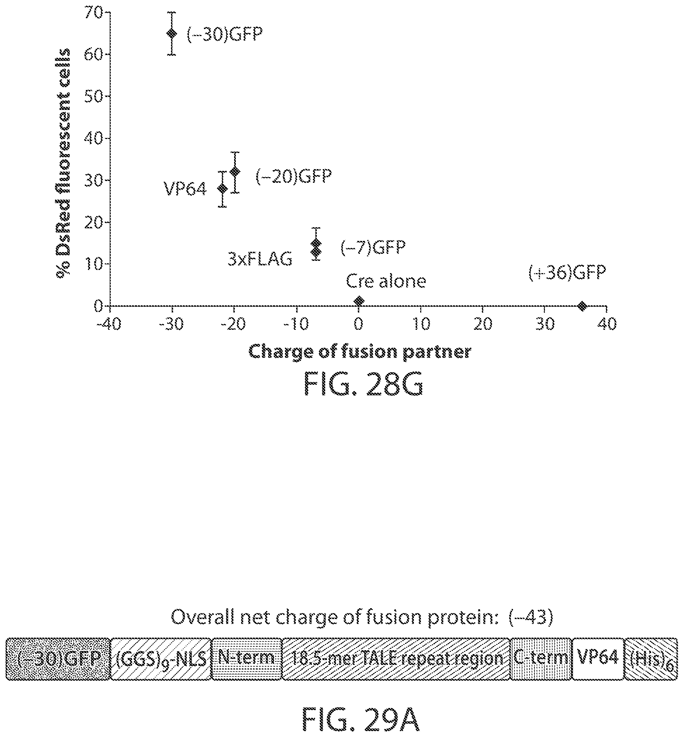

*Figure 28A-G.* Delivery of Cre recombinase to cultured human cells. In Figure 28(A), the fusion of either highly cationic (+36)GFP or highly anionic (-30)GFP to Cre recombinase is shown. A HeLa reporter cell line that expresses DsRed upon Cre-mediated recombination was used to evaluate Cre delivery efficiency; (GGS)9 corresponds to SEQ ID NO : 252 and His6 corresponds to SEQ ID NO: 253. In Figure 28(B) HeLa dsRed cells treated with 10 nM (-30)GFP-Cre and the cationic lipid RNAiMAX. Cells were visualized after incubation for 48 hours in media containing 10% fetal bovine serum (FBS). Figure 28(C) shows the delivery of (+36)GFP-Cre in 10% FBS media or in serum-free media, and (-30)GFP-Cre with or without the cationic lipid RNAiMAX (0.8 µL) in full-serum media. Figure 28(D) presents the effect of cationic lipid dose on functional (-30)GFP-Cre delivery efficacy after 48 hours in 275 µL media containing 10% FBS. Figure 28(E) is a comparison of several commercially available cationic lipids and polymers for functional delivery efficacy of (-30)dGFP-Cre. Figure 28(F) shows the RNAiMAX-mediated delivery of multiple anionic peptide or protein sequences fused to Cre. Figure 28(G) shows RNAiMAX-mediated delivery of multiple anionic peptide or protein sequences fused to Cre. The net theoretical charge of the VP64 activation domain and the 3xFLAG tag is -22 and -7, respectively. All experiments were performed with 25 nM protein in 48-well plate format using 275 µL DMEM with 10% FBS and no antibiotics. Error bars reflect the standard deviation from three biological replicates performed on different days.

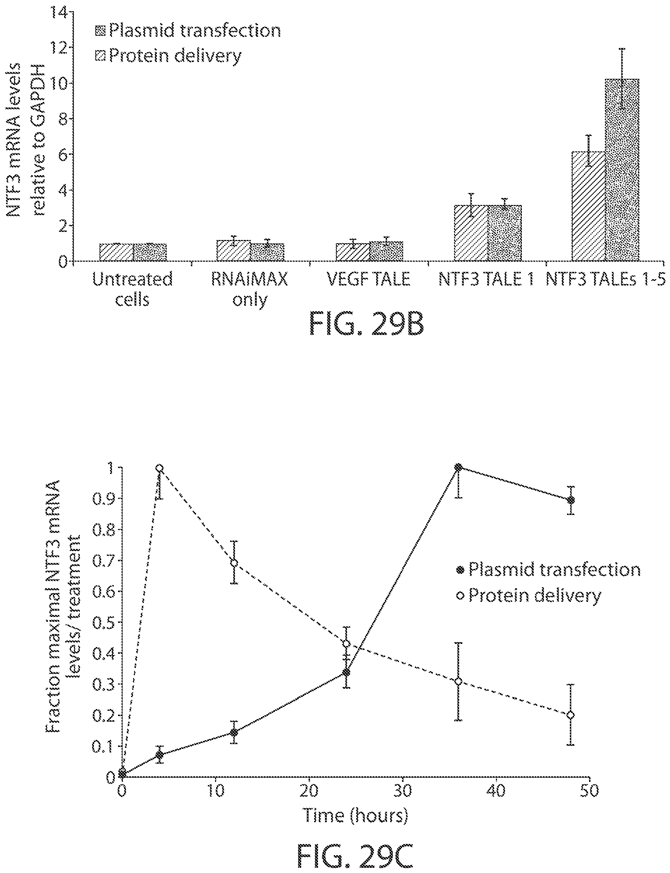

*Figure 29A-C.* Delivery of TALE transcriptional activators into cultured human cells. Figure 29(A) shows the design of an 18.5-repeat TALE activator fused C-terminally to a VP64 activation domain and N-terminally to (-30)GFP and an NLS; (GGS)9 corresponds to SEQ ID NO: 252 and His6 corresponds to SEQ ID NO: 253. The overall net theoretical charge of the fusion is -43. Figure 29(B) demonstrates the activation of NTF3 transcription by traditional transfection of plasmids encoding TALE-VP64 activators that target sites in the NTF3 gene, or by RNAiMAX cationic lipid-mediated delivery of the corresponding NTF3-targeting (-30)GFP-TALE-VP64 proteins. Gene expression levels were measured by qRT-PCR and are normalized to GAPDH expression levels. Incubation times for TALE activators by plasmid transfection and protein delivery were those found to give maximal increases in NTF3 mRNA levels. Figure 29(C) shows the time course of TALE activation for protein delivery and plasmid transfection by measuring NTF3 mRNA levels and then normalizing each method to the highest activation value achieved over any time point for that method. Optimal protein (25-50 nM) and lipid dosage (1.5 µL RNAiMAX) was used for each delivery technique. Error bars reflect the standard deviation from three biological replicates performed on different days.

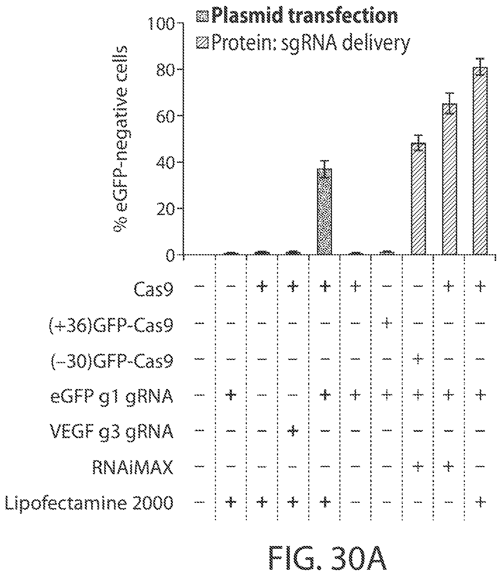

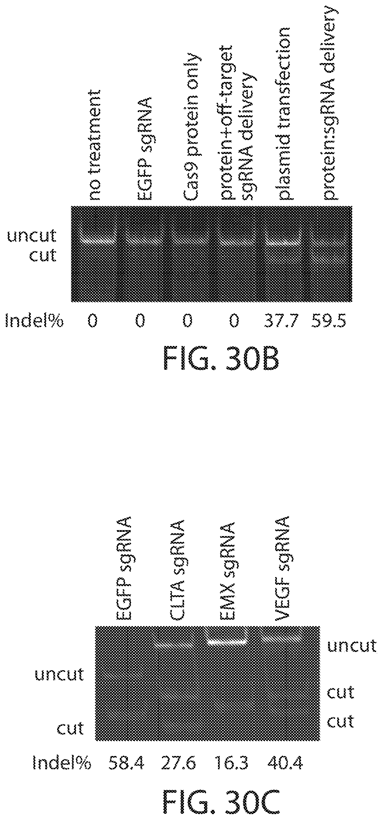

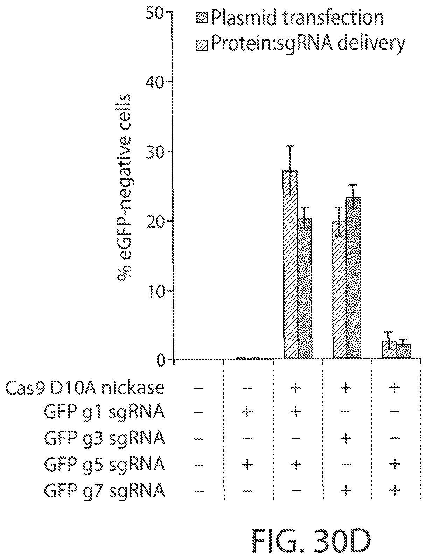

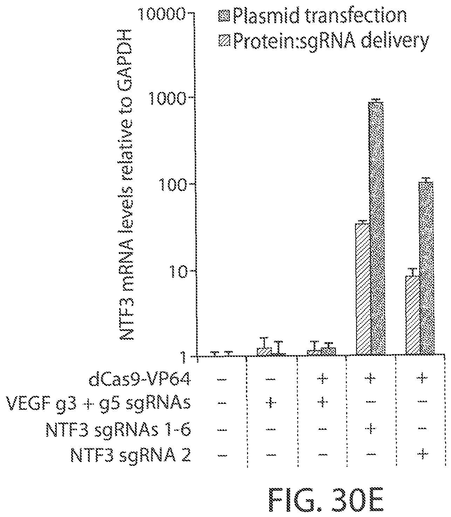

*Figure 30A-E.* Delivery of Cas9:sgRNA, Cas9 D10A nickase, and dCas9-VP64 transcriptional activators to cultured human cells. Figure 30(A) demonstrates the cationic lipid-mediated delivery of Cas9 protein variants complexed with an EGFP-targeting sgRNA or a VEGF-targeting sgRNA to U2OS EGFP reporter cells. U2OS EGFP reporter cells were treated with 100 nM of the Cas9 protein variant shown, 0.8 µL of the cationic lipid shown, and either 50 nM of the sgRNA shown for Cas9 protein treatment, or 125 nM of the sgRNA shown for (+36)dGFP-NLS-Cas9 and (-30)dGFP-NLS-Cas9 treatment. The fraction of cells lacking EGFP expression was measured by flow cytometry. Plasmid DNA transfection of 750 ng Cas9 and 250 ng sgRNA expression plasmids using 0.8 µL Lipofectamine 2000 is shown as well. Results are compared to that of standard transfection of Cas9 and sgRNA expression plasmids. Figure 30(B) shows the results of a T7 endonuclease I (T7EI) assay to measure the modification of EGFP from no treatment (lane 1), treatment with EGFP-targeting sgRNA alone (lane 2), Cas9 protein alone (lane 3), Cas9 protein + VEGF-targeting sgRNA + RNAiMAX (lane 4), DNA transfection of plasmids expressing Cas9 and EGFP-targeting sgRNA (lane 5), or Cas9 protein + EGFP-targeting sgRNA + RNAiMAX (lane 6). Indel efficiencies calculated by densitometry are shown below the gel image. Figure 30(C) presents the results of a T7EI assay of genome modification at EGFP and three endogenous genes with a single delivery of Cas9 complexed with four sgRNAs and RNAiMAX. T7EI assay of simultaneous genome modification at EGFP and three endogenous genes in U2OS cells 48 hours after a single treatment of 100 nM Cas9 protein, 25 nM of each of the four sgRNAs shown (100 nM total sgRNA), and 0.8 µL RNAiMAX. Indel efficiencies calculated by densitometry are shown below the gel image. Figure 30(D) shows the delivery of Cas9 D10A nickase and pairs of sgRNAs either by plasmid transfection or by RNAiMAX-mediated protein:RNA complex delivery. Delivery of Cas9 D10A nickase and pairs of sgRNAs either by plasmid transfection or by RNAiMAX-mediated protein: sgRNA complex delivery under conditions described in Figure 30(A) with 50 nM *EGFP*-disrupting sgRNAs (25 nM each) for protein delivery, and 250 ng sgRNA-expressing plasmids (125 ng each) for DNA delivery. EGFP-disrupting sgRNAs GFP g1 + GFP g5, or GFP g3 + GFP g7, are expected to result in gene disruption, while GFP g5 + GFP g7 target the same strand and are therefore expected to be non-functional. Figure 30(E) shows the delivery of catalytically dead (dCas9)-VP64 transcriptional activators that target NTF3 either by plasmid transfection or RNAiMAX-mediated protein delivery. Delivery of both VEGF g3 and VEGF g5 sgRNAs served as a negative control for NTF3 gene activation. Error bars reflect the standard deviation from six biological replicates performed on different days.

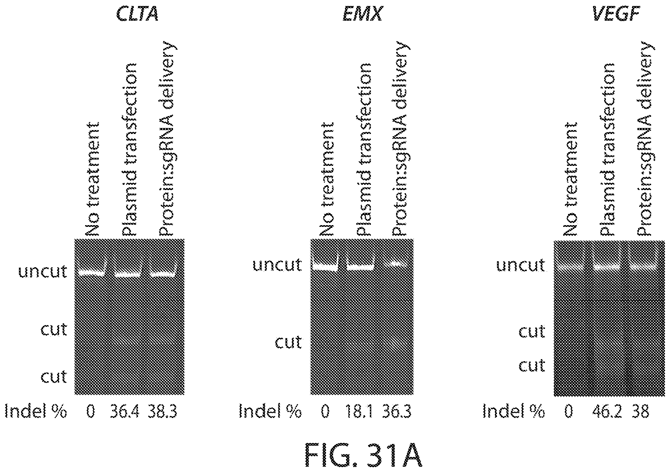

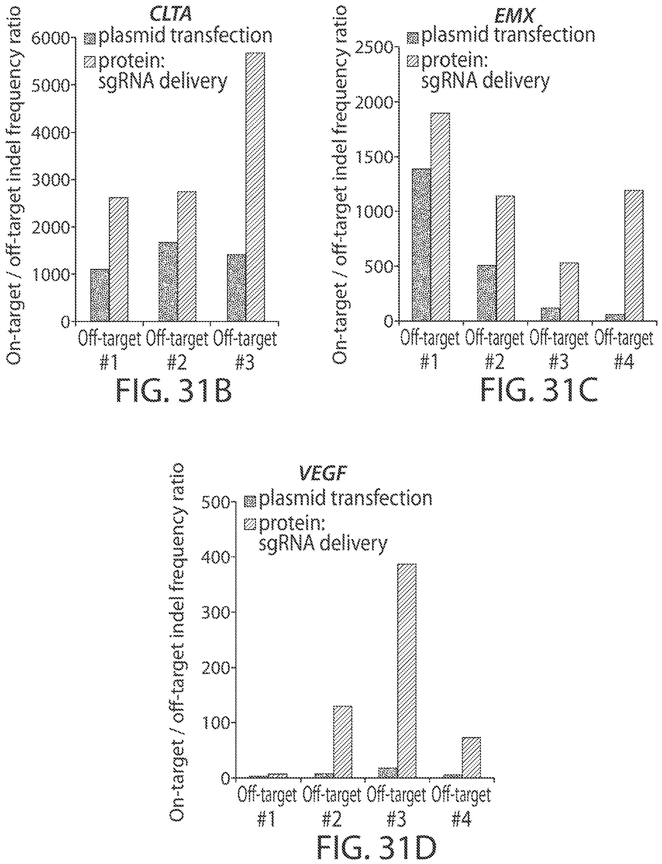

*Figure 31A-D.* The DNA sequence specificity of Cas9-mediated endogenous gene cleavage in cultured human cells by plasmid transfection or by cationic lipid-mediated protein: sgRNA delivery is shown. In Figure 31(A), a T7EI assay was performed for on-target modification of endogenous CLTA, EMX, and VEGF genes. In Figure 31(B-D) the on-target: off-target DNA modification ratio resulting from Cas9:sgRNA for plasmid transfection or cationic lipid-mediated protein:sgRNA delivery is shown. The conditions for each treatment were adjusted to result in -10% on-target cleavage, enabling a comparison of DNA cleavage specificity between the two delivery methods under conditions in which on-target gene modification efficiencies are comparable. P values are listed in Table 2. Each on- and off-target sample was sequenced once with > 10,000 sequences analyzed per on-target sample and an average of > 111,000 sequences analyzed per off-target sample (Table 2). All protein:sgRNA deliveries and plasmid transfections were performed in 24-well format using 1.6 µL RNAiMAX in 550 µL DMEM-FBS without antibiotics.

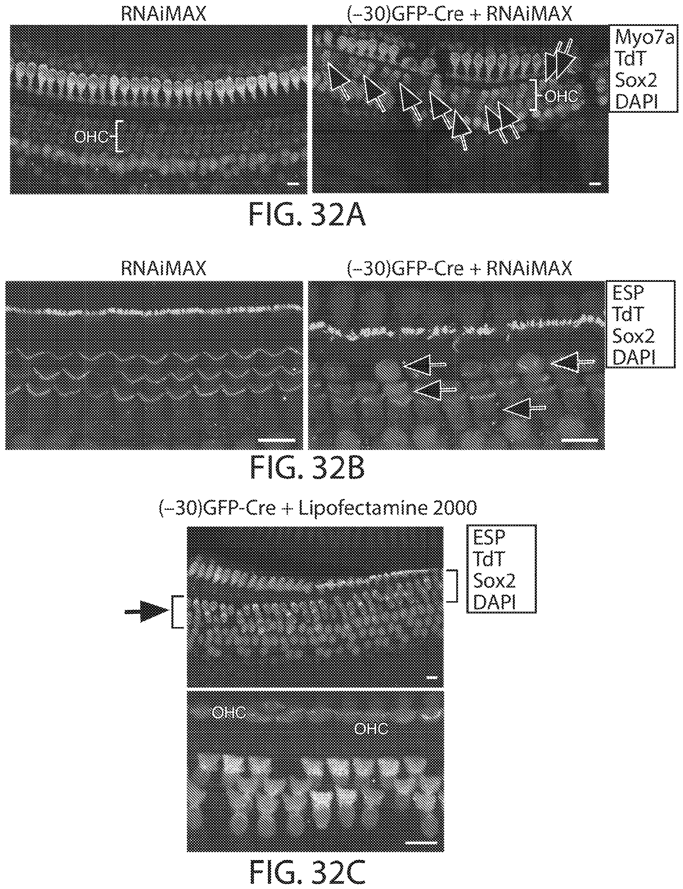

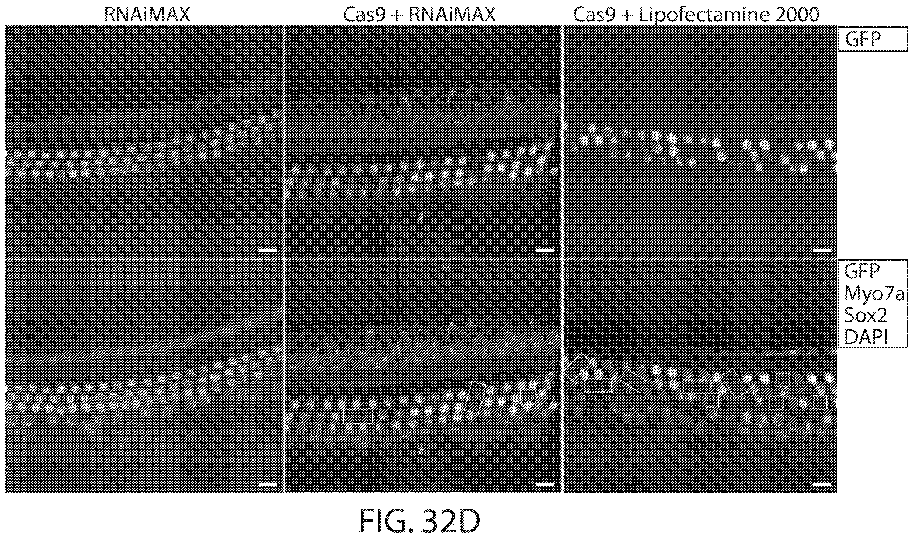

*Figures 32A-D.* The *in vivo* delivery of Cre recombinase and Cas9:sgRNA complexes to hair cells in the mouse inner ear is shown. In Figure 32(A), the scala media (cochlear duct) of P0 floxP-tdTomato mice (n = 4) were injected with 0.3 µL of 23 µM (-30)GFP-Cre in 50% RNAiMAX or with RNAiMAX alone (control). After 5 days, tdTomato expression indicative of Cre-mediated recombination was visualized using immunohistology. Red = tdTomato; green = Myo7a; white = Sox2; blue = DAPI. Yellow brackets indicate the outer hair cell (OHC) layer. Figure 32(B) shows that, ten days after (-30)GFP-Cre delivery, intact espin (Esp)-expressing stereocilia of tdTomato-positive outer hair cells were present (arrow), similar to stereocilia in control cochlea. Red = tdTomato; green = Esp; white = Sox2; blue = DAPI. Figure 32(C) is identical to Figure 32(A) except using Lipofectamine 2000 instead of RNAiMAX. (n = 4). The upper and lower panels are images of mice cochlea at low and high magnification, respectively, detailing the efficiency of delivery as well as the effect on cochlear architecture and hair cell loss. Figure 32(D) shows the results when the scala media (cochlear duct) of P2 Atoh1-GFP mice (n = 3) were injected with 0.3 µL of 33 µM Cas9, 33 µM sgRNA in 50% RNAiMAX, 16.5 µM EGFP sgRNA in 50% RNAiMAX or Lipofectamine 2000. Cas9-mediated gene disruption results in the loss of GFP expression when visualized 10 days later. The upper panels show GFP signal only, while lower panels include additional immunohistological markers. Yellow boxes in the lower panels highlight hair cells that have lost GFP expression. Red = tdTomato; green = Myo7a; white/light blue = Sox2; blue = DAPI. All scale bars, shown in white, are 10 µm.

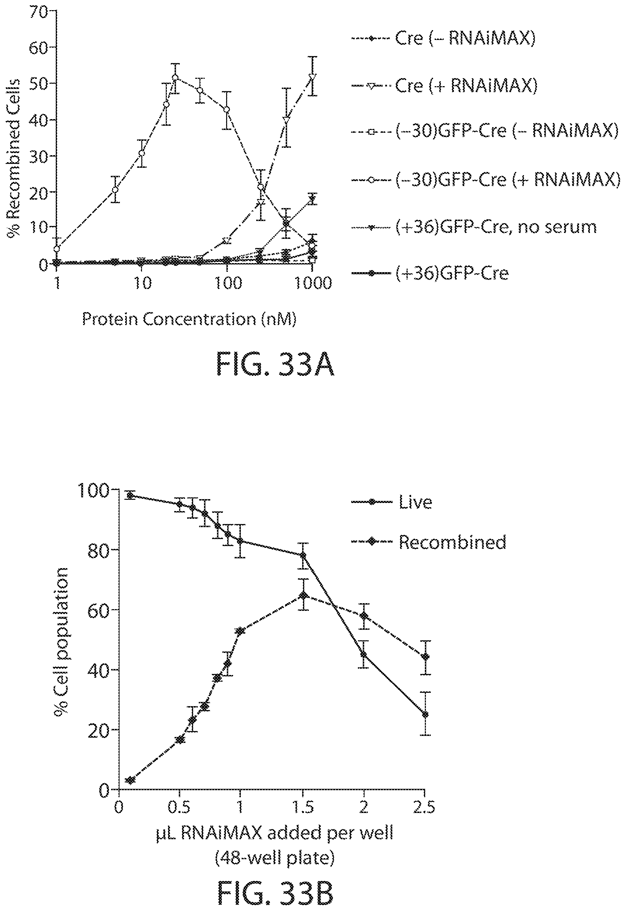

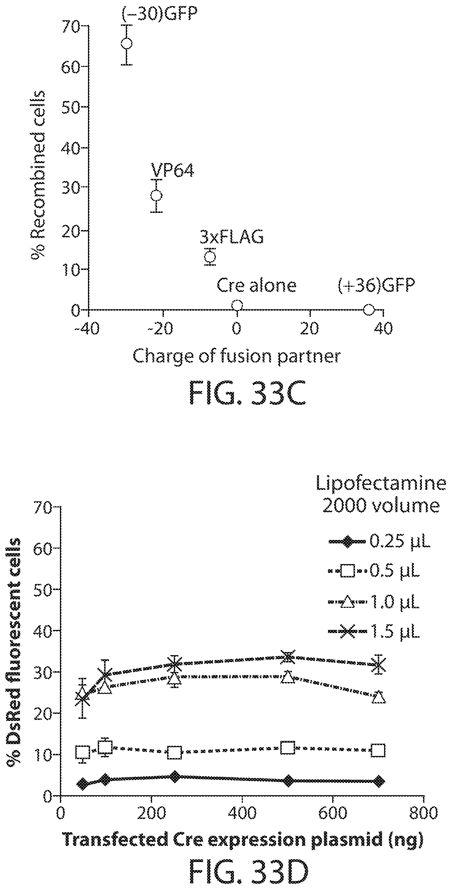

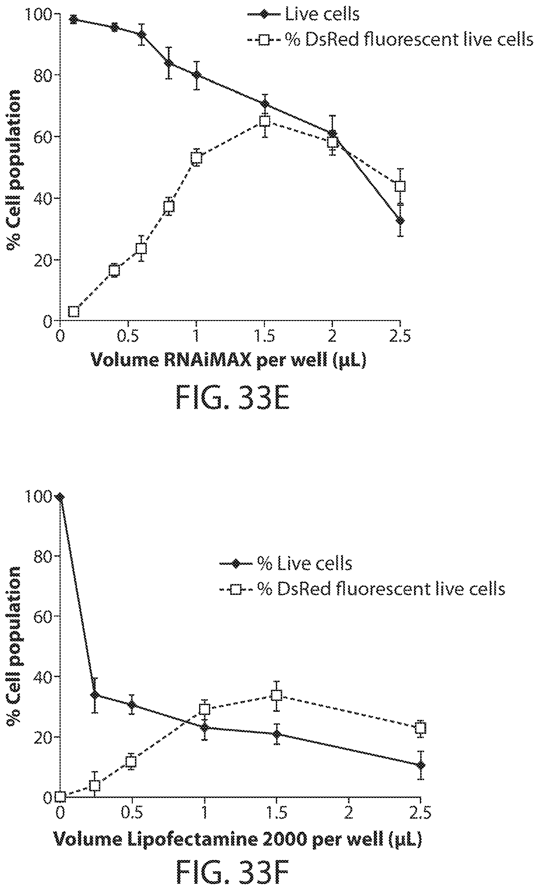

*Figure 33A-F.* Optimization of cationic lipid-mediated delivery of Cre recombinase and comparison to delivery using (+36)GFP-Cre and plasmid transfection. Figure 33(A) shows the optimization of (-30)GFP-Cre delivery in BSR-TdTomato cells, a second reporter cell line used for measuring Cre recombination efficiency. Figure 33(B) demonstrates the effect of RNAiMAX dosage on (-30)GFP-Cre recombination efficiency in HeLa dsRed reporter cells and toxicity as measured by FACS. HeLa cells were sorted by forward-scatter and side-scatter gating to identify live cells that retained normal morphology. Figure 33(C) illustrates the relationship between net charge of the protein fused to Cre recombinase and cationic lipid-mediated functional Cre delivery efficiency. Cre recombinase fused to the domains listed at 25 nM were combined with 1.5 µL RNAiMAX and incubated with HeLa dsRed reporter cells. After 2 days, recombination efficiency was measured by FACS. Figure 33(D) shows the optimization of Cre expression plasmid transfection in HeLa DsRed reporter cells by varying both plasmid dose and Lipofectamine 2000 dose and measuring the presence of DsRed fluorescent cells 48 hours after transfection by flow cytometry. Based on these results, 500 ng of Cre expression plasmid was chosen for 48-well format experiments using 275 µL of DMEM-FBS without antibiotics. Figure 33(E) demonstrates the effect of RNAiMAX dosage on (-30)GFP-Cre recombination efficiency in HeLa dsRed reporter cells and corresponding toxicity as measured by flow cytometry using the TO-PRO-3 live/dead stain (Life Technologies). Figure 33(F) shows the effect of Lipofectamine 2000 dosage on transfected Cre plasmid DsRed recombination efficiency and corresponding toxicity as measured by flow cytometry using the TO-PRO-3 live/dead stain. Error bars reflect the standard deviation from three biological replicates performed on different days.

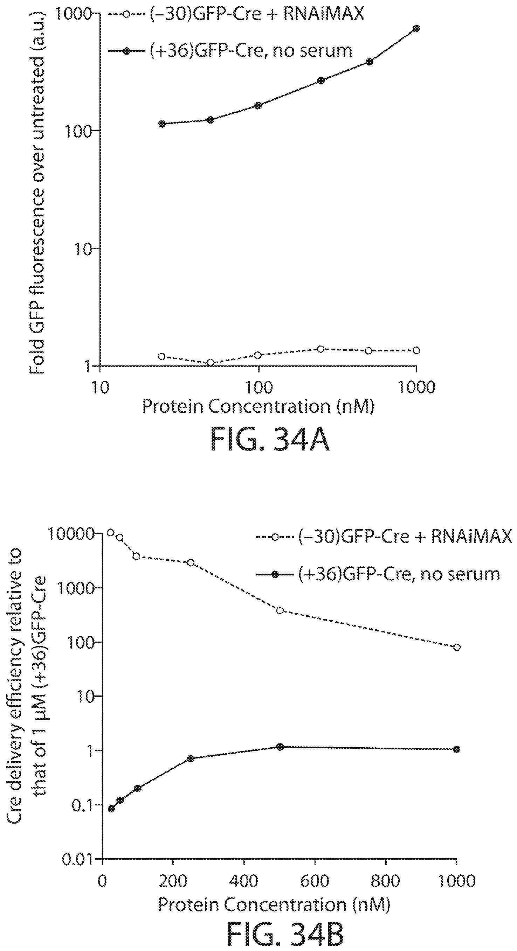

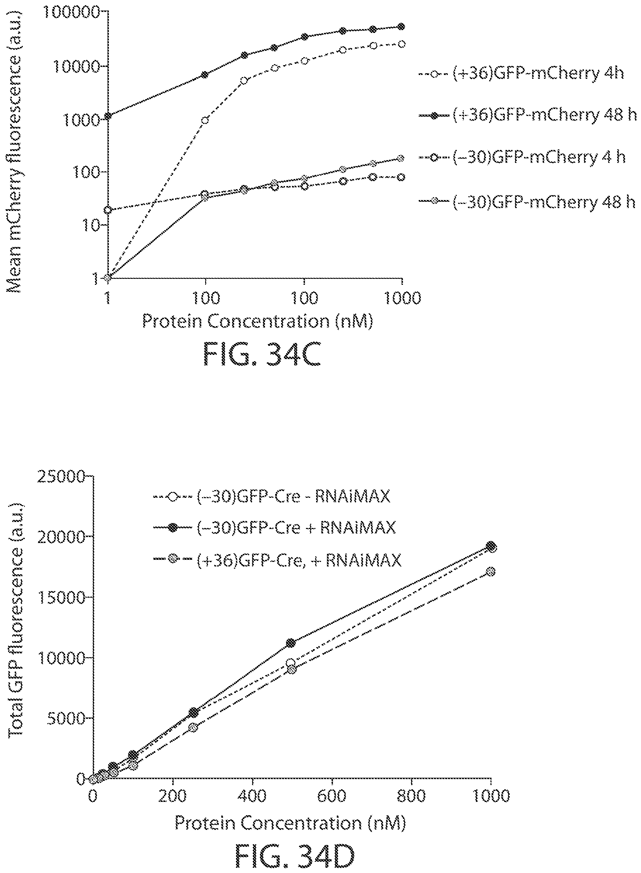

*Figures 34A-D.* Protein uptake by cationic lipid-mediated delivery versus superpositively charged cationic protein delivery. Figure 34(A) quantifies GFP fluorescence from cells treated with either (-30)GFP-Cre and RNAiMAX or (+36)GFP-Cre after washing cells with PBS + heparin (20 U/mL) to remove unbound protein. Figure 34(B) shows the functional Cre recombinase delivery efficiency of (-30)GFP-Cre + 1.5 µL RNAiMAX relative to Cre recombinase delivery efficiency arising from fusion with (+36)GFP. Figure 34(C) provides a comparison of mCherry uptake by (-30)GFP-fusion + 1.5 µM RNAiMAX treatment versus (+36)GFP fusion by measuring mean mCherry fluorescence of total cell population 48 hours after treatment and washing cells with PBS + heparin. Figure 34(D) shows the total cellular GFP fluorescence of (-30)GFP-Cre or (+36)GFP-Cre in the presence or absence of RNAiMAX. Data shown reflect a single biological replicate.

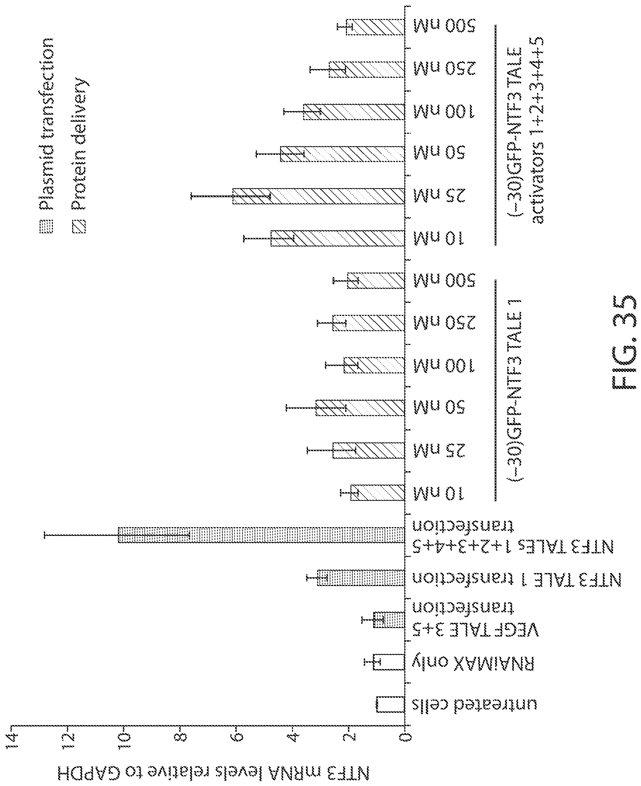

*Figure 35**.* Delivery optimization of TALE activators designed to target the *NTF3* gene. HEK293T cells were treated with either NTF3 TALE plasmid by transfection of by liposomal delivery of NTF3 TALE proteins. Cells were harvested after empirically determined optimal incubation time for both treatments and analyzed by qRT-PCR for mRNA levels of NTF3. Optimal protein (25-50 nM) and lipid dosage (1.5 µL RNAiMAX) was chosen for comparison of two delivery techniques in Figure 29B. All protein-delivery and transfection experiments were performed in a 48-well plate with 275 µL DMEM-FBS without antibiotics. Error bars reflect the standard deviation from six biological replicates performed on different days.

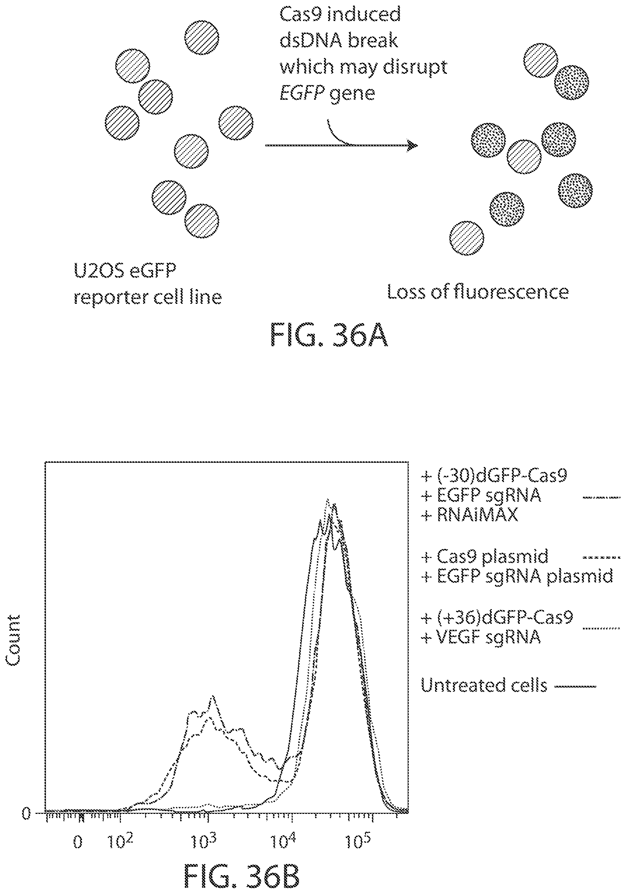

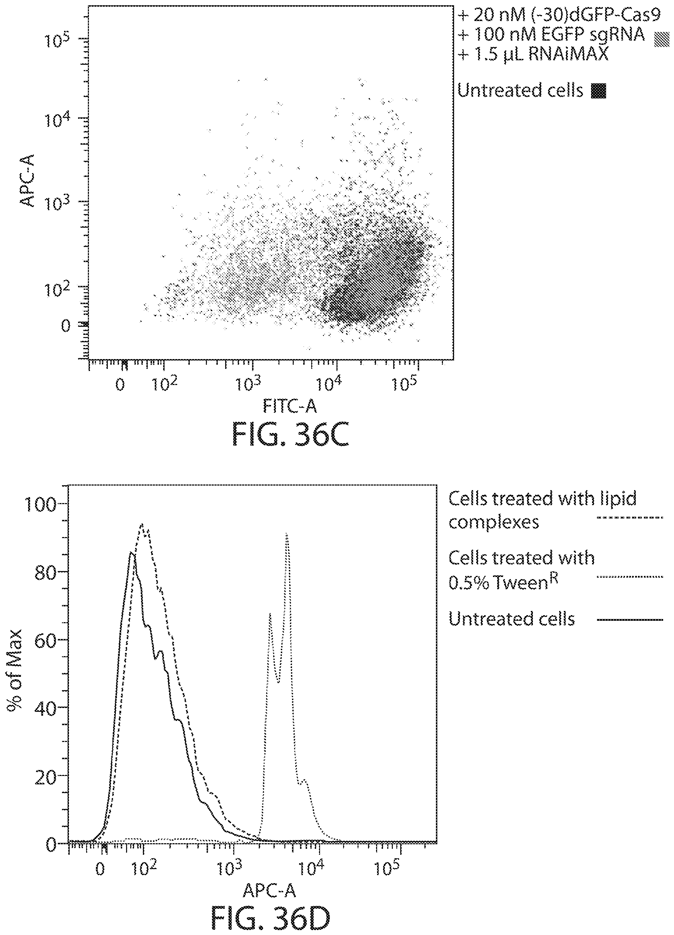

*Figures 36A-D.* Determination of gene disruption frequency of an *EGFP* reporter gene by delivery of Cas9:sgRNA and analyzing by flow cytometry. Figure 36(A) provides a schematic of EGFP disruption in U2OS cells by NHEJ induced by Cas9 double-stranded breaks. Figure 36(B) shows the delivery of *EGFP*-targeting sgRNA or an off-target sgRNA complexed with (-30)dGFP-Cas9 using RNAiMAX along with a plasmid transfection positive control (orange). Figure 36(C) provides confirmation that disruption of EGFP fluorescence is not a result of cellular toxicity by treating samples with the TO-PRO-3 live/dead stain (Life Technologies, Carlsbad CA) and analyzing the resulting cells by flow cytometry. Figure 36(D) shows testing of the TO-PRO-3 stain by addition of a cell permeabilizing, but not completely membrane lysing, detergent (0.5% Tween).

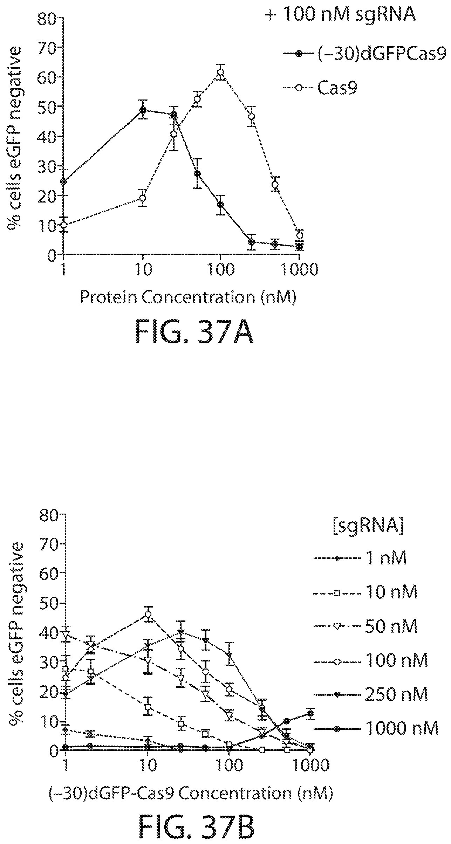

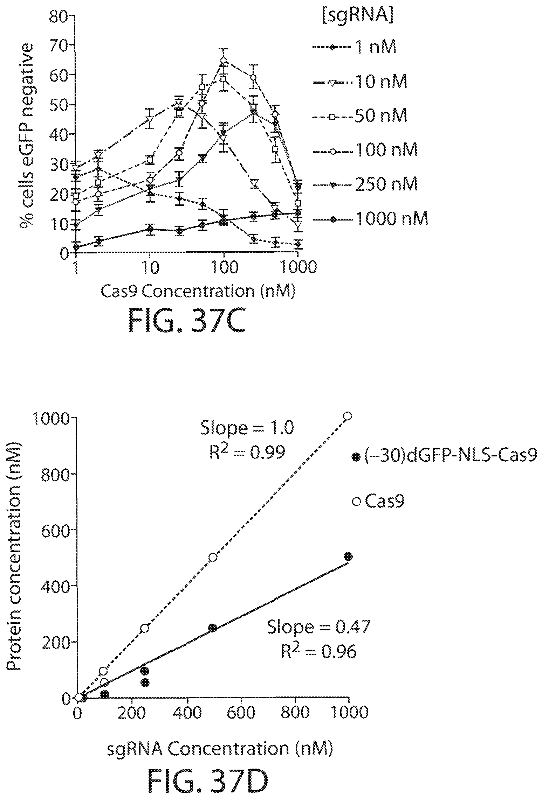

*Figures 37A-D.* Optimization of Cas9:sgRNA functional delivery. In Figure 37(A), cationic lipid-mediated delivery efficiency of two tested constructs shows that the more anionic (-30)dGFP-NLS-Cas9 facilitates more efficient delivery at low protein and sgRNA concentrations compared with native Cas9. Figure 37(B) shows the delivery optimization of (-30)dGFP-NLS-Cas9 as a function of protein and sgRNA concentration. Figure 37(C) shows the delivery of Cas9 protein without any fusions or tags as a function of protein and sgRNA concentration. Figure 37(D) provides the optimal sgRNA to protein ratio for RNAiMAX-mediated delivery of (-30)dGFP-NLS-Cas9 and native Cas9. Error bars reflect standard deviation from three biological replicates performed on different days. All experiments were performed in a 48-well plate using a volume of 275 µL DMEM-FBS without antibiotics and EGFP gene disruption was measured by flow cytometry.

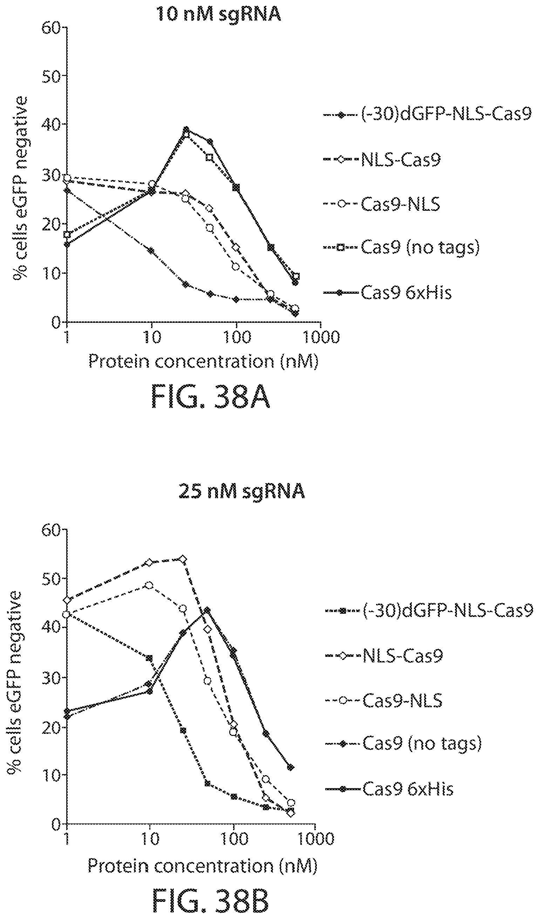

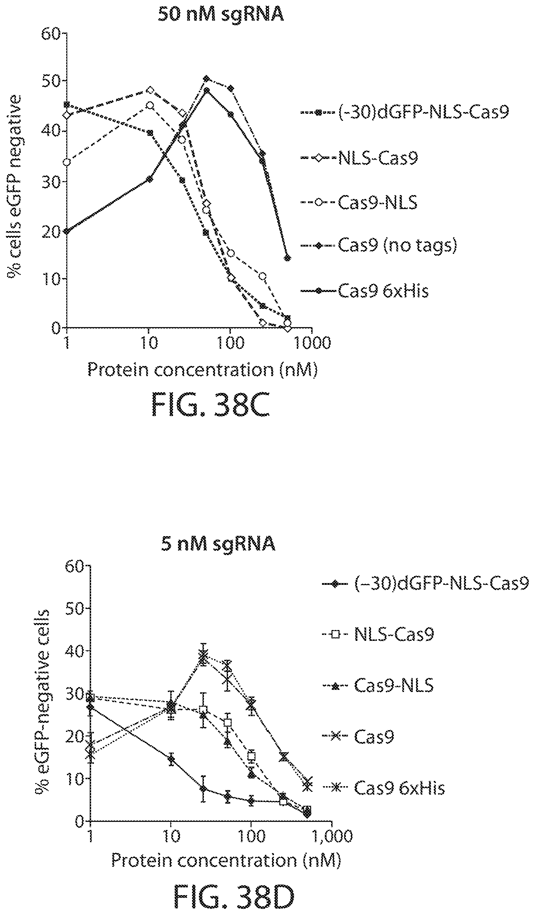

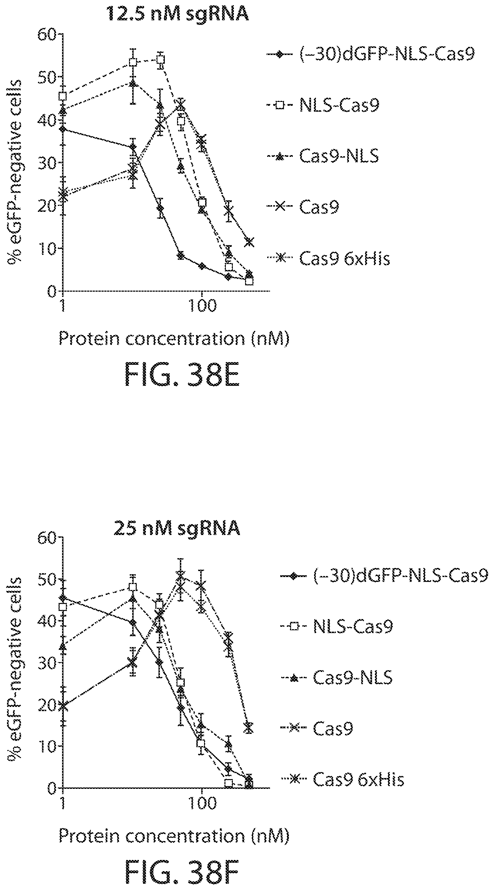

*Figures 38A-F.* Effect of an *N*- or C-terminal NLS, an N-terminal (-30)dGFP fusion, or a C-terminal His-tag on functional Cas9 delivery as a function of both sgRNA and Cas9 concentration. *EGFP* gene disruption was measured at three fixed sgRNA concentrations: 10 nM (Figure 38(A)), 25 nM (Figure 38(B)), and 50 nM (Figure 38(C)), along with varying protein concentrations show in the graphs. *EGFP* gene disruption in U2OS EGFP reporter cell line was measured at three additional fixed sgRNA concentrations: (Figure 38(D)) 5 nM, (Figure 38(E)) 12.5 nM, and (Figure 38(F)) 25 nM, along with varying protein concentrations show in the graphs. Delivery was performed using 0.8 µL RNAiMAX in 48-well format using 275 µL DMEM-FBS without antibiotics and assayed by FACS 48 hours later for loss of EGFP fluorescence signal. Error bars (Figures 38(D-F)) reflect standard deviation from three biological replicates performed on different days.

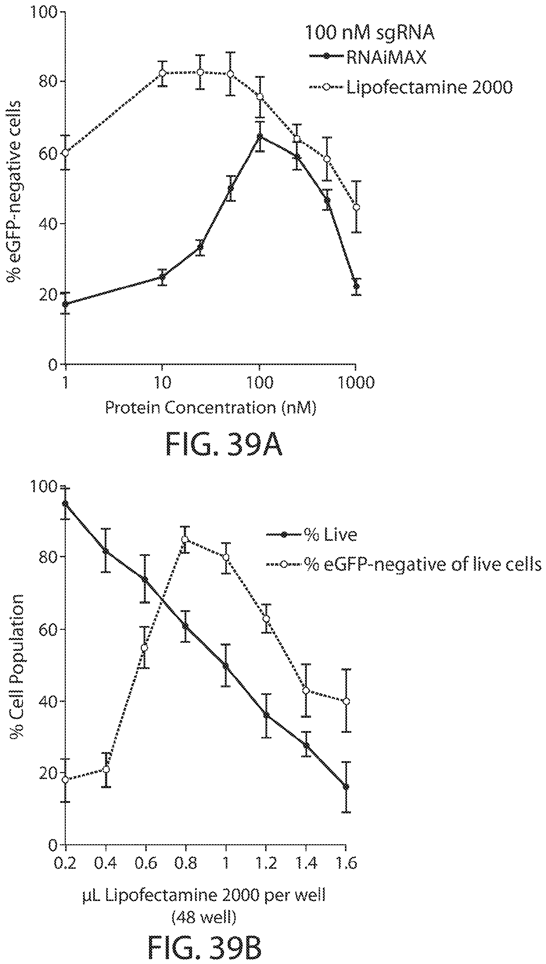

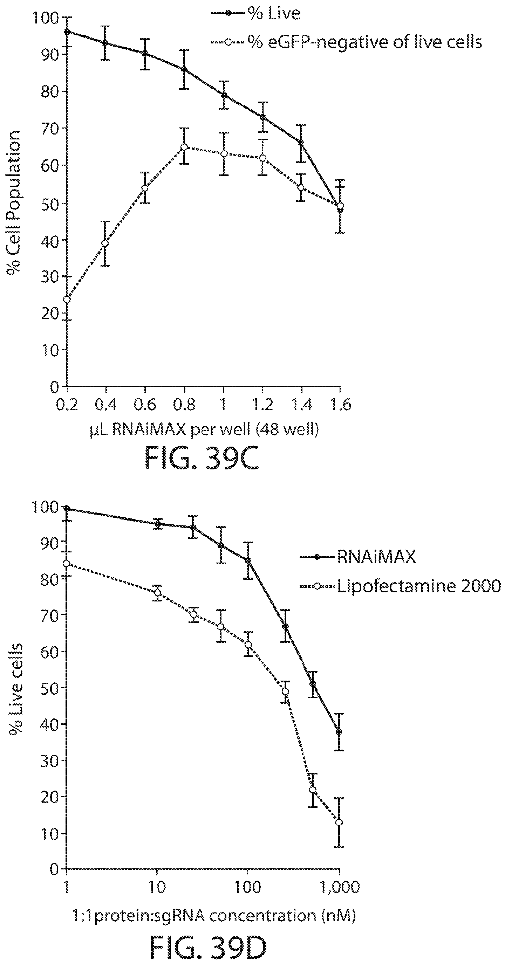

*Figures 39A-D.* Effects of RNAiMAX and Lipofectamine 2000 on Cas9: sgRNA delivery efficiency and cellular toxicity. In Figure 39(A), *EGFP* gene disruption at different Cas9 protein concentrations and a constant dose of 100 nM EGFP sgRNA in U2OS EGFP reporter cells treated for 16 hours with either 0.8 µL of RNAiMAX or 0.8 µL Lipofectamine 2000. After 16 hours, media was removed and fresh media was added to cells until end point of assay 48-72 hours post protein delivery treatment. The live cell population was determined by FACS using TO-PRO-3 Live/Dead stain. Figure 39(B) shows the toxicity profile for Cas9:sgRNA delivery to U2OS cells as a function of Lipofectamine 2000 dose. Figure 39(C) provides the toxicity profile for cells as a function of RNAiMAX dose. Figure 39(D) shows the cellular toxicity for a broad range of Cas9: sgRNA treatments using 1:1 protein: sgRNA delivery conditions at optimal doses of RNAiMAX or Lipofectamine 2000 by TO-PRO-3 live/dead stain and flow cytometry. Dose of RNAiMAX and Lipofectamine 2000 were both 0.8 µL in a volume of 275 µL in a 48-well plate format. Error bars reflect standard deviation from three biological replicates performed on different days.

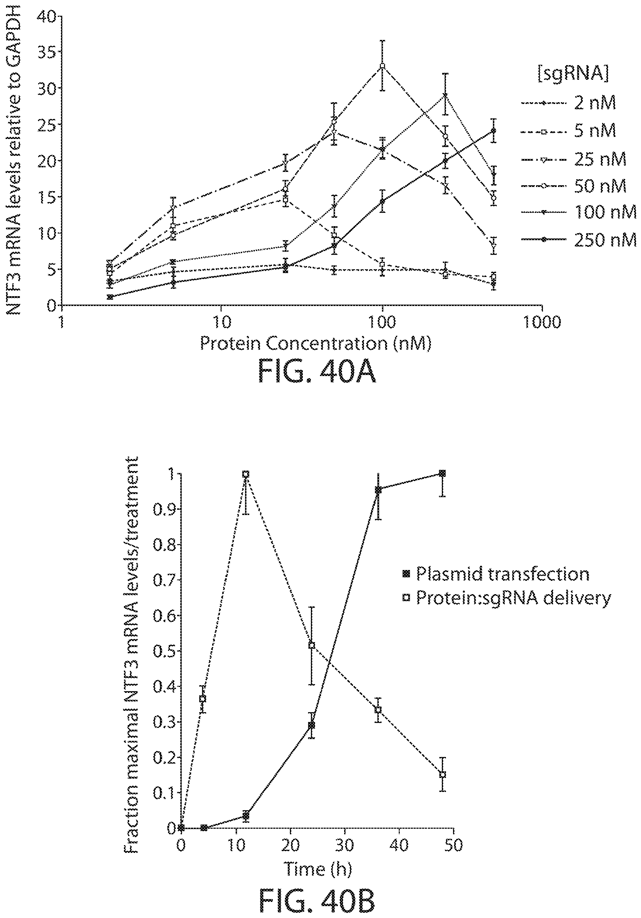

*Figures 40A-B.* Optimization of dCas9-VP64 delivery targeting the *NTF3* gene at varying concentrations of protein and sgRNA. Figure 40(A) shows HEK293T cells treated with dCas9-VP64 activator and either *NTF3*-targeting gRNA g2 or a mixture of all six *NTF3-*targeting sgRNAs for 16 hours and 0.8 µL RNAiMAX in 48-well plate format (275 µL final volume). NTF3 mRNA levels were determined by qRT-PCR and normalized to those of GAPDH. Total sgRNA concentrations are listed (each sgRNA is present at one-sixth of the listed total concentration). Figure 40(B) shows the time course for *NTF3* gene activation by protein: sgRNA delivery and plasmid transfection. NTF3 mRNA levels were measured at several time points using all six sgRNAs either from expression plasmids (in the case of the dCas9-VP64 activator plasmid transfection treatment), or as *in vitro* transcribed sgRNAs complexed with 100 nM dCas9-VP64 activator and cationic lipids (in the case of protein: sgRNA delivery). Error bars reflect standard deviation from six biological replicates performed on different days.

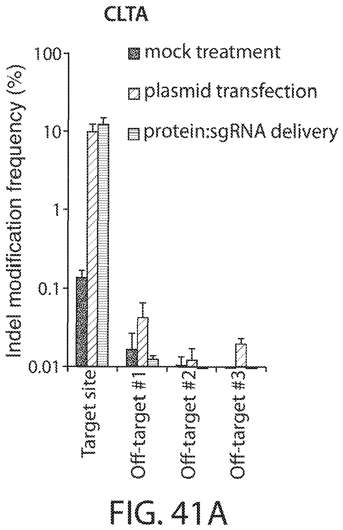

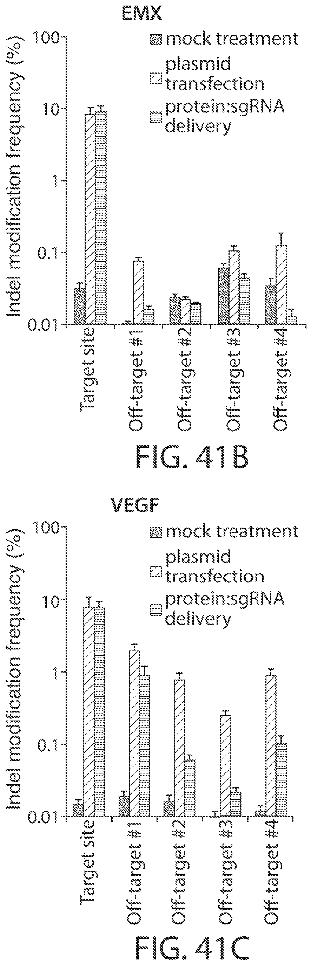

*Figures 41A-C.* Indel frequencies, measured by high-throughput sequencing, of several human genes treated either by a mock treatment, by transfection of Cas9 plasmid and sgRNA linear DNA PCR product, or by cationic lipid-mediated protein:sgRNA delivery are depicted. Mock treatment involved cationic lipid-mediated protein: sgRNA delivery of *EGFP-*targeting sgRNA instead of one of the three human gene-targeting sgRNAs. Figure 41(A) shows the on-target and off-target indel frequencies for the *CLTA* gene. Figure 41(B) provides the on-target and off-target indel frequencies for the *EMX* gene. Figure 41(C) demonstrates the on-target and off-target indel frequencies for the *VEGF* gene. Each on- and off-target sample was sequenced once with > 10,000 sequences analyzed per on-target sample and an average of > 111,000 sequences analyzed per off-target sample (Table 2). Error bars reflect standard deviation from three biological replicates performed on different days.

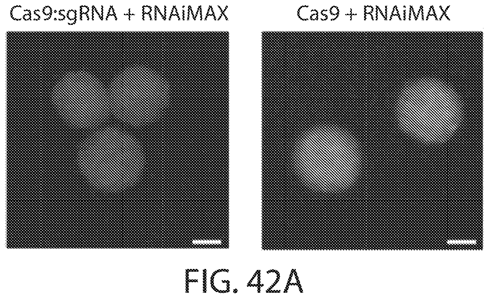

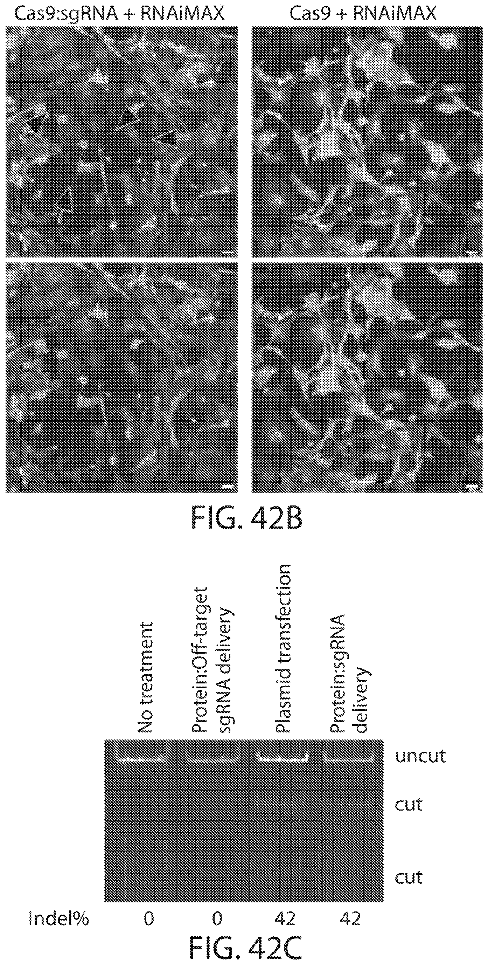

*Figures 42A-C.* Delivery of Cas9 endonuclease to mouse embryonic stem cells. Figure 42(A) shows floating spheres treated with 100 nM Cas9 protein and 0.8 µL RNAiMAX but no sgRNA (control) retained strong GFP fluorescence (right), while those treated with 100 nM Cas9:sgRNA and 0.8 µL RNAiMAX exhibited decreased GFP fluorescence (left). Scale bars are 100 µm. Figure 42(B) shows the control progenitor cells after cell attachment, and virtually all the control progenitor cells were GFP positive (right panels). Cas9:sgRNA treatment led to significant reduction in GFP expression (left panels) and many progenitor cells showed complete GFP knockdown (arrows) after cell attachment. Scale bars are 20 µm. Figure 42(C) shows a T7EI assay on stem cells harvested after imaging confirm cleavage of GFP reporter. Similar gene target modification efficiencies were observed from cationic lipid-mediated Cas9:sgRNA delivery (24%) and from co-transfection of Cas9 and EGFP sgRNA plasmids (20%).

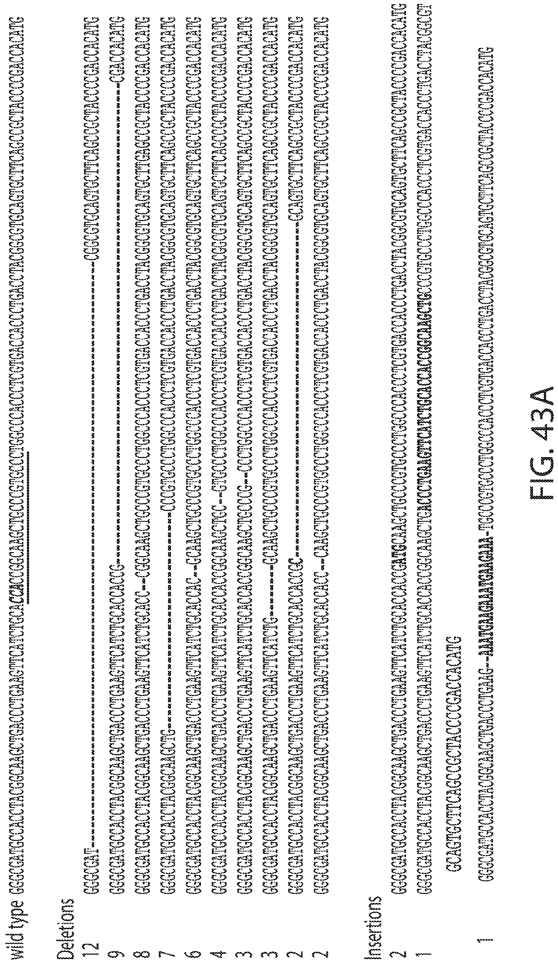

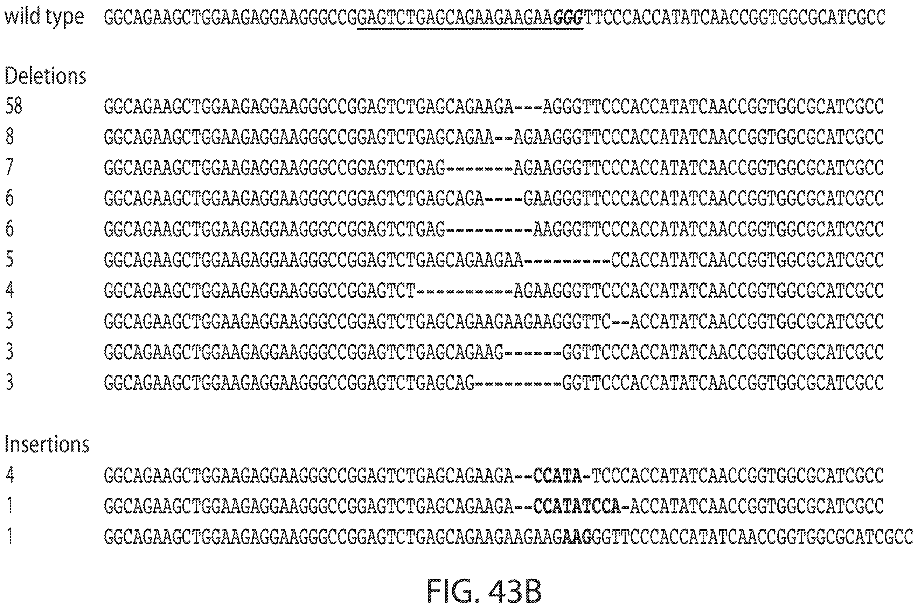

*Figures 43A-B.* Genome modification induced by cationic lipid-mediated protein delivery of Cas9 nuclease and sgRNA at endogenous loci *in vivo.* Approximately 10 days after injection of Cas9:sgRNA protein into Atoh1-GFP mice under identical conditions described in Figure 32(D), ~15 mg of mouse hair cell tissue was dissected. 150 ng of isolated genomic DNA was prepared for high-throughput sequencing. Figure 43(A) shows representative examples of genomic DNA sequences at the *EGFP* on-target locus that are modified following cationic lipid-mediated delivery of Cas9 and EGFP sgRNA in mouse hair cells. For each example shown, the unmodified genomic site is the first sequence, followed by the most abundant eight sequences containing deletions and three sequences containing insertions. The numbers before each sequence indicate sequencing counts. The sgRNA target sites are bold. Insertions and deletions are shown. PAM site is shown as well. Figure 43(B) shows an identical analysis as in Figure 42(A) for *EMX* on-target site in mouse hair cells. Indels shown here for both the *EGFP* and *EMX* genomic loci are from a single biological replicate chosen from a representative set of sequenced samples all showing similar indel profiles. The sequences shown in Figure 43(A), from top to bottom, correspond to SEQ ID NOs:223-236; and the sequences shown in Figure 43(B), from top to bottom, correspond to SEQ ID NOs:237-250.

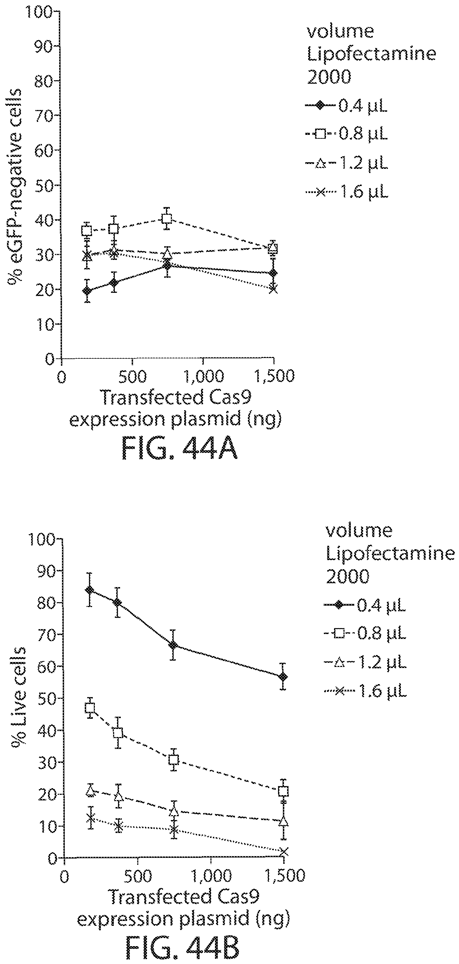

*Figures 44A-B.* Optimization of Cas9 plasmid transfection conditions and measurement of cellular toxicity at different doses of Lipofectamine 2000 is depicted. Figure 44(A) shows the optimization of transfection efficiency for Cas9 expression plasmid in U2OS EGFP reporter cell line was performed by varying both the amount of Cas9 plasmid and the dose of Lipofectamine 2000. Input sgRNA expression plasmid was held constant at 250 ng input DNA for all treatments. All treatments were performed in a 48-well plate with 275 µL DMEM-FBS without antibiotics. After 48 hours, cells were assayed for loss of EGFP by flow cytometry. Figure 44(B) measures the toxicity of various Cas9 plasmid/Lipofectamine 2000 transfection conditions after 48 hours using TO-PRO-3 live/dead stain and quantifying cellular toxicity by flow cytometry. From Figure 44(A) and Figure 44(B) a Cas9 plasmid dose of 750 ng and a Lipofectamine 2000 dose of 0.8 µL were chosen as plasmid transfection conditions that resulted in maximal gene disruption for the remaining studies in this work. For Figure 44(A) and Figure 44(B), error bars reflect standard deviation from three biological replicates performed on different days.

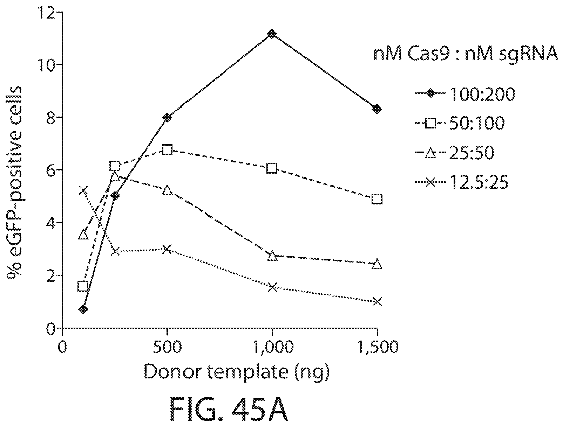

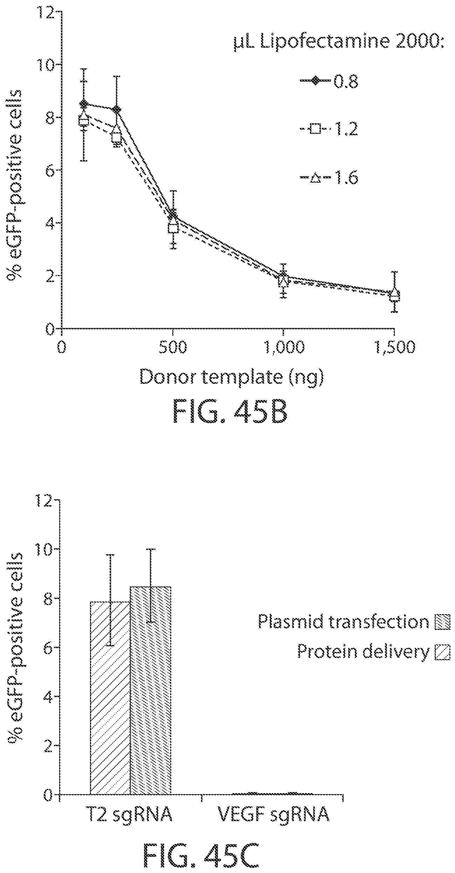

*Figures 45A-C.* Optimization and comparison of homology-directed repair (HDR) efficiency for Cas9:sgRNA delivery by cationic lipids and plasmid transfection is shown. Figure 45(A) shows Cas9:sgRNA protein delivery optimization of HDR efficiency in a reporter cell line that expresses EGFP upon repair of a disrupted *EGFP* reporter gene⁵⁷ using cationic lipid-mediated protein delivery, a 2:1 ratio of T2 sgRNA:Cas9 protein, 1 µL Lipofectamine 2000, and variable amounts of ssODN donor template (see materials and methods below) performed as a single treatment. Figure 45(B) shows optimization of plasmid transfection-mediated HDR using 700 ng Cas9 plasmid and 250 ng sgRNA plasmid with variable doses of Lipofectamine 2000 and ssODN donor template. Figure 45(C) HDR efficiency comparison of cationic lipid-mediated protein: sgRNA delivery and plasmid DNA transfection at optimized conditions for both techniques using on-target (T2) and non-target (VEGF) sgRNAs. For Figure 45(B-C), error bars reflect standard deviation of three independent biological replicates performed on different days.

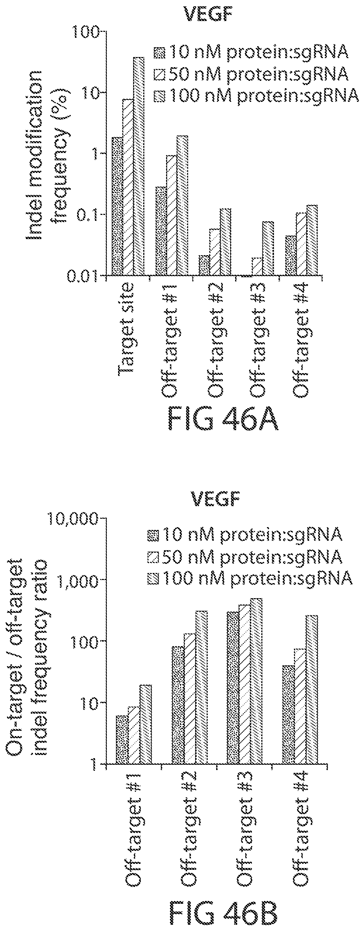

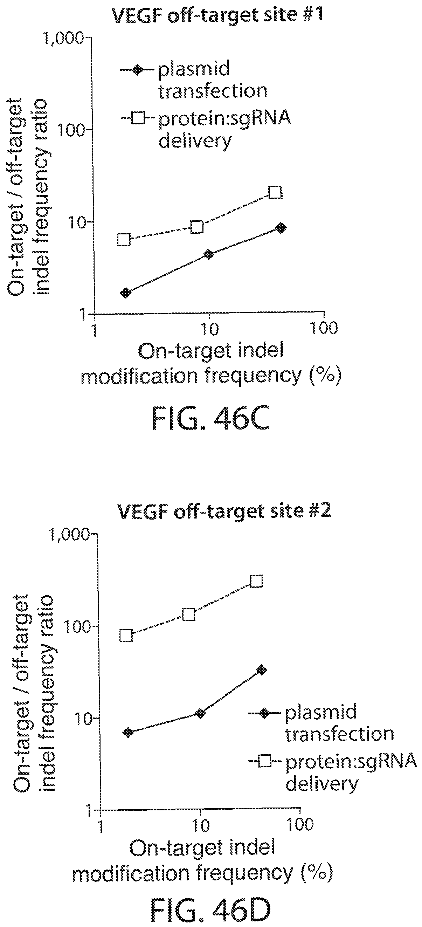

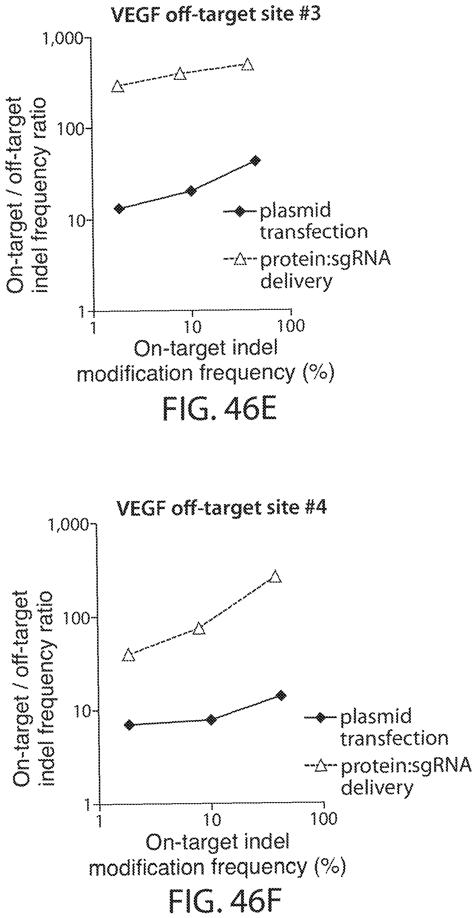

*Figures 46A-F.* Concentration dependence of on-target and off-target indel modification frequencies for Cas9 plasmid transfection or lipid-mediated protein: sgRNA delivery is shown. Figure 46(A) shows Indel modification frequencies measured by high-throughput sequencing for *VEGF* on- and off-target sites at varying doses of Cas9: sgRNA. Figure 46(B) shows on-target: off-target specificity ratio at different Cas9:sgRNA concentrations. Figure 46(C) shows a comparison of on-target: off target specificity ratio for protein delivery and plasmid transfection at *VEGF* off-target site #1 as a function of on-target indel modification frequency at a range of modification frequencies for both treatments (~1% to ~40 % indel modification frequency). Figures 46(D, E, F) show the same as Figure 46(C) for VEGF off-target sites #2, #3, and #4. Each on- and off-target sample was sequenced once with >10,000 sequences analyzed per on-target sample and an average of >111,000 sequences analyzed per off-target sample. All data shown were from a single biological replicate.

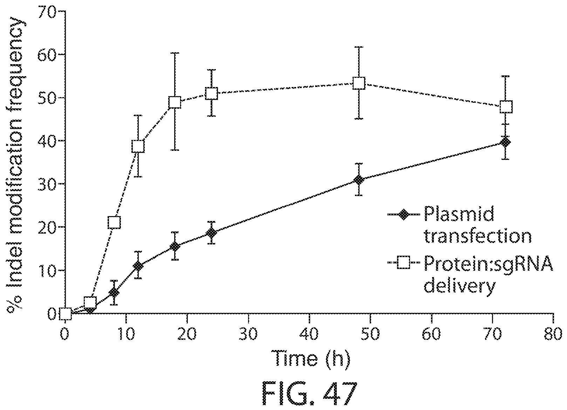

*Figure 47**.* A time course of Cas9 nuclease activity from protein: sgRNA delivery and plasmid transfection is shown. U2OS EGFP reporter cells were treated with either 50 nM Cas9 protein and 50 nM sgRNA and 0.8 µL Lipofectamine 2000 in 275 µL DMEM-FBS without antibiotics, or transfected with 750 ng Cas9 expression plasmid and 250 ng EGFP sgRNA expression plasmid for 2 hours. Media was either removed and samples collected after another 2 hours, or replaced with fresh DMEM-FBS without delivery agents and collected at later time points, as shown. Samples were analyzed for indels in the EGFP gene using a Surveyor T7E1 cleavage assay. Bands were quantified by ImageJ software. Error bars reflect standard deviation from three biological replicates performed on different days.

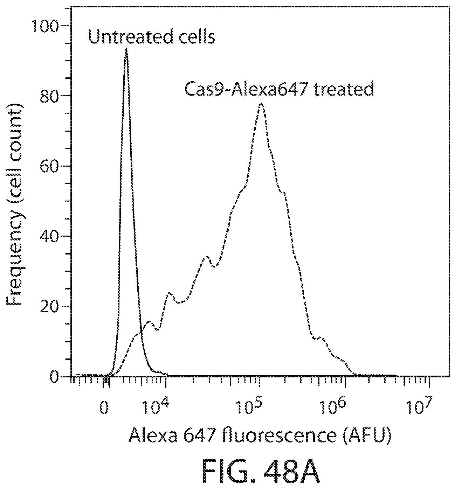

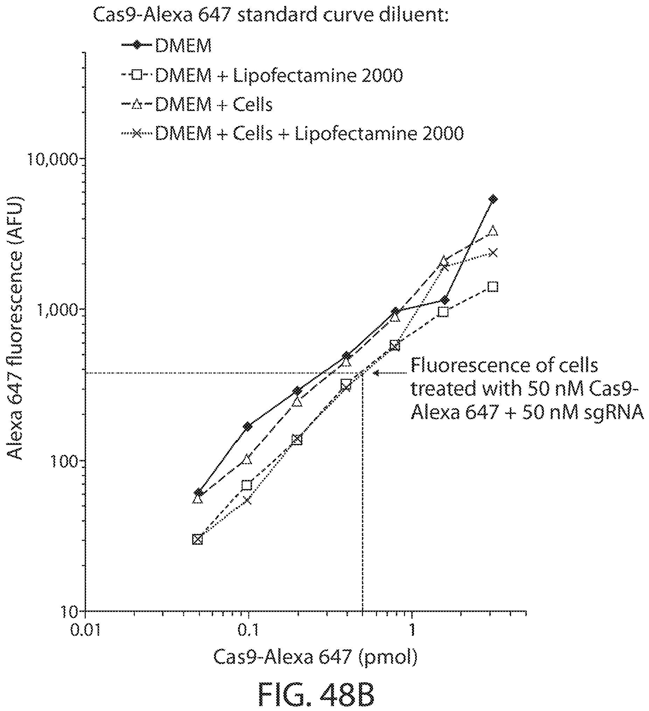

*Figures 48A-B.* Quantification of Cas9 protein uptake into U2OS EGFP reporter cells is shown. Figure 48(A) shows flow cytometry plots showing Alexa647 fluorescence of cells treated with 50 nM Alexa647-conjugated Cas9 and 50 nM EGFP sgRNA, or of untreated cells. Figure 48(B) U2OS EGFP reporter cells were treated with 50 nM Alexa647-conjugated Cas9 protein, 50 nM EGFP sgRNA, and 0.8 µL of Lipofectamine 2000. After a 4-hour incubation at 37 °C, cells were washed extensively with PBS containing 20 U/mL of heparin to remove electrostatically-bound cationic lipid complexes, and then trypsinized. In a plate reader (Tecan M1000 Pro) with fluorescence excitation at 650 nm and emission at 665 nm, wells each containing 10,000 Cas9-Alexa647-treated cells were measured for whole population fluorescence. Standard curves were established by measuring the fluorescence of known quantities of Cas9-Alexa647 in either DMEM containing 10% FBS, or in a suspension of trypsinized U2OS cells at 10,000 cells per well, with protein either diluted directly, or pre-complexed with 0.8 µL Lipofectamine 2000 then diluted. A two-fold serial dilution starting from 50 pmol to 0.048 pmols was performed to generate the standard curve samples. Values for 0.048 pmol to 3.125 pmol are shown. The intersection of the dotted black lines shows the measured total Alexa647 fluorescence of 10,000 cells treated with 50 nM Alexa647-conjugated Cas9 and 50 nM EGFP sgRNA and washed as described above. 50 nM Cas9-Alexa647-treated cells showed a total cell-associated Alexa647-labeled protein signal of 0.5 pmol per well. This quantity represents 4% of the input protein in the Cas9-Alexa647: sgRNA treatment, and corresponds to (6.02×10²³)*5.0×10⁻¹³ moles Cas9-Alexa647 / 10,000 cells per well = 3×10⁷ molecules of Cas9-Alexa647 per cell. Assuming a total protein content per cell of roughly 7.9×10⁹ molecules (estimate from *Molecular Cell Biology,* Section 1.2, 4th edition), internalized Cas9-Alexa647 represented 0.4% of total cellular protein. All values shown are the average of three technical replicates.

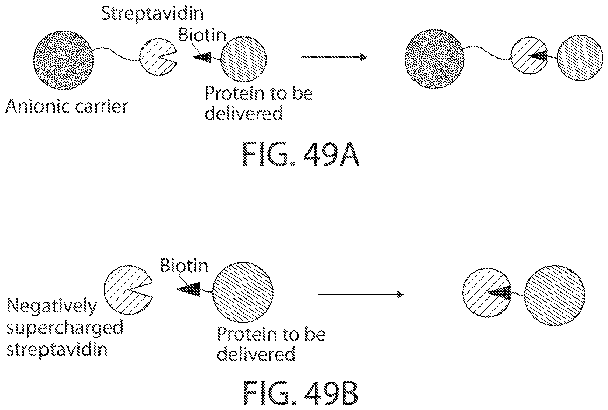

*Figures 49 A-B.* Generation of exemplary negatively charged protein complexes comprising biotinylated proteins to be delivered. Figure 49A shows an exemplary embodiment, in which a biotinylated protein to be delivered is complexed with an anionic carrier, *e.g.,* a negatively charged fluorescent protein, such as -30GFP, an anionic naturally occurring protein, an anionic peptide, or a synthetic anionic polymer. The anionic carrier is conjugated to streptavidin and contacted with the biotinylated protein to be delivered. Biotin can be linked to the protein to be delivered in any suitable manner, for example, chemically, *e.g*., via click chemistry, NHS ester, or maleimide, or enzymatically. The linkage of biotin to the protein to be delivered and/or the linkage of streptavidin to the anionic carrier may be permanent or cleavable, *e.g.,* by cellular proteases, esterases, or by a reducing cellular environment. Figure 49B shows an exemplary embodiment, in which a biotinylated protein to be delivered is complexed with an anionic streptavidin variant, *e.g*., with a negatively supercharged streptavidin variant, such as, for example, -40SAV. The anionic streptavidin variant is contacted with the biotinylated protein to be delivered. Biotin can be linked to the protein to be delivered in any suitable manner, for example, chemically, *e.g.,* via click chemistry, NHS ester, or maleimide, or enzymatically. The linkage of biotin to the protein to be delivered may be permanent or cleavable, *e.g.,* by cellular proteases, esterases, or by a reducing cellular environment. The protein complexes illustrated in Figure 49A-B can be contacted with a cationic polymer or a cationic lipid for cellular delivery.

### DETAILED DESCRIPTION OF CERTAIN EMBODIMENTS OF THE INVENTION

The present invention provide complexes, compositions, preparations, kits, systems, and related methods for the delivery of proteins to cells using cationic polymers or cationic lipids. The inventive concepts can be applied to the delivery of proteins that are charged or uncharged, naturally occurring or engineered. Typically, the protein to be delivered is introduced into the interior of a cell, *e.g.,* to cause a measurable biological effect or transformation in the cell. For example, in some embodiments, the biological effect comprises a therapeutic benefit to a subject in which the cell is found. The complexes, compositions, preparations, systems, kits, and related methods for delivery of proteins are thus useful for introducing proteins into a cell, *e.g.,* in the context of manipulating the cell for a diagnostic, research, or therapeutic purpose. The compositions, preparations, systems, kits, and related methods for delivery of proteins provided herein exhibit improved efficacy and/or reduced cytotoxicity, and ease of preparation as compared to current technologies. They are also widely applicable to a variety of proteins. For example, the compositions, preparations, systems, kits, and related methods for delivery of negatively charged proteins provided herein are applicable to the exemplary proteins listed in any of Tables 3-6. The cationic polymer or cationic lipid-mediated delivery of proteins using the compositions, preparations, systems, kits, and related methods provided herein allows for the manipulation/modification of the host cell *in vitro* or *in vivo* while avoiding the use of more invasive delivery methods, such as viral delivery of vectors encoding proteins to be delivered.

Some aspects of this disclosure provide compositions comprising a protein to be delivered and a cationic polymer or lipid. In some embodiments, the protein to be delivered is an anionic protein, *e.g.,* a protein exhibiting a negative net charge. In some embodiments, the protein to be delivered is associated with a nucleic acid. For example, in some such embodiments, the protein to be delivered is a nucleic acid-binding protein. One exemplary nucleic acid binding protein is Cas9 and its nucleic acid-binding variants. Other suitable nucleic acid-binding proteins are provided herein or will be apparent to those of skill in the art based on the present disclosure, which is not limited in this respect. In some embodiments, the protein to be delivered is associated with a supernegatively charged protein, wherein the net charge of the protein to be delivered and the supernegatively charged protein is negative.

In some embodiments, the inventive technology uses a supercharged protein to deliver a protein into a cell. In certain embodiments, the supercharged protein is an engineered protein. In some embodiments, the supercharged protein is a naturally occurring supercharged protein.

Some aspects of this invention are based on the recognition that negatively charged proteins or protein complexes, for example, supernegatively charged proteins, naturally occurring negatively charged proteins, proteins associated with nucleic acids, or fusion proteins with a net negative charge, can be associated with cationic polymers or cationic lipids, and that such protein:polymer or protein: lipid complexes are endocytosed by cells. Typically, proteins to be delivered are effectively taken up by cells together with the cationic polymer or lipid, are able to escape the cellular endosomes, and retain their biological function after cellular uptake.

In some embodiments, the protein to be delivered is a negatively charged protein, for example, a protein listed in any one of Tables 3-6. In some embodiments, the protein to be delivered is a functional effector protein (*e.g.,* a nuclease, transcriptional activator/repressor, a recombinases, or a Cas9 protein, or variants or fusions thereof). In some embodiments, proteins to be delivered are useful as therapeutic agents, diagnostic agents, or research tools. In some embodiments, a protein to be delivered, such as, for example, an enzyme, transcription factor, or binding protein, may be therapeutically active, *e.g.,* in that it modulates or ameliorates aberrant expression of a gene associated with a disease or disorder.

In some embodiments, methods are provided in which a cell is contacted with an inventive composition described herein to introduce the protein to be delivered into the cell. In some embodiments, an inventive composition is administered to a subject in need thereof to introduce a protein to be delivered into a cell within the subject, *e.g.,* into a cell associated with a disease or disorder. Suitable cells and cell types and proteins for delivery according to some aspects of this disclosure are listed herein and include, but are not limited to, human cells, mammalian cells, T-cells, neurons, stem cells, progenitor cells, blood cells, fibroblasts, epithelial cells, neoplastic cells, and tumor cells. Additional suitable cells, cell types, and proteins will be apparent to those of skill in the art, and the disclosure is not limited in this respect.

### Delivery of negatively charged proteins or protein complexes

Some aspects of this disclosure provide compositions for delivering proteins to cells. In some embodiments, the protein to be delivered is itself negatively charged, or is associated with a negatively charged molecule, such as a nucleic acid molecule or a negatively charged protein (*e.g.,* a supernegatively charged protein with a net charge of less than -5, less than -10, less than -20, less than -30, less than -40, less than -50, or less than -100), wherein the resulting complex (*e.g.,* the protein: nucleic acid complex or the protein: protein complex) exhibits a net negative charge. Such negatively charged proteins or protein complexes can be contacted with a cationic polymer or a cationic lipid, resulting in compositions that are effectively taken up by cells via endocytosis. Accordingly, some aspects of this disclosure provide compositions comprising a protein to be delivered and a cationic polymer or cationic lipid.

In some embodiments, the protein to be delivered is negatively charged. In some embodiments, the protein to be delivered is associated with a negatively charged molecule (*e.g.,* a negatively charged molecule with a net charge of less than -5, less than -10, less than -20, less than -30, less than -40, less than -50, or less than -100), for example, with a nucleic acid or with a supernegatively charged protein.

In some embodiments, the protein to be delivered is a naturally occurring negatively charged protein, or a negatively charged fragment thereof. In some embodiments, the protein to be delivered is a supernegatively charged protein or a negatively charged fragment thereof. In some embodiments, the fragment comprises a sequence of at least 10, at least 20, at least 30, at least 40, at least 50, at least 75, or at least 100 consecutive amino acids.

In some embodiments, in which the protein to be delivered is associated with a nucleic acid or with a negatively charged protein, the protein to be delivered is not negatively charged. In some such embodiments, the combined net charge of the protein to be delivered and of the nucleic acid is negative. In some embodiments, the combined net charge of the protein to be delivered and the associated supernegatively charged protein is negative. In some embodiments, the protein to be delivered is fused to a supernegatively charged protein thus forming a fusion protein.

In some embodiments, the net charge of the protein to be delivered, or the combined net charge of the protein to be delivered and the nucleic acid associated with the protein to be delivered, or the combined net charge of the protein to be delivered and the supernegatively charged protein associated with the protein to be delivered is less than -10, less than -20, less than -30, less than -40, less than -50, less than -60, less than -70, less than -80, less than -90, less than -100, less than -110, less than -120, less than -130, less than -140, less than - 150, less than -160, less than -170, less than -180, less than -190, less than -200, less than -250, less than -300, or less than -400.

In some embodiments, the charge:molecular weight ratio of the protein to be delivered, or the combined charge:molecular weight ratio of the protein to be delivered and the nucleic acid associated with the protein to be delivered, or the combined charge: molecular weight ratio of the protein to be delivered and the supernegatively charged protein associated with the protein to be delivered is less than -0.03, less than -0.04, less than -0.05, less than -0.06, less than -0.07, less than -0.08, less than -0.09, less than -0.1, less than -0.2, less than -0.3, less than -0.4, less than -0.5, less than -0.6, less than -0.7, less than -0.8, less than -0.9, less than -1, less than -1.1, less than -1.2, less than -1.3, less than -1.4, less than -1.5, less than -1.6, less than - 1.7, less than -1.8, less than -1.9, less than -2, less than -2.1, less than -2.2, less than -2.3, less than -2.4, less than -2.5, less than -2.6, less than -2.7, less than -2.8, less than -2.9, less than -3, less than -3.1, less than -3.2, less than -3.3, less than -3.4, less than -3.5, less than -3.6, less than - 3.7, less than -3.8, less than -3.9, or less than -4.

In some embodiments, the protein to be delivered is a protein listed in Table 3. In some embodiments, the protein to be delivered is implicated in a disease or disorder. In some embodiments, the protein to be delivered is listed in any of Tables 4, 5, and 6. In some embodiments, the protein to be delivered is a tumor suppressor. In some embodiments, the protein to be delivered is listed in Table 6. In some embodiments, the protein to be delivered is Sirt1 (-59, 86 kDa), PPARg (-13, 54 kDa), PRDM16 (-23, 140 kDa), PGC1a (-15, 91 kDa), TP53BP1 (-148, 213 kDa), Utrophin (-142, 394 kDa), Dystrophin (-89, 426 kDa), Bik (net charge -17, 18 kDa), IκBα (-29, 35 kDa), Von Hippel-Lindau disease tumor suppressor (-18, 24 kDa), an E3 ubiquitin ligase, or a metal-binding protein.

In some embodiments, the supernegatively charged protein associated with the protein to be delivered comprises a 3xFLAG sequence, a VP64 sequence, or a supernegatively charged fluorescent protein or streptavidin. In some embodiments, the supernegatively charged protein comprises -7 GFP or -20GFP, or a negatively charged fragment thereof. In some embodiments, the fragment comprises a sequence of at least 10, at least 20, at least 30, at least 40, at least 50, at least 75, or at least 100 consecutive amino acids.

In some embodiments, the protein to be delivered is associated with biotin thus forming a biotinylated version of the protein to be delivered. In some embodiments, the protein to be delivered is associated to the biotin via a linker. In some embodiments, the linker comprises a covalent bond generated via click chemistry, NHS ester chemistry, or maleimide chemistry. In some embodiments, the linker is a cleavable linker. In some embodiments, the linker is cleaved by a protease, an esterases, or by a reducing environment. In some embodiments, the linker is cleaved by an enzyme present in endosomes or under conditions present in endosomes. In some embodiments, the biotinylated protein to be delivered is associated with the supernegatively charged protein via non-covalent interaction. In some embodiments, the supernegatively charged protein is a supernegatively charged avidin or avidin variant, or a biotin-binding fragment thereof. In some embodiments, the supernegatively charged avidin or avidin variant is a supernegatively charged streptavidin or a biotin-binding fragment thereof. In some embodiments, the supernegatively charged protein is fused to an avidin or avidin variant. In some embodiments, the avidin or avidin variant is streptavidin, or a biotin-binding fragment thereof.

In some embodiments, the cationic polymer or the cationic lipid is suitable for delivery of an agent bound by the polymer or lipid to a cell. In some embodiments, the cationic lipid is selected from the group consisting of Lipofectamine^{®} 2000, Lipofectamine^{®} 3000, Lipofectamine^{®} RNAiMAX, and Lipofectamine^{®} LTX.

In some embodiments, the composition exhibits low toxicity when administered to a population of cells. In some embodiments, the at least 60%, at least 65%, at least 70%, at least 75%, at least 80%, at least 85%, at least 90%, at least 95%, or at least 99% of the cells are viable 24 hours after administration of an amount of the composition effective for delivery of the protein to be delivered into at least 1%, at least 2%, at least 5%, at least 10%, at least 20%, at least 25%, at least 30%, or at least 50% of the cells.

In some embodiments, the composition is a pharmaceutical composition. Some aspects of this disclosure provide compositions comprising (a) a protein to be delivered; and (b) a negatively charged molecule conjugated to the protein to be delivered resulting in a complex that is negatively charged. In some embodiments, the negatively charged molecule is a nucleic acid. In some embodiments, the negatively charged molecule is a negatively charged protein. In some embodiments, the negatively charged protein is a supernegatively charged protein, e.g., a supernegatively charged protein provided herein or otherwise known to those of skill in the art. In some embodiments, the supernegatively charged protein is a supernegatively charged fluorescent protein or a supernegatively charged streptavidin.

Some aspects of this disclosure provide methods for delivering a protein to be delivered to a cell, comprising contacting the cell with a composition provided herein. In some embodiments, the contacting is *in vitro.* In some embodiments, the contacting is *in vivo.*

Some aspects of this disclosure provide kits comprising a composition as provided herein or kits for carrying out a delivery method as provided herein.

### Supercharged Proteins

Supercharged proteins for use in the present invention can be produced by changing non-conserved amino acids on the surface of a protein to more polar or charged amino acid residues. In certain embodiments, non-conserved amino acids on the surface of the protein are mutated into amino acids that are positively charged at physiological pH (pH ~7.4). The amino acid residues to be modified may be hydrophobic, hydrophilic, charged, or a combination thereof. Supercharged proteins can also be produced by the attachment of charged moieties to the protein in order to supercharge the protein. Supercharged proteins frequently are resistant to aggregation, have an increased ability to refold, resist improper folding, have improved solubility, and are generally more stable under a wide range of conditions, including denaturing conditions such as heat or the presence of a detergent.

Supercharged proteins suitable for use according to aspects of this disclosure are known in the art and include, without limitation, those supercharged proteins disclosed in international PCT patent application, PCT/US07/70254, filed June 1, 2007, published as WO 2007/143574 on December 13, 2007; in international PCT application, PCT/US09/041984, filed on April 28, 2009, published as WO 2009/134808 on November 5, 2009; and in international PCT application, PCT/US10/001250, filed on April 28, 2010, published as WO 2010/129023 on November 11, 2010; the entire contents of each of which are incorporated herein by reference. In some embodiments, the supercharged protein is an engineered supercharged protein. In some embodiments, the supercharged protein is a naturally occurring supercharged protein, *e.g.,* a naturally supercharged protein disclosed in international PCT application, PCT/US10/001250, filed on April 28, 2010, published as WO 2010/129023 on November 11, 2010; each of which is incorporated herein by reference. In some embodiments, the supercharged protein, engineered or naturally occurring, exhibits a charge: molecular weight ratio of greater than 0.8, *e.g.,* ≥0.85, ≥0.9, ≥0.95, ≥1, ≥1.1, ≥1.2, ≥1.3, ≥1.4, ≥1.5, ≥1.6, ≥1.7, ≥1.8, ≥1.9, ≥2, ≥2.5, ≥3, ≥4, ≥5, ≥6, ≥7, ≥8, or ≥10.

The supercharged protein employed may be derived from any species of plant, animal, and/or microorganism. In certain embodiments, the supercharged protein is a mammalian protein. In certain embodiments, the supercharged protein is a human protein. In certain embodiments, the protein is derived from an organism typically used in research. For example, the protein to be modified may be from a primate (*e.g.,* ape, monkey), rodent (*e.g.,* rabbit, hamster, gerbil), pig, dog, cat, fish (*e.g., Danio rerio*)*,* nematode (*e.g., C. elegans*)*,* yeast (*e.g., Saccharomyces cerevisiae*)*,* or bacteria (*e.g., E. coli*)*.* In certain embodiments, the protein is non-immunogenic. In certain embodiments, the protein is non-antigenic. In certain embodiments, the protein does not have inherent biological activity or has been modified to have no biological activity. In certain embodiments, the protein is chosen based on its targeting ability. In certain embodiments, the protein is a green fluorescent protein. In some embodiments, the supercharged protein is supercharged glutathione S-transferase (GST). In some embodiments, the supercharged protein is supercharged streptavidin.

In some embodiments, a supercharged protein is used that has been modified to increase the overall net charge, or to increase the total number of charged residues on the protein surface. In certain embodiments, the theoretical net charge of the supercharged protein is increased by at least +1, at least +2, at least +3, at least +4, at least +5, at least +10, at least +15, at least +20, at least +25, at least +30, at least +35, or at least +40 as compared to the unmodified protein. In certain embodiments, the theoretical net charge of the supercharged protein is at least +1, at least +2, at least +3, at least +4, at least +5, at least +10, at least +15, at least +20, at least +25, at least +30, at least +35, or at least +40 at physiological pH (*i.e.,* ~7.4).

In other embodiments, for example those involving use of cationic lipids and/or cationic polymers, a supercharged protein is used that has been modified to decrease the overall net charge, or to decrease the total number of charged residues on the protein surface. In certain embodiments, the theoretical net charge of the supercharged protein is decreased ("minus" or "negative" represented by '-') by at least -1, at least -2, at least -3, at least -4, at least -5, at least -10, at least -15, at least -20, at least -25, at least -30, at least -35, at least -40, at least -45, or at least -50 as compared to the unmodified protein. In certain embodiments, the theoretical net charge of the supercharged protein is at least -1, at least -2, at least -3, at least -4, at least -5, at least -10, at least -15, at least -20, at least -25, at least -30, at least -35, at least -40, at least -45, or at least -50.

While some exemplary supercharged proteins are described herein in order to exemplify the inventive technology, the disclosure is not limited in this respect. Those of skill in the art will be able to ascertain additional suitable supercharged proteins for delivering functional effector proteins to cells based on the instant disclosure. A number of naturally occurring proteins may be modified to generate suitable supercharged proteins. The desired modifications in such proteins may be accomplished using any techniques known in the art. Recombinant DNA techniques for introducing such changes in a protein sequence are well known in the art. In certain embodiments, the modifications are made by site-directed mutagenesis of the polynucleotide encoding the protein. Other techniques for introducing mutations are discussed in Molecular Cloning: A Laboratory Manual, 2nd Ed., ed. by Sambrook, Fritsch, and Maniatis (Cold Spring Harbor Laboratory Press: 1989); the treatise, Methods in Enzymology (Academic Press, Inc., N.Y.); Ausubel et al., Current Protocols in Molecular Biology (John Wiley & Sons, Inc., New York, 1999); each of which is incorporated herein by reference.

Supercharged proteins may be further modified. Proteins including supercharged proteins can be modified using techniques known to those of skill in the art. For example, supercharged proteins may be modified chemically or biologically. One or more amino acids may be added, deleted, or changed from the primary sequence. For example, a poly-histidine tag or other tag may be added to the supercharged protein to aid in the purification of the protein. Other peptides or proteins may be added onto the supercharged protein to alter the biological, biochemical, and/or biophysical properties of the protein. For example, an endosomolytic peptide may be added to the primary sequence of the supercharged protein, or a targeting peptide, may be added to the primary sequence of the supercharged protein. Other modifications of the supercharged protein include, but are not limited to, post-translational modifications (*e.g*., glycosylation, phosphorylation, acylation, lipidation, farnesylation, acetylation, proteolysis, *etc.).* In certain embodiments, the supercharged protein is modified to reduce its immunogenicity. In certain embodiments, the supercharged protein is modified to enhance its ability to deliver a functional effector protein (*e.g*., nucleases, transcriptional activators/repressors, recombinases, Cas9 proteins including variants and fusions thereof, *etc*.) to a cell. In certain embodiments, the supercharged protein is conjugated to a polymer. For example, the protein may be PEGylated by conjugating the protein to a polyethylene glycol (PEG) polymer. Other methods can be used to produce supercharged proteins without modification of the protein sequence. For example, moieties that alter the net charge can be attached to proteins (*e.g*., by chemical or enzymatic reactions) to provide surface charge to achieve supercharging. In certain embodiments, the method of modifying proteins described in Shaw et al., Protein Science 17: 1446, 2008 is used to supercharge a protein that is used in the instantly disclosed inventive technology.

The design and creation of variants of several different supercharged proteins suitable for use with the instantly disclosed technology is described in international PCT patent application, PCT/US07/70254, filed June 1, 2007, published as WO 2007/143574 on December 13, 2007; in international PCT application, PCT/US09/041984, filed on April 28, 2009, published as WO 2009/134808 on November 5, 2009; and in international PCT application PCT/US10/001250, filed on April 28, 2010, published as WO 2010/129023 on November 11, 2010; the entire contents of each of which are incorporated herein by reference. Some of the disclosed supercharged proteins described therein have been shown to be more stable and to retain their biological function, *e.g*., their fluorescence in the case of fluorescent proteins. For example, a green fluorescent protein (GFP) from *Aequorea victoria* is described in GenBank Accession Number P42212, incorporated herein by reference. The amino acid sequence of this wild type GFP is as follows:

MSKGEELFTGVVPILVELDGDVNGHKFSVSGEGEGDATYGKLTLKFICTTGKLPVPWPTLVTTFSYGVQCFSRYPDH
MKQHDFFKSAMPEGYVQERTIFFKDDGNYKTRAEVKFEGDTLVNRIELKGIDFKEDGNILGHKLEYNYNSHNVYIMA
DKQKNGIKVNFKIRHNIEDGSVQLADHYQQNTPIGDGPVLLPDNHYLSTQSALSKDPNEKRDHMVLLEFVTAAGITH
GMDELYK (SEQ ID NO:14)

Wild type GFP has a theoretical net charge of -7. Variants with a theoretical net charge of -29, -30, -25, +15, +25, +36, +48, and +49 have been reported, *e.g.,* in in international PCT application PCT/US10/001250, filed on April 28, 2010, published as WO 2010/129023 on November 11, 2010, the entire contents of which are incorporated herein by reference. Even after heating the +36 GFP to 95 °C, 100% of the variant protein is soluble and the protein retains ≥70% of its fluorescence.

Some aspects of this disclosure are based on the discovery that +36 GFP efficiently delivers functional effector proteins (*e.g*., nucleases, transcriptional activators/repressors, recombinases, Cas9 proteins including variants and fusions thereof, *etc*.) to target cells, and that the effector proteins so delivered retain their biological function. Therefore, GFP or other proteins with a net charge of at least +15, at least +25, at least +30, at least +35, or at least +40 are thought to be particularly useful for introducing functional effector proteins into a cell.

In some embodiments, particularly useful supercharged proteins are proteins that allow for a charge distribution or a surface charge density similar to that of +36 GFP. Further, in some embodiments, particularly useful supercharged proteins are proteins exhibiting a stable folded structure not easily perturbed by supercharging, thus allowing the supercharged protein to be well folded. In some embodiments, particularly useful supercharged proteins are proteins sharing a structural feature with a supercharged protein described herein or in international PCT patent application, PCT/US07/70254, filed June 1, 2007, published as WO 2007/143574 on December 13, 2007; in international PCT application, PCT/US09/041984, filed on April 28, 2009, published as WO 2009/134808 on November 5, 2009; and in international PCT application, PCT/US10/001250, filed on April 28, 2010, published as WO 2010/129023 on November 11, 2010; the entire contents of each of which are incorporated herein by reference; for example, a globular structure, or a β-barrel structure. Protein folding, protein fold structure stability and perturbation of protein folding by substitution of specific amino acids with differently charged amino acids, charge distribution, and surface charge density can be modeled *in silico* by methods and algorithms provided herein and others known to those of skill in the art. Accordingly, it will be apparent to those of skill in the art from no more than routine experimentation, whether a supercharged protein in question will be well folded. Thus, those of skill in the art will be able to identify from a given amino acid sequence whether a given supercharged protein will be useful for cellular delivery of a functional effector protein according to the technology described herein.

Some exemplary, suitable variants of GFP include, without limitation:
+15 GFP: 
MGHHHHHHGGASKGERLFTGVVPILVELDGDVNGHKFSVRGEGEGDATRGKLTLKFICTTGKLPVPWPTLVTTLTYG
VQCFSRYPKHMKRHDFFKSAMPEGYVQERTISFKKDGTYKTRAEVKFEGRTLVNRIELKGRDFKEKGNILGHKLEYN
FNSHNVYITADKRKNGIKANFKIRHNVKDGSVQLADHYQQNTPIGRGPVLLPRNHYLSTRSALSKDPKEKRDHMVLL
EFVTAAGITHGMDELYK (SEQ ID NO:15)
+25 GFP: 
MGHHHHHHGGASKGERLFTGVVPILVELDGDVNGHKFSVRGKGKGDATRGKLTLKFICTTGKLPVPWPTLVTTLTYG
VQCFSRYPKHMKRHDFFKSAMPKGYVQERTISFKKDGTYKTRAEVKFEGRTLVNRIKLKGRDFKEKGNILGHKLRYN
FNSHNVYITADKRKNGIKANFKIRHNVKDGSVQLADHYQQNTPIGRGPVLLPRNHYLSTRSALSKDPKEKRDHMVLL
EFVTAAGITHGMDELYK (SEQ ID NO:16)
+36 GFP: 
MGHHHHHHGGASKGERLFRGKVPILVELKGDVNGHKFSVRGKGKGDATRGKLTLKFICTTGKLPVPWPTLVTTLTYG
VQCFSRYPKHMKRHDFFKSAMPKGYVQERTISFKKDGKYKTRAEVKFEGRTLVNRIKLKGRDFKEKGNILGHKLRYN
FNSHKVYITADKRKNGIKAKFKIRHNVKDGSVQLADHYQQNTPIGRGPVLLPRNHYLSTRSKLSKDPKEKRDHMVLL
EFVTAAGIKHGRDERYK (SEQ ID NO:17)
+42 GFP: 
MGHHHHHHGGRSKGKRLFRGKVPILVELKGDVNGHKFSVRGKGKGDATRGKLTLKFICTTGKLPVPWPTLVTTLTYG
VQCFSRYPKHMKRHDFFKSAMPKGYVQERTISFKKDGKYKTRAEVKFEGRTLVNRIKLKGRDFKEKGNILGHKLRYN
FNSHKVYITADKRKNGIKAKFKIRHNVKDGSVQLADHYQQNTPIGRGPVLLPRKHYLSTRSKLSKDPKEKRDHMVLL
EFVTAAGIKHGRKERYK (SEQ ID NO:18)
+48 GFP: 
MGHHHHHHGGRSKGKRLFRGKVPILVKLKGDVNGHKFSVRGKGKGDATRGKLTLKFICTTGKLPVPWPTLVTTLTYG
VQCFSRYPKHMKRHDFFKSAMPKGYVQERTISFKKDGKYKTRAEVKFKGRTLVNRIKLKGRDFKEKGNILGHKLRYN
FNSHKVYITADKRKNGIKAKFKIRHNVKDGSVQLAKHYQQNTPIGRGPVLLPRKHYLSTRSKLSKDPKEKRDHMVLL
EFVTAAGIKHGRKERYK (SEQ ID NO:19)
+49 GFP: 
MGHHHHHHGGRSKGKRLFRGKVPILVKLKGDVNGHKFSVRGKGKGDATRGKLTLKFICTTGKLPVPWPTLVTTLTYG
VQCFSRYPKHMKRHDFFKSAMPKGYVQERTISFKKDGKYKTRAEVKFKGRTLVNRIKLKGRDFKEKGNILGHKLRYN
FNSHKVYITADKRKNGIKAKFKIRHNVKDGSVQLAKHYQQNTPIGRGPVLLPRKHYLSTRSKLSKDPKEKRDHMVLK
EFVTAAGIKHGRKERYK (SEQ ID NO:20)
-7 GFP: 
MGHHHHHHGGASKGEELFTGVVPILVELDGDVNGHKFSVRGEGEGDATNGKLTLKFICTTGKLPVPWPTLVTTLTYG
VQCFSRYPDHMKQHDFFKSAMPEGYVQERTISFKDDGTYKTRAEVKFEGDTLVNRIELKGIDFKEDGNILGHKLEYN
FNSHNVYITADKQKNGIKANFKIRHNVEDGSVQLADHYQQNTPIGDGPVLLPDNHYLSTQSALSKDPNEKRDHMVLL
EFVTAAGITHGMDELYK ( SEQ ID NO:275)
-25 GFP: 
MGHHHHHHGGASKGEELFTGVVPILVELDGDVNGHEFSVRGEGEGDATEGELTLKFICTTGELPVPWPTLVTTLTYG
VQCFSRYPDHMKQHDFFKSAMPEGYVQERTISFKDDGTYKTRAEVKFEGDTLVNRIELKGIDFKEDGNILGHKLEYN
FNSHDVYITADKQENGIKAEFEIRHNVEDGSVQLADHYQQNTPIGDGPVLLPDDHYLSTESALSKDPNEDRDHMVLL
EFVTAAGIDHGMDELYK ( SEQ ID NO:276)
-29 GFP: 
MGHHHHHHGGASKGEELFDGEVPILVELDGDVNGHEFSVRGEGEGDATEGELTLKFICTTGELPVPWPTLVTTLTYG
VQCFSRYPDHMDQHDFFKSAMPEGYVQERTISFKDDGTYKTRAEVKFEGDTLVNRIELKGIDFKEDGNILGHKLEYN
FNSHDVYITADKQENGIKAEFEIRHNVEDGSVQLADHYQQNTPIGDGPVLLPDDHYLSTESALSKDPNEDRDHMVLL
EFVTAAGIDHGMDELYK ( SEQ ID NO:277)
(-)30 GFP:

MGHHHHHHGGASKGEELFDGVVPILVELDGDVNGHEFSVRGEGEGDATEGELTLKFICTTGELPVPWPTLVTTLTYG
VQCFSDYPDHMDQHDFFKSAMPEGYVQERTISFKDDGTYKTRAEVKFEGDTLVNRIELKGIDFKEDGNILGHKLEYN
FNSHDVYITADKQENGIKAEFEIRHNVEDGSVQLADHYQQNTPIGDGPVLLPDDHYLSTESALSKDPNEDRDHMVLL
EFVTAAGIDHGMDELYK (SEQ ID NO:21)

In some embodiments, a supercharged variant of streptavidin (SAV) is used for delivery of a protein to be delivered to a target cell. Such variants retain the capability of wild-type streptavidin to bind biotin. The amino acid sequence of wild-type streptavidin and of some exemplary useful streptavidin variants are provided below. Wild type streptavidin has a theoretical net charge of -4. The provided SAV variants with a theoretical net charge of -40 and +52 are soluble and bind biotin.
Wild type SAV: 
AAEAGITGTWYNQLGSTFIVTAGADGALTGTYESAVGNAESRYVLTGRYDSAPATDGSGTALGWTVAWKNNYRNAHS
ATTWSGQYVGGAEARINTQWLLTSGTTEANAWKSTLVGHDTFTKVKPSAAS (SEQ ID NO:278)
-40 SAV: 
MGHHHHHHGGAEAGITGTWYNQLGSTFIVTAGADGALTGTYESAVGDAESEYVLTGRYDSAPATDGSGTALGWTVAW
KNDYENAHSATTWSGQYVGGAEARINTQWLLTSGTTEADAWKSTLVGHDTFTKVEPSAAS (SEQ ID NO:279)
+52 SAV

MGHHHHHHGGAKAGITGTWYNQLGSTFIVTAGAKGALTGTYESAVGNAKSRYVLTGRYDSAPATKGSGTALGWTVA
WKNKYRNAHSAlTWSGQYVGGAKARINTQWLLTSGTTKAKAWKSTLVGHDTFTKVKPSAAS (SEQ ID NO:
280)

Additional suitable supercharged proteins and protein variants will be apparent to those of skill in the art. It will also be apparent to the skilled artisan that some of the sequences provided herein, *e.g.*, some of the sequences provided immediately above, include an artificial tag, *e.g.,* an N-terminal His6 tag, and that sequences without such a tag or with a different tag are also suitable.

In order to promote the biological function of the functional effector protein (*e.g.,* nucleases, transcriptional activators/repressors, recombinases, Cas9 proteins including variants and fusions thereof, *etc*.) after delivery to a cell, it may be desirable to enhance endosomal escape of the functional effector protein after cellular uptake. A supercharged protein or a functional effector protein may be fused to or associated with a protein, peptide, or other entity known to enhance endosome degradation or lysis of the endosome. In certain embodiments, the peptide is hemagglutinin 2 (HA2) peptide which is known to enhance endosome degradation. In certain particular embodiments, HA2 peptide is fused to supercharged GFP (*e.g.,* +36 GFP). In certain particular embodiments, the fused protein is of the sequence:
+36 GFP-HA2

MGHHHHHHGGASKGERLFRGKVPILVELKGDVNGHKFSVRGKGKGDATRGKLTLKFICTTGKLPVPWPTLVTTLTYG
VQCFSRYPKHMKRHDFFKSAMPKGYVQERTISFKKDGKYKTRAEVKFEGRTLVNRIKLKGRDFKEKGNILGHKLRYN
FNSHKVYITADKRKNGIKAKFKIRHNVKDGSVQLADHYQQNTPIGRGPVLLPRNHYLSTRSKLSKDPKEKRDHMVLL
EFVTAAGIKHGRDERYKGSAGSAAGSGEFGLFGAIAGFIENGWEGMIDG (SEQ ID NO:22)

In certain embodiments, the endosomolytic peptide is melittin peptide (GIGAVLKVLTTGLPALISWIKRKRQQ, SEQ ID NO: 23) (Meyer et al., JACS 130(11): 3272-3273, 2008; which is incorporated herein by reference). In certain embodiments, the melittin peptide is modified by one, two, three, four, or five amino acid substitutions, deletions, and/or additions. In certain embodiments, the melittin peptide is of the sequence: CIGAVLKVLTTGLPALISWIKRKRQQ (SEQ ID NO:24). In certain particular embodiments, the melittin peptide is fused to supercharged GFP (*e.g.,* +36 GFP).

In certain embodiments, the endosomolytic peptide is penetratin peptide (RQIKIWFQNRRMKWKK-amide, SEQ ID NO:25), bovine PrP (1-30) peptide (MVKSKIGSWILVLFVAMWSDVGLCKKRPKP-amide, SEQ ID NO: 26), MPGΔ^{NLS} peptide (which lacks a functional nuclear localization sequence because of a K->S substitution) (GALFLGWLGAAGSTMGAPKSKRKV, SEQ ID NO:27), TP-10 peptide (AGYLLGKINLKALAALAKKIL-amide, SEQ ID NO:28), and/or EB1 peptide (LIRLWSHLIHIWFQNRRLKWKKK-amide, SEQ ID NO:29) (Lundberg et al., 2007, FASEB J. 21:2664; incorporated herein by reference). In certain embodiments, the penetratin, PrP (1-30), MPG, TP-10, and/or EB1 peptide is modified by one, two, three, four, or five amino acid substitutions, deletions, and/or additions. In certain particular embodiments, the PrP (1-30), MPG, TP-10, and/or EB1 peptide is fused to supercharged GFP (*e.g.,* +36 GFP). In some embodiments, an Aurein peptide is fused to the supercharged protein.

Other peptides or proteins may also be fused to the supercharged protein or to a fusion protein comprising a supercharged protein and a functional effector protein (*e.g.,* nucleases, transcriptional activators/repressors, recombinases, Cas9 proteins including variants and fusions thereof, *etc*.). For example, a targeting peptide may be fused to the supercharged protein in order to selectively deliver a functional effector protein to a particular cell type. Peptides or proteins that enhance cellular uptake of the functional effector protein may also be used. In certain embodiments, the peptide fused to the supercharged protein is a peptide hormone. In certain embodiments, the peptide fused to the supercharged protein is a peptide ligand.

The exemplary supercharged proteins described in detail herein are not meant to limit the disclosure, and one of skill in the art will appreciate that other supercharged proteins may be used for the cellular delivery of functional effector proteins (*e.g*., nucleases, transcriptional activators/repressors, recombinases, Cas9 proteins including variants and fusions thereof, *etc.*), including, but not limited to, other GFP-style fluorescent proteins. In certain embodiments, the supercharged protein is a supercharged version of blue fluorescent protein. In certain embodiments, the supercharged protein is a supercharged version of cyan fluorescent protein. In certain embodiments, the supercharged protein is a supercharged version of yellow fluorescent protein. Exemplary suitable fluorescent proteins include, but are not limited to, enhanced green fluorescent protein (EGFP), AcGFP, TurboGFP, Emerald, Azami Green, ZsGreen, EBFP, Sapphire, T-Sapphire, ECFP, mCFP, Cerulean, CyPet, AmCyan1, Midori-Ishi Cyan, mTFP1 (Teal), enhanced yellow fluorescent protein (EYFP), Topaz, Venus, mCitrine, YPet, PhiYFP, ZsYellow1, mBanana, Kusabira Orange, mOrange, dTomato, dTomato-Tandem, DsRed, DsRed2, DsRed-Express (T1), DsRed-Monomer, mTangerine, mStrawberry, AsRed2, mRFP1, JRed, mCherry, HcRed1, mRaspberry, HcRed1, HcRed-Tandem, mPlum, and AQ143.

Yet other proteins that may be supercharged and used, *e.g.,* in the delivery of functional effector proteins as disclosed herein (*e.g*., nucleases, transcriptional activators/repressors, recombinases, Cas9 proteins including variants and fusions thereof, *etc*.), include histone components or histone-like proteins, high-mobility-group proteins (HMGs), enzymes (*e.g*., amylases, pectinases, hydrolases, proteases, glucose isomerase, lipases, phytases, alglucerase, imiglucerase, agalsidase beta, α-1-iduronidase, acid α-glucosidase, and iduronate-2-sulfatase, *N*-acetylgalactosamine-4-sulfatase.

Charged polymers other than proteins may also be used to deliver functional effector proteins. Additionally, as described in greater detail herein, cationic lipids and lipid-like materials as well as cationic polymers can also be used to deliver functional effector proteins. Suitable cationic lipids, lipid-like materials and cationic polymers are disclosed herein and additional suitable lipids and lipid-like materials are known to those of skill in the art (see, *e.g.,* those described in Akinc et al., Nature Biotechnology 26, 561 - 569 (2008), the entire contents of which are incorporated herein by reference).

### Delivery of functional effector proteins using supercharged proteins

The present invention provides systems and methods for the delivery of functional effector proteins (*e.g*., nucleases, transcriptional activators/repressors, recombinases, Cas9 proteins including variants and fusions thereof, *etc*.) to cells *in vivo, ex vivo,* or *in vitro.* Such systems and methods typically involve association of the functional effector protein with a supercharged protein to form a complex or a fusion protein, and delivery of the complex or fusion protein to a cell. In some embodiments, the functional effector protein to be delivered by the supercharged protein has therapeutic activity. In some embodiments, delivery of the complex or fusion protein to a cell involves administering the complex or fusion protein comprising a supercharged protein associated with a functional effector protein to a subject in need thereof.

In some embodiments, a functional effector protein (*e.g*., nucleases, transcriptional activators/repressors, recombinases, Cas9 proteins including variants and fusions thereof, *etc*.) by itself may not be able to enter a cell, but is able to enter a cell when associated with a supercharged protein, for example, via a covalent bond or a non-covalent interaction. In some embodiments, a composition is provided that includes a functional effector protein that is covalently bound to a supercharged protein. In some embodiments, the composition includes a functional effector protein fused to a supercharged protein via a peptide bond, for example, via direct fusion or via a peptide linker. In some embodiments, the composition includes a functional effector protein that is bound to a supercharged protein by non-covalent interaction. In some embodiments, a supercharged protein is utilized to allow a functional effector protein to enter a cell. In some embodiments, the functional effector protein delivered to the cell associated with a supercharged protein is separated from the supercharged protein after delivery to the cell, for example, by cleavage of a linker peptide by a cellular protease (*e.g*., an endosomal protease) or by dissociation of the functional effector protein from the supercharged protein in a specific cellular microenvironment, for example, in the endosome. In some embodiments, functional effector proteins delivered to a cell by a system or method provided by this disclosure have therapeutic activity.

In some embodiments, a functional effector protein (*e.g*., nucleases, transcriptional activators/repressors, recombinases, Cas9 proteins including variants and fusions thereof, *etc*.) is delivered to a cell *in vivo, ex vivo,* or *in vitro* by a system, composition, or method provided herein. In some embodiments, a functional effector protein is a protein able to carry out a biological function within the target cell, for example, an enzyme able to bind its substrate and to catalyze an enzymatic reaction in the target cell, *e.g.,* a nuclease able to bind and cut a nucleic acid molecule within a target cell, or a transcription factor able to interact with the genome of a target cell and to activate or inhibit transcription of a target gene in the cell.

In some embodiments, a method for generating a fusion of a functional effector protein and a supercharged protein includes the generation of an expression nucleic acid construct containing the coding sequences of the functional protein and the supercharged protein, as well as, optionally, a peptide linker, in frame; the expression of such a recombinant fusion protein in a prokaryotic or eukaryotic cell in culture, the extraction and purification of the fusion protein of the fusion protein. In some embodiments, a nucleic acid construct is generated in the form of an expression vector, for example, a vector suitable for propagation in a bacterial host and for expression in a prokaryotic or eukaryotic cell.

In some embodiments, a vector suitable for fusion protein expression is generated by cloning of a nucleotide sequence coding for a functional effector protein to be delivered into a cloning vector including a nucleotide sequence coding for a supercharged protein under the control of a eukaryotic and/or a prokaryotic promoter, by a cloning approach that results in both coding sequences being in frame with each other. In some embodiments, the cloning vector includes a nucleotide sequence coding for a peptide linker between a nucleotide sequence coding for a supercharged protein and a restriction site useful for inserting a nucleotide sequence coding for a protein in frame with the linker and the supercharged protein. In some embodiments, the cloning vector further includes an additional sequence enhancing expression of a fusion protein in a prokaryotic or eukaryotic cell or facilitating purification of expressed fusion proteins from such cells, for example, a sequence stabilizing a transcript encoding the fusion protein, such as a poly-A signal, a spliceable intron, a sequence encoding an in-frame peptide or protein domain tag (*e.g.,* an Arg-tag, calmodulin-binding peptide tag, cellulose-binding domain tag, DsbA tag, c-myc-tag, glutathione S-transferase tag, FLAG-tag, HAT-tag, His-tag, maltose-binding protein tag, NusA tag, S-tag, SBP-tag, Strep-tag, or thioredoxin tag), or a selection marker or reporter cassette allowing for identification of cells harboring and expressing the expression construct and/or quantifying the level of expression in such cells. Methods for cloning and expressing fusion proteins are well known to those in the art, see, for example Sambrook et al., Molecular Cloning: A Laboratory Manual, Volume 1-3, CSHL Press (1989); Gellissen et al., Production of recombinant proteins, Wiley-VCH, 2005.

In some embodiments, the functional effector protein is associated with a supercharged GFP, for example, +36 GFP or -30 GFP, for delivery to a target cell. The benefit of endosomal disruption in the delivery of macromolecules by supercharged proteins has been previously demonstrated (Wadia et al., Nat. Med. 10, 310-315, 2004) and in some embodiments, additional steps to effect enhanced endosomal escape, as provided herein or known in the art, are performed. Highly efficient protein internalization, when coupled with effective endosomal release, has the potential to minimize the requisite doses of exogenous protein agents, enhancing their potential as research tools and leads for therapeutic development.

In some embodiments, a composition comprising a functional effector protein associated with a supercharged protein is administered to a target cell after isolation and/or purification. Protein isolation methods and technologies are well known to those of skill in the art and include, for example, affinity chromatography or immunoprecipitation. The methods suitable for isolating and/or purifying a specific functional effector proteins, supercharged proteins, and/or fusion proteins will depend on the nature of the respective protein. For example, a His-tagged fusion protein can readily be isolated and purified via Ni or Co ion chromatography, while fusion proteins tagged with other peptides or domains or untagged fusion proteins can be purified by other well established methods.

Functional effector proteins suitable for delivery to a target cell *in vivo, ex vivo,* or *in vitro,* by a system or method provided herein will be apparent to those of skill in the art and include, for example, DNA-binding proteins, such as transcription factors and nucleases, as well as Cas9 proteins (including variants and fusions thereof).

In some embodiments, a method, composition, or system provided herein is used to deliver a therapeutic functional effector protein to a cell. Examples of therapeutic proteins include, but are not limited to, nucleases and Cas9 proteins (including variants and fusions thereof) targeting a genomic allele associated with a disease or disorder, and transcription factors activating a beneficial gene or repressing a pathogenic gene.

In some embodiments, Cas9 is fused to a supercharged protein for delivery to a cell. In some embodiments, the supercharged protein is positively charged. In some embodiments, the supercharged protein fused to Cas9 is (+36)GFP. In some embodiments, the fusion of Cas9 and (+36)GFP comprises the amino acid sequence of SEQ ID NO:30 (*e.g.,* with or without a nuclear localization signal (NLS) and with or without a 6xHis tag), or comprises an amino acid sequence that is at least 80%, at least 85%, at least 90%, at least 95%, at least 98%, or at least 99% identical to the amino acid sequence of SEQ ID NO:30 (*e.g.,* with or without a nuclear localization signal (NLS) and with or without a 6xHis tag). In some embodiments, the supercharged protein fused to Cas9 is (-30)GFP. In some embodiments, the fusion of Cas9 and (-30)GFP comprises the amino acid sequence of SEQ ID NO:31 (*e.g.,* with or without a nuclear localization signal (NLS) and with or without a 6xHis tag), or comprises an amino acid sequence that is at least 80%, at least 85%, at least 90%, at least 95%, at least 98%, or at least 99% identical to the amino acid sequence of SEQ ID NO:31 (*e.g.,* with or without a nuclear localization signal (NLS) and with or without a 6xHis tag).

### Cas9-6xHis:

MDKKYSIGLAIGTNSVGWAVITDEYKVPSKKFKVLGNTDRHSIKKNLIGALLFDSGETAEATRLKRTARRR
YTRRKNRICYLQEIFSNEMAKVDDSFFHRLEESFLVEEDKKHERHPIFGNIVDEVAYHEKYPTIYHLRKKLV
DSTDKADLRLIYLALAHMIKFRGHFLIEGDLNPDNSDVDKLFIQLVQTYNQLFEENPINASGVDAKAILSAR
LSKSRRLENLIAQLPGEKKNGLFGNLIALSLGLTPNFKSNFDLAEDAKLQLSKDTYDDDLDNLLAQIGDQY
ADLFLAAKNLSDAILLSDILRVNTEITKAPLSASMIKRYDEHHQDLTLLKALVRQQLPEKYKEIFFDQSKNG

YAGYIDGGASQEEFYKFIKPILEKMDGTEELLVKLNREDLLRKQRTFDNGSIPHQIHLGELHAILRRQEDFYP
FLKDNREKIEKILTFRIPYYVGPLARGNSRFAWMTRKSEETITPWNFEEVVDKGASAQSFIERMTNFDKNLP
NEKVLPKHSLLYEYFTVYNELTKVKYVTEGMRKPAFLSGEQKKAIVDLLFKTNRKVTVKQLKEDYFKKIE
CFDSVEISGVEDRFNASLGTYHDLLKIIKDKDFLDNEENEDILEDIVLTLTLFEDREMIEERLKTYAHLFDDK
VMKQLKRRRYTGWGRLSRKLINGIRDKQSGKTILDFLKSDGFANRNFMQLIHDDSLTFKEDIQKAQVSGQG
DSLHEHIANLAGSPAIKKGILQTVKVVDELVKVMGRHKPENIVIEMARENQTTQKGQKNSRERMKRIEEGI
KELGSQILKEHPVENTQLQNEKLYLYYLQNGRDMYVDQELDINRLSDYDVDHIVPQSFLKDDSIDNKVLTR
SDKNRGKSDNVPSEEVVKKMKNYWRQLLNAKLITQRKFDNLTKAERGGLSELDKAGFIKRQLVETRQITK
HVAQILDSRMNTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDPKKYGGFDSPTVAYS
VLVVAKVEKGKSKKLKSVKELLGITIMERSSFEKNPIDFLEAKGYKEVKKDLIIKLPKYSLFELENGRKRML
ASAGELQKGNELALPSKYVNFLYLASHYEKLKGSPEDNEQKQLFVEQHKHYLDEIIEQISEFSKRVILADAN
LDKVLSAYNKHRDKPIREQAENIIHLFTLTNLGAPAAFKYFDTTIDRKRYTSTKEVLDATLIHQSITGLYETRI
DLSQLGGDHHHHHH (SEQ ID NO: 260)

### (+36)dGFP-NLS-Cas9-6xHis (Y67S):

MGASKGERLFRGKVPILVELKGDVNGHKFSVRGKGKGDATRGKLTLKFICTTGKLPVPWPTLVTTLTSGV
QCFSRYPKHMKRHDFFKSAMPKGYVQERTISFKKDGKYKTRAEVKFEGRTLVNRIKLKGRDFKEKGNILG
HKLRYNFNSHKVYITADKRKNGIKAKFKIRHNVKDGSVQLADHYQQNTPIGRGPVLLPRNHYLSTRSKLS
KDPKEKRDHMVLLEFVTAAGIKHGRDERYKTGGSGGSGGSGGSGGSGGSGGSGGSGGTALALPKKKRKVM
DKKYSIGLDIGTNSVGWAVITDEYKVPSKKFKVLGNTDRHSIKKNLIGALLFDSGETAEATRLKRTARRR
YTRRKNRICYLQEIFSNEMAKVDDSFFHRLEESFLVEEDKKHERHPIFGNIVDEVAYHEKYPTIYHLRKK
LVDSTDKADLRLIYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKDTYDDDLDNLLAQ
IGDQYADLFLAAKNLSDAILLSDILRVNTEITKAPLSASMIKRYDEHHQDLTLLKALVRQQLPEKYKEIF
FDQSKNGYAGYIDGGASQEEFYKFIKPILEKMDGTEELLVKLNREDLLRKQRTFDNGSIPHQIHLGELHA
ILRRQEDFYPFLKDNREKIEKILTFRIPYYVGPLARGNSRFAWMTRKSEETITPWNFEEVVDKGASAQSF
IERMTNFDKNLPNEKVLPKHSLLYEYFTVYNELTKVKYVTEGMRKPAFLSGEQKKAIVDLLFKTNRKVTV
KQLKEDYFKKIECFDSVEISGVEDRFNASLGTYHDLLKIIKDKDFLDNEENEDILEDIVLTLTLFEDREM
IEERLKTYAHLFDDKVMKQLKRRRYTGWGRLSRKLINGIRDKQSGKTILDFLKSDGFANRNFMQLIHDDS
LTFKEDIQKAQVSGQGDSLHEHIANLAGSPAIKKGILQTVKVVDELVKVMGRHKPENIVIEMARENQTTQ
KGQKNSRERMKRIEEGIKELGSQILKEHPVENTQLQNEKLYLYYLQNGRDMYVDQELDINRLSDYDVDHI
VPQSFLKDDSIDNKVLTRSDKNRGKSDNVPSEEVVKKMKNYWRQLLNAKLITQRKFDNLTKAERGGLSEL
DKAGFIKRQLVETRQITKHVAQILDSRMNTKYDENDKLIREVKVITLKSKLVSDFRKDFQFYKVREINNY
HHAHDAYLNAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILPKRNSDKLIA
RKKDWDPKKYGGFDSPTVAYSVLVVAKVEKGKSKKLKSVKELLGITIMERSSFEKNPIDFLEAKGYKEVK
KDLIIKLPKYSLFELENGRKRMLASAGELQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPAAFKYFDTTI
DRKRYTSTKEVLDATLIHQSITGLYETRIDLSQLGGDHHHHHH (SEQ ID NO:30)

ATGGGTGCTAGCAAAGGTGAACGTCTGTTTCGTGGTAAAGTACCGATCTTAGTGGAATTAAAGGGCGACGTGAACGG
TCATAAATTTAGCGTGCGCGGCAAAGGCAAAGGTGACGCTACCCGTGGTAAATTGACCCTGAAGTTTATTTGCACAA
CAGGCAAATTACCCGTTCCGTGGCCCACCTTAGTGACCACCCTGACCTCCGGCGTTCAGTGCTTCAGTCGTTACCCT
AAACATATGAAACGTCACGATTTTTTCAAATCAGCCATGCCTAAAGGATATGTTCAAGAGCGTACAATCAGCTTCAA
GAAGGATGGCAAATATAAAACGCGTGCGGAAGTGAAATTTGAAGGCCGCACATTAGTAAATCGTATCAAACTGAAAG
GTCGTGACTTCAAAGAAAAAGGCAACATTTTAGGCCATAAACTGCGTTATAACTTTAATTCTCATAAGGTGTATATT
ACGGCCGATAAACGCAAGAATGGTATCAAGGCAAAATTCAAAATTCGCCATAACGTGAAAGACGGCAGCGTTCAATT

AGCGGATCATTATCAACAAAACACGCCGATTGGTCGCGGGCCTGTACTGTTACCTCGCAACCACTACCTGAGCACCC
GTTCTAAACTGAGCAAAGATCCGAAAGAAAAACGCGATCACATGGTTCTGTTAGAATTCGTGACCGCTGCAGGCATT
AAGCACGGACGCGACGAACGCTACAAGACCGGTGGTAGCGGTGGTTCTGGTGGTTCTGGTGGTAGCGGCGGTAGCGG
TGGTAGCGGTGGTAGCGGTGGCAGCGGCGGTACCGCGCTCGCGCTGCCCAAGAAGAAGAGGAAGGTGATGGATAAGA
AATACTCAATAGGCTTAGATATCGGCACAAATAGCGTCGGATGGGCGGTGATCACTGATGAATATAAGGTTCCGTCT
AAAAAGTTCAAGGTTCTGGGAAATACAGACCGCCACAGTATCAAAAAAAATCTTATAGGGGCTCTTTTATTTGACAG
TGGAGAGACAGCGGAAGCGACTCGTCTCAAACGGACAGCTCGTAGAAGGTATACACGTCGGAAGAATCGTATTTGTT
ATCTACAGGAGATTTTTTCAAATGAGATGGCGAAAGTAGATGATAGTTTCTTTCATCGACTTGAAGAGTCTTTTTTG
GTGGAAGAAGACAAGAAGCATGAACGTCATCCTATTTTTGGAAATATAGTAGATGAAGTTGCTTATCATGAGAAATA
TCCAACTATCTATCATCTGCGAAAAAAATTGGTAGATTCTACTGATAAAGCGGATTTGCGCTTAATCTATTTGGCCT
TAGCGCATATGATTAAGTTTCGTGGTCATTTTTTGATTGAGGGAGATTTAAATCCTGATAATAGTGATGTGGACAAA
CTATTTATCCAGTTGGTACAAACCTACAATCAATTATTTGAAGAAAACCCTATTAACGCAAGTGGAGTAGATGCTAA
AGCGATTCTTTCTGCACGATTGAGTAAATCAAGACGATTAGAAAATCTCATTGCTCAGCTCCCCGGTGAGAAGAAAA
ATGGCTTATTTGGGAATCTCATTGCTTTGTCATTGGGTTTGACCCCTAATTTTAAATCAAATTTTGATTTGGCAGAA
GATGCTAAATTACAGCTTTCAAAAGATACTTACGATGATGATTTAGATAATTTATTGGCGCAAATTGGAGATCAATA
TGCTGATTTGTTTTTGGCAGCTAAGAATTTATCAGATGCTATTTTACTTTCAGATATCCTAAGAGTAAATACTGAAA
TAACTAAGGCTCCCCTATCAGCTTCAATGATTAAACGCTACGATGAACATCATCAAGACTTGACTCTTTTAAAAGCT
TTAGTTCGACAACAACTTCCAGAAAAGTATAAAGAAATCTTTTTTGATCAATCAAAAAACGGATATGCAGGTTATAT
TGATGGGGGAGCTAGCCAAGAAGAATTTTATAAATTTATCAAACCAATTTTAGAAAAAATGGATGGTACTGAGGAAT
TATTGGTGAAACTAAATCGTGAAGATTTGCTGCGCAAGCAACGGACCTTTGACAACGGCTCTATTCCCCATCAAATT
CACTTGGGTGAGCTGCATGCTATTTTGAGAAGACAAGAAGACTTTTATCCATTTTTAAAAGACAATCGTGAGAAGAT
TGAAAAAATCTTGACTTTTCGAATTCCTTATTATGTTGGTCCATTGGCGCGTGGCAATAGTCGTTTTGCATGGATGA
CTCGGAAGTCTGAAGAAACAATTACCCCATGGAATTTTGAAGAAGTTGTCGATAAAGGTGCTTCAGCTCAATCATTT
ATTGAACGCATGACAAACTTTGATAAAAATCTTCCAAATGAAAAAGTACTACCAAAACATAGTTTGCTTTATGAGTA
TTTTACGGTTTATAACGAATTGACAAAGGTCAAATATGTTACTGAAGGAATGCGAAAACCAGCATTTCTTTCAGGTG
AACAGAAGAAAGCCATTGTTGATTTACTCTTCAAAACAAATCGAAAAGTAACCGTTAAGCAATTAAAAGAAGATTAT
TTCAAAAAAATAGAATGTTTTGATAGTGTTGAAATTTCAGGAGTTGAAGATAGATTTAATGCTTCATTAGGTACCTA
CCATGATTTGCTAAAAATTATTAAAGATAAAGATTTTTTGGATAATGAAGAAAATGAAGATATCTTAGAGGATATTG
TTTTAACATTGACCTTATTTGAAGATAGGGAGATGATTGAGGAAAGACTTAAAACATATGCTCACCTCTTTGATGAT
AAGGTGATGAAACAGCTTAAACGTCGCCGTTATACTGGTTGGGGACGTTTGTCTCGAAAATTGATTAATGGTATTAG
GGATAAGCAATCTGGCAAAACAATATTAGATTTTTTGAAATCAGATGGTTTTGCCAATCGCAATTTTATGCAGCTGA
TCCATGATGATAGTTTGACATTTAAAGAAGACATTCAAAAAGCACAAGTGTCTGGACAAGGCGATAGTTTACATGAA
CATATTGCAAATTTAGCTGGTAGCCCTGCTATTAAAAAAGGTATTTTACAGACTGTAAAAGTTGTTGATGAATTGGT
CAAAGTAATGGGGCGGCATAAGCCAGAAAATATCGTTATTGAAATGGCACGTGAAAATCAGACAACTCAAAAGGGCC
AGAAAAATTCGCGAGAGCGTATGAAACGAATCGAAGAAGGTATCAAAGAATTAGGAAGTCAGATTCTTAAAGAGCAT
CCTGTTGAAAATACTCAATTGCAAAATGAAAAGCTCTATCTCTATTATCTCCAAAATGGAAGAGACATGTATGTGGA
CCAAGAATTAGATATTAATCGTTTAAGTGATTATGATGTCGATCACATTGTTCCACAAAGTTTCCTTAAAGACGATT
CAATAGACAATAAGGTCTTAACGCGTTCTGATAAAAATCGTGGTAAATCGGATAACGTTCCAAGTGAAGAAGTAGTC
AAAAAGATGAAAAACTATTGGAGACAACTTCTAAACGCCAAGTTAATCACTCAACGTAAGTTTGATAATTTAACGAA
AGCTGAACGTGGAGGTTTGAGTGAACTTGATAAAGCTGGTTTTATCAAACGCCAATTGGTTGAAACTCGCCAAATCA
CTAAGCATGTGGCACAAATTTTGGATAGTCGCATGAATACTAAATACGATGAAAATGATAAACTTATTCGAGAGGTT
AAAGTGATTACCTTAAAATCTAAATTAGTTTCTGACTTCCGAAAAGATTTCCAATTCTATAAAGTACGTGAGATTAA
CAATTACCATCATGCCCATGATGCGTATCTAAATGCCGTCGTTGGAACTGCTTTGATTAAGAAATATCCAAAACTTG
AATCGGAGTTTGTCTATGGTGATTATAAAGTTTATGATGTTCGTAAAATGATTGCTAAGTCTGAGCAAGAAATAGGC
AAAGCAACCGCAAAATATTTCTTTTACTCTAATATCATGAACTTCTTCAAAACAGAAATTACACTTGCAAATGGAGA
GATTCGCAAACGCCCTCTAATCGAAACTAATGGGGAAACTGGAGAAATTGTCTGGGATAAAGGGCGAGATTTTGCCA
CAGTGCGCAAAGTATTGTCCATGCCCCAAGTCAATATTGTCAAGAAAACAGAAGTACAGACAGGCGGATTCTCCAAG
GAGTCAATTTTACCAAAAAGAAATTCGGACAAGCTTATTGCTCGTAAAAAAGACTGGGATCCAAAAAAATATGGTGG
TTTTGATAGTCCAACGGTAGCTTATTCAGTCCTAGTGGTTGCTAAGGTGGAAAAAGGGAAATCGAAGAAGTTAAAAT

CCGTTAAAGAGTTACTAGGGATCACAATTATGGAAAGAAGTTCCTTTGAAAAAAATCCGATTGACTTTTTAGAAGCT
AAAGGATATAAGGAAGTTAAAAAAGACTTAATCATTAAACTACCTAAATATAGTCTTTTTGAGTTAGAAAACGGTCG
TAAACGGATGCTGGCTAGTGCCGGAGAATTACAAAAAGGAAATGAGCTGGCTCTGCCAAGCAAATATGTGAATTTTT
TATATTTAGCTAGTCATTATGAAAAGTTGAAGGGTAGTCCAGAAGATAACGAACAAAAACAATTGTTTGTGGAGCAG
CATAAGCATTATTTAGATGAGATTATTGAGCAAATCAGTGAATTTTCTAAGCGTGTTATTTTAGCAGATGCCAATTT
AGATAAAGTTCTTAGTGCATATAACAAACATAGAGACAAACCAATACGTGAACAAGCAGAAAATATTATTCATTTAT
TTACGTTGACGAATCTTGGAGCTCCCGCTGCTTTTAAATATTTTGATACAACAATTGATCGTAAACGATATACGTCT
ACAAAAGAAGTTTTAGATGCCACTCTTATCCATCAATCCATCACTGGTCTTTATGAAACACGCATTGATTTGAGTCA
GCTAGGAGGTGACCATCACCACCACCATCAC (SEQ ID NO: 261)

### (-30)dGFP-NLS-Cas9-6xHis (Y67S):

MGASKGEELFDGVVPILVELDGDVNGHEFSVRGEGEGDATEGELTLKFICTTGELPVPWPTLVTTLTSGV
QCFSDYPDHMDQHDFFKSAMPEGYVQERTISFKDDGTYKTRAEVKFEGDTLVNRIELKGIDFKEDGNILG
HKLEYNFNSHDVYITADKQENGIKAEFEIRHNVEDGSVQLADHYQQNTPIGDGPVLLPDDHYLSTESALS
KDPNEDRDHMVLLEFVTAAGIDHGMDELYKTGGSGGSGGSGGSGGSGGSGGSGGTALALPKKKRKVM
DKKYSIGLDIGTNSVGWAVITDEYKVPSKKFKVLGNTDRHSIKKNLIGALLFDSGETAEATRLKRTARRR
YTRRKNRICYLQEIFSNEMAKVDDSFFHRLEESFLVEEDKKHERHPIFGNIVDEVAYHEKYPTIYHLRKK
LVDSTDKADLRLIYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKDTYDDDLDNLLAQ
IGDQYADLFLAAKNLSDAILLSDILRVNTEITKAPLSASMIKRYDEHHQDLTLLKALVRQQLPEKYKEIF
FDQSKNGYAGYIDGGASQEEFYKFIKPILEKMDGTEELLVKLNREDLLRKQRTFDNGSIPHQIHLGELHA
ILRRQEDFYPFLKDNREKIEKILTFRIPYYVGPLARGNSRFAWMTRKSEETITPWNFEEVVDKGASAQSF
IERMTNFDKNLPNEKVLPKHSLLYEYFTVYNELTKVKYVTEGMRKPAFLSGEQKKAIVDLLFKTNRKVTV
KQLKEDYFKKIECFDSVEISGVEDRFNASLGTYHDLLKIIKDKDFLDNEENEDILEDIVLTLTLFEDREM
IEERLKTYAHLFDDKVMKQLKRRRYTGWGRLSRKLINGIRDKQSGKTILDFLKSDGFANRNFMQLIHDDS
LTFKEDIQKAQVSGQGDSLHEHIANLAGSPAIKKGILQTVKVVDELVKVMGRHKPENIVIEMARENQTTQ
KGQKNSRERMKRIEEGIKELGSQILKEHPVENTQLQNEKLYLYYLQNGRDMYVDQELDINRLSDYDVDHI
VPQSFLKDDSIDNKVLTRSDKNRGKSDNVPSEEVVKKMKNYWRQLLNAKLITQRKFDNLTKAERGGLSEL
DKAGFIKRQLVETRQITKHVAQILDSRMNTKYDENDKLIREVKVITLKSKLVSDFRKDFQFYKVREINNY
HHAHDAYLNAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILPKRNSDKLIA
RKKDWDPKKYGGFDSPTVAYSVLVVAKVEKGKSKKLKSVKELLGITIMERSSFEKNPIDFLEAKGYKEVK
KDLIIKLPKYSLFELENGRKRMLASAGELQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPAAFKYFDTTI
DRKRYTSTKEVLDATLIHQSITGLYETRIDLSQLGGDHHHHHH (SEQ ID NO:31)

ATGGGTGCTAGCAAAGGTGAAGAGCTGTTTGACGGTGTAGTACCGATCTTAGTGGAATTAGACGGCGACGTGAACGG
TCACGAATTTAGCGTGCGCGGCGAGGGCGAAGGTGACGCTACCGAGGGTGAATTGACCCTGAAGTTTATTTGCACAA
CAGGCGAATTACCCGTTCCGTGGCCCACCTTAGTGACCACCCTGACCTCCGGCGTTCAGTGCTTCAGTGATTACCCA
GATCATATGGATCAACACGATTTTTTCAAATCAGCCATGCCTGAAGGATATGTTCAAGAGCGTACAATCAGCTTCAA
GGACGATGGCACCTATAAAACGCGTGCGGAAGTGAAATTTGAAGGCGACACATTAGTAAACCGTATCGAACTGAAAG
GTATCGACTTCAAAGAAGACGGCAACATTTTAGGCCATAAGCTGGAATATAACTTTAATTCTCATGACGTGTATATT
ACGGCCGATAAACAGGAAAACGGTATCAAGGCAGAATTTGAAATTCGCCATAACGTGGAGGACGGCAGCGTTCAATT
AGCGGATCATTATCAACAAAACACGCCGATTGGTGATGGGCCTGTACTGTTACCTGACGATCACTACCTGAGCACGG
AGTCAGCCCTGAGCAAAGATCCGAACGAAGACCGCGATCACATGGTTCTGTTAGAATTCGTGACCGCTGCAGGCATT
GATCATGGAATGGACGAGCTGTACAAGACCGGTGGTAGCGGTGGTTCTGGTGGTTCTGGTGGTAGCGGCGGTAGCGG
TGGTAGCGGTGGTAGCGGTGGCAGCGGCGGTACCGCGCTCGCGCTGCCCAAGAAGAAGAGGAAGGTGATGGATAAGA
AATACTCAATAGGCTTAGATATCGGCACAAATAGCGTCGGATGGGCGGTGATCACTGATGAATATAAGGTTCCGTCT
AAAAAGTTCAAGGTTCTGGGAAATACAGACCGCCACAGTATCAAAAAAAATCTTATAGGGGCTCTTTTATTTGACAG
TGGAGAGACAGCGGAAGCGACTCGTCTCAAACGGACAGCTCGTAGAAGGTATACACGTCGGAAGAATCGTATTTGTT
ATCTACAGGAGATTTTTTCAAATGAGATGGCGAAAGTAGATGATAGTTTCTTTCATCGACTTGAAGAGTCTTTTTG

GTGGAAGAAGACAAGAAGCATGAACGTCATCCTATTTTTGGAAATATAGTAGATGAAGTTGCTTATCATGAGAAATA
TCCAACTATCTATCATCTGCGAAAAAAATTGGTAGATTCTACTGATAAAGCGGATTTGCGCTTAATCTATTTGGCCT
TAGCGCATATGATTAAGTTTCGTGGTCATTTTTTGATTGAGGGAGATTTAAATCCTGATAATAGTGATGTGGACAAA
CTATTTATCCAGTTGGTACAAACCTACAATCAATTATTTGAAGAAAACCCTATTAACGCAAGTGGAGTAGATGCTAA
AGCGATTCTTTCTGCACGATTGAGTAAATCAAGACGATTAGAAAATCTCATTGCTCAGCTCCCCGGTGAGAAGAAAA
ATGGCTTATTTGGGAATCTCATTGCTTTGTCATTGGGTTTGACCCCTAATTTTAAATCAAATTTTGATTTGGCAGAA
GATGCTAAATTACAGCTTTCAAAAGATACTTACGATGATGATTTAGATAATTTATTGGCGCAAATTGGAGATCAATA
TGCTGATTTGTTTTTGGCAGCTAAGAATTTATCAGATGCTATTTTACTTTCAGATATCCTAAGAGTAAATACTGAAA
TAACTAAGGCTCCCCTATCAGCTTCAATGATTAAACGCTACGATGAACATCATCAAGACTTGACTCTTTTAAAAGCT
TTAGTTCGACAACAACTTCCAGAAAAGTATAAAGAAATCTTTTTTGATCAATCAAAAAACGGATATGCAGGTTATAT
TGATGGGGGAGCTAGCCAAGAAGAATTTTATAAATTTATCAAACCAATTTTAGAAAAAATGGATGGTACTGAGGAAT
TATTGGTGAAACTAAATCGTGAAGATTTGCTGCGCAAGCAACGGACCTTTGACAACGGCTCTATTCCCCATCAAATT
CACTTGGGTGAGCTGCATGCTATTTTGAGAAGACAAGAAGACTTTTATCCATTTTTAAAAGACAATCGTGAGAAGAT
TGAAAAAATCTTGACTTTTCGAATTCCTTATTATGTTGGTCCATTGGCGCGTGGCAATAGTCGTTTTGCATGGATGA
CTCGGAAGTCTGAAGAAACAATTACCCCATGGAATTTTGAAGAAGTTGTCGATAAAGGTGCTTCAGCTCAATCATTT
ATTGAACGCATGACAAACTTTGATAAAAATCTTCCAAATGAAAAAGTACTACCAAAACATAGTTTGCTTTATGAGTA
TTTTACGGTTTATAACGAATTGACAAAGGTCAAATATGTTACTGAAGGAATGCGAAAACCAGCATTTCTTTCAGGTG
AACAGAAGAAAGCCATTGTTGATTTACTCTTCAAAACAAATCGAAAAGTAACCGTTAAGCAATTAAAAGAAGATTAT
TTCAAAAAAATAGAATGTTTTGATAGTGTTGAAATTTCAGGAGTTGAAGATAGATTTAATGCTTCATTAGGTACCTA
CCATGATTTGCTAAAAATTATTAAAGATAAAGATTTTTTGGATAATGAAGAAAATGAAGATATCTTAGAGGATATTG
TTTTAACATTGACCTTATTTGAAGATAGGGAGATGATTGAGGAAAGACTTAAAACATATGCTCACCTCTTTGATGAT
AAGGTGATGAAACAGCTTAAACGTCGCCGTTATACTGGTTGGGGACGTTTGTCTCGAAAATTGATTAATGGTATTAG
GGATAAGCAATCTGGCAAAACAATATTAGATTTTTTGAAATCAGATGGTTTTGCCAATCGCAATTTTATGCAGCTGA
TCCATGATGATAGTTTGACATTTAAAGAAGACATTCAAAAAGCACAAGTGTCTGGACAAGGCGATAGTTTACATGAA
CATATTGCAAATTTAGCTGGTAGCCCTGCTATTAAAAAAGGTATTTTACAGACTGTAAAAGTTGTTGATGAATTGGT
CAAAGTAATGGGGCGGCATAAGCCAGAAAATATCGTTATTGAAATGGCACGTGAAAATCAGACAACTCAAAAGGGCC
AGAAAAATTCGCGAGAGCGTATGAAACGAATCGAAGAAGGTATCAAAGAATTAGGAAGTCAGATTCTTAAAGAGCAT
CCTGTTGAAAATACTCAATTGCAAAATGAAAAGCTCTATCTCTATTATCTCCAAAATGGAAGAGACATGTATGTGGA
CCAAGAATTAGATATTAATCGTTTAAGTGATTATGATGTCGATCACATTGTTCCACAAAGTTTCCTTAAAGACGATT
CAATAGACAATAAGGTCTTAACGCGTTCTGATAAAAATCGTGGTAAATCGGATAACGTTCCAAGTGAAGAAGTAGTC
AAAAAGATGAAAAACTATTGGAGACAACTTCTAAACGCCAAGTTAATCACTCAACGTAAGTTTGATAATTTAACGAA
AGCTGAACGTGGAGGTTTGAGTGAACTTGATAAAGCTGGTTTTATCAAACGCCAATTGGTTGAAACTCGCCAAATCA
CTAAGCATGTGGCACAAATTTTGGATAGTCGCATGAATACTAAATACGATGAAAATGATAAACTTATTCGAGAGGTT
AAAGTGATTACCTTAAAATCTAAATTAGTTTCTGACTTCCGAAAAGATTTCCAATTCTATAAAGTACGTGAGATTAA
CAATTACCATCATGCCCATGATGCGTATCTAAATGCCGTCGTTGGAACTGCTTTGATTAAGAAATATCCAAAACTTG
AATCGGAGTTTGTCTATGGTGATTATAAAGTTTATGATGTTCGTAAAATGATTGCTAAGTCTGAGCAAGAAATAGGC
AAAGCAACCGCAAAATATTTCTTTTACTCTAATATCATGAACTTCTTCAAAACAGAAATTACACTTGCAAATGGAGA
GATTCGCAAACGCCCTCTAATCGAAACTAATGGGGAAACTGGAGAAATTGTCTGGGATAAAGGGCGAGATTTTGCCA
CAGTGCGCAAAGTATTGTCCATGCCCCAAGTCAATATTGTCAAGAAAACAGAAGTACAGACAGGCGGATTCTCCAAG
GAGTCAATTTTACCAAAAAGAAATTCGGACAAGCTTATTGCTCGTAAAAAAGACTGGGATCCAAAAAAATATGGTGG
TTTTGATAGTCCAACGGTAGCTTATTCAGTCCTAGTGGTTGCTAAGGTGGAAAAAGGGAAATCGAAGAAGTTAAAAT
CCGTTAAAGAGTTACTAGGGATCACAATTATGGAAAGAAGTTCCTTTGAAAAAAATCCGATTGACTTTTTAGAAGCT
AAAGGATATAAGGAAGTTAAAAAAGACTTAATCATTAAACTACCTAAATATAGTCTTTTTGAGTTAGAAAACGGTCG
TAAACGGATGCTGGCTAGTGCCGGAGAATTACAAAAAGGAAATGAGCTGGCTCTGCCAAGCAAATATGTGAATTTTT
TATATTTAGCTAGTCATTATGAAAAGTTGAAGGGTAGTCCAGAAGATAACGAACAAAAACAATTGTTTGTGGAGCAG
CATAAGCATTATTTAGATGAGATTATTGAGCAAATCAGTGAATTTTCTAAGCGTGTTATTTTAGCAGATGCCAATTT
AGATAAAGTTCTTAGTGCATATAACAAACATAGAGACAAACCAATACGTGAACAAGCAGAAAATATTATTCATTTAT
TTACGTTGACGAATCTTGGAGCTCCCGCTGCTTTTAAATATTTTGATACAACAATTGATCGTAAACGATATACGTCT
ACAAAAGAAGTTTTAGATGCCACTCTTATCCATCAATCCATCACTGGTCTTTATGAAACACGCATTGATTTGAGTCA
GCTAGGAGGTGACCATCACCACCACCATCAC (SEQ ID NO: 262)

### Compositions of functional effector proteins and cationic lipids

Certain aspects of the disclosure relate to the use of cationic lipids for the delivery of effector proteins (*e.g.*, nucleases, transcriptional activators/repressors, recombinases, Cas9 proteins including variants and fusions thereof, *etc*.), for example as opposed to delivering "naked" protein preparations. Surprisingly, existing liposomal delivery reagents that have been engineered for the delivery of nucleic acids such as DNA and RNA were found to effectively deliver certain effector proteins (*e.g*., Cas9 proteins including variants and fusions thereof) both *in vitro* and *in vivo,* as described herein. Nucleic acid delivery has benefited greatly from the development of liposomal reagents over the past two decades. Cationic liposomal formulations have enabled DNA and RNA transfection to become a routine technique in basic research and have even been used in clinical trials. The lipid bilayer of the liposome protects encapsulated nucleic acids from degradation and can prevent specific neutralization by antibodies that can bind naked preparations of the nucleic acids. Importantly, fusion of the liposome with the endosomal membrane during endosomal maturation can enable highly efficient endosomal escape of cationic lipid-delivered cargo. Other non-cationic, but reversibly ionizable, lipid nanoparticle formulations have enabled efficient encapsulation and delivery of nucleic acids, while avoiding non-specific electrostatic interactions and consequent sequestration. However, proteins are chemically diverse, and therefore unlike highly anionic nucleic acids, liposomal formulations have not been similarly successful for the efficient delivery of proteins. For example, while proteins can be encapsulated non-specifically and delivered by rehydrated lipids *in vitro,* the efficacy of encapsulation is dependent on protein concentration and is generally inefficient, and thus has not seen widespread application. Aspects of the present disclosure relate to the recognition that anionic proteins or protein complexes (including those proteins associated with nucleic acids) may be able to take advantage of the same electrostatics-driven encapsulation used by cationic liposomal reagents for nucleic acid delivery. While few proteins natively possess the density of negative charges found in the phosphate backbone of nucleic acids, translational fusion to, or non-covalent association with, an anionic carrier such as a negatively supercharged protein or a nucleic acid as described herein render the resulting effector protein or protein complex sufficiently anionic to drive efficient encapsulation of such protein cargoes by cationic liposomal reagents.

In some embodiments, association or fusion with an engineered supernegatively charged GFP is capable of driving efficient encapsulation and delivery of proteins into cultured mammalian cells by cationic lipids commonly used to transfect nucleic acids. This approach is effective even at low nanomolar protein concentrations and in the presence of serum, resulting in up to 1,000-fold more efficient functional protein delivery than protein delivery methods that use fusion to cationic peptides or proteins. As shown in the Examples, the efficacy of delivery depends, in some embodiments, on *e.g.,* the theoretical net charge of the fusion tag, and that popular natively anionic peptide tags *e.g*., 3xFLAG and VP64, can likewise enable liposomal protein delivery.

The Examples further show that Cas9 nuclease protein associated with polyanionic guide RNAs (gRNA) can be efficiently delivered in functional form into mammalian cells by these common cationic liposomal formulations because, while not wishing to be bound by any particular theory, it is believed that the gRNA acts as a polyanionic mediator between the otherwise cationic Cas9 protein and the cationic lipids. Delivery of Cas9:gRNA complexes is not only highly efficient (*e.g.,* up to 80% modification from a single treatment) but also results in markedly higher genome modification specificity compared with plasmid transfection, typically resulting in >10-fold higher on-target: off-target modification ratios, presumably due to the transient nature of the delivered Cas9:gRNA activity. In some embodiments, delivery of Cas9:gRNA complexes results in at least a 2-fold, 3-fold, 4-fold, 5-fold, 6-fold, 7-fold, 8-fold, 9-fold, 10-fold, 11-fold, 12-fold, 13-fold, 14-fold, 15-fold, 20-fold or 25-fold or higher on-target: off-target modification ratio. The Examples also demonstrate that this protein delivery approach can be effective *in vivo,* for example by delivering functional Cre recombinase and functional Cas9:gRNA complexes to hair cells in the inner ear of mice.

Accordingly, some aspects of the disclosure provide compositions comprising a Cas9 protein (*e.g.,* as described herein; see *e.g., Cas9 effector proteins* below) and a cationic lipid capable of delivering the Cas9 protein to the interior of a cell. In some embodiments, the Cas9 protein is associated with a gRNA, which *e.g*., provides anionic charge to the complex thereby allowing the Cas9:gRNA complex to be encapsulated by the cationic lipids. In some embodiments, the Cas9 protein need not be associated with a gRNA for effective encapsulation by a cationic lipid, but instead is associated with a negatively supercharged protein, as described herein. In some embodiments where a Cas9 protein is associated with a negatively supercharged protein, the Cas9 protein is also associated with a gRNA. In some embodiments, the Cas9 protein is a wild type Cas9 protein, a fragment of a wild type Cas9 protein, or a variant of a wild type Cas9 protein. In some embodiments, the Cas9 protein comprises a dCas9 domain (*e.g.*, as described herein). In some embodiments, the Cas9 protein is a fusion protein comprising a dCas9 domain (*e.g*., as described herein). In some embodiments, the Cas9 protein is a Cas9 nickase.

In other embodiments, compositions comprising an effector protein (*e.g.*, other than a Cas9 protein) and a cationic lipid are provided which are capable of delivering the effector protein to the interior of a cell (*e.g.,* to the nucleus of the cell). The effector protein is either naturally negatively charged, is modified to have a net overall negative charge, or is associated with a negatively supercharged protein, as described herein. In some embodiments, the effector protein is any effector protein described herein. In some embodiments, the effector protein is a recombinase, *e.g.*, any recombinase described herein. In some embodiments, the recombinase is Cre recombinase. In some embodiments, the Cre recombinase comprises the amino acid sequence of SEQ ID NO:32 (*e.g.,* with or without the 6xHis tag). In some embodiments, the Cre recombinase comprises an amino acid sequence that is at least 80%, at least 85%, at least 90%, at least 95%, at least 98%, or at least 99% identical to the amino acid sequence of SEQ ID NO:32 (*e.g.,* with or without the 6xHis tag). In some embodiments, the Cre recombinase is fused to a supercharged protein (*e.g.,* +36 GFP or -30GFP). In some embodiments, the Cre recombinase fused to a supercharged protein comprises the amino acid sequence of SEQ ID NO:33 (*e.g.,* with or without the 6xHis tag) or SEQ ID NO:34 (*e.g.,* with or without the 6xHis tag), or comprises an amino acid sequence that is at least 80%, at least 85%, at least 90%, at least 95%, at least 98%, or at least 99% identical to the amino acid sequence of SEQ ID NO:33 or SEQ ID NO:34 (*e.g.,* with or without the 6xHis tag). In some embodiments, the effector protein is a TALE protein, (*e.g.,* as described herein including those provided in the Examples). In some embodiments, the TALE protein comprises one or more of a VP64 transcriptional activator domain (*e.g*., SEQ ID NO:35). In some embodiments, the TALE protein with a VP64 transcriptional activator domain further comprises an amino acid sequence selected from the group consisting of SEQ ID NO:36-39 (*e.g.,* with or without the 6xHis tag). In some embodiments, the TALE protein with a VP64 transcriptional activator domain comprises an amino acid sequence that is at least 80%, at least 85%, at least 90%, at least 95%, at least 98%, or at least 99% identical to an amino acid sequence selected from the group consisting of SEQ ID NO:36-39 (*e.g.,* with or without the 6xHis tag). In some embodiments, the TALE effector protein comprises a (-30)GFP domain (*e.g.*, SEQ ID NO:21 or SEQ ID NO:40), a N-terminal region of a TALE domain (*e.g*., SEQ ID NO:41), a variable repeat domain (*e.g.*, an 18.5mer repeat domain as provided in Maeder et al., "Robust, synergistic regulation of human gene expression using TALE activators." Nat. Methods. 2013; 10, 243-245), a C-terminal TALE domain (*e.g.*, SEQ ID NO:42), a VP64 activation domain (*e.g.,* SEQ ID NO:35), and optionally one or more linkers (*e.g.,* GGS(9), SEQ ID NO: 252) between any domain and optionally a sequence tag (*e.g.*, 6xHis, SEQ ID NO:253).

While liposomal delivery of cargo such as DNA and RNA has been known to induce toxicity in targeted cells, it was found that the inventive compositions described herein deliver their cargo both *in vitro* and *in vivo* surprisingly with no or low toxicity. For example, in some embodiments, the compositions comprising a Cas9 protein or other effector proteins described herein exhibit low toxicity when administered to a population of cells (*e.g., in vitro* or *in vivo*). In some embodiments, at least 60%, at least 65%, at least 70%, at least 75%, at least 80%, at least 85%, at least 90%, at least 95%, or at least 99% of the cells in a population are viable following administration of an inventive composition comprising a Cas9 protein or other effector protein and cationic lipids. Methods for assessing the toxicity of a composition when administered to a population of cells are well known in the art and include those described in the Examples.

### Cre-6xHis (6xHis tag underlined):

MASNLLTVHQNLPALPVDATSDEVRKNLMDMFRDRQAFSEHTWKMLLSVCRSWAAWCKLNNRKWFPAEPE
DVRDYLLYLQARGLAVKTIQQHLGQLNMLHRRSGLPRPSDSNAVSLVMRRIRKENVDAGERAKQALAFER
TDFDQVRSLMENSDRCQDIRNLAFLGIAYNTLLRIAEIARIRVKDISRTDGGRMLIHIGRTKTLVSTAGV
EKALSLGVTKLVERWISVSGVADDPNNYLFCRVRKNGVAAPSATSQLSTRALEGIFEATHRLIYGAKDDS
GQRYLAWSGHSARVGAARDMARAGVSIPEIMQAGGWTNVNIVMNYIRNLDSETGAMVRLLEDGDGGSHHH
HHH (SEQ ID NO:32)

ATGGCGAGCAATTTACTGACCGTACACCAAAATTTGCCTGCATTGCCGGTCGATGCAACGAGTGATGA
GGTTCGCAAGAACCTGATGGACATGTTCAGGGATCGCCAGGCGTTTTCTGAGCATACCTGGAAAATGC
TTCTGTCCGTTTGCCGGTCGTGGGCGGCATGGTGCAAGTTGAATAACCGGAAATGGTTTCCCGCAGAA
CCTGAAGATGTTCGCGATTATCTTCTATATCTTCAGGCGCGCGGTCTGGCAGTAAAAACTATCCAGCAA
CATTTGGGCCAGCTAAACATGCTTCATCGTCGGTCCGGGCTGCCACGACCAAGTGACAGCAATGCTGT
TTCACTGGTTATGCGGCGTATCCGAAAAGAAAACGTTGATGCCGGTGAACGTGCAAAACAGGCTCTAG
CGTTCGAACGCACTGATTTCGACCAGGTTCGTTCACTCATGGAAAATAGCGATCGCTGCCAGGATATA
CGTAATCTGGCATTTCTGGGGATTGCTTATAACACCCTGTTACGTATAGCCGAAATTGCCAGGATCAGG
GTTAAAGATATCTCACGTACTGACGGTGGGAGAATGTTAATCCATATTGGCAGAACGAAAACGCTGGT
TAGCACCGCAGGTGTAGAGAAGGCACTTAGCCTGGGGGTAACTAAACTGGTCGAGCGATGGATTTCCG
TCTCTGGTGTAGCTGATGATCCGAATAACTACCTGTTTTGCCGGGTCAGAAAAAATGGTGTTGCCGCGC
CATCTGCCACCAGCCAGCTATCAACTCGCGCCCTGGAAGGGATTTTTGAAGCAACTCATCGATTGATTT
ACGGCGCTAAGGATGACTCTGGTCAGAGATACCTGGCCTGGTCTGGACACAGTGCCCGTGTCGGAGCC
GCGCGAGATATGGCCCGCGCTGGAGTTTCAATACCGGAGATCATGCAAGCTGGTGGCTGGACCAATGT
AAATATTGTCATGAACTATATCCGTAACCTGGATAGTGAAACAGGGGCAATGGTGCGCCTGCTGGAAG
ATGGCGACGGCGGATCCCATCACCACCACCATCAC (SEQ ID NO: 263)

### (+36)GFP-Cre-6xHis (+36 GFP double-underlined; 6xHis tag underlined):

MGASKGERLFRGKVPILVELKGDVNGHKFSVRGKGKGDATRGKLTLKFICTTGKLPVPWPTLVTTLTYGV
QCFSRYPKHMKRHDFFKSAMPKGYVQERTISFKKDGKYKTRAEVKFEGRTLVNRIKLKGRDFKEKGNILG
HKLRYNFNSHKVYITADKRKNGIKAKFKIRHNVKDGSVQLADHYQQNTPIGRGPVLLPRNHYLSTRSKLS
KDPKEKRDHMVLLEFVTAAGIKHGRDERYKTGGSGGSGGSGGSGGSGGSGGSGGSGGTASNLLTVHQNLP
ALPVDATSDEVRKNLMDMFRDRQAFSEHTWKMLLSVCRSWAAWCKLNNRKWFPAEPEDVRDYLLYLQARG
LAVKTIQQHLGQLNMLHRRSGLPRPSDSNAVSLVMRRIRKENVDAGERAKQALAFERTDFDQVRSLMENS
DRCQDIRNLAFLGIAYNTLLRIAEIARIRVKDISRTDGGRMLIHIGRTKTLVSTAGVEKALSLGVTKLVE
RWISVSGVADDPNNYLFCRVRKNGVAAPSATSQLSTRALEGIFEATHRLIYGAKDDSGQRYLAWSGHSAR
VGAARDMARAGVSIPEIMQAGGWTNVNIVMNYIRNLDSETGAMVRLLEDGDGGSHHHHHH (SEQ ID
NO:33)

### (-30)GFP-Cre-6xHis (-30 GFP double-underlined; 6xHis tag underlined):

MGASKGEELFDGVVPILVELDGDVNGHEFSVRGEGEGDATEGELTLKFICTTGELPVPWPTLVTTLTYGV
QCFSDYPDHMDQHDFFKSAMPEGYVQERTISFKDDGTYKTRAEVKFEGDTLVNRIELKGIDFKEDGNILG
HKLEYNFNSHDVYITADKQENGIKAEFEIRHNVEDGSVQLADHYQQNTPIGDGPVLLPDDHYLSTESALS
KDPNEDRDHMVLLEFVTAAGIDHGMDELYKTGGSGGSGGSGGSGGSGGSGGSGGTASNLLTVHQNLP
ALPVDATSDEVRKNLMDMFRDRQAFSEHTWKMLLSVCRSWAAWCKLNNRKWFPAEPEDVRDYLLYLQARG
LAVKTIQQHLGQLNMLHRRSGLPRPSDSNAVSLVMRRIRKENVDAGERAKQALAFERTDFDQVRSLMENS
DRCQDIRNLAFLGIAYNTLLRIAEIARIRVKDISRTDGGRMLIHIGRTKTLVSTAGVEKALSLGVTKLVE
RWISVSGVADDPNNYLFCRVRKNGVAAPSATSQLSTRALEGIFEATHRLIYGAKDDSGQRYLAWSGHSAR
VGAARDMARAGVSIPEIMQAGGWTNVNIVMNYIRNLDSETGAMVRLLEDGDGGSHHHHHH (SEQ ID
NO:34)

ATGGGTGCTAGCAAAGGTGAAGAGCTGTTTGACGGTGTAGTACCGATCTTAGTGGAATTAGACGGCGA
CGTGAACGGTCACGAATTTAGCGTGCGCGGCGAGGGCGAAGGTGACGCTACCGAGGGTGAATTGACC
CTGAAGTTTATTTGCACAACAGGCGAATTACCCGTTCCGTGGCCCACCTTAGTGACCACCCTGACCTAT
GGCGTTCAGTGCTTCAGTGATTACCCAGATCATATGGATCAACACGATTTTTTCAAATCAGCCATGCCT
GAAGGATATGTTCAAGAGCGTACAATCAGCTTCAAGGACGATGGCACCTATAAAACGCGTGCGGAAG
TGAAATTTGAAGGCGACACATTAGTAAACCGTATCGAACTGAAAGGTATCGACTTCAAAGAAGACGG
CAACATTTTAGGCCATAAGCTGGAATATAACTTTAATTCTCATGACGTGTATATTACGGCCGATAAACA
GGAAAACGGTATCAAGGCAGAATTTGAAATTCGCCATAACGTGGAGGACGGCAGCGTTCAATTAGCG
GATCATTATCAACAAAACACGCCGATTGGTGATGGGCCTGTACTGTTACCTGACGATCACTACCTGAG
CACGGAGTCAGCCCTGAGCAAAGATCCGAACGAAGACCGCGATCACATGGTTCTGTTAGAATTCGTGA
CCGCTGCAGGCATTGATCATGGAATGGACGAGCTGTACAAGACCGGTGGTAGCGGTGGTTCTGGTGGT
TCTGGTGGTAGCGGCGGTAGCGGTGGTAGCGGTGGTAGCGGTGGCAGCGGCGGTACCGCGAGCAATT
TACTGACCGTACACCAAAATTTGCCTGCATTGCCGGTCGATGCAACGAGTGATGAGGTTCGCAAGAAC
CTGATGGACATGTTCAGGGATCGCCAGGCGTTTTCTGAGCATACCTGGAAAATGCTTCTGTCCGTTTGC
CGGTCGTGGGCGGCATGGTGCAAGTTGAATAACCGGAAATGGTTTCCCGCAGAACCTGAAGATGTTCG
CGATTATCTTCTATATCTTCAGGCGCGCGGTCTGGCAGTAAAAACTATCCAGCAACATTTGGGCCAGCT
AAACATGCTTCATCGTCGGTCCGGGCTGCCACGACCAAGTGACAGCAATGCTGTTTCACTGGTTATGC
GGCGTATCCGAAAAGAAAACGTTGATGCCGGTGAACGTGCAAAACAGGCTCTAGCGTTCGAACGCAC
TGATTTCGACCAGGTTCGTTCACTCATGGAAAATAGCGATCGCTGCCAGGATATACGTAATCTGGCATT
TCTGGGGATTGCTTATAACACCCTGTTACGTATAGCCGAAATTGCCAGGATCAGGGTTAAAGATATCT
CACGTACTGACGGTGGGAGAATGTTAATCCATATTGGCAGAACGAAAACGCTGGTTAGCACCGCAGGT
GTAGAGAAGGCACTTAGCCTGGGGGTAACTAAACTGGTCGAGCGATGGATTTCCGTCTCTGGTGTAGC
TGATGATCCGAATAACTACCTGTTTTGCCGGGTCAGAAAAAATGGTGTTGCCGCGCCATCTGCCACCA
GCCAGCTATCAACTCGCGCCCTGGAAGGGATTTTTGAAGCAACTCATCGATTGATTTACGGCGCTAAG
GATGACTCTGGTCAGAGATACCTGGCCTGGTCTGGACACAGTGCCCGTGTCGGAGCCGCGCGAGATAT
GGCCCGCGCTGGAGTTTCAATACCGGAGATCATGCAAGCTGGTGGCTGGACCAATGTAAATATTGTCA
TGAACTATATCCGTAACCTGGATAGTGAAACAGGGGCAATGGTGCGCCTGCTGGAAGATGGCGACGG

CGGATCCCATCACCACCACCATCAC (SEQ ID NO: 264)

### (-7)GFP-Cre-6xHis:

MGASKGEELFTGVVPILVELDGDVNGHKFSVRGEGEGDATNGKLTLKFICTTGKLPVPWPTLVTTLTYGV
QCFSRYPDHMKQHDFFKSAMPEGYVQERTISFKDDGTYKTRAEVKFEGDTLVNRIELKGIDFKEDGNILGH
KLEYNFNSHNVYITADKQKNGIKANFKIRHNVEDGSVQLADHYQQNTPIGDGPVLLPDNHYLSTQSALSKD
PNEKRDHMVLLEFVTAAGITHGMDELYKTGGSGGSGGSGGSGGSGGSGGSGGTASNLLTVHQNLPAL
PVDATSDEVRKNLMDMFRDRQAFSEHTWKMLLSVCRSWAAWCKLNNRKWFPAEPEDVRDYLLYLQAR
GLAVKTIQQHLGQLNMLHRRSGLPRPSDSNAVSLVMRRIRKENVDAGERAKQALAFERTDFDQVRSLMEN
SDRCQDIRNLAFLGIAYNTLLRIAEIARIRVKDISRTDGGRMLIHIGRTKTLVSTAGVEKALSLGVTKLVERW
ISVSGVADDPNNYLFCRVRKNGVAAPSATSQLSTRALEGIFEATHRLIYGAKDDSGQRYLAWSGHSARVG
AARDMARAGVSIPEIMQAGGWTNVNIVMNYIRNLDSETGAMVRLLEDGDGGSHHHHHH (SEQ ID NO:
265)

ATGGGTGCTAGCAAAGGTGAAGAGCTGTTTACGGGTGTAGTACCGATCTTAGTGGAATTAGACGGCGA
CGTGAACGGTCACAAATTTAGCGTGCGCGGCGAAGGCGAAGGTGACGCTACCAATGGTAAATTGACC
CTGAAGTTTATTTGCACAACAGGCAAATTACCCGTTCCGTGGCCCACCTTAGTGACCACCCTGACCTAT
GGCGTTCAGTGCTTCAGTCGTTACCCAGATCATATGAAACAACACGATTTTTTCAAATCAGCCATGCCT
GAAGGATATGTTCAAGAGCGTACAATCAGCTTCAAGGACGATGGCACCTATAAAACGCGTGCGGAAG
TGAAATTTGAAGGCGACACATTAGTAAACCGTATCGAACTGAAAGGTATCGACTTCAAAGAAGACGG
CAACATTTTAGGCCATAAGCTGGAATATAACTTTAATTCTCATAACGTGTATATTACGGCCGATAAACA
GAAAAACGGTATCAAGGCAAATTTCAAAATTCGCCATAACGTGGAAGACGGCAGCGTTCAATTAGCG
GATCATTATCAACAAAACACGCCGATTGGTGACGGGCCTGTACTGTTACCTGACAACCACTACCTGAG
CACCCAGTCAGCACTGAGCAAAGATCCGAACGAAAAACGCGATCACATGGTTCTGTTAGAATTCGTGA
CCGCTGCAGGCATTACTCACGGAATGGACGAACTCTACAAGACCGGTGGTAGCGGTGGTTCTGGTGGT
TCTGGTGGTAGCGGCGGTAGCGGTGGTAGCGGTGGTAGCGGTGGCAGCGGCGGTACCGCGAGCAATT
TACTGACCGTACACCAAAATTTGCCTGCATTGCCGGTCGATGCAACGAGTGATGAGGTTCGCAAGAAC
CTGATGGACATGTTCAGGGATCGCCAGGCGTTTTCTGAGCATACCTGGAAAATGCTTCTGTCCGTTTGC
CGGTCGTGGGCGGCATGGTGCAAGTTGAATAACCGGAAATGGTTTCCCGCAGAACCTGAAGATGTTCG
CGATTATCTTCTATATCTTCAGGCGCGCGGTCTGGCAGTAAAAACTATCCAGCAACATTTGGGCCAGCT
AAACATGCTTCATCGTCGGTCCGGGCTGCCACGACCAAGTGACAGCAATGCTGTTTCACTGGTTATGC
GGCGTATCCGAAAAGAAAACGTTGATGCCGGTGAACGTGCAAAACAGGCTCTAGCGTTCGAACGCAC
TGATTTCGACCAGGTTCGTTCACTCATGGAAAATAGCGATCGCTGCCAGGATATACGTAATCTGGCATT
TCTGGGGATTGCTTATAACACCCTGTTACGTATAGCCGAAATTGCCAGGATCAGGGTTAAAGATATCT
CACGTACTGACGGTGGGAGAATGTTAATCCATATTGGCAGAACGAAAACGCTGGTTAGCACCGCAGGT
GTAGAGAAGGCACTTAGCCTGGGGGTAACTAAACTGGTCGAGCGATGGATTTCCGTCTCTGGTGTAGC
TGATGATCCGAATAACTACCTGTTTTGCCGGGTCAGAAAAAATGGTGTTGCCGCGCCATCTGCCACCA
GCCAGCTATCAACTCGCGCCCTGGAAGGGATTTTTGAAGCAACTCATCGATTGATTTACGGCGCTAAG
GATGACTCTGGTCAGAGATACCTGGCCTGGTCTGGACACAGTGCCCGTGTCGGAGCCGCGCGAGATAT
GGCCCGCGCTGGAGTTTCAATACCGGAGATCATGCAAGCTGGTGGCTGGACCAATGTAAATATTGTCA
TGAACTATATCCGTAACCTGGATAGTGAAACAGGGGCAATGGTGCGCCTGCTGGAAGATGGCGACGG
CGGATCCCATCACCACCACCATCAC (SEQ ID NO:266)

### (-20)GFP-Cre-6xHis:

MGASKGEELFTGVVPILVELDGDVNGHKFSVRGEGEGDATNGKLTLKFICTTGKLPVPWPTLVTTLTYGV
QCFSRYPDHMDQHDFFKSAMPEGYVQERTISFKDDGTYKTRAEVKFEGDTLVNRIELKGIDFKEDGNILGH
KLEYNFNSHDVYITADKQENGIKAEFEIRHNVEDGSVQLADHYQQNTPIGDGPVLLPDDHYLSTESALSKD
PNEDRDHMVLLEFVTAAGIDHGMDELYKTGGSGGSGGSGGSGGSGGSGGSGGTASNLLTVHQNLPA
LPVDATSDEVRKNLMDMFRDRQAFSEHTWKMLLSVCRSWAAWCKLNNRKWFPAEPEDVRDYLLYLQAR
GLAVKTIQQHLGQLNMLHRRSGLPRPSDSNAVSLVMRRIRKENVDAGERAKQALAFERTDFDQVRSLMEN
SDRCQDIRNLAFLGIAYNTLLRIAEIARIRVKDISRTDGGRMLIHIGRTKTLVSTAGVEKALSLGVTKLVERW
ISVSGVADDPNNYLFCRVRKNGVAAPSATSQLSTRALEGIFEATHRLIYGAKDDSGQRYLAWSGHSARVG
AARDMARAGVSIPEIMQAGGWTNVNIVMNYIRNLDSETGAMVRLLEDGDGGSHHHHHH (SEQ ID
NO:267)

ATGGGTGCTAGCAAAGGTGAAGAGCTGTTTACGGGTGTAGTACCGATCTTAGTGGAATTAGACGGCGA
CGTGAACGGTCACAAATTTAGCGTGCGCGGCGAAGGCGAAGGTGACGCTACCAATGGTAAATTGACC
CTGAAGTTTATTTGCACAACAGGCAAATTACCCGTTCCGTGGCCCACCTTAGTGACCACCCTGACCTAT
GGCGTTCAGTGCTTCAGTCGTTACCCAGATCATATGGATCAACACGATTTTTTCAAATCAGCCATGCCT
GAAGGATATGTTCAAGAGCGTACAATCAGCTTCAAGGACGATGGCACCTATAAAACGCGTGCGGAAG
TGAAATTTGAAGGCGACACATTAGTAAACCGTATCGAACTGAAAGGTATCGACTTCAAAGAAGACGG
CAACATTTTAGGCCATAAGCTGGAATATAACTTTAATTCTCATGACGTGTATATTACGGCCGATAAACA
GGAAAACGGTATCAAGGCAGAATTTGAAATTCGCCATAACGTGGAGGACGGCAGCGTTCAATTAGCG
GATCATTATCAACAAAACACGCCGATTGGTGATGGGCCTGTACTGTTACCTGACGATCACTACCTGAG
CACGGAGTCAGCCCTGAGCAAAGATCCGAACGAAGACCGCGATCACATGGTTCTGTTAGAATTCGTGA
CCGCTGCAGGCATTGATCATGGAATGGACGAGCTGTACAAGACCGGTGGTAGCGGTGGTTCTGGTGGT
TCTGGTGGTAGCGGCGGTAGCGGTGGTAGCGGTGGTAGCGGTGGCAGCGGCGGTACCGCGAGCAATT
TACTGACCGTACACCAAAATTTGCCTGCATTGCCGGTCGATGCAACGAGTGATGAGGTTCGCAAGAAC
CTGATGGACATGTTCAGGGATCGCCAGGCGTTTTCTGAGCATACCTGGAAAATGCTTCTGTCCGTTTGC
CGGTCGTGGGCGGCATGGTGCAAGTTGAATAACCGGAAATGGTTTCCCGCAGAACCTGAAGATGTTCG
CGATTATCTTCTATATCTTCAGGCGCGCGGTCTGGCAGTAAAAACTATCCAGCAACATTTGGGCCAGCT
AAACATGCTTCATCGTCGGTCCGGGCTGCCACGACCAAGTGACAGCAATGCTGTTTCACTGGTTATGC
GGCGTATCCGAAAAGAAAACGTTGATGCCGGTGAACGTGCAAAACAGGCTCTAGCGTTCGAACGCAC
TGATTTCGACCAGGTTCGTTCACTCATGGAAAATAGCGATCGCTGCCAGGATATACGTAATCTGGCATT
TCTGGGGATTGCTTATAACACCCTGTTACGTATAGCCGAAATTGCCAGGATCAGGGTTAAAGATATCT
CACGTACTGACGGTGGGAGAATGTTAATCCATATTGGCAGAACGAAAACGCTGGTTAGCACCGCAGGT
GTAGAGAAGGCACTTAGCCTGGGGGTAACTAAACTGGTCGAGCGATGGATTTCCGTCTCTGGTGTAGC
TGATGATCCGAATAACTACCTGTTTTGCCGGGTCAGAAAAAATGGTGTTGCCGCGCCATCTGCCACCA
GCCAGCTATCAACTCGCGCCCTGGAAGGGATTTTTGAAGCAACTCATCGATTGATTTACGGCGCTAAG
GATGACTCTGGTCAGAGATACCTGGCCTGGTCTGGACACAGTGCCCGTGTCGGAGCCGCGCGAGATAT
GGCCCGCGCTGGAGTTTCAATACCGGAGATCATGCAAGCTGGTGGCTGGACCAATGTAAATATTGTCA
TGAACTATATCCGTAACCTGGATAGTGAAACAGGGGCAATGGTGCGCCTGCTGGAAGATGGCGACGG
CGGATCCCATCACCACCACCATCAC (SEQ ID NO:268)

### (+36)GFP-PPARy-TALE-2 (+36 GFP double-underlined; 6xHis tag underlined):

MGASKGERLFRGKVPILVELKGDVNGHKFSVRGKGKGDATRGKLTLKFICTTGKLPVPWPTLVTTLTYGV
QCFSRYPKHMKRHDFFKSAMPKGYVQERTISFKKDGKYKTRAEVKFEGRTLVNRIKLKGRDFKEKGNILG
HKLRYNFNSHKVYITADKRKNGIKAKFKIRHNVKDGSVQLADHYQQNTPIGRGPVLLPRNHYLSTRSKLS
KDPKEKRDHMVLLEFVTAAGIKHGRDERYKTGGSGGSGGSGGSGGSGGSGGSGGSGGTAPKKKRKVGIHR
GVPMVDLRTLGYSQQQQEKIKPKVRSTVAQHHEALVGHGFTHAHIVALSQHPAALGTVAVKYQDMIAALP
EATHEAIVGVGKQWSGARALEALLTVAGELRGPPLQLDTGQLLKIAKRGGVTAVEAVHAWRNALTGAPLN
LTPDQVVAIASNIGGKQALETVQRLLPVLCQDHGLTPEQVVAIASNIGGKQALETVQRLLPVLCQAHGLT
PDQVVAIASNGGGKQALETVQRLLPVLCQAHGLTPAQVVAIANNNGGKQALETVQRLLPVLCQDHGLTPD
QVVAIASNGGGKQALETVQRLLPVLCQDHGLTPEQVVAIASNIGGKQALETVQRLLPVLCQAHGLTPDQV
VAIASHDGGKQALETVQRLLPVLCQAHGLTPAQVVAIASHDGGKQALETVQRLLPVLCQDHGLTPDQVVA
IASNIGGKQALETVQRLLPVLCQDHGLTPEQVVAIASNIGGKQALETVQRLLPVLCQAHGLTPDQVVAIA
NNNGGKQALETVQRLLPVLCQAHGLTPAQVVAIASNGGGKQALETVQRLLPVLCQDHGLTPDQVVAIASH
DGGKQALETVQRLLPVLCQDHGLTPEQVVAIASNGGGKQALETVQRLLPVLCQAHGLTPDQVVAIASNGG
GKQALETVQRLLPVLCQAHGLTPAQVVAIANNNGGKQALETVQRLLPVLCQDHGLTPDQVVAIASHDGGK
QALETVQRLLPVLCQDHGLTPEQVVAIASHDGGKQALETVQRLLPVLCQAHGLTPEQVVAIASNIGGRPA
LESIVAQLSRPDPALAALTNDHLVALACLGGRPALDAVKKGLPHAPALIKRTNRRIPERTSHRVADHAQV
VRVLGFFQCHSHPAQAFDDAMTQFGMSGGGSGRADALDDFDLDMLGSDALDDFDLDMLGSDALDDFDLDM
LGSDALDDFDLDMLHHHHHH (SEQ ID NO:36)

### (+36)GFP-PRDM16_TALE-3(+36 GFP double-underlined; 6xHis tag underlined):

MGASKGERLFRGKVPILVELKGDVNGHKFSVRGKGKGDATRGKLTLKFICTTGKLPVPWPTLVTTLTYGV
QCFSRYPKHMKRHDFFKSAMPKGYVQERTISFKKDGKYKTRAEVKFEGRTLVNRIKLKGRDFKEKGNILG
HKLRYNFNSHKVYITADKRKNGIKAKFKIRHNVKDGSVQLADHYQQNTPIGRGPVLLPRNHYLSTRSKLS
KDPKEKRDHMVLLEFVTAAGIKHGRDERYKTGGSGGSGGSGGSGGSGGSGGSGGSGGTAPKKKRKVGIHR
GVPMVDLRTLGYSQQQQEKIKPKVRSTVAQHHEALVGHGFTHAHIVALSQHPAALGTVAVKYQDMIAALP
EATHEAIVGVGKQWSGARALEALLTVAGELRGPPLQLDTGQLLKIAKRGGVTAVEAVHAWRNALTGAPLN
LTPDQVVAIASNGGGKQALETVQRLLPVLCQDHGLTPEQVVAIANNNGGKQALETVQRLLPVLCQAHGLT
PDQVVAIANNNGGKQALETVQRLLPVLCQAHGLTPAQVVAIASHDGGKQALETVQRLLPVLCQDHGLTPD
QVVAIASHDGGKQALETVQRLLPVLCQDHGLTPEQVVAIASHDGGKQALETVQRLLPVLCQAHGLTPDQV
VAIASHDGGKQALETVQRLLPVLCQAHGLTPAQVVAIANNNGGKQALETVQRLLPVLCQDHGLTPDQVVA
IANNNGGKQALETVQRLLPVLCQDHGLTPEQVVAIASHDGGKQALETVQRLLPVLCQAHGLTPDQVVAIA
NNNGGKQALETVQRLLPVLCQAHGLTPAQVVAIASNIGGKQALETVQRLLPVLCQDHGLTPDQVVAIANN
NGGKQALETVQRLLPVLCQDHGLTPEQVVAIANNNGGKQALETVQRLLPVLCQAHGLTPDQVVAIANNNG
GKQALETVQRLLPVLCQAHGLTPAQVVAIANNNGGKQALETVQRLLPVLCQDHGLTPDQVVAIASNGGGK
QALETVQRLLPVLCQDHGLTPEQVVAIANNNGGKQALETVQRLLPVLCQAHGLTPEQVVAIASNGGGRPA
LESIVAQLSRPDPALAALTNDHLVALACLGGRPALDAVKKGLPHAPALIKRTNRRIPERTSHRVADHAQV
VRVLGFFQCHSHPAQAFDDAMTQFGMSGGGSGRADALDDFDLDMLGSDALDDFDLDMLGSDALDDFDLDM
LGSDALDDFDLDMLHHHHHH (SEQ ID NO:37)

### (-30)GFP-PPARγ-TALE-2 (-30 GFP double-underlined; 6xHis tag underlined):

MGASKGEELFDGVVPILVELDGDVNGHEFSVRGEGEGDATEGELTLKFICTTGELPVPWPTLVTTLTYGV
QCFSDYPDHMDQHDFFKSAMPEGYVQERTISFKDDGTYKTRAEVKFEGDTLVNRIELKGIDFKEDGNILG
HKLEYNFNSHDVYITADKQENGIKAEFEIRHNVEDGSVQLADHYQQNTPIGDGPVLLPDDHYLSTESALS
KDPNEDRDHMVLLEFVTAAGIDHGMDELYKAPKKKRKVGIHRGVPMVDLRTLGYSQQQQEKIKPKVRSTV
AQHHEALVGHGFTHAHIVALSQHPAALGTVAVKYQDMIAALPEATHEAIVGVGKQWSGARALEALLTVAG
ELRGPPLQLDTGQLLKIAKRGGVTAVEAVHAWRNALTGAPLNLTPDQVVAIASNIGGKQALETVQRLLPV
LCQDHGLTPEQVVAIASNIGGKQALETVQRLLPVLCQAHGLTPDQVVAIASNGGGKQALETVQRLLPVLC
QAHGLTPAQVVAIANNNGGKQALETVQRLLPVLCQDHGLTPDQVVAIASNGGGKQALETVQRLLPVLCQD
HGLTPEQVVAIASNIGGKQALETVQRLLPVLCQAHGLTPDQVVAIASHDGGKQALETVQRLLPVLCQAHG
LTPAQVVAIASHDGGKQALETVQRLLPVLCQDHGLTPDQVVAIASNIGGKQALETVQRLLPVLCQDHGLT
PEQVVAIASNIGGKQALETVQRLLPVLCQAHGLTPDQVVAIANNNGGKQALETVQRLLPVLCQAHGLTPA
QVVAIASNGGGKQALETVQRLLPVLCQDHGLTPDQVVAIASHDGGKQALETVQRLLPVLCQDHGLTPEQV
VAIASNGGGKQALETVQRLLPVLCQAHGLTPDQVVAIASNGGGKQALETVQRLLPVLCQAHGLTPAQVVA
IANNNGGKQALETVQRLLPVLCQDHGLTPDQVVAIASHDGGKQALETVQRLLPVLCQDHGLTPEQVVAIA
SHDGGKQALETVQRLLPVLCQAHGLTPEQVVAIASNIGGRPALESIVAQLSRPDPALAALTNDHLVALAC
LGGRPALDAVKKGLPHAPALIKRTNRRIPERTSHRVADHAQVVRVLGFFQCHSHPAQAFDDAMTQFGMSG
GGSGRADALDDFDLDMLGSDALDDFDLDMLGSDALDDFDLDMLGSDALDDFDLDMLHHHHHH (SEQ ID
NO:38)

### (-30)GFP-PRDM16_TALE-3(-30 GFP double-underlined; 6xHis tag underlined):

MGASKGEELFDGVVPILVELDGDVNGHEFSVRGEGEGDATEGELTLKFICTTGELPVPWPTLVTTLTYGV
QCFSDYPDHMDQHDFFKSAMPEGYVQERTISFKDDGTYKTRAEVKFEGDTLVNRIELKGIDFKEDGNILG
HKLEYNFNSHDVYITADKQENGIKAEFEIRHNVEDGSVQLADHYQQNTPIGDGPVLLPDDHYLSTESALS
KDPNEDRDHMVLLEFVTAAGIDHGMDELYKAPKKKRKVGIHRGVPMVDLRTLGYSQQQQEKIKPKVRSTV
AQHHEALVGHGFTHAHIVALSQHPAALGTVAVKYQDMIAALPEATHEAIVGVGKQWSGARALEALLTVAG
ELRGPPLQLDTGQLLKIAKRGGVTAVEAVHAWRNALTGAPLNLTPDQVVAIASNGGGKQALETVQRLLPV
LCQDHGLTPEQVVAIANNNGGKQALETVQRLLPVLCQAHGLTPDQVVAIANNNGGKQALETVQRLLPVLC

QAHGLTPAQVVAIASHDGGKQALETVQRLLPVLCQDHGLTPDQVVAIASHDGGKQALETVQRLLPVLCQD
HGLTPEQVVAIASHDGGKQALETVQRLLPVLCQAHGLTPDQVVAIASHDGGKQALETVQRLLPVLCQAHG
LTPAQVVAIANNNGGKQALETVQRLLPVLCQDHGLTPDQVVAIANNNGGKQALETVQRLLPVLCQDHGLT
PEQVVAIASHDGGKQALETVQRLLPVLCQAHGLTPDQVVAIANNNGGKQALETVQRLLPVLCQAHGLTPA
QVVAIASNIGGKQALETVQRLLPVLCQDHGLTPDQVVAIANNNGGKQALETVQRLLPVLCQDHGLTPEQV
VAIANNNGGKQALETVQRLLPVLCQAHGLTPDQVVAIANNNGGKQALETVQRLLPVLCQAHGLTPAQVVA
IANNNGGKQALETVQRLLPVLCQDHGLTPDQVVAIASNGGGKQALETVQRLLPVLCQDHGLTPEQVVAIA
NNNGGKQALETVQRLLPVLCQAHGLTPEQVVAIASNGGGRPALESIVAQLSRPDPALAALTNDHLVALAC
LGGRPALDAVKKGLPHAPALIKRTNRRIPERTSHRVADHAQVVRVLGFFQCHSHPAQAFDDAMTQFGMSG
GGSGRADALDDFDLDMLGSDALDDFDLDMLGSDALDDFDLDMLGSDALDDFDLDMLHHHHHH (SEQ ID
NO:39)

### (-30)GFP:

MGASKGEELFDGVVPILVELDGDVNGHEFSVRGEGEGDATEGELTLKFICTTGELPVPWPTLVTTLTYGV
QCFSDYPDHMDQHDFFKSAMPEGYVQERTISFKDDGTYKTRAEVKFEGDTLVNRIELKGIDFKEDGNILG
HKLEYNFNSHDVYITADKQENGIKAEFEIRHNVEDGSVQLADHYQQNTPIGDGPVLLPDDHYLSTESALS
KDPNEDRDHMVLLEFVTAAGIDHGMDELYK (SEQ ID NO:40)

### N-terminal TALE domain:

APKKKRKVGIHRGVPMVDLRTLGYSQQQQEKIKPKVRSTVAQHHEALVGHGFTHAHIVALSQHPAALGTV
AVKYQDMIAALPEATHEAIVGVGKQWSGARALEALLTVAGELRGPPLQLDTGQLLKIAKRGGVTAVEAVH
AWRNALTGAPLNL (SEQ ID NO:41)

### C-terminal TALE domain:

LESIVAQLSRPDPALAALTNDHLVALACLGGRPALDAVKKGLPHAPALIKRTNRRIPERTSHRVADHAQV
VRVLGFFQCHSHPAQAFDDAMTQFGMSGGGS (SEQ ID NO:42)

VP64 activation domain:
GRADALDDFDLDMLGSDALDDFDLDMLGSDALDDFDLDMLGSDALDDFDLDML (SEQ ID NO:35)

### Compositions of functional effector proteins and cationic polymers

Certain aspects of the disclosure relate to the use of cationic polymers for the delivery of effector proteins (*e.g*., nucleases, transcriptional activators/repressors, recombinases, Cas9 proteins including variants and fusions thereof, *etc*.), for example as opposed to delivering "naked" protein preparations. As with cationic lipids, aspects of the present disclosure relate to the recognition that anionic proteins or protein complexes (including those proteins associated with nucleic acids) can take advantage of electrostatics-driven encapsulation by and/or association with cationic polymers for delivery of functional effector proteins. While few proteins natively possess the density of negative charges found in the phosphate backbone of nucleic acids, translational fusion to, or non-covalent association with, an anionic carrier such as a negatively supercharged protein or a nucleic acid as described herein render the resulting effector protein or protein complex sufficiently anionic to drive efficient encapsulation/association of such protein cargoes by cationic polymers.

In some embodiments, association or fusion with an engineered supernegatively charged GFP is capable of driving efficient encapsulation/association and delivery of proteins into cultured mammalian cells by cationic polymers. In some embodiments, Cas9 protein associated with polyanionic guide RNAs (gRNA) can be efficiently delivered in functional form into mammalian cells using cationic polymers. Accordingly, in some embodiments, a composition comprising a Cas9 protein and a cationic polymer is provided, wherein the Cas9 protein is associated with a gRNA, and the composition is capable of delivering the Cas9 protein to the interior of a cell. In some embodiments, delivery of Cas9:gRNA complexes using cationic polymers results in at least a 2-fold, 3-fold, 4-fold, 5-fold, 6-fold, 7-fold, 8-fold, 9-fold, 10-fold, 11-fold, 12-fold, 13-fold, 14-fold, 15-fold, 20-fold or 25-fold or higher on-target: off-target modification ratio as compared with plasmid transfection of the Cas9 protein.

Accordingly, some aspects of the disclosure provide compositions comprising a Cas9 protein (*e.g.,* as described herein; see *e.g., Cas9 effector proteins* below) and a cationic polymer capable of delivering the Cas9 protein to the interior of a cell. In some embodiments, the Cas9 protein is associated with a gRNA, which *e.g.*, provides anionic charge to the complex thereby allowing the Cas9:gRNA complex to be encapsulated and/or associated with the cationic polymers. In some embodiments, the Cas9 protein need not be associated with a gRNA for effective encapsulation by and/or association with a cationic lipid, but instead is associated with a negatively supercharged protein, as described herein. In some embodiments where a Cas9 protein is associated with a negatively supercharged protein, the Cas9 protein is also associated with a gRNA. In some embodiments, the Cas9 protein is a wild type Cas9 protein, a fragment of a wild type Cas9 protein, or a variant of a wild type Cas9 protein. In some embodiments, the Cas9 protein comprises a dCas9 domain (*e.g.*, as described herein). In some embodiments, the Cas9 protein is a fusion protein comprising a dCas9 domain (*e.g.*, as described herein). In some embodiments, the Cas9 protein is a Cas9 nickase.

In other embodiments, compositions comprising an effector protein (*e.g*., other than a Cas9 protein) and a cationic polymer are provided which are capable of delivering the effector protein to the interior of a cell (*e.g.,* to the nucleus of the cell). The effector protein is either naturally negatively charged, is modified to have a net overall negative charge, or is associated with a negatively supercharged protein, as described herein. In some embodiments, the effector protein is any effector protein described herein. In some embodiments, the effector protein is a recombinase, *e.g*., any recombinase described herein. In some embodiments, the recombinase is Cre recombinase. In some embodiments, the Cre recombinase comprises the amino acid sequence of SEQ ID NO:32 (*e.g.,* with or without the 6xHis tag). In some embodiments, the Cre recombinase comprises an amino acid sequence that is at least 80%, at least 85%, at least 90%, at least 95%, at least 98%, or at least 99% identical to the amino acid sequence of SEQ ID NO:32 (*e.g.,* with or without the 6xHis tag). In some embodiments, the Cre recombinase is fused to a supercharged protein (*e.g.,* +36 GFP or -30GFP). In some embodiments, the Cre recombinase fused to a supercharged protein comprises the amino acid sequence of SEQ ID NO:33 (*e.g.,* with or without the 6xHis tag) or SEQ ID NO:34 (*e.g.,* with or without the 6xHis tag), or comprises an amino acid sequence that is at least 80%, at least 85%, at least 90%, at least 95%, at least 98%, or at least 99% identical to the amino acid sequence of SEQ ID NO:33 or SEQ ID NO:34 (*e.g.,* with or without the 6xHis tag). In some embodiments, the effector protein is a TALE protein, (*e.g.,* as described herein including those provided in the Examples). In some embodiments, the TALE protein comprises one or more of a VP64 transcriptional activator domain (*e.g.*, SEQ ID NO:35). In some embodiments, the TALE protein with a VP64 transcriptional activator domain further comprises an amino acid sequence selected from the group consisting of SEQ ID NO:36-39 (*e.g.*, with or without the 6xHis tag). In some embodiments, the TALE protein with a VP64 transcriptional activator domain comprises an amino acid sequence that is at least 80%, at least 85%, at least 90%, at least 95%, at least 98%, or at least 99% identical to an amino acid sequence selected from the group consisting of SEQ ID NO:36-39 (*e.g.,* with or without the 6xHis tag). In some embodiments, the TALE effector protein comprises a (-30)GFP domain (*e.g*., SEQ ID NO:21 or SEQ ID NO:40), a N-terminal region of a TALE domain (*e.g.,* SEQ ID NO:41), a variable repeat domain (*e.g.,* an 18.5mer repeat domain as provided in Maeder *et al.,* "Robust, synergistic regulation of human gene expression using TALE activators." *Nat. Methods.* 2013; **10**, 243-245), a C-terminal TALE domain (*e.g.,* SEQ ID NO:42), a VP64 activation domain (*e.g.,* SEQ ID NO:35), and optionally one or more linkers (*e.g.*, GGS(9), SEQ ID NO: 252) between any domain and optionally a sequence tag (*e.g.,* 6xHis, SEQ ID NO: 253).

In some embodiments, the compositions comprising a Cas9 protein or other effector proteins described herein and a cationic polymer exhibit low toxicity when administered to a population of cells (*e.g., in vitro* or *in vivo*). In some embodiments, at least 60%, at least 65%, at least 70%, at least 75%, at least 80%, at least 85%, at least 90%, at least 95%, or at least 99% of the cells in a population are viable following administration of an inventive composition comprising a Cas9 protein or other effector protein and cationic polymers. Methods for assessing the toxicity of a composition when administered to a population of cells are well known in the art and include those described in the Examples.

### Cas9 effector proteins

In some embodiments, effector proteins comprising a RNA-programmable protein (or fragment or variant thereof) is delivered to a target cell by a system or method provided herein. In some embodiments, an RNA-guided or RNA-programmable nuclease is delivered to a target cell by a system or method provided herein. In some embodiments, the RNA-programmable protein is a Cas9 nuclease, a Cas9 variant, or a fusion of a Cas9 protein, which is delivered to a target cell by a system or method provided herein.

In some embodiments, the RNA-programmable nuclease is a (CRISPR-associated system) Cas9 endonuclease, for example, Cas9 (Csn1) from *Streptococcus pyogenes* (see, *e.g.,* "Complete genome sequence of an M1 strain of Streptococcus pyogenes." Ferretti J.J., McShan W.M., Ajdic D.J., Savic D.J., Savic G., Lyon K., Primeaux C., Sezate S., Suvorov A.N., Kenton S., Lai H.S., Lin S.P., Qian Y., Jia H.G., Najar F.Z., Ren Q., Zhu H., Song L. expand/collapse author list McLaughlin R.E., Proc. Natl. Acad. Sci. U.S.A. 98:4658-4663(2001); "CRISPR RNA maturation by trans-encoded small RNA and host factor RNase III." Deltcheva E., Chylinski K., Sharma C.M., Gonzales K., Chao Y., Pirzada Z.A., Eckert M.R., Vogel J., Charpentier E., Nature 471:602-607(2011); and "A programmable dual-RNA-guided DNA endonuclease in adaptive bacterial immunity." Jinek M., Chylinski K., Fonfara I., Hauer M., Doudna J.A., Charpentier E. Science 337:816-821(2012), the entire contents of each of which are incorporated herein by reference. Because RNA-programmable nucleases (*e.g*., Cas9) use RNA:DNA hybridization to determine target DNA cleavage sites, these proteins are able to cleave, in principle, any sequence specified by the guide RNA. Methods of using RNA-programmable nucleases, such as Cas9, for site-specific cleavage (*e.g.,* to modify a genome) are known in the art (see *e.g.,* Cong, L. et al. Multiplex genome engineering using CRISPR/Cas systems. Science 339, 819-823 (2013); Mali, P. et al. RNA-guided human genome engineering via Cas9. Science 339, 823-826 (2013); Hwang, W.Y. et al. Efficient genome editing in zebrafish using a CRISPR-Cas system. Nature biotechnology 31, 227-229 (2013); Jinek, M. et al. RNA-programmed genome editing in human cells. eLife 2, e00471 (2013); Dicarlo, J.E. et al. Genome engineering in Saccharomyces cerevisiae using CRISPR-Cas systems. Nucleic acids research (2013); Jiang, W. et al. RNA-guided editing of bacterial genomes using CRISPR-Cas systems. Nature biotechnology 31, 233-239 (2013); the entire contents of each of which are incorporated herein by reference).

A Cas9 nuclease may also be referred to sometimes as a casn1 nuclease or a CRISPR (clustered regularly interspaced short palindromic repeat)-associated nuclease. CRISPR is an adaptive immune system that provides protection against mobile genetic elements (viruses, transposable elements and conjugative plasmids). CRISPR clusters contain spacers, sequences complementary to antecedent mobile elements, and target invading nucleic acids. CRISPR clusters are transcribed and processed into CRISPR RNA (crRNA). In type II CRISPR systems correct processing of pre-crRNA requires a trans-encoded small RNA (tracrRNA), endogenous ribonuclease 3 (rnc) and a Cas9 protein. The tracrRNA serves as a guide for ribonuclease 3-aided processing of pre-crRNA. Subsequently, Cas9/crRNA/tracrRNA endonucleolytically cleaves linear or circular dsDNA target complementary to the spacer. The target strand that is not complementary to the crRNA is first cut endonucleolytically, then trimmed 3'-5' exonucleolytically. In nature, DNA-binding and cleavage typically requires protein and both RNA. However, single guide RNAs ("sgRNA", or simply "gNRA") can be engineered so as to incorporate aspects of both the crRNA and tracrRNA into a single RNA species. See *e.g.,* Jinek M., Chylinski K., Fonfara I., Hauer M., Doudna J.A., Charpentier E. Science 337:816-821(2012), the entire contents of which is hereby incorporated by reference. Cas9 recognizes a short motif in the CRISPR repeat sequences (the PAM or protospacer adjacent motif) to help distinguish self versus non-self. Cas9 nuclease sequences and structures are well known to those of skill in the art (see, *e.g.,* "Complete genome sequence of an M1 strain of Streptococcus pyogenes." Ferretti J.J., McShan W.M., Ajdic D.J., Savic D.J., Savic G., Lyon K., Primeaux C., Sezate S., Suvorov A.N., Kenton S., Lai H.S., Lin S.P., Qian Y., Jia H.G., Najar F.Z., Ren Q., Zhu H., Song L. expand/collapse author list McLaughlin R.E., Proc. Natl. Acad. Sci. U.S.A. 98:4658-4663(2001); "CRISPR RNA maturation by trans-encoded small RNA and host factor RNase III." Deltcheva E., Chylinski K., Sharma C.M., Gonzales K., Chao Y., Pirzada Z.A., Eckert M.R., Vogel J., Charpentier E., Nature 471:602-607(2011); and "A programmable dual-RNA-guided DNA endonuclease in adaptive bacterial immunity." Jinek M., Chylinski K., Fonfara I., Hauer M., Doudna J.A., Charpentier E. Science 337:816-821(2012), the entire contents of each of which are incorporated herein by reference).

Cas9 orthologs have been described in various species, including, but not limited to, *S. pyogenes* and *S. thermophilus.* Additional suitable Cas9 nucleases and sequences will be apparent to those of skill in the art based on this disclosure, and such Cas9 nucleases and sequences include Cas9 sequences from the organisms and loci disclosed in Chylinski, Rhun, and Charpentier, "The tracrRNA and Cas9 families of type II CRISPR-Cas immunity systems" (2013) RNA Biology 10:5, 726-737; the entire contents of which are incorporated herein by reference. In some embodiments, proteins comprising Cas9 proteins or fragments thereof are referred to as "Cas9 variants." A Cas9 variant shares homology to Cas9, or a fragment thereof. For example, a Cas9 variant is at least about 70% identical, at least about 80% identical, at least about 90% identical, at least about 95% identical, at least about 98% identical, at least about 99% identical, at least about 99.5% identical, or at least about 99.9% to wild type Cas9. In some embodiments, the Cas9 variant comprises a fragment of Cas9 (*e.g.,* a gRNA binding domain or a DNA-cleavage domain, an N-terminal domain or a C-terminal domain, *etc*.), such that the fragment is at least about 70% identical, at least about 80% identical, at least about 90% identical, at least about 95% identical, at least about 98% identical, at least about 99% identical, at least about 99.5% identical, or at least about 99.9% to the corresponding fragment of wild type Cas9. In some embodiments, wild type Cas9 corresponds to Cas9 from *Streptococcus pyogenes* (NCBI Reference Sequence: NC_017053.1, SEQ ID NO:43 (nucleotide); SEQ ID NO:44 (amino acid)). In some embodiments, a Cas9 protein has an inactive (*e.g*., an inactivated) DNA cleavage domain. A nuclease-inactivated Cas9 protein may interchangeably be referred to as a "dCas9" protein (for nuclease "dead" Cas9). In some embodiments, dCas9 corresponds to, or comprises in part or in whole, the amino acid set forth as SEQ ID NO:45, below. In some embodiments, variants of dCas9 (*e.g.*, variants of SEQ ID NO:45) are provided. For example, in some embodiments, variants having mutations other than D10A and H840A are provided, which result in nuclease inactivated Cas9 (dCas9). Such mutations, by way of example, include other amino acid substitutions at D10 and H840, or other substitutions within the nuclease domain of Cas9 (*e.g*., substitutions in the HNH nuclease subdomain and/or the RuvC1 subdomain). In some embodiments, variants or homologues of dCas9 (*e.g*., variants of SEQ ID NO:45) are provided which are at least about 70% identical, at least about 80% identical, at least about 90% identical, at least about 95% identical, at least about 98% identical, at least about 99% identical, at least about 99.5% identical, or at least about 99.9% to SEQ ID NO:45. In some embodiments, variants of dCas9 (*e.g*., variants of SEQ ID NO:45) are provided having amino acid sequences which are shorter, or longer than SEQ ID NO:45, by about 5 amino acids, by about 10 amino acids, by about 15 amino acids, by about 20 amino acids, by about 25 amino acids, by about 30 amino acids, by about 40 amino acids, by about 50 amino acids, by about 75 amino acids, by about 100 amino acids, or more. In some embodiments, Cas9 "nickases" are provided which comprise a mutation which inactivates a single nuclease domain in Cas9. Such nickases induce a single strand break in a target nucleic acid as opposed to a double strand break.

### Cas9

ATGGATAAGAAATACTCAATAGGCTTAGATATCGGCACAAATAGCGTCGGATGGGCGGTGATCACTGATGATTATAA
GGTTCCGTCTAAAAAGTTCAAGGTTCTGGGAAATACAGACCGCCACAGTATCAAAAAAAATCTTATAGGGGCTCTTT
TATTTGGCAGTGGAGAGACAGCGGAAGCGACTCGTCTCAAACGGACAGCTCGTAGAAGGTATACACGTCGGAAGAAT
CGTATTTGTTATCTACAGGAGATTTTTTCAAATGAGATGGCGAAAGTAGATGATAGTTTCTTTCATCGACTTGAAGA
GTCTTTTTTGGTGGAAGAAGACAAGAAGCATGAACGTCATCCTATTTTTGGAAATATAGTAGATGAAGTTGCTTATC
ATGAGAAATATCCAACTATCTATCATCTGCGAAAAAAATTGGCAGATTCTACTGATAAAGCGGATTTGCGCTTAATC
TATTTGGCCTTAGCGCATATGATTAAGTTTCGTGGTCATTTTTTGATTGAGGGAGATTTAAATCCTGATAATAGTGA
TGTGGACAAACTATTTATCCAGTTGGTACAAATCTACAATCAATTATTTGAAGAAAACCCTATTAACGCAAGTAGAG
TAGATGCTAAAGCGATTCTTTCTGCACGATTGAGTAAATCAAGACGATTAGAAAATCTCATTGCTCAGCTCCCCGGT
GAGAAGAGAAATGGCTTGTTTGGGAATCTCATTGCTTTGTCATTGGGATTGACCCCTAATTTTAAATCAAATTTTGA
TTTGGCAGAAGATGCTAAATTACAGCTTTCAAAAGATACTTACGATGATGATTTAGATAATTTATTGGCGCAAATTG
GAGATCAATATGCTGATTTGTTTTTGGCAGCTAAGAATTTATCAGATGCTATTTTACTTTCAGATATCCTAAGAGTA
AATAGTGAAATAACTAAGGCTCCCCTATCAGCTTCAATGATTAAGCGCTACGATGAACATCATCAAGACTTGACTCT
TTTAAAAGCTTTAGTTCGACAACAACTTCCAGAAAAGTATAAAGAAATCTTTTTTGATCAATCAAAAAACGGATATG
CAGGTTATATTGATGGGGGAGCTAGCCAAGAAGAATTTTATAAATTTATCAAACCAATTTTAGAAAAAATGGATGGT
ACTGAGGAATTATTGGTGAAACTAAATCGTGAAGATTTGCTGCGCAAGCAACGGACCTTTGACAACGGCTCTATTCC
CCATCAAATTCACTTGGGTGAGCTGCATGCTATTTTGAGAAGACAAGAAGACTTTTATCCATTTTTAAAAGACAATC
GTGAGAAGATTGAAAAAATCTTGACTTTTCGAATTCCTTATTATGTTGGTCCATTGGCGCGTGGCAATAGTCGTTTT
GCATGGATGACTCGGAAGTCTGAAGAAACAATTACCCCATGGAATTTTGAAGAAGTTGTCGATAAAGGTGCTTCAGC
TCAATCATTTATTGAACGCATGACAAACTTTGATAAAAATCTTCCAAATGAAAAAGTACTACCAAAACATAGTTTGC
TTTATGAGTATTTTACGGTTTATAACGAATTGACAAAGGTCAAATATGTTACTGAGGGAATGCGAAAACCAGCATTT
CTTTCAGGTGAACAGAAGAAAGCCATTGTTGATTTACTCTTCAAAACAAATCGAAAAGTAACCGTTAAGCAATTAAA
AGAAGATTATTTCAAAAAAATAGAATGTTTTGATAGTGTTGAAATTTCAGGAGTTGAAGATAGATTTAATGCTTCAT
TAGGCGCCTACCATGATTTGCTAAAAATTATTAAAGATAAAGATTTTTTGGATAATGAAGAAAATGAAGATATCTTA
GAGGATATTGTTTTAACATTGACCTTATTTGAAGATAGGGGGATGATTGAGGAAAGACTTAAAACATATGCTCACCT
CTTTGATGATAAGGTGATGAAACAGCTTAAACGTCGCCGTTATACTGGTTGGGGACGTTTGTCTCGAAAATTGATTA
ATGGTATTAGGGATAAGCAATCTGGCAAAACAATATTAGATTTTTTGAAATCAGATGGTTTTGCCAATCGCAATTTT

ATGCAGCTGATCCATGATGATAGTTTGACATTTAAAGAAGATATTCAAAAAGCACAGGTGTCTGGACAAGGCCATAG
TTTACATGAACAGATTGCTAACTTAGCTGGCAGTCCTGCTATTAAAAAAGGTATTTTACAGACTGTAAAAATTGTTG
ATGAACTGGTCAAAGTAATGGGGCATAAGCCAGAAAATATCGTTATTGAAATGGCACGTGAAAATCAGACAACTCAA
AAGGGCCAGAAAAATTCGCGAGAGCGTATGAAACGAATCGAAGAAGGTATCAAAGAATTAGGAAGTCAGATTCTTAA
AGAGCATCCTGTTGAAAATACTCAATTGCAAAATGAAAAGCTCTATCTCTATTATCTACAAAATGGAAGAGACATGT
ATGTGGACCAAGAATTAGATATTAATCGTTTAAGTGATTATGATGTCGATCACATTGTTCCACAAAGTTTCATTAAA
GACGATTCAATAGACAATAAGGTACTAACGCGTTCTGATAAAAATCGTGGTAAATCGGATAACGTTCCAAGTGAAGA
AGTAGTCAAAAAGATGAAAAACTATTGGAGACAACTTCTAAACGCCAAGTTAATCACTCAACGTAAGTTTGATAATT
TAACGAAAGCTGAACGTGGAGGTTTGAGTGAACTTGATAAAGCTGGTTTTATCAAACGCCAATTGGTTGAAACTCGC
CAAATCACTAAGCATGTGGCACAAATTTTGGATAGTCGCATGAATACTAAATACGATGAAAATGATAAACTTATTCG
AGAGGTTAAAGTGATTACCTTAAAATCTAAATTAGTTTCTGACTTCCGAAAAGATTTCCAATTCTATAAAGTACGTG
AGATTAACAATTACCATCATGCCCATGATGCGTATCTAAATGCCGTCGTTGGAACTGCTTTGATTAAGAAATATCCA
AAACTTGAATCGGAGTTTGTCTATGGTGATTATAAAGTTTATGATGTTCGTAAAATGATTGCTAAGTCTGAGCAAGA
AATAGGCAAAGCAACCGCAAAATATTTCTTTTACTCTAATATCATGAACTTCTTCAAAACAGAAATTACACTTGCAA
ATGGAGAGATTCGCAAACGCCCTCTAATCGAAACTAATGGGGAAACTGGAGAAATTGTCTGGGATAAAGGGCGAGAT
TTTGCCACAGTGCGCAAAGTATTGTCCATGCCCCAAGTCAATATTGTCAAGAAAACAGAAGTACAGACAGGCGGATT
CTCCAAGGAGTCAATTTTACCAAAAAGAAATTCGGACAAGCTTATTGCTCGTAAAAAAGACTGGGATCCAAAAAAAT
ATGGTGGTTTTGATAGTCCAACGGTAGCTTATTCAGTCCTAGTGGTTGCTAAGGTGGAAAAAGGGAAATCGAAGAAG
TTAAAATCCGTTAAAGAGTTACTAGGGATCACAATTATGGAAAGAAGTTCCTTTGAAAAAAATCCGATTGACTTTTT
AGAAGCTAAAGGATATAAGGAAGTTAAAAAAGACTTAATCATTAAACTACCTAAATATAGTCTTTTTGAGTTAGAAA
ACGGTCGTAAACGGATGCTGGCTAGTGCCGGAGAATTACAAAAAGGAAATGAGCTGGCTCTGCCAAGCAAATATGTG
AATTTTTTATATTTAGCTAGTCATTATGAAAAGTTGAAGGGTAGTCCAGAAGATAACGAACAAAAACAATTGTTTGT
GGAGCAGCATAAGCATTATTTAGATGAGATTATTGAGCAAATCAGTGAATTTTCTAAGCGTGTTATTTTAGCAGATG
CCAATTTAGATAAAGTTCTTAGTGCATATAACAAACATAGAGACAAACCAATACGTGAACAAGCAGAAAATATTATT
CATTTATTTACGTTGACGAATCTTGGAGCTCCCGCTGCTTTTAAATATTTTGATACAACAATTGATCGTAAACGATA
TACGTCTACAAAAGAAGTTTTAGATGCCACTCTTATCCATCAATCCATCACTGGTCTTTATGAAACACGCATTGATT
TGAGTCAGCTAGGAGGTGACTGA (SEQ ID NO:43)

MDKKYSIGLAIGTNSVGWAVITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILSARLSKSRRLENLIAQLPG
EKKNGLFGNLIALSLGLTPNFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTFRIPYYVGPLARGNSRF
AWMTRKSEETITPWNFEEVVDKGASAQSFIERMTNFDKNLPNEKVLPKHSLLYEYFTVYNELTKVKYVTEGMRKPAF
LSGEQKKAIVDLLFKTNRKVTVKQLKEDYFKKIECFDSVEISGVEDRFNASLGTYHDLLKIIKDKDFLDNEENEDIL
EDIVLTLTLFEDREMIEERLKTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDHIVPQSFL
KDDSIDNKVLTRSDKNRGKSDNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHHAHDAYLNAVVGTALIKKY
PKLESEFVYGDYKVYDVRKMIAKSEQEIGKATAKYFFYSNIMNFFKTEITLANGEIRKRPLIETNGETGEIVWDKGR
DFATVRKVLSMPQVNIVKKTEVQTGGFSKESILPKRNSDKLIARKKDWDPKKYGGFDSPTVAYSVLVVAKVEKGKSK
KLKSVKELLGITIMERSSFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLSAYNKHRDKPIREQAENI
IHLFTLTNLGAPAAFKYFDTTIDRKRYTSTKEVLDATLIHQSITGLYETRIDLSQLGGD (SEQ ID NO:44)

(single underline: HNH domain; double underline: RuvC domain)

### dCas9 (D10A and H840A):

MDKKYSIGLAIGTNSVGWAVITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILSARLSKSRRLENLIAQLPG
EKKNGLFGNLIALSLGLTPNFKSNFDLAEDAKLQLSKDTYDDDLDNLLAQIGDQYADLFLAAKNLSDAILLSDILRV

NTEITKAPLSASMIKRYDEHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTFRIPYYVGPLARGNSRF
AWMTRKSEETITPWNFEEVVDKGASAQSFIERMTNFDKNLPNEKVLPKHSLLYEYFTVYNELTKVKYVTEGMRKPAF
LSGEQKKAIVDLLFKTNRKVTVKQLKEDYFKKIECFDSVEISGVEDRFNASLGTYHDLLKIIKDKDFLDNEENEDIL
EDIVLTLTLFEDREMIEERLKTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDAIVPQSFL
KDDSIDNKVLTRSDKNRGKSDNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHHAHDAYLNAVVGTALIKKY
PKLESEFVYGDYKVYDVRKMIAKSEQEIGKATAKYFFYSNIMNFFKTEITLANGEIRKRPLIETNGETGEIVWDKGR
DFATVRKVLSMPQVNIVKKTEVQTGGFSKESILPKRNSDKLIARKKDWDPKKYGGFDSPTVAYSVLVVAKVEKGKSK
KLKSVKELLGITIMERSSFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLSAYNKHRDKPIREQAENI
IHLFTLTNLGAPAAFKYFDTTIDRKRYTSTKEVLDATLIHQSITGLYETRIDLSQLGGD (SEQ ID NO:45)

(single underline: HNH domain; double underline: RuvC domain)

### Cas9 nickase (D10A):

MDKKYSIGLAIGTNSVGWAVITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILSARLSKSRRLENLIAQLPG
EKKNGLFGNLIALSLGLTPNFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTFRIPYYVGPLARGNSRF
AWMTRKSEETITPWNFEEVVDKGASAQSFIERMTNFDKNLPNEKVLPKHSLLYEYFTVYNELTKVKYVTEGMRKPAF
LSGEQKKAIVDLLFKTNRKVTVKQLKEDYFKKIECFDSVEISGVEDRFNASLGTYHDLLKIIKDKDFLDNEENEDIL
EDIVLTLTLFEDREMIEERLKTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDHIVPQSFL
KDDSIDNKVLTRSDKNRGKSDNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHHAHDAYLNAVVGTALIKKY
PKLESEFVYGDYKVYDVRKMIAKSEQEIGKATAKYFFYSNIMNFFKTEITLANGEIRKRPLIETNGETGEIVWDKGR
DFATVRKVLSMPQVNIVKKTEVQTGGFSKESILPKRNSDKLIARKKDWDPKKYGGFDSPTVAYSVLVVAKVEKGKSK
KLKSVKELLGITIMERSSFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLSAYNKHRDKPIREQAENI
IHLFTLTNLGAPAAFKYFDTTIDRKRYTSTKEVLDATLIHQSITGLYETRIDLSQLGGD (SEQ ID NO:46)

In some embodiments, fusion proteins comprising a Cas9 protein are provided for use in any of the compositions and methods described herein. In some embodiments, the fusion protein comprises a dCas9 protein (*e.g.,* as described herein). In some embodiments, the fusion protein comprises a linker (*e.g.,* as described herein) between dCas9 and one or more domains (*e.g.*, enzymatic domains). In some embodiments, the fusion protein comprises dCas9 and a transcriptional activator domain, a transcriptional repressor domain, a recombinase domain, a gene editing domain (*e.g.,* a deaminase domain), or an epigenetic modifier domain.

In some embodiments, the general architecture of exemplary fusion proteins provided herein comprises the structure:
[NH₂]-[enzymatic domain]-[dCas9]-[COOH] or
[NH₂]- [dCas9] - [enzymatic domain]-[COOH];
wherein NH₂ is the N-terminus of the fusion protein, COOH is the C-terminus of the fusion protein, and the enzymatic domain comprises a nuclease domain (*e.g*., FokI), a recombinase catalytic domain (*e.g*., Hin, Gin, or Tn3 recombinase domains), a nucleic acid-editing domain (*e.g.,* a deaminase domain), a transcriptional activator domain (*e.g.,* VP64, p65), a transcriptional repressor domain (*e.g.,* KRAB, SID), or an epigenetic modifier (*e.g.,* LSD1 histone demethylase, TET1 hydroxylase).

Additional features may be present, for example, one or more linker sequences between certain domains. Other exemplary features that may be present are localization sequences, such as nuclear localization sequences (NLS; *e.g.*, MAPKKKRKVGIHRGVP (SEQ ID NO:47)); cytoplasmic localization sequences; export sequences, such as nuclear export sequences; or other localization sequences, as well as sequence tags that are useful for solubilization, purification, or detection of the fusion proteins. Suitable localization signal sequences and sequences of protein tags are provided herein and are known in the art, and include, but are not limited to, biotin carboxylase carrier protein (BCCP) tags, myc-tags, calmodulin-tags, FLAG-tags (*e.g.*, 3xFLAG TAG: MDYKDHDGDYKDHDIDYKDDDDK (SEQ ID NO:48)), hemagglutinin (HA) tags, polyhistidine tags, also referred to as histidine tags or His-tags, maltose binding protein (MBP)-tags, nus-tags, glutathione-S-transferase (GST) tags, green fluorescent protein (GFP) tags, thioredoxin-tags, S-tags, Softags (*e.g.*, Softag 1, Softag 3), strep-tags , biotin ligase tags, FlAsH tags, V5 tags, and SBP-tags. Additional suitable sequences will be apparent to those of skill in the art.

In some embodiments, the enzymatic domain comprises a nuclease or a catalytic domain thereof. For example, in some embodiments, the general architecture of exemplary ligand-dependent dCas9 fusion proteins with a nuclease domain comprises the structure:
[NH₂]-[NLS]-[dCas9]-[nuclease]-[COOH],
[NH₂]-[NLS]-[nuclease]-[dCas9]-[COOH],
[NH₂]-[dCas9]-[nuclease]-[COOH], or
[NH₂]-[nuclease]-[dCas9]-[COOH];
wherein NLS is a nuclear localization signal, NH₂ is the N-terminus of the fusion protein, and COOH is the C-terminus of the fusion protein. In some embodiments, a linker is inserted between the dCas9 and the nuclease domain. In some embodiments, a linker is inserted between the NLS and the nuclease and/or dCas9 domain. In some embodiments, the NLS is located C-terminal of the nuclease and/or the dCas9 domain. In some embodiments, the NLS is located between the nuclease and the dCas9 domain. Additional features, such as sequence tags, may also be present. In some aspects, the nuclease domain is a nuclease requiring dimerization (*e.g*., the coming together of two monomers of the nuclease) in order to cleave a target nucleic acid (*e.g.,* DNA). In some embodiments, the nuclease domain is a monomer of the FokI DNA cleavage domain. The FokI DNA cleavage domain is known, and in some aspects corresponds to amino acids 388-583 of FokI (NCBI accession number J04623). In some embodiments, the FokI DNA cleavage domain corresponds to amino acids 300-583, 320-583, 340-583, or 360-583 of FokI. See also Wah et al., "Structure of FokI has implications for DNA cleavage" Proc. Natl. Acad. Sci. USA. 1998; 1;95(18):10564-9; Li et al., "TAL nucleases (TALNs): hybrid proteins composed of TAL effectors and FokI DNA-cleavage domain" Nucleic Acids Res. 2011; 39(1):359-72; Kim et al., "Hybrid restriction enzymes: zinc finger fusions to Fok I cleavage domain" Proc. Natl Acad. Sci. USA. 1996; 93:1156-1160; the entire contents of each are herein incorporated by reference). In some embodiments, the FokI DNA cleavage domain corresponds to, or comprises in part or whole, the amino acid sequence set forth as SEQ ID NO:49. In some embodiments, the FokI DNA cleavage domain is a variant of FokI (*e.g.,* a variant of SEQ ID NO:49), as described herein. Other exemplary compositions and methods of using dCas9-nuclease fusion proteins can be found in U.S. patent application U.S.S.N 14/320,498; titled "Cas9-FokI fusion Proteins and Uses Thereof," filed June 30, 2014; the entire contents of which are incorporated herein by reference.

### FokI nuclease domain:

GSQLVKSELEEKKSELRHKLKYVPHEYIELIEIARNSTQDRILEMKVMEFFMKVYGYRGKHLGGSRKPDGAIYTVGS
PIDYGVIVDTKAYSGGYNLPIGQADEMQRYVEENQTRNKHINPNEWWKVYPSSVTEFKFLFVSGHFKGNYKAQLTRL
NHITNCNGAVLSVEELLIGGEMIKAGTLTLEEVRRKFNNGEINF (SEQ ID NO:49)

### fCas9 (e.g., dCas9-NLS-GGS3linker-FokI):

ATGGATAAAAAGTATTCTATTGGTTTAGCTATCGGCACTAATTCCGTTGGATGGGCTGTCATAACCGATGAATACAA
AGTACCTTCAAAGAAATTTAAGGTGTTGGGGAACACAGACCGTCATTCGATTAAAAAGAATCTTATCGGTGCCCTCC

TATTCGATAGTGGCGAAACGGCAGAGGCGACTCGCCTGAAACGAACCGCTCGGAGAAGGTATACACGTCGCAAGAAC
CGAATATGTTACTTACAAGAAATTTTTAGCAATGAGATGGCCAAAGTTGACGATTCTTTCTTTCACCGTTTGGAAGA
GTCCTTCCTTGTCGAAGAGGACAAGAAACATGAACGGCACCCCATCTTTGGAAACATAGTAGATGAGGTGGCATATC
ATGAAAAGTACCCAACGATTTATCACCTCAGAAAAAAGCTAGTTGACTCAACTGATAAAGCGGACCTGAGGTTAATC
TACTTGGCTCTTGCCCATATGATAAAGTTCCGTGGGCACTTTCTCATTGAGGGTGATCTAAATCCGGACAACTCGGA
TGTCGACAAACTGTTCATCCAGTTAGTACAAACCTATAATCAGTTGTTTGAAGAGAACCCTATAAATGCAAGTGGCG
TGGATGCGAAGGCTATTCTTAGCGCCCGCCTCTCTAAATCCCGACGGCTAGAAAACCTGATCGCACAATTACCCGGA
GAGAAGAAAAATGGGTTGTTCGGTAACCTTATAGCGCTCTCACTAGGCCTGACACCAAATTTTAAGTCGAACTTCGA
CTTAGCTGAAGATGCCAAATTGCAGCTTAGTAAGGACACGTACGATGACGATCTCGACAATCTACTGGCACAAATTG
GAGATCAGTATGCGGACTTATTTTTGGCTGCCAAAAACCTTAGCGATGCAATCCTCCTATCTGACATACTGAGAGTT
AATACTGAGATTACCAAGGCGCCGTTATCCGCTTCAATGATCAAAAGGTACGATGAACATCACCAAGACTTGACACT
TCTCAAGGCCCTAGTCCGTCAGCAACTGCCTGAGAAATATAAGGAAATATTCTTTGATCAGTCGAAAAACGGGTACG
CAGGTTATATTGACGGCGGAGCGAGTCAAGAGGAATTCTACAAGTTTATCAAACCCATATTAGAGAAGATGGATGGG
ACGGAAGAGTTGCTTGTAAAACTCAATCGCGAAGATCTACTGCGAAAGCAGCGGACTTTCGACAACGGTAGCATTCC
ACATCAAATCCACTTAGGCGAATTGCATGCTATACTTAGAAGGCAGGAGGATTTTTATCCGTTCCTCAAAGACAATC
GTGAAAAGATTGAGAAAATCCTAACCTTTCGCATACCTTACTATGTGGGACCCCTGGCCCGAGGGAACTCTCGGTTC
GCATGGATGACAAGAAAGTCCGAAGAAACGATTACTCCATGGAATTTTGAGGAAGTTGTCGATAAAGGTGCGTCAGC
TCAATCGTTCATCGAGAGGATGACCAACTTTGACAAGAATTTACCGAACGAAAAAGTATTGCCTAAGCACAGTTTAC
TTTACGAGTATTTCACAGTGTACAATGAACTCACGAAAGTTAAGTATGTCACTGAGGGCATGCGTAAACCCGCCTTT
CTAAGCGGAGAACAGAAGAAAGCAATAGTAGATCTGTTATTCAAGACCAACCGCAAAGTGACAGTTAAGCAATTGAA
AGAGGACTACTTTAAGAAAATTGAATGCTTCGATTCTGTCGAGATCTCCGGGGTAGAAGATCGATTTAATGCGTCAC
TTGGTACGTATCATGACCTCCTAAAGATAATTAAAGATAAGGACTTCCTGGATAACGAAGAGAATGAAGATATCTTA
GAAGATATAGTGTTGACTCTTACCCTCTTTGAAGATCGGGAAATGATTGAGGAAAGACTAAAAACATACGCTCACCT
GTTCGACGATAAGGTTATGAAACAGTTAAAGAGGCGTCGCTATACGGGCTGGGGACGATTGTCGCGGAAACTTATCA
ACGGGATAAGAGACAAGCAAAGTGGTAAAACTATTCTCGATTTTCTAAAGAGCGACGGCTTCGCCAATAGGAACTTT
ATGCAGCTGATCCATGATGACTCTTTAACCTTCAAAGAGGATATACAAAAGGCACAGGTTTCCGGACAAGGGGACTC
ATTGCACGAACATATTGCGAATCTTGCTGGTTCGCCAGCCATCAAAAAGGGCATACTCCAGACAGTCAAAGTAGTGG
ATGAGCTAGTTAAGGTCATGGGACGTCACAAACCGGAAAACATTGTAATCGAGATGGCACGCGAAAATCAAACGACT
CAGAAGGGGCAAAAAAACAGTCGAGAGCGGATGAAGAGAATAGAAGAGGGTATTAAAGAACTGGGCAGCCAGATCTT
AAAGGAGCATCCTGTGGAAAATACCCAATTGCAGAACGAGAAACTTTACCTCTATTACCTACAAAATGGAAGGGACA
TGTATGTTGATCAGGAACTGGACATAAACCGTTTATCTGATTACGACGTCGATGCCATTGTACCCCAATCCTTTTTG
AAGGACGATTCAATCGACAATAAAGTGCTTACACGCTCGGATAAGAACCGAGGGAAAAGTGACAATGTTCCAAGCGA
GGAAGTCGTAAAGAAAATGAAGAACTATTGGCGGCAGCTCCTAAATGCGAAACTGATAACGCAAAGAAAGTTCGATA
ACTTAACTAAAGCTGAGAGGGGTGGCTTGTCTGAACTTGACAAGGCCGGATTTATTAAACGTCAGCTCGTGGAAACC
CGCCAAATCACAAAGCATGTTGCACAGATACTAGATTCCCGAATGAATACGAAATACGACGAGAACGATAAGCTGAT
TCGGGAAGTCAAAGTAATCACTTTAAAGTCAAAATTGGTGTCGGACTTCAGAAAGGATTTTCAATTCTATAAAGTTA
GGGAGATAAATAACTACCACCATGCGCACGACGCTTATCTTAATGCCGTCGTAGGGACCGCACTCATTAAGAAATAC
CCGAAGCTAGAAAGTGAGTTTGTGTATGGTGATTACAAAGTTTATGACGTCCGTAAGATGATCGCGAAAAGCGAACA
GGAGATAGGCAAGGCTACAGCCAAATACTTCTTTTATTCTAACATTATGAATTTCTTTAAGACGGAAATCACTCTGG
CAAACGGAGAGATACGCAAACGACCTTTAATTGAAACCAATGGGGAGACAGGTGAAATCGTATGGGATAAGGGCCGG
GACTTCGCGACGGTGAGAAAAGTTTTGTCCATGCCCCAAGTCAACATAGTAAAGAAAACTGAGGTGCAGACCGGAGG
GTTTTCAAAGGAATCGATTCTTCCAAAAAGGAATAGTGATAAGCTCATCGCTCGTAAAAAGGACTGGGACCCGAAAA
AGTACGGTGGCTTCGATAGCCCTACAGTTGCCTATTCTGTCCTAGTAGTGGCAAAAGTTGAGAAGGGAAAATCCAAG
AAACTGAAGTCAGTCAAAGAATTATTGGGGATAACGATTATGGAGCGCTCGTCTTTTGAAAAGAACCCCATCGACTT
CCTTGAGGCGAAAGGTTACAAGGAAGTAAAAAAGGATCTCATAATTAAACTACCAAAGTATAGTCTGTTTGAGTTAG
AAAATGGCCGAAAACGGATGTTGGCTAGCGCCGGAGAGCTTCAAAAGGGGAACGAACTCGCACTACCGTCTAAATAC
GTGAATTTCCTGTATTTAGCGTCCCATTACGAGAAGTTGAAAGGTTCACCTGAAGATAACGAACAGAAGCAACTTTT
TGTTGAGCAGCACAAACATTATCTCGACGAAATCATAGAGCAAATTTCGGAATTCAGTAAGAGAGTCATCCTAGCTG
ATGCCAATCTGGACAAAGTATTAAGCGCATACAACAAGCACAGGGATAAACCCATACGTGAGCAGGCGGAAAATATT
ATCCATTTGTTTACTCTTACCAACCTCGGCGCTCCAGCCGCATTCAAGTATTTTGACACAACGATAGATCGCAAACG
ATACACTTCTACCAAGGAGGTGCTAGACGCGACACTGATTCACCAATCCATCACGGGATTATATGAAACTCGGATAG
ATTTGTCACAGCTTGGGGGTGACGGATCCCCCAAGAAGAAGAGGAAAGTCTCGAGCGACTACAAAGACCATGACGGT
GATTATAAAGATCATGACATCGATTACAAGGATGACGATGACAAGGCTGCAGGATCAGGTGGAAGTGGCGGCAGCGG
AGGTTCTGGATCCCAACTAGTCAAAAGTGAACTGGAGGAGAAGAAATCTGAACTTCGTCATAAATTGAAATATGTGC
CTCATGAATATATTGAATTAATTGAAATTGCCAGAAATTCCACTCAGGATAGAATTCTTGAAATGAAGGTAATGGAA
TTTTTTATGAAAGTTTATGGATATAGAGGTAAACATTTGGGTGGATCAAGGAAACCGGACGGAGCAATTTATACTGT
CGGATCTCCTATTGATTACGGTGTGATCGTGGATACTAAAGCTTATAGCGGAGGTTATAATCTGCCAATTGGCCAAG

CAGATGAAATGCAACGATATGTCGAAGAAAATCAAACACGAAACAAACATATCAACCCTAATGAATGGTGGAAAGTC
TATCCATCTTCTGTAACGGAATTTAAGTTTTTATTTGTGAGTGGTCACTTTAAAGGAAACTACAAAGCTCAGCTTAC
ACGATTAAATCATATCACTAATTGTAATGGAGCTGTTCTTAGTGTAGAAGAGCTTTTAATTGGTGGAGAAATGATTA
AAGCCGGCACATTAACCTTAGAGGAAGTCAGACGGAAATTTAATAACGGCGAGATAAACTTT (SEQ ID NO:50)

### fCas9 (e.g., NLS- dCas9-GGS3linker-FokI):

ATGGACTACAAAGACCATGACGGTGATTATAAAGATCATGACATCGATTACAAGGATGACGATGACAAGATGGCCCC
CAAGAAGAAGAGGAAGGTGGGCATTCACCGCGGGGTACCTATGGATAAAAAGTATTCTATTGGTTTAGCTATCGGCA
CTAATTCCGTTGGATGGGCTGTCATAACCGATGAATACAAAGTACCTTCAAAGAAATTTAAGGTGTTGGGGAACACA
GACCGTCATTCGATTAAAAAGAATCTTATCGGTGCCCTCCTATTCGATAGTGGCGAAACGGCAGAGGCGACTCGCCT
GAAACGAACCGCTCGGAGAAGGTATACACGTCGCAAGAACCGAATATGTTACTTACAAGAAATTTTTAGCAATGAGA
TGGCCAAAGTTGACGATTCTTTCTTTCACCGTTTGGAAGAGTCCTTCCTTGTCGAAGAGGACAAGAAACATGAACGG
CACCCCATCTTTGGAAACATAGTAGATGAGGTGGCATATCATGAAAAGTACCCAACGATTTATCACCTCAGAAAAAA
GCTAGTTGACTCAACTGATAAAGCGGACCTGAGGTTAATCTACTTGGCTCTTGCCCATATGATAAAGTTCCGTGGGC
ACTTTCTCATTGAGGGTGATCTAAATCCGGACAACTCGGATGTCGACAAACTGTTCATCCAGTTAGTACAAACCTAT
AATCAGTTGTTTGAAGAGAACCCTATAAATGCAAGTGGCGTGGATGCGAAGGCTATTCTTAGCGCCCGCCTCTCTAA
ATCCCGACGGCTAGAAAACCTGATCGCACAATTACCCGGAGAGAAGAAAAATGGGTTGTTCGGTAACCTTATAGCGC
TCTCACTAGGCCTGACACCAAATTTTAAGTCGAACTTCGACTTAGCTGAAGATGCCAAATTGCAGCTTAGTAAGGAC
ACGTACGATGACGATCTCGACAATCTACTGGCACAAATTGGAGATCAGTATGCGGACTTATTTTTGGCTGCCAAAAA
CCTTAGCGATGCAATCCTCCTATCTGACATACTGAGAGTTAATACTGAGATTACCAAGGCGCCGTTATCCGCTTCAA
TGATCAAAAGGTACGATGAACATCACCAAGACTTGACACTTCTCAAGGCCCTAGTCCGTCAGCAACTGCCTGAGAAA
TATAAGGAAATATTCTTTGATCAGTCGAAAAACGGGTACGCAGGTTATATTGACGGCGGAGCGAGTCAAGAGGAATT
CTACAAGTTTATCAAACCCATATTAGAGAAGATGGATGGGACGGAAGAGTTGCTTGTAAAACTCAATCGCGAAGATC
TACTGCGAAAGCAGCGGACTTTCGACAACGGTAGCATTCCACATCAAATCCACTTAGGCGAATTGCATGCTATACTT
AGAAGGCAGGAGGATTTTTATCCGTTCCTCAAAGACAATCGTGAAAAGATTGAGAAAATCCTAACCTTTCGCATACC
TTACTATGTGGGACCCCTGGCCCGAGGGAACTCTCGGTTCGCATGGATGACAAGAAAGTCCGAAGAAACGATTACTC
CATGGAATTTTGAGGAAGTTGTCGATAAAGGTGCGTCAGCTCAATCGTTCATCGAGAGGATGACCAACTTTGACAAG
AATTTACCGAACGAAAAAGTATTGCCTAAGCACAGTTTACTTTACGAGTATTTCACAGTGTACAATGAACTCACGAA
AGTTAAGTATGTCACTGAGGGCATGCGTAAACCCGCCTTTCTAAGCGGAGAACAGAAGAAAGCAATAGTAGATCTGT
TATTCAAGACCAACCGCAAAGTGACAGTTAAGCAATTGAAAGAGGACTACTTTAAGAAAATTGAATGCTTCGATTCT
GTCGAGATCTCCGGGGTAGAAGATCGATTTAATGCGTCACTTGGTACGTATCATGACCTCCTAAAGATAATTAAAGA
TAAGGACTTCCTGGATAACGAAGAGAATGAAGATATCTTAGAAGATATAGTGTTGACTCTTACCCTCTTTGAAGATC
GGGAAATGATTGAGGAAAGACTAAAAACATACGCTCACCTGTTCGACGATAAGGTTATGAAACAGTTAAAGAGGCGT
CGCTATACGGGCTGGGGACGATTGTCGCGGAAACTTATCAACGGGATAAGAGACAAGCAAAGTGGTAAAACTATTCT
CGATTTTCTAAAGAGCGACGGCTTCGCCAATAGGAACTTTATGCAGCTGATCCATGATGACTCTTTAACCTTCAAAG
AGGATATACAAAAGGCACAGGTTTCCGGACAAGGGGACTCATTGCACGAACATATTGCGAATCTTGCTGGTTCGCCA
GCCATCAAAAAGGGCATACTCCAGACAGTCAAAGTAGTGGATGAGCTAGTTAAGGTCATGGGACGTCACAAACCGGA
AAACATTGTAATCGAGATGGCACGCGAAAATCAAACGACTCAGAAGGGGCAAAAAAACAGTCGAGAGCGGATGAAGA
GAATAGAAGAGGGTATTAAAGAACTGGGCAGCCAGATCTTAAAGGAGCATCCTGTGGAAAATACCCAATTGCAGAAC
GAGAAACTTTACCTCTATTACCTACAAAATGGAAGGGACATGTATGTTGATCAGGAACTGGACATAAACCGTTTATC
TGATTACGACGTCGATGCCATTGTACCCCAATCCTTTTTGAAGGACGATTCAATCGACAATAAAGTGCTTACACGCT
CGGATAAGAACCGAGGGAAAAGTGACAATGTTCCAAGCGAGGAAGTCGTAAAGAAAATGAAGAACTATTGGCGGCAG
CTCCTAAATGCGAAACTGATAACGCAAAGAAAGTTCGATAACTTAACTAAAGCTGAGAGGGGTGGCTTGTCTGAACT
TGACAAGGCCGGATTTATTAAACGTCAGCTCGTGGAAACCCGCCAAATCACAAAGCATGTTGCACAGATACTAGATT
CCCGAATGAATACGAAATACGACGAGAACGATAAGCTGATTCGGGAAGTCAAAGTAATCACTTTAAAGTCAAAATTG
GTGTCGGACTTCAGAAAGGATTTTCAATTCTATAAAGTTAGGGAGATAAATAACTACCACCATGCGCACGACGCTTA
TCTTAATGCCGTCGTAGGGACCGCACTCATTAAGAAATACCCGAAGCTAGAAAGTGAGTTTGTGTATGGTGATTACA
AAGTTTATGACGTCCGTAAGATGATCGCGAAAAGCGAACAGGAGATAGGCAAGGCTACAGCCAAATACTTCTTTTAT
TCTAACATTATGAATTTCTTTAAGACGGAAATCACTCTGGCAAACGGAGAGATACGCAAACGACCTTTAATTGAAAC
CAATGGGGAGACAGGTGAAATCGTATGGGATAAGGGCCGGGACTTCGCGACGGTGAGAAAAGTTTTGTCCATGCCCC
AAGTCAACATAGTAAAGAAAACTGAGGTGCAGACCGGAGGGTTTTCAAAGGAATCGATTCTTCCAAAAAGGAATAGT
GATAAGCTCATCGCTCGTAAAAAGGACTGGGACCCGAAAAAGTACGGTGGCTTCGATAGCCCTACAGTTGCCTATTC
TGTCCTAGTAGTGGCAAAAGTTGAGAAGGGAAAATCCAAGAAACTGAAGTCAGTCAAAGAATTATTGGGGATAACGA
TTATGGAGCGCTCGTCTTTTGAAAAGAACCCCATCGACTTCCTTGAGGCGAAAGGTTACAAGGAAGTAAAAAAGGAT
CTCATAATTAAACTACCAAAGTATAGTCTGTTTGAGTTAGAAAATGGCCGAAAACGGATGTTGGCTAGCGCCGGAGA

GCTTCAAAAGGGGAACGAACTCGCACTACCGTCTAAATACGTGAATTTCCTGTATTTAGCGTCCCATTACGAGAAGT
TGAAAGGTTCACCTGAAGATAACGAACAGAAGCAACTTTTTGTTGAGCAGCACAAACATTATCTCGACGAAATCATA
GAGCAAATTTCGGAATTCAGTAAGAGAGTCATCCTAGCTGATGCCAATCTGGACAAAGTATTAAGCGCATACAACAA
GCACAGGGATAAACCCATACGTGAGCAGGCGGAAAATATTATCCATTTGTTTACTCTTACCAACCTCGGCGCTCCAG
CCGCATTCAAGTATTTTGACACAACGATAGATCGCAAACGATACACTTCTACCAAGGAGGTGCTAGACGCGACACTG
ATTCACCAATCCATCACGGGATTATATGAAACTCGGATAGATTTGTCACAGCTTGGGGGTGACTCAGGTGGAAGTGG
CGGCAGCGGAGGTTCTGGATCCCAACTAGTCAAAAGTGAACTGGAGGAGAAGAAATCTGAACTTCGTCATAAATTGA
AATATGTGCCTCATGAATATATTGAATTAATTGAAATTGCCAGAAATTCCACTCAGGATAGAATTCTTGAAATGAAG
GTAATGGAATTTTTTATGAAAGTTTATGGATATAGAGGTAAACATTTGGGTGGATCAAGGAAACCGGACGGAGCAAT
TTATACTGTCGGATCTCCTATTGATTACGGTGTGATCGTGGATACTAAAGCTTATAGCGGAGGTTATAATCTGCCAA
TTGGCCAAGCAGATGAAATGCAACGATATGTCGAAGAAAATCAAACACGAAACAAACATATCAACCCTAATGAATGG
TGGAAAGTCTATCCATCTTCTGTAACGGAATTTAAGTTTTTATTTGTGAGTGGTCACTTTAAAGGAAACTACAAAGC
TCAGCTTACACGATTAAATCATATCACTAATTGTAATGGAGCTGTTCTTAGTGTAGAAGAGCTTTTAATTGGTGGAG
AAATGATTAAAGCCGGCACATTAACCTTAGAGGAAGTCAGACGGAAATTTAATAACGGCGAGATAAACTTT (SEQ
ID NO:51)

### fCas9 (e.g., FokI-GGS3linker-dCas9-NLS):

ATGGGATCCCAACTAGTCAAAAGTGAACTGGAGGAGAAGAAATCTGAACTTCGTCATAAATTGAAATATGTGCCTCA
TGAATATATTGAATTAATTGAAATTGCCAGAAATTCCACTCAGGATAGAATTCTTGAAATGAAGGTAATGGAATTTT
TTATGAAAGTTTATGGATATAGAGGTAAACATTTGGGTGGATCAAGGAAACCGGACGGAGCAATTTATACTGTCGGA
TCTCCTATTGATTACGGTGTGATCGTGGATACTAAAGCTTATAGCGGAGGTTATAATCTGCCAATTGGCCAAGCAGA
TGAAATGCAACGATATGTCGAAGAAAATCAAACACGAAACAAACATATCAACCCTAATGAATGGTGGAAAGTCTATC
CATCTTCTGTAACGGAATTTAAGTTTTTATTTGTGAGTGGTCACTTTAAAGGAAACTACAAAGCTCAGCTTACACGA
TTAAATCATATCACTAATTGTAATGGAGCTGTTCTTAGTGTAGAAGAGCTTTTAATTGGTGGAGAAATGATTAAAGC
CGGCACATTAACCTTAGAGGAAGTCAGACGGAAATTTAATAACGGCGAGATAAACTTTGGCGGTAGTGGGGGATCTG
GGGGAAGTATGGATAAAAAGTATTCTATTGGTTTAGCTATCGGCACTAATTCCGTTGGATGGGCTGTCATAACCGAT
GAATACAAAGTACCTTCAAAGAAATTTAAGGTGTTGGGGAACACAGACCGTCATTCGATTAAAAAGAATCTTATCGG
TGCCCTCCTATTCGATAGTGGCGAAACGGCAGAGGCGACTCGCCTGAAACGAACCGCTCGGAGAAGGTATACACGTC
GCAAGAACCGAATATGTTACTTACAAGAAATTTTTAGCAATGAGATGGCCAAAGTTGACGATTCTTTCTTTCACCGT
TTGGAAGAGTCCTTCCTTGTCGAAGAGGACAAGAAACATGAACGGCACCCCATCTTTGGAAACATAGTAGATGAGGT
GGCATATCATGAAAAGTACCCAACGATTTATCACCTCAGAAAAAAGCTAGTTGACTCAACTGATAAAGCGGACCTGA
GGTTAATCTACTTGGCTCTTGCCCATATGATAAAGTTCCGTGGGCACTTTCTCATTGAGGGTGATCTAAATCCGGAC
AACTCGGATGTCGACAAACTGTTCATCCAGTTAGTACAAACCTATAATCAGTTGTTTGAAGAGAACCCTATAAATGC
AAGTGGCGTGGATGCGAAGGCTATTCTTAGCGCCCGCCTCTCTAAATCCCGACGGCTAGAAAACCTGATCGCACAAT
TACCCGGAGAGAAGAAAAATGGGTTGTTCGGTAACCTTATAGCGCTCTCACTAGGCCTGACACCAAATTTTAAGTCG
AACTTCGACTTAGCTGAAGATGCCAAATTGCAGCTTAGTAAGGACACGTACGATGACGATCTCGACAATCTACTGGC
ACAAATTGGAGATCAGTATGCGGACTTATTTTTGGCTGCCAAAAACCTTAGCGATGCAATCCTCCTATCTGACATAC
TGAGAGTTAATACTGAGATTACCAAGGCGCCGTTATCCGCTTCAATGATCAAAAGGTACGATGAACATCACCAAGAC
TTGACACTTCTCAAGGCCCTAGTCCGTCAGCAACTGCCTGAGAAATATAAGGAAATATTCTTTGATCAGTCGAAAAA
CGGGTACGCAGGTTATATTGACGGCGGAGCGAGTCAAGAGGAATTCTACAAGTTTATCAAACCCATATTAGAGAAGA
TGGATGGGACGGAAGAGTTGCTTGTAAAACTCAATCGCGAAGATCTACTGCGAAAGCAGCGGACTTTCGACAACGGT
AGCATTCCACATCAAATCCACTTAGGCGAATTGCATGCTATACTTAGAAGGCAGGAGGATTTTTATCCGTTCCTCAA
AGACAATCGTGAAAAGATTGAGAAAATCCTAACCTTTCGCATACCTTACTATGTGGGACCCCTGGCCCGAGGGAACT
CTCGGTTCGCATGGATGACAAGAAAGTCCGAAGAAACGATTACTCCATGGAATTTTGAGGAAGTTGTCGATAAAGGT
GCGTCAGCTCAATCGTTCATCGAGAGGATGACCAACTTTGACAAGAATTTACCGAACGAAAAAGTATTGCCTAAGCA
CAGTTTACTTTACGAGTATTTCACAGTGTACAATGAACTCACGAAAGTTAAGTATGTCACTGAGGGCATGCGTAAAC
CCGCCTTTCTAAGCGGAGAACAGAAGAAAGCAATAGTAGATCTGTTATTCAAGACCAACCGCAAAGTGACAGTTAAG
CAATTGAAAGAGGACTACTTTAAGAAAATTGAATGCTTCGATTCTGTCGAGATCTCCGGGGTAGAAGATCGATTTAA
TGCGTCACTTGGTACGTATCATGACCTCCTAAAGATAATTAAAGATAAGGACTTCCTGGATAACGAAGAGAATGAAG
ATATCTTAGAAGATATAGTGTTGACTCTTACCCTCTTTGAAGATCGGGAAATGATTGAGGAAAGACTAAAAACATAC
GCTCACCTGTTCGACGATAAGGTTATGAAACAGTTAAAGAGGCGTCGCTATACGGGCTGGGGACGATTGTCGCGGAA
ACTTATCAACGGGATAAGAGACAAGCAAAGTGGTAAAACTATTCTCGATTTTCTAAAGAGCGACGGCTTCGCCAATA
GGAACTTTATGCAGCTGATCCATGATGACTCTTTAACCTTCAAAGAGGATATACAAAAGGCACAGGTTTCCGGACAA
GGGGACTCATTGCACGAACATATTGCGAATCTTGCTGGTTCGCCAGCCATCAAAAAGGGCATACTCCAGACAGTCAA
AGTAGTGGATGAGCTAGTTAAGGTCATGGGACGTCACAAACCGGAAAACATTGTAATCGAGATGGCACGCGAAAATC

AAACGACTCAGAAGGGGCAAAAAAACAGTCGAGAGCGGATGAAGAGAATAGAAGAGGGTATTAAAGAACTGGGCAGC
CAGATCTTAAAGGAGCATCCTGTGGAAAATACCCAATTGCAGAACGAGAAACTTTACCTCTATTACCTACAAAATGG
AAGGGACATGTATGTTGATCAGGAACTGGACATAAACCGTTTATCTGATTACGACGTCGATGCCATTGTACCCCAAT
CCTTTTTGAAGGACGATTCAATCGACAATAAAGTGCTTACACGCTCGGATAAGAACCGAGGGAAAAGTGACAATGTT
CCAAGCGAGGAAGTCGTAAAGAAAATGAAGAACTATTGGCGGCAGCTCCTAAATGCGAAACTGATAACGCAAAGAAA
GTTCGATAACTTAACTAAAGCTGAGAGGGGTGGCTTGTCTGAACTTGACAAGGCCGGATTTATTAAACGTCAGCTCG
TGGAAACCCGCCAAATCACAAAGCATGTTGCACAGATACTAGATTCCCGAATGAATACGAAATACGACGAGAACGAT
AAGCTGATTCGGGAAGTCAAAGTAATCACTTTAAAGTCAAAATTGGTGTCGGACTTCAGAAAGGATTTTCAATTCTA
TAAAGTTAGGGAGATAAATAACTACCACCATGCGCACGACGCTTATCTTAATGCCGTCGTAGGGACCGCACTCATTA
AGAAATACCCGAAGCTAGAAAGTGAGTTTGTGTATGGTGATTACAAAGTTTATGACGTCCGTAAGATGATCGCGAAA
AGCGAACAGGAGATAGGCAAGGCTACAGCCAAATACTTCTTTTATTCTAACATTATGAATTTCTTTAAGACGGAAAT
CACTCTGGCAAACGGAGAGATACGCAAACGACCTTTAATTGAAACCAATGGGGAGACAGGTGAAATCGTATGGGATA
AGGGCCGGGACTTCGCGACGGTGAGAAAAGTTTTGTCCATGCCCCAAGTCAACATAGTAAAGAAAACTGAGGTGCAG
ACCGGAGGGTTTTCAAAGGAATCGATTCTTCCAAAAAGGAATAGTGATAAGCTCATCGCTCGTAAAAAGGACTGGGA
CCCGAAAAAGTACGGTGGCTTCGATAGCCCTACAGTTGCCTATTCTGTCCTAGTAGTGGCAAAAGTTGAGAAGGGAA
AATCCAAGAAACTGAAGTCAGTCAAAGAATTATTGGGGATAACGATTATGGAGCGCTCGTCTTTTGAAAAGAACCCC
ATCGACTTCCTTGAGGCGAAAGGTTACAAGGAAGTAAAAAAGGATCTCATAATTAAACTACCAAAGTATAGTCTGTT
TGAGTTAGAAAATGGCCGAAAACGGATGTTGGCTAGCGCCGGAGAGCTTCAAAAGGGGAACGAACTCGCACTACCGT
CTAAATACGTGAATTTCCTGTATTTAGCGTCCCATTACGAGAAGTTGAAAGGTTCACCTGAAGATAACGAACAGAAG
CAACTTTTTGTTGAGCAGCACAAACATTATCTCGACGAAATCATAGAGCAAATTTCGGAATTCAGTAAGAGAGTCAT
CCTAGCTGATGCCAATCTGGACAAAGTATTAAGCGCATACAACAAGCACAGGGATAAACCCATACGTGAGCAGGCGG
AAAATATTATCCATTTGTTTACTCTTACCAACCTCGGCGCTCCAGCCGCATTCAAGTATTTTGACACAACGATAGAT
CGCAAACGATACACTTCTACCAAGGAGGTGCTAGACGCGACACTGATTCACCAATCCATCACGGGATTATATGAAAC
TCGGATAGATTTGTCACAGCTTGGGGGTGACGGATCCCCCAAGAAGAAGAGGAAAGTCTCGAGCGACTACAAAGACC
ATGACGGTGATTATAAAGATCATGACATCGATTACAAGGATGACGATGACAAGGCTGCAGGA (SEQ ID NO:52)

### fCas9 (e.g., NLS -FokI-GGS3linker-dCas9):

ATGGACTACAAAGACCATGACGGTGATTATAAAGATCATGACATCGATTACAAGGATGACGATGACAAGATGGCCCC
CAAGAAGAAGAGGAAGGTGGGCATTCACCGCGGGGTACCTGGAGGTTCTATGGGATCCCAACTAGTCAAAAGTGAAC
TGGAGGAGAAGAAATCTGAACTTCGTCATAAATTGAAATATGTGCCTCATGAATATATTGAATTAATTGAAATTGCC
AGAAATTCCACTCAGGATAGAATTCTTGAAATGAAGGTAATGGAATTTTTTATGAAAGTTTATGGATATAGAGGTAA
ACATTTGGGTGGATCAAGGAAACCGGACGGAGCAATTTATACTGTCGGATCTCCTATTGATTACGGTGTGATCGTGG
ATACTAAAGCTTATAGCGGAGGTTATAATCTGCCAATTGGCCAAGCAGATGAAATGCAACGATATGTCGAAGAAAAT
CAAACACGAAACAAACATATCAACCCTAATGAATGGTGGAAAGTCTATCCATCTTCTGTAACGGAATTTAAGTTTTT
ATTTGTGAGTGGTCACTTTAAAGGAAACTACAAAGCTCAGCTTACACGATTAAATCATATCACTAATTGTAATGGAG
CTGTTCTTAGTGTAGAAGAGCTTTTAATTGGTGGAGAAATGATTAAAGCCGGCACATTAACCTTAGAGGAAGTCAGA
CGGAAATTTAATAACGGCGAGATAAACTTTGGCGGTAGTGGGGGATCTGGGGGAAGTATGGATAAAAAGTATTCTAT
TGGTTTAGCTATCGGCACTAATTCCGTTGGATGGGCTGTCATAACCGATGAATACAAAGTACCTTCAAAGAAATTTA
AGGTGTTGGGGAACACAGACCGTCATTCGATTAAAAAGAATCTTATCGGTGCCCTCCTATTCGATAGTGGCGAAACG
GCAGAGGCGACTCGCCTGAAACGAACCGCTCGGAGAAGGTATACACGTCGCAAGAACCGAATATGTTACTTACAAGA
AATTTTTAGCAATGAGATGGCCAAAGTTGACGATTCTTTCTTTCACCGTTTGGAAGAGTCCTTCCTTGTCGAAGAGG
ACAAGAAACATGAACGGCACCCCATCTTTGGAAACATAGTAGATGAGGTGGCATATCATGAAAAGTACCCAACGATT
TATCACCTCAGAAAAAAGCTAGTTGACTCAACTGATAAAGCGGACCTGAGGTTAATCTACTTGGCTCTTGCCCATAT
GATAAAGTTCCGTGGGCACTTTCTCATTGAGGGTGATCTAAATCCGGACAACTCGGATGTCGACAAACTGTTCATCC
AGTTAGTACAAACCTATAATCAGTTGTTTGAAGAGAACCCTATAAATGCAAGTGGCGTGGATGCGAAGGCTATTCTT
AGCGCCCGCCTCTCTAAATCCCGACGGCTAGAAAACCTGATCGCACAATTACCCGGAGAGAAGAAAAATGGGTTGTT
CGGTAACCTTATAGCGCTCTCACTAGGCCTGACACCAAATTTTAAGTCGAACTTCGACTTAGCTGAAGATGCCAAAT
TGCAGCTTAGTAAGGACACGTACGATGACGATCTCGACAATCTACTGGCACAAATTGGAGATCAGTATGCGGACTTA
TTTTTGGCTGCCAAAAACCTTAGCGATGCAATCCTCCTATCTGACATACTGAGAGTTAATACTGAGATTACCAAGGC
GCCGTTATCCGCTTCAATGATCAAAAGGTACGATGAACATCACCAAGACTTGACACTTCTCAAGGCCCTAGTCCGTC
AGCAACTGCCTGAGAAATATAAGGAAATATTCTTTGATCAGTCGAAAAACGGGTACGCAGGTTATATTGACGGCGGA
GCGAGTCAAGAGGAATTCTACAAGTTTATCAAACCCATATTAGAGAAGATGGATGGGACGGAAGAGTTGCTTGTAAA
ACTCAATCGCGAAGATCTACTGCGAAAGCAGCGGACTTTCGACAACGGTAGCATTCCACATCAAATCCACTTAGGCG
AATTGCATGCTATACTTAGAAGGCAGGAGGATTTTTATCCGTTCCTCAAAGACAATCGTGAAAAGATTGAGAAAATC
CTAACCTTTCGCATACCTTACTATGTGGGACCCCTGGCCCGAGGGAACTCTCGGTTCGCATGGATGACAAGAAAGTC

CGAAGAAACGATTACTCCATGGAATTTTGAGGAAGTTGTCGATAAAGGTGCGTCAGCTCAATCGTTCATCGAGAGGA
TGACCAACTTTGACAAGAATTTACCGAACGAAAAAGTATTGCCTAAGCACAGTTTACTTTACGAGTATTTCACAGTG
TACAATGAACTCACGAAAGTTAAGTATGTCACTGAGGGCATGCGTAAACCCGCCTTTCTAAGCGGAGAACAGAAGAA
AGCAATAGTAGATCTGTTATTCAAGACCAACCGCAAAGTGACAGTTAAGCAATTGAAAGAGGACTACTTTAAGAAAA
TTGAATGCTTCGATTCTGTCGAGATCTCCGGGGTAGAAGATCGATTTAATGCGTCACTTGGTACGTATCATGACCTC
CTAAAGATAATTAAAGATAAGGACTTCCTGGATAACGAAGAGAATGAAGATATCTTAGAAGATATAGTGTTGACTCT
TACCCTCTTTGAAGATCGGGAAATGATTGAGGAAAGACTAAAAACATACGCTCACCTGTTCGACGATAAGGTTATGA
AACAGTTAAAGAGGCGTCGCTATACGGGCTGGGGACGATTGTCGCGGAAACTTATCAACGGGATAAGAGACAAGCAA
AGTGGTAAAACTATTCTCGATTTTCTAAAGAGCGACGGCTTCGCCAATAGGAACTTTATGCAGCTGATCCATGATGA
CTCTTTAACCTTCAAAGAGGATATACAAAAGGCACAGGTTTCCGGACAAGGGGACTCATTGCACGAACATATTGCGA
ATCTTGCTGGTTCGCCAGCCATCAAAAAGGGCATACTCCAGACAGTCAAAGTAGTGGATGAGCTAGTTAAGGTCATG
GGACGTCACAAACCGGAAAACATTGTAATCGAGATGGCACGCGAAAATCAAACGACTCAGAAGGGGCAAAAAAACAG
TCGAGAGCGGATGAAGAGAATAGAAGAGGGTATTAAAGAACTGGGCAGCCAGATCTTAAAGGAGCATCCTGTGGAAA
ATACCCAATTGCAGAACGAGAAACTTTACCTCTATTACCTACAAAATGGAAGGGACATGTATGTTGATCAGGAACTG
GACATAAACCGTTTATCTGATTACGACGTCGATGCCATTGTACCCCAATCCTTTTTGAAGGACGATTCAATCGACAA
TAAAGTGCTTACACGCTCGGATAAGAACCGAGGGAAAAGTGACAATGTTCCAAGCGAGGAAGTCGTAAAGAAAATGA
AGAACTATTGGCGGCAGCTCCTAAATGCGAAACTGATAACGCAAAGAAAGTTCGATAACTTAACTAAAGCTGAGAGG
GGTGGCTTGTCTGAACTTGACAAGGCCGGATTTATTAAACGTCAGCTCGTGGAAACCCGCCAAATCACAAAGCATGT
TGCACAGATACTAGATTCCCGAATGAATACGAAATACGACGAGAACGATAAGCTGATTCGGGAAGTCAAAGTAATCA
CTTTAAAGTCAAAATTGGTGTCGGACTTCAGAAAGGATTTTCAATTCTATAAAGTTAGGGAGATAAATAACTACCAC
CATGCGCACGACGCTTATCTTAATGCCGTCGTAGGGACCGCACTCATTAAGAAATACCCGAAGCTAGAAAGTGAGTT
TGTGTATGGTGATTACAAAGTTTATGACGTCCGTAAGATGATCGCGAAAAGCGAACAGGAGATAGGCAAGGCTACAG
CCAAATACTTCTTTTATTCTAACATTATGAATTTCTTTAAGACGGAAATCACTCTGGCAAACGGAGAGATACGCAAA
CGACCTTTAATTGAAACCAATGGGGAGACAGGTGAAATCGTATGGGATAAGGGCCGGGACTTCGCGACGGTGAGAAA
AGTTTTGTCCATGCCCCAAGTCAACATAGTAAAGAAAACTGAGGTGCAGACCGGAGGGTTTTCAAAGGAATCGATTC
TTCCAAAAAGGAATAGTGATAAGCTCATCGCTCGTAAAAAGGACTGGGACCCGAAAAAGTACGGTGGCTTCGATAGC
CCTACAGTTGCCTATTCTGTCCTAGTAGTGGCAAAAGTTGAGAAGGGAAAATCCAAGAAACTGAAGTCAGTCAAAGA
ATTATTGGGGATAACGATTATGGAGCGCTCGTCTTTTGAAAAGAACCCCATCGACTTCCTTGAGGCGAAAGGTTACA
AGGAAGTAAAAAAGGATCTCATAATTAAACTACCAAAGTATAGTCTGTTTGAGTTAGAAAATGGCCGAAAACGGATG
TTGGCTAGCGCCGGAGAGCTTCAAAAGGGGAACGAACTCGCACTACCGTCTAAATACGTGAATTTCCTGTATTTAGC
GTCCCATTACGAGAAGTTGAAAGGTTCACCTGAAGATAACGAACAGAAGCAACTTTTTGTTGAGCAGCACAAACATT
ATCTCGACGAAATCATAGAGCAAATTTCGGAATTCAGTAAGAGAGTCATCCTAGCTGATGCCAATCTGGACAAAGTA
TTAAGCGCATACAACAAGCACAGGGATAAACCCATACGTGAGCAGGCGGAAAATATTATCCATTTGTTTACTCTTAC
CAACCTCGGCGCTCCAGCCGCATTCAAGTATTTTGACACAACGATAGATCGCAAACGATACACTTCTACCAAGGAGG
TGCTAGACGCGACACTGATTCACCAATCCATCACGGGATTATATGAAACTCGGATAGATTTGTCACAGCTTGGGGGT
GAC (SEQ ID NO:53)

### fCas9:

ATGGACTACAAAGACCATGACGGTGATTATAAAGATCATGACATCGATTACAAGGATGACGATGACAAGATGGCCCC
CAAGAAGAAGAGGAAGGTGGGCATTCACCGCGGGGTACCTGGAGGTTCTGGATCCCAACTAGTCAAAAGTGAACTGG
AGGAGAAGAAATCTGAACTTCGTCATAAATTGAAATATGTGCCTCATGAATATATTGAATTAATTGAAATTGCCAGA
AATTCCACTCAGGATAGAATTCTTGAAATGAAGGTAATGGAATTTTTTATGAAAGTTTATGGATATAGAGGTAAACA
TTTGGGTGGATCAAGGAAACCGGACGGAGCAATTTATACTGTCGGATCTCCTATTGATTACGGTGTGATCGTGGATA
CTAAAGCTTATAGCGGAGGTTATAATCTGCCAATTGGCCAAGCAGATGAAATGCAACGATATGTCGAAGAAAATCAA
ACACGAAACAAACATATCAACCCTAATGAATGGTGGAAAGTCTATCCATCTTCTGTAACGGAATTTAAGTTTTTATT
TGTGAGTGGTCACTTTAAAGGAAACTACAAAGCTCAGCTTACACGATTAAATCATATCACTAATTGTAATGGAGCTG
TTCTTAGTGTAGAAGAGCTTTTAATTGGTGGAGAAATGATTAAAGCCGGCACATTAACCTTAGAGGAAGTCAGACGG
AAATTTAATAACGGCGAGATAAACTTTAGCGGCAGCGAGACTCCCGGGACCTCAGAGTCCGCCACACCCGAAAGTGA
TAAAAAGTATTCTATTGGTTTAGCTATCGGCACTAATTCCGTTGGATGGGCTGTCATAACCGATGAATACAAAGTAC
CTTCAAAGAAATTTAAGGTGTTGGGGAACACAGACCGTCATTCGATTAAAAAGAATCTTATCGGTGCCCTCCTATTC
GATAGTGGCGAAACGGCAGAGGCGACTCGCCTGAAACGAACCGCTCGGAGAAGGTATACACGTCGCAAGAACCGAAT
ATGTTACTTACAAGAAATTTTTAGCAATGAGATGGCCAAAGTTGACGATTCTTTCTTTCACCGTTTGGAAGAGTCCT
TCCTTGTCGAAGAGGACAAGAAACATGAACGGCACCCCATCTTTGGAAACATAGTAGATGAGGTGGCATATCATGAA
AAGTACCCAACGATTTATCACCTCAGAAAAAAGCTAGTTGACTCAACTGATAAAGCGGACCTGAGGTTAATCTACTT
GGCTCTTGCCCATATGATAAAGTTCCGTGGGCACTTTCTCATTGAGGGTGATCTAAATCCGGACAACTCGGATGTCG

ACAAACTGTTCATCCAGTTAGTACAAACCTATAATCAGTTGTTTGAAGAGAACCCTATAAATGCAAGTGGCGTGGAT
GCGAAGGCTATTCTTAGCGCCCGCCTCTCTAAATCCCGACGGCTAGAAAACCTGATCGCACAATTACCCGGAGAGAA
GAAAAATGGGTTGTTCGGTAACCTTATAGCGCTCTCACTAGGCCTGACACCAAATTTTAAGTCGAACTTCGACTTAG
CTGAAGATGCCAAATTGCAGCTTAGTAAGGACACGTACGATGACGATCTCGACAATCTACTGGCACAAATTGGAGAT
CAGTATGCGGACTTATTTTTGGCTGCCAAAAACCTTAGCGATGCAATCCTCCTATCTGACATACTGAGAGTTAATAC
TGAGATTACCAAGGCGCCGTTATCCGCTTCAATGATCAAAAGGTACGATGAACATCACCAAGACTTGACACTTCTCA
AGGCCCTAGTCCGTCAGCAACTGCCTGAGAAATATAAGGAAATATTCTTTGATCAGTCGAAAAACGGGTACGCAGGT
TATATTGACGGCGGAGCGAGTCAAGAGGAATTCTACAAGTTTATCAAACCCATATTAGAGAAGATGGATGGGACGGA
AGAGTTGCTTGTAAAACTCAATCGCGAAGATCTACTGCGAAAGCAGCGGACTTTCGACAACGGTAGCATTCCACATC
AAATCCACTTAGGCGAATTGCATGCTATACTTAGAAGGCAGGAGGATTTTTATCCGTTCCTCAAAGACAATCGTGAA
AAGATTGAGAAAATCCTAACCTTTCGCATACCTTACTATGTGGGACCCCTGGCCCGAGGGAACTCTCGGTTCGCATG
GATGACAAGAAAGTCCGAAGAAACGATTACTCCATGGAATTTTGAGGAAGTTGTCGATAAAGGTGCGTCAGCTCAAT
CGTTCATCGAGAGGATGACCAACTTTGACAAGAATTTACCGAACGAAAAAGTATTGCCTAAGCACAGTTTACTTTAC
GAGTATTTCACAGTGTACAATGAACTCACGAAAGTTAAGTATGTCACTGAGGGCATGCGTAAACCCGCCTTTCTAAG
CGGAGAACAGAAGAAAGCAATAGTAGATCTGTTATTCAAGACCAACCGCAAAGTGACAGTTAAGCAATTGAAAGAGG
ACTACTTTAAGAAAATTGAATGCTTCGATTCTGTCGAGATCTCCGGGGTAGAAGATCGATTTAATGCGTCACTTGGT
ACGTATCATGACCTCCTAAAGATAATTAAAGATAAGGACTTCCTGGATAACGAAGAGAATGAAGATATCTTAGAAGA
TATAGTGTTGACTCTTACCCTCTTTGAAGATCGGGAAATGATTGAGGAAAGACTAAAAACATACGCTCACCTGTTCG
ACGATAAGGTTATGAAACAGTTAAAGAGGCGTCGCTATACGGGCTGGGGACGATTGTCGCGGAAACTTATCAACGGG
ATAAGAGACAAGCAAAGTGGTAAAACTATTCTCGATTTTCTAAAGAGCGACGGCTTCGCCAATAGGAACTTTATGCA
GCTGATCCATGATGACTCTTTAACCTTCAAAGAGGATATACAAAAGGCACAGGTTTCCGGACAAGGGGACTCATTGC
ACGAACATATTGCGAATCTTGCTGGTTCGCCAGCCATCAAAAAGGGCATACTCCAGACAGTCAAAGTAGTGGATGAG
CTAGTTAAGGTCATGGGACGTCACAAACCGGAAAACATTGTAATCGAGATGGCACGCGAAAATCAAACGACTCAGAA
GGGGCAAAAAAACAGTCGAGAGCGGATGAAGAGAATAGAAGAGGGTATTAAAGAACTGGGCAGCCAGATCTTAAAGG
AGCATCCTGTGGAAAATACCCAATTGCAGAACGAGAAACTTTACCTCTATTACCTACAAAATGGAAGGGACATGTAT
GTTGATCAGGAACTGGACATAAACCGTTTATCTGATTACGACGTCGATGCCATTGTACCCCAATCCTTTTTGAAGGA
CGATTCAATCGACAATAAAGTGCTTACACGCTCGGATAAGAACCGAGGGAAAAGTGACAATGTTCCAAGCGAGGAAG
TCGTAAAGAAAATGAAGAACTATTGGCGGCAGCTCCTAAATGCGAAACTGATAACGCAAAGAAAGTTCGATAACTTA
ACTAAAGCTGAGAGGGGTGGCTTGTCTGAACTTGACAAGGCCGGATTTATTAAACGTCAGCTCGTGGAAACCCGCCA
AATCACAAAGCATGTTGCACAGATACTAGATTCCCGAATGAATACGAAATACGACGAGAACGATAAGCTGATTCGGG
AAGTCAAAGTAATCACTTTAAAGTCAAAATTGGTGTCGGACTTCAGAAAGGATTTTCAATTCTATAAAGTTAGGGAG
ATAAATAACTACCACCATGCGCACGACGCTTATCTTAATGCCGTCGTAGGGACCGCACTCATTAAGAAATACCCGAA
GCTAGAAAGTGAGTTTGTGTATGGTGATTACAAAGTTTATGACGTCCGTAAGATGATCGCGAAAAGCGAACAGGAGA
TAGGCAAGGCTACAGCCAAATACTTCTTTTATTCTAACATTATGAATTTCTTTAAGACGGAAATCACTCTGGCAAAC
GGAGAGATACGCAAACGACCTTTAATTGAAACCAATGGGGAGACAGGTGAAATCGTATGGGATAAGGGCCGGGACTT
CGCGACGGTGAGAAAAGTTTTGTCCATGCCCCAAGTCAACATAGTAAAGAAAACTGAGGTGCAGACCGGAGGGTTTT
CAAAGGAATCGATTCTTCCAAAAAGGAATAGTGATAAGCTCATCGCTCGTAAAAAGGACTGGGACCCGAAAAAGTAC
GGTGGCTTCGATAGCCCTACAGTTGCCTATTCTGTCCTAGTAGTGGCAAAAGTTGAGAAGGGAAAATCCAAGAAACT
GAAGTCAGTCAAAGAATTATTGGGGATAACGATTATGGAGCGCTCGTCTTTTGAAAAGAACCCCATCGACTTCCTTG
AGGCGAAAGGTTACAAGGAAGTAAAAAAGGATCTCATAATTAAACTACCAAAGTATAGTCTGTTTGAGTTAGAAAAT
GGCCGAAAACGGATGTTGGCTAGCGCCGGAGAGCTTCAAAAGGGGAACGAACTCGCACTACCGTCTAAATACGTGAA
TTTCCTGTATTTAGCGTCCCATTACGAGAAGTTGAAAGGTTCACCTGAAGATAACGAACAGAAGCAACTTTTTGTTG
AGCAGCACAAACATTATCTCGACGAAATCATAGAGCAAATTTCGGAATTCAGTAAGAGAGTCATCCTAGCTGATGCC
AATCTGGACAAAGTATTAAGCGCATACAACAAGCACAGGGATAAACCCATACGTGAGCAGGCGGAAAATATTATCCA
TTTGTTTACTCTTACCAACCTCGGCGCTCCAGCCGCATTCAAGTATTTTGACACAACGATAGATCGCAAACGATACA
CTTCTACCAAGGAGGTGCTAGACGCGACACTGATTCACCAATCCATCACGGGATTATATGAAACTCGGATAGATTTG
TCACAGCTTGGGGGTGAC (SEQ ID NO:54)

In some embodiments, the enzymatic domain comprises a recombinase or catalytic domain thereof. For example, in some embodiments, the general architecture of exemplary ligand-dependent dCas9 fusion proteins with a recombinase domain comprises the structure:
[NH₂]-[NLS]-[dCas9]-[recombinase]-[COOH],
[NH₂]-[NLS]-[ recombinase]-[dCas9]-[COOH],
[NH₂]-[dCas9]-[ recombinase]-[COOH], or
[NH₂]-[ recombinase]-[dCas9]-[COOH];
wherein NLS is a nuclear localization signal, NH₂ is the N-terminus of the fusion protein, and COOH is the C-terminus of the fusion protein. In some embodiments, a linker is inserted between the dCas9 and the recombinase domain. In some embodiments, a linker is inserted between the NLS and the recombinase and/or dCas9 domain. In some embodiments, the NLS is located C-terminal of the recombinase domain and/or the dCas9 domain. In some embodiments, the NLS is located between the recombinase domain and the dCas9 domain. Additional features, such as sequence tags, may also be present. By "catalytic domain of a recombinase," it is meant that a fusion protein includes a domain comprising an amino acid sequence of (*e.g.,* derived from) a recombinase, such that the domain is sufficient to induce recombination when contacted with a target nucleic acid (either alone or with additional factors including other recombinase catalytic domains which may or may not form part of the fusion protein). In some embodiments, a catalytic domain of a recombinase does not include the DNA binding domain of the recombinase. In some embodiments, the catalytic domain of a recombinase includes part or all of a recombinase, *e.g*., the catalytic domain may include a recombinase domain and a DNA binding domain, or parts thereof, or the catalytic domain may include a recombinase domain and a DNA binding domain that is mutated or truncated to abolish DNA binding activity. Recombinases and catalytic domains of recombinases are known to those of skill in the art, and include, for example, those described herein. In some embodiments, the catalytic domain is derived from any recombinase. In some embodiments, the recombinase catalytic domain is a catalytic domain of aTn3 resolvase, a Hin recombinase, or a Gin recombinase. In some embodiments, the catalytic domain comprises a Tn3 resolvase (*e.g.*, Stark Tn3 recombinase) that is encoded by a nucleotide sequence comprising, in part or in whole, SEQ ID NO: 55, as provided below. In some embodiments, a Tn3 catalytic domain is encoded by a variant of SEQ ID NO:55. In some embodiments, a Tn3 catalytic domain is encoded by a polynucleotide (or a variant thereof) that encodes the polypeptide corresponding to SEQ ID NO:56. In some embodiments, the catalytic domain comprises a Hin recombinase that is encoded by a nucleotide sequence comprising, in part or in whole, SEQ ID NO:57, as provided below. In some embodiments, a Hin catalytic domain is encoded by a variant of SEQ ID NO:57. In some embodiments, a Hin catalytic domain is encoded by a polynucleotide (or a variant thereof) that encodes the polypeptide corresponding to SEQ ID NO:58. In some embodiments, the catalytic domain comprises a Gin recombinase (*e.g.*, Gin beta recombinase) that is encoded by a nucleotide sequence comprising, in part or in whole, SEQ ID NO:59, as provided below. In some embodiments, a Gin catalytic domain is encoded by a variant of SEQ ID NO:59. In some embodiments, a Gin catalytic domain is encoded by a polynucleotide (or a variant thereof) that encodes the polypeptide corresponding to SEQ ID NO:60. Other exemplary compositions and methods of using dCas9-recombinase fusion proteins can be found in U.S. patent application U.S.S.N 14/320,467; titled "Cas9 Variants and Uses Thereof," filed June 30, 2014; the entire contents of which are incorporated herein by reference.

### Stark Tn3 recombinase (nucleotide: SEQ ID NO:55; amino acid: SEQ ID NO:56):

ATGGCCCTGTTTGGCTACGCACGCGTGTCTACCAGTCAACAGTCACTCGATTTGCAAGTGAGGGCTCTTAAAGATGC
CGGAGTGAAGGCAAACAGAATTTTTACTGATAAGGCCAGCGGAAGCAGCACAGACAGAGAGGGGCTGGATCTCCTGA
GAATGAAGGTAAAGGAGGGTGATGTGATCTTGGTCAAAAAATTGGATCGACTGGGGAGAGACACAGCTGATATGCTT
CAGCTTATTAAAGAGTTTGACGCTCAGGGTGTTGCCGTGAGGTTTATCGATGACGGCATCTCAACCGACTCCTACAT
TGGTCTTATGTTTGTGACAATTTTGTCCGCTGTGGCTCAGGCTGAGCGGAGAAGGATTCTCGAAAGGACGAATGAGG
GACGGCAAGCAGCTAAGTTGAAAGGTATCAAATTTGGCAGACGAAGG (SEQ ID NO:55)

MALFGYARVSTSQQSLDLQVRALKDAGVKANRIFTDKASGSSTDREGLDLLRMKVEGDVILVKKLDRLGRDTADML
QLIKEFDAQGVAVRFIDDGISTDSYIGLMFVTILSAVAQAERRRILERTNEGRQAAKLKGIKFGRRR (SEQ ID
NO:56)

### Hin Recombinase (nucleotide: SEQ ID NO:57; amino acid: SEQ ID NO:58):

ATGGCAACCATTGGCTACATAAGGGTGTCTACCATCGACCAAAATATCGACCTGCAGCGCAACGCTCTGACATCCGC
CAACTGCGATCGGATCTTCGAGGATAGGATCAGTGGCAAGATCGCCAACCGGCCCGGTCTGAAGCGGGCTCTGAAGT
ACGTGAATAAGGGCGATACTCTGGTTGTGTGGAAGTTGGATCGCTTGGGTAGATCAGTGAAGAATCTCGTAGCCCTG
ATAAGCGAGCTGCACGAGAGGGGTGCACATTTCCATTCTCTGACCGATTCCATCGATACGTCTAGCGCCATGGGCCG
ATTCTTCTTTTACGTCATGTCCGCCCTCGCTGAAATGGAGCGCGAACTTATTGTTGAACGGACTTTGGCTGGACTGG
CAGCGGCTAGAGCACAGGGCCGACTTGGA (SEQ ID NO:57)

MATIGYIRVSTIDQNIDLQRNALTSANCDRIFEDRISGKIANRPGLKRALKYVNKGDTLVVWKLDRLGRSVKNLVAL
ISELHERGAHFHSLTDSIDTSSAMGRFFFYVMSALAEMERELIVERTLAGLAAARAQGRLG (SEQ ID NO:58)

### Gin beta recombinase (nucleotide: SEQ ID NO: 59; amino acid: SEQ ID NO:60):

ATGCTCATTGGCTATGTAAGGGTCAGCACCAATGACCAAAACACAGACTTGCAACGCAATGCTTTGGTTTGCGCCGG

ATGTGAACAGATATTTGAAGATAAACTGAGCGGCACTCGGACAGACAGACCTGGGCTTAAGAGAGCACTGAAAAGAC
TGCAGAAGGGGGACACCCTGGTCGTCTGGAAACTGGATCGCCTCGGACGCAGCATGAAACATCTGATTAGCCTGGTT
GGTGAGCTTAGGGAGAGAGGAATCAACTTCAGAAGCCTGACCGACTCCATCGACACCAGTAGCCCCATGGGACGATT
CTTCTTCTATGTGATGGGAGCACTTGCTGAGATGGAAAGAGAGCTTATTATCGAAAGAACTATGGCTGGTATCGCTG
CTGCCCGGAACAAAGGCAGACGGTTCGGCAGACCGCCGAAGAGCGGC (SEQ ID NO:59)

MLIGYVRVSTNDQNTDLQRNALVCAGCEQIFEDKLSGTRTDRPGLKRALKRLQKGDTLVVWKLDRLGRSMKHLISLV
GELRERGINFRSLTDSIDTSSPMGRFFFYVMGALAEMERELIIERTMAGIAAARNKGRRFGRPPKSG (SEQ ID
NO:60)

In some embodiments, the enzymatic domain comprises a deaminase or a catalytic domain thereof. For example, in some embodiments, the general architecture of exemplary dCas9 fusion proteins with a deaminase enzyme or domain comprises the structure:
[NH₂]-[NLS]-[Cas9]-[deaminase]-[COOH],
[NH₂]-[NLS]-[deaminase]-[Cas9]-[COOH],
[NH₂]-[Cas9]-[deaminase]-[COOH], or
[NH₂]-[deaminase]-[Cas9]-[COOH]; wherein NLS is a nuclear localization signal, NH₂ is the N-terminus of the fusion protein, and COOH is the C-terminus of the fusion protein. In some embodiments, a linker is inserted between the dCas9 and the deaminase domain. In some embodiments, a linker is inserted between the NLS and the deaminase and/or dCas9 domain. In some embodiments, the NLS is located C-terminal of the deaminase and/or the dCas9 domain. In some embodiments, the NLS is located between the deaminase domain and the dCas9 domain. Additional features, such as sequence tags, may also be present. One exemplary suitable type of nucleic acid-editing enzymes and domains are cytosine deaminases, for example, of the apolipoprotein B mRNA-editing complex (APOBEC) family of cytosine deaminase enzymes, including activation-induced cytidine deaminase (AID) and apolipoprotein B editing complex 3 (APOBEC3) enzyme. Another exemplary suitable type of nucleic acid-editing enzyme and domain thereof suitable for use in the present invention include adenosine deaminases. For example, an ADAT family adenosine deaminase can be fused to a dCas9 domain. Some exemplary suitable nucleic-acid editing enzymes and domains, *e.g.*, deaminases and deaminase domains, that can be fused to dCas9 domains according to aspects of this disclosure are provided below. It will be understood that, in some embodiments, the active domain of the respective sequence can be used, *e.g.,* the domain without a localizing signal (nuclear localizing signal, without nuclear export signal, cytoplasmic localizing signal). Other exemplary compositions and methods of using dCas9-nuclease fusion proteins can be found in U.S. patent application U.S.S.N 14/325,815; titled "Fusions of Cas9 Domains and Nucleic Acid-Editing Domains," filed July 8, 2014; the entire contents of which are incorporated herein by reference.

### Human AID:



 (SEQ ID NO:61)

(underline: nuclear localization signal; double underline: nuclear export signal)

### Mouse AID:

MDSLLMKQKKFLYHFKNVRWAKGRHETYLCYVVKRRDSATSCSLDFGHLRNKSGCHVELLFLRYISDWDLDPGRCYR
VTWFTSWSPCYDCARHVAEFLRWNPNLSLRIFTARLYFCEDRKAEPEGLRRLHRAGVQIGIMTFKDYFYCWNTFVEN
RERTFKAWEGLHENSVRLTRQLRRILLPLYEVDDLRDAFRMLGF (SEQ ID NO:62)

(underline: nuclear localization signal; double underline: nuclear export signal)

### Dog AID:

MDSLLMKQRKFLYHFKNVRWAKGRHETYLCYVVKRRDSATSFSLDFGHLRNKSGCHVELLFLRYISDWDLDPGRCYR
VTWFTSWSPCYDCARHVADFLRGYPNLSLRIFAARLYFCEDRKAEPEGLRRLHRAGVQIAIMTFKDYFYCWNTFVEN
REKTFKAWEGLHENSVRLSRQLRRILLPLYEVDDLRDAFRTLGL (SEQ ID NO:63)

(underline: nuclear localization signal; double underline: nuclear export signal)

### Bovine AID:

MDSLLKKQRQFLYQFKNVRWAKGRHETYLCYVVKRRDSPTSFSLDFGHLRNKAGCHVELLFLRYISDWDLDPGRCYR
VTWFTSWSPCYDCARHVADFLRGYPNLSLRIFTARLYFCDKERKAEPEGLRRLHRAGVQIAIMTFKDYFYCWNTFVE
NHERTFKAWEGLHENSVRLSRQLRRILLPLYEVDDLRDAFRTLGL (SEQ ID NO:64)

(underline: nuclear localization signal; double underline: nuclear export signal)

### Mouse APOBEC-3:

MGPFCLGCSHRKCYSPIRNLISQETFKFHFKNLGYAKGRKDTFLCYEVTRKDCDSPVSLHHGVFKNKDNIHAEICFL
YWFHDKVLKVLSPREEFKITWYMSWSPCFECAEQIVRFLATHHNLSLDIFSSRLYNVQDPETQQNLCRLVQEGAQVA
AMDLYEFKKCWKKFVDNGGRRFRPWKRLLTNFRYQDSKLQEILRPCYIPVPSSSSSTLSNICLTKGLPETRFCVEGR
RMDPLSEEEFYSQFYNQRVKHLCYYHRMKPYLCYQLEQFNGQAPLKGCLLSEKGKQHAEILFLDKIRSMELSQVTIT
CYLTWSPCPNCAWQLAAFKRDRPDLILHIYTSRLYFHWKRPFQKGLCSLWQSGILVDVMDLPQFTDCWTNFVNPKRP
FWPWKGLEIISRRTQRRLRRIKESWGLQDLVNDFGNLQLGPPMS (SEQ ID NO:65)

(underline: nucleic acid editing domain)

### Rat APOBEC-3:

MGPFCLGCSHRKCYSPIRNLISQETFKFHFKNLRYAIDRKDTFLCYEVTRKDCDSPVSLHHGVFKNKDNIHAEICFL

YWFHDKVLKVLSPREEFKITWYMSWSPCFECAEQVLRFLATHHNLSLDIFSSRLYNIRDPENQQNLCRLVQEGAQVA
AMDLYEFKKCWKKFVDNGGRRFRPWKKLLTNFRYQDSKLQEILRPCYIPVPSSSSSTLSNICLTKGLPETRFCVERR
RVHLLSEEEFYSQFYNQRVKHLCYYHGVKPYLCYQLEQFNGQAPLKGCLLSEKGKQHAEILFLDKIRSMELSQVIIT
CYLTWSPCPNCAWQLAAFKRDRPDLILHIYTSRLYFHWKRPFQKGLCSLWQSGILVDVMDLPQFTDCWTNFVNPKRP
FWPWKGLEIISRRTQRRLHRIKESWGLQDLVNDFGNLQLGPPMS (SEQ ID NO:66)

(underline: nucleic acid editing domain)

### Rhesus macaque APOBEC-3G:

MVEPMDPRTFVSNFNNRPILSGLNTVWLCCEVKTKDPSGPPLDAKIFQGKVYSKAKYHPEMRFLRWFHKWRQLHHDQ
EYKVTWYVSWSPCTRCANSVATFLAKDPKVTLTIFVARLYYFWKPDYQQALRILCQKRGGPHATMKIMNYNEFQDCW
NKFVDGRGKPFKPRNNLPKHYTLLQATLGELLRHLMDPGTFTSNFNNKPWVSGQHETYLCYKVERLHNDTWVPLNQH
RGFLRNQAPNIHGFPKGRHAELCFLDLIPFWKLDGQQYRVTCFTSWSPCFSCAQEMAKFISNNEHVSLCIFAARIYD
DQGRYQEGLRALHRDGAKIAMMNYSEFEYCWDTFVDRQGRPFQPWDGLDEHSQALSGRLRAI (SEQ ID NO:67)
(bold italic: nucleic acid editing domain; underline: cytoplasmic localization signal)

### Chimpanzee APOBEC-3G:

MKPHFRNPVERMYQDTFSDNFYNRPILSHRNTVWLCYEVKTKGPSRPPLDAKIFRGQVYSKLKYHPEMRFFHWFSKW
RKLHRDQEYEVTWYISWSPCTKCTRDVATFLAEDPKVTLTIFVARLYYFWDPDYQEALRSLCQKRDGPRATMKIMNY
DEFQHCWSKFVYSQRELFEPWNNLPKYYILLHIMLGEILRHSMDPPTFTSNFNNELWVRGRHETYLCYEVERLHNDT
WVLLNQRRGFLCNQAPHKHGFLEGRHAELCFLDVIPFWKLDLHQDYRVTCFTSWSPCFSCAQEMAKFISNNKHVSLC
IFAARIYDDQGRCQEGLRTLAKAGAKISIMTYSEFKHCWDTFVDHQGCPFQPWDGLEEHSQALSGRLRAILQNQGN
(SEQ ID NO:68)

(underline: nucleic acid editing domain; double underline: cytoplasmic localization signal)

### Green monkey APOBEC-3G:

MNPQIRNMVEQMEPDIFVYYFNNRPILSGRNTVWLCYEVKTKDPSGPPLDANIFQGKLYPEAKDHPEMKFLHWFRKW
RQLHRDQEYEVTWYVSWSPCTRCANSVATFLAEDPKVTLTIFVARLYYFWKPDYQQALRILCQERGGPHATMKIMNY
NEFQHCWNEFVDGQGKPFKPRKNLPKHYTLLHATLGELLRHVMDPGTFTSNFNNKPWVSGQRETYLCYKVERSHNDT
WVLLNQHRGFLRNQAPDRHGFPKGRHAELCFLDLIPFWKLDDQQYRVTCFTSWSPCFSCAQKMAKFISNNKHVSLCI
FAARIYDDQGRCQEGLRTLHRDGAKIAVMNYSEFEYCWDTFVDRQGRPFQPWDGLDEHSQALSGRLRAI (SEQ ID
NO:69)

(underline: nucleic acid editing domain; double underline: cytoplasmic localization signal)

### Human APOBEC-3G:

MKPHFRNTVERMYRDTFSYNFYNRPILSRRNTVWLCYEVKTKGPSRPPLDAKIFRGQVYSELKYHPEMRFFHWFSKW
RKLHRDQEYEVTWYISWSPCTKCTRDMATFLAEDPKVTLTIFVARLYYFWDPDYQEALRSLCQKRDGPRATMKIMNY
DEFQHCWSKFVYSQRELFEPWNNLPKYYILLHIMLGEILRHSMDPPTFTFNFNNEPWVRGRHETYLCYEVERMHNDT
WVLLNQRRGFLCNQAPHKHGFLEGRHAELCFLDVIPFWKLDLDQDYRVTCFTSWSPCFSCAQEMAKFISKNKHVSLC
IFTARIYDDQGRCQEGLRTLAEAGAKISIMTYSEFKHCWDTFVDHQGCPFQPWDGLDEHSQDLSGRLRAILQNQEN
(SEQ ID NO:70)

(underline: nucleic acid editing domain; double underline: cytoplasmic localization signal)

### Human APOBEC-3F:

MKPHFRNTVERMYRDTFSYNFYNRPILSRRNTVWLCYEVKTKGPSRPRLDAKIFRGQVYSQPEHHAEMCFLSWFCGN
QLPAYKCFQITWFVSWTPCPDCVAKLAEFLAEHPNVTLTISAARLYYYWERDYRRALCRLSQAGARVKIMDDEEFAY
CWENFVYSEGQPFMPWYKFDDNYAFLHRTLKEILRNPMEAMYPHIFYFHFKNLRKAYGRNESWLCFTMEVVKHHSPV
SWKRGVFRNQVDPETHCHAERCFLSWFCDDILSPNTNYEVTWYTSWSPCPECAGEVAEFLARHSNVNLTIFTARLYY
FWDTDYQEGLRSLSQEGASVEIMGYKDFKYCWENFVYNDDEPFKPWKGLKYNFLFLDSKLQEILE (SEQ ID
NO:71)

(underline: nucleic acid editing domain)

### Human APOBEC-3B:

MNPQIRNPMERMYRDTFYDNFENEPILYGRSYTWLCYEVKIKRGRSNLLWDTGVFRGQVYFKPQYHAEMCFLSWFCG
NQLPAYKCFQITWFVSWTPCPDCVAKLAEFLSEHPNVTLTISAARLYYYWERDYRRALCRLSQAGARVTIMDYEEFA
YCWENFVYNEGQQFMPWYKFDENYAFLHRTLKEILRYLMDPDTFTFNFNNDPLVLRRRQTYLCYEVERLDNGTWVLM
DQHMGFLCNEAKNLLCGFYGRHAELRFLDLVPSLQLDPAQIYRVTWFISWSPCFSWGCAGEVRAFLQENTHVRLRIF
AARIYDYDPLYKEALQMLRDAGAQVSIMTYDEFEYCWDTFVYRQGCPFQPWDGLEEHSQALSGRLRAILQNQGN
(SEQ ID NO:72)

(underline: nucleic acid editing domain)

### Human APOBEC-3C:

MNPQIRNPMKAMYPGTFYFQFKNLWEANDRNETWLCFTVEGIKRRSVVSWKTGVFRNQVDSETHCHAERCFLSWFCD
DILSPNTKYQVTWYTSWSPCPDCAGEVAEFLARHSNVNLTIFTARLYYFQYPCYQEGLRSLSQEGVAVEIMDYEDFK
YCWENFVYNDNEPFKPWKGLKTNFRLLKRRLRESLQ (SEQ ID NO:73)

(underline: nucleic acid editing domain)

### Human APOBEC-3A:

MEASPASGPRHLMDPHIFTSNFNNGIGRHKTYLCYEVERLDNGTSVKMDQHRGFLHNQAKNLLCGFYGRHAELRFLD
LVPSLQLDPAQIYRVTWFISWSPCFSWGCAGEVRAFLQENTHVRLRIFAARIYDYDPLYKEALQMLRDAGAQVSIMT
YDEFKHCWDTFVDHQGCPFQPWDGLDEHSQALSGRLRAILQNQGN (SEQ ID NO:74)

(underline: nucleic acid editing domain)

### Human APOBEC-3H:

MALLTAETFRLQFNNKRRLRRPYYPRKALLCYQLTPQNGSTPTRGYFENKKKCHAEICFINEIKSMGLDETQCYQVT
CYLTWSPCSSCAWELVDFIKAHDHLNLGIFASRLYYHWCKPQQKGLRLLCGSQVPVEVMGFPKFADCWENFVDHEKP
LSFNPYKMLEELDKNSRAIKRRLERIKIPGVRAQGRYMDILCDAEV (SEQ ID NO:75)

(underline: nucleic acid editing domain)

### Human APOBEC-3D:

MNPQIRNPMERMYRDTFYDNFENEPILYGRSYTWLCYEVKIKRGRSNLLWDTGVFRGPVLPKRQSNHRQEVYFRFEN
HAEMCFLSWFCGNRLPANRRFQITWFVSWNPCLPCVVKVTKFLAEHPNVTLTISAARLYYYRDRDWRWVLLRLHKAG
ARVKIMDYEDFAYCWENFVCNEGQPFMPWYKFDDNYASLHRTLKEILRNPMEAMYPHIFYFHFKNLLKACGRNESWL
CFTMEVTKHHSAVFRKRGVFRNQVDPETHCHAERCFLSWFCDDILSPNTNYEVTWYTSWSPCPECAGEVAEFLARHS
NVNLTIFTARLCYFWDTDYQEGLCSLSQEGASVKIMGYKDFVSCWKNFVYSDDEPFKPWKGLQTNFRLLKRRLREIL

Q (SEQ ID NO:76)

(underline: nucleic acid editing domain)

### Human APOBEC-1:

MTSEKGPSTGDPTLRRRIEPWEFDVFYDPRELRKEACLLYEIKWGMSRKIWRSSGKNTTNHVEVNFIKKFTSERDFH
PSMSCSITWFLSWSPCWECSQAIREFLSRHPGVTLVIYVARLFWHMDQQNRQGLRDLVNSGVTIQIMRASEYYHCWR
NFVNYPPGDEAHWPQYPPLWMMLYALELHCIILSLPPCLKISRRWQNHLTFFRLHLQNCHYQTIPPHILLATGLIHP
SVAWR (SEQ ID NO:77)

### Mouse APOBEC-1:

MSSETGPVAVDPTLRRRIEPHEFEVFFDPRELRKETCLLYEINWGGRHSVWRHTSQNTSNHVEVNFLEKFTTERYFR
PNTRCSITWFLSWSPCGECSRAITEFLSRHPYVTLFIYIARLYHHTDQRNRQGLRDLISSGVTIQIMTEQEYCYCWR
NFVNYPPSNEAYWPRYPHLWVKLYVLELYCIILGLPPCLKILRRKQPQLTFFTITLQTCHYQRIPPHLLWATGLK
(SEQ ID NO:78)

### Rat APOBEC-1:




(SEQ ID NO:79)

### Human ADAT-2:

MEAKAAPKPAASGACSVSAEETEKWMEEAMHMAKEALENTEVPVGCLMVYNNEVVGKGRNEVNQTKNATRHAEMVAI
DQVLDWCRQSGKSPSEVFEHTVLYVTVEPCIMCAAALRLMKIPLVVYGCQNERFGGCGSVLNIASADLPNTGRPFQC
IPGYRAEEAVEMLKTFYKQENPNAPKSKVRKKECQKS (SEQ ID NO:80)

### Mouse ADAT-2:

MEEKVESTTTPDGPCVVSVQETEKWMEEAMRMAKEALENIEVPVGCLMVYNNEVVGKGRNEVNQTKNATRHAEMVAI
DQVLDWCHQHGQSPSTVFEHTVLYVTVEPCIMCAAALRLMKIPLVVYGCQNERFGGCGSVLNIASADLPNTGRPFQC
IPGYRAEEAVELLKTFYKQENPNAPKSKVRKKDCQKS (SEQ ID NO:81)

### Mouse ADAT-1:

MWTADEIAQLCYAHYNVRLPKQGKPEPNREWTLLAAVVKIQASANQACDIPEKEVQVTKEVVSMGTGTKCIGQSKMR
ESGDILNDSHAEIIARRSFQRYLLHQLHLAAVLKEDSIFVPGTQRGLWRLRPDLSFVFFSSHTPCGDASIIPMLEFE
EQPCCPVIRSWANNSPVQETENLEDSKDKRNCEDPASPVAKKMRLGTPARSLSNCVAHHGTQESGPVKPDVSSSDLT
KEEPDAANGIASGSFRVVDVYRTGAKCVPGETGDLREPGAAYHQVGLLRVKPGRGDRTCSMSCSDKMARWNVLGCQG
ALLMHFLEKPIYLSAVVIGKCPYSQEAMRRALTGRCEETLVLPRGFGVQELEIQQSGLLFEQSRCAVHRKRGDSPGR
LVPCGAAISWSAVPQQPLDVTANGFPQGTTKKEIGSPRARSRISKVELFRSFQKLLSSIADDEQPDSIRVTKKLDTY
QEYKDAASAYQEAWGALRRIQPFASWIRNPPDYHQFK (SEQ ID NO:82) (underline: nucleic acid editing
domain)

### Human ADAT-1:

MWTADEIAQLCYEHYGIRLPKKGKPEPNHEWTLLAAVVKIQSPADKACDTPDKPVQVTKEVVSMGTGTKCIGQSKMR

KNGDILNDSHAEVIARRSFQRYLLHQLQLAATLKEDSIFVPGTQKGVWKLRRDLIFVFFSSHTPCGDASIIPMLEFE
DQPCCPVFRNWAHNSSVEASSNLEAPGNERKCEDPDSPVTKKMRLEPGTAAREVTNGAAHHQSFGKQKSGPISPGIH
SCDLTVEGLATVTRIAPGSAKVIDVYRTGAKCVPGEAGDSGKPGAAFHQVGLLRVKPGRGDRTRSMSCSDKMARWNV
LGCQGALLMHLLEEPIYLSAVVIGKCPYSQEAMQRALIGRCQNVSALPKGFGVQELKILQSDLLFEQSRSAVQAKRA
DSPGRLVPCGAAISWSAVPEQPLDVTANGFPQGTTKKTIGSLQARSQISKVELFRSFQKLLSRIARDKWPHSLRVQK
LDTYQEYKEAASSYQEAWSTLRKQVFGSWIRNPPDYHQFK (SEQ ID NO:83) (underline: nucleic acid
editing domain)

In some embodiments, the enzymatic domain comprises one or more of a transcriptional activator. For example, in some embodiments, the general architecture of exemplary dCas9 fusion proteins with a transcriptional activator domain comprises the structure:
[NH₂]-[NLS]-[Cas9]-[(transcriptional activator)ₙ]-[COOH],
[NH₂]-[NLS]-[ (transcriptional activator)ₙ]-[Cas9]- -[COOH],
[NH₂]-[Cas9]-[ (transcriptional activator)ₙ]-[COOH], or
[NH₂]-[ (transcriptional activator)ₙ]-[Cas9]-[COOH];
wherein NLS is a nuclear localization signal, NH₂ is the N-terminus of the fusion protein, and COOH is the C-terminus of the fusion protein. In some embodiments, the fusion proteins comprises one or more repeats of the transcriptional activator, for example wherein n = 1-10 (*e.g.,* n is 1, 2, 3, 4, 5, 6, 7, 8, 9, or 10). In some embodiments, n = 1-20. In some embodiments, a linker is inserted between the dCas9 and the transcriptional activator domain. In some embodiments, a linker is inserted between the NLS and the transcriptional activator and/or dCas9 domain. In some embodiments, the NLS is located C-terminal of the transcriptional activator and/or the dCas9 domain. In some embodiments, the NLS is located between the transcriptional activator domain and the dCas9 domain. Additional features, such as sequence tags, may also be present. In some embodiments, the transcriptional activator is selected from the group consisting of VP64, (SEQ ID NO:84 or SEQ ID NO:35), VP16 (SEQ ID NO:85), and p65 (SEQ ID NO:86). In some embodiments, a dCas9-VP64 fusion protein comprises the amino acid sequence of SEQ ID NO:87 (*e.g.*, with or without the 6xHis tag) or comprises an amino acid sequence that is at least 80%, at least 85%, at least 90%, at least 95%, at least 98%, or at least 99% identical to the amino acid sequence of SEQ ID NO: 87 (*e.g.,* with or without the 6xHis tag).

### VP64

GSGRADALDDFDLDMLGSDALDDFDLDMLGSDALDDFDLDMLGSDALDDFDLDMLIN (SEQ ID NO:84)

### VP16

APPTDVSLGDELHLDGEDVAMAHADALDDFDLDMLGDGDSPGPGFTPHDSAPYGALDMADFEFEQMFTDA
LGIDEYGGEFPGIRR (SEQ ID NO:85)

### p65:

PSGQISNQALALAPSSAPVLAQTMVPSSAMVPLAQPPAPAPVLTPGPPQSLSAPVPKSTQAGEGTLSEAL
LHLQFDADEDLGALLGNSTDPGVFTDLASVDNSEFQQLLNQGVSMSHSTAEPMLMEYPEAITRLVTGSQR
PPDPAPTPLGTSGLPNGLSGDEDFSSIADMDFSALLSQISSSGQ (SEQ ID NO:86)

### dCas9-VP64-6xHis:

MDKKYSIGLAIGTNSVGWAVITDEYKVPSKKFKVLGNTDRHSIKKNLIGALLFDSGETAEATRLKRTARR
RYTRRKNRICYLQEIFSNEMAKVDDSFFHRLEESFLVEEDKKHERHPIFGNIVDEVAYHEKYPTIYHLRK
KLVDSTDKADLRLIYLALAHMIKFRGHFLIEGDLNPDNSDVDKLFIQLVQTYNQLFEENPINASGVDAKA
ILSARLSKSRRLENLIAQLPGEKKNGLFGNLIALSLGLTPNFKSNFDLAEDAKLQLSKDTYDDDLDNLLA
QIGDQYADLFLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSIPHQIHLGELH
AILRRQEDFYPFLKDNREKIEKILTFRIPYYVGPLARGNSRFAWMTRKSEETITPWNFEEVVDKGASAQS
FIERMTNFDKNLPNEKVLPKHSLLYEYFTVYNELTKVKYVTEGMRKPAFLSGEQKKAIVDLLFKTNRKVT
VKQLKEDYFKKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFANRNFMQLIHDD
SLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDA
IVPQSFLKDDSIDNKVLTRSDKNRGKSDNVPSEEVVKKMKNYWRQLLNAKLITQRKFDNLTKAERGGLSE
LDKAGFIKRQLVETRQITKHVAQILDSRMNTKYDENDKLIREVKVITLKSKLVSDFRKDFQFYKVREINN
YHHAHDAYLNAVVGTALIKKYPKLESEFVYGDYKVYDVRKMIAKSEQEIGKATAKYFFYSNIMNFFKTEI
TLANGEIRKRPLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPIDFLEAKGYKEV
KKDLIIKLPKYSLFELENGRKRMLASAGELQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGAPAAFKYFDTT
IDRKRYTSTKEVLDATLIHQSITGLYETRIDLSQLGGDGSPKKKRKVSSDYKDHDGDYKDHDIDYKDDDD
KAAGGGGSGRADALDDFDLDMLGSDALDDFDLDMLGSDALDDFDLDMLGSDALDDFDLDMLHHHHHH
(SEQ ID NO:87)

ATGGATAAGAAATACTCAATAGGCTTAGCTATCGGCACAAATAGCGTCGGATGGGCGGTGATCACTGATG
AATATAAGGTTCCGTCTAAAAAGTTCAAGGTTCTGGGAAATACAGACCGCCACAGTATCAAAAAAAATCT
TATAGGGGCTCTTTTATTTGACAGTGGAGAGACAGCGGAAGCGACTCGTCTCAAACGGACAGCTCGTAGA
AGGTATACACGTCGGAAGAATCGTATTTGTTATCTACAGGAGATTTTTTCAAATGAGATGGCGAAAGTAG
ATGATAGTTTCTTTCATCGACTTGAAGAGTCTTTTTTGGTGGAAGAAGACAAGAAGCATGAACGTCATCC
TATTTTTGGAAATATAGTAGATGAAGTTGCTTATCATGAGAAATATCCAACTATCTATCATCTGCGAAAA
AAATTGGTAGATTCTACTGATAAAGCGGATTTGCGCTTAATCTATTTGGCCTTAGCGCATATGATTAAGT
TTCGTGGTCATTTTTTGATTGAGGGAGATTTAAATCCTGATAATAGTGATGTGGACAAACTATTTATCCA
GTTGGTACAAACCTACAATCAATTATTTGAAGAAAACCCTATTAACGCAAGTGGAGTAGATGCTAAAGCG
ATTCTTTCTGCACGATTGAGTAAATCAAGACGATTAGAAAATCTCATTGCTCAGCTCCCCGGTGAGAAGA
AAAATGGCTTATTTGGGAATCTCATTGCTTTGTCATTGGGTTTGACCCCTAATTTTAAATCAAATTTTGA
TTTGGCAGAAGATGCTAAATTACAGCTTTCAAAAGATACTTACGATGATGATTTAGATAATTTATTGGCG
CAAATTGGAGATCAATATGCTGATTTGTTTTTGGCAGCTAAGAATTTATCAGATGCTATTTTACTTTCAG
ATATCCTAAGAGTAAATACTGAAATAACTAAGGCTCCCCTATCAGCTTCAATGATTAAACGCTACGATGA
ACATCATCAAGACTTGACTCTTTTAAAAGCTTTAGTTCGACAACAACTTCCAGAAAAGTATAAAGAAATC

TTTTTTGATCAATCAAAAAACGGATATGCAGGTTATATTGATGGGGGAGCTAGCCAAGAAGAATTTTATA
AATTTATCAAACCAATTTTAGAAAAAATGGATGGTACTGAGGAATTATTGGTGAAACTAAATCGTGAAGA
TTTGCTGCGCAAGCAACGGACCTTTGACAACGGCTCTATTCCCCATCAAATTCACTTGGGTGAGCTGCAT
GCTATTTTGAGAAGACAAGAAGACTTTTATCCATTTTTAAAAGACAATCGTGAGAAGATTGAAAAAATCT
TGACTTTTCGAATTCCTTATTATGTTGGTCCATTGGCGCGTGGCAATAGTCGTTTTGCATGGATGACTCG
GAAGTCTGAAGAAACAATTACCCCATGGAATTTTGAAGAAGTTGTCGATAAAGGTGCTTCAGCTCAATCA
TTTATTGAACGCATGACAAACTTTGATAAAAATCTTCCAAATGAAAAAGTACTACCAAAACATAGTTTGC
TTTATGAGTATTTTACGGTTTATAACGAATTGACAAAGGTCAAATATGTTACTGAAGGAATGCGAAAACC
AGCATTTCTTTCAGGTGAACAGAAGAAAGCCATTGTTGATTTACTCTTCAAAACAAATCGAAAAGTAACC
GTTAAGCAATTAAAAGAAGATTATTTCAAAAAAATAGAATGTTTTGATAGTGTTGAAATTTCAGGAGTTG
AAGATAGATTTAATGCTTCATTAGGTACCTACCATGATTTGCTAAAAATTATTAAAGATAAAGATTTTTT
GGATAATGAAGAAAATGAAGATATCTTAGAGGATATTGTTTTAACATTGACCTTATTTGAAGATAGGGAG
ATGATTGAGGAAAGACTTAAAACATATGCTCACCTCTTTGATGATAAGGTGATGAAACAGCTTAAACGTC
GCCGTTATACTGGTTGGGGACGTTTGTCTCGAAAATTGATTAATGGTATTAGGGATAAGCAATCTGGCAA
AACAATATTAGATTTTTTGAAATCAGATGGTTTTGCCAATCGCAATTTTATGCAGCTGATCCATGATGAT
AGTTTGACATTTAAAGAAGACATTCAAAAAGCACAAGTGTCTGGACAAGGCGATAGTTTACATGAACATA
TTGCAAATTTAGCTGGTAGCCCTGCTATTAAAAAAGGTATTTTACAGACTGTAAAAGTTGTTGATGAATT
GGTCAAAGTAATGGGGCGGCATAAGCCAGAAAATATCGTTATTGAAATGGCACGTGAAAATCAGACAACT
CAAAAGGGCCAGAAAAATTCGCGAGAGCGTATGAAACGAATCGAAGAAGGTATCAAAGAATTAGGAAGTC
AGATTCTTAAAGAGCATCCTGTTGAAAATACTCAATTGCAAAATGAAAAGCTCTATCTCTATTATCTCCA
AAATGGAAGAGACATGTATGTGGACCAAGAATTAGATATTAATCGTTTAAGTGATTATGATGTCGATGCC
ATTGTTCCACAAAGTTTCCTTAAAGACGATTCAATAGACAATAAGGTCTTAACGCGTTCTGATAAAAATC
GTGGTAAATCGGATAACGTTCCAAGTGAAGAAGTAGTCAAAAAGATGAAAAACTATTGGAGACAACTTCT
AAACGCCAAGTTAATCACTCAACGTAAGTTTGATAATTTAACGAAAGCTGAACGTGGAGGTTTGAGTGAA
CTTGATAAAGCTGGTTTTATCAAACGCCAATTGGTTGAAACTCGCCAAATCACTAAGCATGTGGCACAAA
TTTTGGATAGTCGCATGAATACTAAATACGATGAAAATGATAAACTTATTCGAGAGGTTAAAGTGATTAC
CTTAAAATCTAAATTAGTTTCTGACTTCCGAAAAGATTTCCAATTCTATAAAGTACGTGAGATTAACAAT
TACCATCATGCCCATGATGCGTATCTAAATGCCGTCGTTGGAACTGCTTTGATTAAGAAATATCCAAAAC
TTGAATCGGAGTTTGTCTATGGTGATTATAAAGTTTATGATGTTCGTAAAATGATTGCTAAGTCTGAGCA
AGAAATAGGCAAAGCAACCGCAAAATATTTCTTTTACTCTAATATCATGAACTTCTTCAAAACAGAAATT
ACACTTGCAAATGGAGAGATTCGCAAACGCCCTCTAATCGAAACTAATGGGGAAACTGGAGAAATTGTCT
GGGATAAAGGGCGAGATTTTGCCACAGTGCGCAAAGTATTGTCCATGCCCCAAGTCAATATTGTCAAGAA
AACAGAAGTACAGACAGGCGGATTCTCCAAGGAGTCAATTTTACCAAAAAGAAATTCGGACAAGCTTATT
GCTCGTAAAAAAGACTGGGATCCAAAAAAATATGGTGGTTTTGATAGTCCAACGGTAGCTTATTCAGTCC
TAGTGGTTGCTAAGGTGGAAAAAGGGAAATCGAAGAAGTTAAAATCCGTTAAAGAGTTACTAGGGATCAC
AATTATGGAAAGAAGTTCCTTTGAAAAAAATCCGATTGACTTTTTAGAAGCTAAAGGATATAAGGAAGTT
AAAAAAGACTTAATCATTAAACTACCTAAATATAGTCTTTTTGAGTTAGAAAACGGTCGTAAACGGATGC
TGGCTAGTGCCGGAGAATTACAAAAAGGAAATGAGCTGGCTCTGCCAAGCAAATATGTGAATTTTTTATA
TTTAGCTAGTCATTATGAAAAGTTGAAGGGTAGTCCAGAAGATAACGAACAAAAACAATTGTTTGTGGAG
CAGCATAAGCATTATTTAGATGAGATTATTGAGCAAATCAGTGAATTTTCTAAGCGTGTTATTTTAGCAG
ATGCCAATTTAGATAAAGTTCTTAGTGCATATAACAAACATAGAGACAAACCAATACGTGAACAAGCAGA
AAATATTATTCATTTATTTACGTTGACGAATCTTGGAGCTCCCGCTGCTTTTAAATATTTTGATACAACA
ATTGATCGTAAACGATATACGTCTACAAAAGAAGTTTTAGATGCCACTCTTATCCATCAATCCATCACTG
GTCTTTATGAAACACGCATTGATTTGAGTCAGCTAGGAGGTGACGGTTCTCCCAAGAAGAAGAGGAAAGT
CTCGAGCGACTACAAAGACCATGACGGTGATTATAAAGATCATGACATCGATTACAAGGATGACGATGAC
AAGGCTGCAGGAGGCGGTGGAAGCGGGCGCGCCGACGCGCTGGACGATTTCGATCTCGACATGCTGGGTT
CTGATGCCCTCGATGACTTTGACCTGGATATGTTGGGAAGCGACGCATTGGATGACTTTGATCTGGACAT
GCTCGGCTCCGATGCTCTGGACGATTTCGATCTCGATATGTTACATCACCACCACCATCAC (SEQ ID
NO:269)

### Cas9-NLS-6xHis:

MDKKYSIGLAIGTNSVGWAVITDEYKVPSKKFKVLGNTDRHSIKKNLIGALLFDSGETAEATRLKRTARR
RYTRRKNRICYLQEIFSNEMAKVDDSFFHRLEESFLVEEDKKHERHPIFGNIVDEVAYHEKYPTIYHLRK
KLVDSTDKADLRLIYLALAHMIKFRGHFLIEGDLNPDNSDVDKLFIQLVQTYNQLFEENPINASGVDAKA
ILSARLSKSRRLENLIAQLPGEKKNGLFGNLIALSLGLTPNFKSNFDLAEDAKLQLSKDTYDDDLDNLLA
QIGDQYADLFLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSIPHQIHLGELH
AILRRQEDFYPFLKDNREKIEKILTFRIPYYVGPLARGNSRFAWMTRKSEETITPWNFEEVVDKGASAQS
FIERMTNFDKNLPNEKVLPKHSLLYEYFTVYNELTKVKYVTEGMRKPAFLSGEQKKAIVDLLFKTNRKVT
VKQLKEDYFKKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFANRNFMQLIHDD
SLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDH
IVPQSFLKDDSIDNKVLTRSDKNRGKSDNVPSEEVVKKMKNYWRQLLNAKLITQRKFDNLTKAERGGLSE
LDKAGFIKRQLVETRQITKHVAQILDSRMNTKYDENDKLIREVKVITLKSKLVSDFRKDFQFYKVREINN
YHHAHDAYLNAVVGTALIKKYPKLESEFVYGDYKVYDVRKMIAKSEQEIGKATAKYFFYSNIMNFFKTEI
TLANGEIRKRPLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPIDFLEAKGYKEV
KKDLIIKLPKYSLFELENGRKRMLASAGELQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGAPAAFKYFDTT
IDRKRYTSTKEVLDATLIHQSITGLYETRIDLSQLGGDPKKKRKVMDKHHHHHH (SEQ ID NO:270)

ATGGATAAGAAATACTCAATAGGCTTAGATATCGGCACAAATAGCGTCGGATGGGCGGTGATCACTGATG
AATATAAGGTTCCGTCTAAAAAGTTCAAGGTTCTGGGAAATACAGACCGCCACAGTATCAAAAAAAATCT
TATAGGGGCTCTTTTATTTGACAGTGGAGAGACAGCGGAAGCGACTCGTCTCAAACGGACAGCTCGTAGA
AGGTATACACGTCGGAAGAATCGTATTTGTTATCTACAGGAGATTTTTTCAAATGAGATGGCGAAAGTAG
ATGATAGTTTCTTTCATCGACTTGAAGAGTCTTTTTTGGTGGAAGAAGACAAGAAGCATGAACGTCATCC
TATTTTTGGAAATATAGTAGATGAAGTTGCTTATCATGAGAAATATCCAACTATCTATCATCTGCGAAAA
AAATTGGTAGATTCTACTGATAAAGCGGATTTGCGCTTAATCTATTTGGCCTTAGCGCATATGATTAAGT
TTCGTGGTCATTTTTTGATTGAGGGAGATTTAAATCCTGATAATAGTGATGTGGACAAACTATTTATCCA
GTTGGTACAAACCTACAATCAATTATTTGAAGAAAACCCTATTAACGCAAGTGGAGTAGATGCTAAAGCG
ATTCTTTCTGCACGATTGAGTAAATCAAGACGATTAGAAAATCTCATTGCTCAGCTCCCCGGTGAGAAGA
AAAATGGCTTATTTGGGAATCTCATTGCTTTGTCATTGGGTTTGACCCCTAATTTTAAATCAAATTTTGA
TTTGGCAGAAGATGCTAAATTACAGCTTTCAAAAGATACTTACGATGATGATTTAGATAATTTATTGGCG
CAAATTGGAGATCAATATGCTGATTTGTTTTTGGCAGCTAAGAATTTATCAGATGCTATTTTACTTTCAG
ATATCCTAAGAGTAAATACTGAAATAACTAAGGCTCCCCTATCAGCTTCAATGATTAAACGCTACGATGA
ACATCATCAAGACTTGACTCTTTTAAAAGCTTTAGTTCGACAACAACTTCCAGAAAAGTATAAAGAAATC
TTTTTTGATCAATCAAAAAACGGATATGCAGGTTATATTGATGGGGGAGCTAGCCAAGAAGAATTTTATA
AATTTATCAAACCAATTTTAGAAAAAATGGATGGTACTGAGGAATTATTGGTGAAACTAAATCGTGAAGA
TTTGCTGCGCAAGCAACGGACCTTTGACAACGGCTCTATTCCCCATCAAATTCACTTGGGTGAGCTGCAT
GCTATTTTGAGAAGACAAGAAGACTTTTATCCATTTTTAAAAGACAATCGTGAGAAGATTGAAAAAATCT
TGACTTTTCGAATTCCTTATTATGTTGGTCCATTGGCGCGTGGCAATAGTCGTTTTGCATGGATGACTCG
GAAGTCTGAAGAAACAATTACCCCATGGAATTTTGAAGAAGTTGTCGATAAAGGTGCTTCAGCTCAATCA
TTTATTGAACGCATGACAAACTTTGATAAAAATCTTCCAAATGAAAAAGTACTACCAAAACATAGTTTGC
TTTATGAGTATTTTACGGTTTATAACGAATTGACAAAGGTCAAATATGTTACTGAAGGAATGCGAAAACC
AGCATTTCTTTCAGGTGAACAGAAGAAAGCCATTGTTGATTTACTCTTCAAAACAAATCGAAAAGTAACC
GTTAAGCAATTAAAAGAAGATTATTTCAAAAAAATAGAATGTTTTGATAGTGTTGAAATTTCAGGAGTTG
AAGATAGATTTAATGCTTCATTAGGTACCTACCATGATTTGCTAAAAATTATTAAAGATAAAGATTTTTT
GGATAATGAAGAAAATGAAGATATCTTAGAGGATATTGTTTTAACATTGACCTTATTTGAAGATAGGGAG
ATGATTGAGGAAAGACTTAAAACATATGCTCACCTCTTTGATGATAAGGTGATGAAACAGCTTAAACGTC
GCCGTTATACTGGTTGGGGACGTTTGTCTCGAAAATTGATTAATGGTATTAGGGATAAGCAATCTGGCAA
AACAATATTAGATTTTTTGAAATCAGATGGTTTTGCCAATCGCAATTTTATGCAGCTGATCCATGATGAT
AGTTTGACATTTAAAGAAGACATTCAAAAAGCACAAGTGTCTGGACAAGGCGATAGTTTACATGAACATA

TTGCAAATTTAGCTGGTAGCCCTGCTATTAAAAAAGGTATTTTACAGACTGTAAAAGTTGTTGATGAATT
GGTCAAAGTAATGGGGCGGCATAAGCCAGAAAATATCGTTATTGAAATGGCACGTGAAAATCAGACAACT
CAAAAGGGCCAGAAAAATTCGCGAGAGCGTATGAAACGAATCGAAGAAGGTATCAAAGAATTAGGAAGTC
AGATTCTTAAAGAGCATCCTGTTGAAAATACTCAATTGCAAAATGAAAAGCTCTATCTCTATTATCTCCA
AAATGGAAGAGACATGTATGTGGACCAAGAATTAGATATTAATCGTTTAAGTGATTATGATGTCGATCAC
ATTGTTCCACAAAGTTTCCTTAAAGACGATTCAATAGACAATAAGGTCTTAACGCGTTCTGATAAAAATC
GTGGTAAATCGGATAACGTTCCAAGTGAAGAAGTAGTCAAAAAGATGAAAAACTATTGGAGACAACTTCT
AAACGCCAAGTTAATCACTCAACGTAAGTTTGATAATTTAACGAAAGCTGAACGTGGAGGTTTGAGTGAA
CTTGATAAAGCTGGTTTTATCAAACGCCAATTGGTTGAAACTCGCCAAATCACTAAGCATGTGGCACAAA
TTTTGGATAGTCGCATGAATACTAAATACGATGAAAATGATAAACTTATTCGAGAGGTTAAAGTGATTAC
CTTAAAATCTAAATTAGTTTCTGACTTCCGAAAAGATTTCCAATTCTATAAAGTACGTGAGATTAACAAT
TACCATCATGCCCATGATGCGTATCTAAATGCCGTCGTTGGAACTGCTTTGATTAAGAAATATCCAAAAC
TTGAATCGGAGTTTGTCTATGGTGATTATAAAGTTTATGATGTTCGTAAAATGATTGCTAAGTCTGAGCA
AGAAATAGGCAAAGCAACCGCAAAATATTTCTTTTACTCTAATATCATGAACTTCTTCAAAACAGAAATT
ACACTTGCAAATGGAGAGATTCGCAAACGCCCTCTAATCGAAACTAATGGGGAAACTGGAGAAATTGTCT
GGGATAAAGGGCGAGATTTTGCCACAGTGCGCAAAGTATTGTCCATGCCCCAAGTCAATATTGTCAAGAA
AACAGAAGTACAGACAGGCGGATTCTCCAAGGAGTCAATTTTACCAAAAAGAAATTCGGACAAGCTTATT
GCTCGTAAAAAAGACTGGGATCCAAAAAAATATGGTGGTTTTGATAGTCCAACGGTAGCTTATTCAGTCC
TAGTGGTTGCTAAGGTGGAAAAAGGGAAATCGAAGAAGTTAAAATCCGTTAAAGAGTTACTAGGGATCAC
AATTATGGAAAGAAGTTCCTTTGAAAAAAATCCGATTGACTTTTTAGAAGCTAAAGGATATAAGGAAGTT
AAAAAAGACTTAATCATTAAACTACCTAAATATAGTCTTTTTGAGTTAGAAAACGGTCGTAAACGGATGC
TGGCTAGTGCCGGAGAATTACAAAAAGGAAATGAGCTGGCTCTGCCAAGCAAATATGTGAATTTTTTATA
TTTAGCTAGTCATTATGAAAAGTTGAAGGGTAGTCCAGAAGATAACGAACAAAAACAATTGTTTGTGGAG
CAGCATAAGCATTATTTAGATGAGATTATTGAGCAAATCAGTGAATTTTCTAAGCGTGTTATTTTAGCAG
ATGCCAATTTAGATAAAGTTCTTAGTGCATATAACAAACATAGAGACAAACCAATACGTGAACAAGCAGA
AAATATTATTCATTTATTTACGTTGACGAATCTTGGAGCTCCCGCTGCTTTTAAATATTTTGATACAACA
ATTGATCGTAAACGATATACGTCTACAAAAGAAGTTTTAGATGCCACTCTTATCCATCAATCCATCACTG
GTCTTTATGAAACACGCATTGATTTGAGTCAGCTAGGAGGTGACCCCAAGAAGAAGAGGAAGGTGATGGA
TAAGCATCACCACCACCATCAC (SEQ ID NO:271)

### NLS-Cas9-6xHis:

MPKKKRKVMDKKYSIGLAIGTNSVGWAVITDEYKVPSKKFKVLGNTDRHSIKKNLIGALLFDSGETAEAT
RLKRTARRRYTRRKNRICYLQEIFSNEMAKVDDSFFHRLEESFLVEEDKKHERHPIFGNIVDEVAYHEKY
PTIYHLRKKLVDSTDKADLRLIYLALAHMIKFRGHFLIEGDLNPDNSDVDKLFIQLVQTYNQLFEENPIN
ASGVDAKAILSARLSKSRRLENLIAQLPGEKKNGLFGNLIALSLGLTPNFKSNFDLAEDAKLQLSKDTYD
DDLDNLLAQIGDQYADLFLAAKNLSDAILLSDILRVNTEITKAPLSASMIKRYDEHHQDLTLLKALVRQQ
LPEKYKEIFFDQSKNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSEETITPWNFEEVV
DKGASAQSFIERMTNFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNEENEDILEDIVLT
LTLFEDREMIEERLKTYAHLFDDKVMKQLKRRRYTGWGRLSRKLINGIRDKQSGKTILDFLKSDGFANRN
FMQLIHDDSLTFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRDMYVDQELDINR
LSDYDVDHIVPQSFLKDDSIDNKVLTRSDKNRGKSDNVPSEEVVKKMKNYWRQLLNAKLITQRKFDNLTK
AERGGLSELDKAGFIKRQLVETRQITKHVAQILDSRMNTKYDENDKLIREVKVITLKSKLVSDFRKDFQF
YKVREINNYHHAHDAYLNAVVGTALIKKYPKLESEFVYGDYKVYDVRKMIAKSEQEIGKATAKYFFYSNI
MNFFKTEITLANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERSSFEKNPIDFL

EAKGYKEVKKDLIIKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGDHHHHHH (SEQ ID NO:272)

In some embodiments, the enzymatic domain comprises one or more of a transcriptional repressor. For example, in some embodiments, the general architecture of exemplary dCas9 fusion proteins with a transcriptional repressor domain comprises the structure:
[NH₂]-[NLS]-[Cas9]-[(transcriptional repressor)ₙ]-[COOH],
[NH₂]-[NLS]-[(transcriptional repressor)ₙ]-[Cas9]- -[COOH],
[NH₂]-[Cas9]-[(transcriptional repressor)ₙ]-[COOH], or
[NH₂] - [(transcriptional repressor)ₙ]- [Cas9] - [COOH];
wherein NLS is a nuclear localization signal, NH₂ is the N-terminus of the fusion protein, and COOH is the C-terminus of the fusion protein. In some embodiments, the fusion proteins comprises one or more repeats of the transcriptional repressor, for example wherein n = 1-10 (*e.g.,* n is 1, 2, 3, 4, 5, 6, 7, 8, 9, or 10). In some embodiments, n = 1-20. In some embodiments, a linker is inserted between the dCas9 and the transcriptional repressor domain. In some embodiments, a linker is inserted between the NLS and the transcriptional repressor and/or dCas9 domain. In some embodiments, the NLS is located C-terminal of the transcriptional repressor and/or the dCas9 domain. In some embodiments, the NLS is located between the transcriptional repressor domain and the dCas9 domain. Additional features, such as sequence tags, may also be present. In some embodiments, the transcriptional repressor is selected from the group consisting of the KRAB (Krüppel associated box) domain of Kox1, SID (mSin3 interaction domain), the CS (Chromo Shadow) domain of HP1α, or the WRPW domain of Hes1. These and other repressor domains are known in the art, and in some embodiments correspond to those described in Urrutia, KRAB-containing zinc-finger repressor proteins. Genome Biol. 2003;4(10):231; Gilbert et al. CRISPR-mediated modular RNA-guided regulation of transcription in eukaryotes. Cell. 2013; 154, 442-451; Konermann et al., Optical control of mammalian endogenous transcription and epigenetic states. Nature. 2013; 500, 472-476; and published U.S. patent application U.S.S.N. 14/105,017, published as U.S. 2014/0186958 A1, the entire contents of which are incorporated herein by reference. In some embodiments, the transcription repressor domain comprises one or more repeats (*e.g.,* 2, 3, 4, 5, 6, 7, 8, 9, or 10 repeats) of a KRAB domain. In some embodiments, the KRAB domain comprises an amino acid sequence selected from the group consisting of SEQ ID NOs:88-91. In some embodiments, the transcriptional repressor domains comprises one or more repeats of a SID protein. In some embodiments, the SID protein comprises an amino acid sequence set forth as SEQ ID NO:80. In some embodiments, the repressor domain comprises 2, 3, 4, 5, 6, 7, 8, 9, or 10 repeats of a SID protein (*e.g.,* SEQ ID NO:92). In some embodiments, the repressor domain comprises four repeats of SID (*e.g.,* SID4x; SEQ ID NO:93).

### KRAB (human; GenBank: AAD20972.1)

MNMFKEAVTFKDVAVAFTEEELGLLGPAQRKLYRDVMVENFRNLLSVGHPPFKQDVSPIERNEQLWIMTT
ATRRQGNLDTLPVKALLLYDLAQT (SEQ ID NO:88)

### KRAB protein domain, partial (human; GenBank: CAB52478.1):

EQVSFKDVCVDFTQEEWYLLDPAQKILYRDVILENYSNLVSVGYCITKPEVIFKIEQGEEPWILEKGFPS
QCHP (SEQ ID NO:89)

KRAB A domain, partial (human; GenBank: AAB03530.1):
EAVTFKDVAWFTEEELGLLDPAQRKLYRDVMLENFRNLLSV (SEQ ID NO:90)

KRAB (mouse; C2H2 type domain containing protein; GenBank: CAM27971.1):
MDLVTYDDVHVNFTQDEWALLDPSQKSLYKGVMLETYKNLTAIGYIWEEHTIEDHFQTSRSHGSNKKTH (SEQ ID NO:91)

SID repressor domain:
GSGMNIQMLLEAADYLERREREAEHGYASMLP (SEQ ID NO:92)

### SID4x repressor domain:

GSGMNIQMLLEAADYLERREREAEHGYASMLPGSGMNIQMLLEAADYLERREREAEHGYASMLPGSGMNI
QMLLEAADYLERREREAEHGYASMLPGSGMNIQMLLEAADYLERREREAEHGYASMLPSR (SEQ ID
NO:93)

In some embodiments, the enzymatic domain comprises an epigenetic modifier or a catalytic domain thereof. For example, in some embodiments, the general architecture of exemplary dCas9 fusion proteins with an epigenetic modifier or domain comprises the structure:
[NH₂]-[NLS]-[Cas9]-[epigenetic modifier]-[COOH],
[NH₂]-[NLS]-[epigenetic modifier]-[Cas9]-[COOH],
[NH₂]-[Cas9]-[epigenetic modifier]-[COOH], or
[NH₂]-[epigenetic modifier - [Cas9] - [COOH];
wherein NLS is a nuclear localization signal, NH₂ is the N-terminus of the fusion protein, and COOH is the C-terminus of the fusion protein. In some embodiments, a linker is inserted between the dCas9 and the epigenetic modifier domain. In some embodiments, a linker is inserted between the NLS and the epigenetic modifier and/or dCas9 domain. In some embodiments, the NLS is located C-terminal of the epigenetic modifier and/or the dCas9 domain. In some embodiments, the NLS is located between the epigenetic modifier domain and the dCas9 domain. Additional features, such as sequence tags, may also be present. Epigenetic modifiers are well known in the art, and typically catalyze DNA methylation (and demethylation) or histone modifications (*e.g.,* histone methylation/demethylation, acetylation/deacetylation, ubiquitylation, phosphorylation, sumoylation, *etc*.)*.* The presence of one more epigenetic modifications can affect the transcriptional activity of one or more genes, for example turning genes from an "on" state to an "off' state, and *vice versa.* Epigenetic modifiers include, but are not limited to, histone demethylase, histone methyltransferase, hydroxylase, histone deacetylase, and histone acetyltransferase. Exemplary epigenetic modifying proteins can be found in Konermann et al., Optical control of mammalian endogenous transcription and epigenetic states. Nature. 2013; 500, 472-476; Mendenhall et al., Locus-specific editing of histone modifications at endogenous enhancers. Nat. Biotechnol. 2013; 31, 1133-1136; and Maeder et al., Targeted DNA demethylation and activation of endogenous genes using programmable TALE-TET1 fusion proteins. Nat. Biotechnol. 2013; 31, 1137-1142; the entire contents of each are incorporated herein by reference. In some embodiments, the epigenetic modifier domain is LSD1 (Lysine (K)-specific demethylase 1A) histone demethylase, which in some embodiments, comprises in whole or in part, an amino acid sequence set forth as SEQ ID NO:94 or SEQ ID NO:95. In some embodiments, the epigenetic modifier domain is TET1 hydroxylase catalytic domain, which in some embodiments, comprises an amino acid sequence set forth as SEQ ID NO:96. In some embodiments, the epigenetic modifier is a histone deacetylase (HDAC) effector domain. In some embodiments, the HDAC effector domain comprises in whole in in part, an amino acid sequence corresponding to any of the HDAC effector proteins provided in Supplementary Table 2 of Konermann et al., Optical control of mammalian endogenous transcription and epigenetic states. Nature. 2013; 500, 472-476; SEQ ID NOs:97-108. In some embodiments, the epigenetic modifier is a histone methyltransferase (HMT) effector domain. In some embodiments, the HMT effector domain comprises in whole in in part, an amino acid sequence corresponding to any of the HDAC effector proteins provided in Supplementary Table 3 of Konermann et al., Optical control of mammalian endogenous transcription and epigenetic states. Nature. 2013; 500, 472-476; SEQ ID NOs: 109-118.

### LSD1, isoform a (human):













 (SEQ ID NO:94)

### LSD1, isoform b (human):

MLSGKKAAAAAAAAAAAATGTEAGPGTAGGSENGSEVAAQPAGLSGPAEVGPGAVGERTPRKKEPPRASP
PGGLAEPPGSAGPQAGPTVVPGSATPMETGIAETPEGRRTSRRKRAKVEYREMDESLANLSEDEYYSEEE
RNAKAEKEKKLPPPPPPQAPPEEENESEPEEPSGVEGAAFQSRLPHDRMTSQEAACFPDIISGPQQTQKVF
LFIRNRTLQLWLDNPKIQLTFEATLQQLEAPYNSDTVLVHRVHSYLERHGLINFGIYKRIKPLPTKKTGK
VIIIGSGVSGLAAARQLQSFGMDVTLLEARDRVGGRVATFRKGNYVADLGAMVVTGLGGNPMAVVSKQVN
MELAKIKQKCPLYEANGQAVPKEKDEMVEQEFNRLLEATSYLSHQLDFNVLNNKPVSLGQALEVVIQLQE
KHVKDEQIEHWKKIVKTQEELKELLNKMVNLKEKIKELHQQYKEASEVKPPRDITAEFLVKSKHRDLTAL
CKEYDELAETQGKLEEKLQELEANPPSDVYLSSRDRQILDWHFANLEFANATPLSTLSLKHWDQDDDFEF
TGSHLTVRNGYSCVPVALAEGLDIKLNTAVRQVRYTASGCEVIAVNTRSTSQTFIYKCDAVLCTLPLGVL
KQQPPAVQFVPPLPEWKTSAVQRMGFGNLNKVVLCFDRVFWDPSVNLFGHVGSTTASRGELFLFWNLYKA
PILLALVAGEAAGIMENISDDVIVGRCLAILKGIFGSSAVPQPKETVVSRWRADPWARGSYSYVAAGSSG
NDYDLMAQPITPGPSIPGAPQPIPRLFFAGEHTIRNYPATVHGALLSGLREAGRIADQFLGAMYTLPRQA
TPGVPAQQSPSM (SEQ ID NO:95)

### TET1 catalytic domain:

SIVAQLSRPDPALAALTNDHLVALACLGGRPALDAVKKGLPHAPALIKRTNRRIPERTSHRVADHAQVVR
VLGFFQCHSHPAQAFDDAMTQFGMSGGGSLPTCSCLDRVIQKDKGPYYTHLGAGPSVAAVREIMENRYGQ
KGNAIRIEIVVYTGKEGKSSHGCPIAKWVLRRSSDEEKVLCLVRQRTGHHCPTAVMVVLIMVWDGIPLPM
ADRLYTELTENLKSYNGHPTDRRCTLNENRTCTCQGIDPETCGASFSFGCSWSMYFNGCKFGRSPSPRRF
RIDPSSPLHEKNLEDNLQSLATRLAPIYKQYAPVAYQNQVEYENVARECRLGSKEGRPFSGVTACLDFCA
HPHRDIHNMNNGSTVVCTLTREDNRSLGVIPQDEQLHVLPLYKLSDTDEFGSKEGMEAKIKSGAIEVLAP

RRKKRTCFTQPVPRSGKKRAAMMTEVLAHKIRAVEKKPIPRIKRKNNSTTTNNSKPSSLPTLGSNTETVQ
PEVKSETEPHFILKSSDNTKTYSLMPSAPHPVKEASPGFSWSPKTASATPAPLKNDATASCGFSERSSTP
HCTMPSGRLSGANAAAADGPGISQLGEVAPLPTLSAPVMEPLINSEPSTGVTEPLTPHQPNHQPSFLTSP
QDLASSPMEEDEQHSEADEPPSDEPLSDDPLSPAEEKLPHIDEYWSDSEHIFLDANIGGVAIAPAHGSVL
IECARRELHATTPVEHPNRNHPTRLSLVFYQHKNLNKPQHGFELNKIKFEAKEAKNKKMKASEQKDQAAN
EGPEQSSEVNELNQIPSHKALTLTHDNVVTVSPYALTHVAGPYNHWV (SEQ ID NO:96)

HDAC effector domains:
HDAC8 (*X. laevis*): 
ASSPKKKRKVEASMSRVVKPKVASMEEMAAFHTDAYLQHLHKVSEEGDNDDPETLEYGLGYDCPITEGIY
DYAAAVGGATLTAAEQLIEGKTRIAVNWPGGWHHAKKDEASGFCYLNDAVLGILKLREKFDRVLYVDMDL
HHGDGVEDAFSFTSKVMTVSLHKFSPGFFPGTGDVSDIGLGKGRYYSINVPLQDGIQDDKYYQICEGVLK
EVFTTFNPEAVVLQLGADTIAGDPMCSFNMTPEGIGKCLKYVLQWQLPTLILGGGGYHLPNTARCWTYLT
ALIVGRTLSSEIPDHEFFTEYGPDYVLEITPSCRPDRNDTQKVQEILQSIKGNLKRVVEF (SEQ ID
NO:97)
RPD3 (*S. cerevisiae*): 
ASSPKKKRKVEASRRVAYFYDADVGNYAYGAGHPMKPHRIRMAHSLIMNYGLYKKMEIYRAKPATKQEMC
QFHTDEYIDFLSRVTPDNLEMFKRESVKFNVGDDCPVFDGLYEYCSISGGGSMEGAARLNRGKCDVAVNY
AGGLHHAKKSEASGFCYLNDIVLGIIELLRYHPRVLYIDIDVHHGDGVEEAFYTTDRVMTCSFHKYGEFF
PGTGELRDIGVGAGKNYAVNVPLRDGIDDATYRSVFEPVIKKIMEWYQPSAVVLQCGGDSLSGDRLGCFN
LSMEGHANCVNYVKSFGIPMMVVGGGGYTMRNVARTWCFETGLLNNVVLDKDLPYEF (SEQ ID NO:98)
MesoLo4 (*M. loti*): 
ASSPKKKRKVEASMPLQIVHHPDYDAGFATNHRFPMSKYPLLMEALRARGLASPDALNTTEPAPASWLKL
AHAADYVDQVISCSVPEKIEREIGFPVGPRVSLRAQLATGGTILAARLALRHGIACNTAGGSHHARRAQG
AGFCTFNDVAVASLVLLDEGAAQNILVVDLDVHQGDGTADILSDEPGVFTFSMHGERNYPVRKIASDLDI
ALPDGTGDAAYLRRLATILPELSARARWDIVFYNAGVDVHAEDRLGRLALSNGGLRARDEMVIGHFRALG
IPVCGVIGGGYSTDVPALASRHAILFEVASTYAEF (SEQ ID NO:99)
HDAC11 (human): 
ASSPKKKRKVEASMLHTTQLYQHVPETRWPIVYSPRYNITFMGLEKLHPFDAGKWGKVINFLKEEKLLSD
SMLVEAREASEEDLLVVHTRRYLNELKWSFAVATITEIPPVIFLPNFLVQRKVLRPLRTQTGGTIMAGKL
AVERGWAINVGGGFHHCSSDRGGGFCAYADITLAIKFLFERVEGISRATIIDLDAHQGNGHERDFMDDKR
VYIMDVYNRHIYPGDRFAKQAIRRKVELEWGTEDDEYLDKVERNIKKSLQEHLPDVVVYNAGTDILEGDR
LGGLSISPAGIVKRDELVFRMVRGRRVPILMVTSGGYQKRTARIIADSILNLFGLGLIGPESPSVSAQNS
DTPLLPPAVPEF (SEQ ID NO:100)
HDT1 (*A. thaliana*): 
ASSPKKKRKVEASMEFWGIEVKSGKPVTVTPEEGILIHVSQASLGECKNKKGEFVPLHVKVGNQNLVLGT
LSTENIPQLFCDLVFDKEFELSHTWGKGSVYFVGYKTPNIEPQGYSEEEEEEEEEVPAGNAAKAVAKPKA
KPAEVKPAVDDEEDESDSDGMDEDDSDGEDSEEEEPTPKKPASSKKRANETTPKAPVSAKKAKVAVTPQK
TDEKKKGGKAANQSEF (SEQ ID NO:101)
SIRT3 (human): 
ASSPKKKRKVEASMVGAGISTPSGIPDFRSPGSGLYSNLQQYDLPYPEAIFELPFFFHNPKPFFTLAKEL
YPGNYKPNVTHYFLRLLHDKGLLLRLYTQNIDGLERVSGIPASKLVEAHGTFASATCTVCQRPFPGEDIR
ADVMADRVPRCPVCTGVVKPDIVFFGEPLPQRFLLHVVDFPMADLLLILGTSLEVEPFASLTEAVRSSVP
RLLINRDLVGPLAWHPRSRDVAQLGDVVHGVESLVELLGWTEEMRDLVQRETGKLDGPDKEF (SEQ ID
NO:102)
HST2 (*S. cerevisiae*): 
ASSPKKKRKVEASTEMSVRKIAAHMKSNPNAKVIFMVGAGISTSCGIPDFRSPGTGLYHNLARLKLPYPE
AVFDVDFFQSDPLPFYTLAKELYPGNFRPSKFHYLLKLFQDKDVLKRVYTQNIDTLERQAGVKDDLIIEA
HGSFAHCHCIGCGKVYPPQVFKSKLAEHPIKDFVKCDVCGELVKPAIVFFGEDLPDSFSETWLNDSEWLR
EKITTSGKHPQQPLVIVVGTSLAVYPFASLPEEIPRKVKRVLCNLETVGDFKANKRPTDLIVHQYSDEFA
EQLVEELGWQEDFEKILTAQGGMGEF (SEQ ID NO:103)
CobB (*E. coli* (*K12*)): 
ASSPKKKRKVEASMEKPRVLVLTGAGISAESGIRTFRAADGLWEEHRVEDVATPEGFDRDPELVQAFYNA
RRRQLQQPEIQPNAAHLALAKLQDALGDRFLLVTQNIDNLHERAGNTNVIHMHGELLKVRCSQSGQVLDW
TGDVTPEDKCHCCQFPAPLRPHVVWFGEMPLGMDEIYMALSMADIFIAIGTSGHVYPAAGFVHEAKLHGA
HTVELNLEPSQVGNEFAEKYYGPASQVVPEFVEKLLKGLKAGSIAEF (SEQ ID NO:104)
HST2 (C. *albicans*): 
ASSPKKKRKVEASMPSLDDILKPVAEAVKNGKKVTFFNGAGISTGAGIPDFRSPDTGLYANLAKLNLPFA
EAVFDIDFFKEDPKPFYTLAEELYPGNFAPTKFHHFIKLLQDQGSLKRVYTQNIDTLERLAGVEDKYIVE
AHGSFASNHCVDCHKEMTTETLKTYMKDKKIPSCQHCEGYVKPDIVFFGEGLPVKFFDLWEDDCEDVEVA
IVAGTSLTVFPFASLPGEVNKKCLRVLVNKEKVGTFKHEPRKSDIIALHDCDIVAERLCTLLGLDDKLNE
VYEKEKIKYSKAETKEIKMHEIEDKLKEEAHLKEDKHTTKVDKKEKQNDANDKELEQLIDKAKAEF
(SEQ ID NO:105)
SIRT5 (human): 
ASSPKKKRKVEASSSSMADFRKFFAKAKHIVIISGAGVSAESGVPTFRGAGGYWRKWQAQDLATPLAFAH
NPSRVWEFYHYRREVMGSKEPNAGHRAIAECETRLGKQGRRVVVITQNIDELHRKAGTKNLLEIHGSLFK
TRCTSCGVVAENYKSPICPALSGKGAPEPGTQDASIPVEKLPRCEEAGCGGLLRPHVVWFGENLDPAILE
EVDRELAHCDLCLVVGTSSVVYPAAMFAPQVAARGVPVAEFNTETTPATNRFRFHFQGPCGTTLPEALAC
HENETVSEF (SEQ ID NO:106)
Sir2A (*P. falciparum*): 
ASSPKKKRKVEASMGNLMISFLKKDTQSITLEELAKIIKKCKHVVALTGSGTSAESNIPSFRGSSNSIWS
KYDPRIYGTIWGFWKYPEKIWEVIRDISSDYEIEINNGHVALSTLESLGYLKSVVTQNVDGLHEASGNTK
VISLHGNVFEAVCCTCNKIVKLNKIMLQKTSHFMHQLPPECPCGGIFKPNIILFGEVVSSDLLKEAEEEI
AKCDLLLVIGTSSTVSTATNLCHFACKKKKKIVEINISKTYITNKMSDYHVCAKFSELTKVANILKGSSE
KNKKIMEF (SEQ ID NO:107)
SIRT6 (human):

ASSPKKKRKVEASMSVNYAAGLSPYADKGKCGLPEIFDPPEELERKVWELARLVWQSSSVVFHTGAGIST
ASGIPDFRGPHGVWTMEERGLAPKFDTTFESARPTQTHMALVQLERVGLLRFLVSQNVDGLHVRSGFPRD
KLAELHGNMFVEECAKCKTQYVRDTVVGTMGLKATGRLCTVAKARGLRACRGELRDTILDWEDSLPDRDL
ALADEASRNADLSITLGTSLQIRPSGNLPLATKRRGGRLVIVNLQPTKHDRHADLRIHGYVDEVMTRLMK
HLGLEIPAWDGPRVLERALPPLEF (SEQ ID NO:108)

### HMT effector domains:

NUE (C. *trachomatis*): 
ASSPKKKRKVEASMTTNSTQDTLYLSLHGGIDSAIPYPVRRVEQLLQFSFLPELQFQNAAVKQRIQRLCY
REEKRLAVSSLAKWLGQLHKQRLRAPKNPPVAICWINSYVGYGVFARESIPAWSYIGEYTGILRRRQALW
LDENDYCFRYPVPRYSFRYFTIDSGMQGNVTRFINHSDNPNLEAIGAFENGIFHIIIRAIKDILPGEELC
YHYGPLYWKHRKKREEFVPQEEEF (SEQ ID NO:109)
vSET (*P. bursaria chlorella virus*): 
ASSPKKKRKVEASMFNDRVIVKKSPLGGYGVFARKSFEKGELVEECLCIVRHNDDWGTALEDYLFSRKNM
SAMALGFGAIFNHSKDPNARHELTAGLKRMRIFTIKPIAIGEEITISYGDDYWLSRPRLTQNEF (SEQ
ID NO:110)
SUV39H1 (human): 
ASSPKKKRKVEASNLKCVRILKQFHKDLERELLRRHHRSKTPRHLDPSLANYLVQKAKQRRALRRWEQEL
NAKRSHLGRITVENEVDLDGPPRAFVYINEYRVGEGITLNQVAVGCECQDCLWAPTGGCCPGASLHKFAY
NDQGQVRLRAGLPIYECNSRCRCGYDCPNRVVQKGIRYDLCIFRTDDGRGWGVRTLEKIRKNSFVMEYVG
EIITSEEAERRGQIYDRQGATYLFDLDYVEDVYTVDAAYYGNISHFVNHSCDPNLQVYNVFIDNLDERLP
RIAFFATRTIRAGEELTFDYNMQVDPVDMESTRMDSNFGLAGLPGSPKKRVRIECKCGTESCRKYLFEF
(SEQ ID NO:111)
DIM5 (*N. crassa*): 
ASSPKKKRKVEASMEKAFRPHFFNHGKPDANPKEKKNCHWCQIRSFATHAQLPISIVNREDDAFLNPNFR
FIDHSIIGKNVPVADQSFRVGCSCASDEECMYSTCQCLDEMAPDSDEEADPYTRKKRFAYYSQGAKKGLL
RDRVLQSQEPIYECHQGCACSKDCPNRVVERGRTVPLQIFRTKDRGWGVKCPVNIKRGQFVDRYLGEIIT
SEEADRRRAESTIARRKDVYLFALDKFSDPDSLDPLLAGQPLEVDGEYMSGPTRFINHSCDPNMAIFARV
GDHADKHIHDLALFAIKDIPKGTELTFDYVNGLTGLESDAHDPSKISEMTKCLCGTAKCRGYLWEF
(SEQ ID NO:112)
KYP (*A. thaliana*): 
ASSPKKKRKVEASDISGGLEFKGIPATNRVDDSPVSPTSGFTYIKSLIIEPNVIIPKSSTGCNCRGSCTD
SKKCACAKLNGGNFPYVDLNDGRLIESRDVVFECGPHCGCGPKCVNRTSQKRLRFNLEVFRSAKKGWAVR
SWEYIPAGSPVCEYIGVVRRTADVDTISDNEYIFEIDCQQTMQGLGGRQRRLRDVAVPMNNGVSQSSEDE
NAPEFCIDAGSTGNFARFINHSCEPNLFVQCVLSSHQDIRLARVVLFAADNISPMQELTYDYGYALDSVH
EF (SEQ ID NO:113)
SUVR4 (*A. thaliana*): 
ASSPKKKRKVEASQSAYLHVSLARISDEDCCANCKGNCLSADFPCTCARETSGEYAYTKEGLLKEKFLDT
CLKMKKEPDSFPKVYCKDCPLERDHDKGTYGKCDGHLIRKFIKECWRKCGCDMQCGNRVVQRGIRCQLQV
YFTQEGKGWGLRTLQDLPKGTFICEYIGEILTNTELYDRNVRSSSERHTYPVTLDADWGSEKDLKDEEAL
CLDATICGNVARFINHRCEDANMIDIPIEIETPDRHYYHIAFFTLRDVKAMDELTWDYMIDFNDKSHPVK
AFRCCCGSESCRDRKIKGSQGKSIERRKIVSAKKQQGSKEVSKKRKEF (SEQ ID NO:114)
Set4 (C. *elegans*): 
ASSPKKKRKVEASMQLHEQIANISVTFNDIPRSDHSMTPTELCYFDDFATTLVVDSVLNFTTHKMSKKRR
YLYQDEYRTARTVMKTFREQRDWTNAIYGLLTLRSVSHFLSKLPPNKLFEFRDHIVRFLNMFILDSGYTI
QECKRYSQEGHQGAKLVSTGVWSRGDKIERLSGVVCLLSSEDEDSILAQEGSDFSVMYSTRKRCSTLWLG
PGAYINHDCRPTCEFVSHGSTAHIRVLRDMVPGDEITCFYGSEFFGPNNIDCECCTCEKNMNGAFSYLRG
NENAEPIISEKKTKYELRSRSEF (SEQ ID NO:115)
Set1 (C. *elegans*): 
ASSPKKKRKVEASMKVAAKKLATSRMRKDRAAAASPSSDIENSENPSSLASHSSSSGRMTPSKNTRSRKG
VSVKDVSNHKITEFFQVRRSNRKTSKQISDEAKHALRDTVLKGTNERLLEVYKDVVKGRGIRTKVNFEKG
DFVVEYRGVMMEYSEAKVIEEQYSNDEEIGSYMYFFEHNNKKWCIDATKESPWKGRLINHSVLRPNLKTK
VVEIDGSHHLILVARRQIAQGEELLYDYGDRSAETIAKNPWLVNTEF (SEQ ID NO:116)
SETD8 (human) 
ASSPKKKRKVEASSCDSTNAAIAKQALKKPIKGKQAPRKKAQGKTQQNRKLTDFYPVRRSSRKSKAELQS
EERKRIDELIESGKEEGMKIDLIDGKGRGVIATKQFSRGDFVVEYHGDLIEITDAKKREALYAQDPSTGC
YMYYFQYLSKTYCVDATRETNRLGRLINHSKCGNCQTKLHDIDGVPHLILIASRDIAAGEELLYDYGDRS
KASIEAFPWLKHEF (SEQ ID NO:117)
TgSET8 (*T. gondii*):

ASSPKKKRKVEASASRRTGEFLRDAQAPSRWLKRSKTGQDDGAFCLETWLAGAGDDAAGGERGRDREGAA
DKAKQREERRQKELEERFEEMKVEFEEKAQRMIARRAALTGEIYSDGKGSKKPRVPSLPENDDDALIEII
IDPEQGILKWPLSVMSIRQRTVIYQECLRRDLTACIHLTKVPGKGRAVFAADTILKDDFVVEYKGELCSE
REAREREQRYNRSKVPMGSFMFYFKNGSRMMAIDATDEKQDFGPARLINHSRRNPNMTPRAITLGDFNSE
PRLIFVARRNIEKGEELLVDYGERDPDVIKEHPWLNSEF (SEQ ID NO:118)

Those of skill in the art will understand that any of the exemplary Cas9 proteins, including the exemplary Cas9 nucleases, variants, and fusions thereof, e.g., described herein, can be delivered to cells using the instantly disclosed technology, and that the disclosure is not limited in this respect.

### Nuclease effector proteins

TALE nucleases, or TALENs, are artificial nucleases comprising a transcriptional activator-like effector DNA binding domain associated with a DNA cleavage domain, for example, a FokI domain. A number of modular assembly schemes for generating engineered TALE constructs have been reported (Zhang, Feng; et.al. (February 2011). "Efficient construction of sequence-specific TAL effectors for modulating mammalian transcription". Nature Biotechnology 29 (2): 149-53; Geiβler, R.; Scholze, H.; Hahn, S.; Streubel, J.; Bonas, U.; Behrens, S. E.; Boch, J. (2011), Shiu, Shin-Han. ed. "Transcriptional Activators of Human Genes with Programmable DNA-Specificity". PLoS ONE 6 (5): e19509; Cermak, T.; Doyle, E. L.; Christian, M.; Wang, L.; Zhang, Y.; Schmidt, C.; Baller, J. A.; Somia, N. V. et al. (2011). "Efficient design and assembly of custom TALEN and other TAL effector-based constructs for DNA targeting". Nucleic Acids Research*;* Morbitzer, R.; Elsaesser, J.; Hausner, J.; Lahaye, T. (2011). "Assembly of custom TALE-type DNA binding domains by modular cloning". Nucleic Acids Research*;* Li, T.; Huang, S.; Zhao, X.; Wright, D. A.; Carpenter, S.; Spalding, M. H.; Weeks, D. P.; Yang, B. (2011). "Modularly assembled designer TAL effector nucleases for targeted gene knockout and gene replacement in eukaryotes". Nucleic Acids Research*:,* Weber, E.; Gruetzner, R.; Werner, S.; Engler, C.; Marillonnet, S. (2011). Bendahmane, Mohammed. ed. "Assembly of Designer TAL Effectors by Golden Gate Cloning". PLoS ONE 6 (5): e19722; the entire contents of each of which are incorporated herein by reference). Those of skill in the art will understand that TALE nucleases can be engineered to target virtually any genomic sequence with high specificity, and that such engineered nucleases can be used in embodiments of the present technology to manipulate the genome of a cell, *e.g.,* by delivering the respective TALEN via a method or strategy disclosed herein under circumstances suitable for the TALEN to bind and cleave its target sequence within the genome of the cell. In some embodiments, the delivered TALEN targets a gene or allele associated with a disease or disorder. In some embodiments, delivery of the TALEN to a subject confers a therapeutic benefit to the subject.

Zinc finger nucleases are a class of artificial nucleases that comprise a DNA cleavage domain and a zinc finger DNA binding domain. In some embodiments, the DNA cleavage domain is a non-specific DNA cleavage domain of a restriction endonuclease, for example, of FokI. In some embodiments, the DNA cleavage domain is a domain that only cleaves double-stranded DNA when dimerized with a second DNA cleavage domain of the same type. In some embodiments, the DNA cleavage domain is fused to the C-terminus of the zinc finger domain via a linker, for example, a peptide linker. In some embodiments, the zinc finger domain comprises between about 3 and about 6 zinc fingers and specifically recognizes and binds a target sequence of about 9-20 nucleotides in length. In some embodiments, a plurality of zinc finger nuclease molecules is delivered to a target cell by a system or method provided by this invention, with the zinc finger domain of one zinc finger nuclease molecule binding a target sequence in close proximity of the target sequence of a second zinc finger nuclease molecule. In some embodiments, the zinc finger domains of the zinc finger nuclease molecules binding target sequences in close proximity to each other are different. In some embodiments, a zinc finger nuclease molecule delivered to a cell by a system or method provided herein binds a target nucleic acid sequence in close proximity to the target sequence of another zinc finger nuclease molecule, so that the DNA cleavage domains of the molecules dimerize and cleave a DNA molecule at a site between the two target sequences.

In some embodiments, the genome of the target cell is edited by a nuclease delivered to the cell via a strategy or method disclosed herein, *e.g.,* by a TALEN, or a zinc-finger nuclease, or a plurality or combination of such nucleases. In some embodiments, a single- or double-strand break is introduced at a specific site within the genome of a target cell by the nuclease, resulting in a disruption of the targeted genomic sequence. In some embodiments, the targeted genomic sequence is a nucleic acid sequence within the coding region of a gene. In some embodiments, the strand break introduced by the nuclease leads to a mutation within the target gene that impairs the expression of the encoded gene product. In some embodiments, a nucleic acid is co-delivered to the cell with the nuclease. In some embodiments, the nucleic acid comprises a sequence that is identical or homologous to a sequence adjacent to the nuclease target site. In some such embodiments, the strand break effected by the nuclease is repaired by the cellular DNA repair machinery to introduce all or part of the co-delivered nucleic acid into the cellular DNA at the break site, resulting in a targeted insertion of the co-delivered nucleic acid, or part thereof. In some embodiments, the insertion results in the disruption or repair of a pathogenic allele. In some embodiments, the insertion is detected by a suitable assay, *e.g.,* a DNA sequencing assay, a southern blot assay, or an assay for a reporter gene encoded by the co-delivered nucleic acid, *e.g.,* a fluorescent protein or resistance to an antibiotic. In some embodiments, the nucleic acid is co-delivered by association to a supercharged protein. In some embodiments, the supercharged protein is also associated to the functional effector protein, e.g., the nuclease. In some embodiments, the delivery of a nuclease to a target cell results in a clinically or therapeutically beneficial disruption of the function of a gene.

In some embodiments, cells from a subject are obtained and a nuclease or other effector protein is delivered to the cells by a system or method provided herein *ex vivo.* In some embodiments, the treated cells are selected for those cells in which a desired nuclease-mediated genomic editing event has been effected. In some embodiments, treated cells carrying a desired genomic mutation or alteration are returned to the subject they were obtained from.

Methods for engineering, generation, and isolation of nucleases targeting specific sequences, e.g., TALE, or zinc finger nucleases, and editing cellular genomes at specific target sequences, are well known in the art (see, *e.g.,* Mani et al., Biochemical and Biophysical Research Communications 335:447-457, 2005; Perez et al., Nature Biotechnology 26:808-16, 2008; Kim et al., Genome Research, 19:1279-88, 2009; Urnov et al., Nature 435:646-51, 2005; Carroll et al., Gene Therapy 15:1463-68, 2005; Lombardo et al., Nature Biotechnology 25:1298-306, 2007; Kandavelou et al., Biochemical and Biophysical Research Communications 388:56-61, 2009; and Hockemeyer et al., Nature Biotechnology 27(9):851-59, 2009, as well as the reference recited in the respective section for each nuclease). The skilled artisan will be able to ascertain suitable methods for use in the context of the present disclosure based on the guidance provided herein.

### TALE effector proteins

In some embodiments, effector proteins comprising a TALE domain are delivered to a target cell by a system or method provided herein. In some embodiments, a TALE effector, *e.g.,* an engineered TALE transcription factor comprising a TALE DNA binding domain and a heterologous transcriptional activator or repressor domain, is delivered to a cell by a system or method provided by aspects of this invention. In some embodiments, the TALE effector, *e.g.,* a transcription factor, is delivered to a cell in an amount sufficient to activate or inhibit transcription of a target gene of the transcription factor within the cell. In some embodiments, a transcription factor is delivered in an amount and over a time period sufficient to effect a change in the phenotype of a target cell, for example, a change in cellular function, or a change in developmental potential. Exemplary TALE transcription factors are described herein, and the skilled artisan will be able to identify additional suitable TALE transcription factors based on the guidance provided herein and the knowledge of such TALE transcription factors in the art.

In some embodiments, a target cell, for example, a somatic cell, is contacted with a TALE transcription factor, or a combination of such factors, associated with a supercharged protein provided herein. In some embodiments the target cell is a primary somatic cell and is contacted *in vitro* or *ex vivo* with a TALE transcription factor associated with a supercharged protein. In some embodiments, the TALE transcription factor is associated with a positively charged supercharged protein, *e.g.,* as described herein. In some embodiments, the TALE transcription factor is associated with a negatively charged supercharged proteins, *e.g.,* as described herein. In some embodiments, the TALE transcription factor is associated with a cationic lipid and/or cationic polymer, *e.g.,* as described herein. In some embodiments, the TALE transcription factor is associated with a negatively charged supercharged protein and a cationic lipid and/or cationic polymer, *e.g.,* as described herein.

In some embodiments, a target cell is contacted, or repeatedly contacted, with a TALE transcription factor associated with a supercharged protein (and optionally a cationic lipid and/or cationic polymer) as provided herein, and a desired change in cellular phenotype or gene expression is detected. In some embodiments, a target cell is contacted repeatedly with a TALE transcription factor associated with a supercharged protein (and optionally a cationic lipid and/or cationic polymer) as provided herein until the formation of a desired cellular phenotype is detected. Methods for detecting cellular phenotypes and gene expression are well known to those in the art and include, for example, morphological analysis, and detection of marker gene expression by well-established methods such as immunohistochemistry, fluorescence activated cell sorting (FACS), or fluorescent microscopy. In some embodiments, a target cell is contacted with a TALE transcription factor associated with a supercharged protein as provided herein for a period of at least 3 hours, at least 6 hours, at least 12 hours, at least 1 day, at least 2 days, at least 3 days, at least 4 days, at least 5 days, at least 6 days, at least 7 days, at least 10-12 days, at least 12-15 days, at least 15-20 days, at least 20-25 days, at least 25-30 days, at least 30-40 days, at least 40-50 days, at least 50-60 days, at least 60-70, or at least 70-100 days.

In some embodiments, a target cell is contacted with a TALE transcription factor associated with a supercharged protein (and optionally a cationic lipid and/or cationic polymer) as provided herein in an amount and for a time period effective to program the cell towards a different cell state. As will be apparent to those of skill in the art, the amount necessary to program or re-program a cell will dependent on various factors, for example, on the cell type and the treatment schedule. In general, delivery of a TALE transcription factor to a target somatic cell by a system or method provided herein will be at a concentration below a concentration at which significant toxicity can be observed. The critical concentration will depend, for example, on the specific TALE transcription factor, the supercharged protein it is associated with, the type of association, and the type of cell being treated.

A useful concentration of a functional effector protein associated with a supercharged protein (and optionally a cationic lipid and/or cationic polymer) for delivery to a specific cell type can be established by those of skill in the art by routine experimentation. In some embodiments a target cell is contacted *in vitro* or *ex vivo* with a functional effector protein associated with a supercharged protein (and optionally a cationic lipid and/or cationic polymer) at a concentration of about 1 pM to about 1 µM. In some embodiments, a target cell is contacted *in vitro* or *ex vivo* with the functional effector protein associated to a supercharged protein at a concentration of about 1 pM, about 2.5 pM, about 5 pM, about 7.5 pM, about 10 pM, about 20 pM, about 25 pM, about 30 pM, about 40 pM, about 50 pM, about 60 pM, about 70 pM, about 75 pM, about 80 pM, about 90 pM, about 100 pM, about 200 pM, about 250 pM, about 300 pM, about 400 pM, about 500 pM, about 600 pM, about 700 pM, about 750 pM, about 800 pM, about 900 pM, about 1 nM, about 2nM, about 3nM, about 4nM, about 5nM, about 6nM, about 7 nM, about 8 nM, about 9nM, about 10nM, about 20nM, about 25 nM, about 30 nM, about 40 nM, about 50 nM, about 60 nm, about 70 nM, about 75 nM, about 80 nM, about 90 nM, about 100 nM, about 200 nM, about 250 nM, about 300 nM, about 400 nM, about 500 nM, about 600 nM, about 700 nM, about 750 nM, about 800 nM, about 900 nM, or about 1 µM. A useful time of exposure of the target cell to the functional effector protein, and, if necessary, incubation after administration in the absence of the functional effector protein, as well as a number of administration/incubation cycles useful to achieve a desired biological effect (*e.g.,* change in gene transcription, cleavage of a target site by a delivered nuclease, *etc*.)*,* or a desired cellular phenotype can also be established by those of skill in the art by routine experimentation.

In some embodiments, the target cell for delivery of a functional effector protein by a system or method provided herein, is a primary cell obtained by a biopsy from a subject. In some embodiments, the subject is diagnosed as having a disease. In some embodiments the disease is a degenerative disease characterized by diminished function of a specific cell type, for example, a neural cell. In some embodiments, a cell treated with a functional effector protein according to the strategies or methods disclosed herein, or the progeny of such a cell, is used in a cell-replacement therapeutic approach. In some embodiments, the treated cells are administered to the subject from which the somatic cell was obtained in an autologous cell replacement therapeutic approach.

In some embodiments, a functional effector protein, *e.g.,* TALE transcription factor able to convert a cell from one differentiated state into another, is delivered to a target cell *in vitro* or *in vivo* by a system or method provided herein. Transcription factors that effect transdifferentiation are known in the art (see, *e.g.,* Zhou et al., Nature 455:627-33, 2008). In some embodiments, a TALE transcription factor modulating the expression of PPARy or PRDM16 are delivered to fibroblast cells by a system or method as provided by this invention. It is known in the art that expression these transcription factors is a pivotal step in the programming of fibroblasts towards a brown fat or white fat cell state. In some embodiments, a programmed brown fat cell is generated from a fibroblast obtained from a subject in need of brown fat cells, and is administered to the subject, *e.g.,* used in a cell-replacement therapeutic approach involving the subject.

### Formation of Complexes

The present invention provides complexes comprising supercharged proteins associated with one or more functional effector proteins to be delivered. In some embodiments, supercharged proteins are associated with one or more functional effector proteins to be delivered through non-covalent interactions. In some embodiments, supercharged proteins are associated with one or more functional effector proteins through electrostatic interactions. In certain embodiments, supercharged proteins have an overall net positive charge, and the functional effector proteins to be delivered have an overall net negative charge. In some embodiments, the complex further comprises a cationic lipid and/or cationic polymer. For example, in some embodiments, the supercharged protein of the complex is supernegatively charged, allowing for association with cationic lipids and/or polymers.

In certain embodiments, supercharged proteins are associated with one or more functional effector proteins to be delivered via covalent bond. For example, a supercharged protein may be fused to a functional effector protein to be delivered. Covalent attachment may be direct or indirect (*e.g.,* through a linker). In some embodiments, a covalent attachment is mediated through one or more linkers. In some embodiments, the linker is a cleavable linker . In certain embodiments, the cleavable linker comprises an amide, ester, or disulfide bond. For example, the linker may be an amino acid sequence that is cleavable by a cellular enzyme. In certain embodiments, the enzyme is a protease. In other embodiments, the enzyme is an esterase. In some embodiments, the enzyme is one that is more highly expressed in certain cell types than in other cell types. For example, the enzyme may be one that is more highly expressed in tumor cells than in non-tumor cells. Exemplary linkers and enzymes that cleave those linkers are presented below.

### Cleavable Linkers

| **Linker Sequence** | **Enzyme(s) Targeting Linker** |
|---|---|
| X¹-AGVF-X (SEQ ID NO: 256) | lysosomal thiol proteinases (see, *e.g.,* Duncan et al., 1982, Biosci. Rep., 2:1041-46; incorporated herein by reference) |
| X-GFLG-X (SEQ ID NO: 257) | lysosomal cysteine proteinases (see, *e.g.,* Vasey et al., Clin. Canc. Res., 1999, 5:83-94; incorporated herein by reference) |
| X-FK-X | Cathepsin B - ubiquitous, overexpressed in many solid tumors, such as breast cancer (see, *e.g.,* Dubowchik et al., 2002, Bioconjugate Chem., 13:855-69; incorporated herein by reference) |
| X-A*L-X | Cathepsin B - ubiquitous, overexpressed in many solid tumors, such as breast cancer (see, *e.g.,* Trouet et al., 1982, Proc. Natl. Acad. Sci., USA, 79:626-29; incorporated herein by reference) |
| X-A*LA*L-X (SEQ ID NO: 258) | Cathepsin B - ubiquitous, overexpressed in many solid tumors (see, *e.g.,* Schmid et al., 2007, Bioconjugate Chem, 18:702-16; incorporated herein by reference) |
| X-AL* AL* A-X (SEQ ID NO: 259) | Cathepsin D - ubiquitous (see, *e.g.,* Czerwinski et al., 1998, Proc. Natl. Acad. Sci., USA, 95: 11520-25; incorporated herein by reference) |

| | |
|---|---|
| ¹ X denotes a supercharged protein or a functional effector protein to be delivered * refers to observed cleavage site | |

To give but one particular example, a +36 GFP may be associated with a functional effector protein to be delivered by a cleavable linker, such as ALAL (SEQ ID NO: 254), to generate +36 GFP-(GGS)₄-ALAL-(GGS)₄-[functional effector protein X] (SEQ ID NO: 255).

In certain embodiments, the functional effector protein to be delivered is contacted with the supercharged protein to form a complex. In some embodiments, formation of complexes is carried out at or around pH 7. In some embodiments, formation of complexes is carried out at about pH 5, about pH 6, about pH 7, about pH 8, or about pH 9. Formation of complexes is typically carried out at a pH that does not negatively affect the function of the supercharged protein and/or the functional effector protein. In some embodiments, formation of complexes is carried out at room temperature. In some embodiments, formation of complexes is carried out at or around 37 °C. In some embodiments, formation of complexes is carried out below 4 °C, at about 4 °C, at about 10 °C, at about 15 °C, at about 20 °C, at about 25 °C, at about 30 °C, at about 35 °C, at about 37 °C, at about 40 °C, or higher than 40 °C. Formation of complexes is typically carried out at a temperature that does not negatively affect the function of the supercharged protein and/or functional effector protein. In some embodiments, formation of complexes is carried out in serum-free medium. In some embodiments, formation of complexes is carried out in the presence of CO₂ (*e.g.,* about 1%, about 2%, about 3%, about 4%, about 5%, about 6%, or more).

In some embodiments, formation of complexes is carried out using concentrations of functional effector protein of about 100 nM. In some embodiments, formation of complexes is carried out using concentrations of functional effector protein of about 25 nM, about 50 nM, about 75 nM, about 90 nM, about 100 nM, about 110 nM, about 125 nM, about 150 nM, about 175 nM, or about 200 nM. In some embodiments, formation of complexes is carried out using concentrations of supercharged protein of about 40 nM. In some embodiments, formation of complexes is carried out using concentrations of supercharged protein of about 10 nM, about 20 nM, about 30 nM, about 40 nM, about 50 nM, about 60 nM, about 70 nM, about 80 nM, about 90 nM, or about 100 nM.

In some embodiments, formation of complexes is carried out under conditions of excess functional effector protein. In some embodiments, formation of complexes is carried out with ratios of functional effector protein: supercharged protein of about 20:1, about 10:1, about 9:1, about 8:1, about 7:1, about 6:1, about 5:1, about 4:1, about 3:1, about 2: 1, or about 1:1. In some embodiments, formation of complexes is carried out with ratios of functional effector protein: supercharged protein of about 3:1. In some embodiments, formation of complexes is carried out with ratios of supercharged protein: functional effector protein of about 20:1, about 10:1, about 9:1, about 8:1, about 7:1, about 6:1, about 5:1, about 4:1, about 3:1, about 2:1, or about 1:1.

In some embodiments, formation of complexes is carried out by mixing supercharged protein with functional effector protein, and agitating the mixture (*e.g.,* by inversion). In some embodiments, formation of complexes is carried out by mixing supercharged protein with functional effector protein, and allowing the mixture to sit still. In some embodiments, the formation of the complex is carried out in the presence of a pharmaceutically acceptable carrier or excipient. In some embodiments, the complex is further combined with a pharmaceutically acceptable carrier or excipient. Exemplary excipients or carriers include water, solvents, lipids, proteins, peptides, endosomolytic agents (*e.g.,* chloroquine, pyrene butyric acid), small molecules, carbohydrates, buffers, natural polymers, synthetic polymers (*e.g.,* PLGA, polyurethane, polyesters, polycaprolactone, polyphosphazenes), pharmaceutical agents, *etc.*

In some embodiments, complexes comprising supercharged protein and functional effector protein may migrate more slowly in gel electrophoresis assays than either the supercharged protein alone or the functional effector protein alone.

### Applications

The present invention provides compositions comprising supercharged proteins, naturally occurring or engineered, associated with functional effector proteins (*e.g.,* nucleases, transcriptional activators/repressors, recombinases, Cas9 proteins including variants and fusions thereof, *etc*.) to be delivered to a cell, as well as methods of using such compositions and uses of such compositions. In certain embodiments, compositions are provided comprising a Cas9 protein (e*.g.,* wherein the Cas9 protein is associated with a gRNA) and a cationic lipid. In certain embodiments, compositions are provided comprising a Cas9 protein (*e.g.,* wherein the Cas9 protein is associated with a gRNA) and a cationic polymer. The inventive compositions may be used to treat or prevent any disease that can benefit, *e.g.,* from the delivery of an agent to a cell. The inventive compositions may also be used to transfect or treat cells for research purposes.

In some embodiments, compositions in accordance with the invention may be used for research purposes, *e.g.,* to efficiently deliver functional effector proteins to cells in a research context. In some embodiments, compositions in accordance with the present invention may be used for therapeutic purposes. In some embodiments, compositions in accordance with the present invention may be used for treatment of any of a variety of diseases, disorders, and/or conditions, including, but not limited to, one or more of the following: autoimmune disorders (*e.g.,* diabetes, lupus, multiple sclerosis, psoriasis, rheumatoid arthritis); inflammatory disorders *(e.g.,* arthritis, pelvic inflammatory disease); infectious diseases (*e.g.,* viral infections (*e.g.,* HIV, HCV, RSV), bacterial infections, fungal infections, sepsis); neurological disorders (*e.g.* Alzheimer's disease, Huntington's disease; autism; Duchenne muscular dystrophy); cardiovascular disorders (*e.g.* atherosclerosis, hypercholesterolemia, thrombosis, clotting disorders, angiogenic disorders such as macular degeneration); proliferative disorders (*e.g.* cancer, benign neoplasms); respiratory disorders (*e.g.* chronic obstructive pulmonary disease); digestive disorders (*e.g.* inflammatory bowel disease, ulcers); musculoskeletal disorders *(e.g.* fibromyalgia, arthritis); endocrine, metabolic, and nutritional disorders (*e.g.* diabetes, osteoporosis); urological disorders (*e.g.* renal disease); psychological disorders *(e.g.* depression, schizophrenia); skin disorders (*e.g.* wounds, eczema); blood and lymphatic disorders *(e.g.* anemia, hemophilia); *etc.*

Compositions of the invention may be used in a clinical setting. For example, a supercharged protein may be associated with a functional effector protein that can be used for therapeutic applications. Such functional effector protein may be, for example, nucleases or transcriptional activators. Other compositions comprising a Cas9 protein and a cationic lipid may also be used for therapeutic applications.

In some embodiments, the supercharged protein or functional effector protein associated with a supercharged protein includes a detectable label. These molecules can be used in detection, imaging, disease staging, diagnosis, or patient selection. Suitable labels include fluorescent, chemiluminescent, enzymatic labels, colorimetric, phosphorescent, density-based labels, *e.g.,* labels based on electron density, and in general contrast agents, and/or radioactive labels.

### Pharmaceutical Compositions

The present invention provides compositions comprising supercharged proteins associated with at least one functional effector protein to be delivered, and in some embodiments are encapsulated by cationic lipids. Other compositions comprising a Cas9 protein and a cationic lipid are provided. Thus, the present invention provides pharmaceutical compositions comprising one or more supercharged proteins associated with a functional effector protein, and/or one or more functional effector proteins associated with a cationic lipid and/or cationic polymer, and one or more pharmaceutically acceptable excipients. Pharmaceutical compositions may optionally comprise one or more additional therapeutically active substances. In accordance with some embodiments, a method of administering pharmaceutical compositions comprising one or more supercharged proteins associated with a functional effector protein to be delivered to a subject in need thereof is provided. In some embodiments, compositions are administered to humans. For the purposes of the present disclosure, the phrase "active ingredient" generally refers to a Cas9 protein and/or supercharged protein associated with a functional effector protein, or to the functional effector protein to be delivered as described herein.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Subjects to which administration of the pharmaceutical compositions is contemplated include, but are not limited to, humans and/or other primates; mammals, including commercially relevant mammals such as cattle, pigs, horses, sheep, cats, dogs, mice, and/or rats; and/or birds, including commercially relevant birds such as chickens, ducks, geese, and/or turkeys.

Formulations of the pharmaceutical compositions described herein may be prepared by any method known or hereafter developed in the art of pharmacology. In general, such preparatory methods include the step of bringing the active ingredient into association with an excipient and/or one or more other accessory ingredients, and then, if necessary and/or desirable, shaping and/or packaging the product into a desired single- or multi-dose unit.

A pharmaceutical composition in accordance with the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition in accordance with the invention will vary, depending upon the identity, size, and/or condition of the subject treated and further depending upon the route by which the composition is to be administered. By way of example, the composition may comprise between 0.1% and 100% (w/w) active ingredient.

Pharmaceutical formulations may additionally comprise a pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro (Lippincott, Williams & Wilkins, Baltimore, MD, 2006; incorporated herein by reference) discloses various excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

In some embodiments, a pharmaceutically acceptable excipient is at least 95%, at least 96%, at least 97%, at least 98%, at least 99%, or 100% pure. In some embodiments, an excipient is approved for use in humans and for veterinary use. In some embodiments, an excipient is approved by United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in pharmaceutical formulations. Excipients such as cocoa butter and suppository waxes, coloring agents, coating agents, sweetening, flavoring, and/or perfuming agents can be present in the composition, according to the judgment of the formulator.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, *etc.,* and/or combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked poly(vinyl-pyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, *etc.,* and/or combinations thereof.

Exemplary surface active agents and/or emulsifiers include, but are not limited to, natural emulsifiers (*e.g.* acacia, agar, alginic acid, sodium alginate, tragacanth, chondrux, cholesterol, xanthan, pectin, gelatin, egg yolk, casein, wool fat, cholesterol, wax, and lecithin), colloidal clays (*e.g.* bentonite [aluminum silicate] and Veegum^{®} [magnesium aluminum silicate]), long chain amino acid derivatives, high molecular weight alcohols (*e.g.* stearyl alcohol, cetyl alcohol, oleyl alcohol, triacetin monostearate, ethylene glycol distearate, glyceryl monostearate, and propylene glycol monostearate, polyvinyl alcohol), carbomers (*e.g.* carboxy polymethylene, polyacrylic acid, acrylic acid polymer, and carboxyvinyl polymer), carrageenan, cellulosic derivatives (*e.g.* carboxymethylcellulose sodium, powdered cellulose, hydroxymethyl cellulose, hydroxypropyl cellulose, hydroxypropyl methylcellulose, methylcellulose), sorbitan fatty acid esters (*e.g.* polyoxyethylene sorbitan monolaurate (Tween^{®}20), polyoxyethylene sorbitan (Tween^{®}60), polyoxyethylene sorbitan monooleate [Tween^{®}80], sorbitan monopalmitate [Span^{®}40], sorbitan monostearate [Span^{®}60], sorbitan tristearate [Span^{®}65], glyceryl monooleate, sorbitan monooleate [Span^{®}80]), polyoxyethylene esters (*e.g.* polyoxyethylene monostearate [Myrj^{®}45], polyoxyethylene hydrogenated castor oil, polyethoxylated castor oil, polyoxymethylene stearate, and Solutol^{®}), sucrose fatty acid esters, polyethylene glycol fatty acid esters *(e.g.* Cremophor^{®}), polyoxyethylene ethers, *(e.g.* polyoxyethylene lauryl ether (Brij^{®}30)), poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic^{®}F 68, Poloxamer^{®}188, cetrimonium bromide, cetylpyridinium chloride, benzalkonium chloride, docusate sodium, *etc.* and/or combinations thereof.

Exemplary binding agents include, but are not limited to, starch (*e.g.* cornstarch and starch paste); gelatin; sugars (*e.g.* sucrose, glucose, dextrose, dextrin, molasses, lactose, lactitol, mannitol,); natural and synthetic gums (*e.g.* acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, poly(vinyl-pyrrolidone), magnesium aluminum silicate (Veegum^{®}), and larch arabogalactan); alginates; polyethylene oxide; polyethylene glycol; inorganic calcium salts; silicic acid; polymethacrylates; waxes; water; alcohol; *etc.;* and combinations thereof.

Exemplary preservatives may include, but are not limited to, antioxidants, chelating agents, antimicrobial preservatives, antifungal preservatives, alcohol preservatives, acidic preservatives, and/or other preservatives. Exemplary antioxidants include, but are not limited to, alpha tocopherol, ascorbic acid, ascorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and/or sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and/or trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and/or thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and/or sorbic acid. Exemplary alcohol preservatives include, but are not limited to, ethanol, polyethylene glycol, phenol, phenolic compounds, bisphenol, chlorobutanol, hydroxybenzoate, and/or phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and/or phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus^{®}, Phenonip^{®}, methylparaben, Germall^{®}115, Germaben^{®}II, Neolone^{™}, Kathon^{™}, and/or Euxyl^{®}.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, *etc.,* and/or combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, *etc.,* and combinations thereof.

Exemplary oils include, but are not limited to, almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and/or combinations thereof.

Liquid dosage forms for oral and parenteral administration include, but are not limited to, pharmaceutically acceptable emulsions, microemulsions, solutions, suspensions, syrups, and/or elixirs. In addition to active ingredients, liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, oral compositions can include adjuvants such as wetting agents, emulsifying and suspending agents, sweetening, flavoring, and/or perfuming agents. In certain embodiments for parenteral administration, compositions are mixed with solubilizing agents such as Cremophor^{®}, alcohols, oils, modified oils, glycols, polysorbates, cyclodextrins, polymers, and/or combinations thereof.

Injectable preparations, for example, sterile injectable aqueous or oleaginous suspensions may be formulated according to the known art using suitable dispersing agents, wetting agents, and/or suspending agents. Sterile injectable preparations may be sterile injectable solutions, suspensions, and/or emulsions in nontoxic parenterally acceptable diluents and/or solvents, for example, as a solution in 1,3-butanediol. Among the acceptable vehicles and solvents that may be employed are water, Ringer's solution, U.S.P., and isotonic sodium chloride solution. Sterile, fixed oils are conventionally employed as a solvent or suspending medium. For this purpose any bland fixed oil can be employed including synthetic mono- or diglycerides. Fatty acids such as oleic acid can be used in the preparation of injectables.

Injectable formulations can be sterilized, for example, by filtration through a bacterial-retaining filter, and/or by incorporating sterilizing agents in the form of sterile solid compositions which can be dissolved or dispersed in sterile water or other sterile injectable medium prior to use.

In order to prolong the effect of an active ingredient, it is often desirable to slow the absorption of the active ingredient from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are prepared by entrapping the drug in liposomes or microemulsions which are compatible with body tissues.

Compositions for rectal or vaginal administration are typically suppositories which can be prepared by mixing compositions with suitable non-irritating excipients such as cocoa butter, polyethylene glycol or a suppository wax which are solid at ambient temperature but liquid at body temperature and therefore melt in the rectum or vaginal cavity and release the active ingredient.

Solid dosage forms for oral administration include capsules, tablets, pills, powders, and granules. In such solid dosage forms, an active ingredient is mixed with at least one inert, pharmaceutically acceptable excipient such as sodium citrate or dicalcium phosphate and/or fillers or extenders *(*e.g. starches, lactose, sucrose, glucose, mannitol, and silicic acid), binders (*e.g.* carboxymethylcellulose, alginates, gelatin, polyvinylpyrrolidinone, sucrose, and acacia), humectants (*e.g.* glycerol), disintegrating agents (*e.g.* agar, calcium carbonate, potato or tapioca starch, alginic acid, certain silicates, and sodium carbonate), solution retarding agents *(e.g.* paraffin), absorption accelerators *(e.g.* quaternary ammonium compounds), wetting agents *(e.g.* cetyl alcohol and glycerol monostearate), absorbents *(e.g.* kaolin and bentonite clay), and lubricants (*e.g.* talc, calcium stearate, magnesium stearate, solid polyethylene glycols, sodium lauryl sulfate), and mixtures thereof. In the case of capsules, tablets and pills, the dosage form may comprise buffering agents.

Solid compositions of a similar type may be employed as fillers in soft and hard-filled gelatin capsules using such excipients as lactose or milk sugar as well as high molecular weight polyethylene glycols and the like. Solid dosage forms of tablets, dragees, capsules, pills, and granules can be prepared with coatings and shells such as enteric coatings and other coatings well known in the pharmaceutical formulating art. They may optionally comprise opacifying agents and can be of a composition that they release the active ingredient(s) only, or preferentially, in a certain part of the intestinal tract, optionally, in a delayed manner. Examples of embedding compositions which can be used include polymeric substances and waxes. Solid compositions of a similar type may be employed as fillers in soft and hard-filled gelatin capsules using such excipients as lactose or milk sugar as well as high molecular weight polyethylene glycols and the like.

Dosage forms for topical and/or transdermal administration of a composition may include ointments, pastes, creams, lotions, gels, powders, solutions, sprays, inhalants and/or patches. Generally, an active ingredient is admixed under sterile conditions with a pharmaceutically acceptable excipient and/or any needed preservatives and/or buffers as may be required. Additionally, the present invention contemplates the use of transdermal patches, which often have the added advantage of providing controlled delivery of a compound to the body. Such dosage forms may be prepared, for example, by dissolving and/or dispensing the compound in the proper medium. Alternatively or additionally, rate may be controlled by either providing a rate controlling membrane and/or by dispersing the compound in a polymer matrix and/or gel.

Suitable devices for use in delivering intradermal pharmaceutical compositions described herein include short needle devices. Intradermal compositions may be administered by devices which limit the effective penetration length of a needle into the skin and functional equivalents thereof. Jet injection devices which deliver liquid compositions to the dermis via a liquid jet injector and/or via a needle which pierces the stratum corneum and produces a jet which reaches the dermis are suitable. Ballistic powder/particle delivery devices which use compressed gas to accelerate vaccine in powder form through the outer layers of the skin to the dermis are suitable. Alternatively or additionally, conventional syringes may be used in the classical mantoux method of intradermal administration.

Formulations suitable for topical administration include, but are not limited to, liquid and/or semi liquid preparations such as liniments, lotions, oil in water and/or water in oil emulsions such as creams, ointments and/or pastes, and/or solutions and/or suspensions. Topically-administrable formulations may, for example, comprise from about 1% to about 10% (w/w) active ingredient, although the concentration of active ingredient may be as high as the solubility limit of the active ingredient in the solvent. Formulations for topical administration may further comprise one or more of the additional ingredients described herein.

A pharmaceutical composition may be prepared, packaged, and/or sold in a formulation suitable for pulmonary administration via the buccal cavity. Such a formulation may comprise dry particles which comprise the active ingredient and which have a diameter in the range from about 0.5 nm to about 7 nm or from about 1 nm to about 6 nm. Such compositions are conveniently in the form of dry powders for administration using a device comprising a dry powder reservoir to which a stream of propellant may be directed to disperse the powder and/or using a self-propelling solvent/powder dispensing container such as a device comprising the active ingredient dissolved and/or suspended in a low-boiling propellant in a sealed container. Such powders comprise particles wherein at least 98% of the particles by weight have a diameter greater than 0.5 nm and at least 95% of the particles by number have a diameter less than 7 nm. Alternatively, at least 95% of the particles by weight have a diameter greater than 1 nm and at least 90% of the particles by number have a diameter less than 6 nm. Dry powder compositions may include a solid fine powder diluent such as sugar and are conveniently provided in a unit dose form.

Low boiling propellants generally include liquid propellants having a boiling point of below 65 °F at atmospheric pressure. Generally the propellant may constitute 50% to 99.9% (w/w) of the composition, and active ingredient may constitute 0.1% to 20% (w/w) of the composition. A propellant may further comprise additional ingredients such as a liquid nonionic and/or solid anionic surfactant and/or a solid diluent (which may have a particle size of the same order as particles comprising the active ingredient).

Pharmaceutical compositions formulated for pulmonary delivery may provide an active ingredient in the form of droplets of a solution and/or suspension. Such formulations may be prepared, packaged, and/or sold as aqueous and/or dilute alcoholic solutions and/or suspensions, optionally sterile, comprising active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate. Droplets provided by this route of administration may have an average diameter in the range from about 0.1 nm to about 200 nm.

Formulations described herein as being useful for pulmonary delivery are useful for intranasal delivery of a pharmaceutical composition. Another formulation suitable for intranasal administration is a coarse powder comprising the active ingredient and having an average particle from about 0.2 µm to 500 µm. Such a formulation is administered in the manner in which snuff is taken, *i.e.* by rapid inhalation through the nasal passage from a container of the powder held close to the nose.

Formulations suitable for nasal administration may, for example, comprise from about as little as 0.1% (w/w) and as much as 100% (w/w) of active ingredient, and may comprise one or more of the additional ingredients described herein. A pharmaceutical composition may be prepared, packaged, and/or sold in a formulation suitable for buccal administration. Such formulations may, for example, be in the form of tablets and/or lozenges made using conventional methods, and may, for example, 0.1% to 20% (w/w) active ingredient, the balance comprising an orally dissolvable and/or degradable composition and, optionally, one or more of the additional ingredients described herein. Alternately, formulations suitable for buccal administration may comprise a powder and/or an aerosolized and/or atomized solution and/or suspension comprising active ingredient. Such powdered, aerosolized, and/or aerosolized formulations, when dispersed, may have an average particle and/or droplet size in the range from about 0.1 nm to about 200 nm, and may further comprise one or more of any additional ingredients described herein.

A pharmaceutical composition may be prepared, packaged, and/or sold in a formulation suitable for ophthalmic administration. Such formulations may, for example, be in the form of eye drops including, for example, a 0.1/1.0% (w/w) solution and/or suspension of the active ingredient in an aqueous or oily liquid excipient. Such drops may further comprise buffering agents, salts, and/or one or more other of any additional ingredients described herein. Other opthalmically-administrable formulations which are useful include those which comprise the active ingredient in microcrystalline form and/or in a liposomal preparation. Ear drops and/or eye drops are contemplated as being within the scope of this invention.

General considerations in the formulation and/or manufacture of pharmaceutical agents may be found, for example, in Remington: The Science and Practice of Pharmacy 21st ed., Lippincott Williams & Wilkins, 2005 (incorporated herein by reference).

### Administration

The present invention provides methods comprising administering compositions of supercharged proteins associated with functional effector proteins to a subject in need thereof. In some embodiments, methods of administering compositions comprising other functional effector proteins *(e.g.,* a Cas9 protein) and cationic lipid and/or cationic polymers are provided. Such compositions may be administered to a subject using any amount and any route of administration effective for preventing, treating, diagnosing, or imaging a disease, disorder, and/or condition. The exact amount required will vary from subject to subject, depending on the species, age, and general condition of the subject, the severity of the disease, the particular composition, its mode of administration, its mode of activity, and the like. Compositions in accordance with the invention are typically formulated in dosage unit form for ease of administration and uniformity of dosage. It will be understood, however, that the total daily usage of the compositions of the present invention will be decided by the attending physician within the scope of sound medical judgment. The specific therapeutically effective, prophylactially effective, or appropriate imaging dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

Compositions of supercharged proteins associated with functional effector proteins to be delivered as well as compositions comprising *e.g.,* a Cas9 protein and cationic lipid may be administered by any route. In some embodiments, such compositions are administered by one or more of a variety of routes, including oral, intravenous, intramuscular, intra-arterial, intramedullary, intrathecal, subcutaneous, intraventricular, transdermal, interdermal, rectal, intravaginal, intraperitoneal, topical *(e.g.,* by powders, ointments, creams, gels, lotions, and/or drops), mucosal, nasal, buccal, enteral, vitreal, intratumoral, sublingual; by intratracheal instillation, bronchial instillation, and/or inhalation; as an oral spray, nasal spray, and/or aerosol, and/or through a portal vein catheter. In some embodiments, supercharged proteins or complexes, and/or pharmaceutical, prophylactic, diagnostic, or imaging compositions thereof, are administered by systemic intravenous injection. In specific embodiments, supercharged proteins or complexes and/or pharmaceutical, prophylactic, diagnostic, or imaging compositions thereof may be administered intravenously and/or orally. In specific embodiments, such compositions may be administered in a way which allows the functional effector protein to cross the blood-brain barrier, vascular barrier, or other epithelial barrier.

In certain embodiments, compositions in accordance with the invention may be administered at dosage levels sufficient to deliver an amount of functional effector protein of from about 0.0001 mg/kg to about 100 mg/kg, from about 0.01 mg/kg to about 50 mg/kg, from about 0.1 mg/kg to about 40 mg/kg, from about 0.5 mg/kg to about 30 mg/kg, from about 0.01 mg/kg to about 10 mg/kg, from about 0.1 mg/kg to about 10 mg/kg, or from about 1 mg/kg to about 25 mg/kg, of subject body weight per day, one or more times a day, to obtain the desired therapeutic, diagnostic, prophylactic, or imaging effect. The desired dosage may be delivered three times a day, two times a day, once a day, every other day, every third day, every week, every two weeks, every three weeks, or every four weeks. In certain embodiments, the desired dosage may be delivered using multiple administrations (*e.g.,* two, three, four, five, six, seven, eight, nine, ten, eleven, twelve, thirteen, fourteen, or more administrations).

Compositions comprising supercharged proteins associated with functional effector proteins may be administered in combination with one or more other therapeutic, prophylactic, diagnostic, or imaging agents. By "in combination with," it is not intended to imply that the agents must be administered at the same time and/or formulated for delivery together, although these methods of delivery are within the scope of the invention. Compositions can be administered concurrently with, prior to, or subsequent to, one or more other desired therapeutics or medical procedures. In general, each agent will be administered at a dose and/or on a time schedule determined for that agent. In some embodiments, the invention encompasses the delivery of pharmaceutical, prophylactic, diagnostic, or imaging compositions in combination with agents that may improve their bioavailability, reduce and/or modify their metabolism, inhibit their excretion, and/or modify their distribution within the body.

### Kits

The invention provides a variety of kits for conveniently and/or effectively carrying out methods of the present invention. Typically kits will comprise sufficient amounts and/or numbers of components to allow a user to perform multiple treatments of a subject(s) and/or to perform multiple experiments. In some embodiments, kits comprise one or more of (i) a supercharged protein, as described herein; (ii) a functional effector protein to be delivered; (ii) a cationic lipid and/or cationic polymer; and (iv) instructions for formulating a composition comprising the functional protein associated to the supercharged protein. In some embodiments, the kits comprise a Cas9 protein and a cationic lipid. In some embodiments, kits comprise a nucleic acid encoding for the supercharged protein and/or the functional protein to be delivered. In some embodiments, the kit comprises a cloning vector encoding a supercharged protein and a cloning site allowing the in-frame cloning of a functional effector protein to generate a fusion protein. In some embodiments, kits comprise a pharmaceutical composition provided herein comprising a supercharged protein associated with a functional effector protein; a syringe, needle, or applicator for administration of the pharmaceutical composition to a subject; and instructions for administration of the pharmaceutical composition to the subject.

These and other aspects of the present invention will be further appreciated upon consideration of the following Examples, which are intended to illustrate certain particular embodiments of the invention but are not intended to limit its scope, as defined by the claims.

### EXAMPLES

### Example 1: Delivery of TALE Activators fused to supercharged GFP

A major target for reprogramming fibroblast cell fate towards brown or white adipocyte cell fate lies in the switch from White Adipose Tissue (WAT) to Brown Adipose Tissue (BAT), which is governed by expression of PRDM16 and PPARγ. Robust TALE transcriptional activators fused to a +36 GFP were engineered that target PPARγ and PRDM16 genomic sequences in fibroblasts. Fusion proteins were purified using a heparin column and/or an SEC and gels show a single band at 130 kD The modulation of expression and effect on cellular phenotype after delivery of the TALE activators was compared to the modulation after viral delivery of a PPARγ cDNA followed by 7-day treatment with adipogenesis cocktail. It was observed that adipocytes formed upon treatment with +36 GFP TALEPRDM16 fusion. Expression of white adipose tissue marker genes was detected after delivery of supercharged PRDM16 TALE activators.

A one-time supercharged protein-mediated delivery of a TALE activator for PPARγ was found to induce expression of white-fat genes and to differentiate fibroblasts into white-fat cells. Supercharged protein-mediated delivery of both a PPARγ and PRDM16 TALE activator induced the differentiation of fat cells with increased expression of brown-fat markers such as PRDM16, cox8b, elovl3, and cidea as well as a small increase in thermogenic gene expression markers PGC1a and UCP1.

An Aurein peptide was fused to the N-terminus of the +36GFP-TALE-activator fusion protein. Delivery of Aurein +36 GFP TALE purified by heparin column was observed by detecting fluorescence in nucleus of the treated cells.

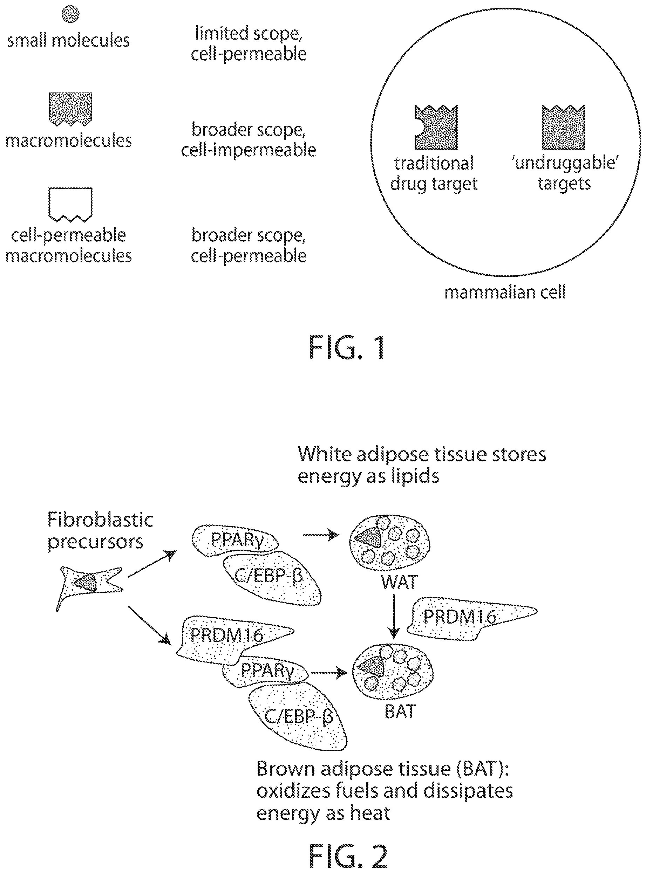

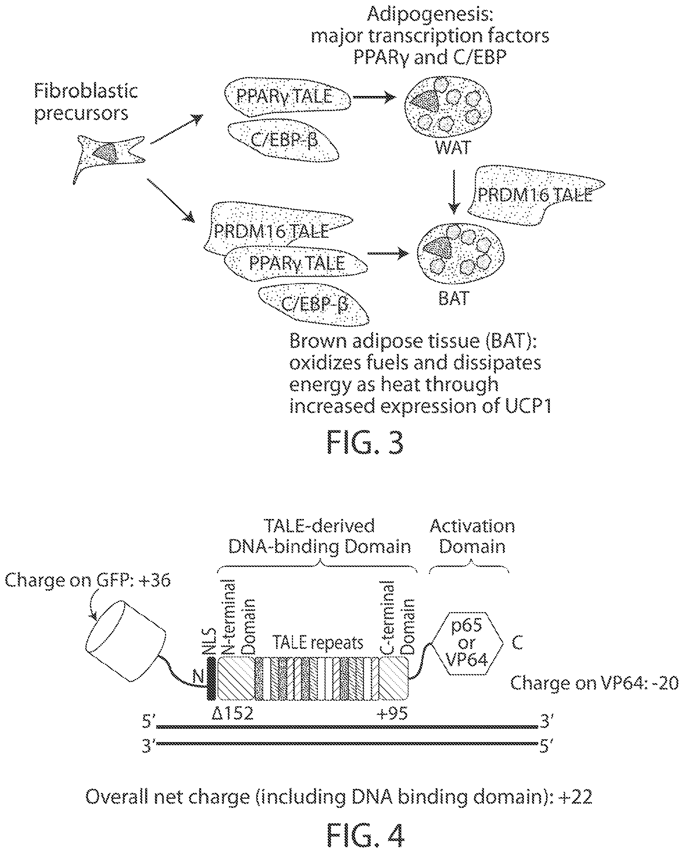

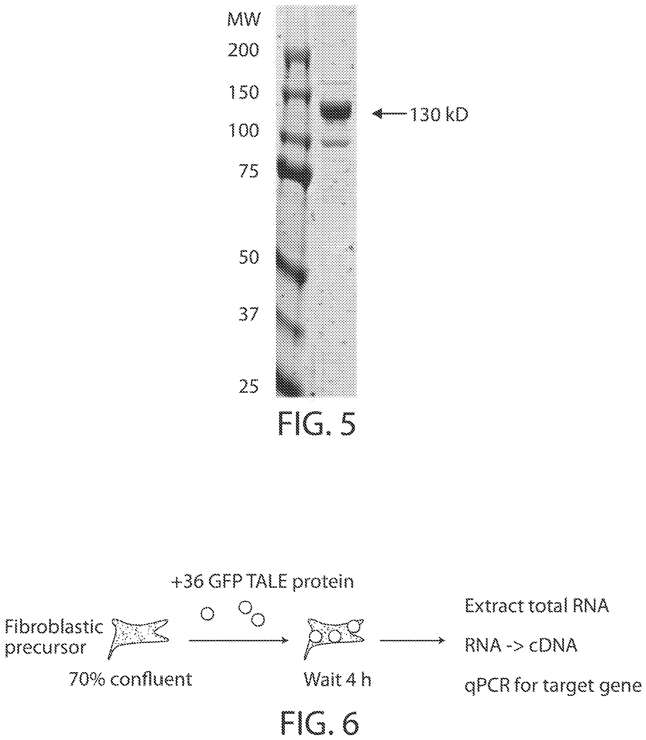

Figure 1 shows a schematic of macromolecular delivery into mammalian cells. Figure 2 shows an overview of the switch from White Adipose Tissue (WAT) to Brown Adipose Tissue (BAT). Figure 3 shows a schematic of supercharged delivery platforms to deliver TALE activators programmed to target PPARγ or PRDM16. Figure 4 shows a schematic of a fusion protein comprising a +36 GFP fusion, an 18.5 mer TALE domain, and a VP64 activation domain. Figure 5 shows expression and purification of the +36 GFP-TALE activator-fusion protein. Figure 6 shows testing assays for activation of fat cell regulator genes upon delivery of +36 GFP PPARγ and PRDM16 TALE activator fusion proteins.

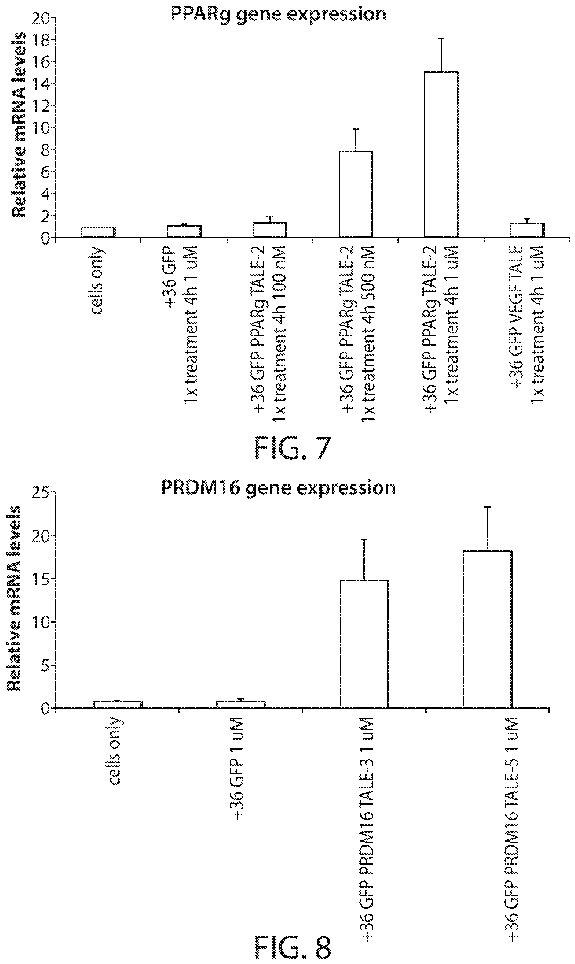

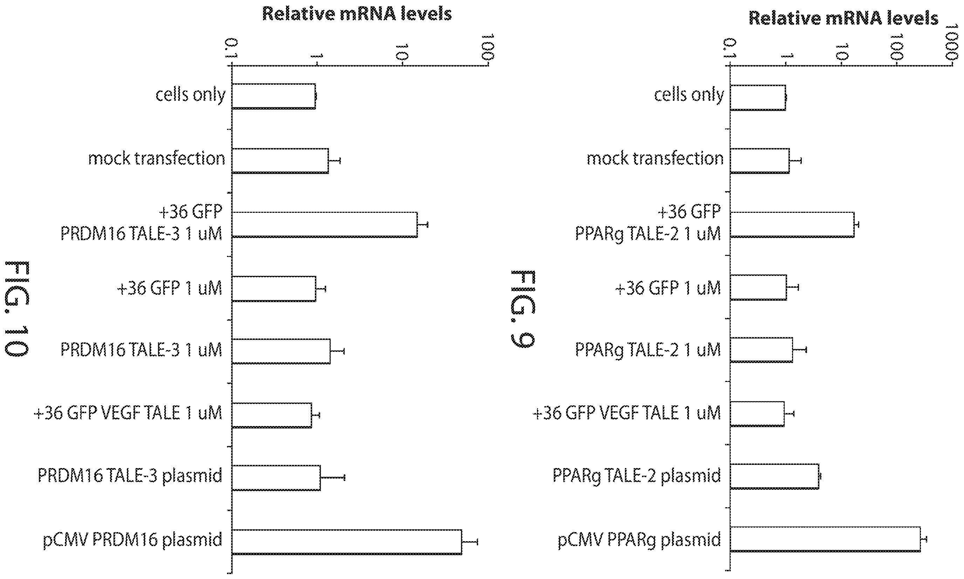

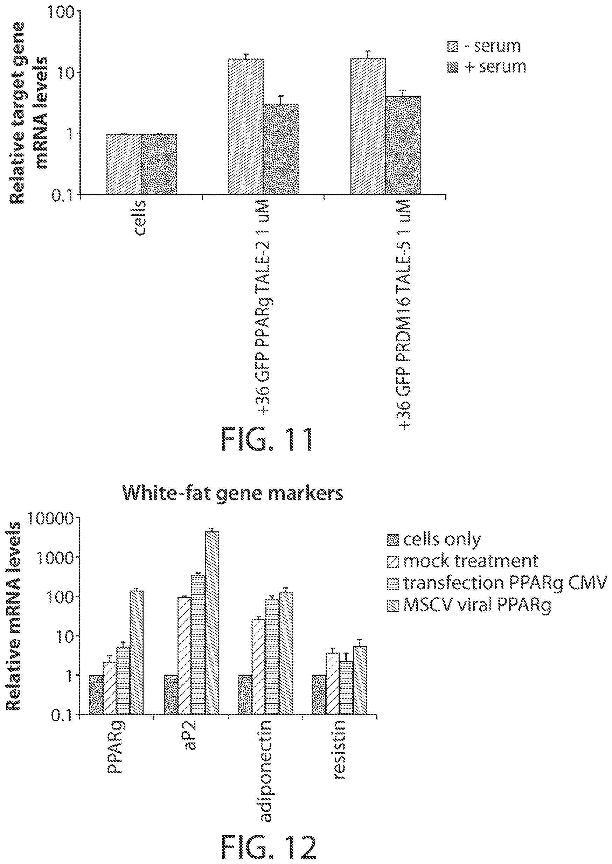

Figure 7 shows delivery efficacy of +36 GFP TALE activator fusion proteins at different concentrations. Figure 8 shows a comparison of delivery efficacy of two different +36 GFP-PRDM16 TALE fusion proteins in NIH 3T3 cells. Figure 9 shows PPARγ gene expression after delivery of PPARγ-TALE activator fusion and comparison to various controls. Figure 10 shows PRDM16 gene expression after delivery of RDM16-TALE activator fusion and comparison to various controls. Figure 11 shows moderate TALE activity observed in the presence of serum.

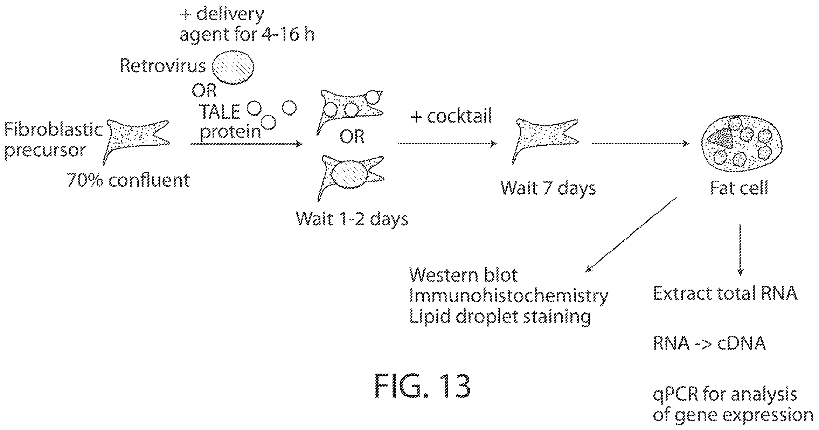

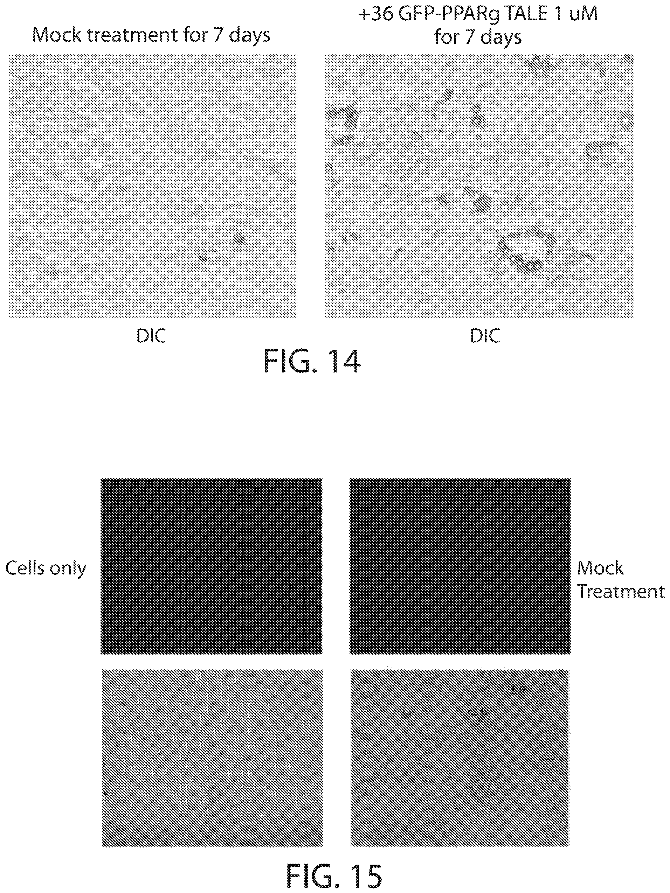

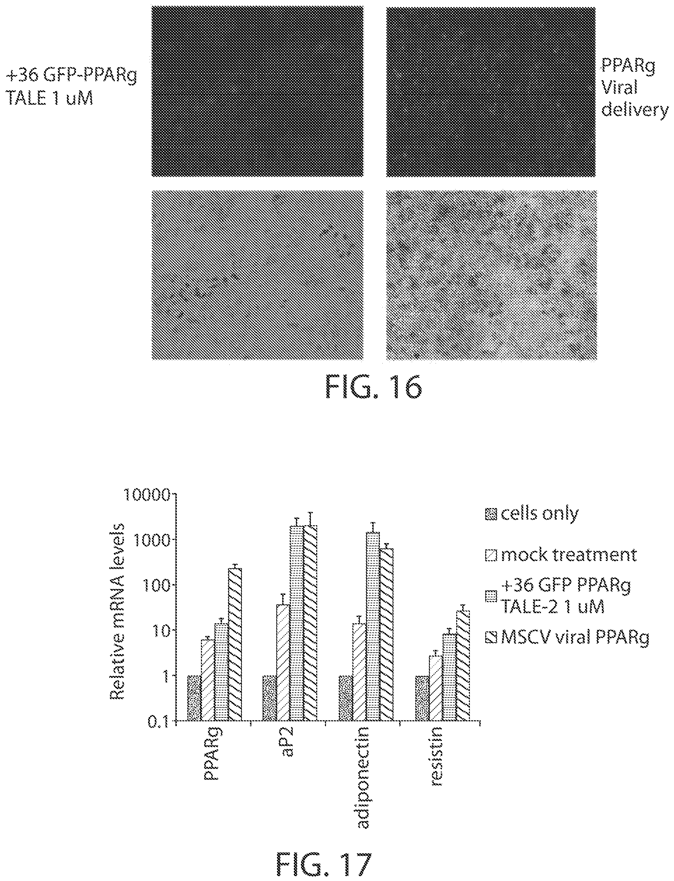

Figure 12 shows a validation of viral delivery of PPARγ followed by 7-day treatment with adipogenesis cocktail. Figure 13 shows a schematic of an assay for programming fibroblasts into WAT and BAT. Figure 14 shows adipocyte formation observed upon treatment with +36 GFP TALE activator fusion protein. Figure 15 shows staining of various treatments after 7 days with LipidTOX red, demonstrating formation of adipocytes after viral delivery as well as after delivery of supercharged PPARγ TALE activator fusion protein. Figure 16 shows staining of cells after various treatments after 7 days with LipidTOX red, demonstrating formation of adipocytes after viral delivery as well as after delivery of supercharged PPARγ TALE activator fusion protein. Figure 17 shows expression of WAT biomarker genes after viral delivery as well as after delivery of supercharged PPARγ TALE activator fusion protein.

### Example 2: In vivo delivery of TALE Activators fused to supercharged GFP

NIH 3T3 cells were grown to 70-90% confluence and treated with 1 µM or between 0.5-5 µM of +36 GFP PPARγ TALE and/or +36 GFP PRDM16 TALE fusion protein in DMEM without serum. A serum-free medium was chosen, because serum can decrease the effectiveness of protein-based delivery. Cells were incubated with the respective fusion protein solution for 4 hours before the media was removed and full DMEM containing serum was added back to cells. Control cells were infected with a viral construct encoding PPARγ or PRDM16 in order to serve as a positive control for expression of WAT and BAT genes according to known protocols (see, *e.g.,* Seale et al. 2008 Nature 454, 961-967, the entire contents of which are incorporated herein by reference). Once all cells reached 100% confluence an adipogenesis cocktail containing isobutylmethylxanthine, insulin, rosiglitazone, dexamethosone, T3, and indomethacin was added to the cells and replaced 48 hours later with a form of the cocktail containing only insulin, T3, and rosiglitazone. At 48 hours after this second replacement of cocktail another dosage of T3, insulin, and rosiglitazone was added to the cells. The next day, which is now one week from the start of the experiment, cells were harvested with TRIzol, total RNA was extracted, and qRT-PCR was performed to measure gene expression levels of PPARγ, PRDM16, and other brown fat marker genes such as UCP1, PGC1a, Elovl3, and Cidea.

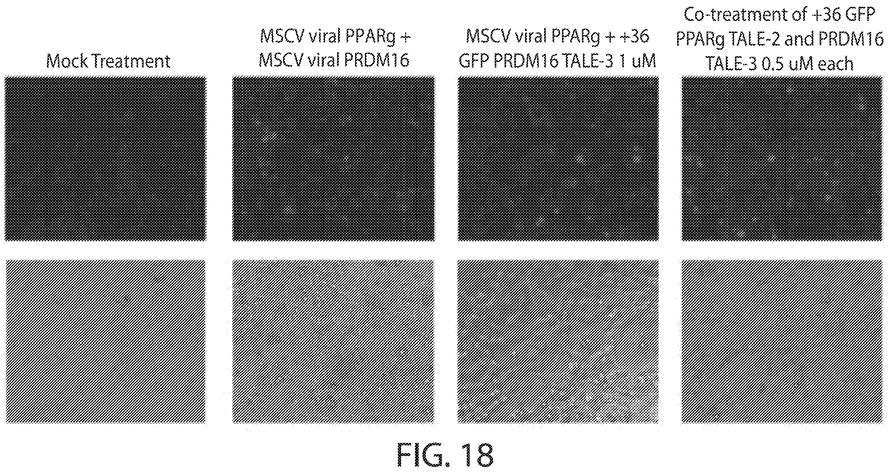

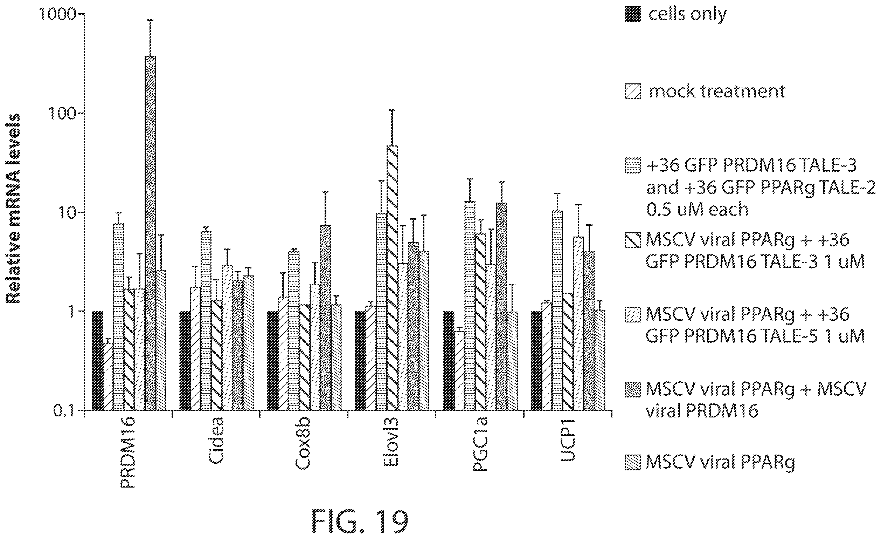

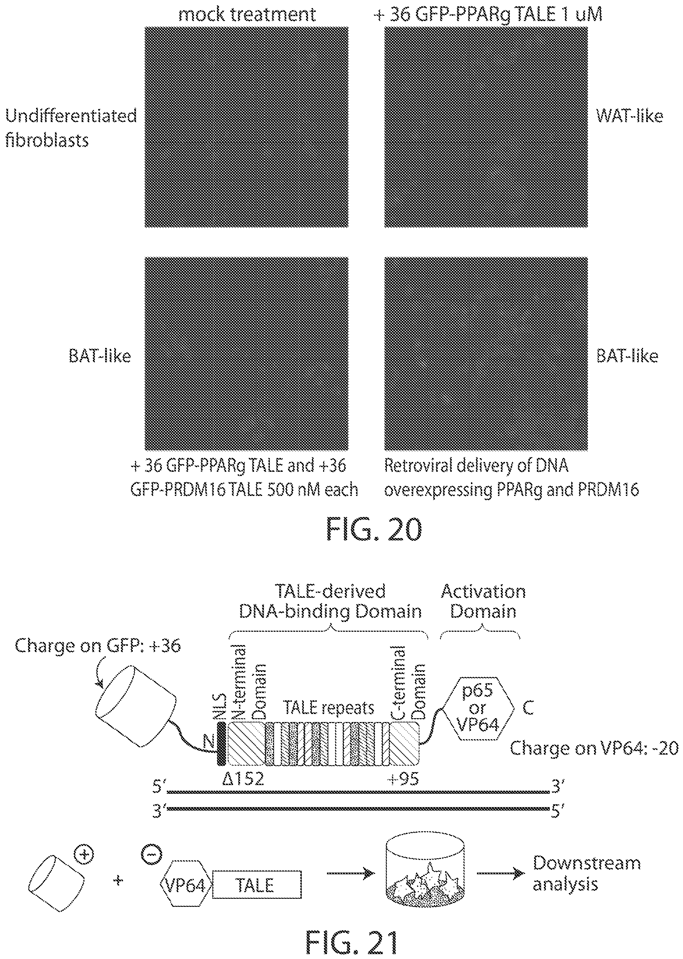

Figure 18 shows delivery of supercharged PRDM16 TALE activator fusion proteins to induce brown-fat adipocytes *in vivo.* Robust adipocyte formation was observed after viral delivery of PPARγ and PRDM16 and also after delivery of supercharged TALE activator protein fusions. Figure 19 shows a comparison of supercharged (TALE) and viral delivery of PPARγ and PRDM16 to cells. The figure shows TALE/TALE, viral/TALE, and viral/viral-induced expression of brown fat markers by expression of PPARγ and PRDM16. Figure 20 shows RT-qPCR assessments consistent with fat cell differentiation, which were also observed by LipidTOX staining.

### Example 3: Delivery of TALE Activators complexed with supercharged GFP

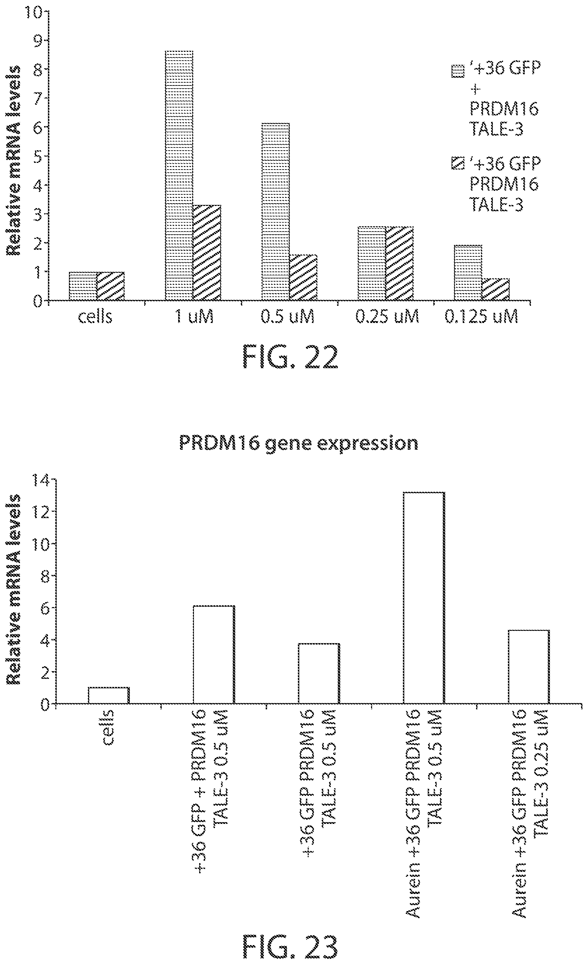

In order to improve delivery efficacy, protein complexes in which the functional protein was non-covalently associated with the supercharged protein were generated and administered to cells. Figure 21. shows that delivery of functional TALE activator fusion proteins as complexes with +36 GFP improves TALE activator activity after delivery. Figure 22 shows PRDM16 gene expression after TALE activator fusion delivery either as a fusion (+36GFP PRDM16 TALE-3) or a complex (+36GFP + PRDM16 TALE-3) with +36GFP. It was observed that delivery of complexes tended to increase TALE activator activity.

### Example 4: Effect of Aurein fusions on delivery efficacy

Figure 23 shows the effect of an N-terminal Aurein peptide fusion to +36GFP on PRDM16 gene expression after TALE activator fusion delivery (either as a fusion or a complex with +36GFP). The Aurein peptide was fused to the N-terminus of the GFP-TALE construct via a GGS(9) (SEQ ID NO: 252) linker, resulting in an Aurein peptide-GGS(9) linker-(+)36 GFP protein-GGS(9) linker -PRDM16 TALE-3 fusion protein. The protein was purified using size exclusion chromatography.

### Example 5: Delivery of TALE Activators complexed with supercharged GFP or cationic lipids

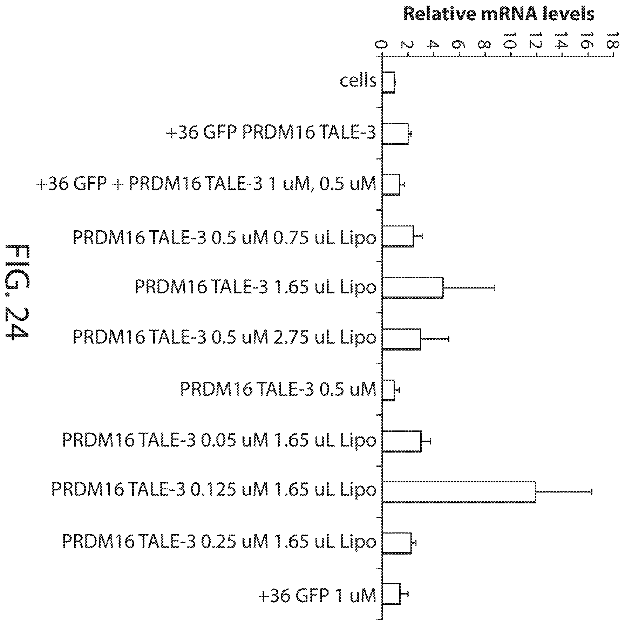

Figure 24 shows PRDM16 gene expression after TALE PRDM16 activator protein delivery either as a fusion with +36 GFP (+36GFP PRDM16 TALE-3), a complex with +36 GFP (+36GFP + PRDM16 TALE-3), or a complex with Lipofectamine LTX, for which an increase in gene expression was observed.

### Example 6: Delivery of Cas9 fused to supercharged GFP

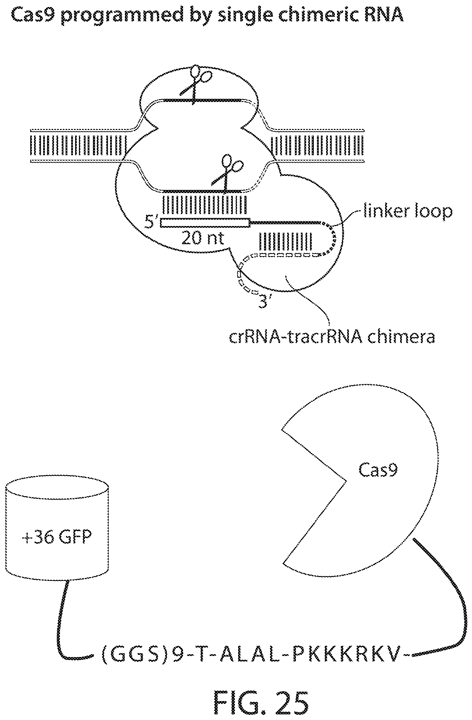

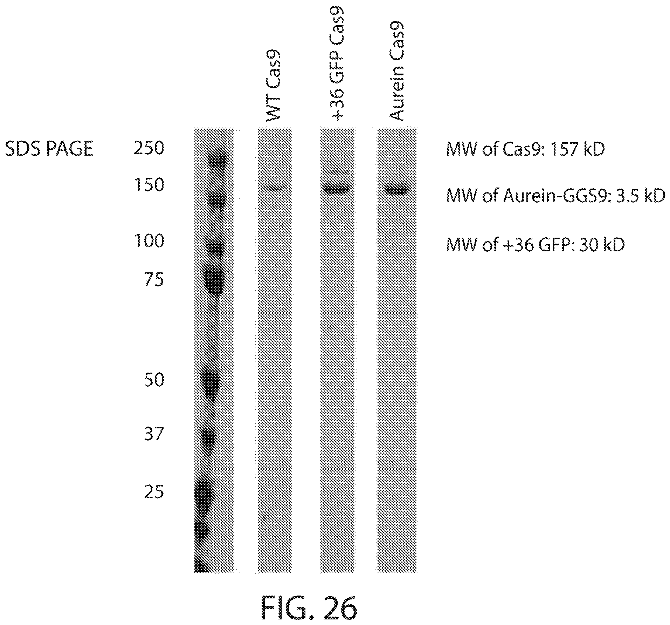

Supercharged delivery of Cas9 into mammalian cells would allow the application of powerful RNA-programmable nuclease technology in cells without the drawbacks of prior delivery methods. To this end, a Cas9 fusion with +36GFP was generated, using an ALAL linker. Figure 25 shows a schematic of the supercharged fusion protein with Cas9. Figure 26 shows the purification of wild-type Cas9 protein and Cas9 fusion proteins with +36GFP and Aurein-GGS9. The fusion protein is administered to cells in the same manner as the TALE activator fusion proteins above. The Cas9, once delivered to the cells, binds and cleaves its target site in the cellular genome. Nuclease activity in the target cells is detected via a suitable assay, e.g., via southern blot or sequencing assay.

### Example 7: Efficient Delivery of Genome Editing Proteins In Vitro and In Vivo

Efficient intracellular delivery of proteins to the nucleus or cytoplasm is needed to fully realize the potential of protein therapeutics including genome-editing agents. Current methods of protein delivery often suffer from low tolerance for serum proteins, poor endosomal escape, and limited *in vivo* efficacy. As demonstrated in this Example, common cationic lipid reagents originally developed for nucleic acid transfection can potently deliver proteins that are fused to negatively supercharged proteins, that contain natural anionic domains, or that natively bind to anionic nucleic acids. This approach mediates the functional delivery of Cre recombinase, TALE- and Cas9-based transcriptional activators, and Cas9:sgRNA nuclease complexes into cultured human cells at low nanomolar concentrations in media containing 10% serum. Lipid-based delivery can be > 1,000-fold more potent than cationic protein delivery strategies. Delivery of Cas9: sgRNA complexes resulted in genome modification efficiencies as high as 80% with substantially higher specificity compared to standard DNA transfection, likely due to the transient nature of delivered Cas9:sgRNA complexes. This approach also mediated efficient delivery of Cre recombinase and Cas9:sgRNA complexes into the mouse inner ear *in vivo,* achieving up to 90% Cre-mediated recombination and 20% Cas9-mediated genome modification in the targeted hair-cell population.

### Example 8: Delivery of biotinylated proteins to cells.

Efficient intracellular delivery of proteins to the nucleus or cytoplasm is effected by providing a protein to be delivered in a biotinylated form, complexing it with a negatively supercharged streptavidin (-40SAV), contacting the resulting negatively charged protein complex with a cationic lipid, and contacting cells with the resulting composition. Methods for generating complexes of biotinylated proteins to be delivered and anionic carriers, e.g., a negatively supercharged streptavidin or a negatively charged protein conjugated to streptavidin are illustrated in Figure 49. A protein to be delivered, for example, a genome-editing protein, is associated to biotin via a linker, for example using click chemistry, NHS ester chemistry, or maleimide chemistry. The linker comprises a cleavage site that is cleaved by an endosomal protease present in endosomes of the cell into which the protein is to be delivered. The biotinylated form of the protein to be delivered is conjugated to a supernegatively charged streptavidin variant, for example, to a supernegatively charged streptavidin variant disclosed in International Application international PCT application PCT/US09/041984, filed on April 28, 2009, published as WO 2009/134808 on November 5, 2009, the entire contents of which are incorporated herein by reference. The biotinylated protein associated with the supernegatively charged streptavidin is contacted with a cationic lipid, *e.g.,* with Lipofectamine^{®}, and the resulting composition is contacted with the cells into which the protein is to be delivered. The composition is used at a concentration at which the cells exhibit less than 5% mortality 24 hours after being contacted with the composition. The protein to be delivered is subsequently observed in at least 10% of the target cells..

### Materials and Methods

### Construction of Cas9, Cre, and TALE fusion and sgRNA expression plasmids.

Sequences of all constructs used in this paper are listed below or provided elsewhere in the specification. All protein constructs were generated from previously reported plasmids for protein of interest cloned into a pET29a expression plasmid.

### Expression and purification of S. pyogenes Cas9 and other proteins.

*E. coli* BL21 STAR (DE3) competent cells (Life Technologies) were transformed with pMJ806⁴⁷ encoding the *S. pyogenes* Cas9 fused to an N-terminal 10xHis-tag/maltose binding protein. The resulting expression strain was inoculated in Luria-Bertani (LB) broth containing 100 µg/mL of ampicillin at 37 °C overnight. The cells were diluted 1:100 into the same growth medium and grown at 37 °C to OD₆₀₀ = ~0.6. The culture was incubated at 20 °C for 30 min, and isopropyl β-D-1- thiogalactopyranoside (IPTG) was added at 0.5 mM to induce Cas9 expression. After approximately 16 hours, the cells were collected by centrifugation at 8,000 g and resuspended in lysis buffer (50 mM tris(hydroxymethyl)-aminomethane (Tris)-HCl, pH 8.0, 1 M NaCl, 20 % glycerol, 10 mM tris(2-carboxyethyl)phosphine (TCEP)). The cells were lysed by sonication (1 sec pulse-on, 1 sec pulse-off for 15 minutes total at 6 W output) and the soluble lysate was obtained by centrifugation at 20,000 g for 30 minutes.

The cell lysate was incubated with His-Pur nickel-nitriloacetic acid (nickel-NTA) resin (Thermo Scientific) at 4 °C for 30 minutes to capture His-tagged Cas9. The resin was transferred to a 20-mL column and washed with 20 column volumes of lysis buffer. Cas9 was eluted in 50 mM Tns-HCl (pH 8), 0.1 M NaCl, 20 % glycerol, 10 mM TCEP, and 300 mM imidazole, and concentrated by Amicon ultra centrifugal filter (Millipore, 100-kDa molecular weight cut-off) to -50 mg/mL. The 6xHis tag and maltose-binding protein were removed by TEV protease treatment at 4 °C for 20 hours and captured by a second Ni-affinity purification step. The eluent, containing Cas9, was injected into a HiTrap SP HP column (GE Healthcare) in purification buffer containing 50 mM Tris-HCl (pH 8), 0.1 M NaCl, 20 % glycerol, and 10 mM TCEP. Cas9 was eluted with purification buffer containing a linear NaCl gradient from 0.1 M to 1 M over five column volumes. The eluted fractions containing Cas9 were concentrated down to a concentration of 200 µM as quantified by Bicinchoninic acid assay (BCA) (Pierce Biotechnology), snap-frozen in liquid nitrogen, and stored in aliquots at -80 °C. All other proteins were purified by this method but without TEV cleavage step and proteins containing (-30) GFP were purified by anion exchange using a Hi-Trap Q HP anion exchange column (GE Healthcare) using the same purification protocol.

### In vitro transcription of sgRNAs.

Linear DNA fragments containing the T7 promoter binding site followed by the 20-bp sgRNA target sequence were transcribed *in vitro* using the T7 High Yield RNA Synthesis Kit (NEB) according to the manufacturer's instructions. *In vitro* transcribed RNA was precipitated with ethanol and purified by gel electrophoresis on a Criterion 10% polyacrylamide TBE-Urea gel (Bio-Rad). Excised gel fragments were extracted in 420 µL of 300 mM NaCl overnight on a rocking surface at 4 °C. Gel-purified sgRNA was precipitated with ethanol and redissolved in water and sgRNA concentration was finally quantified by UV absorbance and snap-frozen at -80 °C.

### Plasmid transfection.

Plasmid DNA was transfected using Lipofectamine 2000 (Life Technologies) according the manufacturer's protocol. For TALE activator plasmids, 300 ng of DNA was transfected, and for the activator synergy experiments 60 ng of each of five plasmids was pooled and transfected. For Cas9 nuclease delivery experiments, linear DNA PCR products expressing sgRNAs were used in transfection experiments targeting genomic sites in CLTA, EMX, VEGF, and GFP (sgRNA GFP g1, GFP g3, GFP g5, and GFP g7 for nickase studies). Linear DNA PCR products were generated using plasmid containing the U6 promoter as template and forward primers bearing the U6 promoter upstream sequence and reverse primers containing U6 downstream sequence followed by the sgRNA sequence (20-bp sequence unique to each target plus constant sgRNA backbone architecture sequence). sgRNAs expressed from linear DNA templates contained at least two 5' guanosines to match *in vitro* transcribed sgRNAs that required these bases for T7 transcription. Primer sequences and PCR conditions are listed below. For dCas9 activator experiments, 700 ng of Cas9 or dCas9-VP64 plasmid DNA was co-transfected with 250 ng of the appropriate sgRNA expression plasmid. For activator synergy experiments 50 ng of DNA from each of the six sgRNA was pooled and co-transfected with 700 ng of dCas9-VP64 plasmid.

### Delivery of transcription factor proteins complexed with cationic lipids in cell culture.

A more in-depth description of the delivery of genome-editing proteins both *in vitro* and *in vivo* can be found below. Briefly, cultured cells were plated in 48-well format (250 µL volume) in Dulbecco's Modified Eagle's Media plus GlutaMAX (Life Technologies, Carlsbad, CA) with 10% FBS ("full serum media") and antibiotics at a cell density necessary to reach -70% confluence the next day. Full serum media was replaced with the same media but containing no antibiotics one hour before delivery. Delivery of Cre and TALE proteins was performed by combining 1 nM to 1 µM protein (in 275 µL final volume) with 0.2-2.5 µL of commercially available cationic lipids in 25 µL OPTIMEM media (Life Technologies, Carlsbad, CA) according to the manufacturer's protocol for normal plasmid transfection, including incubation time. For Cas9 delivery *in vitro,* transcribed sgRNA was incubated with Cas9 protein for 5 min before complexing with the cationic lipid reagent. 25 µL lipid complexes in OPTIMEM media were added to cells and media was replaced 12-16 hours later fresh media unless otherwise noted. Cells were assayed for recombination 48 hours after delivery, for gene activation either 4 (TALE) or 12-16 hours (dCas9-VP64) after delivery, and for gene modification (Cas9) 48 and 72 hours after delivery.

### T7 endonuclease I assay to detect genomic modifications.

U2OS-EGFP cells or HEK293T cells were transfected with Cas9 expression and sgRNA expression plasmids or linear DNA PCR products as described above or treated with only Cas9 protein, only *in vitro* transcribed sgRNA, or only RNAiMAX. Genomic DNA was isolated from cells 2 days after transfection using the DNAdvance Kit (Agencourt) following the manufacturer's instructions. 200 ng of genomic DNA was used as template in PCR reactions to amplify the targeted genomic loci with flanking survey primer pairs specified below. PCR products were purified with a QIAquick PCR Purification Kit (Qiagen) and quantified with Quant-iT^{™} PicoGreen ^{®} dsDNA Kit (Life Technologies). 250ng of purified PCR DNA was combined with 2 µL of NEBuffer 2 (NEB) in a total volume of 19 µL and denatured then re-annealed with thermocycling at 95 °C for 5 minutes, 95 to 85 °C at 2 °C/s; 85 to 20 °C at 0.2 °C/s. The re-annealed DNA was incubated with 1 µl of T7 Endonuclease I (10 U/µl, NEB) at 37 °C for 15 minutes. 10 µL of 50 % glycerol was added to the T7 Endonuclease reaction and 12 µL was analyzed on a 5 % TBE 18-well Criterion PAGE gel (Bio-Rad) electrophoresed for 30 minutes at 200 V, then stained with 1× SYBR Gold (Life Technologies) for 30 min. Cas9-induced cleavage bands and the uncleaved band were visualized on an AlphaImager HP (Alpha Innotech) and quantified using ImageJ software⁵⁴. The peak intensities of the cleaved bands were divided by the total intensity of all bands (uncleaved + cleaved bands) to determine the fraction cleaved which was used to estimate gene modification levels as previously described.⁴⁶ For each sample, transfections and subsequent modification measurements were performed in triplicate on different days.

### Stem cell culture and delivery.

Mouse embryonic stem cell (ES) line Tau-GFP containing a permanent GFP gene insertion was cultured in DMEM with 15% FBS (Gibco), 100 mM MEM nonessential amino acids (Gibco), 0.55 mM 2-mercaptoethanol (Sigma), and leukemia inhibitory factor (1,000 units/ml; Chemicon). After 5 days floating spheres were formed that exhibited GFP fluorescence. Complexes of Cas9:sgRNA and RNAiMAX were added to the culture containing the floating spheres for 16 hours. After Cas9:sgRNA treatment, the cells were cultured in the above media for 3 days. The floating spheres were treated with trypsin for 5 minutes then passed through a 70 µm filter to collect single cells. The cells were cultured on laminin-coated slides in DMEM/F12 (1:1) supplemented with 1xN2, 1xB27, penicillin-streptomycin (100 µg/mL; Life Technologies) and 10% FBS for two days before labeling. Immunohistochemistry was performed using an anti-GFP antibody (#ab13970, Abcam) to assess GFP expression. To quantify the number of GFP-negative cells, we counted the total number of GFP-positive and GFP-negative cells from three representative visual fields at 20X magnification, and calculated the average efficiency. Three independent experiments were performed for each condition.

### Microinjection of proteins to mouse inner ear.

P0 floxP-tdTomato mice (The Jackson Laboratory) were used for (-30)GFP-Cre injection and P2 Atoh1-GFP mice (courtesy of Dr. J. Johnson, Southwestern Medical Center, University of Texas) were used for Cas9:sgRNA injection. Animals were used under protocols approved by the Massachusetts Eye & Ear Infirmary (IACUC) committee. Mice were anesthetized by lowering their temperature on ice. Cochleostomies were performed by making an incision behind the ear to expose the cochlea. Glass micropipettes held by a micromanipulator were used to deliver the complex into the scala media, which allows access to inner ear hair cells. For delivery of (-30)GFP-Cre, 3 µL of 45 µM protein was mixed with 3 µL of either RNAiMAX or Lipofectamine 2000 and incubated at room temperature for 30 minutes prior to injection. Four mice were injected per treatment group. For delivery of Cas9:sgRNA complexes, 1 µL of 200 µM Cas9 protein was mixed with 2 µL of 100 µM sgRNA and incubated for 5 minutes at room temperature before mixing with 3 µL of either RNAiMAX or Lipofectamine 2000 and incubating for an additional 30 minutes prior to injection. Three mice were injected per treatment group. The total delivery volume for every injection was 0.3 µL per cochlea and the release was controlled by a micromanipulator at the speed of 3 nL/sec.

### Immunohistochemistry and quantification.

5-10 days after injection, the mice were sacrificed and cochlea were harvested by standard protocols.⁵⁵ For immunohistochemistry, antibodies against hair-cell markers (Myo7a and Esp) and supporting cells (Sox2) were used following a previously described protocol.⁵⁵ To quantify the number of tdTomato positive cells after (-30)GFP-Cre or GFP negative cells after Cas9:sgRNA delivery, we counted the total number of outer hair cells in a region spanning 200 µm around the site of injection in the base turn of the cochlea. The efficiency of (-30)GFP-Cre-induced recombination or Cas9: sgRNA-induced genome modification was calculated as the percentage of outer hair cells that expressed tdTomato or that lost GFP expression.

### High-throughput DNA sequencing of genome modifications.

HEK293T cells were either transfected with Cas9 and sgRNA expression plasmids or linear DNA PCR products or treated with 50 nM Cas9 protein, 125 nM or 250 nM purified sgRNA, and cationic lipids as described earlier for Cas9 protein delivery to U2OS-EGFP reporter cells. For plasmid-based transfection experiments, 700 ng of Cas9 expression plasmid plus 250 ng of sgRNA plasmid or 50 ng of a linear DNA PCR product expressing sgRNA for targeting either the EMX1, CLTA2, or VEGF locus were transfected with Lipofectamine 2000 (Life Technologies) and cells were isolated 2 days later. For protein delivery experiments *in vivo,* ~30 mg of mouse tissue was isolated as previously described⁵⁵ from anesthetized mice and genomic DNA was extracted using the Agencourt DNAAdvance Genomic DNA Isolation Kit (Beckman Coulter). For cell culture experiments genomic DNA was isolated as described above. 150 ng of genomic DNA was used as template to amplify by PCR the on-target and off target genomic sites with flanking HTS primer pairs specified below. Relative amounts of crude PCR products were quantified by gel electrophoresis and samples treated with different sgRNA pairs or Cas9 nuclease types were separately pooled in equimolar concentrations before purification with the QIAquick PCR Purification Kit (Qiagen). Approximately150 ng of pooled DNA was electrophoresed using a 5% TBE 18-well Criterion PAGE gel (BioRad) for 30 min at 200 V and DNAs ~125 bp to -300 bp in length were isolated and purified by QIAquick PCR Purification Kit (Qiagen). Purified DNA was amplified by PCR with primers containing sequencing adapters, purified, and sequenced on a MiSeq high-throughput DNA sequencer (Illumina) as previously described.⁴⁷

### Quantification of Cas9 protein uptake.

We used Alexa Fluor 647 C2 Maleimide (Life Technologies) to fluorescently label Cas9 protein on surface cysteines. A 10 mM stock solution of Alexa 647 was prepared in anhydrous DMSO (Sigma). In a 0.4 mL reaction, 10 nmol of purified Cas9 protein and 200 nmol of Alexa 647 maleimide were combined in buffer conditions used for Cas9 protein storage. The labeling reaction was incubated at 4° C for 16 hours. At the end of the reaction, excess unconjugated Alexa 647 was removed by re-purifying the labeled Cas9 protein by cation exchange chromatography as described above. To measure the amount of protein delivered into treated cells, 20,000 cells were plated in the wells of a 48-well plate 1 day prior to treatment. On the day of treatment, 50 nM of Alexa 647-labeled Cas9 (Cas9-Alexa 647) and 50 nM of EGFP sgRNA were prepared for delivery using 0.8 µL of Lipofectamine 2000 as described above, and applied to the cells. After 4 hours, Cas9-Alexa 647:sgRNA Lipofectamine-containing media was removed, and cells were washed three times with 500 µL of PBS containing 20 U/mL heparin. The cells were trypsinized and prepared for counting and flow cytometry as described above. Cas9-Alexa 647 uptake was measured by flow cytometry, while 10,000 cells of the treated population were transferred to a black, flat-bottomed, opaque 96-well plate. Standard curves of Cas9-Alexa 647 were prepared by complexing 50 pmol of the Cas9-Alexa 647 protein with Lipofectamine 2000 exactly as described for Cas9-Alexa 647 delivery, followed by serial 2-fold dilutions in DMEM with 10% FBS containing 10,000 U2OS cells per well in the 96-well plate. The effect of U2OS cells or complexation with Lipofectamine 2000 on Alexa 647 fluorescence was determined by preparing three additional Cas9-Alexa 647 standard curves: (i) with Lipofectamine 2000 in media lacking U2OS cells, *(ii)* without Lipofectamine 2000 in media containing U2OS cells, and *(iii)* without Lipofectamine 2000 in media lacking U2OS cells.

### Data Analysis

Illumina sequencing reads were filtered and parsed with scripts written in Unix Bash as outlined below. Sample sizes for sequencing experiments were maximized (within practical experimental considerations) to ensure greatest power to detect effects. Statistical analyses for Cas9-modified genomic sites (Table 2) were performed as previously described⁵⁶ with multiple comparison correction using the Bonferroni method.

All scripts (DNA Sequence-Processing Algorithms) were written in bash and described in detail previously.¹⁶

The following is a list of upstream and downstream flanking sequences for each genomic target site.

| **Target Site** | **Downstream genomic sequence** | **Upstream genomic sequence** |
|---|---|---|
| EMX_On | GGCCTGCTTCGTGGCAATGC (SEQ ID NO:119) | ACCTGGGCCAGGGAGGGAGG (SEQ ID NO: 120) |
| EMX_Off1 | CTCACTTAGACTTTCTCTCC (SEQ ID NO:121) | CTCGGAGTCTAGCTCCTGCA (SEQ ID NO: 122) |
| EMX_Off2 | TGGCCCCAGTCTCTCTTCTA (SEQ ID NO:123) | CAGCCTCTGAACAGCTCCCG (SEQ ID NO:124) |
| EMX_Off3 | TGACTTGGCCTTTGTAGGAA (SEQ ID NO:125) | GAGGCTACTGAAACATAAGT (SEQ ID NO: 126) |
| EMX_Off4 | TGCTACCTGTACATCTGCAC (SEQ ID NO:127) | CATCAATGATTGGGCATTTC (SEQ ID NO: 128) |
| VEG_On | ACTCCAGTCCCAAATATGTA (SEQ ID NO:129) | ACTAGGGGGCGCTCGGCCAC (SEQ ID NO:130) |
| VEG_Off1 | CTGAGTCAACTGTAAGCATT (SEQ ID NO:131) | GGCCAGGTGCAGTGATTCAT (SEQ ID NO:132) |
| VEG_Off2 | TCGTGTCATCTTGTTTGTGC (SEQ ID NO:133) | GGCAGAGCCCAGCGGACACT (SEQ ID NO:134) |
| VEG_Off3 | CAAGGTGAGCCTGGGTCTGT (SEQ ID NO:135) | ATCACTGCCCAAGAAGTGCA (SEQ ID NO:136) |
| VEG_Off4 | TTGTAGGATGTTTAGCAGCA (SEQ ID NO:137) | ACTTGCTCTCTTTAGAGAAC (SEQ ID NO:138) |
| CLT2_On | CTCAAGCAGGCCCCGCTGGT (SEQ ID NO:139) | TTTTGGACCAAACCTTTTTG (SEQ ID NO:140) |
| CLT2_Off1 | TGAGGTTATTTGTCCATTGT (SEQ ID NO:141) | TAAGGGGAGTATTTACACCA (SEQ ID NO:142) |
| CLT2_Off2 | TCAAGAGCAGAAAATGTGAC (SEQ ID NO:143) | CTTGCAGGGACCTTCTGATT (SEQ ID NO:144) |
| CLT2_Off3 | TGTGTGTAGGACTAAACTCT (SEQ ID NO:145) | GATAGCAGTATGACCTTGGG (SEQ ID NO:146) |
| EGFP | AGCGTGTCCGGCGAGGGCGA (SEQ ID NO:147) | AGCGTGTCCGGCGAGGGCGA (SEQ ID NO:148) |
| MusEMX | CAGAATCGGAGGACAAAATA CAAAC (SEQ ID NO:149) | ACGAAGCAGGCCAACGGGGAG GACA (SEQ ID NO:150) |

### Primers used for generating PCR products to serve as substrates for T7 transcription of sgRNAs.

T7_gRNA-Rev was used in all cases. DNA template used was EGFP sgRNA plasmid as noted above. NTF3 and VEGF sgRNAs for dCas9-VP64 activator experiments were reported previously (Maeder et al., CRISPR RNA-guided activation of endogenous human genes. Nat. Methods. 2013; 10, 977-979). The T2 sgRNA target was previously reported¹¹. All oligonucleotides were purchased from Integrated DNA Technologies.
T7_EGFP1-Fwd TAA TAC GAC TCA CTA TA GGGCACGGGCAGCTTGCCGG (SEQ ID NO:151)
T7-GFP g1-Fwd TAA TAC GAC TCA CTA TA GGCCTCGAACTTCACCTCGGCG GAAAGGACGAAACACC (SEQ ID NO: 152)
T7-GFP g5-Fwd TAA TAC GAC TCA CTA TA GGCTGAAGGGCATCGACTTCA GAAAGGACGAAACACC (SEQ ID NO: 153)
T7-GFP g3-Fwd TAA TAC GAC TCA CTA TA GGCAGCTCGATGCGGTTCACCA GAAAGGACGAAACACC (SEQ ID NO: 154)
T7-GFP g7-Fwd TAA TAC GAC TCA CTA TA GGCAAGGAGGACGGCAACATCC GAAAGGACGAAACACC (SEQ ID NO: 155)
T7-EMX-Fwd TAA TAC GAC TCA CTA TA GGAGTCCGAGCAGAAGAAGAA GAAAGGACGAAACACC (SEQ ID NO: 156)
T7-VEG-Fwd TAA TAC GAC TCA CTA TA GGGGTGGGGGGAGTTTGCTCC GAAAGGACGAAACACC (SEQ ID NO: 157)
T7-CLT2-Fwd TAA TAC GAC TCA CTA TA GGCAGATGTAGTGTTTCCACA GAAAGGACGAAACACC (SEQ ID NO: 158)
T7-T2 HDR-Fwd TAA TAC GAC TCA CTA TA GGGGCCACTAGGGACAGGAT GAAAGGACGAAACACC (SEQ ID NO:273)
T7_gRNA-Rev AAAAAAAGCACCGACTCGGTG (SEQ ID NO: 159)

**Sequence of single-stranded oligonucleotide donor template (ssODN) used in HDR studies.**

CGACCACATGAAGCAGCACGACTTCTTCAAGTCCGCCATGCCCGAAGGCTACGTCC
AGGAGCGCACCATCTTCTTCAAGGACGACGGCAACTACAAGACCCGCGCCGAGGTG
AAGTTCGAGGGCGACACCCTGGTGAACCGCATCGAGCTGAAGGGCATCGACTTCAA
GGAGGACGGCAACATCCTGGGGCACAAGCTGG (SEQ ID NO:274)

### Primers for generating linear DNA PCR product for transfection.

PCR extension at (72 °C, 3 min) on plasmid containing U6 promoter as template with PCR_sgRNA-fwd1, PCR_sgRNA-rev2 and appropriate PCR_sgRNA primers listed below.
PCR_gRNA-fwd1 CTGTACAAAAAAGCAGGCTTTA (SEQ ID NO:160)
PCR_gRNA-rev2 AAAAAAAGCACCGACTCGGTGCCACTTTTTCAAGTTGATAACGG ACTAGCCTTATTTTAACTTGCTATTTCTAGCTCTAAAAC (SEQ ID NO:161)
   PCR-G-GFP1 GAAAGGACGAAACACC GGCCTCGAACTTCACCTCGGCGGTTTTAGAGCTAGAAATAGCAA (SEQ ID NO:162)
   PCR-G-GFP3 GAAAGGACGAAACACC GGCAGCTCGATGCGGTTCACCAGTTTTAGAGCTAGAAATAGCAA (SEQ ID NO:163)
   PCR-G-GFP5 GAAAGGACGAAACACC
   GGCTGAAGGGCATCGACTTCAGTTTTAGAGCTAGAAATAGCAA (SEQ ID NO:164)
   PCR-G-GFP7 GAAAGGACGAAACACC GGCAAGGAGGACGGCAACATCCGTTTTAGAGCTAGAAATAGCAA (SEQ ID NO: 165)
   PCR-G-CLT2 GAAAGGACGAAACACC GGCAGATGTAGTGTTTCCACAGTTTTAGAGCTAGAAATAGCAA (SEQ ID NO:166)
   PCR-G-EMX GAAAGGACGAAACACC GGAGTCCGAGCAGAAGAAGAAGTTTTAGAGCTAGAAATAGCAA (SEQ ID NO:167)
   PCR-G-VEG GAAAGGACGAAACACC GGGGTGGGGGGAGTTTGCTCCGTTTTAGAGCTAGAAATAGCAA (SEQ ID NO:168)

### Primers for performing T7 endonuclease I DNA cleavage assay.

| | |
|---|---|
| Survey_GFP-fwd | TACGGCAAGCTGACCCTGAA (SEQ ID NO:169) |
| Survey_GFP-rev | GTCCATGCCGAGAGTGATCC (SEQ ID NO:170) |
| Survey_CLTA-fwd | GCCAGGGGCTGTTATCTTGG (SEQ ID NO:171) |
| Survey_CLTA-rev | ATGCACAGAAGCACAGGTTGA (SEQ ID NO:172) |
| Survey_EMX-fwd | CTGTGTCCTCTTCCTGCCCT (SEQ ID NO:173) |
| Survey_EMX-rev | CTCTCCGAGGAGAAGGCCAA (SEQ ID NO:174) |
| Survey_VEGF-fwd | CCACACAGCTTCCCGTTCTC (SEQ ID NO:175) |
| Survey_VEGF-rev | GAGAGCCGTTCCCTCTTTGC (SEQ ID NO:176) |

### Primers for High-throughput sequencing of on-target and off-target sites in human genome.

| | |
|---|---|
| HTS_EMX_ON-fwd | CACTCTTTCCCTACACGACGCTCTTCCGATCT CCTCCCCATTGGCCTGCTTC (SEQ ID NO:177) |
| HTS_EMX_Off1-fwd | CACTCTTTCCCTACACGACGCTCTTCCGATCT TCGTCCTGCTCTCACTTAGAC (SEQ ID NO:178) |
| HTS_EMX_Off2-fwd | CACTCTTTCCCTACACGACGCTCTTCCGATCT TTTTGTGGCTTGGCCCCAGT (SEQ ID NO:179) |
| HTS_EMX_Off3-fwd | CACTCTTTCCCTACACGACGCTCTTCCGATCT TGCAGTCTCATGACTTGGCCT (SEQ ID NO:180) |
| HTS_EMX_Off4-fwd | CACTCTTTCCCTACACGACGCTCTTCCGATCT TTCTGAGGGCTGCTACCTGT (SEQ ID NO:181) |
| HTS_VEFG_ON-fwd | CACTCTTTCCCTACACGACGCTCTTCCGATCT ACATGAAGCAACTCCAGTCCCA (SEQ ID NO:182) |
| HTS_VEGF_Off1-fwd | CACTCTTTCCCTACACGACGCTCTTCCGATCT AGCAGACCCACTGAGTCAACTG (SEQ ID NO:183) |
| HTS_VEGF_Off2-fwd | CACTCTTTCCCTACACGACGCTCTTCCGATCT CCCGCCACAGTCGTGTCAT (SEQ ID NO:184) |
| HTS_VEGF_Off3-fwd | CACTCTTTCCCTACACGACGCTCTTCCGATCT CGCCCCGGTACAAGGTGA (SEQ ID NO:185) |
| HTS_VEGF_Off4-fwd | CACTCTTTCCCTACACGACGCTCTTCCGATCT GTACCGTACATTGTAGGATGTTT (SEQ ID NO:186) |
| HTS_CLTA2_ON-fwd | CACTCTTTCCCTACACGACGCTCTTCCGATCT CCTCATCTCCCTCAAGCAGGC (SEQ ID NO:187) |
| HTS_CLTA2_Off1-fwd | CACTCTTTCCCTACACGACGCTCTTCCGATCT ATTCTGCTCTTGAGGTTATTTGT (SEQ ID NO:188) |
| HTS_CLTA2_Off2-fwd | CACTCTTTCCCTACACGACGCTCTTCCGATCT CACCTCTGCCTCAAGAGCAGAAAA (SEQ ID NO:189) |
| HTS_CLTA2_Off3-fwd | CACTCTTTCCCTACACGACGCTCTTCCGATCT TGTGTGTGTGTGTGTGTAGGACT (SEQ ID NO:190) |
| HTS_EMX_ON-rev | GGAGTTCAGACGTGTGCTCTTCCGATCT TCATCTGTGCCCCTCCCTCC (SEQ ID NO:191) |
| HTS_EMX_Off 1-rev | GGAGTTCAGACGTGTGCTCTTCCGATCT CGAGAAGGAGGTGCAGGAG (SEQ ID NO:192) |
| HTS_EMX_Off2-rev | GGAGTTCAGACGTGTGCTCTTCCGATCT CGGGAGCTGTTCAGAGGCTG (SEQ ID NO:193) |
| HTS_EMX_Off3-rev | GGAGTTCAGACGTGTGCTCTTCCGATCT CTCACCTGGGCGAGAAAGGT (SEQ ID NO:194) |
| HTS_EMX_Off4-rev | GGAGTTCAGACGTGTGCTCTTCCGATCT AAAACTCAAAGAAATGCCCAATCA (SEQ ID NO:195) |
| HTS_VEFG_ON-rev | GGAGTTCAGACGTGTGCTCTTCCGATCT AGACGCTGCTCGCTCCATTC (SEQ ID NO:196) |
| HTS_VEGF_Off1- | GGAGTTCAGACGTGTGCTCTTCCGATCT |
| rev | ACAGGCATGAATCACTGCACCT (SEQ ID NO:197) |
| HTS_VEGF_Off2-rev | GGAGTTCAGACGTGTGCTCTTCCGATCT GCGGCAACTTCAGACAACCGA (SEQ ID NO:198) |
| HTS_VEGF_Off3-rev | GGAGTTCAGACGTGTGCTCTTCCGATCT GACCCAGGGGCACCAGTT (SEQ ID NO:199) |
| HTS_VEGF_Off4-rev | GGAGTTCAGACGTGTGCTCTTCCGATCT CTGCCTTCATTGCTTAAAAGTGGAT (SEQ ID NO:200) |
| HTS_CLTA2_ON-rev | GGAGTTCAGACGTGTGCTCTTCCGATCT ACAGTTGAAGGAAGGAAACATGC (SEQ ID NO:201) |
| HTS_CLTA2_Off1-rev | GGAGTTCAGACGTGTGCTCTTCCGATCT GCTGCATTTGCCCATTTCCA (SEQ ID NO:202) |
| HTS_CLTA2_Off2-rev | GGAGTTCAGACGTGTGCTCTTCCGATCT GTTGGGGGAGGAGGAGCTTAT (SEQ ID NO:203) |
| HTS_CLTA2_Off3-rev | GGAGTTCAGACGTGTGCTCTTCCGATCT CTAAGAGCTATAAGGGCAAATGACT (SEQ ID NO:204) |
| HTS_EGFP-fwd | CACTCTTTCCCTACACGACGCTCTTCCGATCTNNNN ACGTAAACGGCCACAAGTTC (SEQ ID NO:205) |
| HTS_EGFP-rev | GGAGTTCAGACGTGTGCTCTTCCGATCT GTCGTCCTTGAAGAAGATGGTG (SEQ ID NO:206) |
| HTS_MusEMX_ON-fwd | CACTCTTTCCCTACACGACGCTCTTCCGATCT CCAGGTGAAGGTGTGGTTCCAG (SEQ ID NO:207) |
| HTS_MusEMX_ON-rev | GGAGTTCAGACGTGTGCTCTTCCGATCT CCCCTAGTCATTGGAGGTGAC (SEQ ID NO:208) |

### Results

### Highly efficient delivery of Cre recombinase fused to a supernegatively charged protein

It was speculated that imparting the highly anionic electrostatic properties of nucleic acids to genome-editing proteins may enable their efficient delivery into mammalian cells using cationic lipids (Figure 27(A)). For proteins of interest that are not natively highly negatively charged, it was thought that fusion with a natural or engineered supernegatively charged protein¹⁷ would impart a polyanionic character. For nucleic acid-binding proteins, it was speculated that simple complexation with native DNA or RNA substrates might provide sufficient anionic character to support cationic lipid-based delivery (Figure 27(A)).

It was first tested whether the engineered supernegatively charged GFP variant,³⁵ (-30)GFP, could mediate encapsulation and delivery of fused protein cargo (Figure 27(B)). (-30)GFP was fused to Cre recombinase and several commercially available cationic lipids were tested for their ability to functionally deliver the fusion into HeLa cells that only express DsRed upon Cre-mediated recombination (Figure 28(A)). Delivery of 10 nM (-30)GFP-Cre complexed with 1.5 µL Lipofectamine RNAiMAX (hereafter referred to as "RNAiMAX", Life Technologies, Carlsbad CA) in media containing 10% fetal bovine serum (FBS) led to strong DsRed fluorescence signal among treated cells. Fluorescence-activated cell sorting (FACS) revealed that 48 hours after treatment 52% of cells expressed DsRed consistent with Cre recombination (Figure 28(B)).

Optimization resulted in recombination efficiencies of 65% using 25 nM (-30)GFP-Cre complexed with 1.5 µL RNAiMAX in 250 µL of media containing 10% FBS (Figure 28(C)). The potency of lipid-mediated anionic Cre delivery is notable compared to that of cationic protein-mediated delivery. Only 1 nM (-30)GFP-Cre with cationic lipid was needed to result in 15-20% recombined cells, whereas 1 µM (+36)GFP-Cre was required to achieve this extent of recombination, representing a 1,000-fold difference in delivery potency (Figure 28(C)). Nearly identical results were observed in a second Cre reporter cell line (BSR TdTomato) (Figure 33(A)). Increasing the amount of cationic lipid increased toxicity (Figure 33(B)) and it was found that 1.5 µL RNAiMAX per 250 µL sample maximized recombination efficiency while inducing minimal cell toxicity. Under these conditions, cationic lipids did not increase the delivery potency of neutral or cationic Cre recombinase fusions (Figure 28(C) and Figure 33(C)), indicating that the strongly negative charge of (-30)GFP-Cre was required to participate in cationic lipid-mediated delivery. It was also observed that increasing the amount of cationic lipid increased the concentration of protein required for maximal recombination, consistent with a model in which deliverable proteins are complexed with specific stoichiometries of cationic lipids (Figure 28(D)). These observations collectively indicate that cationic lipids can mediate the potent delivery of polyanionic proteins into mammalian cells even in the presence of serum.

To determine if the higher potency of cationic lipid-mediated (-30)GFP-Cre delivery relative to cationic protein-mediated delivery arises from more total protein uptake by cells, or from a higher fraction of functional, non-endosomal protein molecules that enter cells, flow cytometry was used to measure GFP fluorescence of cells treated with either (+36)GFP-Cre or liposomal (-30)GFP-Cre under their respective optimal Cre delivery conditions. Comparison of cellular fluorescence and recombination efficiency reveals that lipid-mediated functional delivery of (-30)GFP-Cre is 9,800-fold more potent per amount of endocytosed protein than delivery of (+36)GFP-Cre (Figure 34). Taken together, these results suggest that the unusually high potency of lipid-mediated delivery of anionic proteins does not arise from unusually high protein uptake in each cell, but rather from post-endocytosis processes that likely include endosomal escape into the cytoplasm and the avoidance of lysosomal protein degradation.

To test whether the ability to deliver polyanionic proteins is dependent on proprietary components in RNAiMAX or if other cationic lipids are capable of mediating similarly potent delivery, several other transfection reagents designed to deliver nucleic acids were tested (Figure 28(E)). While RNAiMAX remained the most effective functional delivery agent for (-30)GFP-Cre, other cationic lipid formulations also resulted in potent delivery. Lipofectamine 2000 and Lipofectamine LTX (Life Technologies, Carlsbad CA), two plasmid transfection reagents based on cationic lipid formulations,²¹ and SAINT-Red (Synvolux Therapeutics, Groningen Netherlands), an siRNA delivery formulation containing a synthetic pyridium-containing cationic lipid, all resulted in strong functional (-30)GFP-Cre delivery over a range of concentrations (Figure 28(E)). In contrast, strong deliveries with the cationic lipid DOTAP (Roche Diagnostics, Indianapolis IN) or the peptide-based nucleic acid delivery agent EZ-PLEX (Ascension Bio, Tampa FL) were not observed (Figure 28(E)). These observations collectively indicate that several (but not all) cationic lipids are able to encapsulate and deliver negatively charged proteins into human cells.

It was speculated that it should be possible to use cationic lipids to deliver polyanionic proteins other than (-30)GFP. Engineered polyanionic protein domains commonly used in biomedical research include the VP64 activation domain (-22 net theoretical charge) widely used in fusions with engineered zinc finger arrays, TALE repeat arrays, or dCas9 for transcriptional activation, and 3x FLAG (-7 net theoretical charge), an epitope tag used for protein purification and visualization (Figure 28(F)). It was observed that both VP64 and 3x FLAG enhance functional delivery of Cre recombinase with cationic lipids, though not as effectively as (-30)GFP, likely due to their lower overall negative charge (Figure 33(C)). These observations demonstrate that unusually negatively charged proteins beyond (-30)GFP can mediate efficient cationic lipid-based delivery into mammalian cells. Protein delivery efficacy by cationic lipids is predominantly a function of total negative charge, and does not require a particular distribution of anionic residues.

### Comparison of recombination efficiency and cellular toxicity for liposomal protein delivery of (-30)GFP-Cre versus optimized plasmid DNA transfection

We optimized plasmid transfection of HeLa reporter cells across a range of plasmid and Lipofectamine 2000 doses, and found that transfection efficiency in this cell line yielded a maximum of 33% DsRed fluorescent cells (Figure 27(B)). These findings suggest that cationic lipid-based (-30)GFP-Cre protein delivery can result in more functional Cre recombinase activity than well-established plasmid DNA transfection methods.

As nucleic acid transfection by cationic lipids is to known to induce cellular toxicity,¹ we also characterized the toxicity of cationic lipid-mediated (-30)GFP-Cre protein delivery and compared the results with those of plasmid transfection methods (Figures 27(B-C)). Cells undergoing protein delivery or plasmid transfection were analyzed for cell survival by flow cytometry using the TO-PRO-3 live/dead cell stain (Life Technologies). While increasing the amount of RNAiMAX predictably increased toxicity (Figure 27(B)), the use of 1.5 µL RNAiMAX per 275 µL sample maximized recombination efficiency (> 50% DsRed-positive live cells) while inducing minimal cell toxicity (> 80% live cells, Figure 27(C)). In contrast, all efficacious plasmid DNA delivery conditions tested exhibited much greater toxicity (Figure 27(D)), with fewer than 40% of cells surviving plasmid transfection under any condition that resulted in > 5% DsRed-positive live cells. These results indicate that optimized cationic lipid-mediated delivery of anionic Cre recombinase achieves substantially greater delivered Cre activity with much lower toxicity than optimized plasmid DNA transfection.

### Delivery efficacy of various anionic proteins fused to Cre

We observed that both VP64 and 3x FLAG enhance functional delivery of Cre recombinase with cationic lipids, though not as effectively as (-30)GFP, likely due to their lower overall negative charge (Figure 28(F)). To further probe the relationship between net anionic charge and protein delivery efficiency, we generated two new anionic GFP-Cre fusions of comparable charge as 3xFLAG-Cre and VP64-Cre using (-7)GFP and (-20)GFP, respectively. The (-7)GFP-Cre and (-20)GFP-Cre fusions showed nearly identical protein delivery efficacy as their like-charged anionic peptide-tagged counterparts (Figure 28(F)), suggesting that the efficacy of delivery by cationic lipids is predominantly a function of the total negative charge, and not the distribution or density of charged residues.

### Functional delivery of TALE activator proteins

The lipid-mediated delivery of TALE-VP64 transcriptional activators (approximately +4 theoretical net charge, depending on TALE variant used) into cultured human cells was tested. While modestly effective cleavage of endogenous genes by delivered TALEN proteins has been demonstrated in mammalian cells in the absence of serum using cationic peptides such as Arg₉,³⁶ the delivery of TALE-based transcription factor proteins has not yet been reported, and no effective delivery of TALE proteins in serum has been previously described to our knowledge. The gene for neurotrophin-3 (NTF3), a neural growth factor that has been associated with neurodegenerative diseases, was targeted.³⁷ A previously described NTF3-targetting TALE-VP64³⁸ was fused to (-30)GFP (Figure 29(A)) and treated HEK293T cells with 25 nM (-30)GFP-NTF3 TALE1-VP64 and RNAiMAX under the conditions optimized for Cre delivery. Gene expression levels of NTF3 4 hours after treatment were 3.5-fold higher in cells treated with 25 nM (-30)GFP-NTF3 TALE-VP64 and RNAiMAX than untreated cells, cells treated with RNAiMAX only, or cells treated with a VEGF-targeting TALE transcriptional activator (Figure 29(B)). Comparable levels of NTF3 expression were observed 48 hours after transfection of plasmids encoding the same NTF3-targeting TALE-VP64 (Figure 29(B)).

Since the synergistic expression of multiple TALE activators targeting different sites on the same gene has been shown to augment gene activation,³⁸ five distinct NTF3-targeting TALE activators fused to (-30)GFP using RNAiMAX were simultaneously delivered. Protein-lipid complexes were prepared as above by adding the five (-30)GFP-NTF3-TALE-VP64 proteins at 5 nM each, for a total of 25 nM protein. A 7-fold increase in NTF3 expression was observed after a 4-hour incubation (Figure 29(B) and Figure 35), while plasmid co-transfection of all five NTF3 TALE activators, followed by a 48-hour incubation, resulted in a 10-fold increase in NTF3 expression levels (Figure 29(B)). To characterize TALE activity over time using these two methods, NTF3 mRNA levels were measured over a 48-hour period following protein or DNA delivery. TALE activator activity following protein delivery peaks ~4 hours post-treatment and falls over the next 44 hours (Figure 29(C)), whereas plasmid DNA transfection required ~24 hours to show above-background levels of NTF3 activation, which plateaued at ~36-48 hours (Figure 29(C)). These findings demonstrate that TALE activator proteins can be delivered using cationic lipids to transiently activate gene expression in human cells. While not limited to such embodiments, this capability may prove especially valuable for proteins that effect a one-time permanent change in cell state or cell fate when transiently expressed.³⁹

### Highly efficient delivery of Cas9:sgRNA protein:RNA complexes into human cells

Given the potent lipid-mediated delivery of polyanionic Cre and TALE activator protein variants in full-serum media, it was speculated that CRISPR-Cas9:sgRNA complexes, either as fusions with (-30)GFP or as native polyanionic Cas9:guide RNA complexes, might also be delivered into human cells using this approach. Using a well-established Cas9-induced gene disruption assay,⁴⁰ specific sites within a genomic EGFP reporter gene in human U2OS cells were targeted (Figure 36(A)). On-target Cas9 cleavage induces non-homologous end joining (NHEJ) in *EGFP* and the loss of cell fluorescence. To avoid interference from the fluorescence of (-30)GFP, a Y67S mutation was introduced into (-30)GFP to eliminate its fluorescence, and designated this non-fluorescent variant as (-30)dGFP.

Treatment of U2OS reporter cells with 25 nM (-30)dGFP-NLS-Cas9 and 50 nM EGFP-targeting sgRNA with RNAiMAX in media containing 10% FBS showed loss of EGFP expression in 48% of cells (Figure 30(A)). Cotransfection of plasmids expressing Cas9 or sgRNA resulted in similar EGFP loss in 37% of cells (Figure 30(A)). No significant *EGFP* disruption was observed upon transfection of plasmids encoding EGFP sgRNA alone, Cas9 alone, or cotransfection of plasmids encoding Cas9 and an sgRNA designed to target a *VEGF* locus (Figure 30(A), Figure 36(B)). It was confirmed that the robust disruption of EGFP was not a result of cellular toxicity (Figures 36(C)-(D)). It was also observed that treatment of cells with (+36)dGFP-NLS-Cas9 and sgRNA in the presence of 10% FBS serum did not lead to efficient gene disruption (Figure 30(A)), suggesting that cationic-peptide based methods of delivery for Cas9 and sgRNA are not effective perhaps due to interference of gRNA: Cas9 complex formation or nuclease function by superpositively charged proteins.⁴¹ Together, these results establish that cationic lipid-mediated delivery of (-30)dGFP-NLS-Cas9:sgRNA complexes can result in efficient sgRNA-dependent target gene disruption in human cells. Cas9 and sgRNA in the presence of 10% FBS did not lead to efficient gene disruption (Figure 30(A)), suggesting that cationic-protein based methods of delivery for Cas9 and sgRNA may not be effective, perhaps due to interference of Cas9:sgRNA complex formation or nuclease function by cationic proteins,⁴¹ consistent with a recent study describing the delivery of Cas9 protein with an oligoarginine peptide tag which achieved only moderate levels of gene disruption.⁵⁸ Optimization of plasmid transfection conditions did not yield higher than 40% EGFP disruption (Figure 30(A)and Figure 31(A)), and the transfection conditions required to achieve this level of gene disruption resulted in high levels of cellular toxicity (Figure 31(B)). Together, these results establish that cationic lipid-mediated delivery of (-30)dGFP-NLS-Cas9: sgRNA complexes can result in efficient sgRNA-dependent target gene disruption in human cells with minimal toxicity, unlike cationic peptide-based protein delivery or plasmid DNA transfection methods.

### Polyanionic sgRNA is necessary and sufficient for efficient lipid-mediated Cas9 delivery

Since the complex of native Cas9 protein (+22 net theoretical charge) and an sgRNA (-103 anionic phosphate groups) should be overall highly anionic, next it was tested if native Cas9:sgRNA complexes without fusion to polyanionic proteins can be delivered into human cells using cationic lipids. Treatment of U2OS EGFP reporter cells with 100 nM Cas9, 100 nM EGFP sgRNA, and 0.8 µL RNAiMAX resulted in 65% disruption of the EGFP reporter gene (Figure 30(A)). Treatment of cells with Cas9 protein and sgRNA, but without RNAiMAX, resulted in no loss of GFP fluorescence (Figure 30(A)). These observations suggest that sgRNA alone, even in the absence of a supernegatively charged fusion protein, can provide the highly anionic character needed to mediate cationic lipid-based delivery of Cas9.

Treatment of U2OS EGFP reporter cells with 100 nM Cas9, 50 nM EGFP sgRNA, and 0.8 µL RNAiMAX resulted in 65% disruption of the *EGFP* reporter gene (Figure 30(A)). These observations suggest that sgRNA alone, even in the absence of a supernegatively charged fusion protein, can provide the highly anionic character needed to mediate cationic lipid-based delivery of Cas9. We evaluated several different Cas9 constructs over a broad range of conditions (Figures 37(A-D), Figures 38(D-F) and results herein) and lipid formulations (Figure 39(A) and results herein) for their effect on *EGFP* disruption and observed that up to 80% targeted gene disruption resulted from Cas9: sgRNA complexed with Lipofectamine 2000 (Figure 30(A)). Due to the modestly higher toxicity of Lipofectamine 2000 compared to RNAiMAX across a range of doses (Figures 39(B-D) and results herein), we continued using RNAiMAX for cell culture studies unless otherwise noted.

Comparison of gene disruption efficiency arising from the cationic lipid-mediated delivery of (-30)dGFP-NLS-Cas9: sgRNA versus Cas9:sgRNA revealed that at low doses (-30)dGFP-NLS-Cas9 results in more efficient gene disruption than native Cas9 (Figure 37(A)), it is outperformed by native Cas9 at higher concentrations, as well as at the respective optimal protein: sgRNA dose of either protein (Figures 37(B)-37(C)). These results further establish that sgRNA can supply sufficient negative charge to support cationic lipid-based delivery of complexed Cas9 protein.

It was also observed that while overall less protein was required for optimal delivery of (-30)dGFP-NLS-Cas9 than Cas9, a higher sgRNA:protein ratio was required for maximal (-30)dGFP-NLS-Cas9-mediated EGFP gene disruption than for native Cas9-mediated gene disruption (Figure 37(D)). It was speculated that more equivalents of sgRNA are needed to complex with (-30)dGFP-NLS-Cas9 since fused (-30)dGFP may electrostatically interfere with Cas9:sgRNA complexation. As the ideal protein dose for (-30)dGFP-NLS-Cas9 mediated EGFP gene disruption is 10-fold lower than that of wild-type Cas9, the results also suggest that (-30)dGFP-Cas9 is better encapsulated by cationic liposomes than Cas9:sgRNA due to its higher overall negative charge, but this charge magnitude may interfere with Cas9:sgRNA interactions, necessitating more sgRNA per protein and potentially reducing total delivered Cas9 activity. In addition, NLS-Cas9 and Cas9-NLS proteins were generated and tested, and it was observed that while the presence of an NLS in (-30)dGFP-NLS-Cas9 could at least partially explain differences in delivery efficacy at very low concentrations, Cas9, NLS-Cas9, and Cas9-NLS all result in higher efficiency of *EGFP* disruption than (-30)dGFP-NLS-Cas9 at 25 nM or higher concentrations (Figures 38(A)-(C)) the reduction in activity due to the presence of the large anionic fusion partner to Cas9 compromises its overall performance.

Cas9: sgRNA delivery with cationic lipid formulations other than RNAiMAX was also tested. Delivery with Lipofectamine 2000 was notably more efficient than with RNAiMAX, resulting in up to 80% Cas9-mediated gene disruption (Figure 39(A)), and maintaining high efficiency (60% gene disruption) even at 1 nM protein (Figure 39(A)). However, due to the somewhat higher toxicity of Lipofectamine 2000 compared to RNAiMAX under cell culture conditions (Figures 33(B)-C)), RNAiMAX was used for all subsequent cell culture studies.

To verify that EGFP disruption arose from genome modification and not only from Cas9 binding,⁴² the T7 endonuclease I (T7EI) assay⁴³ was used to detect and quantify the frequency of Cas9-mediated genomic insertion/deletion mutations (indels) at the target EGFP locus (Figure 30(B)). The T7EI assay results showed that only those cells treated with both Cas9 and EGFP sgRNA plasmids, or Cas9 protein and purified EGFP sgRNA, contained indels at the target site 48 hours after treatment. Taken together, these findings establish that active Cas9:sgRNA complexes can be potently delivered into human cells with cationic lipids in a manner dependent on the negative charge provided by the sgRNA.

U2OS EGFP reporter cells were also treated with a single lipid-mediated delivery treatment of Cas9 complexed with a mixture of four gRNAs targeting *EGFP, CLTA, EMX,* and *VEGF.* This treatment resulted in efficient disruption of all four targets, with cleavage efficiencies of 58%, 28%, 16%, and 40%, respectively, as measured by T7E1 cleavage assay. These high gene disruption efficiencies from a single delivery of 50 nM Cas9 and 25 nM of each sgRNA (100 nM total sgRNA) demonstrate that lipid-mediated Cas9:sgRNA delivery can support efficient multiplexed genome editing (Figure 30(C)).

We also tested whether delivered Cas9 nuclease: sgRNA complexes are capable of effecting homology-directed repair (HDR) using an EGFP-repair reporter cell line.⁵⁷ We combined Cas9 and *EGFP-*targeting sgRNA, mixed the resulting protein:RNA complexes with varying concentrations of single-stranded DNA oligonucleotide (ssODN) donor template , and delivered the Cas9:sgRNA + ssODN mixture using Lipofectamine 2000 (Figure 44(A)). Cas9:sgRNA delivery achieved EGFP HDR frequencies of ~8-11%, similar to that of optimized plasmid transfection-based HDR (Figures 44(B-C)), and consistent with previous reports using the same reporter cell line, suggesting that cationic lipid-based delivery of Cas9:sgRNA is a viable approach to efficient HDR.

Next we determined whether cationic lipid-based protein delivery could be applied to deliver other Cas9-derived genome engineering tools such as Cas9 nickases⁴⁴ and Cas9-based transcriptional activators.⁴⁵ We measured gene disruption efficiency in U2OS EGFP reporter cells resulting from delivery of Cas9 D10A nickase (Figure 30(D)and results herein) and achieved results similar to previous reports using plasmid transfection.⁴⁶ Delivery of dCas9-VP64 activators either by plasmid transfection or RNAiMAX-mediated protein delivery resulted in strong (≥ ~10-fold) activation of *NTF3* transcription (Figure 30(E) and Figure 40(A)). As observed above with TALE activators (Figure 29(C)), dCas9-VP64 protein delivery resulted in fast-acting and transient transcriptional activation compared to DNA delivery (Figure 40(B) and results herein). These results collectively indicate that both Cas9 nickases and Cas9 transcriptional activators can also be delivered effectively by cationic lipid-mediated protein: sgRNA complex delivery.

### Functional delivery of Cas9 nickases and dCas9 activators

Next, whether cationic lipid-based protein delivery could be extended to deliver other Cas9-derived genome engineering tools such as Cas9 nickases⁴⁴ and Cas9-based transcriptional activators was tested.⁴⁵ Gene disruption efficiency in U2OS EGFP reporter cells resulting from delivery of Cas9 D10A nickase was measured, either by cotransfection of nickase and appropriate paired EGFP-targeting sgRNA plasmids, or as purified protein complexed with pairs of EGFP sgRNAs using RNAiMAX (Figure 30(D)). Both plasmid and cationic lipid-mediated protein:RNA delivery of dual Cas9 nickases resulted in EGFP disruption with similar efficiencies (Figure 30(D)) only in the presence of sgRNA pairs targeting opposite strands, (sgRNA pairs g1+g5, and g3+g7), but not with sgRNA pairs targeting the same strand (sgRNA pair g5+g7) (Figure 30(D)), consistent with previous reports of Cas9 nickase cleavage requirements.⁴⁶

The *NTF3* transcriptional activation efficiencies in HEK293T cells resulting from either plasmid transfection or direct protein: sgRNA complex delivery of dCas9 fused to a VP64 activation domain were also compared.⁴⁵ Delivery of dCas9-VP64 activators either by plasmid transfection or RNAiMAX-mediated protein delivery resulted in strong (≥ ~10-fold) activation of *NTF3* transcription (Figure 30(E) and Figure 40). Transcriptional activation levels resulting from plasmid transfection were more potent than activation resulting from protein delivery at optimal assay times for each delivery method (Figure 30(E)), potentially due to the sustained expression both Cas9 activator protein and sgRNA from the plasmids compared to the transient, single dose of purified protein and RNA. While the above results indicate that such factors do not limit the potency of irreversible genome modification by delivered Cas9 nuclease and nickase proteins (Figures 40(A) and 40(D)), the low dose and transient nature of the delivered protein may more strongly limit potency of dynamic processes such as transcriptional activation. Nevertheless, these results collectively indicate that both Cas9 nickases and Cas9 transcriptional activators can also be delivered effectively by cationic lipid-mediated protein: sgRNA complex delivery.

### Comparison of Lipofectamine 2000 and RNAiMAX for Cas9 delivery efficiency and toxicity

We tested Cas9:sgRNA delivery with cationic lipid formulations other than RNAiMAX. *EGFP* disruption with Lipofectamine 2000 was notably more efficient than with RNAiMAX, resulting in up to 80% Cas9-mediated gene disruption (Figure 39(A)), and maintaining high efficiency (60% gene disruption) even at 1 nM protein (Figure 39(A)). However, due to the somewhat higher toxicity of Lipofectamine 2000 (Figure 39(B)) for protein: sgRNA delivery compared to that of RNAiMAX (Figure 39(C)) under cell culture conditions, we continued to use RNAiMAX for subsequent cell culture studies. We also observed that increasing the dosage of Cas9:sgRNA increased toxicity at constant amounts of either RNAiMAX or Lipofectamine 2000 (Figure 39(D)).

### Cas9:sgRNA delivery modifies genomes with greater specificity than DNA transfection

DNA-free delivery of functional Cas9:sgRNA complexes circumvents risks associated with viral or other gene delivery methods and has the potential to improve the specificity of genome modification by avoiding the unnecessary expression of genome-editing agent after the target locus is modified. Transient delivery of functional Cas9: sgRNA protein:RNA complexes circumvents risks associated with viral or other gene delivery methods and has the potential to improve the specificity of genome editing by minimizing the opportunity of agents to modify off-target substrates after the target locus is modified, or to reverse on-target modification. To test if the described approach can disrupt endogenous genes in human cells, genomic loci in the *EMX1, CLTA2,* and *VEGF* genes were targeted due to their potential biomedical relevance and their use in previous studies^{40,46,47} of Cas9 off-target cleavage activity. Cationic lipid-mediated delivery of Cas9:sgRNA complexes into HEK293T cells resulted in robust cleavage of all three human genes with efficiencies comparable to or greater than those of plasmid transfection methods as revealed by the T7EI assay using the same Cas9: sgRNA delivery conditions previously optimized for U2OS cells (Figure 31(A)).

To compare the endogenous gene modification specificity of plasmid versus protein:RNA delivery methods for Cas9, the on-target locus was amplified as well as several known off-target sites (Table 1) from genomic DNA isolated from HEK293 cells treated either by transfection of Cas9 and sgRNA expression plasmids, or by RNAiMAX-mediated Cas9:sgRNA complex delivery under conditions that resulted in comparable on-target modification efficiencies. The indel frequencies at the three on-target and 11 off-target sites were assayed by high-throughput DNA sequencing (Table 2). For all three target genes, the frequency of on-target DNA modification resulting from either plasmid or protein: sgRNA delivery was approximately 10%±2% (Figure 41), enabling the comparison of off-target modification under conditions that result in very similar on-target modification efficiencies. Importantly, the frequency of off-target genome modification for all 11 off-target sites was lower from protein: sgRNA delivery compared with plasmid delivery (Figures 41(A-C)), and as a result the ratio of on-target to off-target modification ratio for all sites tested was up to 19-fold higher for protein: sgRNA delivery than for plasmid delivery (Figures 31(B-D)).

We also observed that the increase in specificity for Cas9 protein delivery relative to DNA transfection persists across a wide range of on-target cleavage efficiencies (-1%, -10%, and ~40%) (Figure 46andresults herein). This increase in specificity using protein delivery is consistent with the transient nature of the delivered protein: sgRNA complexes compared to plasmid transfection (Figure 47and results herein). We also measured the amount of protein internalized by cells using our cationic lipid-based protein delivery approach and determined that ~4% of the total protein used in the treatment was internalized by cells (Figure 48and results herein). We note that the majority of this protein likely exists within endosomes and may not be available to effect genome modification.^{18, 59}

We tested whether the observed increase in specificity for Cas9 protein delivery holds at different cleavage efficiencies, focusing on the *VEGF* on-target and its four known off-target sites. We tuned Cas9-mediated on-target modification rates over a broad range by scaling the amount of Cas9:sgRNA delivered by plasmid transfection and liposomal protein delivery, resulting in conditions that yield low (-1%), moderate (-10%), and high (-40%) on-target DNA modification. We observed that across all levels of on-target modification, Cas9: sgRNA delivery always resulted in substantially (typically ~10-fold) higher on: off-target modification ratios than comparable Cas9 plasmid DNA transfections (Figure 46). This increase in specificity can likely be explained by the transient nature of the delivered protein: sgRNA complexes (Figure 47) as well as the quality of the sgRNA complexed with the Cas9 protein compared to that of the endogenously produced sgRNA transcripts. There is the potential for degraded or otherwise modified sgRNAs to interact with the Cas9 protein and allow it to mediate unintended and unpredictable genome modifications. We also note that RNA pol III transcription has an error rate of ~10⁻⁵, while published T7 RNAP error rates may be up to 10-times lower. In a given 20-base spacer target sequence, there would be one incorrect version per every 5,000 transcripts versus one in every 50,000 for our pre-complex sgRNAs. Such differences may further account for the observed increases in specificity.

DNA modification specificity was higher for protein: sgRNA delivery than for plasmid delivery at loci with high levels of off-target modification (such as the four *VEGF* off-target sites, for which plasmid delivery yielded average on-target off-target modification ratios between 4- and 20-fold but protein:RNA delivery yielded average on-target: off-target modification ratios between 9- and 400-fold) as well as for loci with lower levels of off-target modification (such as the three *EMX* off-target loci, for which plasmid delivery yielded average on-target: off-target modification ratios as low as 64-fold but protein:RNA delivery yielded average on-target off-target modification ratios of 500- to 2,000-fold). Taken together, these results indicate that the delivery of Cas9: sgRNA complexes using cationic lipids can effect target gene modification at high efficiency and with substantially greater specificity than the delivery of DNA expressing Cas9 and sgRNA.

### Time course of gene disruption from Cas9:sgRNA delivery versus plasmid DNA transfection

The remarkable increases in Cas9 specificity for protein: sgRNA delivery is likely a result of the transient nature of the delivered protein that was directly observed with both TALE-activator and dCas9-activator delivery (Figure 35(A) and Figure 40(B)). We performed a time course experiment that measured indel modification rate by Surveyor assay from protein: sgRNA or plasmid DNA delivery over the course of 72 hours post-treatment (Figure 47). Whereas indel formation in U2OS EGFP reporter cells following Cas9 plasmid transfection continued to increase 72 hours after DNA delivery, protein: sgRNA delivery leads to near-maximal indel modification between 12 and 24 hours after treatment (Figure 47). Together, these results suggest that protein: sgRNA delivery rapidly achieves a transient dose of Cas9:sgRNA activity that mediates efficient genome modification and is degraded before off-target modifications can accumulate to the extent that arises from long-term expression.

### Quantification of total Cas9 protein uptake into cells

Finally, we quantitated the amount of protein internalized by cells using our cationic lipid-based protein delivery approach. We labeled Cas9 protein with Alexa 647 and delivered it to U2OS cells at 50 nM with 100 nM sgRNA. After 4 hours, cells were washed extensively to remove bound protein and trypsinized. Cellular Alexa 647 fluorescence was measured and compared to that of a standard curve of known Cas9-Alexa 647 amounts in the presence of an identical composition of media, cells, and lipid. Nearly all treated cells were found to have internalized the Cas9-Alexa 647 protein (Figure 48(A)), and 4% of the total protein used in the treatment was internalized by cells (Figure 48(A)). Comparison with the standard curve suggests that ~3×10⁷ molecules of Cas9-Alexa 647 entered each cell, corresponding to 0.4% of total cellular protein.⁶⁰ We note, however, that the majority of this protein is likely sequestered within endosomes and may not be immediately available to effect genome modification. ^{18, 59}

### Delivery of Cas9:sgRNA into mouse embryonic stem cells

The rapid, potent, and transient cationic lipid-mediated delivery of Cas9:sgRNA to effect genome editing could be especially useful in stem cells, where Cas9 off-target activity over the course of multiple cell divisions could lead to both unwanted mutations, and mosaicism. To test the effectiveness of Cas9: sgRNA delivery in stem cells, we treated mouse embryonic stem cells expressing Tau-EGFP⁴⁸ with Cas9 and an *EGFP*-targeting sgRNA. Under standard stem-cell culture conditions, EGFP-positive floating spheres were formed. We treated these floating spheres with Cas9:sgRNA complexed with Lipofectamine 2000, or with Cas9 and Lipofectamine 2000 without sgRNA as a control. Three days post-treatment, we observed a reduction in GFP fluorescence in the Cas9:sgRNA-treated spheres compared to the control samples (Figure 42(A)). The treated spheres were dissociated, and the cells were allowed to attach to a laminin-coated dish and differentiate into progenitor cells. Immunohistochemistry using an anti-GFP antibody confirmed knockdown of EGFP expression in the cells of Cas9: sgRNA treated samples, with many nuclei lacking any apparent EGFP. In contrast, all cells derived from control spheres were EGFP positive (Figure 42(B)). Genomic DNA harvested from Cas9:sgRNA-treated cells was subjected to T7EI assay, resulting in clear evidence of indels at the Tau-EGFP locus (Figure 42(C)). From this assay we calculated an indel frequency of 24% from cationic lipid-mediated Cas9:sgRNA delivery and 20% from DNA transfection. No target modification was detected in control samples lacking Cas9:sgRNA or containing Cas9 and an unrelated gRNA. These findings demonstrate that cationic lipid-mediated Cas9:sgRNA delivery can effect efficient gene disruption in mouse embryonic stem cells.

### Delivery of Cas9:sgRNA into mouse embryonic stem cells

The potent and transient cationic lipid-mediated delivery of Cas9:sgRNA to effect efficient, permanent, and highly specific gene editing could be especially useful in stem cells. To test this possibility, mouse embryonic stem cells expressing Tau-EGFP⁴⁸ were treated with Cas9 and an *EGFP*-targeting sgRNA. Under standard stem-cell culture conditions, EGFP-positive floating spheres were formed. The floating spheres were treated with Cas9:sgRNA complexed with RNAiMAX, or with Cas9 and RNAiMAX without sgRNA as a control. Three days post-treatment, a reduction in GFP fluorescence in the Cas9:sgRNA-treated spheres compared to the control samples was observed (Figure 42(A)). The treated spheres were dissociated, and the cells were allowed to attach to a gelatin-coated dish and differentiate into progenitor cells. Immunohistochemistry using an anti-GFP antibody confirmed knockdown of EGFP expression in the cells of Cas9: sgRNA treated samples, with many nuclei lacking any apparent EGFP. In contrast, all cells derived from control spheres were EGFP positive (Figure 42(B)). Genomic DNA harvested from Cas9:sgRNA-treated cells was subjected to T7EI assay, resulting in clear evidence of indels at the Tau-EGFP locus (Figure 42(C)). From this assay, an indel frequency of 42% was calculated from both cationic lipid-mediated Cas9:sgRNA delivery and transfection of Cas9 and sgRNA DNA. No target modification was detected in control samples lacking Cas9:sgRNA or containing Cas9 and an unrelated gRNA. These findings demonstrate that cationic lipid-mediated Cas9: sgRNA delivery can effect highly efficient gene disruption in mouse embryonic stem cells.

### In vivo cationic lipid-mediated delivery of Cre recombinase and Cas9:sgRNA

The high-efficiency delivery of functional genome-editing proteins *in vivo* enables a wide range of applications including non-viral therapeutic genome editing to correct genetic diseases. To evaluate the protein delivery method described above in a living mammal, delivery to the mouse inner ear was chosen, due to its confined space, well-characterized inner ear cell types, and the existence of genetic deafness mouse models that may enable future hearing recovery studies. The *in vivo* deliveries of two types of proteins into the mouse inner year were attempted. First, the delivery of (-30)GFP-Cre protein was tested to assess the targeting of inner ear cell types and the efficiency of functional protein delivery. Second, the delivery of Cas9: sgRNA complexes to the inner ear were evaluated to determine if cationic lipid-mediated protein:gRNA complex delivery can support CRISPR-based gene editing *in vivo.*

It has been previously shown that (+36)GFP-Cre can be delivered to mouse retina,¹⁶ although the protein resulted in only modest levels of recombinant conversion suggestive of inefficient *in vivo* delivery. For our initial inner ear delivery trials, (-30)GFP-Cre was complexed with RNAiMAX and the complex was injected into the cochlea of postnatal day 1 (P1) reporter mice with a genomically integrated floxed-STOP tdTomato reporter. As with the previously described *in vitro* Cre reporter cell line, functional delivery of Cre to the inner ear cells, followed by endosomal escape, nuclear localization, and Cre-mediated recombination results in expression of tdTomato. After injection, the cochleas were harvested for immunolabeling with inner ear cell markers for co-localization with tdTomato. RNAiMAX injection alone was used as control. Five days following injection of (-30)GFP-Cre and RNAiMAX, cochlear outer hair cells, the auditory sensory cells that detect sound, showed strong tdTomato signal that co-localized with the hair cell marker myosin VIIa (Myo7a), demonstrating functional Cre delivery to hair cells (Figures 32(A)-(B)). No tdTomato expression was detected in control cochleas (Figure 32(A)). The tdTomato signal was concentrated in the region of the injection site at the basal turn of the cochlea. On average 33±3% of outer hair cells were tdTomato positive at the base of the cochlea (P < 0.001; mean ± SEM, *n* = 4) and intact sterocilia were observed indicative of healthy hair cells (Figure 32(B)). We also tested delivery using Lipofectamine 2000 due to its higher potency *in vitro* (Figure 39(A)) and observed dramatically higher recombination efficiency: 91 ± 5% outer hair cells in cochleas treated with (-30)GFP-Cre + Lipofectamine 2000 were tdTomato positive (Figure 32(C)). In comparison to control samples, some outer hair cell loss was observed (Figure 42(C)), consistent with our previous observation of higher cell toxicity of Lipofectamine 2000, although overall cochlear architecture was preserved.

To further determine the effect of cationic lipid-mediated (-30)GFP-Cre protein delivery on targeted cells, hair cell stereocilia, a delicate structure that is essential for hearing, were examined 10 days post-injection. TdTomato positive outer hair cells had typical stereocilia structure as imaged by espin expression, similar to control stereocilia (Figure 32(B)). No tdTomato expression was detected in control cochleas. These observations indicate that cationic lipid-mediated delivery of (-30)GFP-Cre protein effects recombination in cochlear outer hair cells without apparently affecting hair cell architecture.

Because target volume, protein dose, and sgRNA dose *in vivo* are different than in cell culture experiments, the above experiments were repeated under different delivery conditions. Delivery using Lipofectamine 2000 was tested due to its higher potency *in vitro* (Figure 39(A)) and dramatically higher recombination efficiency was observed: over 90% outer hair cells in cochleas treated with (-30)GFP-Cre + Lipofectamine 2000 were tdTomato positive (Figure 32(C)). In comparison to control samples, some outer hair cell loss was observed (Figure 32(C)), consistent with the previous observation of the higher cell toxicity of Lipofectamine 2000, although the overall cochlear architecture was preserved.

To test the effectiveness of Cas9:sgRNA delivery *in vivo,* Cas9 and sgRNA targeting *EGFP* were combined with RNAiMAX and the resulting complexes were injected into postnatal day 2 (P2) transgenic Atoh1-GFP mouse cochlea in which all hair cells express GFP under the control of a hair cell-specific enhancer for transcription factor Atoh1.⁴⁹ Using this model, Cas9:sgRNA-mediated disruption of *EGFP* results in loss of EGFP fluorescence in outer hair cells. Ten days after injection of Cas9:sgRNA with cationic lipid, the absence of GFP was observed in 13% of outer hair cells near the injection site. In contrast, control cochlea injected with Cas9 protein and RNAiMAX without any sgRNA showed no loss of EGFP signal (Figure 32(D)). The outer hair cells of cochlea injected with Cas9:sgRNA RNAiMAX complexes appeared to be otherwise unaffected, with stereotypical expression of Myo7a and healthy nuclei, consistent with minimal hair cell toxicity (Figure 32(D)). High-throughput DNA sequencing of genomic DNA isolated from cochlea tissue samples revealed indels consistent with *GFP* target gene disruption in the treated samples, but not in the control samples that lacked sgRNA (Figure 43(A)). In addition, the inner ear *in vivo* delivery of Cas9: sgRNA using an sgRNA that targets the *EMX* gene was repeated and indels in the *EMX* gene in treated animals, but not control animals were similarly observed (Figure 43(B)).

After validating Cas9:sgRNA delivery in reporter cells (Figures 30(A-E)), and in neuron-derived mouse embryonic stem cells (Figure 42 and results herein), we tested Cas9:sgRNA delivery *in vivo.* Cas9 and sgRNA targeting *EGFP* were combined with RNAiMAX and the resulting complexes were injected into postnatal day 2 (P2) transgenic Atoh1-GFP mouse cochlea in which all hair cells express GFP under the control of a hair cell-specific enhancer for transcription factor Atoh1.⁴⁹ Using this model, Cas9:sgRNA-mediated disruption of *Atoh1-GFP* results in loss of GFP fluorescence in outer hair cells. Ten days after injection of Cas9:sgRNA with cationic lipid, we observed the absence of GFP in 13% of outer hair cells near the injection site. In contrast, control cochlea injected with Cas9 protein and RNAiMAX without any sgRNA showed no loss of EGFP signal (Figure 32(D)). The outer hair cells of cochlea injected with Cas9:sgRNA RNAiMAX complexes appeared to be otherwise unaffected, with stereotypical expression of Myo7a and healthy nuclei, consistent with minimal hair cell toxicity (Figure 32(D)). High-throughput DNA sequencing of genomic DNA isolated from cochlea tissue samples revealed indels consistent with *GFP* target gene disruption in the treated samples, but not in the control samples that lacked sgRNA (Figure 43(A)). In addition, we repeated inner ear *in vivo* delivery of Cas9:sgRNA using an sgRNA that targets the *EMX* gene and similarly observed indels in the *EMX* gene in treated animals, but not control animals (Figure 43(B)).).

As (-30)GFP-Cre complexed with Lipofectamine 2000 resulted in more efficient modification of the target hair cell population than (-30)GFP-Cre complexed with RNAiMAX (Figures 32(A) and 32(C)), its use on Cas9:sgRNA delivery to Atoh1-GFP cochlea was tested as above. Loss of GFP expression was observed in 20% ± 3% of outer hair cells near the injection site after 10 days, whereas all outer hair cells maintained strong GFP expression in control cochlea injected with Cas9 and Lipofectamine 2000 but no sgRNA (Figure 32(D)). In contrast to modest hair cell loss observed following Lipofectamine 2000 delivery of (-30)GFP-Cre (Figure 32(C)), outer hair cells targeted by Cas9:sgRNA exhibited no obvious toxicity or structural alteration (Figure 32(D)).

As with (-30)GFP-Cre, virus-free, cationic lipid-mediated delivery of Cas9: sgRNA into the mouse inner ear successfully modified a specific genomic locus in the outer hair cell population, leading to loss of target gene expression. Nearly half of all types of genetic deafness arise from hair cell loss or dysfunction,⁵⁰ the results presented herein suggest a potential strategy based on the delivery of Cas9:sgRNA complexes to genetically modify these cells to effect hearing recovery. These findings suggest that cationic lipid-mediated delivery of genome-editing proteins can serve as a powerful tool and a potential *in vivo* strategy for the treatment of genetic disease.

### Determination of protein delivery efficacy for (-30)GFP-Cre

To determine if the higher potency of liposome-mediated (-30)GFP-Cre delivery compared with that of cationic protein delivery arises from more total protein uptake by cells or from a higher fraction of functional, non-endosomal protein molecules taken up by the cells, flow cytometry was used to measure GFP fluorescence of cells treated with either (+36)GFP-Cre or liposomal (-30)GFP-Cre under their respective optimal Cre delivery conditions. Cell fluorescence reports total endocytosed (-30)GFP-Cre or (+36)GFP-Cre regardless of endosomal or non-endosomal localization.¹ Lipid-mediated protein delivery resulted in surprisingly small increases in total protein uptake (Figure 34(A)), despite the high efficiency of lipid-mediated functional Cre delivery. While (+36)GFP-Cre treatment increased cellular GFP fluorescence by up to three orders of magnitude in a dose-dependent manner (Figure 34(A)), consistent with previous reports,^{1,2} liposomal (-30)GFP-Cre treatment induced at most 5-fold increases in cellular GFP fluorescence (Figure 34(A)). Comparison of cellular fluorescence and recombination efficiency reveals that lipid-mediated functional delivery of (-30)GFP-Cre is 9,800-fold more potent per amount of endocytosed protein than delivery of (+36)GFP-Cre (Figure 34(B)).

To test if complexation of anionic (-30)GFP with cationic lipids interferes with GFP fluorescence and thus masks the true amount of cargo that enters the cell mCherry, which is fluorescent but not highly anionic, was fused to either (-30)GFP or (+36)GFP and delivered both protein fusions to HeLa cells. After washing away protein that may have adhered to cell surface but did not enter the cell with PBS + heparin (20 U/mL), the cells were analyzed by FACS for mCherry fluorescence 4 hours and 24 hours after treatment. It was observed that lipid-mediated delivery of (-30)GFP-fused mCherry results in only slight increases in cellular mCherry fluorescence, whereas mCherry fluorescence upon delivery of (+36)GFP-mCherry was generally ≥ 100-fold higher (Figure 34(C)) suggesting that fusion to (-30)GFP does not cause substantial amounts of protein cargo to enter the cell. Moreover, addition of lipids to (-30)GFP-Cre did not measurably alter the GFP fluorescence signal (Figure 34(D)), despite the fact that cationic lipids and anionic (-30)GFP clearly interact. Taken together, these results suggest that the unusually high potency of lipid-mediated delivery of anionic proteins does not arise from unusually high protein uptake in each cell, but rather from post-endocytosis processes that likely include avoidance of protein degradation and endosomal escape into the cytoplasm.

### Sensitivity limit of off-target cleavage assays

The sensitivity of the high-throughput sequencing method for detecting genomic off-target cleavage is limited by the amount genomic DNA (gDNA) input into the PCR amplification of each genomic target site. A 1 ng sample of human gDNA represents only approximately 330 unique genomes, and thus only approximately 330 unique copies of each genomic site are present. PCR amplification for each genomic target was performed on a total of 150 ng of input gDNA, which provides amplicons derived from at most 50,000, unique gDNA copies, respectively. Therefore, the high-throughput sequencing assay cannot detect rare genome modification events that occur at a frequency of less than 1 in 50,000 (0.002%). This limit is noted in Table 2.

Taken together, these findings suggest that cationic lipid-mediated delivery of genome-editing proteins can serve as a powerful tool and an *in vivo* strategy for the treatment of genetic disease.

### Conclusions

Efficient intracellular protein delivery *in vitro* and especially *in vivo* has been a persistent challenge in biomedical research and protein therapeutics. While delivery using cationic peptides and proteins has been widely studied for over two decades, sensitivity to serum proteins, neutralization by antibodies, degradation by extracellular and intracellular proteases, and poor endosomal escape post-internalization have limited the scope of protein delivery applications using that approach.

In the current Example, a general strategy for protein delivery that makes use of anionic protein complexation with cationic liposomes is demonstrated. This method was used to deliver diverse protein classes, including the Cre tyrosine recombinase, TALE transcriptional activators, and Cas9 nucleases, nickases, and transcriptional activators (Figure 27(A)) to cultured cell lines, stem cell colonies, and therapeutically relevant *in vivo* sites within the mouse inner ear. The described approach is highly efficient, producing modification rates on par with established nucleic acid transfection methods in cell culture, and enabling Cre recombinase and Cas9-mediated genome modification rates of up to 90% and 20%, respectively, within the inner ear hair cell population of live mice (Figures 32(C)-(D)). For Cas9 nuclease delivery, this approach also typically results in >10-fold more specific genome modification than traditional plasmid transfection (Figures 32(B-D)), likely due to the transient window of Cas9 activity to which each genome is exposed (Figure 47) compared to DNA delivery methods, consistent with previous reports.⁶¹ These results also suggest that it may be possible to use cationic lipids to efficiently deliver other nucleic acid-binding proteins, including transcription factors that induce therapeutically relevant changes in cell fate, by complexing them with nucleic acids.

Cationic lipid-based anionic protein delivery outperforms a potent cationic protein delivery fusion partner, (+36)GFP, by up to 9,800-fold per amount of endocytosed protein, inducing more efficient modification of treated cells with orders of magnitude lower doses of protein (Figures 28(C) 34). For Cas9 nuclease delivery, this approach also results in >10-fold more specific genome modification than traditional plasmid transfection (Figures 31(B)-(D)), likely due to the transient window of Cas9 activity to which each genome is exposed compared to DNA delivery methods, consistent with previous reports.⁵¹

The described approach is simple to implement, requiring only the purified deliverable protein and the use of popular commercial nucleic acid transfection reagents (Figure 27(B)). Rendering a given protein amenable to this approach requires simple translational fusion to a highly anionic partner, such as (-30)GFP (Figure 27(A)), and is even effective with common translational fusion tags including the VP64 activation domain, and the 3xFLAG affinity tag (Figure 28(F) and Figure 33(C)). In certain cases, as with the Cas9 protein, pre-complexation with a cognate nucleic acid (sgRNA in this case) is sufficient (Figure 30(A)), as the partially exposed bound nucleic acid likely provides sufficient anionic charge to mediate complexation with cationic lipids.

Others groups have reported the *in vivo* delivery of Cas9 expression constructs in DNA or mRNA form.^{52,53} The present Example demonstrates that protein delivery is a viable approach to *in vivo* genome editing. These results also demonstrate that cationic lipids can efficiently deliver other proteins *in vitro* and *in vivo,* including natively anionic proteins or proteins that can be fused or bound to polyanionic macromolecules.

### Table 1.

**Table 1. On-target and known off-target substrates of Cas9: sgRNAs that target sites in EMX, VEGF, and CLTA. A list of genomic on-target and off-targets sites of the EMX, VEGF, and CLTA are shown with mutations from the on-target sequence shown in lower case and bold. PAMs are shown in underline.**

| | |
|---|---|
| | |
| EMX_On | GAGTCCGAGCAGAAGAAGAAGGG (SEQ ID NO:209) |
| EMX_Off1 | GAG**g**CCGAGCAGAAGAA**ag**ACGG (SEQ ID NO:210) |
| EMX_Off2 | GAGTCCtAGCAG**g**AGAAGAAGaG (SEQ ID NO:211) |
| EMX_Off3 | GAGTC**ta**AGCAGAAGAAGAAG**a**G (SEQ ID NO:212) |
| EMX_Off4 | GAGT**ta**GAGCAGAAGAAGAAAGG (SEQ ID NO:213) |
| | |
| VEGF On | GGGTGGGGGGAGTTTGCTCCTGG (SEQ ID NO:214) |
| VEGF_Off1 | GG**a**TGG**a**GGGAGTTTGCTCCTGG (SEQ ID NO:215) |
| VEGF_Off2 | GGG**a**GGG**t**GGAGTTTGCTCCTGG (SEQ ID NO:216) |
| VEGF_Off3 | **c**GG**g**GG**a**GGGAGTTTGCTCCTGG (SEQ ID NO:217) |
| VEGF_Off4 | GGG**ga**GGGG**a**AGTTTGCTCCTGG (SEQ ID NO:218) |
| | |
| CLTA_On | GCAGATGTAGTGTTTCCACAGGG (SEQ ID NO:219) |
| CLTA_Off1 | **a**CA**a**ATGTAGT**a**TTTCCACAGGG (SEQ ID NO:220) |
| CLTA_Off2 | **c**CAGATGTAGT**a**TT**c**CCACAGGG (SEQ ID NO:221) |
| CLTA_Off3 | **ct**AGATG**a**AGTG**c**TTCCACATGG (SEQ ID NO:222) |

### Table 2.

**Table 2. Indel frequencies, P values, and on-target: off-target cleavage specificity ratios for EMX, CLTA, and VEGF on-target sites and 11 known off-target sites. CLTA sites: Total: total number of sequence counts; only the first 10,000 sequences were analyzed for the on-target site sequences. Modified: number of indels divided by total number of sequences as percentages. Upper limits of potential modification were calculated for sites with no observed indels by assuming there is less than one indel then dividing by the total sequence count to arrive at an upper limit modification percentage, or taking the theoretical limit of detection (1/49,500; see Results above), whichever value was larger. P-values: for mock treatment, Cas9 plasmid transfection, and liposomal Cas9 protein: sgRNA delivery, P-values were calculated as using a two-sided Fisher's exact test between each CLTA-targeted treatment sample (either DNA transfection or protein: sgRNA delivery) versus the control sample (mock treatment) treated with Cas9 protein and an sgRNA targeting EGFP. On: off specificity is the ratio of on-target to off-target genomic modification frequency for each site. EMX sites shows the experimental and analytic methods of CLTA analysis applied to EMX target sites. VEGF sites shows the experimental and analytic methods of CLTA analysis as applied to VEGF target sites. Indel numbers in the mock treatment control were subtracted from both plasmid transfection and protein:sgRNA delivery indel numbers for determining total number of indels and for calculating on-target off-target ratios in Figure 31 in the main text and also for Figure 41.**

| CLTA Site | **Mock treatment** | **Plasmid transfection** | **Protein: sgRNA delivery** |
|---|---|---|---|
| CLTA_On | | | |
| Indels | 14 | 1228 | 1498 |
| Total | 10000 | 10000 | 10000 |
| Modified (%) | 0.140 | 12.280 | 14.980 |
| P-value | | <1.0E-300 | <1.0E-300 |
| On:off specificity | 1 | 1 | 1 |
| CLTA_Off1 | | | |
| Indels | 7 | 29 | 14 |
| Total | 41518 | 205204 | 125370 |
| Modified (%) | 0.017 | 0.014 | 0.011 |
| P-value | | 6.6E-01 | 4.5E-01 |
| On:off specificity | | 869 | 1341 |
| CLTA_Off2 | | | |
| Indels | 5 | 11 | 8 |
| Total | 25338 | 83944 | 54409 |
| Modified (%) | 0.020 | 0.013 | 0.015 |
| P-value | | 5.5E-01 | 5.7E-01 |
| On: off specificity | | 937 | 1019 |
| CLTA_Off3 | | | |
| Indels | 6 | 22 | 8 |
| Total | 41643 | 189886 | 76863 |
| Modified (%) | 0.014 | 0.012 | 0.010 |
| P-value | | 6.2E-01 | 5.8E-01 |
| On: off specificity | | 1060 | 1439 |

| EMX Sites | **Mock treatment** | **Plasmid transfection** | **Protein: sgRNA delivery** |
|---|---|---|---|
| EMX_On | | | |
| Indels | 3 | 930 | 1140 |
| Total | 10000 | 10000 | 10000 |
| Modified (%) | | 0.030 | 9.300 |
| P-value | | 1.6E-264 | <1.0E-300 |
| On:off specificity | 1 | 1 | 1 |
| EMX_Off1 | | | |
| Indels | 0 | 6 | 6 |
| Total | 24623 | 90935 | 100778 |
| Modified (%) | | <0.002 | 0.007 |
| P-value | | 3.5E-01 | 6.1E-01 |
| On:off specificity | | 1409 | 1915 |
| EMX_Off2 | | | |
| Indels | 16 | 53 | 38 |
| Total | 36061 | 204068 | 130084 |
| Modified (%) | | 0.044 | 0.026 |
| P-value | | 6.4E-02 | 1.8E-01 |
| On:off specificity | | 358 | 390 |
| EMX_Off3 | | | |
| Indels | 20 | 147 | 44 |
| Total | 32575 | 157848 | 110878 |
| Modified (%) | | 0.061 | 0.093 |
| P-value | | 8.1E-02 | 1.3E-01 |
| On:off specificity | | 100 | 287 |
| EMX_Off4 | | | |
| Indels | 16 | 141 | 23 |
| Total | 45548 | 86586 | 73451 |
| Modified (%) | | 0.035 | 0.163 |
| P-value | | 2.8E-12 | 7.4E-01 |
| On:off specificity | | 57 | 364 |

| VEGF Sites | **Mock treatment** | **Plasmid transfection** | **Protein:sgRNA delivery** |
|---|---|---|---|
| VEGF_On | | | |
| Indels | 1 | 989 | 785 |
| Total | | 10000 | 10000 |
| Modified (%) | 0.010 | 9.890 | 7.850 |
| P-value | | 1.5E-285 | 5.7E-228 |
| On:off specificity | 1 | 1 | 1 |
| VEGF_Off1 | | | |
| Indels | 4 | 4240 | 602 |
| Total | | 38625 | 184554 |
| Modified (%) | 0.010 | 2.297 | 0.394 |
| P-value | | <1.0E-300 | 3.7E-52 |
| On:off specificity | | 4 | 20 |
| VEGF_Off2 | | | |
| Indels | 5 | 727 | 18 |
| Total | | 30301 | 79164 |
| Modified (%) | 0.017 | 0.918 | <0.002 |
| P-value | | 4.7E-93 | 1.3E-04 |
| On:off specificity | | 11 | 3925 |
| VEGF_Off3 | | | |
| Indels | 2 | 536 | 21 |
| Total | | 26379 | 110902 |
| Modified (%) | 0.008 | 0.483 | 0.022 |
| P-value | | 2.0E-46 | 2.0E-01 |
| On:off specificity | | 20 | 352 |
| VEGF_Off4 | | | |
| Indels | 0 | 1531 | 45 |
| Total | | 26012 | 122403 |

**Table 3. Exemplary naturally occurring negatively supercharged proteins. Proteins listed have a negative net charge of -10 or less. For each protein, a unique Uniprot identifier is provided in bold. In parentheses, an exemplary name of the gene encoding the respective protein as well as its molecular weight, charge, and molecular weight charge ratio are provided.**

| | | |
|---|---|---|
| **Q8WXI7** (MUC16;2,353,-426,-0.18) | **Q8N4C6** (NIN;243,-138,-0.56) | **Q8NI35** (INADL;196,-97,-0.49) |
| **Q8WXG9** (GPR98;693,-412,-0.59) | **Q13023** (AKAP6;257,-137,-0.53) | **Q5TI25** (NBPF14;106,-97,-0.91) |
| **Q8WUY3** (PRUNE2;341,-348,-1.02) | **P35556** (FBN2;315,-137,-0.43) | **Q8TCU4** (ALMS1;461,-96,-0.20) |
| **Q8WZ42** (TTN;3,816,-344,-0.09) | **Q5JRA6** (MIA3;214,-136,-0.63) | **Q86TB3** (ALPK2;237,-95,-0.40) |
| **P13611** (VCAN;373,-322,-0.86) | **Q15413** (RYR3;552,-135,-0.24) | **Q9Y4E1** (FAM21C;145,-95,-0.65) |
| **Q03001** (DST;861,-316,-0.36) | **Q75N90** (FBN3;300,-134,-0.44) | **O43164** (PJA2;78,-95,-1.21) |
| **Q8NF91** (SYNE1;1,011,-315,-0.31) | **O60494** (CUBN;399,-133,-0.33) | **Q9P1V8** (SAMD15;77,-95,-1.23) |
| **Q8N3K9** (CMYA5;449,-312,-0.69) | **P35555** (FBN1;312,-132,-0.42) | **Q92628** (KIAA0232;155,-95,-0.61) |
| **Q8IWN7** (RP1L1;261,-278,-1.06) | **Q70CQ2** (USP34;404,-131,-0.32) | **O75970** (MPDZ;222,-94,-0.42) |
| **Q8WXH0** (SYNE2;796,-271,-0.34) | **Q8NDA2** (HMCN2;543,-128,-0.23) | **Q7Z3T8** (ZFYVE16;169,-94,-0.55) |
| **P16112** (ACAN;250,-269,-1.07) | **P12270** (TPR;267,-127,-0.47) | **Q641Q2** (FAM21A; 147,-94,-0.63) |
| **Q9NZW4** (DSPP;131,-266,-2.02) | **Q3T8J9** (GON4L;249,-126,-0.50) | **P78509** (RELN;388,-94,-0.24) |
| **Q9UPN3** (MACF1;838,-263,-0.31) | **P46531** (NOTCH1;273,-125, -0.45) | **Q8IWT3** (CUL9;281,-93,-0.33) |
| **Q14517** (FAT1;506,-256,-0.50) | **A2VEC9** (SSPO;548,-125,-0.22) | **Q9NYC9** (DNAH9;512,-93,-0.18) |
| **Q685J3** (MUC17;452,-255,-0.56) | **Q8IZL8** (PELP1;120,-122,-1.01) | **Q96ST2** (IWS1;92,-93,-1.01) |
| **P98164** (LRP2;522,-243,-0.46) | **043719** (HTATSF1;86,-122,-1.42) | **P21675** (TAF1;213,-93,-0.43) |
| **Q8TDW7** (FAT3;506,-237,-0.46) | **Q92736** (RYR2;565,-119,-0.21) | **Q5VU43** (PDE4DIP;265,-92,-0.34) |
| **Q99996** (AKAP9;454,-233,-0.51) | **Q12802** (AKAP13;308,-118,-0.38) | **Q9P2P6** (STARD9;516,-92,-0.17) |
| **Q6V0I7** (FAT4;543,-230,-0.42) | **Q9NR09** (BIRC6;530,-118,-0.22) | **Q01082** (SPTBN1;275,-90,-0.32) |
| **Q02952** (AKAP12;191,-226,-1.18) | **P11137** (MAP2;200,-118,-0.59) | **Q15643** (TRIP11;228,-90,-0.39) |
| **Q9UKN1** (MUC12;558,-226,-0.40) | **Q04721** (NOTCH2;265,-117,-0.44) | **Q6ULP2** (AFTPH;102,-89,-0.87) |
| **Q9H251** (CDH23;369,-223,-0.60) | **P49321** (NASP;85,-115,-1.34) | **Q9BYE9** (CDHR2;142,-89,-0.62) |
| **Q01484** (ANK2;434,-212,-0.48) | **Q7Z5P9** (MUC19;598,-115,-0.19) | **Q9UGM3** (DMBT1;261,-89,-0.34) |
| **P21817** (RYR1;565,-207,-0.36) | **A6H8Y1** (BDP1;294,-114,-0.38) | **Q8IVF4** (DNAH10;515,-89,-0.17) |
| **Q14789** (GOLGB1;376,-205,-0.54) | **Q9H0E9** (BRD8;135,-114,-0.84) | **P04275** (VWF;309,-89,-0.28) |
| **Q6V1P9** (DCHS2;322,-202,-0.62) | **P24821** (TNC;241,-114,-0.47) | **P11532** (DMD;427,-89,-0.20) |
| **Q9NYQ8** (FAT2;479,-199,-0.41) | **B4DH59** (NBPF26;104,-114,-1.09) | **Q86WI1** (PKHD1L1;466,-89,-0.19) |
| **P48681** (NES;177,-199,-1.12) | **P58107** (EPPK1;556,-113,-0.20) | **Q9UFH2** (DNAH17;512,-88,-0.17) |
| **Q9NZR2** (LRP1B;515,-196,-0.38) | **Q5H9T9** (FSCB;88,-113,-1.28) | **Q13387** (MAPK8IP2;88,-88,-1.00) |
| **Q9Y6R7** (FCGBP;572,-184,-0.32) | **Q4G0P3** (HYDIN;576,-113,-0.19) | **O60732** (MAGEC1;124,-88,-0.71) |
| **O95359** (TACC2;309,-183,-0.59) | **Q13813** (SPTAN1;285,-113,-0.39) | **Q96NY7** (CLIC6;73,-87,-1.19) |
| **Q9NU22** (MDN1;633,-180,-0.28) | **Q96JN2** (CCDC136;134,-112,-0.83) | **Q5T1H1** (EYS;351,-87,-0.24) |
| **Q07954** (LRP1;505,-178,-0.35) | **Q7KZ85** (SUPT6H;199,-112,-0.56) | **Q63HN8** (RNF213;591,-87,-0.14) |
| **Q7Z6Z7** (HUWE1;482,-173,-0.35) | **Q5RHP9** (ERICH3;168,-111,-0.65) | **Q5SNT6** (FAM21 B;137,-87,-0.63) |
| **Q5VST9** (OBSCN;868,-173,-0.19) | **P08519** (LPA;501,-111,-0.22) | **Q13439** (GOLGA4;261,-87,-0.33) |
| **P78559** (MAP1A;305,-170,-0.55) | **Q15751** (HERC1;532,-111,-0.20) | **Q8IWJ2** (GCC2;196,-86,-0.43) |
| **P46821** (MAP1B;271,-169,-0.62) | **Q9HAW4** (CLSPN;151,-110,-0.72) | **Q09666** (AHNAK;629,-85,-0.13) |
| **P49454** (CENPF;368,-169,-0.45) | **Q86TD4** (SRL;101,-109,-1.08) | **Q6VMQ6** (ATF7IP;136,-85,-0.62) |
| **Q86XX4** (FRAS1;443,-168,-0.37) | **P11277** (SPTB;246,-107,-0.43) | **Q01538** (MYT1;122,-85,-0.69) |
| **Q8NFC6** (BOD1L1;330,-162, -0.49) | **Q5TCS8** (AK9;221,-106,-0.47) | **Q9C0C2** (TNKS1 BP1; 182,-85,-0.46) |
| **Q96JQ0** (DCHS1;346,-162,-0.46) | **Q9NQC3** (RTN4;130,-106,-0.81) | **Q86TY3** (C14orf37;84,-85,-1.00) |
| **O94854** (KIAA0754; 135,-162,-1.19) | **Q5JY77** (GPRASP1;157,-105,-0.66) | **Q8NG31** (CASC5;265,-84,-0.31) |
| **E2RYF6** (MUC22;173,-161,-0.92) | **Q9UPS6** (SETD1B;213,-104,-0.48) | **O00461** (GOLIM4;82,-83,-1.01) |
| **O15069** (NACAD;161,-160,-0.99) | **Q4LDE5** (SVEP1;390,-104,-0.26) | **O75096** (LRP4;212,-83,-0.39) |
| **Q5SZK8** (FREM2;351,-158,-0.44) | **Q8WVC0** (LEO1;75,-103,-1.36) | **Q15149** (PLEC;532,-83,-0.15) |
| **Q8IVF2** (AHNAK2;617,-157,-0.25) | **Q15154** (PCM1;229,-103,-0.45) | **P98160** (HSPG2;469,-83,-0.17) |
| **P23327** (HRC;80,-157,-1.95) | **Q9HCU4** (CELSR2;317,-102,-0.32) | **O95714** (HERC2;527,-83,-0.15) |
| **Q8IWU2** (LMTK2;165,-156,-0.94) | **Q5T4S7** (UBR4;574,-102,-0.17) | **Q7Z407** (CSMD3;406,-82,-0.20) |
| **P02549** (SPTA1;280,-153,-0.54) | **094915** (FRYL;340,-101,-0.29) | **Q6UVK1** (CSPG4;251,-82,-0.32) |
| **O14686** (KMT2D;593,-148,-0.24) | **Q13316** (DMP1;56,-101,-1.81) | **Q9BQS8** (FYCO1;167,-82,-0.49) |
| **Q12888** (TP53BP1;214,-148,-0.69) | **Q9BZV3** (IMPG2;139,-100,-0.72) | **Q9ULT8** (HECTD1;289,-82,-0.28) |
| **Q9BV73** (CEP250;281,-144,-0.51) | **P0C091** (FREM3;238,-100,-0.41) | **P50851** (LRBA;319,-82,-0.25) |
| **P22105** (TNXB;464,-144,-0.31) | **Q6ZMQ8** (AATK;145,-98,-0.67) | **Q3BBV2** (NBPF8;99,-82,-0.82) |
| **O95613** (PCNT;378,-142,-0.37) | **Q69YN4** (KIAA1429;202,-98,-0.48) | **Q9GZU2** (PEG3;181,-82,-0.45) |
| **P46939** (UTRN;394,-142,-0.35) | **Q3BBV0** (NBPF1;139,-98,-0.70) | **Q96PZ7** (CSMD1;389,-81,-0.20) |
| **P23471** (PTPRZ1;255,-139,-0.54) | **Q3ZCN5** (OTOGL;262,-98,-0.37) | **Q5TD94** (RSPH4A;81,-81,-1.00) |
| **Q0VD83** (APOBR;115,-138,-1.20) | **P07942** (LAMB1;198,-98,-0.49) | **Q9H0K4** (RSPH6A;81,-81,-1.00) |
| **Q96QF7** (ACRC;76,-81,-1.06) | **Q9NZ53** (PODXL2;65,-73,-1.12) | **Q2M3C7** (SPHKAP;186,-66,-0.35) |
| **Q3BBV1** (NBPF20;109,-81,-0.74) | **O15061** (SYNM;173,-73,-0.42) | **A6NDB9** (PALM3;72,-66,-0.92) |
| **Q2M2H8** (0;278,-81,-0.29) | **Q5RGN0** (NBPF24;90,-73,-0.80) | **Q96R06** (SPAG5;134,-66,-0.49) |
| **Q02224** (CENPE;316,-81,-0.25) | **Q7Z6I6** (ARHGAP30;119,-73,-0.61) | **Q5UIP0** (RIF1;274,-66,-0.24) |
| **Q14028** (CNGB1;140,-80,-0.57) | **Q9C0D2** (KIAA1731;295,-73,-0.24) | **Q9Y485** (DMXL1;338,-65,-0.19) |
| **Q6ZNL6** (FGD5;160,-80,-0.50) | **Q9Y2F5** (ICE1;248,-72,-0.29) | **Q96RW7** (HMCN1;613,-65,-0.10) |
| **Q9Y2I1** (NISCH;167,-80,-0.48) | **Q8N108** (MIER1;58,-72,-1.24) | **Q8NFP9** (NBEA;328,-65,-0.19) |
| **Q9Y4B6** (VPRBP;169,-80,-0.47) | **A6NKG5** (RTL1;155,-72,-0.46) | **O43157** (PLXNB1;232,-65,-0.27) |
| **Q9BTT0** (ANP32E;31,-80,-2.60) | **Q93008** (USP9X;292,-72,-0.24) | **Q96S38** (RPS6KC1;119,-65,-0.54) |
| **Q8N3D4** (EHBP1L1;162,-80,-0.49) | **O00507** (USP9Y;291,-72,-0.24) | **O15027** (SEC16A;234,-65,-0.27) |
| **Q9UM47** (NOTCH3;244,-80,-0.32) | **A5YM69** (ARHGEF35;53,-71,-1.33) | **O15047** (SETD1A; 186,-65,-0.34) |
| **Q86UQ4** (ABCA13;576,-80,-0.13) | **P31415** (CASQ1;45,-71,-1.57) | **P49747** (COMP;83,-65,-0.78) |
| **Q7Z408** (CSMD2;380,-79,-0.20) | **Q96JQ2** (CLMN;112,-71,-0.63) | **P82279** (CRB1;154,-65,-0.42) |
| **Q32MZ4** (LRRFIP1;89,-79,-0.88) | **Q96QU1** (PCDH15;216,-71,-0.32) | **O75962** (TRIO;347,-65,-0.18) |
| **Q96T23** (RSF1;164,-79,-0.48) | **Q96JI7** (SPG11;279,-71,-0.25) | **Q5CZC0** (FSIP2;781,-65,-0.08) |
| **P35442** (THBS2;130,-79,-0.60) | **P38398** (BRCA1;208,-70,-0.33) | **Q06481** (APLP2;87,-64,-0.73) |
| **Q9UKA4** (AKAP11;211,-78,-0.37) | **O95153** (BZRAP1;200,-70,-0.34) | **P05067** (APP;87,-64,-0.73) |
| **Q5JTC6** (AMER1;124,-78,-0.62) | **Q9Y222** (DMTF1;84,-70,-0.82) | **Q96GW7** (BCAN;99,-64,-0.64) |
| **Q9P2D7** (DNAH1;494,-78,-0.15) | **Q5TBA9** (FRY;339,-70,-0.20) | **O75369** (FLNB;278,-64,-0.23) |
| **O60229** (KALRN;340,-78,-0.22) | **Q96Q04** (LMTK3;154,-70,-0.45) | **P42858** (HTT;348,-64,-0.18) |
| **Q9H094** (NBPF3;73,-78,-1.06) | **Q14160** (SCRIB;175,-70,-0.40) | **P11047** (LAMC1;178,-64,-0.36) |
| **Q9Y2I6** (NINL;156,-78,-0.49) | **Q8IWB6** (TEX14;168,-70,-0.41) | **O75147** (OBSL1;207,-64,-0.30) |
| **Q9UPN7** (PPP6R1;97,-78,-0.80) | **Q96PC5** (MIA2;62,-70,-1.13) | **O75807** (PPP1R15A;73,-64,-0.87) |
| **Q5H9R7** (PPP6R3;98,-78,-0.79) | **Q14112** (NID2;151,-70,-0.46) | **Q9BYH1** (SEZ6L;112,-64,-0.57) |
| **Q6P3W6** (NBPF10;96,-78,-0.80) | **Q9BZQ8** (FAM129A;103,-69,-0.66) | **Q9H254** (SPTBN4;289,-64,-0.22) |
| **Q14767** (LTBP2;195,-77,-0.39) | **Q5H8C1** (FREM1;244,-69,-0.28) | **Q7Z3K6** (MIER3;61,-64,-1.04) |
| **Q9UL68** (MYT1L;133,-77,-0.57) | **Q2LD37** (KIAA1109;555,-69,-0.12) | **P07199** (CENPB;65,-64,-0.98) |
| **Q9BXP8** (PAPPA2;199,-77,-0.38) | **A6NMS7** (LRRC37A;188,-69,-0.36) | **Q6ZMV5** (SMEK3P;96,-64,-0.66) |
| **O75154** (RAB1 1 FIP3;82,-77,-0.93) | **P07197** (NEFM;102,-69,-0.67) | **A6NFI3** (ZNF316;108,-64,-0.59) |
| **Q5T011** (SZT2;378,-77,-0.20) | **Q92834** (RPGR;113,-69,-0.60) | **Q86UP3** (ZFHX4;394,-64,-0.16) |
| **O43149** (ZZEF1;331,-77,-0.23) | **Q92673** (SORL1;248,-69,-0.27) | **Q12955** (ANK3;480,-64,-0.13) |
| **Q5QJ38** (TCHHL1;99,-76,-0.76) | **P37275** (ZEB1;124,-69,-0.55) | **Q9NYF5** (FAM13B;105,-63,-0.60) |
| **Q96DX7** (TRIM44;38,-76,-1.97) | **Q14515** (SPARCL1;75,-69,-0.91) | **O00410** (IPO5;124,-63,-0.50) |
| **P07476** (IVL;68,-76,-1.10) | **Q86T75** (NBPF11;99,-69,-0.69) | **Q99466** (NOTCH4;210,-63,-0.30) |
| **P98095** (FBLN2;127,-76,-0.60) | **Q96K76** (USP47;157,-69,-0.43) | **P35443** (THBS4;106,-63,-0.59) |
| **Q8IWZ3** (ANKHD1;269,-75,-0.27) | **A6NM11** (LRRC37A2;188,-69,-0.36) | **Q14DG7** (TMEM132B;119,-63,-0.52) |
| **O94985** (CLSTN1;110,-75,-0.68) | **O95405** (ZFYVE9;156,-68,-0.43) | **Q9BXT5** (TEX15;315,-63,-0.19) |
| **Q7Z7A1** (CNTRL;269,-75,-0.27) | **Q3BBW0** (NBPF9;100,-68,-0.68) | **Q96DT5** (DNAH11;521,-63,-0.12) |
| **Q16643** (DBN1;71,-75,-1.04) | **O43847** (NRD1;132,-68,-0.51) | **P39687** (ANP32A;29,-62,-2.16) |
| **O15355** (PPM1G;59,-75,-1.26) | **Q92688** (ANP32B;29,-67,-2.32) | **Q4LE39** (ARID4B;148,-62,-0.41) |
| **Q9H2G4** (TSPYL2;79,-75,-0.94) | **Q96BY7** (ATG2B;233,-67,-0.28) | **Q8NEP3** (DNAAF1;80,-62,-0.77) |
| **Q6PJW8** (CNST;80,-74,-0.92) | **Q9NYQ6** (CELSR1;329,-67,-0.20) | **Q76N89** (HECW1;180,-62,-0.34) |
| **O60309** (LRRC37A3;181,-74,-0.40) | **Q8TE73** (DNAH5;529,-67,-0.12) | **Q9P2P5** (HECW2;176,-62,-0.35) |
| **Q02817** (MUC2;540,-74,-0.13) | **Q9C0G6** (DNAH6;476,-67,-0.14) | **Q96P70** (IPO9;116,-62,-0.53) |
| **Q86UW6** (N4BP2;199,-74,-0.37) | **Q96M86** (DNHD1;534,-67,-0.12) | **Q14643** (ITPR1;314,-62,-0.19) |
| **Q6ZRI0** (OTOG;315,-74,-0.23) | **P42566** (EPS15;99,-67,-0.67) | **Q8NEZ4** (KMT2C;541,-62,-0.11) |
| **Q96RG2** (PASK;143,-74,-0.51) | **Q9Y4D8** (HECTD4;439,-67,-0.15) | **A2RRP1** (NBAS;269,-62,-0.23) |
| **P49746** (THBS3;104,-74,-0.71) | **Q16821** (PPP1R3A; 126,-67,-0.53) | **O60271** (SPAG9;146,-62,-0.42) |
| **Q15911** (ZFHX3;404,-74,-0.18) | **Q8TF05** (PPP4R1;107,-67,-0.62) | **Q9UPW8** (UNC13A;193,-62,-0.32) |
| **H0YM25** (GOLGA6L22;108,-74,-0.68) | **Q76I76** (SSH2;158,-67,-0.42) | **Q9HCK1** (ZDBF2;266,-62,-0.23) |
| **Q92752** (TNR;150,-74,-0.49) | **P07996** (THBS1;129,-67,-0.51) | **P27824** (CANX;68,-62,-0.91) |
| **O60522** (TDRD6;237,-74,-0.31) | **Q9C0A1** (ZFHX2;274,-67,-0.24) | **A8MZA4** (GOLGA6L6;94,-62,-0.65) |
| **A6NC98** (CCDC88B;165,-73,-0.44) | **Q05DH4** (FAM160A1;117,-67,-0.57) | **Q8TF21** (ANKRD24;124,-61,-0.49) |
| **Q8TDJ6** (DMXL2;340,-73,-0.21) | **Q8IZX4** (TAF1L;207,-67,-0.32) | **P29374** (ARID4A;143,-61 ,-0.42) |
| **Q8WXX0** (DNAH7;461,-73,-0.15) | **Q562E7** (WDR81;212,-67,-0.31) | **Q96SN8** (CDK5RAP2;215,-61,-0.28) |
| **Q86SJ6** (DSG4;114,-73,-0.64) | **Q9UPV0** (CEP164;164,-66,-0.40) | **Q8IWE5** (PLEKHM2;113,-61,-0.54) |
| **O95373** (IPO7;120,-73,-0.61) | **P14314** (PRKCSH;59,-66,-1.11) | **Q8TDY2** (RB1CC1;183,-61,-0.33) |
| **P82094** (TMF1;123,-61,-0.49) | **Q53GL7** (PARP10;110,-57,-0.51) | **Q81UX7** (AEBP1;131,-53,-0.40) |
| **Q8N660** (NBPF15;78,-61,-0.78) | **P20908** (COL5A1;184,-57,-0.31) | **P05060** (CHGB;78,-53,-0.67) |
| **Q5SXJ2** (NBPF16;78,-61,-0.78) | **Q8IVV2** (LOXHD1;222,-57,-0.25) | **Q99715** (COL12A1;333,-53,-0.15) |
| **P35499** (SCN4A;208,-61,-0.29) | **P35580** (MYH10;229,-57,-0.24) | **Q49AJ0** (FAM135B;156,-53,-0.34) |
| **P16157** (ANK1;206,-60,-0.29) | **P12259** (F5;252,-57,-0.22) | **Q8NBR6** (FAM63B;67,-53,-0.78) |
| **P0C7V8** (DCAF8L2;71,-60,-0.84) | **Q96JB1** (DNAH8;515,-57,-0.11) | **Q58FF7** (HSP90AB3P;68,-53,-0.77) |
| **Q9Y6X4** (FAM169A;75,-60,-0.80) | **P49418** (AMPH;76,-56,-0.73) | **P21815** (IBSP;35,-53,-1.50) |
| **A1L4K1** (FSD2;85,-60,-0.70) | **Q12774** (ARHGEF5;177,-56,-0.31) | **Q92794** (KAT6A;225,-53,-0.23) |
| **Q9ULW6** (NAP1L2;53,-60,-1.14) | **Q8WYN3** (CSRNP3;65,-56,-0.86) | **O60333** (KIF1B;204,-53,-0.25) |
| **O75170** (PPP6R2;105,-60,-0.57) | **Q6XZF7** (DNMBP;177,-56,-0.31) | **Q14596** (NBR1;107,-53,-0.49) |
| **Q9NWF9** (RNF216;99,-60,-0.60) | **Q15029** (EFTUD2;109,-56,-0.51) | **Q9P2E7** (PCDH10;113,-53,-0.46) |
| **Q9H2G2** (SLK;143,-60,-0.42) | **P02751** (FN1;263,-56,-0.21) | **Q7Z442** (PKD1L2;273,-53,-0.19) |
| **O95071** (UBR5;309,-60,-0.19) | **Q14C86** (GAPVD1;165,-56,-0.33) | **Q86YN6** (PPARGC1B;113,-53,-0.46) |
| **P19338** (NCL;77,-60,-0.78) | **P25391** (LAMA1;337,-56,-0.16) | **Q6GYQ0** (RALGAPA1;230,-53,-0.23) |
| **Q96M32** (AK7;83,-60,-0.72) | **Q99698** (LYST;429,-56,-0.13) | **O15020** (SPTBN2;271,-53,-0.19) |
| **Q5VXU9** (C9orf84; 165,-60,-0.36) | **O14594** (NCAN;143,-56,-0.39) | **Q6ZRS2** (SRCAP;344,-53,-0.15) |
| **Q02410** (APBA1;93,-59,-0.63) | **Q81UM7** (NPAS4;87,-56,-0.64) | **Q01105** (SET;33,-53,-1.58) |
| **P27797** (CALR;48,-59,-1.22) | **O95197** (RTN3;113,-56,-0.49) | **Q5SWA1** (PPP1R15B;79,-53,-0.66) |
| **P22223** (CDH3;91,-59,-0.64) | **Q9C093** (SPEF2;210,-56,-0.26) | **P01266** (TG;305,-53,-0.17) |
| **Q9P225** (DNAH2;508,-59,-0.11) | **Q9C0C9** (UBE2O;141,-56,-0.39) | **Q9NZN5** (ARHGEF12;173,-52,-0.30) |
| **Q9P2D6** (FAM135A;170,-59,-0.34) | **Q9C0G0** (ZNF407;247,-56,-0.22) | **Q4G0X9** (CCDC40;130,-52,-0.39) |
| **Q5VWN6** (FAM208B;269,-59,-0.21) | **P98155** (VLDLR;96,-56,-0.58) | **Q9NR16** (CD163L1;159,-52,-0.32) |
| **Q5SYB0** (FRMPD1;173,-59,-0.34) | **O60518** (RANBP6; 125,-56,-0.44) | **Q9BZQ6** (EDEM3;105,-52,-0.49) |
| **Q9UBN7** (HDAC6;131,-59,-0.44) | **P39059** (COL15A1;142,-56,-0.39) | **Q6PRD1** (GPR179;257,-52,-0.20) |
| **Q92824** (PCSK5;207,-59,-0.28) | **O75443** (TECTA;240,-56,-0.23) | **Q9H1H9** (KIF13A;202,-52,-0.25) |
| **Q96EB6** (SIRT1;82,-59,-0.72) | **O14525** (ASTN1;145,-55,-0.37) | **Q9Y4W2** (LAS1L;83,-52,-0.62) |
| **Q9UBS9** (SUCO;139,-59,-0.42) | **P14625** (HSP90B1;92,-55,-0.59) | **Q14114** (LRP8;106,-52,-0.49) |
| **Q6ZS81** (WDFY4;354,-59,-0.16) | **Q14676** (MDC1;227,-55,-0.24) | **O00566** (MPHOSPH10;79,-52,-0.65) |
| **O14958** (CASQ2;46,-59,-1.27) | **Q7RTP6** (MICAL3;224,-55,-0.24) | **Q9HC84** (MUC5B;596,-52,-0.08) |
| **Q96MT7** (WDR52;112,-59,-0.52) | **Q6PFW1** (PPIP5K1;160,-55,-0.34) | **Q9UKK3** (PARP4;193,-52,-0.26) |
| **P10745** (RBP3;135,-59,-0.43) | **O60437** (PPL;205,-55,-0.26) | **O60721** (SLC24A1;121,-52,-0.42) |
| **Q2TAZ0** (ATG2A;213,-58,-0.27) | **Q9BXT8** (RNF17;185,-55,-0.29) | **Q9P2D8** (UNC79;295,-52,-0.17) |
| **Q96KC9** (CABS1;43,-58,-1.34) | **P56715** (RP1;241,-55,-0.22) | **Q5THJ4** (VPS13D;492,-52,-0.10) |
| **A2RUR9** (CCDC144A;165,-58,-0.35) | **P0DME0** (SETSIP;35,-55,-1.57) | **Q9C0E2** (XPO4;130,-52,-0.39) |
| **O94986** (CEP152;196,-58,-0.29) | **A4UGR9** (XIRP2;382,-55,-0.14) | **O15085** (ARHGEF11;168,-51,-0.30) |
| **Q14315** (FLNC;291,-58,-0.19) | **Q99689** (FEZ1;45,-55,-1.21) | **Q9Y6J0** (CABIN1;246,-51,-0.20) |
| **Q8TEX9** (IPO4;119,-58,-0.48) | **Q8TBY9** (WDR66;130,-55,-0.42) | **Q9HBT6** (CDH20;89,-51,-0.57) |
| **Q5T7N2** (L1TD1;99,-58,-0.58) | **Q99767** (APBA2;83,-54,-0.65) | **P30622** (CLIP1;162,-51,-0.31) |
| **O75197** (LRP5;179,-58,-0.32) | **P12830** (CDH1;97,-54,-0.55) | **Q05707** (COL 14A 1; 194,-51 ,-0.26) |
| **Q9BZA7** (PCDH11X;148,-58,-0.39) | **Q9UJ99** (CDH22;89,-54,-0.60) | **O95196** (CSPG5;60,-51,-0.84) |
| **Q68CZ1** (RPGRIP1L;151,-58,-0.38) | **Q5VT06** (CEP350;351,-54,-0.15) | **P0C2W7** (CT47B1;31,-51,-1.62) |
| **Q7Z7G8** (VPS13B;449,-58,-0.12) | **P21333** (FLNA;281,-54,-0.19) | **Q9Y2J2** (EPB41L3;121,-51,-0.42) |
| **O75581** (LRP6;180,-58,-0.32) | **Q6ZWJ8** (KCP;160,-54,-0.33) | **Q14CM0** (FRMPD4;144,-51,-0.35) |
| **Q9P2G1** (ANKIB1;122,-57,-0.46) | **O43451** (MGAM;210,-54,-0.25) | **P54257** (HAP1;76,-51,-0.67) |
| **Q5TH69** (ARFGEF3;241,-57,-0.23) | **Q02505** (MUC3A;266,-54,-0.20) | **Q16659** (MAPK6;83,-51,-0.61) |
| **Q9Y5Q5** (CORIN;116,-57,-0.48) | **P55209** (NAP1L1;45,-54,-1.19) | **P35749** (MYH11;227,-51,-0.22) |
| **Q14204** (DYNC1 H1;532,-57,-0.10) | **Q14494** (NFE2L1;85,-54,-0.63) | **Q06190** (PPP2R3A; 130, -51, -0 .39) |
| **Q5W0A0** (ERICH6B;82,-57,-0.69) | **Q9Y6V0** (PCLO;553,-54,-0.09) | **Q9NRC6** (SPTBN5;417,-51,-0.12) |
| **Q8N3X1** (FNBP4;110,-57,-0.51) | **P50542** (PEX5;71,-54,-0.76) | **A6NEF3** (GOLGA6L4;68,-51,-0.75) |
| **Q92538** (GBF1;206,-57,-0.27) | **P08F94** (PKHD1;447,-54,-0.12) | **Q08379** (GOLGA2;113,-51,-0.45) |
| **Q9UHV7** (MED13;239,-57,-0.23) | **Q16799** (RTN1;84,-54,-0.64) | **O43423** (ANP32C;27,-50,-1.86) |
| **Q8N7H5** (PAF1;60,-57,-0.95) | **P28290** (SSFA2;138,-54,-0.39) | **Q8N1W1** (ARHGEF28;192,-50,-0.26) |
| **Q9BZA8** (PCDH11Y;147,-57,-0.38) | **O00267** (SUPT5H;121,-54,-0.44) | **O75309** (CDH16;90,-50,-0.55) |
| **Q9HCL0** (PCDH18;126,-57,-0.45) | **Q9Y4G6** (TLN2;272,-54,-0.19) | **Q14999** (CUL7;191,-50,-0.26) |
| **P09619** (PDGFRB;124,-57,-0.45) | **Q6ZSY5** (PPP1R3F;83,-54,-0.65) | **Q9UPY3** (DICER1;219,-50,-0.22) |
| **O60216** (RAD21;72,-57,-0.79) | **P55286** (CDH8;88,-54,-0.61) | **O75923** (DYSF;237,-50,-0.21) |
| **O14967** (CLGN;70,-57,-0.81) | **Q96QE4** (LRRC37B;106,-54,-0.51) | **Q8NC44** (FAM134A;58,-50,-0.86) |
| **Q5T7N3** (KANK4;107,-50,-0.46) | **P55287** (CDH11;88,-48,-0.54) | **Q5T9S5** (CCDC18; 169,-46,-0.27) |
| **Q9UPT6** (MAPK8IP3;147,-50,-0.33) | **P14410** (SI;209,-48,-0.22) | **P11055** (MYH3;224,-46,-0.20) |
| **Q8N344** (MIER2;60,-50,-0.83) | **Q14839** (CHD4;218,-48,-0.22) | **Q5TZA2** (CROCC;229,-45,-0.19) |
| **Q96PX1** (RNF157;74,-50,-0.67) | **Q9UPS8** (ANKRD26;196,-47,-0.23) | **A6NGE4** (DCAF8L1;67,-45,-0.66) |
| **Q96RS0** (TGS1;97,-50,-0.51) | **P19022** (CDH2;100,-47,-0.47) | **Q8TD57** (DNAH3;471,-45,-0.09) |
| **H0YKK7** (GOLGA6L19;64,-50,-0.77) | **O15078** (CEP290;290,-47,-0.16) | **Q6PGQ1** (DRICH1;25,-45,-1.79) |
| **Q96M43** (NBPF4;72,-50,-0.69) | **Q9P2D1** (CHD7;336,-47,-0.13) | **P11717** (IGF2R;274,-45,-0.16) |
| **Q5VWK0** (NBPF6;72,-50,-0.69) | **Q9HCE0** (EPG5;292,-47,-0.16) | **O60502** (MGEA5;103,-45,-0.43) |
| **Q6ZRS4** (CCDC129;115,-50,-0.43) | **Q9UK61** (FAM208A;189,-47,-0.24) | **P13533** (MYH6;224,-45,-0.20) |
| **Q8WUM0** (NUP133;129,-50,-0.38) | **Q5T1M5** (FKBP15; 134,-47,-0.35) | **Q13018** (PLA2R1;169,-45,-0.26) |
| **Q9H159** (CDH19;87,-49,-0.56) | **Q16236** (NFE2L2;68,-47,-0.69) | **Q9NY27** (PPP4R2;47,-45,-0.95) |
| **Q5TB80** (CEP162;162,-49,-0.30) | **Q12769** (NUP160;162,-47,-0.28) | **Q8NDX1** (PSD4;116,-45,-0.38) |
| **P28715** (ERCC5;133,-49,-0.36) | **Q9HC10** (OTOF;227,-47,-0.20) | **P13521** (SCG2;71,-45,-0.63) |
| **Q14974** (KPNB1;97,-49,-0.50) | **P16234** (PDGFRA;123,-47,-0.38) | **Q6ZT12** (UBR3;212,-45,-0.21) |
| **P07196** (NEFL;62,-49,-0.79) | **A6NGW2** (STRCP1;192,-47,-0.24) | **Q9UHP3** (USP25;122,-45,-0.36) |
| **Q9ULJ8** (PPP1R9A;123,-49,-0.39) | **Q5H9L4** (TAF7L;53,-47,-0.89) | **Q96RL7** (VPS13A;360,-45,-0.12) |
| **Q15276** (RABEP1;99,-49,-0.49) | **O94972** (TRIM37; 108,-47,-0.43) | **Q9BUR4** (WRAP53;59,-45,-0.75) |
| **P49792** (RANBP2;358,-49,-0.13) | **Q8N3P4** (VPS8;162,-47,-0.29) | **Q8N8U3** (ZCCHC5;53,-45,-0.85) |
| **Q14257** (RCN2;37,-49,-1.32) | **Q9NZT2** (OGFR;73,-47,-0.64) | **Q9GZS0** (DNAI2;69,-45,-0.65) |
| **Q96Q15** (SMG1;411,-49, -0.11) | **P78362** (SRPK2;78,-47,-0.60) | **Q9Y5Q9** (GTF3C3;101,-45,-0.44) |
| **Q9UKZ4** (TENM1;305,-49,-0.16) | **Q3MJ40** (CCDC144B;83,-47,-0.56) | **O14787** (TNPO2;101,-45,-0.44) |
| **Q9UNS1** (TIMELESS;139,-49,-0.35) | **P55283** (CDH4;100,-47,-0.46) | **Q08174** (PCDH1;115,-45,-0.39) |
| **Q96A61** (TRIM52;35,-49,-1.41) | **Q9Y5G9** (PCDHGA4;101,-47,-0.46) | **Q96KN7** (RPGRIP1;147,-45,-0.30) |
| **O60763** (USO1;108,-49,-0.45) | **Q9Y5G8** (PCDHGA5;101,-47,-0.46) | **Q14766** (LTBP1;187,-45,-0.24) |
| **O43379** (WDR62;166,-49,-0.29) | **Q9Y5G6** (PCDHGA7;102,-47,-0.46) | **Q9Y623** (MYH4;223,-45,-0.20) |
| **Q68DK2** (ZFYVE26;285,-49,-0.17) | **Q8N2S1** (LTBP4;173,-47,-0.27) | **Q8NB66** (UNC13C;251,-45,-0.17) |
| **P10645** (CHGA;51,-49,-0.96) | **O60303** (KIAA0556;181,-47,-0.25) | **Q9Y6D6** (ARFGEF1;209,-44,-0.21) |
| **O15240** (VGF;67,-49,-0.72) | **Q7RTU9** (STRC;193,-47,-0.24) | **Q8IW35** (CEP97;97,-44,-0.45) |
| **Q8NEM2** (SHCBP1;76,-49,-0.64) | **P13535** (MYH8;223,-47,-0.21) | **Q8TD26** (CHD6;305,-44,-0.14) |
| **Q12797** (ASPH;86,-49,-0.57) | **P12882** (MYH1;223,-47,-0.21) | **P54105** (CLNS1A;26,-44,-1.67) |
| **Q5VZP5** (DUSP27;130,-49,-0.37) | **P35579** (MYH9;227,-47,-0.20) | **P53675** (CLTCL1;187,-44,-0.23) |
| **Q9UJ98** (STAG3;139,-49,-0.35) | **H7BZ55** (0;248,-47,-0.18) | **Q16531** (DDB1;127,-44,-0.34) |
| **Q08378** (GOLGA3;167,-49,-0.29) | **Q8IZF6** (GPR112;333,-47,-0.14) | **Q96F46** (IL17RA;96,-44,-0.45) |
| **Q8IWV7** (UBR1;200,-49,-0.24) | **Q92667** (AKAP1;97,-46,-0.47) | **Q8N201** (INTS1;244,-44,-0.18) |
| **Q9UKX3** (MYH13;224,-49,-0.21) | **Q9H4D0** (CLSTN2;107,-46,-0.42) | **Q9NPG4** (PCDH12;129,-44,-0.34) |
| **Q9Y493** (ZAN;306,-49,-0.16) | **Q5JQC4** (CT 47A 1 ;30,-46,-1.52) | **O14917** (PCDH17;126,-44,-0.34) |
| **Q9BXK5** (BCL2L13;53,-48,-0.91) | **Q6ZR08** (DNAH12;357,-46,-0.12) | **Q9UN71** (PCDHGB4;100,-44,-0.44) |
| **Q9P1Z2** (CALCOCO1;77,-48,-0.62) | **Q5JWR5** (DOPEY1;277,-46,-0.16) | **P06454** (PTMA;12,-44,-3.60) |
| **P12107** (COL11A1;181,-48,-0.26) | **Q8NCM8** (DYNC2H1;493,-46,-0.09) | **Q5HYW3** (RGAG4;65,-44,-0.67) |
| **Q9UER7** (DAXX;81,-48,-0.58) | **Q96KQ7** (EHMT2;132,-46,-0.34) | **Q15393** (SF3B3;136,-44,-0.32) |
| **Q96RT1** (ERBB2IP;158,-48,-0.30) | **Q8IWE2** (FAM114A1;61,-46,-0.75) | **Q9BXU1** (STK31;116,-44,-0.38) |
| **P48551** (IFNAR2;58,-48,-0.83) | **A1Z1Q3** (MACROD2;50,-46,-0.91) | **Q9H7C4** (SYNC;55,-44,-0.79) |
| **O15397** (IPO8;120,-48,-0.40) | **Q71F56** (MED13L;243,-46,-0.18) | **O95759** (TBC1D8;131,-44,-0.33) |
| **Q14678** (KANK1;147,-48,-0.32) | **Q14980** (NUMA1;238,-46,-0.19) | **Q96RU2** (USP28;122,-44,-0.35) |
| **Q8WYB5** (KAT6B;231,-48,-0.20) | **Q8WY54** (PPM1E;85,-46,-0.54) | **Q93009** (USP7;128,-44,-0.34) |
| **P50748** (KNTC1;251,-48,-0.19) | **Q96SB3** (PPP1R9B;89,-46,-0.51) | **Q96ER3** (SAAL1;54,-44,-0.82) |
| **Q6ZNJ1** (NBEAL2;303,-48,-0.15) | **Q6UXD5** (SEZ6L2;98,-46,-0.47) | **Q9H069** (LRRC48;61,-44,-0.72) |
| **P14543** (NID1;136,-48,-0.35) | **P10451** (SPP1;35,-46,-1.29) | **P55289** (CDH12;88,-44,-0.49) |
| **Q9BYW2** (SETD2;288,-48,-0.16) | **Q9NY15** (STAB1;275,-46,-0.16) | **Q9ULB4** (CDH9;89,-44,-0.49) |
| **P32418** (SLC8A1;109,-48,-0.44) | **Q66K14** (TBC1D9B;141,-46,-0.32) | **P08048** (ZFY;91,-44,-0.48) |
| **Q6IN85** (SMEK1;95,-48,-0.50) | **Q92973** (TNPO1;102,-46,-0.44) | **Q9P260** (KIAA1468;135,-44,-0.32) |
| **Q5MIZ7** (SMEK2;97,-48,-0.49) | **Q8N3L3** (TXLNB;77,-46,-0.60) | **A4FU69** (EFCAB5;173,-44,-0.25) |
| **O75643** (SNRNP200;245,-48,-0.19) | **Q9UPU5** (USP24;294,-46,-0.15) | **Q13219** (PAPPA;181,-44,-0.24) |
| **P18583** (SON;264,-48,-0.18) | **Q7Z5K2** (WAPAL;133,-46,-0.34) | **Q8N7Z5** (ANKRD31;211,-44,-0.20) |
| **O75410** (TACC1;88,-48,-0.54) | **Q58FF6** (HSP90AB4P;58,-46,-0.78) | **Q9H1A4** (ANAPC1;217,-43,-0.19) |
| **Q13685** (AAMP;47,-48,-1.02) | **D6RB28** (GOLGA6L18;63,-46,-0.73) | **O15013** (ARHGEF10;152,-43,-0.28) |
| **P52739** (ZNF131;71,-48,-0.67) | **Q51J48** (CRB2;134,-46,-0.34) | **Q9NW68** (BSDC1;47,-43,-0.91) |
| **Q86UP0** (CDH24;88,-43,-0.49) | **Q8IYF3** (TEX11;108,-42,-0.38) | **P57103** (SLC8A3;103,-40,-0.38) |
| **Q9BQT9** (CLSTN3;106,-43,-0.40) | **P00450** (CP;122,-42,-0.34) | **Q8WXE9** (STON2;101,-40,-0.39) |
| **P55884** (EIF3B;92,-43,-0.46) | **O43432** (EIF4G3;177,-42,-0.23) | **Q9Y6A5** (TACC3;90,-40,-0.44) |
| **Q04637** (EIF4G1;175,-43,-0.24) | **Q14524** (SCN5A;227,-42,-0.18) | **Q6N022** (TENM4;308,-40,-0.12) |
| **Q2NKX8** (ERCC6L;141,-43,-0.30) | **Q14573** (ITPR3;304,-42,-0.13) | **Q9Y4E8** (USP15;112,-40,-0.35) |
| **Q8NB25** (FAM184A;133,-43,-0.32) | **Q14571** (ITPR2;308,-42,-0.13) | **Q86YA3** (ZGRF1;237,-40,-0.16) |
| **Q8IYD8** (FANCM;232,-43,-0.18) | **Q9P1Z9** (CCDC180;191,-41,-0.21) | **O43852** (CALU;37,-40,-1.07) |
| **Q17R60** (IMPG1;89,-43,-0.48) | **Q9HCK8** (CHD8;291,-41,-0.14) | **P0C7A2** (FAM153B;44,-40,-0.91) |
| **P41229** (KDM5C;176,-43,-0.24) | **P24386** (CHM;73,-41,-0.55) | **Q00987** (MDM2;55,-40,-0.72) |
| **Q9UQF2** (MAPK81P1;78,-43,-0.55) | **Q00610 (CLTC; 192,-41 ,-0.21)** | **Q92681** (RSC1A1;67,-40,-0.59) |
| **Q7Z406** (MYH14;228,-43,-0.18) | **Q9Y5B0** (CTDP1;104,-41,-0.39) | **Q0VDD7** (C19orf57;70,-40,-0.57) |
| **Q9UKX2** (MYH2;223,-43,-0.19) | **Q5JWF2** (GNAS;111,-41,-0.36) | **Q9Y5F1** (PCDHB12;87,-40,-0.46) |
| **Q6ZS30** (NBEAL1;307,-43,-0.13) | **P07900** (HSP90AA1;85,-41,-0.48) | **P19838** (NFKB1;105,-40,-0.37) |
| **Q61BW4** (NCAPH2;68,-43,-0.63) | **Q9BW6** (KIAA0586;169,-41,-0.24) | **O00291** (HIP1;116,-40,-0.34) |
| **Q9H5I5** (PIEZO2;318,-43,-0.13) | **P24043** (LAMA2;344,-41,-0.11) | **Q6ZRK6** (CCDC73;124,-40,-0.32) |
| **Q86YS3** (RAB11 FIP4;72,-43,-0.59) | **Q9UBF1** (MAGEC2;41,-41,-0.99) | **Q9HC56** (PCDH9;136,-40,-0.29) |
| **Q2NKQ1** (SGSM1;130,-43,-0.33) | **Q13615** (MTMR3;134,-41,-0.30) | **Q6Q759** (SPAG17;252,-40,-0.15) |
| **Q8N3U4** (STAG2;141,-43,-0.30) | **P13591** (NCAM1;95,-41,-0.43) | **Q9NY61** (AATF;63,-39,-0.61) |
| **Q8N1F8** (STK11IP;121,-43,-0.35) | **Q15003** (NCAPH;83,-41,-0.49) | **Q13085** (ACACA;266,-39,-0.14) |
| **Q9UMZ2** (SYNRG;141,-43,-0.30) | **O94916** (NFAT5;166,-41,-0.24) | **Q8N4S0** (CCDC82;64,-39,-0.60) |
| **Q6YHU6** (THADA;220,-43,-0.19) | **Q6ZVD8** (PHLPP2;147,-41,-0.27) | **Q8IUR6** (CREBRF;72,-39,-0.54) |
| **P98073** (TMPRSS15;113,-43,-0.38) | **Q5THK1** (PRR14L;237,-41,-0.17) | **Q9Y6B2** (EID1;21,-39,-1.86) |
| **Q8IWV8** (UBR2;201,-43,-0.21) | **Q07283** (TCHH;254,-41,-0.16) | **Q7L0X2** (ERICH6;75,-39,-0.51) |
| **Q9HCC9** (ZFYVE28;96,-43,-0.44) | **Q96RT7** (TUBGCP6;200,-41,-0.20) | **Q6H9L7** (ISM2;64,-39,-0.61) |
| **Q13438** (OS9;76,-43,-0.56) | **Q14139** (UBE4A;123,-41,-0.33) | **Q92845** (KIFAP3;91,-39,-0.42) |
| **Q9Y5H3** (PCDHGA10;101,-43,-0.42) | **Q9HAU5** (UPF2;148,-41,-0.27) | **Q6UXC1** (MAMDC4;131,-39,-0.29) |
| **Q9Y5H1** (PCDHGA2;101,-43,-0.42) | **Q9H321** (VCX3B;27,-41,-1.52) | **O60462** (NRP2;105,-39,-0.37) |
| **Q8N6L0** (CCDC155;63,-42,-0.66) | **Q702N8** (XIRP1;199,-41,-0.20) | **Q9UN73** (PCDHA6;103,-39,-0.37) |
| **Q9P219** (CCDC88C;228,-42,-0.18) | **Q8N7Z2** (GOLGA6L1;77,-41,-0.53) | **Q12923** (PTPN13;277,-39,-0.14) |
| **P16070** (CD44;82,-42,-0.51) | **O00418** (EEF2K;82,-41,-0.49) | **Q9H5N1** (RABEP2;64,-39,-0.61) |
| **Q02413** (DSG1;114,-42,-0.36) | **P01130** (LDLR;95,-41,-0.42) | **Q9Y3P9** (RABGAP1;122,-39,-0.32) |
| **Q14126** (DSG2;122,-42,-0.34) | **Q9Y5G7** (PCDHGA6;101,-41,-0.40) | **O15034** (RIMBP2;116,-39,-0.33) |
| **Q86X53** (ERICH1;49,-42,-0.85) | **Q9Y5H4** (PCDHGA1;101,-41,-0.40) | **P08922** (ROS1;264,-39,-0.14) |
| **Q9H501** (ESF1;99,-42,-0.42) | **Q9Y5F6** (PCDHGC5;102,-41,-0.40) | **Q8IZE3** (SCYL3;83,-39,-0.47) |
| **P14317** (HCLS1;54,-42,-0.77) | **O43707** (ACTN4;105,-41,-0.39) | **Q8IY92** (SLX4;200,-39,-0.19) |
| **P82970** (HMGN5;32,-42,-1.33) | **Q8N2E2** (VWDE;177,-41,-0.23) | **Q9BYV6** (TRIM55;60,-39,-0.64) |
| **Q1KMD3** (HNRNPUL2;85,-42,-0.49) | **P60006** (ANAPC15;14,-40,-2.80) | **Q9NYU2** (UGGT1;177,-39,-0.22) |
| **Q5VYJ5** (MALRD1;164,-42,-0.25) | **Q9UNK9** (ANGEL1;75,-40,-0.53) | **P51784** (USP11;110,-39,-0.35) |
| **Q6ZN16** (MAP3K15;147,-42,-0.28) | **Q9C0F0** (ASXL3;242,-40,-0.16) | **Q8IZQ1** (WDFY3;395,-39,-0.09) |
| **P12883** (MYH7;223,-42,-0.18) | **P55291** (CDH15;89,-40,-0.44) | **Q96B54** (ZNF428;20,-39,-1.90) |
| **P27815** (PDE4A;98,-42,-0.42) | **Q9ULB5** (CDH7;87,-40,-0.45) | **A1L162** (ERICH2;18,-39,-2.20) |
| **Q7Z5L2** (R3HCC1L;88,-42,-0.47) | **Q9P2I0** (CPSF2;88,-40,-0.45) | **P55822** (SH3BGR;26,-39,-1.49) |
| **Q9H2M9** (RAB3GAP2;156,-42,-0.26) | **Q96S65** (CSRNP1;64,-40,-0.62) | **Q96F63** (CCDC97;39,-39,-1.00) |
| **P46060** (RANGAP1;64,-42,-0.66) | **Q7L7V1** (DHX32;84,-40,-0.47) | **P10912** (GHR;72,-39,-0.54) |
| **Q9H446** (RWDD1;28,-42,-1.50) | **O95905** (ECD;73,-40,-0.54) | **Q9Y5E8** (PCDHB15;86,-39,-0.45) |
| **Q92543** (SNX19;109,-42,-0.38) | **Q9H9B1** (EHMT1;141,-40,-0.28) | **O00408** (PDE2A;106,-39,-0.36) |
| **Q8WVM7** (STAG1;144,-42,-0.29) | **Q5W0V3** (FAM160B1;87,-40,-0.46) | **Q53EL9** (SEZ6;107,-39,-0.36) |
| **Q6ZT07** (TBC1D9;143,-42,-0.29) | **Q4V328** (GRIPAP1;96,-40,-0.41) | **Q7Z3S7** (CACNA2D4;128,-39,-0.30) |
| **Q9H1E5** (TMX4;39,-42,-1.07) | **Q9BXL5** (HEMGN;55,-40,-0.72) | **Q5KSL6** (DGKK;142,-39,-0.27) |
| **Q8WV44** (TRIM41;72,-42,-0.58) | **P08238** (HSP90AB1;83,-40,-0.48) | **Q9UQP3** (TNN;144,-39,-0.27) |
| **P45974** (USP5;96,-42,-0.43) | **Q96RY7** (IFT140;165,-40,-0.24) | **Q9UKP4** (ADAMTS7;184,-39,-0.21) |
| **Q5MJ10** (SPANXN2;20,-42,-2.10) | **Q9BY66** (KDM5D;174,-40,-0.22) | **Q9UMZ3** (PTPRQ;261,-39,-0.14) |
| **A6NEY3** (GOLGA6L3;56,-42,-0.75) | **Q96Q89** (KIF20B;211,-40,-0.18) | **P98161** (PKD1;463,-39,-0.08) |
| **Q7Z6P3** (RAB44;78,-42,-0.54) | **Q6A162** (KRT40;48,-40,-0.83) | **O75445** (USH2A;576,-39,-0.06) |
| **Q6NZY4** (ZCCHC8;79,-42,-0.53) | **Q9Y2K3** (MYH15;225,-40,-0.17) | **Q9Y2D5** (AKAP2;95,-38,-0.40) |
| **P17010** (ZFX;91,-42,-0.46) | **Q9HD67** (MYO10;237,-40,-0.16) | **O96018** (APBA3;61,-38,-0.61) |
| **Q9Y5G1** (PCDHGB3;101,-42,-0.41) | **Q8TAB3** (PCDH19;126,-40,-0.31) | **Q8IYA2** (CCDC144CP;143,-38,-0.26) |
| **Q8TC90** (CCER1;46,-38,-0.81) | **Q86WI3** (NLRC5;205,-37,-0.18) | **Q5T481** (RBM20;134,-36,-0.26) |
| **Q9NYQ7** (CELSR3;358,-38,-0.10) | **Q8N6Y1** (PCDH20;105,-37,-0.35) | **Q8NHU2** (C20orf26; 141 ,-36,-0.25) |
| **Q8TDI0** (CHD5;223,-38,-0.17) | **Q9UN67** (PCDHB10;88,-37,-0.42) | **Q9H7P9** (PLEKHG2;148,-36,-0.24) |
| **Q6P2E9** (EDC4;152,-38,-0.25) | **Q9UN66** (PCDHB8;88,-37,-0.42) | **Q6IE36** (OVOS2;161 ,-36,-0.22) |
| **Q15075** (EEA1;162,-38,-0.23) | **Q08499** (PDE4D;91,-37,-0.40) | **P78363** (ABCA4;256,-35,-0.13) |
| **Q6P6B1** (ERICH5;40,-38,-0.95) | **Q07864** (POLE;262,-37,-0.14) | **Q5T1B0** (AXDND1;118,-35,-0.29) |
| **Q9UNN5** (FAF1;74,-38,-0.51) | **Q15262** (PTPRK;162,-37,-0.22) | **O60566** (BUB1B;120,-35,-0.29) |
| **P49327** (FASN;273,-38,-0.13) | **Q2PPJ7** (RALGAPA2;211,-37,-0.17) | **O60840** (CACNA1F;221,-35,-0.15) |
| **Q9UK22** (FBXO2;33,-38,-1.14) | **Q09028** (RBBP4;48,-37,-0.77) | **Q9ULU8** (CADPS;153,-35,-0.22) |
| **Q9BQQ3** (GORASP1;46,-38,-0.81) | **Q15424** (SAFB;103,-37,-0.36) | **Q0VF96** (CGNL1;149,-35,-0.23) |
| **Q9BQS7** (HEPH;130,-38,-0.29) | **O60279** (SUSD5;68,-37,-0.54) | **Q86UF2** (CTAGE6;88,-35,-0.39) |
| **Q9UPZ3** (HPS5;127,-38,-0.29) | **Q8WVT3** (TRAPPC12;79,-37,-0.46) | **Q8WYA6** (CTNNBL1;65,-35,-0.53) |
| **Q9UG01** (IFT172;198,-38,-0.19) | **Q2M329** (CCDC96;63,-37,-0.59) | **P32926** (DSG3;108,-35,-0.32) |
| **Q9NQT8** (KIF13B;203,-38,-0.18) | **Q9BQI7** (PSD2;85,-37,-0.43) | **O60344** (ECE2;100,-35,-0.35) |
| **Q99683** (MAP3K5;155,-38,-0.24) | **Q9Y5G3** (PCDHGB1;100,-37,-0.36) | **Q13144** (EIF2B5;80,-35,-0.43) |
| **O75095** (MEGF6;161,-38,-0.23) | **P35606** (COPB2;102,-37,-0.36) | **Q8TF40** (FNIP1;131,-35,-0.26) |
| **Q9UBG0** (MRC2;167,-38,-0.22) | **043491** (EPB41L2;113,-37,-0.32) | **P29083** (GTF2E1;49,-35,-0.70) |
| **Q9Y4A8** (NFE2L3;76,-38,-0.49) | **O75976** (CPD;153,-37,-0.24) | **Q92539** (LPIN2;99,-35,-0.35) |
| **Q92621** (NUP205;228,-38,-0.16) | **O00763** (ACACB;277,-36,-0.13) | **Q96PY6** (NEK1;143,-35,-0.24) |
| **Q9Y5H5** (PCDHA9;102,-38,-0.37) | **Q9Y4K1** (AIM1;189,-36,-0.19) | **Q8N543** (OGFOD1;63,-35,-0.55) |
| **O60330** (PCDHGA12;101,-38,-0.37) | **O75179** (ANKRD17;274,-36,-0.13) | **Q9NWQ8** (PAG1;47,-35,-0.74) |
| **Q6P2P2** (PRMT9;95,-38,-0.40) | **Q6ZP65** (CCDC64;65,-36,-0.55) | **Q9Y5F0** (PCDHB13;88,-35,-0.39) |
| **Q13200** (PSMD2;100,-38,-0.37) | **Q9HBB8** (CDHR5;88,-36,-0.40) | **Q07343** (PDE4B;83,-35,-0.41) |
| **Q86WV1** (SKAP1;41,-38,-0.91) | **Q9UQ88** (CDK11A;91,-36,-0.39) | **Q6P1J6** (PLB1;163,-35,-0.21) |
| **O15040** (TECPR2;154,-38,-0.24) | **P26374** (CHML;74,-36,-0.48) | **Q6WKZ4** (RAB11FIP1;137,-35,-0.25) |
| **Q13610** (PWP1;56,-38,-0.68) | **Q9NZV1** (CRIM1;114,-36,-0.31) | **Q16576** (RBBP7;48,-35,-0.73) |
| **P30203** (CD6;72,-38,-0.52) | **Q8NEL9** (DDHD1;100,-36,-0.35) | **Q8WYR4** (RSPH1;35,-35,-0.99) |
| **Q9Y5F2** (PCDHB11;87,-38,-0.43) | **Q8NDI1** (EHBP1;140,-36,-0.25) | **Q8WTS6** (SETD7;41,-35,-0.85) |
| **P55285** (CDH6;88,-38,-0.43) | **Q9NZJ5** (EIF2AK3;125,-36,-0.28) | **Q92922** (SMARCC1;123,-35,-0.28) |
| **Q9Y6N8** (CDH10;88,-38,-0.42) | **060841** (EIF5B;139,-36,-0.25) | **Q8TAQ2** (SMARCC2;133,-35,-0.26) |
| **Q96D09** (GPRASP2;94,-38,-0.40) | **Q7Z2Y8** (GVINP1;279,-36,-0.12) | **Q8WWQ8** (STAB2;277,-35,-0.12) |
| **Q9Y5H0** (PCDHGA3;101,-38,-0.37) | **Q9NQC8** (IFT46;34,-36,-1.04) | **Q9P273** (TENM3;301,-35,-0.11) |
| **Q9Y5H2** (PCDHGA11;102,-38,-0.37) | **Q86UP2** (KTN1;156,-36,-0.23) | **Q9Y490** (TLN1;270,-35,-0.12) |
| **Q9Y5H8** (PCDHA3;102,-38,-0.37) | **P55268** (LAMB2;196,-36,-0.18) | **O60296** (TRAK2;101,-35,-0.34) |
| **P12814** (ACTN1;103,-38,-0.36) | **A6NHM9** (MOXD2P;56,-36,-0.63) | **Q6BDS2** (UHRF1BP1;159,-35,-0.21) |
| **A1L4H1** (SSC5D;166,-38,-0.22) | **Q86W25** (NLRP13;119,-36,-0.30) | **Q96DT7** (ZBTB10;95,-35,-0.36) |
| **Q9NZ56** (FMN2;180,-38,-0.21) | **P07237** (P4HB;57,-36,-0.63) | **A1YPR0** (ZBTB7C;69,-35,-0.50) |
| **P16144** (ITGB4;202,-38,-0.18) | **Q08493** (PDE4C;80,-36,-0.45) | **Q8TCN5** (ZNF507;106,-35,-0.33) |
| **O00213** (APBB1;77,-37,-0.47) | **P35913** (PDE6B;98,-36,-0.36) | **Q8IY42** (C4orf19;34,-35,-1.03) |
| **Q9H1I8** (ASCC2;86,-37,-0.42) | **P40855** (PEX19;33,-36,-1.09) | **A8MU46** (SMTNL1;49,-35,-0.71) |
| **O75952** (CABYR;53,-37,-0.70) | **O15067** (PFAS;145,-36,-0.24) | **Q8N0Z9** (VSIG10;59,-35,-0.59) |
| **Q8N163** (CCAR2;103,-37,-0.35) | **Q15042** (RAB3GAP1;111,-36,-0.32) | **Q8NG66** (NEK11;74,-35,-0.47) |
| **Q13634** (CDH18;88,-37,-0.42) | **Q9NRP7** (STK36;144,-36,-0.25) | **Q96TA0** (PCDHB18;80,-35,-0.43) |
| **Q9H175** (CSRNP2;60,-37,-0.62) | **O14795** (UNC13B;181,-36,-0.19) | **Q14872** (MTF1;81,-35,-0.43) |
| **Q58WW2** (DCAF6;96,-37,-0.38) | **Q8NDM7** (WDR96;192,-36,-0.18) | **Q9Y5E7** (PCDHB2;87,-35,-0.40) |
| **Q9Y3R5** (DOPEY2;258,-37,-0.14) | **Q8TBC5** (ZSCAN18;55,-36,-0.65) | **Q9Y5G2** (PCDHGB2;101,-35,-0.34) |
| **O14576** (DYNC1I1;73,-37,-0.50) | **Q49AR2** (C5orf22;50,-36,-0.72) | **Q9Y5F8** (PCDHGB7;101,-35,-0.34) |
| **Q9Y6C2** (EMILIN1;107,-37,-0.34) | **Q9H6L5** (FAM134B;55,-36,-0.65) | **Q9Y5H6** (PCDHA8;103,-35,-0.33) |
| **Q9UNN4** (GTF2A1L;52,-37,-0.70) | **Q6PI26** (SHQ1;65,-36,-0.55) | **Q8TEV9** (SMCR8;105,-35,-0.33) |
| **Q969F1** (GTF3C6;24,-37,-1.53) | **Q9UJC3** (HOOK1;85,-36,-0.42) | **P38570** (ITGAE;130,-35,-0.26) |
| **O95163** (IKBKAP;150,-37,-0.24) | **Q8IV76** (PASD1;87,-36,-0.41) | **Q9NVE5** (USP40;140,-35,-0.24) |
| **P24394** (IL4R;90,-37,-0.41) | **Q9Y5E9** (PCDHB14;88,-36,-0.41) | **Q99707** (MTR;141,-35,-0.24) |
| **Q9UI26** (IPO11;113,-37,-0.32) | **Q9Y5G4** (PCDHGA9;102,-36,-0.35) | **Q13576** (IQGAP2;181,-35,-0.19) |
| **P09848** (LCT;219,-37,-0.16) | **Q9UN74** (PCDHA4;102,-36,-0.35) | **P27708** (CAD;243,-35,-0.14) |
| **A7E2Y1** (MYH7B;221,-37,-0.16) | **P57740** (NUP107;106,-36,-0.33) | **Q9P2R3** (ANKFY1;128,-34,-0.26) |
| **Q99733** (NAP1L4;43,-37,-0.86) | **Q68DQ2** (CRYBG3;116,-36,-0.30) | **Q2M1Z3** (ARHGAP31;157,-34,-0.21) |
| **Q96JN8** (NEURL4;167,-37,-0.22) | **P01133** (EGF;134,-36,-0.26) | **Q9Y4X5** (ARIH1;64,-34,-0.53) |
| **Q8WZ55** (BSND;35,-34,-0.96) | **Q96JB5** (CDK5RAP3;57,-33,-0.57) | **Q86V87** (FAM160B2;82,-32,-0.38) |
| **Q16790** (CA9;50,-34,-0.68) | **O00533** (CHL1;135,-33,-0.24) | **Q9BXW9** (FANCD2;166,-32,-0.19) |
| **O75155** (CAND2;135,-34,-0.25) | **P33076** (CIITA;124,-33,-0.26) | **O75420** (GIGYF1;115,-32,-0.27) |
| **P17655** (CAPN2;80,-34,-0.42) | **Q2KHT3** (CLEC16A;118,-33,-0.28) | **Q6Y7W6** (GIGYF2;150,-32,-0.21) |
| **Q86VB7** (CD163;125,-34,-0.27) | **Q9NVR5** (DNAAF2;91,-33,-0.36) | **Q9BQ67** (GRWD1;49,-32,-0.64) |
| **O15320** (CTAGE5;91,-34,-0.37) | **Q9UBC2** (EPS15L1;94,-33,-0.35) | **Q9Y4L1** (HYOU1;111,-32,-0.28) |
| **O14529** (CUX2;162,-34,-0.21) | **Q86XD5** (FAM131B;36,-33,-0.92) | **Q27J81** (INF2;136,-32,-0.23) |
| **Q13409** (DYNC1I2;71,-34,-0.47) | **Q9Y4F9** (FAM65B;119,-33,-0.27) | **P08514** (ITGA2B;113,-32,-0.28) |
| **Q9P2K8** (EIF2AK4;187,-34,-0.18) | **Q16665** (HIF1A;93,-33,-0.35) | **Q8TD91** (MAGEC3;72,-32,-0.44) |
| **P52655** (GTF2A1;42,-34,-0.81) | **Q96DU7** (ITPKC;75,-33,-0.43) | **Q9H8L6** (MMRN2;104,-32,-0.30) |
| **Q03933** (HSF2;60,-34,-0.56) | **Q32P28** (LEPRE1;83,-33,-0.39) | **Q9Y4B5** (MTCL1;210,-32,-0.15) |
| **Q14568** (HSP90AA2;39,-34,-0.86) | **Q92791** (LEPREL4;50,-33,-0.65) | **Q9NYA4** (MTMR4;133,-32,-0.23) |
| **P32019** (INPP5B;113,-34,-0.30) | **O94898** (LRIG2;119,-33,-0.27) | **Q8N307** (MUC20;72,-32,-0.44) |
| **O94829** (IPO13;108,-34,-0.31) | **Q8IXH7** (NELFCD;66,-33,-0.49) | **Q92614** (MYO18A;233,-32,-0.13) |
| **P20701** (ITGAL;129,-34,-0.26) | **O60500** (NPHS1;135,-33,-0.24) | **O75113** (N4BP1;100,-32,-0.31) |
| **Q53EV4** (LRRC23;40,-34,-0.85) | **P80303** (NUCB2;50,-33,-0.65) | **Q9ULI1** (NWD2;197,-32,-0.16) |
| **Q9C099** (LRRCC1;120,-34,-0.28) | **Q9BTK6** (PAGR1;28,-33,-1.19) | **Q504Q3** (PAN2;135,-32,-0.23) |
| **Q96JM4** (LRRIQ1;199,-34,-0.17) | **O60245** (PCDH7;116,-33,-0.28) | **Q96AQ6** (PBXIP1;81,-32,-0.39) |
| **Q96GA3** (LTV1;55,-34,-0.61) | **Q9Y5I0** (PCDHA13;102,-33,-0.32) | **Q9Y5G5** (PCDHGA8;101,-32,-0.31) |
| **P43363** (MAGEA 10;41,-34,-0.83) | **Q9UN72** (PCDHA7;101,-33,-0.32) | **Q9NQM4** (PIH1D3;24,-32,-1.32) |
| **P49736** (MCM2;102,-34,-0.33) | **O75864** (PPP1 R37;75,-33,-0.44) | **O15031** (PLXNB2;205,-32,-0.15) |
| **Q96NT1** (NAP1L5;20,-34,-1.73) | **Q9BRK5** (SDF4;42,-33,-0.78) | **Q9NVM4** (PRMT7;78,-32,-0.40) |
| **Q9Y4C0** (NRXN3;181,-34,-0.18) | **Q9UPR5** (SLC8A2;100,-33,-0.32) | **Q12913** (PTPRJ;146,-32,-0.21) |
| **Q9Y5I2** (PCDHA10;103,-34,-0.33) | **Q9NRL3** (STRN4;81,-33,-0.40) | **Q8IZ41** (RASEF;83,-32,-0.38) |
| **Q9Y5E1** (PCDHB9;87,-34,-0.39) | **Q96A49** (SYAP1;40,-33,-0.82) | **Q9ULF5** (SLC39A10;94,-32,-0.33) |
| **Q58EX7** (PLEKHG4;131,-34,-0.25) | **Q86VP1** (TAX1BP1;91,-33,-0.36) | **P02730** (SLC4A1;102,-32,-0.31) |
| **Q9ULL4** (PLXNB3;207,-34,-0.16) | **Q9NYB0** (TERF2IP;44,-33,-0.74) | **Q5TCY1** (TTBK1;143,-32,-0.22) |
| **Q9HAZ2** (PRDM16;140,-34,-0.24) | **Q8NFQ8** (TOR1AIP2;51,-33,-0.64) | **Q05086** (UBE3A;101,-32,-0.31) |
| **Q96D15** (RCN3;37,-34,-0.90) | **Q9Y3S1** (WNK2;243,-33,-0.13) | **O60287** (URB1;254,-32,-0.12) |
| **Q9BWU0** (SLC4A1AP;89,-34,-0.38) | **O60784** (TOM1;54,-33,-0.61) | **Q9UFB7** (ZBTB47;83,-32,-0.38) |
| **Q13033** (STRN3;87,-34,-0.38) | **A6NI86** (GOLGA6L10;55,-33,-0.59) | **Q5BKZ1** (ZNF326;66,-32,-0.48) |
| **Q9Y5L0** (TNPO3;104,-34,-0.32) | **P20810** (CAST;77,-33,-0.43) | **Q9UID6** (ZNF639;56,-32,-0.57) |
| **P48553** (TRAPPC10;142,-34,-0.23) | **Q9Y5E6** (PCDHB3;87,-33,-0.38) | **O75312** (ZPR1;51,-32,-0.62) |
| **Q96RT8** (TUBGCP5;118,-34,-0.28) | **Q9Y5E3** (PCDHB6;87,-33,-0.37) | **Q9UHY8** (FEZ2;40,-32,-0.80) |
| **Q86VQ3** (TXNDC2;60,-34,-0.56) | **Q8NDB2** (BANK1;89,-33,-0.36) | **Q8N9T8** (KRI1;83,-32,-0.38) |
| **P41226** (UBA7;112,-34,-0.30) | **Q12864** (CDH17;92,-33,-0.35) | **Q9UN75** (PCDHA12;102,-32,-0.31) |
| **O94763** (URI1;60,-34,-0.56) | **Q9Y5F9** (PCDHGB6;101,-33,-0.32) | **Q99460** (PSMD1;106,-32,-0.30) |
| **O75717** (WDHD1;126,-34,-0.26) | **P35609** (ACTN2;104,-33,-0.31) | **Q5W43** (KIAA0319;118,-32,-0.27) |
| **P0C2Y1** (NBPF7;48,-34,-0.70) | **Q9H158** (PCDHAC1;104,-33,-0.31) | **O14976** (GAK;143,-32,-0.22) |
| **Q9H6Z4** (RANBP3;60,-34,-0.56) | **Q8N8A2** (ANKRD44;108,-33,-0.30) | **Q9H4Z2** (ZNF335;145,-32,-0.22) |
| **Q9Y692** (GMEB1;63,-34,-0.54) | **Q9Y219** (JAG2;133,-33,-0.24) | **Q8N1P7** (AIM1L;69,-31,-0.45) |
| **P54829** (PTPN5;64,-34,-0.53) | **A4D0S4** (LAMB4;194,-33,-0.17) | **O43823** (AKAP8;76,-31,-0.40) |
| **B2RXH4** (BTBD18;78,-34,-0.43) | **Q99102** (MUC4;232,-33,-0.14) | **Q96PE2** (ARHGEF17;222,-31,-0.13) |
| **Q86UA1** (PRPF39;78,-34,-0.43) | **O15084** (ANKRD28;113,-32,-0.28) | **Q8TER5** (ARHGEF40;165,-31,-0.18) |
| **Q5H9M0** (MUM1L1;79,-34,-0.43) | **Q10567** (AP1B1;105,-32,-0.30) | **Q9NT62** (ATG3;36,-31,-0.86) |
| **O14867** (BACH1;82,-34,-0.41) | **Q86WG3** (ATCAY;42,-32,-0.75) | **Q8TC20** (CAGE1;90,-31,-0.34) |
| **O43815** (STRN;86,-34,-0.39) | **P51587** (BRCA2;384,-32,-0.08) | **Q9UKL3** (CASP8AP2;223,-31,-0.13) |
| **Q9Y5E4** (PCDHB5;86,-34,-0.39) | **Q9P0X4** (CACNA11;245,-32,-0.13) | **Q3V6T2** (CCDC88A;216,-31,-0.14) |
| **Q9BRC7** (PLCD4;88,-34,-0.38) | **Q5T0F9** (CC2D1B;94,-32,-0.33) | **P55290** (CDH13;78,-31,-0.39) |
| **Q9HBM0** (VEZT;89,-34,-0.38) | **Q96AJ1** (CLUAP1;48,-32,-0.66) | **P21127** (CDK11B;93,-31,-0.33) |
| **Q9BYV9** (BACH2;93,-34,-0.36) | **O75153** (CLUH;147,-32,-0.21) | **Q96JK2** (DCAF5;104,-31,-0.29) |
| **Q9Y5G0** (PCDHGB5;100,-34,-0.34) | **Q96PW8** (CSPG4P5;49,-32,-0.65) | **O60610** (DIAPH1;141,-31,-0.21) |
| **Q9Y5I3** (PCDHA1;103,-34,-0.33) | **A4D2H0** (CTAGE15;88,-32,-0.36) | **Q0VG06** (FAAP100;93,-31,-0.33) |
| **Q9HCM3** (KIAA1549;211,-34,-0.16) | **Q5TAQ9** (DCAF8;67,-32,-0.47) | **Q9NRY5** (FAM114A2;55,-31,-0.55) |
| **Q8IZ07** (ANKRD13A;68,-33,-0.48) | **Q09013** (DMPK;69,-32,-0.46) | **Q9UHL3** (FAM153A;35,-31,-0.89) |
| **Q6TDU7** (CASC1;83,-33,-0.39) | **Q96EV8** (DTNBP1;39,-32,-0.81) | **Q8N5J2** (FAM63A;52,-31,-0.59) |
| **Q96A33** (CCDC47;56,-33,-0.59) | **P04626** (ERBB2;138,-32,-0.23) | **P23142** (FBLN1;77,-31,-0.40) |
| **Q8IX15** (HOMEZ;61,-31,-0.50) | **Q9Y6Y8** (SEC23IP;111,-30,-0.27) | **O60318** (MCM3AP;218,-29,-0.13) |
| **Q9BYX4** (IFIH1;117,-31,-0.26) | **Q9UBV2** (SEL1L;89,-30,-0.33) | **A6BM72** (MEGF11;111,-29,-0.26) |
| **Q9HBE5** (IL21R;59,-31,-0.52) | **Q86VW0** (SESTD1;79,-30,-0.37) | **Q9NZM1** (MYOF;235,-29,-0.12) |
| **P52732** (KIF11;119,-31,-0.26) | **Q14BN4** (SLMAP;95,-30,-0.31) | **Q12968** (NFATC3;116,-29,-0.25) |
| **P43362** (MAGEA9;35,-31,-0.88) | **Q96LK8** (SPATA32;42,-30,-0.70) | **P25963** (NFKBIA;36,-29,-0.81) |
| **Q99435** (NELL2;91,-31,-0.33) | **Q92783** (STAM;59,-30,-0.50) | **Q15653** (NFKBIB;38,-29,-0.76) |
| **Q9P2S2** (NRXN2;185,-31,-0.16) | **Q9NT68** (TENM2;308,-30,-0.09) | **Q8N427** (NME8;67,-29,-0.43) |
| **Q02818** (NUCB1;54,-31,-0.57) | **Q96FV9** (THOC1;76,-30,-0.39) | **Q96RS6** (NUDCD1;67,-29,-0.43) |
| **P08575** (PTPRC;147,-31,-0.21) | **Q9Y6L7** (TLL2;114,-30,-0.26) | **Q96CV9** (OPTN;66,-29,-0.43) |
| **Q9HD43** (PTPRH;122,-31,-0.25) | **Q12899** (TRIM26;62,-30,-0.48) | **P13667** (PDIA4;73,-29,-0.39) |
| **P0DJD0** (RGPD1;197,-31,-0.15) | **Q92995** (USP13;97,-30,-0.30) | **Q6ZMN7** (PDZRN4;117,-29,-0.24) |
| **Q12765** (SCRN1;46,-31,-0.66) | **Q9H4A3** (WNK1;251,-30,-0.11) | **O60346** (PHLPP1;185,-29,-0.15) |
| **Q9H4L7** (SMARCAD1; 117,-31 ,-0.26) | **Q9BYP7** (WNK3;198,-30,-0.15) | **Q8NG27** (PJA1;71,-29,-0.40) |
| **Q24JP5** (TMEM132A;110,-31,-0.28) | **Q86T24** (ZBTB33;74,-30,-0.40) | **Q96KN3** (PKNOX2;52,-29,-0.55) |
| **Q2TAA8** (TSNAXIP1;77,-31,-0.40) | **P20962** (PTMS;12,-30,-2.60) | **Q9ULL1** (PLEKHG1;155,-29,-0.18) |
| **A0JNW5** (UHRF1BP1L;164,-31,-0.18) | **Q9BQE6** (C11orf48;32,-30,-0.95) | **O75145** (PPFIA3;133,-29,-0.21) |
| | **Q96CW6** (SLC7A6CS;35,-30,-0.85) | **O75688** (PPM1B;53,-29,-0.55) |
| **Q8N2C7** (UNC80;363,-31,-0.08) | **Q8IZU1** (FAM9A;37,-30,-0.80) | **Q9Y4B4** (RAD54L2;163,-29,-0.17) |
| **O60315** (ZEB2;136,-31,-0.22) | **Q9NZA1** (CLIC5;47,-30,-0.64) | **P0DJD1** (RGPD2;197,-29,-0.14) |
| **P09871** (C1S;77,-31,-0.40) | **Q9BY79** (MFRP;62,-30,-0.48) | **Q9Y6N7** (ROBO1;181,-29,-0.16) |
| **Q86VS8** (HOOK3;83,-31,-0.37) | **Q9HA65** (TBC1D17;73,-30,-0.41) | **O75563** (SKAP2;41,-29,-0.70) |
| **P33151** (CDH5;88,-31,-0.35) | **Q12934** (BFSP1;75,-30,-0.40) | **060641** (SNAP91;93,-29,-0.31) |
| **Q8IX94** (CTAGE4;88,-31,-0.35) | **Q8TE96** (DQX1;79,-30,-0.37) | **Q13342** (SP140;98,-29,-0.29) |
| **Q9Y5H9** (PCDHA2;102,-31,-0.30) | **Q32M88** (ATHL1;81,-30,-0.37) | **Q69YQ0** (SPECC1L;125,-29,-0.23) |
| **B5ME19** (EIF3CL;105,-31,-0.29) | **A4FU28** (CTAGE9;88,-30,-0.34) | **P07204** (THBD;60,-29,-0.48) |
| **Q6ZU35** (KIAA1211;137,-31,-0.22) | **P49754** (VPS41;99,-30,-0.30) | **P63316** (TNNC1;18,-29,-1.57) |
| **Q9NS15** (LTBP3;139,-31,-0.22) | **Q6ZP82** (CCDC141;101,-30,-0.29) | **Q9P2K2** (TXNDC16;94,-29,-0.30) |
| **P39060** (COL18A1;178,-31,-0.17) | **Q6ZP01** (RBM44;118,-30,-0.25) | **Q9P2H5** (USP35;113,-29,-0.25) |
| **Q13129** (RLF;218,-31,-0.14) | **Q6IE37** (OVOS1;134,-30,-0.22) | **Q6AWC2** (WWC2;134,-29,-0.21) |
| **Q8N302** (AGGF1;81,-30,-0.37) | **Q13464** (ROCK1;158,-30,-0.18) | **P52747** (ZNF143;69,-29,-0.42) |
| **Q8NEU8** (APPL2;74,-30,-0.40) | **Q16363** (LAMA4;203,-30,-0.14) | **O95125** (ZNF202;75,-29,-0.38) |
| **A7KAX9** (ARHGAP32;231,-30,-0.13) | **Q9ULX6** (AKAP8L;72,-29,-0.40) | **P02585** (TNNC2;18,-29,-1.60) |
| **Q9HCE6** (ARHGEF10L;140,-30,-0.21) | **Q9Y6D5** (ARFGEF2;202,-29,-0.14) | **A6NNZ2** (0;50,-29,-0.58) |
| | **Q8TD16** (BICD2;94,-29,-0.31) | **P19235** (EPOR;55,-29,-0.52) |
| **O43313** (ATMIN;88,-30,-0.33) | **Q6PGQ7** (BORA;61,-29,-0.47) | **Q2M3C6** (C15orf27;58,-29,-0.49) |
| **P46379** (BAG6;119,-30,-0.25) | **Q6PJG6** (BRAT1;88,-29,-0.32) | **C9JE40** (PATL2;61,-29,-0.47) |
| **O43497** (CACNA1G;262,-30,-0.11) | **Q8IY82** (CCDC135;103,-29,-0.28) | **Q53GT1** (KLHL22;72,-29,-0.40) |
| **Q99674** (CGREF1;32,-30,-0.94) | **Q9HCU0** (CD248;81,-29,-0.35) | **P0CG23** (ZNF853;75,-29,-0.38) |
| **Q8N137** (CNTROB;101,-30,-0.29) | **Q969H4** (CNKSR1;80,-29,-0.36) | **Q9Y5E5** (PCDHB4;87,-29,-0.33) |
| **Q9UBP4** (DKK3;38,-30,-0.78) | **Q9NQ92** (COPRS;20,-29,-1.44) | **P0CG41** (CTAGE8;88,-29,-0.32) |
| **Q9Y238** (DLEC1;196,-30,-0.15) | **Q6UXH1** (CRELD2;38,-29,-0.75) | **Q14566** (MCM6;93,-29,-0.31) |
| **Q99613** (EIF3C;105,-30,-0.28) | **Q6V1X1** (DPP8;103,-29,-0.28) | **Q60I27** (ALS2CL;108,-29,-0.26) |
| **Q13683** (ITGA7;129,-30,-0.23) | **Q08554** (DSC1;100,-29,-0.29) | **O43592** (XPOT;110,-29,-0.26) |
| **P22459** (KCNA4;73,-30,-0.40) | **P11171** (EPB41;97,-29,-0.29) | **P69849** (NOMO3;134,-29,-0.21) |
| **Q9NS87** (KIF15;160,-30,-0.18) | **Q9UKA1** (FBXL5;79,-29,-0.36) | **Q5JPE7** (NOMO2;139,-29,-0.20) |
| **Q9Y561** (LRP12;95,-30,-0.31) | **A9Z1Z3** (FER1L4;201,-29,-0.14) | **Q14203** (DCTN1;142,-29,-0.20) |
| **Q8WXG6** (MADD;183,-30,-0.16) | **Q2V2M9** (FHOD3;159,-29,-0.18) | **O15118** (NPC1;142,-29,-0.20) |
| **Q7Z3U7** (MON2;190,-30,-0.15) | **Q9UJ14** (GGT7;70,-29,-0.41) | **Q9Y5Y9** (SCN10A;221,-29,-0.13) |
| **Q86XG9** (NBPF5P;41,-30,-0.73) | **Q9UHF4** (IL20RA;62,-29,-0.46) | **Q7Z591** (AKNA;155,-28,-0.18) |
| **Q92823** (NRCAM;144,-30,-0.20) | **Q68E01** (INTS3;118,-29,-0.24) | **Q96QP1** (ALPK1;139,-28,-0.20) |
| **Q86Y26** (NUTM1;120,-30,-0.24) | **P17301** (ITGA2;129,-29,-0.22) | **Q9UJX6** (ANAPC2;94,-28,-0.29) |
| **Q8N573** (OXR1;98,-30,-0.30) | **Q96EK5** (KIAA1279;72,-29,-0.40) | **O14727** (APAF1;142,-28,-0.19) |
| **Q9UPQ7** (PDZRN3;120,-30,-0.25) | **Q12756** (KIF1A;191,-29,-0.15) | **Q6ZUM4** (ARHGAP27;98,-28,-0.28) |
| **O94880** (PHF14;100,-30,-0.29) | **O00629** (KPNA4;58,-29,-0.50) | **O75129** (ASTN2;148,-28,-0.18) |
| **Q6ZUJ8** (PIK3AP1;90,-30,-0.33) | **Q9HBX8** (LGR6;104,-29,-0.27) | **P30530** (AXL;98,-28,-0.28) |
| **Q5R372** (RABGAP1L;93,-30,-0.32) | **P33241** (LSP1;37,-29,-0.77) | **O14977** (AZIN1;50,-28,-0.56) |
| **Q8TEU7** (RAPGEF6;179,-30,-0.16) | **P29966** (MARCKS;32,-29,-0.91) | **Q5VU97** (CACHD1;142,-28,-0.19) |
| **Q52MB2** (CCDC184;20,-28,-1.36) | **Q8N4P2** (TTC30B;76,-28,-0.36) | **Q7Z6M4** (MTERF4;44,-27,-0.61) |
| **O95400** (CD2BP2;38,-28,-0.74) | **Q86WT1** (TTC30A;76,-28,-0.36) | **Q14764** (MVP;99,-27,-0.27) |
| **Q9Y5S2** (CDC42BPB;194,-28,-0.14) | **Q7Z304** (MAMDC2;78,-28,-0.36) | **O75592** (MYCBP2;510,-27,-0.05) |
| **Q8N129** (CNPY4;28,-28,-0.98) | **A6NDK9** (GOLGA6C;80,-28,-0.35) | **P08138** (NGFR;45,-27,-0.59) |
| **Q8WYK1** (CNTNAP5;146,-28,-0.19) | **Q9Y5E2** (PCDHB7;87,-28,-0.32) | **Q99650** (OSMR;111,-27,-0.24) |
| **Q9UKF6** (CPSF3;77,-28,-0.36) | **Q92805** (GOLGA1;88,-28,-0.31) | **Q16549** (PCSK7;86,-27,-0.31) |
| **Q02487** (DSC2;100,-28,-0.28) | **Q9H1K0** (ZFYVE20;89,-28,-0.31) | **Q6P996** (PDXDC1;87,-27,-0.31) |
| **Q9H4G0** (EPB41 L1;99,-28,-0.28) | **P23508** (MCC;93,-28,-0.30) | **P19174** (PLCG1;149,-27,-0.18) |
| **Q6UXB0** (FAM131A;36,-28,-0.77) | **Q5JSH3** (WDR44;101,-28,-0.27) | **Q86W92** (PPFIBP1;114,-27,-0.23) |
| **Q9H8M7** (FAM188A;50,-28,-0.56) | **Q08043** (ACTN3;103,-28,-0.27) | **Q9UD71** (PPP1R1B;23,-27,-1.17) |
| **Q8TB52** (FBXO30;82,-28,-0.34) | **O14983** (ATP2A1;110,-28,-0.25) | **Q92932** (PTPRN2;111,-27,-0.24) |
| **O95302** (FKBP9;63,-28,-0.44) | **P0CG39** (POTEJ;117,-28,-0.23) | **Q92692** (PVRL2;58,-27,-0.46) |
| **Q9NYA3** (GOLGA6A;80,-28,-0.35) | **Q13370** (PDE3B;124,-28,-0.22) | **O95072** (REC8;63,-27,-0.43) |
| **Q96I76** (GPATCH3;59,-28,-0.47) | **Q5T0N1** (TTC18;126,-28,-0.22) | **Q9H9A7** (RMI1;70,-27,-0.38) |
| **Q9UHW5** (GPN3;33,-28,-0.85) | **Q9UPM8** (AP4E1;127,-28,-0.21) | **Q9UBS8** (RNF14;54,-27,-0.50) |
| **Q5TGJ6** (HDGFL1;27,-28,-1.02) | **Q7Z6G8** (ANKS1B;138,-28,-0.20) | **Q99250** (SCN2A;228,-27,-0.11) |
| **Q9NQG7** (HPS4;77,-28,-0.36) | **O75334** (PPFIA2;143,-28,-0.19) | **Q96JE7** (SEC16B;117,-27,-0.23) |
| **Q9NQC1** (JADE2;87,-28,-0.32) | **P54296** (MYOM2;165,-28,-0.16) | **Q6P5W5** (SLC39A4;68,-27,-0.39) |
| **Q7Z3Y9** (KRT26;52,-28,-0.53) | **Q7Z2Y5** (NRK;178,-28,-0.15) | **Q969G3** (SMARCE1;47,-27,-0.57) |
| **Q16626** (MEA1;20,-28,-1.40) | **Q6ZU64** (CCDC108;217,-28,-0.12) | **P09486** (SPARC;35,-27,-0.77) |
| **Q14149** (MORC3;107,-28,-0.26) | **Q86W8** (RTTN;249,-28,-0.11) | **Q8TE77** (SSH3;73,-27,-0.36) |
| **Q13075** (NAIP;160,-28,-0.17) | **A8K2U0** (A2ML1;161,-27,-0.16) | **O75886** (STAM2;58,-27,-0.46) |
| **O15259** (NPHP1;83,-28,-0.33) | **Q9H3P7** (ACBD3;61,-27,-0.44) | **Q8NE28** (STKLD1;76,-27,-0.35) |
| **Q9UHY1** (NRBP1;60,-28,-0.46) | **Q8N4X5** (AFAP1 L2;91,-27,-0.29) | **Q9Y5B9** (SUPT16H;120,-27,-0.22) |
| **Q02509** (OC90;53,-28,-0.52) | **Q96Q42** (ALS2;184,-27,-0.14) | **Q9NUY8** (TBC1D23;78,-27,-0.34) |
| **Q9Y5I1** (PCDHA11;103,-28,-0.27) | **Q8IW19** (APLF;57,-27,-0.47) | **Q96HA7** (TONSL;151,-27,-0.17) |
| **Q9NRJ7** (PCDHB16;85,-28,-0.32) | **Q96P48** (ARAP1;162,-27,-0.16) | **P09493** (TPM1;33,-27,-0.82) |
| **Q9UN70** (PCDHGC3;101,-28,-0.27) | **Q99856** (ARID3A;63,-27,-0.42) | **A6NMA1** (TRPC5OS;12,-27,-2.19) |
| **P16499** (PDE6A;100,-28,-0.28) | **Q9NWV8** (BABAM1;37,-27,-0.73) | **Q712K3** (UBE2R2;27,-27,-0.99) |
| **Q13087** (PDIA2;58,-28,-0.48) | **Q9P287** (BCCIP;36,-27,-0.75) | **Q8N806** (UBR7;48,-27,-0.56) |
| **O00562** (PITPNM1;135,-28,-0.20) | **Q12830** (BPTF;338,-27,-0.07) | **Q9H320** (VCX;22,-27,-1.21) |
| **P47712** (PLA2G4A;85,-28,-0.32) | **P54289** (CACNA2D1;125,-27,-0.21) | **O95365** (ZBTB7A;61,-27,-0.43) |
| **Q9P212** (PLCE1;259,-28,-0.10) | **Q86VP6** (CAND1;136,-27,-0.19) | **Q9ULD5** (ZNF777;85,-27,-0.31) |
| **P09884** (POLA1;166,-28,-0.16) | **Q5WM6** (CCDC30;91,-27,-0.29) | **Q96B23** (C18orf25;43,-27,-0.62) |
| **O14974** (PPP1R12A;115,-28,-0.24) | **Q7Z6B0** (CCDC91;50,-27,-0.54) | **O15379** (HDAC3;49,-27,-0.55) |
| **Q9UKN5** (PRDM4;88,-28,-0.31) | **Q66GS9** (CEP135;133,-27,-0.20) | **Q9H074** (PAIP1;54,-27,-0.50) |
| **Q7Z3J3** (RGPD4;197,-28,-0.14) | **Q9P2M7** (CGN;136,-27,-0.19) | **Q8NAP8** (ZBTB8B;54,-27,-0.49) |
| **P35498** (SCN1A;229,-28,-0.12) | **Q9Y678** (COPG1;98,-27,-0.27) | **O15488** (GYG2;55,-27,-0.48) |
| **Q92563** (SPOCK2;47,-28,-0.59) | **Q9BSW2** (CRACR2A;46,-27,-0.59) | **Q6ZVM7** (TOM1 L2;56,-27,-0.48) |
| **O60284** (ST18;115,-28,-0.24) | **Q16832** (DDR2;97,-27,-0.27) | **Q9BQ31** (KCNS3;56,-27,-0.48) |
| **Q9Y2W6** (TDRKH;62,-28,-0.45) | **P98153** (DGCR2;61,-27,-0.44) | **Q9UK59** (DBR1;62,-27,-0.43) |
| **P07951** (TPM2;33,-28,-0.85) | **Q8NFT8** (DNER;78,-27,-0.34) | **Q6ZVT6** (C3orf67;76,-27,-0.35) |
| **O43156** (TTI1;122,-28,-0.22) | **Q8IUD2** (ERC1; 128,-27,-0.21) | **P0CG33** (GOLGA6D;80,-27,-0.33) |
| **Q14166** (TTLL12;74,-28,-0.37) | **Q9UK99** (FBXO3;55,-27,-0.49) | **Q02447** (SP3;82,-27,-0.32) |
| **Q5BVD1** (TTMP;24,-28,-1.15) | **Q2WGJ9** (FER1L6;209,-27,-0.12) | **Q9NY33** (DPP3;83,-27,-0.32) |
| **Q96QK1** (VPS35;92,-28,-0.30) | **Q53EP0** (FNDC3B;133,-27,-0.20) | **Q68BL8** (OLFML2B;84,-27,-0.32) |
| **Q96J92** (WNK4;135,-28,-0.20) | **Q02153** (GUCY1B3;71,-27,-0.38) | **P18564** (ITGB6;86,-27,-0.31) |
| **Q8NA77** (TEX19;18,-28,-1.51) | **Q9UQL6** (HDAC5;122,-27,-0.22) | **Q05209** (PTPN12;88,-27,-0.30) |
| **Q86VG3** (C11orf74;25,-28,-1.10) | **Q9H583** (HEATR1;242,-27,-0.11) | **Q9Y5F3** (PCDHB1;90,-27,-0.29) |
| **P06753** (TPM3;33,-28,-0.84) | **P34932** (HSPA4;94,-27,-0.28) | **Q5K4E3** (PRSS36;92,-27,-0.29) |
| **Q6PD74** (AAGAB;35,-28,-0.80) | **Q0D2I5** (IFFO1;62,-27,-0.43) | **P52790** (HK3;99,-27,-0.27) |
| **Q14242** (SELPLG;43,-28,-0.64) | **P35968** (KDR;152,-27,-0.17) | **Q8IWG1** (WDR63;103,-27,-0.26) |
| **Q53HC9** (TSSC1;44,-28,-0.64) | **Q9Y468** (L3MBTL1;84,-27,-0.32) | **Q13474** (DRP2;108,-27,-0.25) |
| **P55081** (MFAP1;52,-28,-0.53) | **Q9Y2L9** (LRCH1;81,-27,-0.33) | **Q8NB46** (ANKRD52;115,-27,-0.23) |
| **Q96HR8** (NAF1;54,-28,-0.52) | **P43357** (MAGEA3;35,-27,-0.77) | **P53708** (ITGA8;117,-27,-0.22) |
| **P15260** (IFNGR1;54,-28,-0.51) | **O15151** (MDM4;55,-27,-0.49) | **Q14432** (PDE3A;125,-27,-0.21) |
| **Q5TF58** (IFFO2;57,-28,-0.48) | **Q5U5Q3** (MEX3C;69,-27,-0.38) | **Q7RTW8** (OTOA;129,-27,-0.21) |
| **Q96MR6** (WDR65;145,-27,-0.18) | **Q14C87** (TMEM132D;122,-26,-0.21) | **Q6IA86** (ELP2;93,-25,-0.27) |
| **Q8TE82** (SH3TC1;147,-27,-0.18) | **O14545** (TRAFD1;65,-26,-0.40) | **Q8TAM6** (ERMN;33,-25,-0.76) |
| **P06213** (INSR;156,-27,-0.17) | **Q92574** (TSC1;130,-26,-0.20) | **P78312** (FAM193A;140,-25,-0.17) |
| **P12821** (ACE;150,-26,-0.17) | **Q15714** (TSC22D1;110,-26,-0.23) | **A6H8Z2** (FAM221B;45,-25,-0.55) |
| **Q92625** (ANKS1A;123,-26,-0.21) | **Q13107** (USP4;109,-26,-0.23) | **Q969H0** (FBXW7;80,-25,-0.31) |
| **P63010** (AP2B1;105,-26,-0.24) | **Q709C8** (VPS13C;422,-26,-0.06) | **O95684** (FGFR1 OP;43,-25,-0.58) |
| **O60306** (AQR;171,-26,-0.15) | **Q9P253** (VPS18;110,-26,-0.23) | **P35916** (FLT4;153,-25,-0.16) |
| **O00499** (BIN1;65,-26,-0.40) | **Q15061** (WDR43;75,-26,-0.34) | **Q9C0B1** (FTO;58,-25,-0.42) |
| **O14981** (BTAF1;207,-26,-0.12) | **O94967** (WDR47;102,-26,-0.25) | **Q06547** (GABPB1;42,-25,-0.58) |
| **Q01850** (CDR2;52,-26,-0.50) | **O14980** (XPO1;123,-26,-0.21) | **E2RYF7** (HCG22;26,-25,-0.95) |
| **Q76N32** (CEP68;81,-26,-0.32) | **Q9NUA8** (ZBTB40;138,-26,-0.18) | **O94992** (HEXIM1;41,-25,-0.61) |
| **Q5VXU3** (CHIC1;26,-26,-1.01) | **Q9UPT8** (ZC3H4;140,-26,-0.18) | **Q8TDY8** (IGDCC4;134,-25,-0.18) |
| **O14578** (CIT;231,-26,-0.11) | **O43561** (LAT;28,-26,-0.93) | **O75164** (KDM4A;121 ,-25,-0.20) |
| **P83436** (COG7;86,-26,-0.30) | **P51861** (CDR1;31,-26,-0.83) | **Q2M2Z5** (KIZ;75,-25,-0.33) |
| **Q8NA03** (FSIP1;66,-26,-0.39) | **Q9BVW5** (TIPIN;35,-26,-0.75) | **O00505** (KPNA3;58,-25,-0.43) |
| **A6NDN3** (GOLGA6B;80,-26,-0.32) | **P54725** (RAD23A;40,-26,-0.65) | **P19012** (KRT15;49,-25,-0.50) |
| **Q8IYU2** (HACE1;102,-26,-0.25) | **Q6ZS10** (CLEC17A;43,-26,-0.60) | **Q9UIQ6** (LNPEP;117,-25,-0.21) |
| **Q92619** (HMHA1;125,-26,-0.20) | **Q4V339** (CBWD6;44,-26,-0.59) | **O94822** (LTN1;201,-25,-0.12) |
| **Q58FF8** (HSP90AB2P;44,-26,-0.58) | **Q5JTY5** (CBWD3;44,-26,-0.59) | **P20916** (MAG;69,-25,-0.36) |
| **Q92598** (HSPH1;97,-26,-0.26) | **Q5RIA9** (CBWD5;44,-26,-0.58) | **P43356** (MAGEA2;35,-25,-0.71) |
| **Q9NWB7** (IFT57;49,-26,-0.52) | **Q8IZM8** (ZNF654;66,-26,-0.39) | **P43360** (MAGEA6;35,-25,-0.71) |
| **Q6UXK2** (ISLR2;79,-26,-0.32) | **Q96ED9** (HOOK2;83,-26,-0.31) | **Q8TD90** (MAGEE2;60,-25,-0.41) |
| **P78504** (JAG1;134,-26,-0.19) | **Q96CN9** (GCC1;88,-26,-0.29) | **Q9BUU2** (METTL22;44,-25,-0.56) |
| **Q12767** (KIAA0195;151,-26,-0.17) | **Q9H9E3** (COG4;89,-26,-0.29) | **P84157** (MXRA7;21,-25,-1.16) |
| **Q9P2E2** (KIF17;115,-26,-0.22) | **Q96CN4** (EVI5L;91,-26,-0.28) | **Q15746** (MYLK;211,-25,-0.11) |
| **Q12840** (KIF5A;117,-26,-0.22) | **Q03167** (TGFBR3;93,-26,-0.27) | **Q9BPX3** (NCAPG;114,-25,-0.21) |
| **Q76NI1** (KNDC1;191,-26,-0.13) | **Q9Y5F7** (PCDHGC4;101,-26,-0.25) | **Q96PU5** (NEDD4L;112,-25,-0.22) |
| **O76015** (KRT38;50,-26,-0.51) | **Q8IYH5** (ZZZ3;102,-26,-0.25) | **Q92832** (NELL1;90,-25,-0.27) |
| **Q5S007** (LRRK2;286,-26,-0.09) | **Q92888** (ARHGEF1;102,-26,-0.25) | **Q96MN2** (NLRP4;113,-25,-0.22) |
| **P43364** (MAGEA11;48,-26,-0.54) | **Q6IEE7** (TMEM132E;107,-26,-0.24) | **Q92636** (NSMAF;104,-25,-0.23) |
| **Q9UDY8** (MALT1;92,-26,-0.28) | **Q9Y5I4** (PCDHAC2;109,-26,-0.23) | **O75694** (NUP155;155,-25,-0.16) |
| **O00462** (MANBA;101,-26,-0.25) | **Q96G01** (BICD1;111,-26,-0.23) | **Q9ULW8** (PADI3;75,-25,-0.33) |
| **Q9Y6R4** (MAP3K4;182,-26,-0.14) | **P13637** (ATP1A3;112,-26,-0.23) | **Q9BPZ3** (PAIP2;15,-25,-1.66) |
| **P48740** (MASP1;79,-26,-0.32) | **Q13367** (AP3B2;119,-26,-0.21) | **O15534** (PER1;136,-25,-0.18) |
| **Q7Z7M0** (MEGF8;303,-26,-0.08) | **Q8NG08** (HELB;123,-26,-0.21) | **Q96JS3** (PGBD1;93,-25,-0.27) |
| **Q12866** (MERTK;110,-26,-0.23) | **Q0IIM8** (TBC1D8B;129,-26,-0.20) | **P46020** (PHKA1;137,-25,-0.18) |
| **Q8TDB4** (MGARP;25,-26,-1.02) | **Q9H195** (MUC3B;131,-26,-0.19) | **Q6S8J3** (POTEE;121,-25,-0.20) |
| **060291** (MGRN1;61,-26,-0.42) | **P08069** (IGF1R;155,-26,-0.16) | **A5A3E0** (POTEF;121,-25,-0.20) |
| **P98088** (MUC5AC;527,-26,-0.04) | **Q9ULJ7** (ANKRD50;156,-25,-0.16) | **Q9Y5P8** (PPP2R3B;65,-25,-0.38) |
| **Q8NI08** (NCOA7;106,-26,-0.24) | **Q9HCE9** (ANO8;136,-25,-0.18) | **Q8TCU6** (PREX1;186,-25,-0.13) |
| **Q15155** (NOMO1;134,-26,-0.19) | **Q6Q4G3** (AQPEP;113,-25,-0.22) | **P49810** (PSEN2;50,-25,-0.49) |
| **Q14207** (NPAT;154,-26,-0.16) | **P16615** (ATP2A2;115,-25,-0.21) | **P23470** (PTPRG;162,-25,-0.15) |
| **Q9ULB1** (NRXN1;162,-26,-0.16) | **Q3SYG4** (BBS9;99,-25,-0.25) | **Q16827** (PTPRO;138,-25,-0.18) |
| **P52948** (NUP98;198,-26,-0.13) | **P56945** (BCAR1;93,-25,-0.26) | **P08195** (SLC3A2;68,-25,-0.36) |
| **Q9NP74** (PALMD;63,-26,-0.41) | **Q5T5X7** (BEND3;94,-25,-0.26) | **Q9NWM0** (SMOX;62,-25,-0.40) |
| **Q6QHF9** (PAOX;70,-26,-0.36) | **Q92994** (BRF1;74,-25,-0.33) | **Q6GMV2** (SMYD5;47,-25,-0.52) |
| **A2A3N6** (PIPSL;95,-26,-0.27) | **Q6YHK3** (CD109;162,-25,-0.15) | **Q9BQ16** (SPOCK3;49,-25,-0.50) |
| **O75051** (PLXNA2;211,-26,-0.12) | **Q6DT37** (CDC42BPG;172,-25,-0.14) | **Q5VX71** (SUSD4;54,-25,-0.46) |
| **Q9NYI0** (PSD3;116,-26,-0.22) | **Q14CN2** (CLCA4;101,-25,-0.24) | **Q496J9** (SV2C;82,-25,-0.30) |
| **Q9H4I0** (RAD21L1;63,-26,-0.41) | **Q9Y5P4** (COL4A3BP;71,-25,-0.35) | **Q15542** (TAF5;87,-25,-0.28) |
| **Q15293** (RCN1;39,-26,-0.66) | **Q96BA8** (CREB3L1;57,-25,-0.43) | **Q8IWB9** (TEX2;125,-25,-0.19) |
| **A6NKT7** (RGPD3;197,-26,-0.13) | **P55060** (CSE1L;110,-25,-0.22) | **Q9UBB9** (TFIP11;97,-25,-0.25) |
| **Q5EBL4** (RILPL1;47,-26,-0.55) | **P35222** (CTNNB1;85,-25,-0.29) | **Q6PL24** (TMED8;36,-25,-0.69) |
| **O75116** (ROCK2;161,-26,-0.16) | **O94830** (DDHD2;81,-25,-0.30) | **Q9UPV9** (TRAK1;106,-25,-0.23) |
| **Q15459** (SF3A1;89,-26,-0.29) | **Q96HP0** (DOCK6;230,-25,-0.10) | **O15050** (TRANK1;336,-25,-0.07) |
| **Q15477** (SKIV2L;138,-26,-0.18) | **Q96MC2** (DRC1;87,-25,-0.28) | **P07437** (TUBB;50,-25,-0.50) |
| **Q9HBR0** (SLC38A10;120,-26,-0.21) | **O95967** (EFEMP2;49,-25,-0.50) | **Q13885** (TUBB2A;50,-25,-0.50) |
| **Q13509** (TUBB3;50,-25,-0.49) | **Q14444** (CAPRIN1;78,-24,-0.30) | **Q8WXD2** (SCG3;53,-24,-0.45) |
| **P04350** (TUBB4A;50,-25,-0.50) | **Q9BXL7** (CARD11;133,-24,-0.18) | **Q9NY46** (SCN3A;226,-24,-0.10) |
| **P68371** (TUBB4B;50,-25,-0.50) | **Q9BXL6** (CARD 14;1 13,-24,-0.21) | **Q9UHV2** (SERTAD1;25,-24,-0.97) |
| **P22314** (UBA1;118,-25,-0.21) | **Q96G28** (CCDC104;39,-24,-0.60) | **Q14140** (SERTAD2;34,-24,-0.70) |
| **Q9BSL1** (UBAC1;45,-25,-0.55) | **A1A5D9** (CCDC64B;57,-24,-0.42) | **Q9NRF2** (SH2B1;79,-24,-0.30) |
| **O94888** (UBXN7;55,-25,-0.45) | **O75794** (CDC123;39,-24,-0.61) | **Q8TF17** (SH3TC2;145,-24,-0.16) |
| **Q8NFA0** (USP32;182,-25,-0.13) | **O75419** (CDC45;66,-24,-0.36) | **Q9ULL8** (SHROOM4;165,-24,-0.14) |
| **O43298** (ZBTB43;53,-25,-0.47) | **Q03701** (CEBPZ;121,-24,-0.19) | **Q9BZZ2** (SIGLEC1;183,-24,-0.13) |
| **Q9BT43** (POLR3GL;25,-25,-0.98) | **Q92496** (CFHR4;65,-24,-0.36) | **Q2VWA4** (SKOR2;104,-24,-0.23) |
| **P26436** (ACRV1;28,-25,-0.88) | **Q96JB2** (COG3;94,-24,-0.25) | **P04920** (SLC4A2;137,-24,-0.17) |
| **P67936** (TPM4;29,-25,-0.87) | **Q8IZJ3** (CPAMD8;207,-24,-0.11) | **O00193** (SMAP;20,-24,-1.18) |
| **Q5TZF3** (ANKRD45;32,-25,-0.78) | **Q9NQ79** (CRTAC1;71,-24,-0.33) | **Q9UPR3** (SMG5;114,-24,-0.21) |
| **P57768** (SNX16;39,-25,-0.63) | **P32927** (CSF2RB;97,-24,-0.24) | **P43307** (SSR1;32,-24,-0.74) |
| **Q9H6Y2** (WDR55;42,-25,-0.59) | **Q66K89** (E4F1;83,-24,-0.28) | **Q15468** (STIL;143,-24,-0.16) |
| **Q81UF1** (CBWD2;44,-25,-0.56) | **Q9BSJ8** (ESYT1;123,-24,-0.19) | **Q17RD7** (SYT16;72,-24,-0.33) |
| **Q9BRT8** (CBWD1;44,-25,-0.56) | **Q6ZS17** (FAM65A;132,-24,-0.18) | **Q8IUC6** (TICAM1;76,-24,-0.31) |
| **Q9NQL2** (RRAGD;46,-25,-0.54) | **Q86WN1** (FCHSD1;77,-24,-0.31) | **O43897** (TLL1;115,-24,-0.20) |
| **Q3ZCM7** (TUBB8;50,-25,-0.50) | **Q14318** (FKBP8;45,-24,-0.53) | **Q96JJ7** (TMX3;52,-24,-0.46) |
| **Q9BUF5** (TUBB6;50,-25,-0.50) | **P36888** (FLT3;113,-24,-0.21) | **Q71U36** (TUBA 1A;50,-24,-0.47) |
| **Q9BVA1** (TUBB2B;50,-25,-0.50) | **Q9NQX3** (GPHN;80,-24,-0.30) | **P68366** (TUBA4A;50,-24,-0.48) |
| **Q8IYS4** (C16orf71;56,-25,-0.44) | **Q9C091** (GREB1L;214,-24,-0.11) | **Q9NY65** (TUBA8;50,-24,-0.47) |
| **P30154** (PPP2R1 B;66,-25,-0.37) | **Q99871** (HAUS7;41,-24,-0.58) | **A0AVT1** (UBA6;118,-24,-0.20) |
| **P16471** (PRLR;70,-25,-0.35) | **Q86XA9** (HEATR5A;222,-24,-0.10) | **Q14694** (USP10;87,-24,-0.27) |
| **Q969S8** (HDAC10;71,-25,-0.34) | **Q8IU57** (IFNLR1;58,-24,-0.41) | **Q5W0Q7** (USPL1;120,-24,-0.19) |
| **P21980** (TGM2;77,-25,-0.32) | **Q8N6C5** (IGSF1;149,-24,-0.16) | **Q86XK7** (VSIG1;42,-24,-0.57) |
| **Q99567** (NUP88;84,-25,-0.29) | **Q9ULD6** (INTU;106,-24,-0.22) | **Q9HAV4** (XPO5;136,-24,-0.17) |
| **P55072** (VCP;89,-25,-0.27) | **Q6NY19** (KANK3;88,-24,-0.27) | **Q14966** (ZNF638;221,-24,-0.10) |
| **Q6NUQ1** (RINT1;91,-25,-0.27) | **O76014** (KRT37;50,-24,-0.48) | **Q969E8** (TSR2;21,-24,-1.14) |
| **A8MT70** (ZBBX;91,-25,-0.27) | **P32004** (L1CAM;140,-24,-0.17) | **P68363** (TUBA1B;50,-24,-0.47) |
| **Q86XP0** (PLA2G4D;92,-25,-0.27) | **Q8IVL5** (LEPREL1;81,-24,-0.29) | **Q9UIR0** (BTNL2;50,-24,-0.47) |
| **Q9NSG2** (C1orf112;97,-25,-0.25) | **Q9HAR2** (LPHN3;162,-24,-0.14) | **Q9P0W5** (SCHIP1;53,-24,-0.44) |
| **P52630** (STAT2;98,-25,-0.25) | **Q9BTT6** (LRRC1;59,-24,-0.40) | **Q99456** (KRT12;54,-24,-0.44) |
| **Q12959** (DLG1;100,-25,-0.24) | **P43365** (MAGEA12;35,-24,-0.68) | **Q6ZN18** (AEBP2;54,-24,-0.44) |
| **P10253** (GAA;105,-25,-0.23) | **Q9HCI5** (MAGEE1;103,-24,-0.23) | **Q9HAU8** (RNPEPL1;56,-24,-0.43) |
| **Q9P107** (GMIP;107,-25,-0.23) | **Q86UL8** (MAGI2;159,-24,-0.15) | **Q9Y6I3** (EPN1;60,-24,-0.39) |
| **P49588** (AARS;107,-25,-0.23) | **O00339** (MATN2;107,-24,-0.22) | **P15391** (CD19;61,-24,-0.39) |
| **P12109** (COL6A1;109,-25,-0.23) | **Q9Y6F6** (MRVI1;96,-24,-0.24) | **Q8NEB7** (ACRBP;61,-24,-0.39) |
| **Q07075** (ENPEP;109,-25,-0.22) | **Q8NFW9** (MYRIP;96,-24,-0.25) | **O75553** (DAB1;64,-24,-0.37) |
| **P57678** (GEMIN4;120,-25,-0.20) | **Q99457** (NAP1L3;58,-24,-0.41) | **P30153** (PPP2R1A;65,-24,-0.36) |
| **Q8IZS8** (CACNA2D3;123,-25,-0.20) | **Q8TD19** (NEK9;107,-24,-0.22) | **O94769** (ECM2;80,-24,-0.30) |
| **Q13349** (ITGAD;127,-25,-0.19) | **P06748** (NPM1;33,-24,-0.73) | **P50747** (HLCS;81,-24,-0.29) |
| **Q9Y6N6** (LAMC3;171,-25,-0.14) | **O75459** (PAGE1;16,-24,-1.48) | **Q9C0B2** (KIAA1751;87,-24,-0.27) |
| **Q7Z5N4** (SDK1;242,-25,-0.10) | **Q9Y5H7** (PCDHA5;102,-24,-0.23) | **Q68DD2** (PLA2G4F;95,-24,-0.25) |
| **P04114** (APOB;516,-25,-0.04) | **A6NKB5** (PCNXL2;237,-24,-0.10) | **O95206** (PCDH8;113,-24,-0.21) |
| **O00468** (AGRN;217,-24,-0.11) | **Q13371** (PDCL;34,-24,-0.70) | **P0CG38** (POTEI;121,-24,-0.19) |
| **Q9UPW5** (AGTPBP1;138,-24,-0.17) | **P51160** (PDE6C;99,-24,-0.24) | **P42702** (LIFR;124,-24,-0.19) |
| **P15144** (ANPEP;110,-24,-0.21) | **Q9Y2I7** (PIKFYVE;237,-24,-0.10) | **Q16720** (ATP2B3;134,-24,-0.17) |
| **Q8NFD5** (ARID1B;236,-24,-0.10) | **O60331** (PIP5K1C;73,-24,-0.32) | **P29144** (TPP2;138,-24,-0.17) |
| **Q16515** (ASIC2;58,-24,-0.41) | **Q00G26** (PLIN5;51,-24,-0.47) | **Q9UPP5** (KIAA1107; 156,-24,-0.15) |
| **Q8IXJ9** (ASXL1;165,-24,-0.14) | **Q13136** (PPFIA1;136,-24,-0.17) | **Q8TEQ6** (GEMIN5;169,-24,-0.14) |
| **Q04656** (ATP7A;163,-24,-0.14) | **Q96LW4** (PRIMPOL;64,-24,-0.37) | **Q9H9F9** (ACTR5;68,-23,-0.33) |
| **Q9H3M9** (ATXN3L;41,-24,-0.58) | **O43422** (PRKRIR;88,-24,-0.27) | **Q9UJX5** (ANAPC4;92,-23,-0.24) |
| **Q14457** (BECN1;52,-24,-0.46) | **O60678** (PRMT3;60,-24,-0.40) | **P13798** (APEH;81,-23,-0.28) |
| **Q8NDZ0** (BEND2;88,-24,-0.27) | **P55036** (PSMD4;41,-24,-0.58) | **Q6PL18** (ATAD2;159,-23,-0.14) |
| **Q13137** (CALCOCO2;52,-24,-0.45) | **P10586** (PTPRF;213,-24,-0.11) | **Q9UIF8** (BAZ2B;240,-23,-0.09) |
| **P62158** (CALM1;17,-24,-1.42) | **Q9BWH6** (RPAP1;153,-24,-0.15) | **Q6W2J9** (BCOR;192,-23,-0.11) |
| **A6NHC0** (CAPN8;79,-24,-0.30) | **Q15020** (SART3;110,-24,-0.21) | **A8MW95** (BECN1P1;48,-23,-0.47) |
| **Q86YS7** (C2CD5;110,-23,-0.20) | **Q99592** (ZBTB18;58,-23,-0.39) | **P78352** (DLG4;80,-22,-0.27) |
| **Q86UW7** (CADPS2;148,-23,-0.15) | **Q9NRM2** (ZNF277;53,-23,-0.43) | **Q9BU89** (DOHH;33,-22,-0.66) |
| **Q8IX12** (CCAR1;133,-23,-0.17) | **Q2M1K9** (ZNF423;145,-23,-0.15) | **Q7Z2Z2** (EFTUD1;125,-22,-0.17) |
| **O95273** (CCNDBP1;40,-23,-0.57) | **P36508** (ZNF76;62,-23,-0.37) | **Q99645** (EPYC;37,-22,-0.60) |
| **A6H8M9** (CDHR4;86,-23,-0.26) | **O15318** (POLR3G;26,-23,-0.88) | **P21860** (ERBB3;148,-22,-0.14) |
| **Q4KMG0** (CDON;139,-23,-0.16) | **P09496** (CLTA;27,-23,-0.84) | **Q9UBX5** (FBLN5;50,-22,-0.43) |
| **Q8TEP8** (CEP192;213,-23,-0.10) | **Q9H063** (MAF1;29,-23,-0.79) | **Q5VW36** (FOCAD;200,-22,-0.10) |
| **Q9UEE9** (CFDP1;34,-23,-0.68) | **P45379** (TNNT2;36,-23,-0.64) | **Q8TDT2** (GPR152;51,-22,-0.43) |
| **Q10570** (CPSF1;161,-23,-0.14) | **P0DJD7** (PGA4;42,-23,-0.54) | **P57764** (GSDMD;53,-22,-0.41) |
| **Q14247** (CTTN;62,-23,-0.37) | **Q8WVN8** (UBE2Q2;43,-23,-0.53) | **P51858** (HDGF;27,-22,-0.82) |
| **Q96QB1** (DLC1;171,-23,-0.13) | **O95393** (BMP10;48,-23,-0.47) | **Q9BQA5** (HINFP;60,-22,-0.36) |
| **Q01094** (E2F1;47,-23,-0.49) | **Q969R8** (ITFG2;49,-23,-0.46) | **Q00613** (HSF1;57,-22,-0.38) |
| **Q96EK7** (FAM120B;104,-23,-0.22) | **Q49A92** (C8orf34;50,-23,-0.45) | **P11021** (HSPA5;72,-22,-0.30) |
| **O15360** (FANCA;163,-23,-0.14) | **Q15846** (CLUL1;54,-23,-0.42) | **O75054** (IGSF3; 135,-22,-0.16) |
| **Q6PIJ6** (FBXO38;134,-23,-0.17) | **P16389** (KCNA2;57,-23,-0.40) | **Q13651** (IL10RA;63,-22,-0.34) |
| **Q13045** (FLII;145,-23,-0.15) | **Q14181** (POLA2;66,-23,-0.34) | **Q5VZ66** (JAKMIP3;99,-22,-0.22) |
| **Q06546** (GABPA;51,-23,-0.44) | **P52306** (RAP1 GDS1 ;66,-23,-0.34) | **Q8NAB2** (KBTBD3;69,-22,-0.31) |
| **Q14697** (GANAB;107,-23,-0.21) | **P07911** (UMOD;70,-23,-0.32) | **Q9BQK8** (LPIN3;94,-22,-0.23) |
| **Q9BTY7** (HGH1;42,-23,-0.54) | **Q9H347** (UBQLN3;71,-23,-0.32) | **Q6UXM1** (LRIG3;123,-22,-0.17) |
| **Q8WZA9** (IRGQ;63,-23,-0.36) | **A0MZ66** (KIAA1598;72,-23,-0.32) | **O60449** (LY75;198,-22,-0.11) |
| **P78415** (IRX3;52,-23,-0.44) | **Q8N309** (LRRC43;73,-23,-0.31) | **P80192** (MAP3K9;122,-22,-0.18) |
| **P08648** (ITGA5;115,-23,-0.20) | **Q7Z4Q2** (HEATR3;75,-23,-0.30) | **Q9UHC7** (MKRN1;53,-22,-0.41) |
| **P05106** (ITGB3;87,-23,-0.26) | **P42898** (MTHFR;75,-23,-0.30) | **Q96JB8** (MPP4;73,-22,-0.30) |
| **Q4ADV7** (KIAA1432;159,-23,-0.14) | **Q8IYI6** (EXOC8;82,-23,-0.28) | **Q04912** (MST1R;152,-22,-0.14) |
| **Q96L93** (KIF16B;152,-23,-0.15) | **Q16819** (MEP1A;84,-23,-0.27) | **Q8N3F0** (MTURN;15,-22,-1.47) |
| **Q2TBA0** (KLHL40;69,-23,-0.33) | **Q99523** (SORT1;92,-23,-0.24) | **Q5WJ2** (MYSM1;95,-22,-0.23) |
| **Q8IWV1** (LAX1;44,-23,-0.52) | **Q06033** (ITIH3;100,-23,-0.23) | **Q9BW27** (NUP85;75,-22,-0.29) |
| **P20700** (LMNB1;66,-23,-0.34) | **Q4G0T1** (0;109,-23,-0.21) | **Q8WWZ8** (OIT3;60,-22,-0.36) |
| **P31152** (MAPK4;66,-23,-0.34) | **P05023** (ATP1A1;113,-23,-0.20) | **Q99983** (OMD;49,-22,-0.44) |
| **P55196** (MLLT4;207,-23,-0.11) | **Q8N961** (ABTB2;114,-23,-0.20) | **Q9BYE7** (PCGF6;39,-22,-0.56) |
| **Q7Z745** (MROH2B;181,-23,-0.12) | **Q8N0W3** (FUK;118,-23,-0.19) | **P46019** (PHKA2;138,-22,-0.15) |
| **Q8N987** (NECAB1;41,-23,-0.56) | **P27816** (MAP4;121,-23,-0.19) | **Q9P215** (POGK;69,-22,-0.31) |
| **Q86XW9** (NME9;37,-23,-0.62) | **Q5DID0** (UMODL1;144,-23,-0.15) | **O60237** (PPP1R12B;110,-22,-0.19) |
| **O14786** (NRP1;103,-23,-0.22) | **P31629** (HIVEP2;269,-23,-0.08) | **Q15435** (PPP1R7;42,-22,-0.52) |
| **P16435** (POR;77,-23,-0.29) | **Q13315** (ATM;351,-23,-0.06) | **Q9P1A2** (PPP4R1L;46,-22,-0.47) |
| **P49593** (PPM1F;50,-23,-0.46) | **P01023** (A2M;163,-22,-0.13) | **P55345** (PRMT2;49,-22,-0.44) |
| **Q3YEC7** (RABL6;80,-23,-0.28) | **P78536** (ADAM17;93,-22,-0.23) | **Q96NY8** (PVRL4;55,-22,-0.39) |
| **Q15291** (RBBP5;59,-23,-0.38) | **P24588** (AKAP5;47,-22,-0.46) | **Q99666** (RGPD5;199,-22,-0.11) |
| **O14593** (RFXANK;28,-23,-0.81) | **Q6ZW76** (ANKS3;72,-22,-0.30) | **Q9BQY4** (RHOXF2;32,-22,-0.69) |
| **Q96BU1** (S100PBP;46,-23,-0.50) | **Q8N8V4** (ANKS4B;47,-22,-0.47) | **Q96TC7** (RMDN3;52,-22,-0.42) |
| **Q14151** (SAFB2;107,-23,-0.21) | **Q9UKG1** (APPL1;80,-22,-0.27) | **P13489** (RNH1;50,-22,-0.44) |
| **P18827** (SDC1;32,-23,-0.70) | **O75143** (ATG13;57,-22,-0.38) | **Q96T51** (RUFY1;80,-22,-0.27) |
| **Q86SQ7** (SDCCAG8;83,-23,-0.27) | **P46100** (ATRX;283,-22,-0.07) | **Q9NZJ4** (SACS;521,-22,-0.04) |
| **Q9UGP8** (SEC63;88,-23,-0.26) | **P54252** (ATXN3;42,-22,-0.52) | **Q9Y467** (SALL2;105,-22,-0.20) |
| **Q9Y2K2** (SIK3;140,-23,-0.16) | **O75363** (BCAS1;62,-22,-0.35) | **Q12874** (SF3A3;59,-22,-0.37) |
| **Q9UMX1** (SUFU;54,-23,-0.42) | **Q9NSY1** (BMP2K;129,-22,-0.17) | **Q13435** (SF3B2;100,-22,-0.21) |
| **O94864** (SUPT7L;46,-23,-0.49) | **Q9H8G2** (CAAP1;38,-22,-0.57) | **P60896** (SHFM1;8,-22,-2.65) |
| **Q9C0B7** (TANGO6;121,-23,-0.19) | **Q9NY47** (CACNA2D2;130,-22,-0.16) | **Q8N196** (SIX5;75,-22,-0.29) |
| **Q9BTW9** (TBCD;133,-23,-0.17) | **O14815** (CAPN9;79,-22,-0.27) | **P48751** (SLC4A3;136,-22,-0.16) |
| **Q7Z6L1** (TECPR1;130,-23,-0.17) | **Q9Y2G2** (CARD8;49,-22,-0.44) | **O60749** (SNX2;58,-22,-0.37) |
| **Q07157** (TJP1;195,-23,-0.11) | **Q13042** (CDC16;72,-22,-0.30) | **Q13043** (STK4;56,-22,-0.39) |
| **Q969Q1** (TRIM63;40,-23,-0.57) | **Q96JP9** (CDHR1;94,-22,-0.23) | **Q4ZIN3** (TMEM259;68,-22,-0.32) |
| **Q9BQE3** (TUBA1C;50,-23,-0.46) | **Q8N4Q1** (CHCHD4;16,-22,-1.37) | **Q96A56** (TP53INP1;27,-22,-0.80) |
| **Q13748** (TUBA3C;50,-23,-0.46) | **Q96HD1** (CRELD1;45,-22,-0.48) | **O75157** (TSC22D2;79,-22,-0.27) |
| **Q8TEY7** (USP33;107,-23,-0.21) | **Q9NQC7** (CYLD;107,-22,-0.20) | **Q96J42** (TXNDC15;40,-22,-0.55) |
| **Q8NEZ3** (WDR19;152,-23,-0.15) | **Q9UJU6** (DBNL;48,-22,-0.45) | **Q9UBT2** (UBA2;71,-22,-0.30) |
| **P49750** (YLPM1;220,-23,-0.10) | **Q68CQ4** (DIEXF;87,-22,-0.25) | **Q9GZZ9** (UBA5;45,-22,-0.49) |
| **Q9NZ09** (UBAP1;55,-22,-0.39) | **Q5ZPR3** (CD276;57,-21,-0.36) | **O94885** (SASH1;137,-21,-0.15) |
| **Q6UXZ4** (UNC5D;106,-22,-0.20) | **P49427** (CDC34;27,-21,-0.78) | **O60239** (SH3BP5;50,-21,-0.41) |
| **O94966** (USP19;146,-22,-0.15) | **Q5VT25** (CDC42BPA;197,-21,-0.10) | **Q15043** (SLC39A14;54,-21,-0.38) |
| **Q9Y2K6** (USP20;102,-22,-0.21) | **Q6P2H3** (CEP85;86,-21,-0.24) | **Q5BKX6** (SLC45A4;84,-21,-0.25) |
| **Q9Y334** (VWA7;96,-22,-0.22) | **O76071** (CIAO1;38,-21,-0.55) | **Q92966** (SNAPC3;47,-21,-0.44) |
| **Q96SY0** (VWA9;57,-22,-0.38) | **P35523** (CLCN1;109,-21,-0.19) | **Q13501** (SQSTM1;48,-21,-0.44) |
| **Q14191** (WRN;162,-22,-0.13) | **Q96KN2** (CNDP1;57,-21,-0.37) | **Q92502** (STARD8;113,-21,-0.18) |
| **Q13105** (ZBTB17;88,-22,-0.25) | **O43889** (CREB3;44,-21,-0.47) | **Q13188** (STK3;56,-21,-0.37) |
| **O15156** (ZBTB7B;58,-22,-0.37) | **Q8IUI8** (CRLF3;50,-21,-0.42) | **Q7L1I2** (SV2B;77,-21,-0.27) |
| **Q9H4I2** (ZHX3;105,-22,-0.21) | **Q8IYB7** (DIS3L2;99,-21,-0.21) | **Q96GX1** (TCTN2;77,-21,-0.27) |
| **P17040** (ZSCAN20;118,-22,-0.18) | **O75165** (DNAJC13;254,-21,-0.08) | **Q13263** (TRIM28;89,-21,-0.23) |
| **Q8N402** (0;27,-22,-0.81) | **Q96N67** (DOCK7;243,-21,-0.08) | **A6NGJ6** (TRIM64;52,-21,-0.40) |
| **P0C7M4** (RHOXF2B;32,-22,-0.69) | **Q86YF9** (DZIP1;99,-21,-0.21) | **Q99614** (TTC1;34,-21,-0.62) |
| **Q9UN42** (ATP1B4;42,-22,-0.52) | **O43281** (EFS;59,-21,-0.35) | **Q96AY4** (TTC28;271,-21,-0.07) |
| **P0DJD9** (PGA5;42,-22,-0.52) | **Q8TE02** (ELP5;35,-21,-0.60) | **Q96RL1** (UIMC1;80,-21,-0.26) |
| **Q16254** (E2F4;44,-22,-0.50) | **Q96DN0** (ERP27;30,-21,-0.68) | **Q86UV5** (USP48;119,-21,-0.17) |
| **P31323** (PRKAR2B;46,-22,-0.47) | **Q9BS26** (ERP44;47,-21,-0.44) | **Q7Z7L7** (ZER1;88,-21,-0.23) |
| **O60268** (KIAA0513;47,-22,-0.47) | **Q96AQ9** (FAM131C;30,-21,-0.69) | **Q9Y4E5** (ZNF451;121,-21,-0.17) |
| **Q9NP70** (AMBN;48,-22,-0.45) | **A6NMN3** (FAM170B;32,-21,-0.65) | **Q96IT1** (ZNF496;67,-21,-0.31) |
| **Q6PEY2** (TUBA3E;50,-22,-0.44) | **Q8N128** (FAM177A1;24,-21,-0.88) | **Q9H9R9** (DBNDD1;17,-21,-1.23) |
| **Q86VR2** (FAM134C;51,-22,-0.42) | **Q96AY3** (FKBP10;64,-21,-0.32) | **Q8IVU9** (C10orf107;24,-21,-0.87) |
| **Q16656** (NRF1;54,-22,-0.41) | **Q8N475** (FSTL5;96,-21,-0.21) | **P17677** (GAP43;25,-21,-0.84) |
| **Q9UNF0** (PACSIN2;56,-22,-0.39) | **Q8IYD1** (GSPT2;69,-21,-0.30) | **Q6ZRP0** (PRR23C;28,-21,-0.75) |
| **Q8N4U5** (TCP11L2;58,-22,-0.37) | **O00165** (HAX1;32,-21,-0.66) | **Q9NZQ9** (TMOD4;39,-21,-0.53) |
| **Q9Y3X0** (CCDC9;60,-22,-0.36) | **Q9ULI3** (HEG1;147,-21,-0.14) | **Q4G1C9** (GLIPR1L2;40,-21,-0.52) |
| **Q9NPQ8** (RIC8A;60,-22,-0.36) | **Q9H0H0** (INTS2;134,-21,-0.15) | **O75381** (PEX14;41,-21,-0.50) |
| **P14784** (IL2RB;61,-22,-0.35) | **Q63ZY3** (KANK2;91,-21,-0.23) | **P0DJD8** (PGA3;42,-21,-0.50) |
| **A2RU67** (KIAA1467;67,-22,-0.32) | **Q92806** (KCNJ9;44,-21,-0.47) | **Q8NFH3** (NUP43;42,-21,-0.49) |
| **P51693** (APLP1;72,-22,-0.30) | **Q7Z3Y8** (KRT27;50,-21,-0.42) | **Q81XS6** (PALM2;42,-21,-0.49) |
| **O00187** (MASP2;76,-22,-0.29) | **Q14532** (KRT32;50,-21,-0.41) | **Q9HB90** (RRAGC;44,-21,-0.47) |
| **Q96MT8** (CEP63;81,-22,-0.27) | **O95751** (LDOC1;17,-21,-1.23) | **P49354** (FNTA;44,-21,-0.47) |
| **Q8TBP0** (TBC1 D16;86,-22,-0.25) | **Q15334** (LLGL1;115,-21,-0.18) | **O95810** (SDPR;47,-21,-0.44) |
| **Q96D71** (REPS1;87,-22,-0.25) | **P42704** (LRPPRC;158,-21,-0.13) | **Q9H4B7** (TUBB1;50,-21,-0.41) |
| **P26010** (ITGB7;87,-22,-0.25) | **P43358** (MAGEA4;35,-21,-0.60) | **P16870** (CPE;53,-21,-0.39) |
| **Q8IXH8** (CDH26;95,-22,-0.23) | **P43361** (MAGEA8;35,-21,-0.59) | **Q8WWU5** (TCP11;56,-21,-0.37) |
| **P52789** (HK2;102,-22,-0.21) | **Q6ZSS7** (MFSD6;88,-21,-0.23) | **Q13596** (SNX1;59,-21,-0.35) |
| **Q8TET4** (GANC;104,-22,-0.21) | **P40692** (MLH1;85,-21,-0.24) | **Q8N239** (KLHL34;71,-21,-0.29) |
| **Q6ZVH7** (ESPNL;108,-22,-0.20) | **Q99550** (MPHOSPH9;133,-21,-0.15) | **Q8N9W4** (GOLGA6L2;79,-21,-0.26) |
| **Q13563** (PKD2;110,-22,-0.20) | **Q9UQQ1** (NAALADL1;81,-21,-0.26) | **Q0ZGT2** (NEXN;81,-21,-0.26) |
| **Q6ZN30** (BNC2;122,-22,-0.17) | **Q5TAG4** (NBPF12;31,-21,-0.67) | **Q8N5V2** (NGEF;82,-21,-0.25) |
| **Q15111** (PLCL1;123,-22,-0.17) | **Q92859** (NE01;160,-21,-0.13) | **Q9UMQ6** (CAPN11;84,-21,-0.24) |
| **Q96AE7** (TTC17;130,-22,-0.16) | **Q9NX02** (NLRP2;121,-21,-0.17) | **P0C869** (PLA2G4B;88,-21,-0.23) |
| **Q96C45** (ULK4;142,-22,-0.15) | **P55786** (NPEPPS;103,-21,-0.20) | **P57737** (CORO7;101,-21,-0.20) |
| **O43933** (PEX1;143,-22,-0.15) | **Q9ULR5** (PAIP2B;14,-21,-1.47) | **P40189** (IL6ST;104,-21,-0.20) |
| **Q8IZU2** (WDR17;148,-22,-0.14) | **Q01064** (PDE1B;61,-21,-0.34) | **Q9HBJ7** (USP29;104,-21,-0.20) |
| **Q9BXX3** (ANKRD30A;159,-22,-0.13) | **O76074** (PDE5A;100,-21,-0.21) | **Q93084** (ATP2A3;114,-21,-0.18) |
| **Q13009** (TIAM1;178,-22,-0.12) | **O15055** (PER2;137,-21,-0.15) | **Q13797** (ITGA9;114,-21,-0.18) |
| **Q02388** (COL7A1;295,-22,-0.07) | **Q8IYB4** (PEX5L;70,-21,-0.30) | **Q711Q0** (C10orf71;156,-21,-0.13) |
| **Q9H799** (C5orf42;362,-22,-0.06) | **Q96JA3** (PLEKHA8;58,-21,-0.36) | **Q9H0J4** (QRICH2;181,-21,-0.11) |
| **Q5T8D3** (ACBD5;60,-21,-0.34) | **Q81UK5** (PLXDC1;56,-21,-0.37) | **Q9H6A9** (PCNXL3;222,-21,-0.09) |
| **Q9BYF1** (ACE2;92,-21,-0.22) | **Q9UL42** (PNMA2;42,-21,-0.50) | **Q63HQ0** (AP1AR;34,-20,-0.58) |
| **O95376** (ARIH2;58,-21,-0.36) | **P48147** (PREP;81,-21,-0.26) | **Q13017** (ARHGAP5;172,-20,-0.11) |
| **Q8NHH9** (ATL2;66,-21,-0.31) | **P41743** (PRKCI;68,-21,-0.30) | **Q9Y2Y0** (ARL2BP;19,-20,-1.06) |
| **O94812** (BAIAP3;132,-21,-0.15) | **P07602** (PSAP;58,-21,-0.36) | **P20020** (ATP2B1;139,-20,-0.14) |
| **Q8N9N5** (BANP;56,-21,-0.37) | **Q13905** (RAPGEF1;121,-21,-0.17) | **Q14692** (BMS1;146,-20,-0.13) |
| **Q9UBW5** (BIN2;62,-21,-0.33) | **Q9UKA8** (RCAN3;27,-21,-0.76) | **Q96Q07** (BTBD9;69,-20,-0.28) |
| **P35219** (CA8;33,-21,-0.63) | **O43567** (RNF13;43,-21,-0.49) | **O14936** (CASK;105,-20,-0.19) |
| **Q14790** (CASP8;55,-20,-0.36) | **O75056** (SDC3;45,-20,-0.43) | **O60337** (MARCH6;103,-19,-0.18) |
| **P41180** (CASR;121,-20,-0.16) | **Q99574** (SERPINI1;46,-20,-0.43) | **Q969K4** (ABTB1;54,-19,-0.35) |
| **Q9ULG6** (CCPG1;87,-20,-0.22) | **Q9Y6P5** (SESN1;57,-20,-0.35) | **Q9GZN1** (ACTR6;46,-19,-0.41) |
| **Q6ZTQ4** (CDHR3;98,-20,-0.20) | **Q96HU1** (SGSM3;85,-20,-0.23) | **Q8WYP5** (AHCTF1;252,-19,-0.07) |
| **Q70SY1** (CREB3L2;57,-20,-0.34) | **Q8N1H7** (SIX6OS1;68,-20,-0.29) | **O75891** (ALDH1L1;99,-19,-0.19) |
| **Q99062** (CSF3R;92,-20,-0.21) | **Q6U841** (SLC4A10;126,-20,-0.15) | **Q8N6M6** (AOPEP;94,-19,-0.20) |
| **Q9BQY9** (DBNDD2;28,-20,-0.72) | **Q8TBB6** (SLC7A14;84,-20,-0.23) | **Q2VPB7** (AP5B1;94,-19,-0.20) |
| **O00273** (DFFA;37,-20,-0.54) | **A6NFE2** (SMCC2;39,-20,-0.50) | **Q9Y294** (ASF1A;23,-19,-0.82) |
| **Q0VDD8** (DNAH14;400,-20,-0.05) | **Q9BXP5** (SRRT;101,-20,-0.19) | **Q9Y4P1** (ATG4B;44,-19,-0.42) |
| **Q9BV94** (EDEM2;65,-20,-0.30) | **Q8NFI4** (ST13P5;41,-20,-0.48) | **Q01814** (ATP2B2;137,-19,-0.13) |
| **Q3B7T1** (EDRF1;139,-20,-0.14) | **Q5T4T6** (SYCP2L;94,-20,-0.21) | **P61421** (ATP6V0D1;40,-19,-0.47) |
| **Q9HC35** (EML4;109,-20,-0.18) | **Q15545** (TAF7;40,-20,-0.49) | **P80723** (BASP1;23,-19,-0.83) |
| **Q8TC92** (ENOX1;73,-20,-0.27) | **Q9BQ87** (TBL1Y;57,-20,-0.35) | **Q13873** (BMPR2;115,-19,-0.16) |
| **Q86VI1** (EXOC3L1;82,-20,-0.24) | **O94842** (TOX4;66,-20,-0.30) | **Q12982** (BNIP2;36,-19,-0.52) |
| **A1A519** (FAM170A;37,-20,-0.53) | **O95361** (TRIM16;64,-20,-0.31) | **Q96KV6** (BTN2A3P;66,-19,-0.28) |
| **Q9ULE4** (FAM184B;121,-20,-0.16) | **Q8NB14** (USP38;117,-20,-0.17) | **O43683** (BUB1;122,-19,-0.15) |
| **Q6UN15** (FIP1L1;67,-20,-0.30) | **P19320** (VCAM1;81,-20,-0.24) | **Q81WF9** (CCDC83;49,-19,-0.38) |
| **Q92949** (FOXJ1;45,-20,-0.44) | **Q9NNX9** (VCX3A;20,-20,-0.99) | **Q9BSQ5** (CCM2;49,-19,-0.38) |
| **Q0D2H9** (GOLGA8DP;48,-20,-0.41) | **Q8IWA0** (WDR75;94,-20,-0.21) | **Q81X05** (CD302;26,-19,-0.72) |
| **Q08AF8** (GOLGA8F;;48,-20,-0.41) | **Q9NQW7** (XPNPEP1;70,-20,-0.28) | **Q9BS16** (CENPK;32,-19,-0.60) |
| **Q9NPB8** (GPCPD1;76,-20,-0.26) | **P25490** (YY1;45,-20,-0.44) | **Q6ZU80** (CEP128;128,-19,-0.14) |
| **Q9HCN4** (GPN1;42,-20,-0.47) | **Q8TF50** (ZNF526;74,-20,-0.27) | **Q13111** (CHAF1A;107,-19,-0.17) |
| **Q86UP8** (GTF21RD2;107,-20,-0.18) | **A7E2V4** (ZSWIM8;197,-20,-0.10) | **Q9UHC6** (CNTNAP2;148,-19,-0.12) |
| **Q15034** (HERC3;117,-20,-0.17) | **Q5MJ09** (SPANXN3;16,-20,-1.28) | **P53618** (COPB1;107,-19,-0.17) |
| **Q5GLZ8** (HERC4;119,-20,-0.16) | **Q9HCU9** (BRMS1;28,-20,-0.70) | **Q8N6G5** (CSGALNACT2;63,-19,-0.30) |
| **O14879** (IFIT3;56,-20,-0.35) | **P06870** (KLK1;29,-20,-0.69) | |
| **P14616** (INSRR;144,-20,-0.13) | **I3L3R5** (CCER2;30,-20,-0.65) | **P39880** (CUX1;164,-19,-0.11) |
| **Q8TB96** (ITFG1;68,-20,-0.29) | **Q7Z6L0** (PRRT2;35,-20,-0.57) | **O75398** (DEAF1;59,-19,-0.32) |
| **Q9UKP3** (ITGB1BP2;38,-20,-0.52) | **Q86YD5** (LDLRAD3;37,-20,-0.53) | **Q5F1R6** (DNAJC21;62,-19,-0.30) |
| **Q7Z5Y7** (KCTD20;47,-20,-0.42) | **Q5PSV4** (BRMS1L;38,-20,-0.53) | **Q86TI2** (DPP9;98,-19,-0.19) |
| **O94889** (KLHL18;64,-20,-0.31) | **Q53T59** (HS1BP3;43,-20,-0.46) | **Q9HA90** (EFCC1;66,-19,-0.28) |
| **Q9UJP4** (KLHL21;67,-20,-0.30) | **Q9UNH6** (SNX7;45,-20,-0.44) | **Q9UHY7** (ENOPH1;29,-19,-0.65) |
| **O76009** (KRT33A;46,-20,-0.43) | **Q96C92** (SDCCAG3;48,-20,-0.41) | **Q15303** (ERBB4;147,-19,-0.12) |
| **O76013** (KRT36;52,-20,-0.38) | **Q8TAK5** (GABPB2;49,-20,-0.41) | **O75460** (ERN1;110,-19,-0.17) |
| **O43283** (MAP3K13;108,-20,-0.18) | **P35557** (GCK;52,-20,-0.38) | **Q8N3Y1** (FBXW8;67,-19,-0.28) |
| **Q8IWI9** (MGA;332,-20,-0.06) | **P45452** (MMP13;54,-20,-0.37) | **O94868** (FCHSD2;84,-19,-0.22) |
| **Q13434** (MKRN4P;53,-20,-0.37) | **Q16322** (KCNA10;58,-20,-0.34) | **P22607** (FGFR3;88,-19,-0.21) |
| **A6NES4** (MROH2A;193,-20,-0.10) | **P55259** (GP2;59,-20,-0.33) | **O43524** (FOXO3;71,-19,-0.26) |
| **O15394** (NCAM2;93,-20,-0.21) | **Q9Y6K8** (AK5;63,-20,-0.31) | **Q6MZW2** (FSTL4;93,-19,-0.20) |
| **Q14934** (NFATC4;95,-20,-0.20) | **Q5TAA0** (TTC22;63,-20,-0.31) | **Q9H8Y8** (GORASP2;47,-19,-0.40) |
| **Q00653** (NFKB2;97,-20,-0.20) | **Q8WXK1** (ASB15;66,-20,-0.30) | **Q4ZG55** (GREB1;216,-19,-0.08) |
| **Q8N2Q7** (NLGN1;94,-20,-0.21) | **Q6TGC4** (PADI6;78,-20,-0.25) | **P43080** (GUCA1A;23,-19,-0.82) |
| **Q8NFZ3** (NLGN4Y;92,-20,-0.21) | **O94782** (USP1;88,-20,-0.22) | **Q13547** (HDAC1;55,-19,-0.34) |
| **Q96SU4** (OSBPL9;83,-20,-0.24) | **O75366** (AVIL;92,-20,-0.21) | **Q58FF3** (HSP90B2P;46,-19,-0.41) |
| **Q9Y5B6** (PAXBP1;105,-20,-0.19) | **Q6P3X3** (TTC27;97,-20,-0.20) | **P22304** (IDS;62,-19,-0.30) |
| **Q6UXB8** (PI16;49,-20,-0.40) | **Q9UBF2** (COPG2;98,-20,-0.20) | **O14498** (ISLR;46,-19,-0.41) |
| **Q9P0L9** (PKD2L1;92,-20,-0.21) | **P19021** (PAM;108,-20,-0.18) | **P06756** (ITGAV;116,-19,-0.16) |
| **Q13401** (PMS2P3;19,-20,-1.06) | **Q92622** (KIAA0226;109,-20,-0.18) | **P05556** (ITGB1;88,-19,-0.21) |
| **P61218** (POLR2F;14,-20,-1.38) | **Q8N283** (ANKRD35;110,-20,-0.18) | **Q9Y6Y0** (IVNS1ABP;72,-19,-0.26) |
| **Q9BZL4** (PPP1R12C;85,-20,-0.23) | **Q8TBY8** (PMFBP1;119,-20,-0.16) | **Q9NVX7** (KBTBD4;58,-19,-0.32) |
| **Q9NQV6** (PRDM10;130,-20,-0.15) | **P49796** (RGS3;132,-20,-0.15) | **Q7Z4S6** (KIF21A;187,-19,-0.10) |
| **Q9Y6C5** (PTCH2;131,-20,-0.15) | **Q10571** (MN1;136,-20,-0.14) | **Q53G59** (KLHL12;63,-19,-0.30) |
| **Q15185** (PTGES3;19,-20,-1.06) | **O14841** (OPLAH;137,-20,-0.14) | **P52294** (KPNA1;60,-19,-0.31) |
| **Q15269** (PWP2;102,-20,-0.19) | **Q01970** (PLCB3;139,-20,-0.14) | **O60684** (KPNA6;60,-19,-0.31) |
| **Q9P0K7** (RAI14;110,-20,-0.18) | **Q5VTT5** (MYOM3;162,-20,-0.12) | **Q7Z3Z0** (KRT25;49,-19,-0.38) |
| **P78332** (RBM6;129,-20,-0.15) | **P23468** (PTPRD;215,-20,-0.09) | **Q14525** (KRT33B;46,-19,-0.41) |
| **O14715** (RGPD8;199, -20, -0.10) | **Q13332** (PTPRS;217,-20,-0.09) | **O15230** (LAMA5;400,-19,-0.04) |
| **Q13753** (LAMC2;131,-19,-0.14) | **P17022** (ZNF18;62,-19,-0.30) | **Q07021** (C1QBP;31,-18,-0.57) |
| **Q03252** (LMNB2;68,-19,-0.28) | **P27482** (CALML3;17,-19,-1.12) | **P01024** (C3;187,-18,-0.09) |
| **Q7Z4F1** (LRP10;76,-19,-0.24) | **Q99750** (MDFI;25,-19,-0.75) | **Q8TD86** (CALML6;21,-18,-0.86) |
| **P10911** (MCF2;108,-19,-0.17) | **A6NLC5** (C3orf70;28,-19,-0.68) | **Q8N187** (CARF;81,-18,-0.22) |
| **Q86YR7** (MCF2L2;127,-19,-0.14) | **P62258** (YWHAE;29,-19,-0.65) | **Q8TBZ0** (CCDC110;97,-18,-0.18) |
| **Q99687** (MEIS3;41,-19,-0.46) | **B4DZS4** (TCP11X1;35,-19,-0.54) | **Q16543** (CDC37;44,-18,-0.40) |
| **Q96T76** (MMS19;113,-19,-0.16) | **Q8N5Z5** (KCTD17;36,-19,-0.53) | **Q3L8U1** (CHD9;326,-18,-0.05) |
| **Q96JA4** (MS4A14;77,-19,-0.24) | **Q96IW2** (SHD;38,-19,-0.49) | **Q9Y240** (CLEC11A;36,-18,-0.50) |
| **Q32MK0** (MYLK3;88,-19,-0.21) | **Q13426** (XRCC4;38,-19,-0.49) | **Q9UFF9** (CNOT8;34,-18,-0.53) |
| **Q13562** (NEUROD1;40,-19,-0.47) | **A8K0S8** (MEIS3P2;39,-19,-0.48) | **Q68CJ9** (CREB3L3;49,-18,-0.36) |
| **Q8N0W4** (NLGN4X;92,-19,-0.20) | **Q9BRP1** (PDCD2L;39,-19,-0.48) | **P26232** (CTNNA2;105,-18,-0.17) |
| **Q5SRE5** (NUP188;196,-19,-0.09) | **Q9BUA3** (C11orf84;41,-19,-0.46) | **Q9H467** (CUEDC2;32,-18,-0.56) |
| **Q8N1F7** (NUP93;93,-19,-0.20) | **Q969K3** (RNF34;42,-19,-0.45) | **Q13948** (CUX1;77,-18,-0.23) |
| **Q6DKJ4** (NXN;48,-19,-0.39) | **Q5H9J9** (TCP11X2;46,-19,-0.40) | **Q96SD1** (DCLRE1C;78,-18,-0.22) |
| **O75665** (OFD1;117,-19,-0.16) | **P35716** (SOX11;47,-19,-0.40) | **Q8TF46** (DIS3L;121,-18,-0.14) |
| **Q9BXW6** (OSBPL1A;108,-19,-0.17) | **P21128** (ENDOU;47,-19,-0.40) | **Q8IZD9** (DOCK3;233,-18,-0.07) |
| **O75781** (PALM;42,-19,-0.45) | **A6NEM1** (GOLGA6L9;49,-19,-0.38) | **P24534** (EEF1B2;25,-18,-0.72) |
| **Q6UWI2** (PARM1;32,-19,-0.58) | **Q86TP1** (PRUNE;50,-19,-0.37) | **Q99814** (EPAS1;96,-18,-0.18) |
| **Q8NEN9** (PDZD8;129,-19,-0.14) | **A6NFL8** (GOLGA6L20;50,-19,-0.37) | **Q15375** (EPHA7;112,-18,-0.16) |
| **P55347** (PKNOX1;48,-19,-0.39) | **A6NI03** (TRIM64B;52,-19,-0.36) | **A6NGS2** (ERICH4;14,-18,-1.24) |
| **Q8ND90** (PNMA1;40,-19,-0.47) | **P08670** (VIM;54,-19,-0.35) | **P43268** (ETV4;54,-18,-0.33) |
| **Q9Y535** (POLR3H;23,-19,-0.82) | **Q96QS3** (ARX;58,-19,-0.32) | **P41161** (ETV5;58,-18,-0.31) |
| **P41236** (PPP1R2;23,-19,-0.82) | **P17658** (KCNA6;59,-19,-0.32) | **O60447** (EVI5;93,-18,-0.19) |
| **Q96MT3** (PRICKLE 1;94,-19,-0.20) | **Q96M34** (C3orf30;60,-19,-0.31) | **Q8NE31** (FAM13C;66,-18,-0.27) |
| **Q05513** (PRKCZ;68,-19,-0.28) | **Q8WTU2** (SRCRB4D;61,-19,-0.31) | **Q961P4** (FAM46A;50,-18,-0.36) |
| **P26022** (PTX3;42,-19,-0.45) | **Q6ZS11** (RINL;62,-19,-0.30) | **O95466** (FMNL1;122,-18,-0.14) |
| **P20742** (PZP;164,-19,-0.11) | **Q99504** (EYA3;63,-19,-0.30) | **P01100** (FOS;41,-18,-0.44) |
| **Q12967** (RALGDS;101,-19,-0.18) | **Q8NBE8** (KLHL23;64,-19,-0.29) | **O95257** (GADD45G;17,-18,-1.05) |
| **P47736** (RAP1GAP;73,-19,-0.25) | **Q96MW5** (COG8;68,-19,-0.27) | **Q9HCG7** (GBA2;105,-18,-0.17) |
| **O75901** (RASSF9;50,-19,-0.37) | **Q8NAN2** (FAM73A;71,-19,-0.26) | **Q92990** (GLMN;68,-18,-0.26) |
| **P98175** (RBM10;104,-19,-0.18) | **Q9UN88** (GABRQ;72,-19,-0.26) | **Q8TBA6** (GOLGA5;83,-18,-0.21) |
| **O95199** (RCBTB2;60,-19,-0.31) | **Q8NBF2** (NHLRC2;79,-19,-0.23) | **Q8NBJ4** (GOLM1;45,-18,-0.39) |
| **O00237** (RNF103;79,-19,-0.23) | **Q8TC07** (TBC1D15;79,-19,-0.23) | **Q9HD26** (GOPC;51,-18,-0.35) |
| **Q5VTR2** (RNF20;114,-19,-0.16) | **Q04446** (GBE1;80,-19,-0.23) | **Q68CZ6** (HAUS3;70,-18,-0.25) |
| **Q8N2Y8** (RUSC2;161,-19,-0.11) | **Q9UIU6** (SIX4;83,-19,-0.22) | **Q7Z4H7** (HAUS6;109,-18,-0.16) |
| **Q9UQD0** (SCN8A;225,-19,-0.08) | **O60568** (PLOD3;85,-19,-0.22) | **Q8IV36** (HID1;89,-18,-0.20) |
| **Q9NZV5** (SEPN1;66,-19,-0.28) | **P25205** (MCM3;91,-19,-0.20) | **P28067** (HLA-DMA;29,-18,-0.61) |
| **Q15047** (SETDB1;143,-19,-0.13) | **O60658** (PDE8A;93,-19,-0.20) | **Q96EW2** (HSPBAP1;55,-18,-0.32) |
| **Q9H2J7** (SLC6A15;82,-19,-0.23) | **Q5VTH9** (WDR78;95,-19,-0.20) | **Q8NDH6** (ICA1L;54,-18,-0.33) |
| **Q9HBV2** (SPACA1;32,-19,-0.59) | **P18858** (LIG1;102,-19,-0.18) | **Q9Y366** (IFT52;50,-18,-0.36) |
| **Q8NBT2** (SPC24;22,-19,-0.84) | **Q6EKJ0** (GTF21RD2B;107,-19,-0.17) | **P42701** (IL12RB1;73,-18,-0.24) |
| **Q8WYL5** (SSH1;116,-19,-0.16) | **Q12860** (CNTN1;113,-19,-0.16) | **P46940** (IQGAP1;189,-18,-0.09) |
| **Q7L0J3** (SV2A;83,-19,-0.22) | **P18206** (VCL;124,-19,-0.15) | **Q96I82** (KAZALD1;33,-18,-0.54) |
| **Q9BZK7** (TBL1XR1;56,-19,-0.34) | **Q149M9** (NWD1;175,-19,-0.10) | **Q8IZA0** (KIAA0319L;116,-18,-0.15) |
| **Q8N1K5** (THEMIS;73,-19,-0.25) | **P12111** (COL6A3;344,-19,-0.05) | **Q8TCG1** (KIAA1524;102,-18,-0.17) |
| **Q9Y6Q6** (TNFRSF11A;66,-19,-0.28) | **Q9UKV3** (ACIN1;152,-18,-0.11) | **Q9ULH0** (KIDINS220;197,-18,-0.09) |
| **O75674** (TOM1L1;53,-19,-0.35) | **Q9UP79** (ADAMTS8;96,-18,-0.18) | **O60282** (KIF5C;109,-18,-0.16) |
| **Q7Z7E8** (UBE2Q1;46,-19,-0.41) | **Q3LIE5** (ADPRM;40,-18,-0.45) | **Q15323** (KRT31;47,-18,-0.38) |
| **O95185** (UNC5C;103,-19,-0.18) | **P02771** (AFP;69,-18,-0.26) | **Q92764** (KRT35;50,-18,-0.35) |
| **Q9NX94** (WBP1L;38,-19,-0.50) | **Q9Y3D8** (AK6;20,-18,-0.89) | **A4D1U4** (LCHN;51,-18,-0.34) |
| **Q96S15** (WDR24;102,-19,-0.18) | **P09917** (ALOX5;78,-18,-0.23) | **Q9BXB1** (LGR4;104,-18,-0.17) |
| **Q81X03** (WWC1;125,-19,-0.15) | **P08133** (ANXA6;76,-18,-0.23) | **Q38SD2** (LRRK1;225,-18,-0.07) |
| **P13010** (XRCC5;83,-19,-0.22) | **Q9NRY4** (ARHGAP35;171,-18,-0.10) | **Q9NTJ4** (MAN2C1;116,-18,-0.15) |
| **O15060** (ZBTB39;79,-19,-0.24) | **O14497** (ARID1A;242,-18,-0.07) | **Q12851** (MAP4K2;92,-18,-0.19) |
| **Q86UZ6** (ZBTB46;64,-19,-0.29) | **Q8N8L6** (ARL10;27,-18,-0.65) | **Q96GX5** (MASTL;97,-18,-0.18) |
| **Q8IWT0** (ZBTB8OS;19,-19,-0.97) | **O43681** (ASNA1;39,-18,-0.46) | **O15068** (MCF2L;128,-18,-0.14) |
| **Q8WW38** (ZFPM2;128,-19,-0.14) | **P18848** (ATF4;39,-18,-0.46) | **Q9BTE3** (MCMBP;73,-18,-0.24) |
| **Q03112** (MECOM;118,-18,-0.15) | **Q6ZRG5** (0;24,-18,-0.74) | **Q8N944** (AMER3;90,-17,-0.18) |
| **O75121** (MFAP3L;45,-18,-0.39) | **P40337** (VHL;24,-18,-0.74) | **Q9H9E1** (ANKRA2;34,-17,-0.49) |
| **P08582** (MFI2;80,-18,-0.22) | **Q8WVE0** (N6AMT2;25,-18,-0.73) | **Q8WVL7** (ANKRD49;27,-17,-0.62) |
| **Q969V6** (MKL1;99,-18,-0.18) | **P15374** (UCHL3;26,-18,-0.68) | **Q7Z5R6** (APBB1IP;73,-17,-0.23) |
| **Q9BV36** (MLPH;66,-18,-0.27) | **Q8WUU8** (TMEM174;26,-18,-0.68) | **Q8TDY4** (ASAP3;99,-17,-0.17) |
| **P49959** (MRE11A;81,-18,-0.22) | **A4D2B0** (MBLAC1;27,-18,-0.66) | **Q9UBL3** (ASH2L;69,-17,-0.24) |
| **Q96HT8** (MRFAP1L1;15,-18,-1.21) | **A6NEV1** (PRR23A;28,-18,-0.63) | **Q8WYN0** (ATG4A;45,-17,-0.37) |
| **Q9H3R2** (MUC13;55,-18,-0.32) | **Q9H6K1** (C6orf106;33,-18,-0.54) | **P50993** (ATP1A2;112,-17,-0.15) |
| **Q13564** (NAE1;60,-18,-0.29) | **Q9Y5U2** (TSSC4;34,-18,-0.52) | **Q8NBF6** (AVL9;72,-17,-0.23) |
| **O14777** (NDC80;74,-18,-0.24) | **Q9P2X3** (IMPACT;36,-18,-0.49) | **Q9UIF9** (BAZ2A;211,-17,-0.08) |
| **Q9Y3T9** (NOC2L;85,-18,-0.21) | **P55082** (MFAP3;40,-18,-0.44) | **Q9H165** (BCL11A;91,-17,-0.18) |
| **O94818** (NOL4;71,-18,-0.25) | **Q9UGV2** (NDRG3;41,-18,-0.43) | **Q9BUH8** (BEGAIN;65,-17,-0.26) |
| **Q96MY1** (NOL4L;47,-18,-0.38) | **Q53QW1** (C2orf57;42,-18,-0.43) | **Q13323** (BIK;18,-17,-0.94) |
| **Q9UNZ2** (NSFL 1C;41,-18,-0.44) | **Q5TDH0** (DDI2;45,-18,-0.40) | **Q14137** (BOP1;84,-17,-0.20) |
| **Q8TEM1** (NUP210;205,-18,-0.08) | **Q9HAS0** (C17orf75;45,-18,-0.40) | **P59827** (BPIFB4;65,-17,-0.26) |
| **Q96FW1** (OTUB1;31,-18,-0.57) | **Q9NXR5** (ANKRD10;45,-18,-0.40) | **O00478** (BTN3A3;65,-17,-0.26) |
| **Q5JRK9** (PAGE2B;12,-18,-1.49) | **Q9BSE4** (HERPUD2;45,-18,-0.39) | **Q9BV29** (C15orf57;21,-17,-0.82) |
| **Q86YC2** (PALB2;131,-18,-0.13) | **Q9NWX5** (ASB6;47,-18,-0.38) | **P01031** (C5;188,-17,-0.09) |
| **Q63HM2** (PCNXL4;133,-18,-0.13) | **P09104** (ENO2;47,-18,-0.38) | **Q86V35** (CABP7;24,-17,-0.69) |
| **Q53EL6** (PDCD4;52,-18,-0.34) | **Q9H313** (TTYH1;49,-18,-0.36) | **Q01668** (CACNA1D;245,-17,-0.06) |
| **Q9P2J9** (PDP2;60,-18,-0.30) | **P78396** (CCNA1;52,-18,-0.34) | **Q9BY67** (CADM1;49,-17,-0.35) |
| **Q5T2W1** (PDZK1;57,-18,-0.31) | **P15036** (ETS2;53,-18,-0.33) | **P05937** (CALB1;30,-17,-0.56) |
| **Q9Y4G2** (PLEKHM1;117,-18,-0.15) | **Q09470** (KCNA1;56,-18,-0.31) | **A5YM72** (CARNS1;88,-17,-0.19) |
| **Q9UNA4** (POLI;83,-18,-0.21) | **Q01113** (IL9R;57,-18,-0.31) | **Q9Y3C0** (CCDC53;21,-17,-0.80) |
| **P35813** (PPM1A;42,-18,-0.42) | **Q9Y5P3** (RAI2;57,-18,-0.31) | **Q9H3Q1** (CDC42EP4;38,-17,-0.44) |
| **P13861** (PRKAR2A;46,-18,-0.39) | **Q13705** (ACVR2B;58,-18,-0.31) | **Q6UY09** (CEACAM20;65,-17,-0.26) |
| **Q14699** (RFTN1;63,-18,-0.28) | **P06239** (LCK;58,-18,-0.31) | **Q9BY43** (CHMP4A;25,-17,-0.67) |
| **Q81XI1** (RHOT2;68,-18,-0.26) | **Q2L4Q9** (PRSS53;58,-18,-0.30) | **O75838** (CIB2;22,-17,-0.78) |
| **Q7Z5B4** (RIC3;41,-18,-0.43) | **Q6ZTN6** (ANKRD13D;58,-18,-0.30) | **Q9UIV1** (CNOT7;33,-17,-0.51) |
| **O14730** (RIOK3;59,-18,-0.30) | **P09603** (CSF1;60,-18,-0.29) | **Q9Y2V7** (COG6;73,-17,-0.23) |
| **O75298** (RTN2;59,-18,-0.30) | **P06865** (HEXA;61,-18,-0.29) | **P07333** (CSF1R;108,-17,-0.15) |
| **Q9UHR5** (SAP30BP;34,-18,-0.53) | **Q6ZWJ1** (STXBP4;62,-18,-0.29) | **O60716** (CTNND1;108,-17,-0.15) |
| **Q93073** (SECISBP2L;122,-18,-0.14) | **Q9H5J0** (ZBTB3;62,-18,-0.29) | **P35638** (DDIT3;19,-17,-0.88) |
| **P58004** (SESN2;54,-18,-0.33) | **P32519** (ELF1;67,-18,-0.26) | **Q8IV53** (DENND1C;87,-17,-0.19) |
| **P31947** (SFN;28,-18,-0.64) | **O95677** (EYA4;70,-18,-0.25) | **O75140** (DEPDC5;181,-17,-0.09) |
| **O43147** (SGSM2;113,-18,-0.15) | **Q9H6X5** (C19orf44;71,-18,-0.25) | **Q92620** (DHX38;141,-17,-0.12) |
| **Q9H0K1** (SIK2;104,-18,-0.17) | **Q5VWK5** (IL23R;72,-18,-0.25) | **Q9BVC3** (DSCC1;45,-17,-0.37) |
| **Q9P2F8** (SIPA1L2;190,-18,-0.09) | **Q8WVZ9** (KBTBD7;77,-18,-0.23) | **Q56P03** (EAPP;33,-17,-0.51) |
| **P63208** (SKP1;19,-18,-0.96) | **Q8N3R9** (MPP5;77,-18,-0.23) | **Q9UNE0** (EDAR;49,-17,-0.34) |
| **Q13433** (SLC39A6;85,-18,-0.21) | **Q16820** (MEP1B;80,-18,-0.22) | **O75822** (EIF3J;29,-17,-0.58) |
| **Q9Y3F4** (STRAP;38,-18,-0.46) | **Q9BXC9** (BBS2;80,-18,-0.22) | **O95834** (EML2;71,-17,-0.24) |
| **Q81YJ3** (SYTL1;62,-18,-0.29) | **Q9NVR0** (KLHL11;80,-18,-0.22) | **P34910** (EVI2B;49,-17,-0.34) |
| **Q3MII6** (TBC1D25;76,-18,-0.23) | **Q9UP52** (TFR2;89,-18,-0.20) | **Q8TAG9** (EXOC6;94,-17,-0.18) |
| **Q13445** (TMED1;25,-18,-0.71) | **Q6XPR3** (RPTN;91,-18,-0.19) | **Q6PEV8** (FAM199X;43,-17,-0.39) |
| **Q9C0H2** (TTYH3;58,-18,-0.31) | **A2RTX5** (TARSL2;93,-18,-0.19) | **Q5TGI0** (FAXC;47,-17,-0.36) |
| **Q81ZJ1** (UNC5B;104,-18,-0.17) | **Q5T447** (HECTD3;97,-18,-0.18) | **Q9BSK4** (FEM1A;74,-17,-0.23) |
| **Q8N6Y0** (USHBP1;76,-18,-0.23) | **Q8ND30** (PPFIBP2;99,-18,-0.18) | **P21802** (FGFR2;92,-17,-0.18) |
| **Q96QU8** (XPO6;129,-18,-0.13) | **O15327** (INPP4B;105,-18,-0.17) | **Q75LS8** (FKBP9P1;16,-17,-1.09) |
| **Q96MU7** (YTHDC1;85,-18,-0.21) | **Q17RG1** (KCTD19;105,-18,-0.17) | **Q9UN86** (G3BP2;54,-17,-0.31) |
| **Q05516** (ZBTB16;74,-18,-0.24) | **Q9H0M0** (WWP1;105,-18,-0.17) | **O75293** (GADD45B;18,-17,-0.95) |
| **Q9UBW7** (ZMYM2;155,-18,-0.11) | **Q70J99** (UNC13D;123,-18,-0.14) | **A1L429** (GAGE12B;;13,-17,-1.31) |
| **Q9UDV7** (ZNF282;74,-18,-0.24) | **Q5JYT7** (KIAA1755;131,-18,-0.13) | **Q9UEU5** (GAGE2D;;13,-17,-1.33) |
| **Q9UGI0** (ZRANB1;81,-18,-0.22) | **Q5TEA3** (C20orf194;132,-18,-0.13) | **Q96PP8** (GBP5;67,-17,-0.25) |
| **P07919** (UQCRH;11,-18,-1.67) | **Q2VIQ3** (KIF4B;140,-18,-0.12) | **P16383** (GCFC2;89,-17,-0.19) |
| **Q13068** (GAGE4;13,-18,-1.39) | **P41252** (IARS;144,-18,-0.12) | **P36915** (GNL1;69,-17,-0.24) |
| **P31431** (SDC4;22,-18,-0.83) | **Q9BXX2** (ANKRD30B;158,-18,-0.11) | **P80108** (GPLD1;92,-17,-0.18) |
| **Q5H9L2** (TCEAL5;23,-18,-0.77) | **Q9BZC7** (ABCA2;270,-17,-0.06) | **P15170** (GSPT1;56,-17,-0.30) |
| **Q58FG1** (HSP90AA4P;48,-17,-0.35) | **Q8N0X2** (SPAG16;71,-17,-0.24) | **O14641** (DVL2;79,-17,-0.21) |
| **O95757** (HSPA4L;95,-17,-0.17) | **Q9UEE5** (STK17A;47,-17,-0.36) | **P48380** (RFX3;84,-17,-0.20) |
| **Q96FT9** (IFT43;24,-17,-0.72) | **Q9ULQ0** (STRIP2;95,-17,-0.17) | **Q9UGT4** (SUSD2;90,-17,-0.18) |
| **Q96HW7** (INTS4;108,-17,-0.15) | **Q92797** (SYMPK;141,-17,-0.12) | **P02671** (FGA;95,-17,-0.17) |
| **Q9UPP2** (IQSEC3;128,-17,-0.13) | **A6NDD5** (SYNDIG1L;26,-17,-0.65) | **Q3MJ16** (PLA2G4E;98,-17,-0.17) |
| **O95965** (ITGBL1;54,-17,-0.31) | **Q6ZMZ3** (SYNE3;112,-17,-0.15) | **P86452** (ZBED6;110,-17,-0.15) |
| **P29375** (KDM5A;192,-17,-0.08) | **Q5QJ74** (TBCEL;48,-17,-0.35) | **Q8N398** (VWA5B2;133,-17,-0.12) |
| **O60662** (KLHL41;68,-17,-0.24) | **Q9Y4R8** (TELO2;92,-17,-0.18) | **Q9NZM4** (GLTSCR1;158,-17,-0.10) |
| **Q2M2I5** (KRT24;55,-17,-0.30) | **Q9Y6I9** (TEX264;34,-17,-0.49) | **Q5T5Y3** (CAMSAP1;178,-17,-0.09) |
| **O76011** (KRT34;49,-17,-0.34) | **P22735** (TGM1;90,-17,-0.18) | **Q5JTZ9** (AARS2;107,-16,-0.14) |
| **Q6UX15** (LAYN;43,-17,-0.39) | **Q9UIK5** (TMEFF2;41,-17,-0.41) | **Q96P50** (ACAP3;92,-16,-0.17) |
| **O60711** (LPXN;43,-17,-0.39) | **Q96PN7** (TRERF1;132,-17,-0.12) | **P35611** (ADD1;81,-16,-0.19) |
| **P43355** (MAGEA1;34,-17,-0.49) | **Q9BYV2** (TRIM54;40,-17,-0.42) | **Q9BRR6** (ADPGK;54,-16,-0.29) |
| **P43243** (MATR3;95,-17,-0.17) | **Q8N9V2** (TRIML1;53,-17,-0.32) | **Q9NX46** (ADPRHL2;39,-16,-0.41) |
| **Q8NI22** (MCFD2;16,-17,-1.03) | **Q06418** (TYRO3;97,-17,-0.17) | **P35573** (AGL;175,-16,-0.09) |
| **O14770** (MEIS2;52,-17,-0.32) | **Q6NUM6** (TYW1B;77,-17,-0.22) | **Q96B36** (AKT1S1;27,-16,-0.58) |
| **Q6WCQ1** (MPRIP;117,-17,-0.14) | **Q96S82** (UBL7;41,-17,-0.41) | **O00203** (AP3B1;121,-16,-0.13) |
| **Q5JR59** (MTUS2;150,-17,-0.11) | **Q9Y6N9** (USH1C;62,-17,-0.27) | **P98171** (ARHGAP4;105,-16,-0.15) |
| **Q5SSG8** (MUC21;54,-17,-0.31) | **Q9HCJ6** (VAT1L;46,-17,-0.37) | **P78348** (ASIC1;60,-16,-0.26) |
| **Q9Y6X6** (MYO16;206,-17.-0.08) | **Q9H7D7** (WDR26;72,-17,-0.23) | **Q9HBG4** (ATP6V0A4;96,-16,-0.16) |
| **Q09161** (NCBP1;92,-17,-0.18) | **Q9HAD4** (WDR41;52,-17,-0.32) | **Q8WXE1** (ATRIP;86,-16,-0.18) |
| **P25208** (NFYB;23,-17,-0.74) | **Q9Y2K1** (ZBTB1;82,-17,-0.20) | **Q9UBB4** (ATXN10;53,-16,-0.29) |
| **P59047** (NLRP5;134,-17,-0.12) | **Q8N5A5** (ZGPAT;57,-17,-0.29) | **Q8IWZ6** (BBS7;80,-16,-0.19) |
| **Q16620** (NTRK2;92,-17,-0.18) | **Q96K83** (ZNF521;148,-17,-0.11) | **P50895** (BCAM;67,-16,-0.23) |
| **Q8IVD9** (NUDCD3;41,-17,-0.41) | **Q13070** (GAGE6;13,-17,-1.31) | **Q9C0K0** (BCL11B;96,-16,-0.16) |
| **Q9H1M0** (NUP62CL;21,-17,-0.81) | **Q13069** (GAGE5;13,-17,-1.31) | **Q7Z569** (BRAP;67,-16,-0.23) |
| **A6NDY0** (PABPN1L;30,-17,-0.55) | **Q13067** (GAGE3;13,-17,-1.31) | **Q05682** (CALD1;93,-16,-0.17) |
| **Q9BY11** (PACSIN1;51,-17,-0.33) | **Q8WWF1** (C1orf54;15,-17,-1.13) | **Q9NZT1** (CALML5;16,-16,-1.00) |
| **Q9Y2J8** (PADI2;76,-17,-0.22) | **O75607** (NPM3;19,-17,-0.87) | **P20807** (CAPN3;94,-16,-0.16) |
| **Q7Z2X7** (PAGE2;12,-17,-1.40) | **Q96PQ5** (PPP1R2P1;23,-17,-0.74) | **Q9BWT7** (CARD10;116,-16,-0.13) |
| **P29120** (PCSK1;84,-17,-0.20) | **P09497** (CLTB;25,-17,-0.67) | **Q6ZRH7** (CATSPERG;133,-16,-0.12) |
| **Q15084** (PDIA6;48,-17,-0.35) | **A8MT33** (SYCE1L;27,-17,-0.62) | **Q96JG6** (CCDC132;111,-16,-0.14) |
| **Q9NQ66** (PLCB1;139,-17,-0.12) | **Q8N4E4** (PDCL2;28,-17,-0.60) | **P0C7W6** (CCDC172;31,-16,-0.51) |
| **O95397** (PLEKHA8P1;44,-17,-0.39) | **P12004** (PCNA;29,-17,-0.59) | **Q7Z3E2** (CCDC186;104,-16,-0.15) |
| **O94827** (PLEKHG5;117,-17,-0.14) | **Q96HH4** (TMEM169;34,-17,-0.50) | **Q99467** (CD180;74,-16,-0.21) |
| **O00592** (PODXL;59,-17,-0.28) | **Q96FV0** (LRRC46;35,-17,-0.48) | **Q9NPY3** (CD93;69,-16,-0.23) |
| **P19387** (POLR2C;31,-17,-0.54) | **Q9NRS6** (SNX15;38,-17,-0.44) | **P19835** (CEL;79,-16,-0.20) |
| **P27169** (PON1;40,-17,-0.42) | **Q14012** (CAMK1;41,-17,-0.41) | **Q8N960** (CEP120;113,-16,-0.14) |
| **O75335** (PPFIA4;134,-17,-0.12) | **Q9H756** (LRRC19;42,-17,-0.40) | **O15182** (CETN3;20,-16,-0.81) |
| **Q96I34** (PPP1R16A;58,-17,-0.29) | **P20142** (PGC;42,-17,-0.40) | **Q9ULV3** (CIZ1;100,-16,-0.15) |
| **P53611** (RABGGTB;37,-17,-0.46) | **Q8N831** (TSPYL6;46,-17,-0.37) | **Q96S66** (CLCC1;62,-16,-0.25) |
| **Q15311** (RALBP1;76,-17,-0.22) | **Q14209** (E2F2;48,-17,-0.35) | **Q9C0A0** (CNTNAP4;145,-16,-0.11) |
| **Q9Y4G8** (RAPGEF2;167,-17,-0.10) | **Q9Y664** (KPTN;48,-17,-0.35) | **Q13561** (DCTN2;44,-16,-0.36) |
| **Q99708** (RBBP8;102,-17,-0.16) | **Q96N77** (ZNF641;50,-17,-0.34) | **Q6IQ26** (DENND5A;147,-16,-0.10) |
| **Q5W0B1** (RNF219;81,-17,-0.20) | **P13646** (KRT13;50,-17,-0.34) | **Q8WYQ5** (DGCR8;86,-16,-0.18) |
| **Q9NYV6** (RRN3;74,-17,-0.22) | **Q8TBK2** (SETD6;53,-17,-0.31) | **Q96F81** (DISP1;171,-16,-0.09) |
| **Q96DX4** (RSPRY1;64,-17,-0.26) | **Q7RTX7** (CATSPER4;54,-17,-0.31) | **P27487** (DPP4;88,-16,-0.18) |
| **Q9NXZ1** (SAGE1;99,-17,-0.17) | **A6NK89** (RASSF10;57,-17,-0.29) | **Q16828** (DUSP6;42,-16,-0.37) |
| **P0C264** (SBK3;38,-17,-0.44) | **Q92696** (RABGGTA;65,-17,-0.26) | **Q12805** (EFEMP1;55,-16,-0.29) |
| **Q9Y2G9** (SBNO2;150,-17,-0.11) | **Q07065** (CKAP4;66,-17,-0.25) | **Q9BQI3** (EIF2AK1;71,-16,-0.22) |
| **Q14108** (SCARB2;54,-17,-0.31) | **095671** (ASMTL;69,-17,-0.24) | **P56537** (EIF6;27,-16,-0.60) |
| **Q16586** (SGCA;43,-17,-0.39) | **A6NJ88** (0;69,-17,-0.24) | **Q16206** (ENOX2;70,-16,-0.22) |
| **Q9HC58** (SLC24A3;72,-17,-0.23) | **P04844** (RPN2;69,-17,-0.24) | **P14921** (ETS1;50,-16,-0.31) |
| **Q92504** (SLC39A7;50,-17,-0.33) | **Q9UJY5** (GGA1;70,-17,-0.24) | **Q8IXR5** (FAM178B;94,-16,-0.17) |
| **Q07837** (SLC3A1;79,-17,-0.21) | **Q96M63** (CCDC114;75,-17,-0.22) | **O94952** (FBXO21;72,-16,-0.22) |
| **O75093** (SLIT1;168,-17,-0.10) | **Q7RTS9** (DYM;76,-17,-0.22) | **Q5JSP0** (FGD3;79,-16,-0.20) |
| **O94964** (SOGA1;160,-17,-0.10) | **P52888** (THOP1;79,-17,-0.21) | **Q96M96** (FGD4;87,-16,-0.18) |
| **P11362** (FGFR1;92,-16,-0.17) | **P43686** (PSMC4;47,-16,-0.33) | **Q8WVB3** (HEXDC;54,-16,-0.29) |
| **Q96RU3** (FNBP1;71,-16,-0.22) | **Q14761** (PTPRCAP;21,-16,-0.75) | **Q495W5** (FUT11;56,-16,-0.28) |
| **Q6PJQ5** (FOXR2;36,-16,-0.44) | **P28827** (PTPRM;164,-16,-0.09) | **O15131** (KPNA5;60,-16,-0.26) |
| **Q13283** (G3BP1;52,-16,-0.30) | **Q9H4A4** (RNPEP;73,-16,-0.22) | **P43251** (BTD;61,-16,-0.26) |
| **P24522** (GADD45A;18,-16,-0.87) | **Q7LG56** (RRM2B;41,-16,-0.39) | **P35527** (KRT9;62,-16,-0.25) |
| **Q4V321** (GAGE13;13,-16,-1.24) | **O75995** (SASH3;42,-16,-0.38) | **Q0D2K2** (KLHL30;64,-16,-0.25) |
| **Q13066** (GAGE2B;13,-16,-1.25) | **Q9Y6U3** (SCIN;80,-16,-0.19) | **P37173** (TGFBR2;65,-16,-0.24) |
| **O76087** (GAGE7;13,-16,-1.23) | **P16581** (SELE;67,-16,-0.24) | **Q0VAK6** (LMOD3;65,-16,-0.24) |
| **Q9Y2T3** (GDA;51,-16,-0.31) | **Q9UJW9** (SERTAD3;22,-16,-0.73) | **Q8WW4** (POF1B;68,-16,-0.23) |
| **Q9NZ52** (GGA3;78,-16,-0.20) | **Q2Y0W8** (SLC4A8;123,-16,-0.13) | **Q14651** (PLS1;70,-16,-0.22) |
| **Q6P9H5** (GIMAP6;33,-16,-0.48) | **Q969T3** (SNX21;41,-16,-0.38) | **F8WBI6** (GOLGA8N;72,-16,-0.22) |
| **O43424** (GRID2;113,-16,-0.14) | **O94768** (STK17B;42,-16,-0.37) | **C9JLR9** (C11orf95;73,-16,-0.21) |
| **Q9C0E4** (GRIP2;113,-16,-0.14) | **Q15431** (SYCP1;114,-16,-0.14) | **Q96SB4** (SRPK1;74,-16,-0.21) |
| **Q8TAX9** (GSDMB;47,-16,-0.34) | **Q9H7V2** (SYNDIG1;29,-16,-0.56) | **Q2M243** (CCDC27;75,-16,-0.21) |
| **Q9Y2N7** (HIF3A;72,-16,-0.22) | **Q15750** (TAB1;55,-16,-0.29) | **P20591** (MX1;76,-16,-0.21) |
| **P01906** (HLA-DQA2;28,-16,-0.57) | **O95551** (TDP2;41,-16,-0.39) | **Q96NG3** (TTC25;77,-16,-0.20) |
| **Q92902** (HPS1;79,-16,-0.20) | **Q5JTD0** (TJAP1;62,-16,-0.25) | **Q6P9F0** (CCDC62;78,-16,-0.20) |
| **Q9HBG6** (IFT122;142,-16,-0.11) | **Q4V9L6** (TMEM119;29,-16,-0.54) | **Q9Y6B7** (AP4B1;83,-16,-0.19) |
| **B1AKI9** (ISM1;52,-16,-0.30) | **Q13625** (TP53BP2;126,-16,-0.12) | **Q2TAL8** (QRICH1;86,-16,-0.18) |
| **Q8IWB1** (ITPRIP;62,-16,-0.25) | **P36406** (TRIM23;64,-16,-0.24) | **Q9Y4K0** (LOXL2;87,-16,-0.18) |
| **Q9UNX9** (KCNJ14;48,-16,-0.33) | **Q9NQ86** (TRIM36;83,-16,-0.19) | **P51692** (STAT5B;90,-16,-0.17) |
| **Q8IV33** (KIAA0825;148,-16,-0.10) | **Q9Y5S1** (TRPV2;86,-16,-0.18) | **Q86Y56** (HEATR2;94,-16,-0.17) |
| **A8MWY0** (KIAA 1324L; 114,-16,-0.14) | **Q9H6E5** (TUT1;94,-16,-0.17) | **Q96M69** (LRGUK;94,-16,-0.17) |
| **Q9UH77** (KLHL3;65,-16,-0.24) | **P17480** (UBTF;89,-16,-0.17) | **Q658Y4** (FAM91A1 ;94,-16,-0.17) |
| **P13645** (KRT10;59,-16,-0.27) | **Q9P1Q0** (VPS54;111,-16,-0.14) | **Q9H5Y7** (SLITRK6;95,-16,-0.16) |
| **Q7Z3Y7** (KRT28;51,-16,-0.31) | **Q9BQA1** (WDR77;37,-16,-0.43) | **P42658** (DPP6;98,-16,-0.16) |
| **Q08380** (LGALS3BP;65,-16,-0.24) | **Q96S55** (WRNIP1;72,-16,-0.22) | **Q9UKY1** (ZHX1;98,-16,-0.16) |
| **Q1L5Z9** (LONRF2;84,-16,-0.19) | **P46937** (YAP1;54,-16,-0.29) | **Q92953** (KCNB2;103,-16,-0.15) |
| **O95490** (LPHN2; 163,-16,-0.09) | **Q9HC78** (ZBTB20;81,-16,-0.19) | **Q969F9** (HPS3;114,-16,-0.14) |
| **Q6T4P5** (LPPR3;76,-16,-0.21) | **O15062** (ZBTB5;74,-16,-0.21) | **Q8N3T6** (TMEM132C;122,-16,-0.13) |
| **Q9HBG7** (LY9;72,-16,-0.22) | **Q9UQR1** (ZNF148;89,-16,-0.17) | **Q9BXT4** (TDRD1;132,-16,-0.12) |
| **Q13164** (MAPK7;88,-16,-0.18) | **P21754** (ZP3;47,-16,-0.34) | **Q86XP1** (DGKH;135,-16,-0.11) |
| **Q15528** (MED22;22,-16,-0.72) | **A6NER3** (GAGE12J;13,-16,-1.24) | **Q5TIA1** (MEI1;141,-16,-0.11) |
| **Q8TDZ2** (MICAL1;118,-16,-0.13) | **P0CL80** (GAGE12F;13,-16,-1.23) | **P13942** (COL11A2;172,-16,-0.09) |
| **P08253** (MMP2;74,-16,-0.21) | **P0CL81** (GAGE12G;13,-16,-1.23) | **Q6T4R5** (NHS;179,-16,-0.08) |
| **P22897** (MRC1;166,-16,-0.09) | **P0CL82** (GAGE12I;13,-16,-1.23) | **Q8NF50** (DOCK8;239,-16,-0.06) |
| **Q9BUK6** (MSTO1;62,-16,-0.25) | **Q5T4I8** (C6orf52;17,-16,-0.92) | **Q99965** (ADAM2;82,-15,-0.18) |
| **Q13765** (NACA;23,-16,-0.68) | **Q9BU02** (THTPA;26,-16,-0.62) | **Q8TC27** (ADAM32;88,-15,-0.17) |
| **Q9BTE0** (NAT9;23,-16,-0.68) | **A6NDR6** (MEIS3P1;30,-16,-0.52) | **P35612** (ADD2;81,-15,-0.18) |
| **Q9UBB6** (NCDN;79,-16,-0.20) | **Q9BR61** (ACBD6;31,-16,-0.51) | **P35869** (AHR;96,-15,-0.15) |
| **Q9NZQ3** (NCKIPSD;79,-16,-0.20) | **Q8NF67** (0;31,-16,-0.51) | **Q7LC44** (ARC;45,-15,-0.33) |
| **Q15788** (NCOA1;157,-16,-0.10) | **Q7L4S7** (ARMCX6;33,-16,-0.48) | **O43307** (ARHGEF9;61,-15,-0.24) |
| **P46934** (NEDD4;149,-16,-0.10) | **Q8WTX7** (GATSL3;36,-16,-0.44) | **Q7Z3E5** (ARMC9;92,-15,-0.16) |
| **O60524** (NEMF; 123,-16,-0.13) | **Q15165** (PON2;39,-16,-0.40) | **Q8N3C0** (ASCC3;251,-15,-0.05) |
| **Q9NZ94** (NLGN3;94,-16,-0.17) | **P52788** (SMS;41,-16,-0.38) | **Q86U10** (ASPG;61,-15,-0.24) |
| **Q8WX94** (NLRP7;112,-16,-0.14) | **Q16621** (NFE2;41,-16,-0.38) | **P06576** (ATP5B;57,-15,-0.26) |
| **Q9NXX6** (NSMCE4A;44,-16,-0.36) | **P54727** (RAD23B;43,-16,-0.37) | **Q96A70** (AZIN2;50,-15,-0.30) |
| **Q9NXG6** (P4HTM;57,-16,-0.28) | **P14091** (CTSE;43,-16,-0.36) | **Q9NRL2** (BAZ1A; 179,-15,-0.08) |
| **Q9BVG4** (PBDC1;26,-16,-0.61) | **Q8IZ63** (PRR22;44,-16,-0.36) | **Q6ZW61** (BBS12;79,-15,-0.18) |
| **Q96KB5** (PBK;36,-16,-0.44) | **Q99932** (SPAG8;45,-16,-0.35) | **Q7L3V2** (BOP;39,-15,-0.38) |
| **Q9H2J4** (PDCL3;28,-16,-0.57) | **Q9BXU3** (TEX13A;46,-16,-0.35) | **Q9H8M2** (BRD9;67,-15,-0.22) |
| **Q86TG7** (PEG10;80,-16,-0.19) | **Q04695** (KRT17;48,-16,-0.33) | **Q9NP86** (CABP5;20,-15,-0.75) |
| **P12955** (PEPD;55,-16,-0.29) | **P48165** (GJA8;48,-16,-0.33) | **Q13936** (CACNA1C;249,-15,-0.06) |
| **O00264** (PGRMC1;22,-16,-0.73) | **Q6P531** (GGT6;51,-16,-0.31) | **Q13698** (CACNA1S;212,-15,-0.07) |
| **Q92576** (PHF3;229,-16,-0.06) | **Q9H993** (C6orf211;51,-16,-0.31) | **Q9BXU9** (CALN1;25,-15,-0.60) |
| **Q9UBF8** (PI4KB;91,-16,-0.17) | **A6NLI5** (TRIM64C;51,-16,-0.31) | **Q6P4E1** (CASC4;49,-15,-0.30) |
| **Q8WXW3** (PIBF1;90,-16,-0.17) | **Q5R3I4** (TTC38;53,-16,-0.30) | **Q7Z7H3** (CATIP;44,-15,-0.34) |
| **Q6TFL3** (CCDC171;153,-15,-0.09) | **P57077** (MAP3K7CL;27,-15,-0.55) | **Q8WUA7** (TBC1D22A;59,-15,-0.25) |
| **P50990** (CCT8;60,-15,-0.25) | **Q86XN8** (MEX3D;65,-15,-0.23) | **Q969E4** (TCEAL3;23,-15,-0.66) |
| **Q7L3B6** (CDC37L1;39,-15,-0.38) | **Q96C03** (MIEF2;49,-15,-0.30) | **Q96BS2** (TESC;25,-15,-0.60) |
| **Q8NHQ1** (CEP70;70,-15,-0.21) | **Q13064** (MKRN3;56,-15,-0.26) | **Q12800** (TFCP2;57,-15,-0.26) |
| **Q9H444** (CHMP4B;25,-15,-0.60) | **P09238** (MMP10;54,-15,-0.27) | **P19532** (TFE3;62,-15,-0.24) |
| **Q96Q77** (CIB3;22,-15,-0.68) | **Q8NDA8** (MROH1;181,-15,-0.08) | **Q68CZ2** (TNS3;155,-15,-0.09) |
| **P12277** (CKB;43,-15,-0.35) | **Q00872** (MYBPC1;128,-15,-0.11) | **Q96Q05** (TRAPPC9;129,-15,-0.11) |
| **A8TX70** (COL6A5;290,-15,-0.05) | **P12525** (MYCLP1;41,-15,-0.36) | **Q9C019** (TRIM15;52,-15,-0.28) |
| **Q8N4Y2** (CRACR2B;45,-15,-0.33) | **Q9BZK3** (NACAP1;23,-15,-0.64) | **Q9BZW7** (TSGA10;81,-15,-0.18) |
| **Q9Y4D2** (DAGLA;115,-15,-0.13) | **Q15021** (NCAPD2;157,-15,-0.09) | **Q9H0U9** (TSPYL1;49,-15,-0.30) |
| **P53355** (DAPK1;160,-15,-0.09) | **Q7Z6G3** (NECAB2;43,-15,-0.34) | **Q9ULT0** (TTC7A;96,-15,-0.15) |
| **O60443** (DFNA5;55,-15,-0.27) | **Q8NFZ4** (NLGN2;91,-15,-0.16) | **Q5T124** (UBXN11;57,-15,-0.26) |
| **Q9NRI5** (DISC1;94,-15,-0.16) | **Q9C000** (NLRP1;166,-15,-0.09) | **Q08AM6** (VAC14;88,-15,-0.17) |
| **Q155Q3** (DIXDC1;77,-15,-0.19) | **Q7RTR0** (NLRP9;113,-15,-0.13) | **Q9P2L0** (WDR35;134,-15,-0.11) |
| **Q14574** (DSC3;100,-15,-0.15) | **P0CE71** (OCM2;12,-15,-1.23) | **O95785** (WIZ;179,-15,-0.08) |
| **Q8WWB3** (DYDC1;21,-15,-0.71) | **Q9ULD0** (OGDHL;114,-15,-0.13) | **Q9HCS7** (XAB2;100,-15,-0.14) |
| **P29692** (EEF1D;31,-15,-0.48) | **O15460** (P4HA2;61,-15,-0.24) | **Q9UIA9** (XPO7;124,-15,-0.12) |
| **Q96JJ3** (ELMO2;83,-15,-0.18) | **Q96GU1** (PAGE5;14,-15,-1.06) | **O43829** (ZBTB14;51,-15,-0.29) |
| **Q9NZ08** (ERAP1;107,-15,-0.13) | **O75914** (PAK3;62,-15,-0.24) | **Q8WU90** (ZC3H15;49,-15,-0.30) |
| **Q9H790** (EXO5;42,-15,-0.35) | **Q9ULE6** (PALD1;97,-15,-0.15) | **Q9BRR0** (ZKSCAN3;61,-15,-0.24) |
| **Q96TA1** (FAM129B;84,-15,-0.17) | **Q8NCN5** (PDPR;99,-15,-0.15) | **Q9Y462** (ZNF711;86,-15,-0.17) |
| **O60320** (FAM189A1;57,-15,-0.26) | **Q76G19** (PDZD4;86,-15,-0.17) | **Q9BXV9** (C14orf142;11,-15,-1.38) |
| **O15287** (FANCG;69,-15,-0.21) | **A8MUH7** (PDZK1P1;44,-15,-0.34) | **A6NGK3** (GAGE10;13,-15,-1.18) |
| **P98174** (FGD1;107,-15,-0.14) | **Q5SV97** (PERM1;81,-15,-0.18) | **Q4V326** (GAGE2E;13,-15,-1.17) |
| **P98177** (FOXO4;54,-15,-0.27) | **Q8TDX9** (PKD1L1;315,-15,-0.04) | **A6NDE8** (GAGE12H;13,-15,-1.16) |
| **Q6NT46** (GAGE2A;13,-15,-1.17) | **A1L390** (PLEKHG3;134,-15,-0.11) | **Q16143** (SNCB;14,-15,-1.04) |
| **Q9H2C0** (GAN;68,-15,-0.22) | **Q96PX9** (PLEKHG4B;140,-15,-0.10) | **Q13065** (GAGE1;16,-15,-0.96) |
| **P31150** (GDI1;51,-15,-0.29) | **Q8IYS1** (PM20D2;48,-15,-0.31) | **Q0D2K3** (RIPPLY1;16,-15,-0.91) |
| **Q9H3C7** (GGNBP2;79,-15,-0.18) | **Q9UBK2** (PPARGC1A;91,-15,-0.16) | **Q7Z7G2** (CPLX4;18,-15,-0.81) |
| **Q9UKD1** (GMEB2;56,-15,-0.26) | **Q969Q6** (PPP2R3C;53,-15,-0.28) | **Q7Z4R8** (C6orf120;21,-15,-0.72) |
| **Q9H9Y4** (GPN2;35,-15,-0.43) | **P60510** (PPP4C;35,-15,-0.42) | **A0PJX0** (CIB4;22,-15,-0.68) |
| **P13807** (GYS1;84,-15,-0.17) | **Q92733** (PRCC;52,-15,-0.28) | **Q9NVP2** (ASF1B;22,-15,-0.66) |
| **Q7Z353** (HDX;77,-15,-0.19) | **P41219** (PRPH;54,-15,-0.27) | **Q6NXS1** (PPP1R2P3;23,-15,-0.65) |
| **Q15011** (HERPUD1;44,-15,-0.34) | **P49768** (PSEN1;53,-15,-0.28) | **P28066** (PSMA5;26,-15,-0.56) |
| **O75146** (HIP1R;119,-15,-0.12) | **Q14997** (PSME4;211,-15,-0.07) | **Q6IC83** (C22orf42;28,-15,-0.54) |
| **P20036** (HLA-DPA1;29,-15,-0.51) | **Q92565** (RAPGEF5;68,-15,-0.22) | **Q6ZRT6** (PRR23B;28,-15,-0.53) |
| **Q01581** (HMGCS1;57,-15,-0.26) | **Q96EP0** (RNF31;120,-15,-0.12) | **P61981** (YWHAG;28,-15,-0.52) |
| **P20823** (HNF1A;67,-15,-0.22) | **O75150** (RNF40;114,-15,-0.13) | **Q2T9L4** (C15orf59;32,-15,-0.46) |
| **P52597** (HNRNPF;46,-15,-0.32) | **Q92766** (RREB1;181,-15,-0.08) | **Q9GZS3** (WDR61;34,-15,-0.44) |
| **P78318** (IGBP1;39,-15,-0.38) | **P55735** (SEC13;36,-15,-0.42) | **Q6NSI1** (ANKRD26P1;35,-15,-0.42) |
| **O14920** (IKBKB;87,-15,-0.17) | **P01009** (SERPINA1;47,-15,-0.32) | **P0CV99** (TSPY4;36,-15,-0.41) |
| **Q8NAC3** (IL17RC;86,-15,-0.17) | **Q9UHJ3** (SFMBT1;98,-15,-0.15) | **P0CW01** (TSPY10;36,-15,-0.41) |
| **P01584** (IL1B;31,-15,-0.48) | **Q96EQ0** (SGTB;33,-15,-0.44) | **A8MV65** (VGLL3;36,-15,-0.41) |
| **Q9Y573** (IPP;65,-15,-0.22) | **Q9H173** (SIL1;52,-15,-0.28) | **Q7Z7C7** (STRA8;37,-15,-0.40) |
| **Q6NXR0** (IRGC;50,-15,-0.29) | **P55011** (SLC12A2;131,-15,-0.11) | **Q5WX9** (UBE2U;38,-15,-0.39) |
| **O75578** (ITGA10;128,-15,-0.11) | **Q504Y0** (SLC39A12;77,-15,-0.19) | **Q9NQS1** (AVEN;39,-15,-0.38) |
| **Q3ZCT8** (KBTBD12;71,-15,-0.21) | **Q695T7** (SLC6A19;71,-15,-0.21) | **Q9NYL9** (TMOD3;40,-15,-0.37) |
| **Q8IY47** (KBTBD2;71,-15,-0.21) | **Q92485** (SMPDL3B;51,-15,-0.29) | **Q7Z465** (BNIPL;40,-15,-0.37) |
| **P48051** (KCNJ6;48,-15,-0.30) | **075971** (SNAPC5;11,-15,-1.32) | **P08727** (KRT19;44,-15,-0.34) |
| **Q9H714** (KIAA0226L;73,-15,-0.20) | **Q96RF0** (SNX18;69,-15,-0.21) | **Q5H9B9** (BMP2KL;46,-15,-0.32) |
| **Q96J84** (KIRREL;84,-15,-0.17) | **Q9Y5X1** (SNX9;67,-15,-0.22) | **P49356** (FNTB;49,-15,-0.30) |
| **Q9NXS3** (KLHL28;64,-15,-0.23) | **Q96EA4** (SPDL1;70,-15,-0.21) | **P55010** (EIF5;49,-15,-0.30) |
| **P08779** (KRT16;51,-15,-0.29) | **O43295** (SRGAP3;125,-15,-0.12) | **O95264** (HTR3B;50,-15,-0.29) |
| **P13796** (LCP1;70,-15,-0.21) | **Q13586** (STIM1;77,-15,-0.19) | **P11926** (ODC1;51,-15,-0.29) |
| **P48357** (LEPR;132,-15,-0.11) | **Q9UH65** (SWAP70;69,-15,-0.21) | **Q9NWZ3** (IRAK4;52,-15,-0.29) |
| **Q5JTD7** (LRRC73;33,-15,-0.44) | **O75529** (TAF5L;66,-15,-0.22) | **O14896** (IRF6;53,-15,-0.28) |
| **Q0VAA2** (LRRC74;55,-15,-0.27) | **O60347** (TBC1D12;86,-15,-0.17) | **P17661** (DES;54,-15,-0.28) |
| **A6NEC2** (NPEPPSL1;54,-15,-0.27) | **P35613** (BSG;42,-14,-0.33) | **Q9BZI1** (IRX2;49,-14,-0.28) |
| **P04217** (A1BG;54,-15,-0.27) | **Q9BUW7** (C9orf16;9,-14,-1.54) | **P13612** (ITGA4;115,-14,-0.12) |
| **Q8IVJ1** (SLC41A1;55,-15,-0.27) | **Q86V15** (CASZ1;190,-14,-0.07) | **P14923** (JUP;82,-14,-0.17) |
| **Q92769** (HDAC2;55,-15,-0.27) | **Q9P2K1** (CC2D2A;186,-14,-0.07) | **Q9UGL1** (KDM5B;176,-14,-0.07) |
| **O95497** (VNN1;57,-15,-0.26) | **Q8N998** (CCDC89;44,-14,-0.31) | **Q6A163** (KRT39;56,-14,-0.25) |
| **Q9NUJ3** (TCP11L1;57,-15,-0.26) | **P41002** (CCNF;88,-14,-0.15) | **Q96JM7** (L3MBTL3;88,-14,-0.15) |
| **P55895** (RAG2;59,-15,-0.25) | **P48643** (CCT5;60,-14,-0.23) | **Q8NHL6** (LILRB1;71,-14,-0.19) |
| **Q6DD88** (ATL3;61,-15,-0.24) | **Q9BXL8** (CDCA4;26,-14,-0.53) | **Q9C0E8** (LNP;48,-14,-0.29) |
| **P54750** (PDE1A;61,-15,-0.24) | **Q86X02** (CDR2L;53,-14,-0.26) | **A6NDA9** (LRIT2;60,-14,-0.23) |
| **Q9BVS4** (RIOK2;63,-15,-0.23) | **Q9P209** (CEP72;72,-14,-0.19) | **Q6UWE0** (LRSAM1;84,-14,-0.16) |
| **Q1MX18** (INSC;63,-15,-0.23) | **Q5T4B2** (CERCAM;68,-14,-0.20) | **Q9Y6D9** (MAD1L1;83,-14,-0.16) |
| **O95460** (MATN4;68,-15,-0.21) | **O00748** (CES2;62,-14,-0.22) | **Q12852** (MAP3K12;93,-14,-0.15) |
| **Q6JEL2** (KLHL10;69,-15,-0.21) | **A5YKK6** (CNOT1;267,-14,-0.05) | **P41279** (MAP3K8;53,-14,-0.26) |
| **Q8N9H9** (C1orf127;70,-15,-0.21) | **O75175** (CNOT3;82,-14,-0.17) | **O60336** (MAPKBP1;164,-14,-0.08) |
| **Q53F19** (C17orf85;71,-15,-0.21) | **Q9Y2B0** (CNPY2;21,-14,-0.67) | **Q9NPJ6** (MED4;30,-14,-0.47) |
| **P13797** (PLS3;71,-15,-0.21) | **A8K830** (COLCA2;17,-14,-0.83) | **Q5VZV1** (METTL21C;30,-14,-0.47) |
| **I6L899** (GOLGA8R;71,-15,-0.20) | **Q5KU26** (COLEC12;82,-14,-0.17) | **Q9ULH7** (MKL2;118,-14,-0.11) |
| **A6NCC3** (GOLGA8O;72,-15,-0.20) | **P61201** (COPS2;52,-14,-0.27) | **Q6NW0** (MKRN9P;4,-14,-3.67) |
| **Q96PP9** (GBP4;73,-15,-0.20) | **P15085** (CPA1;47,-14,-0.29) | **Q6UVY6** (MOXD1;70,-14,-0.20) |
| **O15296** (ALOX15B;76,-15,-0.19) | **Q9HC73** (CRLF2;42,-14,-0.33) | **Q9P289** (MST4;47,-14,-0.30) |
| **Q6ZSI9** (CAPN12;81,-15,-0.18) | **Q9UBG3** (CRNN;54,-14,-0.26) | **P04198** (MYCN;50,-14,-0.28) |
| **O43506** (ADAM20;82,-15,-0.18) | **P61962** (DCAF7;39,-14,-0.35) | **Q9Y2A7** (NCKAP1;129,-14,-0.10) |
| **Q9NVE7** (PANK4;86,-15,-0.17) | **Q8N9W5** (DNAAF3;59,-14,-0.23) | **Q92597** (NDRG1;43,-14,-0.32) |
| **P42892** (ECE1;87,-15,-0.17) | **Q92874** (DNASE1L2;33,-14,-0.42) | **Q9UN36** (NDRG2;41,-14,-0.34) |
| **P18084** (ITGB5;88,-15,-0.17) | **Q96FX2** (DPH3;9,-14,-1.51) | **Q86W24** (NLRP14;125,-14,-0.11) |
| **Q9NXL2** (ARHGEF38;89,-15,-0.16) | **Q9H4G8** (DPH3P1;9,-14,-1.60) | **Q9H8H0** (NOL11;81,-14,-0.17) |
| **P42226** (STAT6;94,-15,-0.15) | **A6NNW6** (ENO4;69,-14,-0.20) | **Q86YC3** (NRROS;76,-14,-0.18) |
| **O00206** (TLR4;96,-15,-0.15) | **O14638** (ENPP3;100,-14,-0.13) | **P49902** (NT5C2;65,-14,-0.21) |
| **Q15700** (DLG2;98,-15,-0.15) | **Q9Y6X5** (ENPP4;52,-14,-0.27) | **P0CE72** (OCM;12,-14,-1.14) |
| **Q68CR7** (LRRC66;98,-15,-0.15) | **Q9UJA9** (ENPP5;55,-14,-0.25) | **Q969R2** (OSBP2;101,-14,-0.13) |
| **Q9UI47** (CTNNA3;100,-15,-0.15) | **P29317** (EPHA2;108,-14,-0.12) | **Q6ZW49** (PAXIP1;121,-14,-0.11) |
| **P43246** (MSH2;105,-15,-0.14) | **Q9NQ60** (EQTN;33,-14,-0.42) | **Q9NQP4** (PFDN4;15,-14,-0.91) |
| **P12110** (COL6A2;109,-15,-0.13) | **Q06265** (EXOSC9;49,-14,-0.28) | **Q8NDX5** (PHC3;106,-14,-0.13) |
| **P20648** (ATP4A;114,-15,-0.13) | **O94988** (FAM13A;117,-14,-0.11) | **P42356** (PI4KA;231,-14,-0.06) |
| **Q9BX69** (CARD6;116,-15,-0.12) | **Q5VUB5** (FAM171A1;98,-14,-0.14) | **Q9NRD5** (PICK1;47,-14,-0.30) |
| **O15294** (OGT;117,-15,-0.12) | **P02679** (FGG;52,-14,-0.27) | **Q5SXH7** (PLEKHS1;52,-14,-0.27) |
| **P14735** (IDE;118,-15,-0.12) | **Q02790** (FKBP4;52,-14,-0.27) | **Q9HCM2** (PLXNA4;212,-14,-0.06) |
| **Q9Y3R0** (GRIP1;122,-15,-0.12) | **Q8NFG4** (FLCN;64,-14,-0.21) | **P54317** (PNLIPRP2;52,-14,-0.26) |
| **Q9UKP5** (ADAMTS6;125,-15,-0.11) | **Q16676** (FOXD1;46,-14,-0.30) | **O15160** (POLR1C;39,-14,-0.35) |
| **P56199** (ITGA1;131,-15,-0.11) | **O15353** (FOXN1;69,-14,-0.20) | **Q6NYC8** (PPP1R18;68,-14,-0.20) |
| **Q00722** (PLCB2;134,-15,-0.11) | **Q68DX3** (FRMPD2;144,-14,-0.09) | **P16298** (PPP3CB;59,-14,-0.23) |
| **O75747** (PIK3C2G;166,-15,-0.09) | **P09958** (FURIN;87,-14,-0.16) | **Q5W67** (PPRC1;178,-14,-0.07) |
| **Q5QGS0** (KIAA2022; 168,-15,-0.08) | **P48506** (GCLC;73,-14,-0.19) | **Q4J6C6** (PREPL;84,-14,-0.16) |
| **Q9H2U9** (ADAM7;86,-14,-0.16) | **Q9NWU2** (GID8;27,-14,-0.52) | **Q15139** (PRKD1;102,-14,-0.13) |
| **Q9NZN9** (AIPL1;44,-14,-0.31) | **Q9BRT9** (GINS4;26,-14,-0.53) | **P78527** (PRKDC;469,-14,-0.02) |
| **Q8NAG6** (ANKLE1;67,-14,-0.20) | **P19087** (GNAT2;40,-14,-0.34) | **P07225** (PROS1;75,-14,-0.18) |
| **Q6UB98** (ANKRD12;236,-14,-0.05) | **Q3KR37** (GRAMD1B;85,-14,-0.16) | **Q14289** (PTK2B;116,-14,-0.12) |
| **Q16853** (AOC3;85,-14,-0.16) | **Q9P0R6** (GSKIP;16,-14,-0.89) | **Q9H3S7** (PTPN23;179,-14,-0.07) |
| **Q92870** (APBB2;83,-14,-0.16) | **Q96MB7** (HARBI1;39,-14,-0.35) | **Q9UHX1** (PUF60;60,-14,-0.23) |
| **P07306** (ASGR1;33,-14,-0.42) | **Q9BY41** (HDAC8;42,-14,-0.33) | **O60671** (RAD1;32,-14,-0.43) |
| **Q9ULI0** (ATAD2B;165,-14,-0.08) | **O75330** (HMMR;84,-14,-0.16) | **P20936** (RASA1;116,-14,-0.12) |
| **Q7Z3C6** (ATG9A;94,-14,-0.14) | **Q9NZL4** (HSPBP1;39,-14,-0.35) | **Q13127** (REST;122,-14,-0.11) |
| **P38606** (ATP6V1A;68,-14,-0.20) | **Q14627** (IL13RA2;44,-14,-0.31) | **Q52LD8** (RFTN2;56,-14,-0.25) |
| **P35670** (ATP7B;157,-14,-0.08) | **Q14116** (IL18;22,-14,-0.62) | **Q8HWS3** (RFX6;102,-14,-0.13) |
| **P56817** (BACE1;56,-14,-0.25) | **Q6UWB1** (IL27RA;69,-14,-0.20) | **Q8NET4** (RGAG1; 144,-14,-0.09) |
| **O95429** (BAG4;50,-14,-0.28) | **Q16352** (INA;55,-14,-0.25) | **Q13546** (RIPK1;76,-14,-0.18) |
| **P46736** (BRCC3;36,-14,-0.38) | **O15357** (INPPL1;139,-14,-0.10) | **P12271** (RLBP1;36,-14,-0.38) |
| **Q9Y4L5** (RNF115;34,-14,-0.41) | **P63104** (YWHAZ;28,-14,-0.50) | **A6ND36** (FAM83G;91,-14,-0.15) |
| **Q9ULK6** (RNF150;48,-14,-0.29) | **P27348** (YWHAQ;28,-14,-0.50) | **Q8WUH2** (TGFBRAP1 ;97,-14,-0.14) |
| **Q9P0P0** (RNF181;18,-14,-0.78) | **P31946** (YWHAB;28,-14,-0.49) | **Q2TAC2** (CCDC57;103,-14,-0.13) |
| **Q6ZMZ0** (RNF19B;78,-14,-0.17) | **Q8IWD4** (CCDC117;31,-14,-0.45) | **Q86W56** (PARG;111,-14,-0.12) |
| **Q9UK32** (RPS6KA6;84,-14,-0.16) | **Q92537** (KIAA0247;32,-14,-0.43) | **Q8N9B5** (JMY;111,-14,-0.12) |
| **P08865** (RPSA;33,-14,-0.42) | **A6NDU8** (C5orf51;34,-14,-0.41) | **Q9UQ52** (CNTN6;114,-14,-0.12) |
| **Q86UC2** (RSPH3;64,-14,-0.21) | **O14579** (COPE;34,-14,-0.40) | **A6QL63** (BTBD11;121,-14,-0.11) |
| **Q9BVN2** (RUSC1;96,-14,-0.14) | **P0CW00** (TSPY8;35,-14,-0.39) | **Q5M9N0** (CCDC158;127,-14,-0.11) |
| **O43765** (SGTA;34,-14,-0.41) | **Q99795** (GPA33;36,-14,-0.39) | **Q6MZM0** (HEPHL1;132,-14,-0.10) |
| **Q8IX90** (SKA3;46,-14,-0.30) | **A8MYB1** (TMCO5B;36,-14,-0.39) | **O95487** (SEC24B;137,-14,-0.10) |
| **Q9UP95** (SLC12A4;121,-14,-0.11) | **A6NNH0** (GATSL1;36,-14,-0.38) | **Q07889** (SOS1;152,-14,-0.09) |
| **Q96RN1** (SLC26A8;109,-14,-0.12) | **A2A368** (MAGEB16;36,-14,-0.38) | **Q8WWZ4** (ABCA10;176,-14,-0.07) |
| **Q9Y6M7** (SLC4A7;136,-14,-0.10) | **Q9H6J7** (C11orf49;37,-14,-0.37) | **A6NJZ7** (RIMBP3C;181,-14,-0.07) |
| **A6NLE4** (SMIM23;18,-14,-076) | **B7ZBB8** (PPP1R3G;38,-14,-0.36) | **A6NNM3** (RIMBP3B;181,-14,-0.07) |
| **P60880** (SNAP25;23,-14,-0.60) | **P46597** (ASMT;38,-14,-0.36) | **A6NMZ7** (COL6A6;247,-14,-0.05) |
| **Q81U14** (SNX29P2;27,-14.-0.51) | **Q15166** (PON3;40,-14,-0.35) | **Q81UG5** (MYO18B;285,-14,-0.04) |
| **Q5TF21** (SOGA3;103,-14,-0.13) | **Q13477** (MADCAM1;40,-14,-0.34) | **P15924** (DSP;332,-14,-0.04) |
| **Q9UPU3** (SORCS3;136,-14,-0.10) | **Q8N8Y2** (ATP6V0D2;40,-14,-0.34) | **P68133** (ACTA1;42,-13,-0.30) |
| **Q8NB90** (SPATA5;98,-14,-0.14) | **Q6AI12** (ANKRD40;41,-14,-0.34) | **P62736** (ACTA2;42,-13,-0.30) |
| **P50502** (ST13;41,-14,-0.33) | **Q6ZQY2** (0;42,-14,-0.33) | **P68032** (ACTC1;42,-13,-0.30) |
| **Q8IZP2** (ST13P4;27,-14,-0.51) | **P04220** (0;43,-14,-0.32) | **Q8TDY3** (ACTRT2;42,-13,-0.31) |
| **O95210** (STBD1;39,-14,-0.35) | **Q96G97** (BSCL2;44,-14,-0.31) | **P27037** (ACVR2A;58,-13,-0.22) |
| **Q9Y6Q2** (STON1;83,-14,-0.16) | **Q9Y614** (ACTL7B;45,-14,-0.30) | **P43652** (AFM;69,-13,-0.18) |
| **O60499** (STX10;28,-14,-0.49) | **P06727** (APOA4;45,-14,-0.30) | **P02765** (AHSG;39,-13,-0.33) |
| **Q15544** (TAF11;23,-14,-0.60) | **Q13515** (BFSP2;46,-14,-0.30) | **Q9NP73** (ALG13;126,-13,-0.10) |
| **Q9UPU7** (TBC1D2B;110,-14,-0.12) | **Q14653** (IRF3;47,-14,-0.29) | **P18054** (ALOX12;76,-13,-0.17) |
| **Q96NH3** (TBC1D32;145,-14,-0.09) | **Q0VDG4** (SCRN3;49,-14,-0.28) | **Q9UKV5** (AMFR;73,-13,-0.17) |
| **Q8TEA7** (TBCK;101,-14,-0.13) | **Q5VWJ9** (SNX30;50,-14,-0.28) | **Q16671** (AMHR2;63,-13,-0.20) |
| **A7MCY6** (TBKBP1;68,-14,-0.20) | **P02533** (KRT14;52,-14,-0.27) | **Q9UJ72** (ANXA10;37,-13,-0.34) |
| **Q9BXS4** (TMEM59;36,-14,-0.38) | **P00740** (F9;52,-14,-0.27) | **P08758** (ANXA5;36,-13,-0.36) |
| **Q9BSE2** (TMEM79;44,-14,-0.32) | **P15289** (ARSA;54,-14,-0.26) | **Q8N9N2** (ASCC1;46,-13,-0.28) |
| **Q71RG4** (TMUB2;34,-14,-0.41) | **Q9Y4I5** (MTL5;55,-14,-0.25) | **Q6ZU67** (BEND4;58,-13,-0.22) |
| **O95271** (TNKS;142,-14,-0.09) | **Q8NA56** (TTC29;55,-14,-0.25) | **Q9ULD4** (BRPF3;136,-13,-0.09) |
| **Q59H18** (TNNI3K;93,-14,-0.15) | **Q9NXN4** (GDAP2;56,-14,-0.24) | **Q13410** (BTN1A1;59,-13,-0.22) |
| **Q9Y2L5** (TRAPPC8;161,-14,-0.08) | **Q8NCR0** (B3GALNT2;57,-14,-0.24) | **Q8N5S9** (CAMKK1;56,-13,-0.23) |
| **Q15642** (TRIP10;68,-14,-0.20) | **P09923** (ALPI;57,-14,-0.24) | **Q08AD1** (CAMSAP2;168,-13,-0.07) |
| **Q9NX07** (TRNAU1AP;32,-14,-0.43) | **Q8WUA2** (PPIL4;57,-14,-0.24) | **P07384** (CAPN1;82,-13,-0.15) |
| **Q6PKC3** (TXNDC11;111,-14,-0.12) | **Q8TBB5** (KLHDC4;58,-14,-0.24) | **Q6UXS9** (CASP12;39,-13,-0.33) |
| **Q9UMX0** (UBQLN1;63,-14,-0.22) | **O75191** (XYLB;58,-14,-0.23) | **P83916** (CBX1;21,-13,-0.60) |
| **Q86T82** (USP37;110,-14,-0.12) | **P32942** (ICAM3;60,-14,-0.23) | **Q8WV48** (CCDC107;31,-13,-0.42) |
| **Q8N1B4** (VPS52;82,-14,-0.17) | **Q15822** (CHRNA2;60,-14,-0.23) | **Q9NUG4** (CCM2L;62,-13,-0.20) |
| **Q6UX27** (VSTM1;26,-14,-0.53) | **P51687** (SUOX;60,-14,-0.23) | **P24385** (CCND1;34,-13,-0.38) |
| **Q96DN2** (VWCE;100,-14,-0.14) | **H3BUK9** (POTEB2;62,-14,-0.22) | **Q96SF2** (CCT8L2;59,-13,-0.21) |
| **Q6R2W3** (ZBED9;152,-14,-0.09) | **O95741** (CPNE6;62,-14,-0.22) | **O43866** (CD5L;38,-13,-0.34) |
| **Q8N680** (ZBTB2;57,-14,-0.24) | **Q96A19** (CCDC102A;63,-14,-0.22) | **Q8N8E3** (CEP112;113,-13,-0.11) |
| **Q9NWS9** (ZNF446;49,-14,-0.28) | **A6NN90** (C2orf81;63,-14,-0.22) | **Q9Y592** (CEP83;82,-13,-0.15) |
| **Q96NB3** (ZNF830;42,-14,-0.33) | **Q9NQ89** (C12orf4;64,-14,-0.21) | **Q6UWW8** (CES3;62,-13,-0.20) |
| **Q8WXC6** (MYEOV2;6,-14,-2.25) | **Q9NY59** (SMPD3;71,-14,-0.19) | **Q96EP1** (CHFR;73,-13,-0.17) |
| **P04271** (S100B;11,-14,-1.30) | **Q9H089** (LSG1;75,-14,-0.18) | **Q9Y259** (CHKB;45,-13,-0.28) |
| **Q9NNZ6** (PRM3;11,-14,-1.24) | **Q13480** (GAB1;77,-14,-0.18) | **P11230** (CHRNB1;57,-13,-0.22) |
| **P60660** (MYL6;17,-14,-0.82) | **Q9H892** (TTC12;79,-14,-0.17) | **Q04844** (CHRNE;55,-13,-0.23) |
| **Q6ZWK4** (C1orf186; 19,-14,-072) | **Q8IWA4** (MFN1;84,-14,-0.16) | **Q99828** (CIB1;22,-13,-0.59) |
| **P62256** (UBE2H;21,-14,-0.67) | **Q9UL63** (MKLN1;85,-14,-0.16) | **Q9H2X3** (CLEC4M;45,-13,-0.28) |
| **B2RUY7** (VWC2L;25,-14,-0.56) | **O75843** (AP1G2;87,-14,-0.16) | **Q96SW2** (CRBN;51,-13,-0.25) |
| **Q8IXP5** (C11orf53;25,-14,-0.55) | **P42224** (STAT1;87,-14,-0.16) | **Q8TEY5** (CREB3L4;43,-13,-0.29) |
| **Q9UNT1** (RABL2B;26,-14,-0.53) | **Q58DX5** (NAALADL2;89,-14,-0.15) | **Q6UUV9** (CRTC1;67,-13,-0.19) |
| **Q9UBK7** (RABL2A;26,-14,-0.53) | **O43264** (ZW10;89,-14,-0.15) | **O75534** (CSDE1;89,-13,-0.14) |
| **Q96RT6** (CTAGE1;85,-13,-0.15) | **P36776** (LONP1;106,-13,-0.12) | **O43304** (SEC14L5;79,-13,-0.16) |
| **Q96HY6** (DDRGK1;36,-13,-0.36) | **Q8N386** (LRRC25;33,-13,-0.39) | **Q9UIV8** (SERPINB13;44,-13,-0.29) |
| **Q13838** (DDX39B;49,-13,-0.26) | **Q14392** (LRRC32;72,-13,-0.18) | **O75830** (SERPINI2;46,-13,-0.28) |
| **O00548** (DLL1;78,-13,-0.16) | **Q6UXK5** (LRRN1;81,-13,-0.16) | **P58005** (SESN3;57,-13,-0.22) |
| **Q6P3W2** (DNAJC24;17,-13,-0.76) | **P36941** (LTBR;47,-13,-0.27) | **O14492** (SH2B2;68,-13,-0.19) |
| **P24855** (DNASE1;31,-13,-0.41) | **Q96LR2** (LURAP1;26,-13,-0.50) | **Q99962** (SH3GL2;40,-13,-0.32) |
| **Q92608** (DOCK2;212,-13,-0.06) | **P49641** (MAN2A2;131,-13,-0.09) | **Q81XJ6** (SIRT2;43,-13,-0.30) |
| **Q8N608** (DPP10;91,-13,-0.14) | **Q15555** (MAPRE2;37,-13,-0.35) | **Q86UW2** (SLC51B;14,-13,-0.90) |
| **Q9HAV5** (EDA2R;33,-13,-0.39) | **P49006** (MARCKSL1;20,-13,-0.66) | **Q8TEQ0** (SNX29;91,-13,-0.14) |
| **P05198** (EIF2S1;36,-13,-0.35) | **Q495T6** (MMEL1;89,-13,-0.14) | **O15370** (SOX12;34,-13,-0.38) |
| **Q99607** (ELF4;71,-13,-0.18) | **Q99549** (MPHOSPH8;97,-13,-0.13) | **Q15506** (SPA17;17,-13,-0.74) |
| **Q92556** (ELMO1;84,-13,-0.15) | **P42345** (MTOR;289,-13,-0.04) | **Q8N0X7** (SPG20;73,-13,-0.17) |
| **Q7L775** (EPM2AIP1;70,-13,-0.18) | **Q8N387** (MUC15;36,-13,-0.35) | **O95772** (STARD3NL;27,-13,-0.48) |
| **Q96HE7** (ERO1L;54,-13,-0.23) | **P01106** (MYC;49,-13,-0.26) | **Q5VSL9** (STRIP1;96,-13,-0.13) |
| **P50549** (ETV1;55,-13,-0.23) | **Q92802** (N4BP2L2;67,-13,-0.19) | **Q8N205** (SYNE4;44,-13,-0.29) |
| **Q8NHP7** (EXD1;58,-13,-0.22) | **Q5TF39** (NAGLT1;56,-13,-0.23) | **P15923** (TCF3;68,-13,-0.19) |
| **Q96A65** (EXOC4;110,-13,-0.11) | **P41271** (NBL1;19,-13,-0.66) | **A2RU30** (TESPA1;59,-13,-0.21) |
| **Q9Y2D4** (EXOC6B;94,-13,-0.13) | **Q15596** (NCOA2;159,-13,-0.08) | **Q5TEJ8** (THEMIS2;72,-13,-0.18) |
| **Q96CS3** (FAF2;53,-13,-0.24) | **Q9HC29** (NOD2;115,-13,-0.11) | **P51854** (TKTL1;65,-13,-0.19) |
| **Q8TCP9** (FAM200A;66,-13,-0.19) | **Q7Z494** (NPHP3;151,-13,-0.08) | **Q6P9B6** (TLDC1;51,-13,-0.25) |
| **P0C7Q3** (FAM58BP;29,-13,-0.45) | **Q16288** (NTRK3;94,-13,-0.13) | **Q9Y2Y6** (TMEM98;25,-13,-0.52) |
| **Q96KN4** (FAM84A;32,-13,-0.40) | **Q9H1E3** (NUCKS1;27,-13,-0.47) | **P28289** (TMOD1;41,-13,-0.32) |
| **Q9HB96** (FANCE;59,-13,-0.22) | **Q5W17** (OTUD1;51,-13,-0.25) | **O14763** (TNFRSF10B;48,-13,-0.27) |
| **Q7L513** (FCRLA;39,-13,-0.33) | **Q01804** (OTUD4;124,-13,-0.10) | **O14798** (TNFRSF10C;27,-13,-0.47) |
| **P09769** (FGR;59,-13,-0.21) | **Q86U42** (PABPN1;33,-13,-0.39) | **Q9H2S6** (TNMD;37,-13,-0.35) |
| **Q4L180** (FILIP1L;130,-13,-0.09) | **Q9BYG5** (PARD6B;41,-13,-0.31) | **Q9NS69** (TOMM22;16,-13,-0.83) |
| **P53539** (FOSB;36,-13,-0.36) | **Q16342** (PDCD2;39,-13,-0.33) | **Q9Y4K3** (TRAF6;60,-13,-0.21) |
| **Q14393** (GAS6;80,-13,-0.16) | **Q5VY43** (PEAR1;111,-13,-0.11) | **O43280** (TREH;67,-13,-0.19) |
| **Q8N8V2** (GBP7;73,-13,-0.17) | **O95394** (PGM3;60,-13,-0.21) | **Q9UPN9** (TRIM33;123,-13,-0.10) |
| **Q96QA5** (GSDMA;49,-13,-0.26) | **Q9H814** (PHAX;44,-13,-0.29) | **Q8WW01** (TSEN15;19,-13,-0.69) |
| **Q9Y5Z4** (HEBP2;23,-13,-0.56) | **P40967** (PMEL;70,-13,-0.18) | **Q9UJT2** (TSKS;65,-13,-0.19) |
| **Q5T8I9** (HENMT1;45,-13,-0.29) | **Q9H5K3** (POMK;40,-13,-0.32) | **Q9BSA4** (TTYH2;59,-13,-0.22) |
| **P01903** (HLA-DRA;29,-13,-0.45) | **P37231** (PPARG;58,-13,-0.22) | **Q9UGJ1** (TUBGCP4;76,-13,-0.17) |
| **P13747** (HLA-E;40,-13,-0.32) | **Q9Y2Y8** (PRG3;25,-13,-0.51) | **Q8NBS9** (TXNDC5;48,-13,-0.27) |
| **O60812** (HNRNPCL1;32,-13,-0.40) | **Q9UGI9** (PRKAG3;54,-13,-0.23) | **O43396** (TXNL1;32,-13,-0.40) |
| **Q00839** (HNRNPU;91,-13,-0.14) | **Q99873** (PRMT1;42,-13,-0.31) | **P17643** (TYRP1;61,-13,-0.21) |
| **Q86YM7** (HOMER1;40,-13,-0.32) | **Q96LA8** (PRMT6;42,-13,-0.30) | **Q8TCY9** (URGCP;105,-13,-0.12) |
| **P0CW71** (HSMCR30;33,-13,-0.39) | **Q8N271** (PROM2;92,-13,-0.14) | **Q2YD98** (UVSSA;81,-13,-0.16) |
| **P11142** (HSPA8;71,-13,-0.18) | **P17980** (PSMC3;49,-13,-0.26) | **Q9H867** (VCPKMT;26,-13,-0.50) |
| **Q96LB3** (IFT74;69,-13,-0.18) | **Q16401** (PSMD5;56,-13,-0.23) | **Q9Y4E6** (WDR7;164,-13,-0.07) |
| **Q08334** (IL10RB;37,-13,-0.35) | **Q5TGL8** (PXDC1;27,-13,-0.48) | **Q9ULE0** (WWC3;123,-13,-0.10) |
| **Q01344** (IL5RA;48,-13,-0.27) | **Q9BYM8** (RBCK1;58,-13,-0.22) | **Q9GZV5** (WWTR1;44,-13,-0.29) |
| **Q12905** (ILF2;43,-13,-0.30) | **Q96EV2** (RBM33;130,-13,-0.10) | **Q04917** (YWHAH;28,-13,-0.46) |
| **Q9UKX5** (ITGA11;133,-13,-0.09) | **Q2KHR2** (RFX7;147,-13,-0.08) | **Q14202** (ZMYM3;152,-13,-0.08) |
| **Q6GPH6** (ITPRIPL1;63,-13,-0.20) | **O15211** (RGL2;84,-13,-0.15) | **Q8TD17** (ZNF398;71,-13,-0.18) |
| **Q96AA8** (JAKMIP2;95,-13,-0.13) | **Q05823** (RNASEL;84,-13,-0.15) | **Q86VK4** (ZNF410;52,-13,-0.24) |
| **Q9P0J7** (KCMF1;42,-13,-0.30) | **Q5VTB9** (RNF220;63,-13,-0.20) | **O43309** (ZSCAN12;70,-13,-0.18) |
| **P48544** (KCNJ5;48,-13,-0.27) | **Q9HCK4** (ROBO2;151,-13,-0.08) | **Q9BS18** (ANAPC13;9,-13,-1.52) |
| **Q8NC54** (KCT2;29,-13,-0.44) | **Q01974** (ROR2;105,-13,-0.12) | **A6NHS1** (0;10,-13,-1.27) |
| **Q9H3R0** (KDM4C;120,-13,-0.10) | **Q7L099** (RUFY3;53,-13,-0.24) | **Q53QV2** (LBH;12,-13,-1.06) |
| **Q8N371** (KDM8;47,-13,-0.27) | **Q59EK9** (RUNDC3A;50,-13,-0.26) | **Q92748** (THRSP;17,-13,-0.78) |
| **Q9NSK0** (KLC4;69,-13,-0.18) | **Q9NSI8** (SAMSN1;42,-13,-0.31) | **E9PB15** (PTGES3L;19,-13,-0.68) |
| **Q9NR64** (KLHL1;83,-13,-0.15) | **O95248** (SBF1;208,-13,-0.06) | **Q6P9G4** (TMEM154;20,-13,-0.63) |
| **Q96NJ5** (KLHL32;70,-13,-0.18) | **Q6UWP8** (SBSN;61,-13,-0.21) | **Q8IYI0** (C20orf196;23,-13,-0.56) |
| **P52292** (KPNA2;58,-13,-0.22) | **Q14162** (SCARF1;87,-13,-0.14) | **O75496** (GMNN;24,-13,-0.55) |
| **Q9GZY6** (LAT2;27,-13,-0.48) | **Q9NY72** (SCN3B;25,-13,-0.52) | **Q8WWG9** (KCNE4;24,-13,-0.54) |
| **O75473** (LGR5;100,-13,-0.13) | **Q9NQ36** (SCUBE2;110,-13,-0.11) | **Q9NPB3** (CABP2;24,-13,-0.53) |
| **Q9HAE3** (EFCAB1;24,-13,-0.53) | **O43196** (MSH5;93,-13,-0.13) | **Q9NQI0** (DDX4;79,-12,-0.15) |
| **P20396** (TRH;27,-13,-0.47) | **Q9Y6H5** (SNCAIP;100,-13,-0.12) | **Q9H4E7** (DEF6;74,-12,-0.16) |
| **A6NLX3** (SPDYE4;28,-13,-0.46) | **Q9UDR5** (AASS;102,-13,-0.12) | **Q5VZ89** (DENND4C;187,-12,-0.06) |
| **P20941** (PDC;28,-13,-0.46) | **Q0P6D6** (CCDC15;110,-13,-0.11) | **Q9BSY9** (DESI2;21,-12,-0.55) |
| **Q6ZMS7** (ZNF783;31,-13,-0.41) | **O15197** (EPHB6;111,-13,-0.11) | **O95424** (DEXI;10,-12,-1.15) |
| **Q8WVE6** (TMEM171;35,-13,-0.37) | **Q9BXX0** (EMILIN2;116,-13,-0.11) | **P49366** (DHPS;41,-12,-0.29) |
| **P0CV98** (TSPY3;35,-13,-0.37) | **Q81XT5** (RBM12B;118,-13,-0.11) | **Q7Z6W7** (DNAJB7;35,-12,-0.33) |
| **P62714** (PPP2CB;36,-13,-0.36) | **Q9NNW5** (WDR6;122,-13,-0.10) | **Q96CJ1** (EAF2;29,-12,-0.41) |
| **P0C870** (JMJD7;36,-13,-0.36) | **Q3MJ13** (WDR72;123,-13,-0.10) | **P00533** (EGFR;134,-12,-0.08) |
| **A6NHX0** (GATSL2;36,-13,-0.36) | **Q6ZWH5** (NEK10;133,-13,-0.09) | **Q13347** (EIF3I;37,-12,-0.32) |
| **Q8NFH4** (NUP37;37,-13,-0.35) | **Q9BXT6** (MOV10L1;135,-13,-0.09) | **Q9UBQ5** (EIF3K;25,-12,-0.47) |
| **Q9HBI0** (PARVG;37,-13,-0.34) | **P02452** (COL1A1;139,-13,-0.09) | **Q8TE68** (EPS8L1;80,-12,-0.14) |
| **Q9Y266** (NUDC;38,-13,-0.33) | **P16885** (PLCG2;148,-13,-0.08) | **Q6P179** (ERAP2;110,-12,-0.10) |
| **Q16589** (CCNG2;39,-13,-0.33) | **Q9UHC9** (NPC1L1;149,-13,-0.08) | **Q9Y282** (ERGIC3;43,-12,-0.27) |
| **Q9NTX7** (RNF146;39,-13,-0.33) | **Q07890** (SOS2;153,-13,-0.08) | **Q96DZ1** (ERLEC1;55,-12,-0.21) |
| **Q9Y2Z0** (SUGT1;41,-13,-0.31) | **Q86V21** (AACS;75,-12,-0.15) | **Q8N693** (ESX1;44,-12,-0.27) |
| **Q99638** (RAD9A;43,-13,-0.30) | **O95477** (ABCA1;254,-12,-0.04) | **Q9NV70** (EXOC1;102,-12,-0.11) |
| **O00470** (MEIS1;43,-13,-0.30) | **Q9P2A4** (ABI3;39,-12,-0.30) | **O60645** (EXOC3;87,-12,-0.13) |
| **Q96K31** (C8orf76;43,-13,-0.30) | **Q9NR19** (ACSS2;79,-12,-0.15) | **Q17RC7** (EXOC3L4;80,-12,-0.15) |
| **Q9NNX1** (TUFT1;44,-13,-0.29) | **P63261** (ACTG1;42,-12,-0.28) | **Q15024** (EXOSC7;32,-12,-0.37) |
| **P17050** (NAGA;47,-13,-0.27) | **P63267** (ACTG2;42,-12,-0.28) | **O43909** (EXTL3;105,-12,-0.11) |
| **Q9GZL7** (WDR12;48,-13,-0.27) | **Q86TH1** (ADAMTSL2;105,-12,-0.11) | **P00488** (F13A1;83,-12,-0.14) |
| **Q5VXM1** (CDCP2;49,-13,-0.26) | **Q9UEY8** (ADD3;79,-12,-0.15) | **Q8IW50** (FAM219A;20,-12,-0.58) |
| **Q9UIF3** (TEKT2;50,-13,-0.26) | **O75969** (AKAP3;95,-12,-0.12) | **Q96KN1** (FAM84B;34,-12,-0.34) |
| **P14136** (GFAP;50,-13,-0.26) | **Q9UJX3** (ANAPC7;67,-12,-0.17) | **Q00597** (FANCC;63,-12,-0.18) |
| **A6NHL2** (TUBAL3;50,-13,-0.26) | **Q9GZV1** (ANKRD2;40,-12,-0.30) | **Q969U6** (FBXW5;64,-12,-0.18) |
| **Q9BX59** (TAPBPL;50,-13,-0.25) | **Q53RT3** (ASPRV1;37,-12,-0.32) | **Q9UK73** (FEM1B;70,-12,-0.17) |
| **Q8N594** (MPND;51,-13,-0.25) | **Q8WWH4** (ASZ1;53,-12,-0.22) | **Q9Y613** (FHOD1;127,-12,-0.09) |
| **Q9NRH3** (TUBG2;51,-13,-0.25) | **P15313** (ATP6V1B1;57,-12,-0.21) | **Q14254** (FLOT2;47,-12,-0.25) |
| **P49005** (POLD2;51,-13,-0.25) | **P54687** (BCAT1;43,-12,-0.27) | **O15409** (FOXP2;80,-12,-0.15) |
| **Q8TD10** (MIPOL1;52,-13,-0.25) | **Q5TBC7** (BCL2L15;18,-12,-0.67) | **A2A2Y4** (FRMD3;69,-12,-0.17) |
| **P16871** (IL7R;52,-13,-0.25) | **Q13489** (BIRC3;68,-12,-0.17) | **Q9H227** (GBA3;54,-12,-0.22) |
| **Q92569** (PIK3R3;54,-13,-0.23) | **O75808** (CAPN15;117,-12,-0.10) | **Q9NU53** (GINM1;37,-12,-0.32) |
| **Q96NZ1** (FOXN4;55,-13,-0.23) | **Q68D86** (CCDC102B;60,-12,-0.19) | **Q9Y625** (GPC6;63,-12,-0.19) |
| **Q8WU10** (PYROXD1;56,-13,-0.23) | **Q05D60** (CCDC67;71,-12,-0.16) | **Q8IV16** (GPIHBP1;20,-12,-0.60) |
| **I3L273** (GFY;56,-13,-0.23) | **Q8TD31** (CCHCR1;89,-12,-0.13) | **P81274** (GPSM2;77,-12,-0.15) |
| **O60381** (HBP1;58,-13,-0.22) | **Q8WWL7** (CCNB3;158,-12,-0.07) | **A4D1B5** (GSAP;98,-12,-0.12) |
| **Q9NVN3** (RIC8B;59,-13,-0.22) | **P14209** (CD99;19,-12,-0.63) | **Q9UMX6** (GUCA1B;23,-12,-0.51) |
| **Q9NW07** (ZNF358;59,-13,-0.21) | **Q9BXF3** (CECR2;164,-12,-0.07) | **Q9UKV0** (HDAC9;111,-12,-0.10) |
| **Q9H6K5** (0;60,-13,-0.21) | **Q9C0F1** (CEP44;44,-12,-0.27) | **Q96JB3** (HIC2;66,-12,-0.18) |
| **P14679** (TYR;60,-13,-0.21) | **P08603** (CFH;139,-12,-0.08) | **P30511** (HLA-F;39,-12,-0.30) |
| **Q96JW4** (SLC41A2;62,-13,-0.20) | **Q9NZZ3** (CHMP5;25,-12,-0.48) | **P07910** (HNRNPC;34,-12,-0.35) |
| **P02748** (C9;63,-13,-0.20) | **Q9NRU3** (CNNM1;104,-12,-0.11) | **Q9UMF0** (ICAM5;97,-12,-0.12) |
| **P49023** (PXN;65,-13,-0.20) | **Q6P4Q7** (CNNM4;87,-12,-0.13) | **Q9Y6K9** (IKBKG;48,-12,-0.24) |
| **Q8IV77** (CNGA4;66,-13,-0.19) | **Q53SF7** (COBLL1;132,-12,-0.09) | **Q13422** (IKZF1;58,-12,-0.20) |
| **Q9H900** (ZWILCH;67,-13,-0.19) | **Q8IYK4** (COLGALT2;73,-12,-0.16) | **Q6UXL0** (IL20RB;35,-12,-0.34) |
| **Q5TGY1** (TMCO4;68,-13,-0.19) | **Q7L5N1** (COPS6;36,-12,-0.33) | **Q9NV88** (INTS9;74,-12,-0.16) |
| **Q04864** (REL;69,-13,-0.18) | **Q99829** (CPNE1;59,-12,-0.20) | **Q9ULR0** (ISY1;33,-12,-0.36) |
| **O43187** (IRAK2;69,-13,-0.18) | **P17927** (CR1;224,-12,-0.05) | **Q9H0X4** (ITFG3;60,-12,-0.20) |
| **P00734** (F2;70,-13,-0.18) | **O75718** (CRTAP;47,-12,-0.25) | **Q9Y287** (ITM2B;30,-12,-0.39) |
| **A7E2F4** (GOLGA8A;70,-13,-0.18) | **Q14894** (CRYM;34,-12,-0.35) | **Q15046** (KARS;68,-12,-0.17) |
| **A6PW82** (CXorf30;72,-13,-0.18) | **O95825** (CRYZL1;39,-12,-0.31) | **Q8NFY9** (KBTBD8;69,-12,-0.17) |
| **Q8N1W2** (ZNF710;74,-13,-0.17) | **Q6PD62** (CTR9;134,-12,-0.08) | **Q9NS61** (KCNIP2;31,-12,-0.38) |
| **Q3SXY7** (LRIT3;75,-13,-0.17) | **Q6UX04** (CWC27;54,-12,-0.22) | **P63252** (KCNJ2;48,-12,-0.24) |
| **Q9Y216** (MTMR7;76,-13,-0.17) | **P00167** (CYB5A;15,-12,-0.78) | **Q14667** (KIAA0100;254,-12,-0.04) |
| **P00736** (C1R;80,-13,-0.16) | **P98082** (DAB2;82,-12,-0.14) | **A2VDJ0** (KIAA0922;179,-12,-0.06) |
| **Q9UBK8** (MTRR;80,-13,-0.16) | **O00148** (DDX39A;49,-12,-0.24) | **Q5T5P2** (KIAA1217;214,-12,-0.05) |
| **O95239** (KIF4A;140,-12,-0.08) | **Q3KNS1** (PTCHD3;87,-12,-0.13) | **Q9Y5K5** (UCHL5;38,-12,-0.31) |
| **Q8N7A1** (KLHDC1;47,-12,-0.25) | **Q05397** (PTK2;119,-12,-0.10) | **Q9H3U1** (UNC45A;103,-12,-0.11) |
| **Q8N4N3** (KLHL36;70,-12,-0.17) | **Q92729** (PTPRU;162,-12,-0.07) | **Q15853** (USF2;37,-12,-0.32) |
| **Q9Y5K2** (KLK4;27,-12,-0.44) | **Q9NP90** (RAB9B;23,-12,-0.52) | **P54578** (USP14;56,-12,-0.21) |
| **Q9BQD3** (KXD1;20,-12,-0.61) | **Q7Z6M1** (RABEPK;41,-12,-0.29) | **Q8NEZ2** (VPS37A;44,-12,-0.27) |
| **Q8IVL6** (LEPREL2;82,-12,-0.14) | **Q86X10** (RALGAPB;167,-12,-0.07) | **Q5VIR6** (VPS53;80,-12,-0.15) |
| **Q99538** (LGMN;49,-12,-0.24) | **Q9Y4C8** (RBM19;107,-12,-0.11) | **Q6P4I2** (WDR73;42,-12,-0.28) |
| **Q8NG48** (LINS;86,-12,-0.13) | **P53805** (RCAN1;28,-12,-0.42) | **Q96KV7** (WDR90;187,-12,-0.06) |
| **Q8N448** (LNX2;76,-12,-0.15) | **Q6JBY9** (RCSD1;45,-12,-0.26) | **Q15007** (WTAP;44,-12,-0.27) |
| **O94910** (LPHN1;163,-12,-0.07) | **Q6PCD5** (RFWD3;85,-12,-0.14) | **Q8IZ13** (ZBED8;68,-12,-0.17) |
| **Q12912** (LRMP;62,-12,-0.19) | **Q9NZL6** (RGL1;87,-12,-0.13) | **O15209** (ZBTB22;66,-12,-0.18) |
| **P09960** (LTA4H;69,-12,-0.17) | **Q9BYZ6** (RHOBTB2;83,-12,-0.14) | **Q5VYS8** (ZCCHC6;171 ,-12,-0.07) |
| **Q16539** (MAPK14;41,-12,-0.29) | **Q96NA2** (RILP;44,-12,-0.27) | **P17028** (ZNF24;42,-12,-0.28) |
| **P45984** (MAPK9;48,-12,-0.24) | **Q5TAB7** (RIPPLY2;14,-12,-0.86) | **Q05996** (ZP2;82,-12,-0.14) |
| **P43121** (MCAM;72,-12,-0.16) | **Q6F5E8** (RLTPR;155,-12,-0.07) | **Q3MJ62** (ZSCAN23;45,-12,-0.26) |
| **Q13503** (MED21;16,-12,-0.77) | **Q9Y3C5** (RNF11;17,-12,-0.68) | **Q96AP4** (ZUFSP;66,-12,-0.18) |
| **Q9H1U4** (MEGF9;63,-12,-0.19) | **Q6ZRF8** (RNF207;71,-12,-0.16) | **A6NLX4** (TMEM210;16,-12,-0.77) |
| **P50579** (METAP2;53,-12,-0.22) | **P31350** (RRM2;45,-12,-0.26) | **P52434** (POLR2H;17,-12,-0.69) |
| **Q9H3L0** (MMADHC;33,-12,-0.36) | **P0C263** (SBK2;38,-12,-0.31) | **Q16082** (HSPB2;20,-12,-0.59) |
| **P08473** (MME;86,-12,-0.14) | **Q96NL6** (SCLT1;81,-12,-0.14) | **Q9UMX2** (OAZ3;21,-12,-0.56) |
| **P14780** (MMP9;78,-12,-0.15) | **P01011** (SERPINA3;48,-12,-0.25) | **Q9BQE9** (BCL7B;22,-12,-0.54) |
| **Q9Y605** (MRFAP1;15,-12,-0.81) | **P07988** (SFTPB;42,-12,-0.28) | **P43487** (RANBP1;23,-12,-0.51) |
| **P21757** (MSR1;50,-12,-0.24) | **Q7L8J4** (SH3BP5L;43,-12,-0.27) | **Q07699** (SCN1B;25,-12,-0.48) |
| **P00403** (MT-CO2;26,-12,-0.46) | **A4FU49** (SH3D21;71,-12,-0.17) | **Q6P0A1** (FAM180B;25,-12,-0.47) |
| **P19105** (MYL12A;20,-12,-0.60) | **Q2M3G4** (SHROOM1;91,-12,-0.13) | **Q96MW1** (CCDC43;25,-12,-0.47) |
| **P24844** (MYL9;20,-12,-0.60) | **Q8NDZ2** (SIMC1;97,-12,-0.12) | **Q9Y235** (APOBEC2;26,-12,-0.46) |
| **Q9BSU3** (NAA11;26,-12,-0.46) | **Q96FS4** (SIPA1;112,-12,-0.10) | **O43752** (STX6;29,-12,-0.41) |
| **Q96RE7** (NACC1;57,-12,-0.20) | **Q8IVB4** (SLC9A9;73,-12,-0.16) | **Q6ZUJ4** (C3orf62;30,-12,-0.39) |
| **P55160** (NCKAP1L;128,-12,-0.09) | **Q9H3E2** (SNX25;98,-12,-0.12) | **Q5VT99** (LRRC38;32,-12,-0.37) |
| **O75376** (NCOR1;270,-12,-0.04) | **Q96KW9** (SPACA7;21,-12,-0.55) | **Q6ZW13** (C16orf86;34,-12,-0.35) |
| **Q8WX92** (NELFB;66,-12,-0.18) | **Q7Z6B7** (SRGAP1;124,-12,-0.09) | **Q9HAI6** (CXorf21;34,-12,-0.35) |
| **Q96P20** (NLRP3;118,-12,-0.10) | **O75044** (SRGAP2;121,-12,-0.09) | **O15162** (PLSCR1;35,-12,-0.34) |
| **Q8IXF0** (NPAS3;101,-12,-0.11) | **P42229** (STAT5A;91,-12,-0.13) | **Q13287** (NMI;35,-12,-0.34) |
| **Q8TAT6** (NPLOC4;68,-12,-0.17) | **Q16623** (STX1A;33,-12,-0.36) | **Q9BV68** (RNF126;36,-12,-0.33) |
| **Q9Y2I2** (NTNG1;61,-12,-0.19) | **P61266** (STX1B;33,-12,-0.36) | **P67775** (PPP2CA;36,-12,-0.33) |
| **P04629** (NTRK1;87,-12,-0.13) | **Q9NX95** (SYBU;72,-12,-0.16) | **Q15329** (E2F5;38,-12,-0.31) |
| **Q9Y5A7** (NUB1;71,-12,-0.17) | **Q9BYX2** (TBC1D2;105,-12,-0.11) | **A6NIE6** (RRN3P2;38,-12,-0.31) |
| **P53384** (NUBP1;35,-12,-0.34) | **Q9BQ70** (TCF25;77,-12,-0.15) | **A6NJJ6** (C19orf67;40,-12,-0.30) |
| **Q9BRQ3** (NUDT22;33,-12,-0.36) | **P20061** (TCN1;48,-12,-0.24) | **A6NED2** (RCCD1;40,-12,-0.29) |
| **Q9UKK9** (NUDT5;24,-12,-0.49) | **Q587J7** (TDRD12;133,-12,-0.09) | **F5GYI3** (UBAP1L;41,-12,-0.29) |
| **Q5SWX8** (ODR4;51,-12,-0.23) | **O14948** (TFEC;39,-12,-0.30) | **P60709** (ACTB;42,-12,-0.28) |
| **O95428** (PAPLN;138,-12,-0.08) | **Q86XR7** (TICAM2;27,-12,-0.44) | **P0CW26** (PNMA6C;44,-12,-0.27) |
| **Q8NBP7** (PCSK9;74,-12,-0.16) | **Q9BX73** (TM2D2;23,-12,-0.52) | **P0CZ20** (PNMA6D;44,-12,-0.27) |
| **O95263** (PDE8B;99,-12,-0.12) | **Q9Y3A6** (TMED5;26,-12,-0.46) | **P49441** (INPP1;44,-12,-0.27) |
| **Q9HB75** (PIDD1;100,-12,-0.12) | **Q9NRS4** (TMPRSS4;48,-12,-0.24) | **P22891** (PROZ;45,-12,-0.26) |
| **Q9NWS0** (PIH1D1;32,-12,-0.37) | **P28908** (TNFRSF8;64,-12,-0.18) | **Q5VWP2** (FAM46C;45,-12,-0.26) |
| **P27986** (PIK3R1;84,-12,-0.14) | **Q96F44** (TRIM11;53,-12,-0.22) | **Q9UPV7** (KIAA1045;45,-12,-0.26) |
| **Q16513** (PKN2;112,-12,-0.10) | **P14373** (TRIM27;58,-12,-0.20) | **Q499Z3** (SLFNL1;46,-12,-0.26) |
| **Q8TD55** (PLEKHO2;53,-12,-0.22) | **Q9BSJ1** (TRIM51;52,-12,-0.22) | **Q5T4F4** (ZFYVE27;46,-12,-0.26) |
| **O60664** (PLIN3;47,-12,-0.25) | **Q8N7C3** (TRIML2;44,-12,-0.27) | **Q96P63** (SERPINB12;46,-12,-0.25) |
| **P29590** (PML;98,-12,-0.12) | **Q7Z4N2** (TRPM1;182,-12,-0.06) | **Q9NVG8** (TBC1D13;47,-12,-0.25) |
| **Q9NRF9** (POLE3;17,-12,-0.71) | **Q01534** (TSPY1;35,-12,-0.34) | **Q96FV2** (SCRN2;47,-12,-0.25) |
| **Q08209** (PPP3CA;59,-12,-0.20) | **A6NKD2** (TSPY2;35,-12,-0.34) | **B7U540** (KCNJ18;49,-12,-0.24) |
| **Q13976** (PRKG1;76,-12,-0.15) | **Q9GZX9** (TWSG1;25,-12,-0.47) | **Q8NCJ5** (SPRYD3;50,-12,-0.24) |
| **O14744** (PRMT5;73,-12,-0.16) | **Q8TBC4** (UBA3;52,-12,-0.23) | **P04070** (PROC;52,-12,-0.23) |
| **Q9UIG4** (PSORS1C2;15,-12,-0.79) | **Q9H832** (UBE2Z;38,-12,-0.31) | **Q9H9V9** (JMJD4;52,-12,-0.22) |
| **Q13635** (PTCH1;161,-12,-0.07) | **O95155** (UBE4B;146,-12,-0.08) | **Q9ULV5** (HSF4;53,-12,-0.22) |
| **Q8N9M5** (TMEM102;54,-12,-0.22) | **Q9Y4F5** (CEP170B;172,-11,-0.06) | **Q9UKT9** (IKZF3;58,-11,-0.18) |
| **Q05084** (ICA1;55,-12,-0.21) | **O96017** (CHEK2;61,-11,-0.18) | **P01583** (IL1A;31,-11,-0.35) |
| **Q96P66** (GPR101;57,-12,-0.21) | **Q81UN9** (CLEC10A;35,-11,-0.31) | **Q9NZN1** (IL1RAPL1;80,-11,-0.13) |
| **Q8WU20** (FRS2;57,-12,-0.21) | **Q9UDT6** (CLIP2;116,-11,-0.09) | **Q9NPH2** (ISYNA1;61,-11,-0.18) |
| **Q495B1** (ANKDD1A;58,-12,-0.20) | **Q96KP4** (CNDP2;53,-11,-0.20) | **Q96N16** (JAKMIP1;73,-11,-0.15) |
| **Q9Y575** (ASB3;58,-12,-0.20) | **Q8NE01** (CNNM3;76,-11,-0.14) | **P22001** (KCNA3;64,-11,-0.17) |
| **P32456** (GBP2;67,-12,-0.17) | **Q9BV87** (CNPPD1;45,-11,-0.24) | **Q14500** (KCNJ12;49,-11,-0.22) |
| **Q9H0I9** (TKTL2;68,-12,-0.17) | **Q9P232** (CNTN3;113,-11,-0.09) | **Q14145** (KEAP1;70,-11,-0.15) |
| **P09172** (DBH;69,-12,-0.17) | **Q9BT78** (COPS4;46,-11,-0.23) | **P33176** (KIF5B;110,-11,-0.10) |
| **Q8WZ60** (KLHL6;70,-12,-0.17) | **Q6QEF8** (CORO6;53,-11,-0.20) | **Q9P2K6** (KLHL42;57,-11,-0.19) |
| **P33908** (MAN1A1;73,-12,-0.16) | **P22792** (CPN2;61,-11,-0.18) | **Q9P2G9** (KLHL8;69,-11,-0.15) |
| **Q9UHV5** (RAPGEFL1;73,-12,-0.16) | **Q9HCH3** (CPNE5;66,-11,-0.16) | **P05783** (KRT18;48,-11,-0.22) |
| **Q9BVL4** (SELO;73,-12,-0.16) | **Q9H3G5** (CPVL;54,-11,-0.20) | **Q9UPQ0** (LIMCH1;122,-11,-0.09) |
| **O43572** (AKAP10;74,-12,-0.16) | **Q9NS37** (CREBZF;37,-11,-0.29) | **Q96NW7** (LRRC7;173,-11,-0.06) |
| **O43895** (XPNPEP2;76,-12,-0.15) | **Q86T23** (CROCCP2;12,-11,-0.88) | **P48449** (LSS;83,-11,-0.13) |
| **Q9UKJ8** (ADAM21;81,-12,-0.14) | **O43739** (CYTH3;46,-11,-0.23) | **Q8N653** (LZTR1;95,-11,-0.11) |
| **Q8NA54** (IQUB;93,-12,-0.12) | **Q9NPI6** (DCP1A;63,-11,-0.17) | **Q7Z434** (MAVS;57,-11,-0.19) |
| **P56192** (MARS;101,-12,-0.11) | **Q9P1A6** (DLGAP2;118,-11,-0.09) | **Q93074** (MED12;243,-11,-0.04) |
| **Q9HCR9** (PDE11A;105,-12,-0.11) | **Q5QJE6** (DNTTIP2;84,-11,-0.13) | **Q86XA0** (METTL23;21,-11,-0.51) |
| **P22670** (RFX1;105,-12,-0.11) | **Q9BQC3** (DPH2;52,-11,-0.21) | **Q86U44** (METTL3;64,-11,-0.17) |
| **Q6ZNA4** (RNF111;109,-12,-0.11) | **P21918** (DRD5;53,-11,-0.20) | **Q9HBH9** (MKNK2;52,-11,-0.21) |
| **Q9H2T7** (RANBP17;124,-12,-0.09) | **Q6XUX3** (DSTYK;105,-11,-0.10) | **Q9ULZ9** (MMP17;67,-11,-0.16) |
| **P11498** (PC;130,-12,-0.09) | **Q92997** (DVL3;78,-11,-0.14) | **Q96G30** (MRAP2;24,-11,-0.46) |
| **Q6ZUT9** (DENND5B;145,-12,-0.08) | **O00716** (E2F3;49,-11,-0.22) | **Q9H579** (MROH8;55,-11,-0.20) |
| **O95425** (SVIL;248,-12,-0.04) | **P49770** (EIF2B2;39,-11,-0.28) | **Q8NCY6** (MSANTD4;41,-11,-0.26) |
| **Q92599** (SEPT8;56,-11,-0.19) | **Q7L2H7** (EIF3M;43,-11,-0.25) | **Q9C0I1** (MTMR12;86,-11,-0.12) |
| **Q96GR2** (ACSBG1;81,-11,-0.13) | **Q5MY95** (ENTPD8;54,-11,-0.20) | **O14950** (MYL12B;20,-11,-0.55) |
| **096019** (ACTL6A;47,-11,-0.23) | **P21709** (EPHA1;108,-11,-0.10) | **Q8IZQ8** (MYOCD;102,-11,-0.10) |
| **O94805** (ACTL6B;47,-11,-0.23) | **Q9NVM1** (EVA1B;18,-11,-0.59) | **Q86TC9** (MYPN;145,-11,-0.07) |
| **O60266** (ADCY3;129,-11,-0.08) | **Q96EL1** (FAM212A;31,-11,-0.35) | **Q14CX7** (NAA25;112,-11,-0.09) |
| **Q16186** (ADRM1;42,-11,-0.26) | **P06734** (FCER2;36,-11,-0.30) | **Q9H009** (NACA2;23,-11,-0.47) |
| **P31749** (AKT1;56,-11,-0.19) | **P07332** (FES;93,-11,-0.11) | **P54920** (NAPA;33,-11,-0.33) |
| **P02768** (ALB;69,-11,-0.15) | **Q9P278** (FNIP2;122,-11,-0.09) | **Q9H115** (NAPB;34,-11,-0.32) |
| **Q400G9** (AMZ1;55,-11,-0.20) | **Q9H334** (FOXP1;75,-11,-0.14) | **Q13772** (NCOA4;70,-11,-0.15) |
| **Q6UB99** (ANKRD11;298,-11,-0.03) | **Q8IVH2** (FOXP4;73,-11,-0.14) | **Q68D85** (NCR3LG1;51,-11,-0.21) |
| **Q96NW4** (ANKRD27;117,-11,-0.09) | **Q9BZ68** (FRMD8P1;41,-11,-0.26) | **Q92542** (NCSTN;78,-11,-0.14) |
| **Q9NQ90** (ANO2;114,-11,-0.09) | **P02794** (FTH1;21,-11,-0.51) | **Q14511** (NEDD9;93,-11,-0.11) |
| **P10275** (AR;99,-11,-0.11) | **Q7L5D6** (GET4;37,-11,-0.30) | **P12036** (NEFH;112,-11,-0.09) |
| **Q15052** (ARHGEF6;87,-11,-0.12) | **O76003** (GLRX3;37,-11,-0.29) | **Q9BYH8** (NFKBIZ;78,-11,-0.14) |
| **O43150** (ASAP2;112,-11,-0.09) | **Q6AI39** (GLTSCR1L;115,-11,-0.09) | **P16066** (NPR1;119,-11,-0.09) |
| **Q9NVM9** (ASUN;80,-11,-0.13) | **P04899** (GNAI2;40,-11,-0.27) | **P13056** (NR2C1;67,-11,-0.16) |
| **Q96DT6** (ATG4C;52,-11,-0.20) | **O95467** (GNAS;28,-11,-0.39) | **Q9NSY0** (NRBP2;58,-11,-0.19) |
| **Q9H1Y0** (ATG5;32,-11,-0.33) | **P11488** (GNAT1;40,-11,-0.27) | **Q96CM4** (NXNL1;24,-11,-0.45) |
| **P98196** (ATP11A;130,-11,-0.08) | **Q8N954** (GPATCH11;30,-11,-0.36) | **A1E959** (ODAM;31,-11,-0.35) |
| **Q9H7F0** (ATP13A3;138,-11,-0.07) | **Q8IZF2** (GPR116;149,-11,-0.07) | **Q86WS3** (OOSP2;18,-11,-0.61) |
| **O75185** (ATP2C2;103,-11,-0.10) | **Q13224** (GRIN2B;166,-11,-0.06) | **P13674** (P4HA1;61,-11,-0.18) |
| **Q93050** (ATP6V0A1;96,-11,-0.11) | **Q9BYG8** (GSDMC;58,-11,-0.19) | **Q86U86** (PBRM1;193,-11,-0.05) |
| **Q07817** (BCL2L1;26,-11,-0.42) | **P46976** (GYG1;39,-11,-0.27) | **P35558** (PCK1;69,-11,-0.15) |
| **Q8NFU1** (BEST2;57,-11,-0.19) | **P56524** (HDAC4;119,-11,-0.09) | **Q86YL7** (PDPN;17,-11,-0.65) |
| **Q9NUP1** (BLOC1S4;23,-11,-0.47) | **Q8TDG4** (HELQ;124,-11,-0.08) | **Q9H792** (PEAK1;193,-11,-0.05) |
| **Q86Y37** (CACUL1;41,-11,-0.26) | **O14964** (HGS;86,-11,-0.12) | **Q13608** (PEX6;104,-11,-0.10) |
| **Q8N3J6** (CADM2;48,-11,-0.23) | **P14652** (HOXB2;38,-11,-0.29) | **O00628** (PEX7;36,-11,-0.30) |
| **P22676** (CALB2;32,-11,-0.34) | **Q0VDF9** (HSPA14;55,-11,-0.20) | **Q16512** (PKN1;104,-11,-0.10) |
| **P22681** (CBL;100,-11,-0.11) | **P08107** (HSPA1A;70,-11,-0.15) | **Q9Y263** (PLAA;87,-11,-0.12) |
| **Q5TID7** (CCDC181;60,-11,-0.18) | **Q9Y547** (HSPB11;16,-11,-0.67) | **Q6UX71** (PLXDC2;60,-11,-0.18) |
| **P06731** (CEACAM5;77,-11,-0.14) | **Q5VY09** (IER5;34,-11,-0.32) | **Q9UIW2** (PLXNA1;211,-11,-0.05) |
| **Q9HC77** (CENPJ;153,-11,-0.07) | **Q9UKS7** (IKZF2;60,-11,-0.18) | **P54315** (PNLIPRP1;52,-11,-0.21) |
| **P63098** (PPP3R1;19,-11,-0.56) | **Q9C035** (TRIM5;56,-11,-0.19) | **Q9NY84** (VNN3;56,-11,-0.19) |
| **Q96LZ3** (PPP3R2;20,-11,-0.56) | **Q9NZQ8** (TRPM5;131,-11,-0.08) | **Q6ZNG9** (KRBA2;56,-11,-0.19) |
| **Q8NI37** (PPTC7;33,-11,-0.33) | **Q9NQA5** (TRPV5;83,-11,-0.13) | **O15123** (ANGPT2;57,-11,-0.19) |
| **O43586** (PSTPIP1;48,-11,-0.23) | **Q99576** (TSC22D3;15,-11,-0.74) | **Q9H972** (C14orf93;59,-11,-0.18) |
| **Q7RTS3** (PTF1A;35,-11,-0.31) | **O95801** (TTC4;45,-11,-0.24) | **Q96C11** (FGGY;60,-11,-0.18) |
| **Q14671** (PUM1;126,-11,-0.08) | **Q9NRR5** (UBQLN4;64,-11,-0.17) | **Q6P5Q4** (LMOD2;62,-11,-0.17) |
| **Q9HD47** (RANGRF;20,-11,-0.53) | **Q8TAS1** (UHMK1;47,-11,-0.23) | **Q8IYJ1** (CPNE9;62,-11,-0.17) |
| **Q8WZA2** (RAPGEF4;116,-11,-0.09) | **P09327** (VIL1;93,-11,-0.11) | **Q9BRS2** (RIOK1;66,-11,-0.16) |
| **Q04206** (RELA;60,-11,-0.18) | **O95876** (WDPCP;85,-11,-0.12) | **Q9H201** (EPN3;68,-11,-0.16) |
| **P07949** (RET;124,-11,-0.08) | **P0C1S8** (WEE2;63,-11,-0.17) | **P04843** (RPN1;69,-11,-0.16) |
| **Q6ZWI9** (RFPL4B;30,-11,-0.36) | **Q8WTP9** (XAGE3;12,-11,-0.89) | **Q8NE63** (HIPK4;69,-11,-0.15) |
| **Q9H4X1** (RGCC;15,-11,-0.75) | **Q969M3** (YIPF5;28,-11,-0.39) | **P51168** (SCNN1B;73,-11,-0.15) |
| **Q81XI2** (RHOT1;71,-11,-0.15) | **Q8N8F6** (YIPF7;31,-11,-0.35) | **Q86V97** (KBTBD6;76,-11,-0.14) |
| **Q9UFD9** (RIMBP3;181,-11,-0.06) | **O15391** (YY2;41,-11,-0.26) | **Q5T2E6** (C10orf76;79,-11,-0.13) |
| **Q5XPI4** (RNF123;149,-11,-0.07) | **O96006** (ZBED1;78,-11,-0.14) | **O75074** (LRP3;83,-11,-0.13) |
| **Q9H6Y7** (RNF167;38,-11,-0.28) | **O75132** (ZBED4;130,-11,-0.08) | **Q01826** (SATB1;86,-11,-0.12) |
| **Q86UA6** (RPAIN;25,-11,-0.44) | **O75800** (ZMYND10;50,-11,-0.21) | **Q96II8** (LRCH3;86,-11,-0.12) |
| **P05386** (RPLP1;12,-11,-0.95) | **Q969S3** (ZNF622;54,-11,-0.20) | **Q9Y233** (PDE10A;88,-11,-0.12) |
| **P23297** (S100A1;11,-11,-1.04) | **A8K0R7** (ZNF839;87,-11,-0.12) | **P51790** (CLCN3;91,-11,-0.12) |
| **Q9UBE0** (SAE1;38,-11,-0.28) | **P0DL12** (SMIM17;13,-11,-0.82) | **Q5W041** (ARMC3;96,-11,-0.11) |
| **Q96KG9** (SCYL1;90,-11,-0.12) | **O15514** (POLR2D;16,-11,-0.67) | **Q12979** (ABR;98,-11,-0.11) |
| **P34741** (SDC2;22,-11,-0.49) | **Q01658** (DR1;19,-11,-0.56) | **Q96EN8** (MOCOS;98,-11,-0.11) |
| **Q92503** (SEC14L1;81,-11,-0.13) | **P13693** (TPT1;20,-11,-0.56) | **Q9H6U6** (BCAS3;101,-11,-0.10) |
| **Q9BQF6** (SENP7;120,-11,-0.09) | **Q8IXQ3** (C9orf40;21,-11,-0.52) | **Q96J02** (ITCH;103,-11,-0.10) |
| **P35237** (SERPINB6;43,-11,-0.25) | **O14990** (PPP1R2P9;23,-11,-0.48) | **Q9UNX4** (WDR3;106,-11,-0.10) |
| **Q99961** (SH3GL1;41,-11,-0.26) | **A0PJX2** (TLDC2;24,-11,-0.46) | **O95786** (DDX58;107,-11,-0.10) |
| **Q9BRV8** (SIKE1;24,-11,-0.46) | **Q5GAN6** (RNASE10;24,-11,-0.45) | **Q9UFE4** (CCDC39;110,-11,-0.10) |
| **P42285** (SKIV2L2;118,-11,-0.09) | **Q86SE8** (NPM2;24,-11,-0.45) | **Q02218** (OGDH;116,-11,-0.09) |
| **A0AV02** (SLC12A8;78,-11,-0.14) | **P55327** (TPD52;24,-11,-0.45) | **Q17R98** (ZNF827;119,-11,-0.09) |
| **P36021** (SLC16A2;60,-11,-0.18) | **Q9P1T7** (MDFIC;26,-11,-0.42) | **P31327** (CPS1;165,-11,-0.06) |
| **Q9UI40** (SLC24A2;74,-11,-0.14) | **Q9BZJ3** (TPSD1;27,-11,-0.41) | **O14646** (CHD1;197,-11,-0.05) |
| **Q9Y6M5** (SLC30A1;55,-11,-0.19) | **Q9H3S4** (TPK1;27,-11,-0.40) | **Q4AC94** (C2CD3;260,-11,-0.04) |
| **Q9BRI3** (SLC30A2;35,-11,-0.31) | **P20851** (C4BPB;28,-11,-0.38) | **O43236** (SEPT4;55,-10,-0.18) |
| **Q9NQ40** (SLC52A3;51,-11,-0.21) | **O14618** (CCS;29,-11,-0.37) | **P00813** (ADA;41,-10,-0.24) |
| **Q9GZV3** (SLC5A7;63,-11,-0.17) | **P57796** (CABP4;30,-11,-0.36) | **Q8NDY3** (ADPRHL1;40,-10,-0.24) |
| **Q96T83** (SLC9A7;80,-11,-0.13) | **P30279** (CCND2;33,-11,-0.33) | **Q96MA6** (AK8;55,-10,-0.18) |
| **O15079** (SNPH;54,-11,-0.20) | **Q8IWE4** (DCUN1D3;34,-11,-0.32) | **Q9Y243** (AKT3;56,-10,-0.17) |
| **Q81UW3** (SPATA2L;46,-11,-0.23) | **P29017** (CD1C;38,-11,-0.29) | **P05187** (ALPP;58,-10,-0.17) |
| **Q9P0W8** (SPATA7;68,-11,-0.16) | **Q8N140** (EID3;38,-11,-0.28) | **P10696** (ALPPL2;57,-10,-0.17) |
| **Q9Y5Y6** (ST14;95,-11,-0.11) | **Q6N063** (OGFOD2;39,-11,-0.28) | **Q9P2S6** (ANKMY1;6,-10,-0.09) |
| **Q9Y6E0** (STK24;49,-11,-0.22) | **Q9UNM6** (PSMD13;43,-11,-0.25) | **Q07960** (ARHGAP1;50,-10,-0.19) |
| **Q9P2W9** (STX18;39,-11,-0.28) | **P0C5W0** (PNMA6B;44,-11,-0.24) | **Q9UNA1** (ARHGAP26;92,-10,-0.10) |
| **Q13277** (STX3;33,-11,-0.33) | **P0CW24** (PNMA6A;44,-11,-0.24) | **Q52LW3** (ARHGAP29;142,-10,-0.07) |
| **O00204** (SULT2B1;41,-11,-0.26) | **Q03154** (ACY1;46,-11,-0.23) | **P52566** (ARHGDIB;23,-10,-0.43) |
| **Q8N103** (TAGAP;81,-11,-0.13) | **P35900** (KRT20;48,-11,-0.22) | **P07307** (ASGR2;35,-10,-0.28) |
| **O60907** (TBL1X;62,-11,-0.17) | **A8MYZ6** (FOXO6;51,-11,-0.21) | **O95352** (ATG7;78,-10,-0.12) |
| **Q9UL17** (TBX21;58,-11,-0.18) | **Q8WUX9** (CHMP7;51,-11,-0.21) | **Q8WXF7** (ATL1;64,-10,-0.15) |
| **O43493** (TGOLN2;51,-11,-0.21) | **Q5SQS7** (SH2D4B;51,-11,-0.21) | **P54707** (ATP12A;116,-10,-0.08) |
| **Q9NR97** (TLR8;120,-11,-0.09) | **Q9Y2T4** (PPP2R2C;52,-11,-0.21) | **Q4VNC1** (ATP13A4;134,-10,-0.07) |
| **Q96DC7** (TMCC6;54,-11,-0.20) | **O95219** (SNX4;52,-11,-0.21) | **P0C7T5** (ATXN1L;73,-10,-0.13) |
| **Q8IV31** (TMEM139;24,-11,-0.46) | **Q8IYU4** (UBQLNL;53,-11,-0.20) | **Q9Y5Z0** (BACE2;56,-10,-0.17) |
| **Q9H3N1** (TMX1;32,-11,-0.34) | **P02774** (GC;53,-11,-0.20) | **Q5H9J7** (BEX5;13,-10,-0.79) |
| **Q9NS68** (TNFRSF19;46,-11,-0.23) | **P41586** (ADCYAP1R1;53,-11,-0.20) | **Q96CA5** (BIRC7;33,-10,-0.30) |
| **Q16473** (TNXA;34,-11,-0.32) | **Q6NXE6** (ARMC6;54,-11,-0.20) | **O60238** (BNIP3L;24,-10,-0.41) |
| **Q92547** (TOPBP1;171,-11,-0.06) | **Q9BR76** (CORO1B;54,-11,-0.20) | **Q9NPI1** (BRD7;74,-10,-0.13) |
| **Q9H497** (TOR3A;46,-11,-0.23) | **Q8NCB2** (CAMKV;54,-11,-0.20) | **Q9H6R7** (C2orf44;79,-10,-0.12) |
| **Q9UDY6** (TRIM10;55,-11,-0.19) | **P11597** (CETP;55,-11,-0.20) | **P54284** (CACNB3;55,-10,-0.18) |
| **O15234** (CASC3;76,-10,-0.13) | **P30460** (HLA-B;40,-10,-0.24) | **Q9NPP4** (NLRC4;116,-10,-0.08) |
| **P20273** (CD22;95,-10,-0.10) | **P03989** (HLA-B;40,-10,-0.24) | **Q9NWW6** (NMRK1;23,-10,-0.43) |
| **Q9NPF0** (CD320;29,-10,-0.34) | **P30499** (HLA-C;41,-10,-0.24) | **O75052** (NOS1AP;56,-10,-0.17) |
| **P11912** (CD79A;25,-10,-0.39) | **P17693** (HLA-G;38,-10,-0.26) | **P17342** (NPR3;60,-10,-0.16) |
| **Q96L14** (CEP170P1;33,-10,-0.30) | **P30519** (HMOX2;36,-10,-0.27) | **Q01968** (OCRL;104,-10,-0.09) |
| **Q6NT32** (CES5A;64,-10,-0.15) | **O43364** (HOXA2;41,-10,-0.24) | **Q9ULJ1** (ODF2L;74,-10,-0.13) |
| **Q12798** (CETN1;20,-10,-0.51) | **P48723** (HSPA13;52,-10,-0.19) | **Q9H1P3** (OSBPL2;55,-10,-0.18) |
| **Q99675** (CGRRF1;38,-10,-0.26) | **P17066** (HSPA6;71,-10,-0.14) | **Q96BN8** (OTULIN;40,-10,-0.24) |
| **Q99653** (CHP1;22,-10,-0.44) | **P13284** (IFI30;28,-10,-0.35) | **O95453** (PARN;73,-10,-0.13) |
| **P02708** (CHRNA1;55,-10,-0.18) | **P17181** (IFNAR1;64,-10,-0.15) | **Q96RV3** (PCNX;259,-10,-0.03) |
| **O15111** (CHUK;85,-10,-0.11) | **Q9H7X7** (IFT22;21,-10,-0.47) | **P16519** (PCSK2;71,-10,-0.14) |
| **Q99966** (CITED1;20,-10,-0.50) | **P08833** (IGFBP1;28,-10,-0.35) | **Q6P474** (PDXDC2P;52,-10,-0.19) |
| **Q96MX0** (CMTM3;20,-10,-0.50) | **Q9UPX0** (IGSF9B;147,-10,-0.06) | **P56645** (PER3;132,-10,-0.07) |
| **Q8WXI2** (CNKSR2;118,-10,-0.08) | **Q8N6P7** (IL22RA1;63,-10,-0.15) | **O15173** (PGRMC2;24,-10,-0.41) |
| **Q9H8M5** (CNNM2;97,-10,-0.10) | **P78414** (IRX1;50,-10,-0.20) | **Q96FE7** (PIK3IP1;28,-10,-0.35) |
| **P61923** (COPZ1;20,-10,-0.49) | **P20702** (ITGAX;128,-10,-0.07) | **Q9GZP4** (PITHD1;24,-10,-0.41) |
| **Q96SM3** (CPXM1;82,-10,-0.12) | **P19827** (ITIH1;101,-10,-0.09) | **Q5JRX3** (PITRM1;117,-10,-0.08) |
| **Q8IX95** (CTAGE3P;18,-10,-0.55) | **Q9Y2W7** (KCNIP3;29,-10,-0.34) | **P54277** (PMS1;106,-10,-0.09) |
| **O43310** (CTIF;68,-10,-0.14) | **P48050** (KCNJ4;50,-10,-0.20) | **Q9NVU0** (POLR3E;80,-10,-0.12) |
| **P17812** (CTPS1;67,-10,-0.14) | **Q9ULS6** (KCNS2;54,-10,-0.18) | **Q9HBU9** (POPDC2;40,-10,-0.24) |
| **P07711** (CTSL;38,-10,-0.26) | **Q6UXG2** (KIAA1324;111,-10,-0.08) | **Q6S8J7** (POTEA;56,-10,-0.17) |
| **Q7L576** (CYFIP1;145,-10,-0.06) | **Q2M1P5** (KIF7;151,-10,-0.06) | **Q16633** (POU2AF1;27,-10,-0.36) |
| **Q15438** (CYTH1;46,-10,-0.21) | **Q07866** (KLC1;65,-10,-0.15) | **Q96T49** (PPP1R16B;64,-10,-0.15) |
| **P43146** (DCC;158,-10,-0.06) | **Q96M94** (KLHL15;70,-10,-0.14) | **Q15257** (PPP2R4;41,-10,-0.24) |
| **Q96HY7** (DHTKD1;103,-10,-0.09) | **Q9H0H3** (KLHL25;66,-10,-0.15) | **P0C7W0** (PRR29;21,-10,-0.48) |
| **Q9H3Z4** (DNAJC5;22,-10,-0.45) | **Q9P2J3** (KLHL9;69,-10,-0.14) | **P07478** (PRSS2;26,-10,-0.37) |
| **P49184** (DNASE1L1;34,-10,-0.29) | **P01042** (KNG1;72,-10,-0.13) | **P28065** (PSMB9;23,-10,-0.42) |
| **Q96BY6** (DOCK10;250,-10,-0.04) | **Q6GTX8** (LAIR1;31,-10,-0.31) | **Q96EY7** (PTCD3;79,-10,-0.12) |
| **Q8N350** (DOS;76,-10,-0.13) | **Q5VSP4** (LCN1P1;18,-10,-0.55) | **Q9P2B2** (PTGFRN;99,-10,-0.10) |
| **Q7L8W6** (DPH6;30,-10,-0.32) | **P0C866** (LINC00869;31,-10,-0.32) | **Q4JDL3** (PTPN20A;;48,-10,-0.20) |
| **Q9BTV6** (DPH7;51,-10,-0.19) | **P58215** (LOXL3;83,-10,-0.12) | **O14522** (PTPRT;162,-10,-0.06) |
| **Q8IY85** (EFCAB13;110,-10,-0.09) | **Q14693** (LPIN1;99,-10,-0.10) | **Q8NC74** (RBBP8NL;71,-10,-0.13) |
| **Q14156** (EFR3A;93,-10,-0.10) | **Q5VUJ6** (LRCH2;85,-10,-0.11) | **Q8NDN9** (RCBTB1;58,-10,-0.17) |
| **O00303** (EIF3F;38,-10,-0.26) | **Q8IV03** (LURAP1L;25,-10,-0.40) | **Q8WZ73** (RFFL;41,-10,-0.24) |
| **Q14240** (EIF4A2;46,-10,-0.21) | **Q15759** (MAPK11;41,-10,-0.24) | **Q969X0** (RILPL2;24,-10,-0.41) |
| **O15083** (ERC2;111,-10,-0.09) | **O43513** (MED7;27,-10,-0.36) | **Q8WYP3** (RIN2;100,-10,-0.09) |
| **Q8WUF8** (FAM172A;48,-10,-0.20) | **Q96KG7** (MEGF10;122,-10,-0.08) | **Q06587** (RING1;42,-10,-0.23) |
| **Q8N9W8** (FAM71D;47,-10,-0.21) | **Q96AZ1** (METTL21B;25,-10,-0.40) | **Q8WU17** (RNF139;76,-10,-0.13) |
| **Q7L4E1** (FAM73B;66,-10,-0.15) | **Q9BRT3** (MIEN1;12,-10,-0.80) | **Q8WZ75** (ROBO4;107,-10,-0.09) |
| **Q9NVI1** (FANCI;149,-10,-0.06) | **Q9UGB7** (MIOX;33,-10,-0.30) | **Q96C34** (RUNDC1;68,-10,-0.14) |
| **Q9UKT6** (FBXL21;49,-10,-0.20) | **Q14165** (MLEC;32,-10,-0.31) | **Q86WG5** (SBF2;208,-10,-0.04) |
| **Q96LA6** (FCRL1;47,-10,-0.21) | **Q9BVC4** (MLST8;36,-10,-0.27) | **Q8WU76** (SCFD2;75,-10,-0.13) |
| **Q9NSA1** (FGF21;22,-10,-0.44) | **P08254** (MMP3;54,-10,-0.18) | **Q9GZR1** (SENP6;126,-10,-0.07) |
| **Q9UIM3** (FKBPL;38,-10,-0.26) | **Q96BY2** (MOAP1;40,-10,-0.25) | **P50452** (SERPINB8;43,-10,-0.23) |
| **Q8IVF7** (FMNL3;117,-10,-0.08) | **Q86VX9** (MON1A;62,-10,-0.16) | **Q86TU7** (SETD3;67,-10,-0.14) |
| **Q8TBE3** (FNDC9;25,-10,-0.39) | **Q7L1V2** (MON1B;59,-10,-0.16) | **Q8IWL2** (SFTPA1;26,-10,-0.38) |
| **Q9BZ67** (FRMD8;51,-10,-0.19) | **P40238** (MPL;71,-10,-0.14) | **Q99963** (SH3GL3;39,-10,-0.25) |
| **Q969S9** (GFM2;87,-10,-0.11) | **Q9NZW5** (MPP6;61,-10,-0.16) | **Q13796** (SHROOM2;176,-10,-0.05) |
| **Q14390** (GGTLC2;24,-10,-0.42) | **Q8NCE2** (MTMR14;72,-10,-0.13) | **P84550** (SKOR1;100,-10,-0.10) |
| **Q9Y223** (GNE;79,-10,-0.12) | **P12524** (MYCL;40,-10,-0.24) | **Q8IZD6** (SLC22A15;61,-10,-0.16) |
| **Q81XQ4** (GPALPP1;38,-10,-0.26) | **P41227** (NAA10;26,-10,-0.37) | **Q9C0K1** (SLC39A8;50,-10,-0.20) |
| **Q86YR5** (GPSM1;75,-10,-0.13) | **Q147X3** (NAA30;39,-10,-0.25) | **Q9H1V8** (SLC6A17;81,-10,-0.12) |
| **Q9ULK0** (GRID1;112,-10,-0.08) | **Q6IQ20** (NAPEPLD;46,-10,-0.21) | **Q96PX8** (SLITRK1;78,-10,-0.12) |
| **Q14520** (HABP2;63,-10,-0.15) | **Q86XI2** (NCAPG2;131,-10,-0.07) | **Q9NTJ3** (SMC4;147,-10,-0.06) |
| **O14929** (HAT1;50,-10,-0.20) | **Q9NXR1** (NDE1;39,-10,-0.25) | **Q9UH36** (SRRD;39,-10,-0.25) |
| **Q00341** (HDLBP;141,-10,-0.07) | **Q9GZM8** (NDEL1;38,-10,-0.26) | **Q9NP77** (SSU72;23,-10,-0.44) |
| **Q9NWT6** (HIF1AN;40,-10,-0.24) | **Q9NV92** (NDFIP2;36,-10,-0.27) | **O95630** (STAMBP;48,-10,-0.20) |
| **Q9P246** (STIM2;84,-10,-0.11) | **Q9BV99** (LRRC61;28,-10,-0.35) | **Q96LT7** (C9orf72;54,-10,-0.18) |
| **Q9BR01** (SULT4A1;33,-10,-0.30) | **A6NM15** (CBWD7;28,-10,-0.35) | **Q5SR56** (HIATL1;55,-10,-0.18) |
| **Q92844** (TANK;48,-10,-0.20) | **Q92530** (PSMF1;30,-10,-0.33) | **Q13568** (IRF5;56,-10,-0.17) |
| **Q86TI0** (TBC1 01; 133,-1 0,-0.07) | **P21964** (COMT;30,-10,-0.33) | **Q5I0G3** (MDH1B;59,-10,-0.17) |
| **Q92609** (TBC1D5;89,-10,-0.11) | **P27707** (DCK;31,-10,-0.32) | **Q86V42** (FAM124A;60,-10,-0.16) |
| **Q9BY14** (TEX101;27,-10,-0.37) | **Q6ZRC1** (C4orf50;31,-10,-0.32) | **Q8N5T2** (TBC1D19;60,-10,-0.16) |
| **P19484** (TFEB;53,-10,-0.18) | **O95229** (ZWINT;31,-10,-0.31) | **Q13153** (PAK1;61,-10,-0.16) |
| **P07101** (TH;59,-10,-0.17) | **O75663** (TIPRL;31,-10,-0.31) | **Q96FN4** (CPNE2;61,-10,-0.16) |
| **Q7Z3E1** (TIPARP;76,-10,-0.13) | **Q9UPY8** (MAPRE3;32,-10,-0.31) | **Q01201** (RELB;62,-10,-0.16) |
| **Q9BXR5** (TLR10;95,-10,-0.10) | **O95983** (MBD3;33,-10,-0.30) | **Q9H257** (CARD9;62,-10,-0.16) |
| **O60602** (TLR5;98,-10,-0.10) | **Q96MP8** (KCTD7;33,-10,-0.30) | **Q9H808** (TLE6;63,-10,-0.15) |
| **Q9NZR1** (TMOD2;40,-10,-0.25) | **P50135** (HNMT;33,-10,-0.30) | **Q99502** (EYA1;65,-10,-0.15) |
| **Q9UPQ9** (TNRC6B;194,-10,-0.05) | **Q96NL1** (TMEM74;33,-10,-0.29) | **O75083** (WDR1;66,-10,-0.15) |
| **O14773** (TPP1;61,-10,-0.16) | **A8MZ36** (EVPLL;34,-10,-0.29) | **Q6ZV50** (RFX8;66,-10,-0.15) |
| **A5PLN9** (TRAPPC13;47,-10,-0.21) | **Q6ZSA7** (LRRC55;34,-10,-0.29) | **Q6L8Q7** (PDE12;67,-10,-0.14) |
| **O15016** (TRIM66;135,-10,-0.07) | **Q9NQ48** (LZTFL1;35,-10,-0.28) | **P22303** (ACHE;68,-10,-0.14) |
| **P23258** (TUBG1;51,-10,-0.19) | **Q96AB6** (NTAN1;35,-10,-0.28) | **O76083** (PDE9A;68,-10,-0.14) |
| **Q9NZI7** (UBP1;60,-10,-0.16) | **A6NI79** (CCDC69;35,-10,-0.28) | **P51114** (FXR1;70,-10,-0.14) |
| **Q04323** (UBXN1;33,-10,-0.30) | **Q61PT4** (CYB5RL;36,-10,-0.27) | **Q8N3J3** (C17orf53;70,-10,-0.14) |
| **Q9NYU1** (UGGT2;175,-10,-0.05) | **Q8TC99** (FNDC8;36,-10,-0.27) | **P54652** (HSPA2;70,-10,-0.14) |
| **Q92900** (UPF1;124,-10,-0.08) | **Q16651** (PRSS8;36,-10,-0.27) | **P07359** (GP1BA;72,-10,-0.13) |
| **Q9NQZ2** (UTP3;55,-10,-0.18) | **Q5VZI3** (C9orf91;38,-10,-0.26) | **Q6ZN66** (GBP6;72,-10,-0.13) |
| **Q92558** (WASF1;62,-10,-0.16) | **Q6ZSR9** (0;38,-10,-0.26) | **Q9NW82** (WDR70;73,-10,-0.13) |
| **C4AMC7** (WASH3P;50,-10,-0.20) | **A6NGH7** (CCDC160;38,-10,-0.26) | **Q8NCG7** (DAGLB;74,-10,-0.13) |
| **Q9NQA3** (WASH6P;48,-10,-0.20) | **O75695** (RP2;40,-10,-0.25) | **P05160** (F13B;76,-10,-0.13) |
| **O75554** (WBP4;43,-10,-0.23) | **P09471** (GNAO1;40,-10,-0.24) | **O96005** (CLPTM1;76,-10,-0.13) |
| **Q96FK6** (WDR89;43,-10,-0.23) | **Q504Y3** (ZCWPW2;41,-10,-0.24) | **Q5VTL7** (FNDC7;78,-10,-0.12) |
| **Q8TEU8** (WFIKKN2;64,-10,-0.15) | **E9PGG2** (ANHX;42,-10,-0.23) | **Q9NUQ8** (ABCF3;80,-10,-0.12) |
| **Q9NRH1** (YAE1D1;25,10,-0.39) | **Q562R1** (ACTBL2;42,-10,-0.23) | **Q04725** (TLE2;80,-10,-0.12) |
| **P24278** (ZBTB25;49,-10,-0.20) | **Q8N9B4** (ANKRD42;43,-10,-0.23) | **O00459** (PIK3R2;82,-10,-0.12) |
| **Q8NCN2** (ZBTB34;56,-10,-0.18) | **Q8WTU0** (DDI1;44,-10,-0.22) | **P51178** (PLCD1;86,-10,-0.11) |
| **Q86WB0** (ZC3HC1;55,-10,-0.18) | **Q9HB65** (ELL3;45,-10,-0.22) | **P06396** (GSN;86,-10,-0.11) |
| **O15015** (ZNF646;201,-10,-0.04) | **Q96EP9** (SLC10A4;47,-10,-0.21) | **O43290** (SART1;90,-10,-0.11) |
| **Q9NWK9** (ZNHIT6;54,-10,-0.18) | **P48052** (CPA2;47,-10,-0.21) | **Q9UKN8** (GTF3C4;92,-10,-0.10) |
| **Q96GX2** (ATXN7L3B;11,-10,-0.92) | **P47972** (NPTX2;47,-10,-0.21) | **Q8TB24** (RIN3;108,-10,-0.09) |
| **Q504U0** (C4orf46;12,-10,-0.84) | **Q6NT76** (HMBOX1;47,-10,-0.21) | **Q8IWY4** (SCUBE1;108,-10,-0.09) |
| **Q17R26** (ZNF300P1;16,-10,-0.63) | **Q6NW40** (RGMB;48,-10,-0.21) | **P57679** (EVC;112,-10,-0.08) |
| **Q9H5X1** (FAM96A;18,-10,-0.54) | **Q9UKS6** (PACSIN3;48,-10,-0.20) | **Q05469** (LIPE;117,-10,-0.08) |
| **P60604** (UBE2G2;19,-10,-0.53) | **Q96LV5** (INTS4L1;49,-10,-0.20) | **O94779** (CNTN5;121,-10,-0.08) |
| **Q8N9N8** (EIF1AD;19,-10,-0.52) | **P06280** (GLA;49,-10,-0.20) | **Q9NRM1** (ENAM;129,-10,-0.07) |
| **Q8IZT9** (FAM9C;19,-10,-0.52) | **Q2T9F4** (INTS4L2;49,-10,-0.20) | **Q14896** (MYBPC3;141,-10,-0.07) |
| **Q96LC9** (BMF;21,-10,-0.48) | **Q86YG4** (NT5DC4;49,-10,-0.20) | **P05997** (COL5A2;145,-10,-0.06) |
| **Q68D20** (PMS2CL;21,-10,-0.47) | **C9JR72** (KBTBD13;49,-10,-0.20) | **Q8TER0** (SNED1;152,-10,-0.06) |
| **Q9BUN5** (CCDC28B;22,-10,-0.45) | **P04180** (LCAT;50,-10,-0.20) | **Q9UHC1** (MLH3;164,-10,-0.06) |
| **Q8N0U2** (TMEM61;22,-10,-0.45) | **Q8WW24** (TEKT4;51,-10,-0.19) | |
| **A8MWL6** (0;25,-10,-0.40) | **P04004** (VTN;54,-10,-0.18) | |

**Table 4. Exemplary naturally occurring negatively supercharged proteins that are involved in diseases, disorders, or conditions. Proteins listed have a negative net charge of -10 or less.**

| | | |
|---|---|---|
| **Q8WXG9** (GPR98;693,-412,-0.59) | **O95714** (095714;527,-83,-0.15) | **Q68CZ1** (RPGRIP1L;151,-58,-0.38) |
| **P13611** (VCAN;373,-322,-0.86) | **P98160** (P98160;469,-83,-0.17) | **Q7Z7G8** (VPS13B;449,-58,-0.12) |
| **Q03001** (DST;861,-316,-0.36) | **Q9BQS8** (FYCO1;167,-82,-0.49) | **075581** (O75581;180,-58,-0.32) |
| **Q8NF91** (SYNE1;1,011,-315,-0.31) | **P50851** (LRBA;319,-82,-0.25) | **Q9Y5Q5** (CORIN;116,-57,-0.48) |
| **Q8IWN7** (Q8IWN7;261,-278,-1.06) | **Q5TD94** (RSPH4A;81,-81,-1.00) | **Q14204** (DYNC1H1;532,-57,-0.10) |
| **Q8WXH0** (SYNE2;796,-271,-0.34) | **Q14028** (CNGB1;140,-80,-0.57) | **Q9BZA8** (PCDH11Y;147,-57,-0.38) |
| **P16112** (ACAN;250,-269,-1.07) | **Q9UM47** (Q9UM47;244,-80,-0.32) | **P09619** (PDGFRB;124,-57,-0.45) |
| **Q9NZW4** (Q9NZW4;131,-266,-2.02) | **P35442** (THBS2;130,-79,-0.60) | **O60216** (RAD21;72,-57,-0.79) |
| **P98164** (P98164;522,-243,-0.46) | **Q5JTC6** (AMER1;124,-78,-0.62) | **Q8IVV2** (Q8IVV2;222,-57,-0.25) |
| **Q99996** (AKAP9;454,-233,-0.51) | **O60229** (KALRN;340,-78,-0.22) | **P20908** (P20908;184,-57,-0.31) |
| **Q6V0I7** (FAT4;543,-230,-0.42) | **Q14767** (LTBP2;195,-77,-0.39) | **P12259** (P12259;252,-57,-0.22) |
| **Q9H251** (CDH23;369,-223,-0.60) | **Q5T011** (SZT2;378,-77,-0.20) | **Q15029** (EFTUD2;109,-56,-0.51) |
| **Q01484** (Q01484;434,-212,-0.48) | **Q7Z7A1** (CNTRL;269,-75,-0.27) | **P02751** (FN1;263,-56,-0.21) |
| **P21817** (RYR1;565,-207,-0.36) | **Q6ZRI0** (OTOG;315,-74,-0.23) | **Q99698** (LYST;429,-56,-0.13) |
| **Q7Z6Z7** (HUWE1;482,-173, -0.35) | **Q86SJ6** (DSG4;114,-73,-0.64) | **P98155** (P98155;96,-56,-0.58) |
| **Q5VST9** (OBSCN;868,-173,-0.19) | **Q93008** (USP9X;292,-72,-0.24) | **O75443** (O75443;240,-56,-0.23) |
| **Q86XX4** (FRAS1;443,-168,-0.37) | **O00507** (USP9Y;291,-72,-0.24) | **P56715** (RP1;241,-55,-0.22) |
| **Q96JQ0** (DCHS1;346,-162,-0.46) | **Q96QU1** (PCDH15;216,-71,-0.32) | **P12830** (CDH1;97,-54,-0.55) |
| **Q5SZK8** (Q5SZK8;351,-158,-0.44) | **Q96JI7** (SPG11;279,-71,-0.25) | **P21333** (FLNA;281,-54,-0.19) |
| **P02549** (SPTA1;280,-153,-0.54) | **P38398** (BRCA1;208,-70,-0.33) | **P50542** (PEX5;71,-54,-0.76) |
| **O14686** (KMT2D;593,-148,-0.24) | **Q14160** (SCRIB;175,-70,-0.40) | **P08F94** (PKHD1;447,-54,-0.12) |
| **Q12888** (Q12888;214,-148,-0.69) | **Q5H8C1** (FREM1;244,-69,-0.28) | **Q92794** (KAT6A;225,-53,-0.23) |
| **P22105** (TNXB;464,-144,-0.31) | **Q92834** (RPGR;113,-69,-0.60) | **O60333** (KIF1B;204,-53,-0.25) |
| **O95613** (PCNT;378,-142,-0.37) | **Q92673** (SORL1;248,-69,-0.27) | **O15020** (SPTBN2;271,-53,-0.19) |
| **Q8N4C6** (NIN;243,-138,-0.56) | **P37275** (ZEB1;124,-69,-0.55) | **Q6ZRS2** (SRCAP;344,-53,-0.15) |
| **P35556** (P35556;315,-137,-0.43) | **Q9NYQ6** (CELSR1;329,-67,-0.20) | **Q01105** (Q01105;33,-53,-1.58) |
| **O60494** (CUBN;399,-133,-0.33) | **Q8TE73** (DNAH5;529,-67,-0.12) | **P01266** (P01266;305,-53,-0.17) |
| **P35555** (FBN1;312,-132,-0.42) | **P42566** (EPS15;99,-67,-0.67) | **Q9NZN5** (ARHGEF12;173,-52,-0.30) |
| **P12270** (P12270;267,-127,-0.47) | **Q16821** (PPP1R3A; 126,-67,-0.53) | **Q4G0X9** (CCDC40;130,-52,-0.39) |
| **P46531** (NOTCH1;273,-125,-0.45) | **Q562E7** (Q562E7;212,-67,-0.31) | **Q6PRD1** (GPR179;257,-52,-0.20) |
| **Q92736** (Q92736;565,-119,-0.21) | **Q9UPV0** (CEP164;164,-66,-0.40) | **Q14114** (LRP8;106,-52,-0.49) |
| **Q04721** (Q04721;265,-117,-0.44) | **P14314** (PRKCSH;59,-66,-1.11) | **Q9HC84** (MUC5B;596,-52,-0.08) |
| **P24821** (TNC;241,-114,-0.47) | **Q96RW7** (HMCN1;613,-65,-0.10) | **O60721** (SLC24A1;121,-52,-0.42) |
| **Q4G0P3** (HYDIN;576,-113,-0.19) | **P49747** (P49747;83,-65,-0.78) | **P35749** (MYH11;227,-51,-0.22) |
| **Q13813** (SPTAN1;285,-113,-0.39) | **P82279** (P82279;154,-65,-0.42) | **Q14999** (CUL7;191,-50,-0.26) |
| **P11277** (SPTB;246,-107,-0.43) | **P05067** (APP;87,-64,-0.73) | **Q9UPY3** (DICER1;219,-50,-0.22) |
| **Q15154** (Q15154;229,-103,-0.45) | **O75369** (FLNB;278,-64,-0.23) | **O75923** (DYSF;237,-50,-0.21) |
| **O94915** (FRYL;340,-101,-0.29) | **P42858** (HTT;348,-64,-0.18) | **P28715** (ERCC5;133,-49,-0.36) |
| **Q13316** (Q13316;56,-101,-1.81) | **O75147** (OBSL1;207,-64,-0.30) | **P07196** (NEFL;62,-49,-0.79) |
| **Q9BZV3** (IMPG2;139,-100,-0.72) | **Q86UP3** (Q86UP3;394,-64,-0.16) | **P49792** (RANBP2;358,-49,-0.13) |
| **Q3ZCN5** (OTOGL;262,-98,-0.37) | **Q12955** (Q12955;480,-64,-0.13) | **O43379** (WDR62;166,-49,-0.29) |
| **P07942** (P07942;198,-98,-0.49) | **Q96DT5** (Q96DT5;521,-63,-0.12) | **Q68DK2** (ZFYVE26;285,-49,-0.17) |
| **Q8TCU4** (ALMS1;461,-96,-0.20) | **Q8NEP3** (DNAAF1;80,-62,-0.77) | **Q8IWV7** (Q8IWV7;200,-49,-0.24) |
| **O75970** (MPDZ;222,-94,-0.42) | **Q14643** (ITPR1;314,-62,-0.19) | **P12107** (COL11A1;181,-48,-0.26) |
| **P78509** (P78509;388,-94,-0.24) | **A2RRP1** (NBAS;269,-62,-0.23) | **Q14678** (KANK1;147,-48,-0.32) |
| **P21675** (TAF1;213,-93,-0.43) | **Q96SN8** (CDK5RAP2;215,-61,-0.28) | **Q8WYB5** (KAT6B;231,-48,-0.20) |
| **Q5VU43** (PDE4DIP;265,-92,-0.34) | **P35499** (P35499;208,-61,-0.29) | **Q6ZNJ1** (NBEAL2;303,-48,-0.15) |
| **Q15643** (TRIP11;228,-90,-0.39) | **P16157** (ANK1;206,-60,-0.29) | **Q9BYW2** (SETD2;288,-48,-0.16) |
| **Q9UGM3** (DMBT1;261,-89,-0.34) | **Q9NWF9** (RNF216;99,-60,-0.60) | **O75643** (SNRNP200;245,-48,-0.19) |
| **P04275** (VWF;309,-89,-0.28) | **P22223** (CDH3;91,-59,-0.64) | **P14410** (P14410;209,-48,-0.22) |
| **P11532** (P11532;427,-89,-0.20) | **Q9UBN7** (HDAC6;131,-59,-0.44) | **P55287** (P55287;88,-48,-0.54) |
| **Q5T1H1** (EYS;351,-87,-0.24) | **P10745** (P10745;135,-59,-0.43) | **O15078** (CEP290;290,-47,-0.16) |
| **Q63HN8** (RNF213;591,-87,-0.14) | **O14958** (O14958;46,-59,-1.27) | **Q9P2D1** (CHD7;336,-47,-0.13) |
| **Q8NG31** (CASC5;265,-84,-0.31) | **O94986** (CEP152;196,-58,-0.29) | **Q9HCE0** (EPG5;292,-47,-0.16) |
| **O75096** (LRP4;212,-83,-0.39) | **Q14315** (FLNC;291,-58,-0.19) | **Q9HC10** (OTOF;227,-47,-0.20) |
| **Q15149** (PLEC;532,-83,-0.15) | **O75197** (LRP5;179,-58,-0.32) | **P16234** (PDGFRA;123,-47,-0.38) |
| **O94972** (TRIM37;108,-47,-0.43) | **Q9UG01** (IFT172;198,-38,-0.19) | **P23142** (FBLN1;77,-31,-0.40) |
| **P13535** (P13535;223,-47,-0.21) | **O15040** (TECPR2;154,-38,-0.24) | **Q9BYX4** (IFIH1;117,-31,-0.26) |
| **Q7RTU9** (Q7RTU9;193,-47,-0.24) | **P12814** (P12814;103,-38,-0.36) | **Q9HBE5** (IL21R;59,-31,-0.52) |
| **P35579** (P35579;227,-47,-0.20) | **P16144** (P16144;202,-38,-0.18) | **P52732** (KIF11;119,-31,-0.26) |
| **Q8N2S1** (Q8N2S1;173,-47,-0.27) | **O95163** (IKBKAP;150,-37,-0.24) | **P08575** (PTPRC;147,-31,-0.21) |
| **Q8NCM8** (DYNC2H1;493,-46,-0.09) | **P09848** (LCT;219,-37,-0.16) | **Q9H4L7** (SMARCAD1;117,-31,-0.26) |
| **Q71F56** (MED13L;243,-46,-0.18) | **Q08499** (PDE4D;91,-37,-0.40) | **O60315** (ZEB2;136,-31,-0.22) |
| **P11055** (P11055;224,-46,-0.20) | **Q07864** (POLE;262,-37,-0.14) | **P09871** (P09871;77,-31,-0.40) |
| **P13533** (MYH6;224,-45,-0.20) | **Q8NEL9** (DDHD1;100,-36,-0.35) | **P39060** (P39060;178,-31,-0.17) |
| **Q96RL7** (VPS13A;360,-45,-0.12) | **Q8NDI1** (EHBP1;140,-36,-0.25) | **Q9NS15** (Q9NS15;139,-31,-0.22) |
| **Q9BUR4** (WRAP53;59,-45,-0.75) | **Q9NZJ5** (EIF2AK3;125,-36,-0.28) | **Q8N302** (AGGF1;81,-30,-0.37) |
| **Q9GZS0** (Q9GZS0;69,-45,-0.65) | **P55268** (LAMB2;196,-36,-0.18) | **Q8NEU8** (APPL2;74,-30,-0.40) |
| **Q96KN7** (Q96KN7;147,-45,-0.30) | **P35913** (PDE6B;98,-36,-0.36) | **Q9Y238** (DLEC1;196,-30,-0.15) |
| **Q8TD26** (CHD6;305,-44,-0.14) | **P40855** (PEX19;33,-36,-1.09) | **Q13683** (ITGA7;129,-30,-0.23) |
| **Q96F46** (IL17RA;96,-44,-0.45) | **Q15042** (RAB3GAP1;111,-36,-0.32) | **Q86Y26** (NUTM1;120,-30,-0.24) |
| **O15013** (ARHGEF10;152,-43,-0.28) | **Q5T481** (Q5T481;134,-36,-0.26) | **Q9H4A3** (WNK1;251,-30,-0.11) |
| **Q04637** (EIF4G1;175,-43,-0.24) | **P01133** (P01133;134,-36,-0.26) | **Q16363** (Q16363;203,-30,-0.14) |
| **Q8IYD8** (FANCM;232,-43,-0.18) | **Q9H6L5** (Q9H6L5;55,-36,-0.65) | **Q12934** (Q12934;75,-30,-0.40) |
| **P41229** (KDM5C;176,-43,-0.24) | **P78363** (ABCA4;256,-35,-0.13) | **Q9BY79** (Q9BY79;62,-30,-0.48) |
| **Q9UQF2** (MAPK8IP1;78,-43,-0.55) | **O60566** (BUB1B;120,-35,-0.29) | **Q9Y6D5** (ARFGEF2;202,-29,-0.14) |
| **Q7Z406** (MYH14;228,-43,-0.18) | **O60840** (CACNA1F;221,-35,-0.15) | **Q8TD16** (BICD2;94,-29,-0.31) |
| **Q9UKX2** (MYH2;223,-43,-0.19) | **Q0VF96** (CGNL1;149,-35,-0.23) | **Q6PJG6** (BRAT1;88,-29,-0.32) |
| **Q9H5I5** (PIEZO2;318,-43,-0.13) | **Q13144** (EIF2B5;80,-35,-0.43) | **P11171** (EPB41;97,-29,-0.29) |
| **Q6YHU6** (THADA;220,-43,-0.19) | **Q92539** (LPIN2;99,-35,-0.35) | **Q96EK5** (KIAA1279;72,-29,-0.40) |
| **P98073** (TMPRSS15;113,-43,-0.38) | **Q96PY6** (NEK1;143,-35,-0.24) | **Q12756** (KIF1A;191,-29,-0.15) |
| **Q9P219** (CCDC88C;228,-42,-0.18) | **Q8WYR4** (RSPH1;35,-35,-0.99) | **P25963** (NFKBIA;36,-29,-0.81) |
| **Q02413** (DSG1;114,-42,-0.36) | **Q9P273** (TENM3;301,-35,-0.11) | **Q8N427** (NME8;67,-29,-0.43) |
| **Q14126** (DSG2;122,-42,-0.34) | **Q99707** (Q99707;141,-35,-0.24) | **Q96CV9** (OPTN;66,-29,-0.43) |
| **P12883** (MYH7;223,-42,-0.18) | **Q2M1Z3** (ARHGAP31;157,-34,-0.21) | **Q69YQ0** (SPECC1L;125,-29,-0.23) |
| **Q9H2M9** (RAB3GAP2;156,-42,-0.26) | **Q8WZ55** (BSND;35,-34,-0.96) | **P07204** (THBD;60,-29,-0.48) |
| **P00450** (P00450;122,-42,-0.34) | **O15320** (CTAGE5;91,-34,-0.37) | **P63316** (TNNC1;18,-29,-1.57) |
| **Q14524** (Q14524;227,-42,-0.18) | **Q9P2K8** (EIF2AK4;187,-34,-0.18) | **Q9Y5Y9** (Q9Y5Y9;221,-29,-0.13) |
| **Q9HCK8** (CHD8;291,-41,-0.14) | **Q9HAZ2** (PRDM16;140,-34,-0.24) | **P19235** (P19235;55,-29,-0.52) |
| **P24386** (CHM;73,-41,-0.55) | **Q9HCM3** (Q9HCM3;211,-34,-0.16) | **Q14203** (Q14203;142,-29,-0.20) |
| **Q9Y5B0** (CTDP1; 104,-41 ,-0.39) | **P33076** (CIITA;124,-33,-0.26) | **O15118** (O15118;142,-29,-0.20) |
| **Q5JWF2** (GNAS;111,-41,-0.36) | **Q2KHT3** (CLEC16A;118,-33,-0.28) | **P30530** (AXL;98,-28,-0.28) |
| **P24043** (LAMA2;344,-41,-0.11) | **Q9NVR5** (DNAAF2;91,-33,-0.36) | **Q02487** (DSC2;100,-28,-0.28) |
| **Q96RT7** (TUBGCP6;200,-41,-0.20) | **Q96DU7** (ITPKC;75,-33,-0.43) | **Q9H4G0** (EPB41L1;99,-28,-0.28) |
| **P01130** (P01130;95,-41,-0.42) | **Q32P28** (LEPRE1;83,-33,-0.39) | **Q9NQG7** (HPS4;77,-28,-0.36) |
| **O43707** (043707;105,-41,-0.39) | **O94898** (LRIG2;119,-33,-0.27) | **O15259** (NPHP1;83,-28,-0.33) |
| **Q9C0F0** (ASXL3;242,-40,-0.16) | **O60500** (NPHS1;135,-33,-0.24) | **P16499** (PDE6A;100,-28,-0.28) |
| **P55291** (CDH15;89,-40,-0.44) | **P35609** (P35609;104,-33,-0.31) | **P47712** (PLA2G4A;85,-28,-0.32) |
| **Q9H9B1** (EHMT1;141,-40,-0.28) | **Q8NDB2** (Q8NDB2;89,-33,-0.36) | **Q9P212** (PLCE1;259,-28,-0.10) |
| **Q96RY7** (IFT140;165,-40,-0.24) | **Q86WG3** (ATCAY;42,-32,-0.75) | **P35498** (SCN1A;229,-28,-0.12) |
| **Q8TAB3** (PCDH19;126,-40,-0.31) | **P51587** (BRCA2;384,-32,-0.08) | **P07951** (TPM2;33,-28,-0.85) |
| **O00291** (000291;116,-40,-0.34) | **Q09013** (DMPK;69,-32,-0.46) | **Q96QK1** (VPS35;92,-28,-0.30) |
| **Q00987** (Q00987;55,-40,-0.72) | **Q96EV8** (DTNBP1;39,-32,-0.81) | **Q96J92** (WNK4;135,-28,-0.20) |
| **Q13085** (ACACA;266,-39,-0.14) | **P04626** (ERBB2;138,-32,-0.23) | **O14983** (014983;110,-28,-0.25) |
| **P08922** (ROS1;264,-39,-0.14) | **Q9BXW9** (FANCD2;166,-32,-0.19) | **Q9UPM8** (Q9UPM8;127,-28,-0.21) |
| **Q8IY92** (SLX4;200,-39,-0.19) | **Q6Y7W6** (GIGYF2;150,-32,-0.21) | **Q6PD74** (Q6PD74;35,-28,-0.80) |
| **Q9UMZ3** (Q9UMZ3;261,-39,-0.14) | **Q27J81** (INF2;136,-32,-0.23) | **P15260** (P15260;54,-28,-0.51) |
| **P10912** (P10912;72,-39,-0.54) | **P08514** (ITGA2B;113,-32,-0.28) | **P06753** (P06753;33,-28,-0.84) |
| **P98161** (P98161;463,-39,-0.08) | **P02730** (SLC4A1;102,-32,-0.31) | **Q86W8** (Q86W8;249,-28,-0.11) |
| **Q7Z3S7** (Q7Z3S7;128,-39,-0.30) | **Q05086** (UBE3A;101,-32,-0.31) | **Q96Q42** (ALS2;184,-27,-0.14) |
| **O75445** (O75445;576,-39,-0.06) | **Q5W43** (Q5VV43;118,-32,-0.27) | **Q66GS9** (CEP135;133,-27,-0.20) |
| **Q8TDI0** (CHD5;223,-38,-0.17) | **Q9H4Z2** (Q9H4Z2;145,-32,-0.22) | **Q16832** (DDR2;97,-27,-0.27) |
| **Q9UPZ3** (HPS5;127,-38,-0.29) | **O60610** (DIAPH1;141,-31,-0.21) | **Q8IUD2** (ERC1; 128,-27,-0.21) |
| **P35968** (KDR;152,-27,-0.17) | **Q8NFD5** (ARID1B;236,-24,-0.10) | **Q9NQC7** (CYLD;107,-22,-0.20) |
| **Q5U5Q3** (MEX3C;69,-27,-0.38) | **Q8IXJ9** (ASXL1;165,-24,-0.14) | **P21860** (ERBB3;148,-22,-0.14) |
| **Q99650** (OSMR;111,-27,-0.24) | **Q04656** (ATP7A;163,-24,-0.14) | **Q9UBX5** (FBLN5;50,-22,-0.43) |
| **Q99250** (SCN2A;228,-27,-0.11) | **P62158** (CALM1;17,-24,-1.42) | **P11021** (HSPA5;72,-22,-0.30) |
| **Q6P5W5** (SLC39A4;68,-27,-0.39) | **Q9BXL7** (CARD11;133,-24,-0.18) | **Q13651** (IL10RA;63,-22,-0.34) |
| **Q969G3** (SMARCE1;47,-27,-0.57) | **Q9BXL6** (CARD 14;1 13,-24,-0.21) | **P80192** (MAP3K9;122,-22,-0.18) |
| **P09493** (TPM1;33,-27,-0.82) | **P32927** (CSF2RB;97,-24,-0.24) | **P46019** (PHKA2;138,-22,-0.15) |
| **Q7RTW8** (Q7RTW8;129,-27,-0.21) | **P36888** (FLT3;113,-24,-0.21) | **Q96NY8** (PVRL4;55,-22,-0.39) |
| **P06213** (P06213;156,-27,-0.17) | **Q9NQX3** (GPHN;80,-24,-0.30) | **Q9NZJ4** (SACS;521,-22,-0.04) |
| **P53708** (P53708;117,-27,-0.22) | **Q8N6C5** (IGSF1;149,-24,-0.16) | **Q8N196** (SIX5;75,-22,-0.29) |
| **Q96MR6** (Q96MR6;145,-27,-0.18) | **P32004** (L1CAM;140,-24,-0.17) | **Q13043** (STK4;56,-22,-0.39) |
| **P12821** (ACE;150,-26,-0.17) | **Q8IVL5** (LEPREL1;81,-24,-0.29) | **Q14191** (WRN;162,-22,-0.13) |
| **O00499** (BIN1;65,-26,-0.40) | **P06748** (NPM1;33,-24,-0.73) | **Q9H799** (Q9H799;362,-22,-0.06) |
| **P83436** (COG7;86,-26,-0.30) | **P51160** (PDE6C;99,-24,-0.24) | **O00187** (O00187;76,-22,-0.29) |
| **Q8IYU2** (HACE1;102,-26,-0.25) | **Q9Y2I7** (PIKFYVE;237,-24,-0.10) | **Q02388** (Q02388;295,-22,-0.07) |
| **P78504** (JAG1;134,-26,-0.19) | **O60331** (PIP5K1C;73,-24,-0.32) | **O43933** (043933;143,-22,-0.15) |
| **Q12840** (KIF5A;117,-26,-0.22) | **Q96LW4** (PRIMPOL;64,-24,-0.37) | **Q13563** (Q13563;110,-22,-0.20) |
| **Q5S007** (LRRK2;286,-26,-0.09) | **Q15020** (SART3;110,-24,-0.21) | **Q96MT8** (Q96MT8;81,-22,-0.27) |
| **Q9UDY8** (MALT1;92,-26,-0.28) | **Q8TF17** (SH3TC2;145,-24,-0.16) | **P35219** (CA8;33,-21,-0.63) |
| **O00462** (MANBA;101,-26,-0.25) | **Q9ULL8** (SHROOM4;165,-24,-0.14) | **P35523** (CLCN1;109,-21,-0.19) |
| **P48740** (MASP1;79,-26,-0.32) | **Q15468** (STIL;143,-24,-0.16) | **Q8IYB7** (DIS3L2;99,-21,-0.21) |
| **Q7Z7M0** (MEGF8;303,-26,-0.08) | **Q8IUC6** (TICAM1;76,-24,-0.31) | **Q96AY3** (FKBP10;64,-21,-0.32) |
| **Q12866** (MERTK;110,-26,-0.23) | **O43897** (TLL1;115,-24,-0.20) | **O00165** (HAX1;32,-21,-0.66) |
| **P52948** (NUP98;198,-26,-0.13) | **Q71U36** (TUBA1A;50,-24,-0.47) | **P42704** (LRPPRC;158,-21 ,-0.13) |
| **Q15477** (SKIV2L;138,-26,-0.18) | **Q9NY65** (TUBA8;50,-24,-0.47) | **P40692** (MLH1;85,-21,-0.24) |
| **Q92574** (TSC1;130,-26,-0.20) | **Q99456** (Q99456;54,-24,-0.44) | **O15055** (PER2;137,-21,-0.15) |
| **Q9H9E3** (Q9H9E3;89,-26,-0.29) | **P50747** (P50747;81,-24,-0.29) | **P07602** (PSAP;58,-21,-0.36) |
| **P13637** (P13637;112,-26,-0.23) | **P15391** (P15391;61,-24,-0.39) | **Q13501** (SQSTM1;48,-21,-0.44) |
| **P08069** (P08069;155,-26,-0.16) | **Q9UIR0** (Q9UIR0;50,-24,-0.47) | **Q96GX1** (TCTN2;77,-21,-0.27) |
| **Q6IEE7** (Q6IEE7;107,-26,-0.24) | **Q16720** (Q16720;134,-24,-0.17) | **Q0ZGT2** (Q0ZGT2;81,-21,-0.26) |
| **P16615** (ATP2A2;115,-25,-0.21) | **P42702** (P42702;124,-24,-0.19) | **Q9H4B7** (Q9H4B7;50,-21,-0.41) |
| **Q3SYG4** (BBS9;99,-25,-0.25) | **Q6W2J9** (BCOR;192,-23,-0.11) | **O75381** (075381;41,-21,-0.50) |
| **P35222** (CTNNB1;85,-25,-0.29) | **Q4KMG0** (CDON;139,-23,-0.16) | **Q9Y2Y0** (ARL2BP;19,-20,-1.06) |
| **O94830** (DDHD2;81,-25,-0.30) | **O15360** (FANCA;163,-23,-0.14) | **Q96Q07** (BTBD9;69,-20,-0.28) |
| **Q96HP0** (DOCK6;230,-25,-0.10) | **Q6PIJ6** (FBXO38;134,-23,-0.17) | **O14936** (CASK;105,-20,-0.19) |
| **Q96MC2** (DRC1;87,-25,-0.28) | **P05106** (ITGB3;87,-23,-0.26) | **Q14790** (CASP8;55,-20,-0.36) |
| **O95967** (EFEMP2;49,-25,-0.50) | **Q2TBA0** (KLHL40;69,-23,-0.33) | **P41180** (CASR;121,-20,-0.16) |
| **O95684** (FGFR1OP;43,-25,-0.58) | **P20700** (LMNB1;66,-23,-0.34) | **Q70SY1** (CREB3L2;57,-20,-0.34) |
| **P35916** (FLT4;153,-25,-0.16) | **P55196** (MLLT4;207,-23,-0.11) | **Q99062** (CSF3R;92,-20,-0.21) |
| **Q9C0B1** (FTO;58,-25,-0.42) | **P16435** (POR;77,-23,-0.29) | **Q6UN15** (FIP1L1;67,-20,-0.30) |
| **Q15746** (MYLK;211,-25,-0.11) | **O14593** (RFXANK;28,-23,-0.81) | **Q92949** (FOXJ1;45,-20,-0.44) |
| **P46020** (PHKA1;137,-25,-0.18) | **Q86SQ7** (SDCCAG8;83,-23,-0.27) | **Q86UP8** (GTF2IRD2;107,-20,-0.18) |
| **P49810** (PSEN2;50,-25,-0.49) | **Q9UGP8** (SEC63;88,-23,-0.26) | **Q00653** (NFKB2;97,-20,-0.20) |
| **Q16827** (PTPRO;138,-25,-0.18) | **Q9UMX1** (SUFU;54,-23,-0.42) | **Q9Y6C5** (PTCH2;131,-20,-0.15) |
| **Q13885** (TUBB2A;50,-25,-0.50) | **Q8NEZ3** (WDR19;152,-23,-0.15) | **Q99574** (SERPIN11;46,-20,-0.43) |
| **Q13509** (TUBB3;50,-25,-0.49) | **Q99592** (ZBTB18;58,-23,-0.39) | **Q8TBB6** (SLC7A14;84,-20,-0.23) |
| **P04350** (TUBB4A;50,-25,-0.50) | **Q2M1K9** (ZNF423;145,-23,-0.15) | **O14841** (014841;137,-20,-0.14) |
| **P22314** (UBA1;118,-25,-0.21) | **Q13315** (Q13315;351,-23,-0.06) | **Q7Z6L0** (Q7Z6L0;35,-20,-0.57) |
| **P12109** (P12109;109,-25,-0.23) | **P45379** (P45379;36,-23,-0.64) | **P35557** (P35557;52,-20,-0.38) |
| **P49588** (P49588;107,-25,-0.23) | **P07911** (P07911;70,-23,-0.32) | **Q10571** (Q10571;136,-20,-0.14) |
| **Q9Y6N6** (Q9Y6N6;171,-25,-0.14) | **P42898** (P42898;75,-23,-0.30) | **Q92622** (Q92622;109,-20,-0.18) |
| **P10253** (P10253;105,-25,-0.23) | **Q99523** (Q99523;92,-23,-0.24) | **P45452** (P45452;54,-20,-0.37) |
| **P04114** (P04114;516,-25,-0.04) | **P78536** (ADAM17;93,-22,-0.23) | **Q13873** (BMPR2;115,-19,-0.16) |
| **P55072** (P55072;89,-25,-0.27) | **P46100** (ATRX;283,-22,-0.07) | **Q9BSQ5** (CCM2;49,-19,-0.38) |
| **P16471** (P16471;70,-25,-0.35) | **P54252** (ATXN3;42,-22,-0.52) | **Q9BS16** (CENPK;32,-19,-0.60) |
| **Q9BVA1** (Q9BVA1;50,-25,-0.50) | **Q96JP9** (CDHR1;94,-22,-0.23) | **Q9UHC6** (CNTNAP2;148,-19,-0.12) |
| **O00468** (AGRN;217,-24,-0.11) | **Q96HD1** (CRELD1;45,-22,-0.48) | **Q15303** (ERBB4;147,-19,-0.12) |
| **P22607** (FGFR3;88,-19,-0.21) | **P06865** (P06865;61,-18,-0.29) | **Q9NZQ3** (NCKIPSD;79,-16,-0.20) |
| **O43524** (FOXO3;71,-19,-0.26) | **Q9UP52** (Q9UP52;89,-18,-0.20) | **Q15788** (NCOA1;157,-16,-0.10) |
| **P43080** (GUCA 1A;23,-19,-0.82) | **Q13705** (Q13705;58,-18,-0.31) | **Q9NZ94** (NLGN3;94,-16,-0.17) |
| **P22304** (IDS;62,-19,-0.30) | **Q5VWK5** (Q5VWK5;72,-18,-0.25) | **Q8WX94** (NLRP7;112,-16,-0.14) |
| **Q7Z4S6** (KIF21A;187,-19,-0.10) | **P06239** (P06239;58,-18,-0.31) | **P12955** (PEPD;55,-16,-0.29) |
| **Q13753** (LAMC2;131,-19,-0.14) | **P09603** (P09603;60,-18,-0.29) | **Q7LG56** (RRM2B;41,-16,-0.39) |
| **Q03252** (LMNB2;68,-19,-0.28) | **P40337** (P40337;24,-18,-0.74) | **P46937** (YAP1;54,-16,-0.29) |
| **P10911** (MCF2;108,-19,-0.17) | **P50993** (ATP1A2;112,-17,-0.15) | **P43251** (P43251;61,-16,-0.26) |
| **Q86YR7** (MCF2L2;127,-19,-0.14) | **Q9H165** (BCL11A;91,-17,-0.18) | **P48165** (P48165;48,-16,-0.33) |
| **Q13562** (NEUROD1;40,-19,-0.47) | **P01031** (C5;188,-17,-0.09) | **Q9Y6B7** (Q9Y6B7;83,-16,-0.19) |
| **Q8N0W4** (NLGN4X;92,-19,-0.20) | **Q01668** (CACNA1D;245,-17,-0.06) | **Q86Y56** (Q86Y56;94,-16,-0.17) |
| **Q5SRE5** (NUP188;196,-19,-0.09) | **O75838** (CIB2;22,-17,-0.78) | **Q9H5Y7** (Q9H5Y7;95,-16,-0.16) |
| **O75665** (OFD1;117,-19,-0.16) | **Q9Y2V7** (COG6;73,-17,-0.23) | **P42658** (P42658;98,-16,-0.16) |
| **Q96MT3** (PRICKLE1;94,-19,-0.20) | **P07333** (CSF1R;108,-17,-0.15) | **P51692** (P51692;90,-16,-0.17) |
| **P98175** (RBM10;104,-19,-0.18) | **P35638** (DDIT3;19,-17,-0.88) | **P37173** (P37173;65,-16,-0.24) |
| **Q9UQD0** (SCN8A;225,-19,-0.08) | **O75140** (DEPDC5;181,-17,-0.09) | **Q969F9** (Q969F9;114,-16,-0.14) |
| **Q9NZV5** (SEPN1;66,-19,-0.28) | **Q9UNE0** (EDAR;49,-17,-0.34) | **Q8NF50** (Q8NF50;239,-16,-0.06) |
| **Q9Y6Q6** (TNFRSF11A;66,-19,-0.28) | **P21802** (FGFR2;92,-17,-0.18) | **P35527** (P35527;62,-16,-0.25) |
| **Q8WW38** (ZFPM2;128,-19,-0.14) | **Q96FT9** (IFT43;24,-17,-0.72) | **Q6T4R5** (Q6T4R5;179,-16,-0.08) |
| **P12111** (P12111;344,-19,-0.05) | **P43243** (MATR3;95,-17,-0.17) | **C9JLR9** (C9JLR9;73,-16,-0.21) |
| **P18206** (P18206;124,-19,-0.15) | **Q8NI22** (MCFD2;16,-17,-1.03) | **Q04695** (Q04695;48,-16,-0.33) |
| **P08670** (P08670;54,-19,-0.35) | **Q16620** (NTRK2;92,-17,-0.18) | **Q8WW4** (Q8WVV4;68,-16,-0.23) |
| **Q12860** (Q12860;113,-19,-0.16) | **P29120** (PCSK1;84,-17,-0.20) | **P13942** (P13942;172,-16,-0.09) |
| **Q96MW5** (Q96MW5;68,-19,-0.27) | **Q9NQ66** (PLCB1;139,-17,-0.12) | **P52788** (P52788;41,-16,-0.38) |
| **Q04446** (Q04446;80,-19,-0.23) | **O94827** (PLEKHG5;117,-17,-0.14) | **O43307** (ARHGEF9;61,-15,-0.24) |
| **Q96QS3** (Q96QS3;58,-19,-0.32) | **P27169** (PON1;40,-17,-0.42) | **Q6ZW61** (BBS12;79,-15,-0.18) |
| **O60568** (060568;85,-19,-0.22) | **Q99708** (RBBP8;102,-17,-0.16) | **Q13936** (CACNA1C;249,-15,-0.06) |
| **Q6EKJ0** (Q6EKJ0;107,-19,-0.17) | **Q14108** (SCARB2;54,-17,-0.31) | **Q13698** (CACNA1S;212,-15,-0.07) |
| **O14497** (ARID1A;242,-18,-0.07) | **Q16586** (SGCA;43,-17,-0.39) | **Q9H444** (CHMP4B;25,-15,-0.60) |
| **P01024** (C3;187,-18,-0.09) | **Q07837** (SLC3A1;79,-17,-0.21) | **A8TX70** (COL6A5;290,-15,-0.05) |
| **Q9H467** (CUEDC2;32,-18,-0.56) | **P22735** (TGM1;90,-17,-0.18) | **Q9Y4D2** (DAGLA;115,-15,-0.13) |
| **Q96SD1** (DCLRE1C;78,-18,-0.22) | **Q9Y6N9** (USH1C;62,-17,-0.27) | **O60443** (DFNA5;55,-15,-0.27) |
| **Q8IZD9** (DOCK3;233,-18,-0.07) | **Q96K83** (ZNF521;148,-17,-0.11) | **Q9NRI5** (DISC1;94,-15,-0.16) |
| **Q99814** (EPAS1;96,-18,-0.18) | **Q96M63** (Q96M63;75,-17,-0.22) | **Q14574** (DSC3;100,-15,-0.15) |
| **O60447** (EVI5;93,-18,-0.19) | **P02671** (P02671;95,-17,-0.17) | **O15287** (FANCG;69,-15,-0.21) |
| **Q9HCG7** (GBA2;105,-18,-0.17) | **Q7RTS9** (Q7RTS9;76,-17,-0.22) | **P98174** (FGD1;107,-15,-0.14) |
| **Q92990** (GLMN;68,-18,-0.26) | **Q9Y664** (Q9Y664;48,-17,-0.35) | **P98177** (FOXO4;54,-15,-0.27) |
| **Q8TBA6** (GOLGA5;83,-18,-0.21) | **P13646** (P13646;50,-17,-0.34) | **Q9H2C0** (GAN;68,-15,-0.22) |
| **Q9HD26** (GOPC;51,-18,-0.35) | **Q5JTZ9** (AARS2;107,-16,-0.14) | **P31150** (GDI1;51,-15,-0.29) |
| **Q96EW2** (HSPBAP1;55,-18,-0.32) | **P35573** (AGL;175,-16,-0.09) | **P13807** (GYS1;84,-15,-0.17) |
| **P42701** (IL12RB1;73,-18,-0.24) | **O00203** (AP3B1;121,-16,-0.13) | **P20823** (HNF1A;67,-15,-0.22) |
| **O60282** (KIF5C;109,-18,-0.16) | **Q9HBG4** (ATP6V0A4;96,-16,-0.16) | **P78318** (IGBP1;39,-15,-0.38) |
| **Q9BXB1** (LGR4;104,-18,-0.17) | **Q9UBB4** (ATXN10;53,-16,-0.29) | **O14920** (IKBKB;87,-15,-0.17) |
| **Q96GX5** (MASTL;97,-18,-0.18) | **Q8IWZ6** (BBS7;80,-16,-0.19) | **P08779** (KRT16;51,-15,-0.29) |
| **Q03112** (MECOM;118,-18,-0.15) | **P20807** (CAPN3;94,-16,-0.16) | **P13796** (LCP1;70,-15,-0.21) |
| **Q969V6** (MKL1;99,-18,-0.18) | **P19835** (CEL;79,-16,-0.20) | **P48357** (LEPR;132,-15,-0.11) |
| **Q9BV36** (MLPH;66,-18,-0.27) | **Q9ULV3** (CIZ1;100,-16,-0.15) | **Q13064** (MKRN3;56,-15,-0.26) |
| **P49959** (MRE11A;81,-18,-0.22) | **Q16828** (DUSP6;42,-16,-0.37) | **Q00872** (MYBPC1;128,-15,-0.11) |
| **Q86YC2** (PALB2;131,-18,-0.13) | **Q12805** (EFEMP1;55,-16,-0.29) | **Q9C000** (NLRP1;166,-15,-0.09) |
| **Q9Y4G2** (PLEKHM1;117,-18,-0.15) | **Q96M96** (FGD4;87,-16,-0.18) | **O75914** (PAK3;62,-15,-0.24) |
| **O75298** (RTN2;59,-18,-0.30) | **P11362** (FGFR1;92,-16,-0.17) | **Q92733** (PRCC;52,-15,-0.28) |
| **Q05516** (ZBTB16;74,-18,-0.24) | **Q96RU3** (FNBP1;71,-16,-0.22) | **P49768** (PSEN1;53,-15,-0.28) |
| **Q9UBW7** (ZMYM2;155,-18,-0.11) | **Q92902** (HPS1;79,-16,-0.20) | **P01009** (SERPINA1;47,-15,-0.32) |
| **Q9BXC9** (Q9BXC9;80,-18,-0.22) | **Q9HBG6** (IFT122;142,-16,-0.11) | **Q9H173** (SIL1;52,-15,-0.28) |
| **O95677** (O95677;70,-18,-0.25) | **Q9UH77** (KLHL3;65,-16,-0.24) | **Q695T7** (SLC6A19;71,-15,-0.21) |
| **Q09470** (Q09470;56,-18,-0.31) | **P13645** (KRT10;59,-16,-0.27) | **O43295** (SRGAP3;125,-15,-0.12) |
| **Q70J99** (Q70J99;123,-18,-0.14) | **P08253** (MMP2;74,-16,-0.21) | **Q13586** (STIM1;77,-15,-0.19) |
| **P19532** (TFE3;62,-15,-0.24) | **Q07889** (Q07889;152,-14,-0.09) | **P00734** (P00734;70,-13,-0.18) |
| **Q96Q05** (TRAPPC9;129,-15,-0.11) | **P00740** (P00740;52,-14,-0.27) | **P16885** (P16885;148,-13,-0.08) |
| **Q9H0U9** (TSPYL1;49,-15,-0.30) | **P51687** (P51687;60,-14,-0.23) | **Q9UBK8** (Q9UBK8;80,-13,-0.16) |
| **Q9ULT0** (TTC7A;96,-15,-0.15) | **P15924** (P15924;332,-14,-0.04) | **Q9UDR5** (Q9UDR5;102,-13,-0.12) |
| **Q9P2L0** (WDR35;134,-15,-0.11) | **P15289** (P15289;54,-14,-0.26) | **Q3SXY7** (Q3SXY7;75,-13,-0.17) |
| **Q9Y462** (ZNF711;86,-15,-0.17) | **Q8NCR0** (Q8NCR0;57,-14,-0.24) | **Q8TD10** (Q8TD10;52,-13,-0.25) |
| **Q9UI47** (Q9UI47;100,-15,-0.15) | **P42224** (P42224;87,-14,-0.16) | **Q9NTX7** (Q9NTX7;39,-13,-0.33) |
| **P12110** (P12110;109,-15,-0.13) | **P68133** (ACTA1;42,-13,-0.30) | **Q9Y6H5** (Q9Y6H5;100,-13,-0.12) |
| **P55895** (P55895;59,-15,-0.25) | **P62736** (ACTA2;42,-13,-0.30) | **O00470** (000470;43,-13,-0.30) |
| **Q9Y3R0** (Q9Y3R0;122,-15,-0.12) | **P68032** (ACTC1;42,-13,-0.30) | **P17050** (P17050;47,-13,-0.27) |
| **P43246** (P43246;105,-15,-0.14) | **Q9NP73** (ALG13;126,-13,-0.10) | **P16871** (P16871;52,-13,-0.25) |
| **P42892** (P42892;87,-15,-0.17) | **P18054** (ALOX12;76,-13,-0.17) | **O95477** (ABCA1;254,-12,-0.04) |
| **O00206** (O00206;96,-15,-0.15) | **Q16671** (AMHR2;63,-13,-0.20) | **P63261** (ACTG1;42,-12,-0.28) |
| **Q5QGS0** (Q5QGS0;168,-15,-0.08) | **P08758** (ANXA5;36,-13,-0.36) | **Q86TH1** (ADAMTSL2;105,-12,-0.11) |
| **P17661** (P17661;54,-15,-0.28) | **Q8N9N2** (ASCC1;46,-13,-0.28) | **P15313** (ATP6V1B1;57,-12,-0.21) |
| **Q6DD88** (Q6DD88;61,-15,-0.24) | **Q08AD1** (CAMSAP2;168,-13,-0.07) | **Q13489** (BIRC3;68,-12,-0.17) |
| **O15294** (O15294;117,-15,-0.12) | **P24385** (CCND1;34,-13,-0.38) | **P08603** (CFH;139,-12,-0.08) |
| **P13797** (P13797;71,-15,-0.21) | **P11230** (CHRNB1;57,-13,-0.22) | **Q6P4Q7** (CNNM4;87,-12,-0.13) |
| **Q9NWZ3** (Q9NWZ3;52,-15,-0.29) | **Q04844** (CHRNE;55,-13,-0.23) | **O75718** (CRTAP;47,-12,-0.25) |
| **Q6JEL2** (Q6JEL2;69,-15,-0.21) | **Q96SW2** (CRBN;51,-13,-0.25) | **P00167** (CYB5A;15,-12,-0.78) |
| **O14896** (O14896;53,-15,-0.28) | **Q6UUV9** (CRTC1;67,-13,-0.19) | **P00533** (EGFR;134,-12,-0.08) |
| **Q9H2U9** (ADAM7;86,-14,-0.16) | **P24855** (DNASE1;31,-13,-0.41) | **P00488** (F13A1;83,-12,-0.14) |
| **Q9NZN9** (AIPL1;44,-14,-0.31) | **Q8N608** (DPP10;91,-13,-0.14) | **Q00597** (FANCC;63,-12,-0.18) |
| **P35670** (ATP7B;157,-14,-0.08) | **Q99607** (ELF4;71,-13,-0.18) | **O15409** (FOXP2;80,-12,-0.15) |
| **P46736** (BRCC3;36,-14,-0.38) | **P50549** (ETV1;55,-13,-0.23) | **Q9Y625** (GPC6;63,-12,-0.19) |
| **Q9P2K1** (CC2D2A;186,-14,-0.07) | **Q9HB96** (FANCE;59,-13,-0.22) | **P81274** (GPSM2;77,-12,-0.15) |
| **P48643** (CCT5;60,-14,-0.23) | **P09769** (FGR;59,-13,-0.21) | **Q9UMX6** (GUCA1B;23,-12,-0.51) |
| **Q8N9W5** (DNAAF3;59,-14,-0.23) | **Q08334** (IL10RB;37,-13,-0.35) | **Q9UKV0** (HDAC9;111,-12,-0.10) |
| **P29317** (EPHA2;108,-14,-0.12) | **P48544** (KCNJ5;48,-13,-0.27) | **Q9Y6K9** (IKBKG;48,-12,-0.24) |
| **P02679** (FGG;52,-14,-0.27) | **Q9GZY6** (LAT2;27,-13,-0.48) | **Q13422** (IKZF1;58,-12,-0.20) |
| **Q8NFG4** (FLCN;64,-14,-0.21) | **P01106** (MYC;49,-13,-0.26) | **Q9Y287** (ITM2B;30,-12,-0.39) |
| **O15353** (FOXN1;69,-14,-0.20) | **Q15596** (NCOA2;159,-13,-0.08) | **Q15046** (KARS;68,-12,-0.17) |
| **P48506** (GCLC;73,-14,-0.19) | **Q9HC29** (NOD2;115,-13,-0.11) | **P63252** (KCNJ2;48,-12,-0.24) |
| **P19087** (GNAT2;40,-14,-0.34) | **Q7Z494** (NPHP3;151,-13,-0.08) | **Q9Y5K2** (KLK4;27,-12,-0.44) |
| **Q9BY41** (HDAC8;42,-14,-0.33) | **Q86U42** (PABPN1;33,-13,-0.39) | **Q8NG48** (LINS;86,-12,-0.13) |
| **O15357** (INPPL1;139,-14,-0.10) | **Q9H5K3** (POMK;40,-13,-0.32) | **Q9H3L0** (MMADHC;33,-12,-0.36) |
| **P14923** (JUP;82,-14,-0.17) | **P37231** (PPARG;58,-13,-0.22) | **P14780** (MMP9;78,-12,-0.15) |
| **Q6UWE0** (LRSAM1;84,-14,-0.16) | **Q05823** (RNASEL;84,-13,-0.15) | **P21757** (MSR1;50,-12,-0.24) |
| **Q9Y6D9** (MAD1L1;83,-14,-0.16) | **Q9HCK4** (ROBO2;151,-13,-0.08) | **P00403** (MT-CO2;26,-12,-0.46) |
| **Q9ULH7** (MKL2;118,-14,-0.11) | **Q01974** (ROR2;105,-13,-0.12) | **Q96P20** (NLRP3;118,-12,-0.10) |
| **P04198** (MYCN;50,-14,-0.28) | **O95248** (SBF1;208,-13,-0.06) | **Q8IXF0** (NPAS3;101,-12,-0.11) |
| **Q92597** (NDRG1;43,-14,-0.32) | **Q9NY72** (SCN3B;25,-13,-0.52) | **P04629** (NTRK1;87,-12,-0.13) |
| **P49902** (NT5C2;65,-14,-0.21) | **Q8N0X7** (SPG20;73,-13,-0.17) | **Q8NBP7** (PCSK9;74,-12,-0.16) |
| **O15160** (POLR1C;39,-14,-0.35) | **Q8N205** (SYNE4;44,-13,-0.29) | **O95263** (PDE8B;99,-12,-0.12) |
| **Q4J6C6** (PREPL;84,-14,-0.16) | **P15923** (TCF3;68,-13,-0.19) | **P27986** (PIK3R1;84,-12,-0.14) |
| **P07225** (PROS1;75,-14,-0.18) | **O14763** (TNFRSF10B;48,-13,-0.27) | **P29590** (PML;98,-12,-0.12) |
| **Q14289** (PTK2B;116,-14,-0.12) | **O43280** (TREH;67,-13,-0.19) | **Q13976** (PRKG1;76,-12,-0.15) |
| **Q9UHX1** (PUF60;60,-14,-0.23) | **Q9UPN9** (TRIM33;123,-13,-0.10) | **Q13635** (PTCH1;161,-12,-0.07) |
| **P20936** (RASA1;116,-14,-0.12) | **P17643** (TYRP1;61,-13,-0.21) | **Q05397** (PTK2;119,-12,-0.10) |
| **Q8HWS3** (RFX6;102,-14,-0.13) | **Q2YD98** (UVSSA;81,-13,-0.16) | **P07988** (SFTPB;42,-12,-0.28) |
| **P12271** (RLBP1;36,-14,-0.38) | **Q14202** (ZMYM3;152,-13,-0.08) | **Q8IVB4** (SLC9A9;73,-12,-0.16) |
| **Q96RN1** (SLC26A8;109,-14,-0.12) | **P14679** (P14679;60,-13,-0.21) | **O75044** (SRGAP2;121,-12,-0.09) |
| **Q9BSE2** (TMEM79;44,-14,-0.32) | **P14136** (P14136;50,-13,-0.26) | **Q16623** (STX1A;33,-12,-0.36) |
| **Q13515** (Q13515;46,-14,-0.30) | **Q3MJ13** (Q3MJ13;123,-13,-0.10) | **P14373** (TRIM27;58,-12,-0.20) |
| **Q96G97** (Q96G97;44,-14,-0.31) | **P02452** (P02452;139,-13,-0.09) | **Q7Z4N2** (TRPM1;182,-12,-0.06) |
| **P02533** (P02533;52,-14,-0.27) | **P02748** (P02748;63,-13,-0.20) | **Q01534** (TSPY1;35,-12,-0.34) |
| **Q15822** (Q15822;60,-14,-0.23) | **Q9NPB3** (Q9NPB3;24,-13,-0.53) | **Q8NEZ2** (VPS37A;44,-12,-0.27) |
| **Q9ULV5** (Q9ULV5;53,-12,-0.22) | **Q7RTS3** (PTF1A;35,-11,-0.31) | **Q2M1P5** (KIF7;151,-10,-0.06) |
| **P09172** (P09172;69,-12,-0.17) | **Q04206** (RELA;60,-11,-0.18) | **P01042** (KNG1;72,-10,-0.13) |
| **P60709** (P60709;42,-12,-0.28) | **P07949** (RET;124,-11,-0.08) | **Q14693** (LPIN1;99,-10,-0.10) |
| **Q07699** (Q07699;25,-12,-0.48) | **P35237** (SERPINB6;43,-11,-0.25) | **Q96KG7** (MEGF10;122,-10,-0.08) |
| **P56192** (P56192;101,-12,-0.11) | **Q99961** (SH3GL1;41,-11,-0.26) | **P08254** (MMP3;54,-10,-0.18) |
| **Q9BQE9** (Q9BQE9;22,-12,-0.54) | **A0AV02** (SLC12A8;78,-11,-0.14) | **P40238** (MPL;71,-10,-0.14) |
| **Q9HCR9** (Q9HCR9;105,-12,-0.11) | **P36021** (SLC16A2;60,-11,-0.18) | **Q8NCE2** (MTMR14;72,-10,-0.13) |
| **P11498** (P11498;130,-12,-0.09) | **Q9BRI3** (SLC30A2;35,-11,-0.31) | **P41227** (NAA10;26,-10,-0.37) |
| **Q5T4F4** (Q5T4F4;46,-12,-0.26) | **Q9NQ40** (SLC52A3;51,-11,-0.21) | **Q9NXR1** (NDE1;39,-10,-0.25) |
| **O43572** (043572;74,-12,-0.16) | **Q9GZV3** (SLC5A7;63,-11,-0.17) | **Q01968** (OCRL;104,-10,-0.09) |
| **P04070** (P04070;52,-12,-0.23) | **Q9P0W8** (SPATA7;68,-11,-0.16) | **Q16633** (POU2AF1;27,-10,-0.36) |
| **B7U540** (B7U540;49,-12,-0.24) | **Q9Y5Y6** (ST14;95,-11,-0.11) | **Q8WYP3** (RIN2;100,-10,-0.09) |
| **P31749** (AKT1;56,-11,-0.19) | **Q9UL17** (TBX21;58,-11,-0.18) | **Q8WU17** (RNF139;76,-10,-0.13) |
| **P02768** (ALB;69,-11,-0.15) | **P09327** (VIL1;93,-11,-0.11) | **Q86WG5** (SBF2;208,-10,-0.04) |
| **Q6UB99** (ANKRD11;298,-11 ,-0.03) | **O95876** (WDPCP;85,-11,-0.12) | **Q8IWL2** (SFTPA1;26,-10,-0.38) |
| **P10275** (AR;99,-11,-0.11) | **O75800** (ZMYND10;50,-11,-0.21) | **Q96PX8** (SLITRK1;78,-10,-0.12) |
| **Q15052** (ARHGEF6;87,-11,-0.12) | **Q03154** (Q03154;46,-11,-0.23) | **O95630** (STAMBP;48,-10,-0.20) |
| **P22681** (CBL;100,-11,-0.11) | **Q96C11** (Q96C11;60,-11,-0.18) | **P07101** (TH;59,-10,-0.17) |
| **Q9HC77** (CENPJ;153,-11,-0.07) | **P31327** (P31327;165,-11,-0.06) | **O60602** (TLR5;98,-10,-0.10) |
| **O96017** (CHEK2;61,-11,-0.18) | **Q9UFE4** (Q9UFE4;110,-11,-0.10) | **O14773** (TPP1;61,-10,-0.16) |
| **Q9UDT6** (CLIP2;116,-11,-0.09) | **P11597** (P11597;55,-11,-0.20) | **P23258** (TUBG1;51,-10,-0.19) |
| **P21918** (DRD5;53,-11,-0.20) | **P57796** (P57796;30,-11,-0.36) | **Q9NRM1** (Q9NRM1;129,-10,-0.07) |
| **P49770** (EIF2B2;39,-11,-0.28) | **Q9H6U6** (Q9H6U6;101,-11,-0.10) | **P06396** (P06396;86,-10,-0.11) |
| **P07332** (FES;93,-11,-0.11) | **Q4AC94** (Q4AC94;260,-11,-0.04) | **Q9BUN5** (Q9BUN5;22,-10,-0.45) |
| **Q9H334** (FOXP1;75,-11,-0.14) | **P51168** (P51168;73,-11,-0.15) | **Q9NQ48** (Q9NQ48;35,-10,-0.28) |
| **P02794** (FTH1;21,-11,-0.51) | **Q96J02** (Q96J02;103,-11,-0.10) | **Q99502** (Q99502;65,-10,-0.15) |
| **O95467** (GNAS;28,-11,-0.39) | **Q9H3S4** (Q9H3S4;27,-11,-0.40) | **Q9H257** (Q9H257;62,-10,-0.16) |
| **P11488** (GNAT1;40,-11,-0.27) | **Q96EN8** (Q96EN8;98,-11,-0.11) | **Q14896** (Q14896;141,-10,-0.07) |
| **Q13224** (GRIN2B;166,-11,-0.06) | **P00813** (ADA;41,-10,-0.24) | **P05997** (P05997;145,-10,-0.06) |
| **P46976** (GYG1;39,-11,-0.27) | **Q9Y243** (AKT3;56,-10,-0.17) | **P57679** (P57679;112,-10,-0.08) |
| **P56524** (HDAC4; 119,-11 ,-0.09) | **Q9UNA1** (ARHGAP26;92,-10,-0.10) | **Q96MP8** (Q96MP8;33,-10,-0.30) |
| **Q9NZN1** (IL1RAPL1;80,-11,-0.13) | **Q8WXF7** (ATL1;64,-10,-0.15) | **P09471** (P09471;40,-10,-0.24) |
| **P05783** (KRT18;48,-11,-0.22) | **Q4VNC1** (ATP13A4;134,-10,-0.07) | **P06280** (P06280;49,-10,-0.20) |
| **Q8N653** (LZTR1;95,-11,-0.11) | **Q9NPF0** (CD320;29,-10,-0.34) | **P05160** (P05160;76,-10,-0.13) |
| **Q93074** (MED12;243,-11,-0.04) | **P11912** (CD79A;25,-10,-0.39) | **Q96LT7** (Q96LT7;54,-10,-0.18) |
| **Q96G30** (MRAP2;24,-11,-0.46) | **P02708** (CHRNA1;55,-10,-0.18) | **Q9UHC1** (Q9UHC1;164,-10,-0.06) |
| **Q86TC9** (MYPN;145,-11,-0.07) | **O15111** (CHUK;85,-10,-0.11) | **Q13568** (Q13568;56,-10,-0.17) |
| **Q13772** (NCOA4;70,-11,-0.15) | **Q9H8M5** (CNNM2;97,-10,-0.10) | **P04180** (P04180;50,-10,-0.20) |
| **Q92542** (NCSTN;78,-11,-0.14) | **P43146** (DCC;158,-10,-0.06) | **O00459** (000459;82,-10,-0.12) |
| **P12036** (NEFH;112,-11,-0.09) | **Q96HY7** (DHTKD1;103,-10,-0.09) | **P51178** (P51178;86,-10,-0.11) |
| **Q86U86** (PBRM1;193,-11,-0.05) | **Q9H3Z4** (DNAJC5;22,-10,-0.45) | **C9JR72** (C9JR72;49,-10,-0.20) |
| **P35558** (PCK1;69,-11,-0.15) | **Q9NVI1** (FANCl;149,-10,-0.06) | **P07359** (P07359;72,-10,-0.13) |
| **Q13608** (PEX6;104,-11,-0.10) | **Q9Y223** (GNE;79,-10,-0.12) | **O75695** (075695;40,-10,-0.25) |
| **O00628** (PEX7;36,-11,-0.30) | **P03989** (HLA-B;40,-10,-0.24) | |
| **O43586** (PSTPIP1;48,-11,-0.23) | **O43364** (HOXA2;41,-10,-0.24) | |

For each protein, a unique Uniprot identifier is provided in bold. In parentheses, an exemplary name of the gene encoding the respective protein as well as its molecular weight, charge, and molecular weight charge ratio are provided.

**Table 5. Exemplary naturally occurring negatively supercharged proteins, a deficiency or recessive allele of which is implicated in diseases, disorders, or conditions. Proteins listed have a negative net charge of -10 or less. For each protein, a unique Uniprot identifier is provided in bold. In parentheses, an exemplary name of the gene encoding the respective protein as well as its molecular weight, charge, and molecular weight charge ratio are provided.**

| | | |
|---|---|---|
| **Q03001** (DST;861,-316,-0.36) | **Q68DK2** (ZFYVE26;285,-49,-0.17) | **Q66GS9** (CEP135;133,-27,-0.20) |
| **Q8NF91** (SYNE1;1,011,-315,-0.31) | **Q6ZNJ1** (NBEAL2;303,-48,-0.15) | **Q6P5W5** (SLC39A4;68,-27,-0.39) |
| **P98164** (P98164;522,-243,-0.46) | **P14410** (P14410;209,-48,-0.22) | **Q7RTW8** (Q7RTW8;129,-27,-0.21) |
| **Q6V0I7** (FAT4;543,-230,-0.42) | **O15078** (CEP290;290,-47,-0.16) | **P06213** (P06213;156,-27,-0.17) |
| **Q9H251** (CDH23;369,-223,-0.60) | **Q9P2D1** (CHD7;336,-47,-0.13) | **P12821** (ACE;150,-26,-0.17) |
| **P21817** (RYR1;565,-207,-0.36) | **Q9HCE0** (EPG5;292,-47,-0.16) | **P83436** (COG7;86,-26,-0.30) |
| **Q7Z6Z7** (HUWE1;482,-173, -0.35) | **Q9HC10** (OTOF;227,-47,-0.20) | **Q9UDY8** (MALT1;92,-26,-0.28) |
| **Q96JQ0** (DCHS1;346,-162,-0.46) | **O94972** (TRIM37; 108,-47,-0.43) | **O00462** (MANBA;101,-26,-0.25) |
| **P02549** (SPTA1;280,-153,-0.54) | **Q7RTU9** (Q7RTU9;193,-47,-0.24) | **Q7Z7M0** (MEGF8;303,-26,-0.08) |
| **P22105** (TNXB;464,-144,-0.31) | **Q8NCM8** (DYNC2H1;493,-46,-0.09) | **Q15477** (SKIV2L;138,-26,-0.18) |
| **Q8N4C6** (NIN;243,-138,-0.56) | **Q96RL7** (VPS13A;360,-45,-0.12) | **Q9H9E3** (Q9H9E3;89,-26,-0.29) |
| **O60494** (CUBN;399,-133,-0.33) | **Q9BUR4** (WRAP53;59,-45,-0.75) | **Q3SYG4** (BBS9;99,-25,-0.25) |
| **Q4G0P3** (HYDIN;576,-113,-0.19) | **Q96F46** (IL17RA;96,-44,-0.45) | **O94830** (DDHD2;81,-25,-0.30) |
| **Q13316** (Q13316;56,-101,-1.81) | **P98073** (TMPRSS15;113,-43,-0.38) | **O95967** (EFEMP2;49,-25,-0.50) |
| **Q3ZCN5** (OTOGL;262,-98,-0.37) | **Q9P219** (CCDC88C;228,-42,-0.18) | **Q04656** (ATP7A;163,-24,-0.14) |
| **P07942** (P07942;198,-98,-0.49) | **Q9H2M9** (RAB3GAP2;156,-42,-0.26) | **Q9BXL7** (CARD11; 133,-24,-0.18) |
| **Q8TCU4** (ALMS1;461,-96,-0.20) | **P00450** (P00450;122,-42,-0.34) | **Q9NQX3** (GPHN;80,-24,-0.30) |
| **O75970** (MPDZ;222,-94,-0.42) | **P24386** (CHM;73,-41,-0.55) | **P32004** (L1CAM;140,-24,-0.17) |
| **P04275** (VWF;309,-89,-0.28) | **Q9Y5B0** (CTDP1;104,-41,-0.39) | **O60331** (PIP5K1C;73,-24,-0.32) |
| **P11532** (P11532;427,-89,-0.20) | **Q96RT7** (TUBGCP6;200,-41,-0.20) | **Q8TF17** (SH3TC2;145,-24,-0.16) |
| **Q8NG31** (CASC5;265,-84,-0.31) | **Q96RY7** (IFT140;165,-40,-0.24) | **Q15468** (STIL;143,-24,-0.16) |
| **Q15149** (PLEC;532,-83,-0.15) | **Q13085** (ACACA;266,-39,-0.14) | **P50747** (P50747;81,-24,-0.29) |
| **O95714** (095714;527,-83,-0.15) | **Q9UMZ3** (Q9UMZ3;261,-39,-0.14) | **P15391** (P15391;61,-24,-0.39) |
| **P98160** (P98160;469,-83,-0.17) | **Q9UPZ3** (HPS5;127,-38,-0.29) | **P42702** (P42702;124,-24,-0.19) |
| **P50851** (LRBA;319,-82,-0.25) | **Q9UG01** (IFT172;198,-38,-0.19) | **P16435** (POR;77,-23,-0.29) |
| **Q14767** (LTBP2;195,-77,-0.39) | **O15040** (TECPR2;154,-38,-0.24) | **O14593** (RFXANK;28,-23,-0.81) |
| **Q5T011** (SZT2;378,-77,-0.20) | **P16144** (P16144;202,-38,-0.18) | **Q8NEZ3** (WDR19;152,-23,-0.15) |
| **Q6ZRI0** (OTOG;315,-74,-0.23) | **P09848** (LCT;219,-37,-0.16) | **Q2M1K9** (ZNF423;145,-23,-0.15) |
| **Q93008** (USP9X;292,-72,-0.24) | **Q07864** (POLE;262,-37,-0.14) | **Q13315** (Q13315;351,-23,-0.06) |
| **Q96QU1** (PCDH15;216,-71,-0.32) | **Q8NEL9** (DDHD1;100,-36,-0.35) | **P42898** (P42898;75,-23,-0.30) |
| **Q96JI7** (SPG11;279,-71,-0.25) | **Q9NZJ5** (EIF2AK3;125,-36,-0.28) | **P21860** (ERBB3;148,-22,-0.14) |
| **Q8TE73** (DNAH5;529,-67,-0.12) | **Q9H6L5** (Q9H6L5;55,-36,-0.65) | **Q9UBX5** (FBLN5;50,-22,-0.43) |
| **Q9UPV0** (CEP164;164,-66,-0.40) | **P78363** (ABCA4;256,-35,-0.13) | **Q13043** (STK4;56,-22,-0.39) |
| **P82279** (P82279;154,-65,-0.42) | **Q92539** (LPIN2;99,-35,-0.35) | **Q14191** (WRN;162,-22,-0.13) |
| **O75147** (OBSL1;207,-64,-0.30) | **Q96PY6** (NEK1;143,-35,-0.24) | **O00187** (O00187;76,-22,-0.29) |
| **Q12955** (Q12955;480,-64,-0.13) | **Q99707** (Q99707;141 ,-35,-0.24) | **Q02388** (Q02388;295,-22,-0.07) |
| **Q96DT5** (Q96DT5;521,-63,-0.12) | **Q8WZ55** (BSND;35,-34,-0.96) | **O43933** (043933;143,-22,-0.15) |
| **A2RRP1** (NBAS;269,-62,-0.23) | **Q9P2K8** (EIF2AK4;187,-34,-0.18) | **Q96MT8** (Q96MT8;81,-22,-0.27) |
| **Q96SN8** (CDK5RAP2;215,-61,-0.28) | **P33076** (CIITA;124,-33,-0.26) | **P35523** (CLCN1;109,-21,-0.19) |
| **P16157** (ANK1;206,-60,-0.29) | **O94898** (LRIG2;119,-33,-0.27) | **Q8IYB7** (DIS3L2;99,-21,-0.21) |
| **Q9NWF9** (RNF216;99,-60,-0.60) | **Q96EV8** (DTNBP1;39,-32,-0.81) | **Q96AY3** (FKBP10;64,-21,-0.32) |
| **P22223** (CDH3;91,-59,-0.64) | **Q9H4Z2** (Q9H4Z2;145,-32,-0.22) | **O00165** (HAX1;32,-21,-0.66) |
| **O94986** (CEP152;196,-58,-0.29) | **Q9HBE5** (IL21R;59,-31,-0.52) | **P42704** (LRPPRC;158,-21 ,-0.13) |
| **O75197** (LRP5;179,-58,-0.32) | **P08575** (PTPRC;147,-31,-0.21) | **P40692** (MLH1;85,-21,-0.24) |
| **Q7Z7G8** (VPS13B;449,-58,-0.12) | **P09871** (P09871;77,-31,-0.40) | **P07602** (PSAP;58,-21,-0.36) |
| **Q8IVV2** (Q8IVV2;222,-57,-0.25) | **Q13683** (ITGA7;129,-30,-0.23) | **Q14790** (CASP8;55,-20,-0.36) |
| **P12259** (P12259;252,-57,-0.22) | **Q9H4A3** (WNK1;251,-30,-0.11) | **Q00653** (NFKB2;97,-20,-0.20) |
| **Q99698** (LYST;429,-56,-0.13) | **Q9BY79** (Q9BY79;62,-30,-0.48) | **O14841** (O14841;137,-20,-0.14) |
| **O75443** (C75443;240,-56,-0.23) | **Q9Y6D5** (ARFGEF2;202,-29,-0.14) | **Q92622** (Q92622;109,-20,-0.18) |
| **P08F94** (PKHD1;447,-54,-0.12) | **P11171** (EPB41;97,-29,-0.29) | **Q03252** (LMNB2;68,-19,-0.28) |
| **O15020** (SPTBN2;271,-53,-0.19) | **Q12756** (KIF1A;191,-29,-0.15) | **Q96MT3** (PRICKLE1;94,-19,-0.20) |
| **Q6PRD1** (GPR179;257,-52,-0.20) | **P25963** (NFKBIA;36,-29,-0.81) | **Q9Y6Q6** (TNFRSF11A;66,-19,-0.28) |
| **O60721** (SLC24A1;121,-52,-0.42) | **Q9NQG7** (HPS4;77,-28,-0.36) | **Q8WW38** (ZFPM2;128,-19,-0.14) |
| **Q14999** (CUL7;191,-50,-0.26) | **O15259** (NPHP1;83,-28,-0.33) | **Q12860** (Q12860;113,-19,-0.16) |
| **O75923** (DYSF;237,-50,-0.21) | **P47712** (PLA2G4A;85,-28,-0.32) | **Q96MW5** (Q96MW5;68,-19,-0.27) |
| **P28715** (ERCC5;133,-49,-0.36) | **P15260** (P15260;54,-28,-0.51) | **Q96QS3** (Q96QS3;58,-19,-0.32) |
| **O43379** (WDR62;166,-49,-0.29) | **P06753** (P06753;33,-28,-0.84) | **O60568** (060568;85,-19,-0.22) |
| **P01024** (C3;187,-18,-0.09) | **Q9H173** (SIL1;52,-15,-0.28) | **Q86TH1** (ADAMTSL2;105,-12,-0.11) |
| **Q96SD1** (DCLRE1C;78,-18,-0.22) | **Q695T7** (SLC6A19;71,-15,-0.21) | **P15313** (ATP6V1B1;57,-12,-0.21) |
| **Q9HCG7** (GBA2;105,-18,-0.17) | **Q13586** (STIM1;77,-15,-0.19) | **P08603** (CFH;139,-12,-0.08) |
| **P42701** (IL12RB1;73,-18,-0.24) | **Q96Q05** (TRAPPC9;129,-15,-0.11) | **O75718** (CRTAP;47,-12,-0.25) |
| **Q9BV36** (MLPH;66,-18,-0.27) | **Q9H0U9** (TSPYL1;49,-15,-0.30) | **P00488** (F13A1;83,-12,-0.14) |
| **P49959** (MRE11A;81,-18,-0.22) | **Q9ULT0** (TTC7A;96,-15,-0.15) | **Q9Y625** (GPC6;63,-12,-0.19) |
| **Q9Y4G2** (PLEKHM1;117,-18,-0.15) | **Q9P2L0** (WDR35;134,-15,-0.11) | **P81274** (GPSM2;77,-12,-0.15) |
| **Q9BXC9** (Q9BXC9;80,-18,-0.22) | **P12110** (P12110;109,-15,-0.13) | **Q9Y6K9** (IKBKG;48,-12,-0.24) |
| **P06865** (P06865;61,-18,-0.29) | **P55895** (P55895;59,-15,-0.25) | **Q15046** (KARS;68,-12,-0.17) |
| **P40337** (P40337;24,-18,-0.74) | **Q9NWZ3** (Q9NWZ3;52,-15,-0.29) | **Q8NG48** (LINS;86,-12,-0.13) |
| **P01031** (C5;188,-17,-0.09) | **P35670** (ATP7B;157,-14,-0.08) | **P00403** (MT-CO2;26,-12,-0.46) |
| **O75838** (CIB2;22,-17,-0.78) | **P48643** (CCT5;60,-14,-0.23) | **P04629** (NTRK1;87,-12,-0.13) |
| **Q9Y2V7** (COG6;73,-17,-0.23) | **P02679** (FGG;52,-14,-0.27) | **P27986** (PIK3R1;84,-12,-0.14) |
| **Q9UNE0** (EDAR;49,-17,-0.34) | **Q8NFG4** (FLCN;64,-14,-0.21) | **Q8NEZ2** (VPS37A;44,-12,-0.27) |
| **Q8NI22** (MCFD2;16,-17,-1.03) | **O15353** (FOXN1;69,-14,-0.20) | **P09172** (P09172;69,-12,-0.17) |
| **P29120** (PCSK1;84,-17,-0.20) | **P48506** (GCLC;73,-14,-0.19) | **P11498** (P11498;130,-12,-0.09) |
| **O94827** (PLEKHG5;117,-17,-0.14) | **P14923** (JUP;82,-14,-0.17) | **P04070** (P04070;52,-12,-0.23) |
| **Q99708** (RBBP8;102,-17,-0.16) | **Q92597** (NDRG1;43,-14,-0.32) | **P10275** (AR;99,-11,-0.11) |
| **Q14108** (SCARB2;54,-17,-0.31) | **P49902** (NT5C2;65,-14,-0.21) | **Q15052** (ARHGEF6;87,-11,-0.12) |
| **Q16586** (SGCA;43,-17,-0.39) | **Q4J6C6** (PREPL;84,-14,-0.16) | **Q9HC77** (CENPJ;153,-11,-0.07) |
| **Q07837** (SLC3A1;79,-17,-0.21) | **P07225** (PROS1;75,-14,-0.18) | **Q9NZN1** (IL1RAPL1;80,-11,-0.13) |
| **P22735** (TGM1;90,-17,-0.18) | **P12271** (RLBP1;36,-14,-0.38) | **P35558** (PCK1;69,-11,-0.15) |
| **Q9Y6N9** (USH1C;62,-17,-0.27) | **Q96G97** (Q96G97;44,-14,-0.31) | **P07949** (RET;124,-11,-0.08) |
| **P02671** (P02671;95,-17,-0.17) | **P02533** (P02533;52,-14,-0.27) | **P35237** (SERPINB6;43,-11,-0.25) |
| **Q7RTS9** (Q7RTS9;76,-17,-0.22) | **P00740** (P00740;52,-14,-0.27) | **P36021** (SLC16A2;60,-11,-0.18) |
| **Q9Y664** (Q9Y664;48,-17,-0.35) | **P51687** (P51687;60,-14,-0.23) | **Q9BRI3** (SLC30A2;35,-11,-0.31) |
| **Q5JTZ9** (AARS2;107,-16,-0.14) | **P15924** (P15924;332,-14,-0.04) | **Q9P0W8** (SPATA7;68,-11,-0.16) |
| **O00203** (AP3B1;121,-16,-0.13) | **P15289** (P15289;54,-14,-0.26) | **Q9Y5Y6** (ST14;95,-11,-0.11) |
| **Q9HBG4** (ATP6V0A4;96,-16,-0.16) | **Q8NCR0** (Q8NCR0;57,-14,-0.24) | **O95876** (WDPCP;85,-11,-0.12) |
| **Q8IWZ6** (BBS7;80,-16,-0.19) | **P42224** (P42224;87,-14,-0.16) | **Q03154** (Q03154;46,-11,-0.23) |
| **P20807** (CAPN3;94,-16,-0.16) | **Q9NP73** (ALG13;126,-13,-0.10) | **P31327** (P31327;165,-11,-0.06) |
| **Q16828** (DUSP6;42,-16,-0.37) | **P11230** (CHRNB1;57,-13,-0.22) | **P51168** (P51168;73,-11,-0.15) |
| **Q96M96** (FGD4;87,-16,-0.18) | **Q04844** (CHRNE;55,-13,-0.23) | **Q9H3S4** (Q9H3S4;27,-11,-0.40) |
| **P11362** (FGFR1;92,-16,-0.17) | **Q96SW2** (CRBN;51,-13,-0.25) | **Q96EN8** (Q96EN8;98,-11,-0.11) |
| **Q92902** (HPS1;79,-16,-0.20) | **Q7Z494** (NPHP3;151,-13,-0.08) | **P00813** (ADA;41,-10,-0.24) |
| **Q9UH77** (KLHL3;65,-16,-0.24) | **Q9H5K3** (POMK;40,-13,-0.32) | **P11912** (CD79A;25,-10,-0.39) |
| **P08253** (MMP2;74,-16,-0.21) | **Q01974** (ROR2;105,-13,-0.12) | **Q9Y223** (GNE;79,-10,-0.12) |
| **P12955** (PEPD;55,-16,-0.29) | **O95248** (SBF1;208,-13,-0.06) | **Q2M1P5** (KIF7;151,-10,-0.06) |
| **P43251** (P43251;61,-16,-0.26) | **Q8N0X7** (SPG20;73,-13,-0.17) | **P01042** (KNG1;72,-10,-0.13) |
| **Q9H5Y7** (Q9H5Y7;95,-16,-0.16) | **Q8N205** (SYNE4;44,-13,-0.29) | **Q14693** (LPIN1;99,-10,-0.10) |
| **P51692** (P51692;90,-16,-0.17) | **O43280** (TREH;67,-13,-0.19) | **Q96KG7** (MEGF10;122,-10,-0.08) |
| **Q969F9** (Q969F9;114,-16,-0.14) | **P17643** (TYRP1;61,-13,-0.21) | **P41227** (NAA10;26,-10,-0.37) |
| **Q8NF50** (Q8NF50;239,-16,-0.06) | **Q2YD98** (UVSSA;81,-13,-0.16) | **Q86WG5** (SBF2;208,-10,-0.04) |
| **P13942** (P13942;172,-16,-0.09) | **P14679** (P14679;60,-13,-0.21) | **P07101** (TH;59,-10,-0.17) |
| **P52788** (P52788;41,-16,-0.38) | **P02748** (P02748;63,-13,-0.20) | **O14773** (TPP1;61,-10,-0.16) |
| **Q6ZW61** (BBS12;79,-15,-0.18) | **Q9NPB3** (Q9NPB3;24,-13,-0.53) | **Q9NRM1** (Q9NRM1;129,-10,-0.07) |
| **Q13936** (CACNA1C;249,-15,-0.06) | **P00734** (P00734;70,-13,-0.18) | **Q9BUN5** (Q9BUN5;22,-10,-0.45) |
| **Q9H2C0** (GAN;68,-15,-0.22) | **P16885** (P16885;148,-13,-0.08) | **Q9NQ48** (Q9NQ48;35,-10,-0.28) |
| **P31150** (GDI1;51,-15,-0.29) | **Q9UBK8** (Q9UBK8;80,-13,-0.16) | **Q9H257** (Q9H257;62,-10,-0.16) |
| **O14920** (IKBKB;87,-15,-0.17) | **Q9UDR5** (Q9UDR5;102,-13,-0.12) | **P57679** (P57679;112,-10,-0.08) |
| **P48357** (LEPR;132,-15,-0.11) | **Q3SXY7** (Q3SXY7;75,-13,-0.17) | **Q96MP8** (Q96MP8;33,-10,-0.30) |
| **Q00872** (MYBPC1;128,-15,-0.11) | **P17050** (P17050;47,-13,-0.27) | **P05160** (P05160;76,-10,-0.13) |
| **O75914** (PAK3;62,-15,-0.24) | **P16871** (P16871;52,-13,-0.25) | **P04180** (P04180;50,-10,-0.20) |
| **P01009** (SERPINA1;47,-15,-0.32) | **O95477** (ABCA1;254,-12,-0.04) | |

**Table 6. Exemplary naturally occurring negatively supercharged tumor suppressor proteins that are suitable for delivery to malignant cells. Proteins listed have a negative net charge of - 10 or less. For each protein, a unique Uniprot identifier is provided in bold. In parentheses, an exemplary name of the gene encoding the respective protein as well as its molecular weight, charge, and molecular weight charge ratio are provided.**

| | | |
|---|---|---|
| **O95359** (TACC2;309,-183,-0.59) | **P23508** (MCC;93,-28,-0.30) | **Q9C0K0** (BCL11B;96,-16,-0.16) |
| **Q12888** (TP53BP1;214,-148,-0.69) | **O00499** (BIN1;65,-26,-0.40) | **Q13625** (TP53BP2;126,-16,-0.12) |
| **Q9UGM3** (DMBT1;261,-89,-0.34) | **Q92574** (TSC1;130,-26,-0.20) | **O60443** (DFNA5;55,-15,-0.27) |
| **P38398** (BRCA1;208,-70,-0.33) | **Q66K89** (E4F1;83,-24,-0.28) | **O15287** (FANCG;69,-15,-0.21) |
| **P39687** (ANP32A;29,-62,-2.16) | **Q13315** (ATM;351,-23,-0.06) | **Q8NFG4** (FLCN;64,-14,-0.21) |
| **O95071** (UBR5;309,-60,-0.19) | **Q5VW36** (FOCAD;200,-22,-0.10) | **Q92597** (NDRG1;43,-14,-0.32) |
| **Q9Y2J2** (EPB41L3;121,-51,-0.42) | **Q96A56** (TP53INP1;27,-22,-0.80) | **Q9UN36** (NDRG2;41,-14,-0.34) |
| **Q14678** (KANK1;147,-48,-0.32) | **O43889** (CREB3;44,-21,-0.47) | **P50502** (ST13;41,-14,-0.33) |
| **Q6ZVD8** (PHLPP2;147,-41,-0.27) | **O94885** (SASH1; 137,-21 ,-0.15) | **Q96EP1** (CHFR;73,-13,-0.17) |
| **Q8TDI0** (CHD5;223,-38,-0.17) | **Q10571** (MN1;136,-20,-0.14) | **P41271** (NBL1;19,-13,-0.66) |
| **A1YPR0** (ZBTB7C;69,-35,-0.50) | **Q9NRY4** (ARHGAP35;171,-18,-0.10) | **Q8IXJ6** (SIRT2;43,-13,-0.30) |
| **O94763** (URI1;60,-34,-0.56) | **Q53EL6** (PDCD4;52,-18,-0.34) | **P98082** (DAB2;82,-12,-0.14) |
| **P23142** (FBLN1;77,-31,-0.40) | **P40337** (VHL;24,-18,-0.74) | **Q96CJ1** (EAF2;29,-12,-0.41) |
| **Q9Y238** (DLEC1;196,-30,-0.15) | **Q9BY67** (CADM1;49,-17,-0.35) | **O43909** (EXTL3;105,-12,-0.11) |
| **Q9Y561** (LRP12;95,-30,-0.31) | **Q5JR59** (MTUS2;150,-17,-0.11) | **A2A2Y4** (FRMD3;69,-12,-0.17) |
| **Q9HBX8** (LGR6;104,-29,-0.27) | **Q8N5A5** (ZGPAT;57,-17,-0.29) | **Q9HB75** (PIDD1;100,-12,-0.12) |
| **O60346** (PHLPP1;185,-29,-0.15) | **Q9NZM4** (GLTSCR1;158,-17,-0.10) | **Q13635** (PTCH1;161,-12,-0.07) |

### References

1. Putney, S. D. & Burke, P. A. Improving protein therapeutics with sustained-release formulations. Nat. Biotechnol. 16, 153-157 (1998).
2. Mullen, L. et al. Latent cytokines for targeted therapy of inflammatory disorders. Expert Opin. Drug Deliv. 11, 101-110 (2014).
3. Song, E. et al. Antibody mediated in vivo delivery of small interfering RNAs via cell-surface receptors. Nat. Biotechnol. 23, 709-717 (2005).
4. Leader, B., Baca, Q. J. & Golan, D. E. Protein therapeutics: a summary and pharmacological classification. Nat. Rev. Drug Discov. 7, 21-39 (2008).
5. Hartung, S. D. et al. Correction of Metabolic, Craniofacial, and Neurologic Abnormalities in MPS I Mice Treated at Birth with Adeno-associated Virus Vector Transducing the Human α-L-Iduronidase Gene. Mol. Ther. 9, 866-875 (2004).
6. Wang, J. et al. Neutralizing antibodies to therapeutic enzymes: considerations for testing, prevention and treatment. Nat. Biotechnol. 26, 901-908 (2008).
7. Umov, F. D., Rebar, E. J., Holmes, M. C., Zhang, H. S. & Gregory, P. D. Genome editing with engineered zinc finger nucleases. Nat. Rev. Genet. 11, 636-646 (2010).
8. Sander, J. D. & Joung, J. K. CRISPR-Cas systems for editing, regulating and targeting genomes. Nat. Biotechnol. 32, 347-355 (2014).
9. Gaj, T., Gersbach, C. A. & Barbas, C. F. ZFN, TALEN, and CRISPR/Cas-based methods for genome engineering. Trends Biotechnol. 31, 397-405 (2013).
10. Midoux, P., Pichon, C., Yaouanc, J.-J. & Jaffrès, P.-A. Chemical vectors for gene delivery: a current review on polymers, peptides and lipids containing histidine or imidazole as nucleic acids carriers. Br. J. Pharmacol. 157, 166-178 (2009).
11. Bodles-Brakhop, A. M., Heller, R. & Draghia-Akli, R. Electroporation for the Delivery of DNA-based Vaccines and Immunotherapeutics: Current Clinical Developments. Mol. Ther. 17, 585-592 (2009).
12. Kay, M. A., Glorioso, J. C. & Naldini, L. Viral vectors for gene therapy: the art of turning infectious agents into vehicles of therapeutics. Nat. Med. 7, 33-40 (2001).
13. Zangi, L. et al. Modified mRNA directs the fate of heart progenitor cells and induces vascular regeneration after myocardial infarction. Nat. Biotechnol. 31, 898-907 (2013).
14. Wadia, J. S., Stan, R. V. & Dowdy, S. F. Transducible TAT-HA fusogenic peptide enhances escape of TAT-fusion proteins after lipid raft macropinocytosis. Nat. Med. 10, 310-315 (2004).
15. Daniels, D. S. & Schepartz, A. Intrinsically cell-permeable miniature proteins based on a minimal cationic PPII motif. J. Am. Chem. Soc. 129, 14578-14579 (2007).
16. Cronican, J. J. et al. Potent delivery of functional proteins into Mammalian cells in vitro and in vivo using a supercharged protein. ACS Chem. Biol. 5, 747-752 (2010).
17. Thompson, D. B., Cronican, J. J. & Liu, D. R. Engineering and identifying supercharged proteins for macromolecule delivery into mammalian cells. Methods Enzymol. 503, 293-319 (2012).
18. Thompson, D. B., Villaseñor, R., Dorr, B. M., Zerial, M. & Liu, D. R. Cellular uptake mechanisms and endosomal trafficking of supercharged proteins. Chem. Biol. 19, 831-843 (2012).
19. Heitz, F., Morris, M. C. & Divita, G. Twenty years of cell-penetrating peptides: from molecular mechanisms to therapeutics. Br. J. Pharmacol. 157, 195-206 (2009).
20. Caron, N. J. et al. Intracellular delivery of a Tat-eGFP fusion protein into muscle cells. Mol. Ther. J. Am. Soc. Gene Ther. 3, 310-318 (2001).
21. Chesnoy, S. & Huang, L. Structure and function of lipid-DNA complexes for gene delivery. Annu. Rev. Biophys. Biomol. Struct. 29, 27-47 (2000).
22. Al-Taei, S. et al. Intracellular traffic and fate of protein transduction domains HIV-1 TAT peptide and octaarginine. Implications for their utilization as drug delivery vectors. Bioconjug. Chem. 17, 90-100 (2006).
23. Shete, H. K., Prabhu, R. H. & Patravale, V. B. Endosomal escape: a bottleneck in intracellular delivery. J. Nanosci. Nanotechnol. 14, 460-474 (2014).
24. Aguilera, T. A., Olson, E. S., Timmers, M. M., Jiang, T. & Tsien, R. Y. Systemic in vivo distribution of activatable cell penetrating peptides is superior to that of cell penetrating peptides. Integr. Biol. Quant. Biosci. Nano Macro 1, 371-381 (2009).
25. Coelho, T. et al. Safety and efficacy of RNAi therapy for transthyretin amyloidosis. N. Engl. J. Med. 369, 819-829 (2013).
26. Judge, A. D., Bola, G., Lee, A. C. H. & MacLachlan, I. Design of noninflammatory synthetic siRNA mediating potent gene silencing in vivo. Mol. Ther. J. Am. Soc. Gene Ther. 13, 494-505 (2006).
27. Basha, G. et al. Influence of cationic lipid composition on gene silencing properties of lipid nanoparticle formulations of siRNA in antigen-presenting cells. Mol. Ther. J. Am. Soc. Gene Ther. 19, 2186-2200 (2011).
28. Semple, S. C. et al. Rational design of cationic lipids for siRNA delivery. Nat. Biotechnol. 28, 172-176 (2010).
29. Boeckle, S., Fahrmeir, J., Roedl, W., Ogris, M. & Wagner, E. Melittin analogs with high lytic activity at endosomal pH enhance transfection with purified targeted PEI polyplexes. J. Control. Release Off. J. Control. Release Soc. 112, 240-248 (2006).
30. Allen, T. M. & Cullis, P. R. Liposomal drug delivery systems: from concept to clinical applications. Adv. Drug Deliv. Rev. 65, 36-48 (2013).
31. Zelphati, O. et al. Intracellular delivery of proteins with a new lipid-mediated delivery system. J. Biol. Chem. 276, 35103-35110 (2001).
32. Adrian, J. E. et al. Targeted SAINT-O-Somes for improved intracellular delivery of siRNA and cytotoxic drugs into endothelial cells. J. Control. Release Off. J. Control. Release Soc. 144, 341-349 (2010).
33. Morris, M. C., Depollier, J., Mery, J., Heitz, F. & Divita, G. A peptide carrier for the delivery of biologically active proteins into mammalian cells. Nat. Biotechnol. 19, 1173-1176 (2001).
34. Colletier, J.-P., Chaize, B., Winterhalter, M. & Fournier, D. Protein encapsulation in liposomes: efficiency depends on interactions between protein and phospholipid bilayer. BMC Biotechnol. 2, 9 (2002).
35. Lawrence, M. S., Phillips, K. J. & Liu, D. R. Supercharging proteins can impart unusual resilience. J. Am. Chem. Soc. 129, 10110-10112 (2007).
36. Liu, J., Gaj, T., Patterson, J. T., Sirk, S. J. & Barbas III, C. F. Cell-Penetrating Peptide-Mediated Delivery of TALEN Proteins via Bioconjugation for Genome Engineering. PLoS ONE 9, e85755 (2014).
37. Tessarollo, L., Vogel, K. S., Palko, M. E., Reid, S. W. & Parada, L. F. Targeted mutation in the neurotrophin-3 gene results in loss of muscle sensory neurons. Proc. Natl. Acad. Sci. U. S. A. 91, 11844-11848 (1994).
38. Maeder, M. L. et al. Robust, synergistic regulation of human gene expression using TALE activators. Nat. Methods 10, 243-245 (2013).
39. Jopling, C., Boue, S. & Belmonte, J. C. I. Dedifferentiation, transdifferentiation and reprogramming: three routes to regeneration. Nat. Rev. Mol. Cell Biol. 12, 79-89 (2011).
40. Fu, Y., Sander, J. D., Reyon, D., Cascio, V. M. & Joung, J. K. Improving CRISPR-Cas nuclease specificity using truncated guide RNAs. Nat. Biotechnol. 32, 279-284 (2014).
41. McNaughton, B. R., Cronican, J. J., Thompson, D. B. & Liu, D. R. Mammalian cell penetration, siRNA transfection, and DNA transfection by supercharged proteins. Proc. Natl. Acad. Sci. U. S. A. 106, 6111-6116 (2009).
42. Qi, L. S. et al. Repurposing CRISPR as an RNA-Guided Platform for Sequence-Specific Control of Gene Expression. Cell 152, 1173-1183 (2013).
43. Guschin, D. Y. et al. A rapid and general assay for monitoring endogenous gene modification. Methods Mol. Biol. Clifton NJ 649, 247-256 (2010).
44. Ran, F. A. et al. Double nicking by RNA-guided CRISPR Cas9 for enhanced genome editing specificity. Cell 154, 1380-1389 (2013).
45. Maeder, M. L. et al. CRISPR RNA-guided activation of endogenous human genes. Nat. Methods 10, 977-979 (2013).
46. Guilinger, J. P., Thompson, D. B. & Liu, D. R. Fusion of catalytically inactive Cas9 to FokI nuclease improves the specificity of genome modification. Nat. Biotechnol. 32, 577-582 (2014).
47. Pattanayak, V. et al. High-throughput profiling of off-target DNA cleavage reveals RNA-programmed Cas9 nuclease specificity. Nat. Biotechnol. 31, 839-843 (2013).
48. Li, H. et al. Differentiation of neurons from neural precursors generated in floating spheres from embryonic stem cells. BMC Neurosci. 10, 122 (2009).
49. Lumpkin, E. A. et al. Mathl-driven GFP expression in the developing nervous system of transgenic mice. Gene Expr. Patterns GEP 3, 389-395 (2003).
50. Van Camp, G. & Smith, R. Hereditary Hearing Loss. at <http://hereditaryhearingloss.org>
51. Kim, S., Kim, D., Cho, S. W., Kim, J. & Kim, J.-S. Highly efficient RNA-guided genome editing in human cells via delivery of purified Cas9 ribonucleoproteins. Genome Res. 24, 1012-1019 (2014).
52. Yin, H. et al. Genome editing with Cas9 in adult mice corrects a disease mutation and phenotype. Nat. Biotechnol. 32, 551-553 (2014).
53. Wang, H. et al. One-step generation of mice carrying mutations in multiple genes by CRISPR/Cas-mediated genome engineering. Cell 153, 910-918 (2013).
54. Schneider, C. A., Rasband, W. S. & Eliceiri, K. W. NIH Image to ImageJ: 25 years of image analysis. Nat. Methods 9, 671-675 (2012).
55. Sage, C. et al. Proliferation of functional hair cells in vivo in the absence of the retinoblastoma protein. Science 307, 1114-1118 (2005).
56. Sander, J. D. et al. In silico abstraction of zinc finger nuclease cleavage profiles reveals an expanded landscape of off-target sites. Nucleic Acids Res. 41, e181 (2013).
57. Mali, P. et al. RNA-guided human genome engineering via Cas9. Science 339, 823-826 (2013)
58. Ramakrishna, S. et al. Gene disruption by cell-penetrating peptide-mediated delivery of Cas9 protein and guide RNA. Genome Res. 24, 1020-1027 (2014).
59. Gilleron, J. et al. Image-based analysis of lipid nanoparticle-mediated siRNA delivery, intracellular trafficking and endosomal escape. Nat. Biotechnol. 31, 638-646 (2013).
60. Lodish, H. et al. Molecular Cell Biology. (W. H. Freeman, 2000).
61. Sojung Kim, D. K. Highly efficient RNA-guided genome editing in human cells via delivery of purified Cas9 ribonucleoproteins. Genome Res. (2014). doi:10.1101/gr.171322.113

### EQUIVALENTS AND SCOPE

Those skilled in the art will recognize, or be able to ascertain using no more than routine experimentation, many equivalents to the specific embodiments, described herein. The scope of the present invention is not intended to be limited to the above Description, but rather is as set forth in the appended claims.

Those skilled in the art will recognize, or be able to ascertain using no more than routine experimentation, many equivalents to the specific embodiments in accordance with the invention described herein. The scope of the present invention is not intended to be limited to the above Description, but rather is as set forth in the appended claims.

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process. Furthermore, it is to be understood that the invention encompasses all variations, combinations, and permutations in which one or more limitations, elements, clauses, descriptive terms, *etc.,* from one or more of the listed claims is introduced into another claim. For example, any claim that is dependent on another claim can be modified to include one or more limitations found in any other claim that is dependent on the same base claim. Furthermore, where the claims recite a composition, it is to be understood that methods of using the composition for any of the purposes disclosed herein are included, and methods of making the composition according to any of the methods of making disclosed herein or other methods known in the art are included, unless otherwise indicated or unless it would be evident to one of ordinary skill in the art that a contradiction or inconsistency would arise.

Where elements are presented as lists, *e.g.*, in Markush group format, it is to be understood that each subgroup of the elements is also disclosed, and any element(s) can be removed from the group. It should it be understood that, in general, where the invention, or aspects of the invention, is/are referred to as comprising particular elements, features, *etc.,* certain embodiments of the invention or aspects of the invention consist, or consist essentially of, such elements, features, *etc.* For purposes of simplicity those embodiments have not been specifically set forth *in haec verba* herein. It is also noted that the term "comprising" is intended to be open and permits the inclusion of additional elements or steps.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

In addition, it is to be understood that any particular embodiment of the present invention that falls within the prior art may be explicitly excluded from any one or more of the claims. Since such embodiments are deemed to be known to one of ordinary skill in the art, they may be excluded even if the exclusion is not set forth explicitly herein. Any particular embodiment of the compositions of the invention (*e.g.*, any supercharged protein; any nucleic acid; any method of production; any method of use; *etc*.) can be excluded from any one or more claims, for any reason, whether or not related to the existence of prior art.

All cited sources, for example, references, publications, databases, database entries, and art cited herein, are incorporated into this application by reference, even if not expressly stated in the citation. Where the contents of a database entry may change over time, the contents of the database entry at the time of filing of the present application is incorporated herein by reference. In case of conflicting statements of a cited source and the instant application, the statement in the instant application shall control.

Aspects and features of the present disclosure are set out in the following numbered clauses which contain the subject matter of the claims of the parent application as filed:
1. A composition for delivering a protein into a cell, the composition comprising:
   (a) a protein to be delivered to a cell, wherein the protein is
      (i) negatively charged;
      (ii) associated with a nucleic acid resulting in a complex that is negatively charged; and/or
      (iii) associated with a supernegatively charged protein resulting in a complex that is negatively charged; and
   (b) a cationic polymer or a cationic lipid.
2. The composition of clause 1, wherein the protein to be delivered to a cell is a naturally occurring negatively charged protein, or a negatively charged fragment thereof.
3. The composition of clause 1, wherein the protein to be delivered is a supernegatively charged protein, or a negatively charged fragment thereof.
4. The composition of clause 3, wherein the protein to be delivered has been engineered to be supernegatively charged.
5. The composition of any one of clauses 2-3, wherein the fragment comprises a sequence of at least 10, at least 20, at least 30, at least 40, at least 50, at least 75, or at least 100 consecutive amino acids.
6. The composition of any one of clauses 1, 3, or 4, wherein the supernegatively charged protein to be delivered or the supernegatively charged protein of (a)(iii) has a net charge of less than -5, less than -10, less than -20, less than -30, less than -40, less than -50, less than - 60, less than -70, less than -80, less than -90, less than -100, less than -110, less than -120, less than -130, less than -140, less than -150, less than -160, less than -170, less than -180, less than -190, less than -200, less than -250, less than -300, or less than -400.
7. The composition of any one of clause 1, wherein the protein to be delivered is not negatively charged.
8. The composition of any one of clauses 1-7, wherein the protein to be delivered is fused to the supernegatively charged protein of (a)(iii) thus forming a fusion protein.
9. The composition of any one of clauses 1-8, wherein the protein to be delivered of (a)(i), the protein and the nucleic acid of (a)(ii), or the protein to be delivered and the associated supernegatively charged protein of (a)(iii) has a net charge of less than -5, less than -10, less than -20, less than -30, less than -40, less than -50, less than -60, less than -70, less than -80, less than -90, less than -100, less than -110, less than -120, less than -130, less than -140, less than -150, less than -160, less than -170, less than -180, less than -190, less than -200, less than -250, less than -300, or less than -400.
10. The composition of any one of clauses 1-9, wherein the protein to be delivered of (a)(i), the protein and the nucleic acid of (a)(ii), or the protein to be delivered and the associated supernegatively charged protein of (a)(iii) has a charge:molecular weight ratio of less than -0.03, less than -0.04, less than -0.05, less than -0.06, less than -0.07, less than -0.08, less than -0.09, less than -0.1, less than -0.2, less than -0.3, less than -0.4, less than -0.5, less than -0.6, less than -0.7, less than -0.8, less than -0.9, less than -1, less than -1.1, less than - 1.2, less than -1.3, less than -1.4, less than -1.5, less than -1.6, less than -1.7, less than -1.8, less than -1.9, less than -2, less than -2.1, less than -2.2, less than -2.3, less than -2.4, less than -2.5, less than -2.6, less than -2.7, less than -2.8, less than -2.9, less than -3, less than -3.1, less than -3.2, less than -3.3, less than -3.4, less than -3.5, less than -3.6, less than -3.7, less than -3.8, less than -3.9, or less than -4.
11. The composition of any one of clauses 1-10, wherein the protein to be delivered is a protein listed in Table 3.
12. The composition of any one of clauses 1-11, wherein the protein to be delivered is implicated in a disease or disorder.
13. The composition of any one of clauses 1-12, wherein the protein to be delivered is listed in any of Tables 4 or 5.
14. The composition of any one of clauses 1-13, wherein the protein to be delivered is a tumor suppressor.
15. The composition of any one of clauses 1-14, wherein the protein to be delivered is listed in Table 6.
16. The composition of any one of clauses 1-10, wherein the protein to be delivered is Sirtl, PPARg, PRDM16, PGCla, TP53BP1, Utrophin, Dystrophin, Bik, IκBα, Von Hippel-Lindau disease tumor suppressor, or an E3 ubiquitin ligase.
17. The composition of any one of clauses 1-10, wherein the protein to be delivered is a metal-binding protein.
18. The composition of any one of clauses 1-17, wherein the supernegatively charged protein of (a)(iii) comprises a 3xFLAG sequence, a VP64 sequence, or a supernegatively charged fluorescent protein or streptavidin (SAV).
19. The composition of any one of clauses 1-18, wherein the supernegatively charged protein of (a)(iii) comprises -7 GFP or -20GFP, or a negatively charged fragment thereof.
20. The composition of any one of clauses 1-18, wherein the supernegatively charged protein of (a)(iii) comprises -40 SAV, or a negatively charged fragment thereof.
21. The composition of clause 19 or 20, wherein the fragment comprises a sequence of at least 10, at least 20, at least 30, at least 40, at least 50, at least 75, or at least 100 consecutive amino acids.
22. The composition of any one of clauses 1-21, wherein the protein to be delivered is associated with biotin thus forming a biotinylated version of the protein to be delivered.
23. The composition of clause 22, wherein the protein to be delivered is associated to the biotin via a linker.
24. The composition of clause 23, wherein the linker comprises a covalent bond generated via click chemistry, NHS ester chemistry, or maleimide chemistry.
25. The composition of clause 23 or 24, wherein the linker is a cleavable linker.
26. The composition of clause 25, wherein the linker is cleaved by a protease or an esterase, or by a reducing environment.
27. The composition of clause 25 or 26, wherein the linker is cleaved by an enzyme present in endosomes or under conditions present in endosomes.
28. The composition of any one of clauses 22-27, wherein the biotinylated protein to be delivered is associated with the supernegatively charged protein of (a)(iii) via a non-covalent interaction.
29. The composition of any one of clauses 22-28, wherein the supernegatively charged protein is a supernegatively charged avidin or avidin variant, or a biotin-binding fragment thereof.
30. The composition of clause 29, wherein the supernegatively charged avidin or avidin variant is a supernegatively charged streptavidin, or a biotin-binding fragment thereof.
31. The composition of clause 29, wherein the supernegatively charged protein is fused to an avidin or avidin variant.
32. The composition of any one of clauses 29-31, wherein the avidin or avidin variant is streptavidin, or a biotin-binding fragment thereof.
33. The composition of any one of clauses 1-32, wherein the cationic polymer or the cationic lipid is suitable for delivery of an agent bound by the polymer or lipid to a cell.
34. The composition of any one of clauses 1-33, wherein the composition comprises a cationic lipid.
35. The composition of clause 34, wherein the cationic lipid is selected from the group consisting of Lipofectamine^{®} 2000, Lipofectamine^{®} 3000, Lipofectamine^{®} RNAiMAX, and Lipofectamine^{®} LTX.
36. The composition of any one of clauses 1-35, wherein the composition exhibits low toxicity when administered to a population of cells.
37. The composition of clause 36, wherein at least 60%, at least 65%, at least 70%, at least 75%, at least 80%, at least 85%, at least 90%, at least 95%, or at least 99% of the cells are viable 24 hours after administration of an amount of the composition effective for delivery of the protein to be delivered into at least 1% of the cells.
38. The composition of any one of clauses 1-37, wherein the composition is a pharmaceutical composition.
39. A composition, comprising
   (a) a protein to be delivered; and
   (b) a negatively charged molecule conjugated to the protein to be delivered resulting in a complex that is negatively charged.
40. The composition of clause 39, wherein the negatively charged molecule is a nucleic acid.
41. The composition of clause 39, wherein the negatively charged molecule is a negatively charged protein.
42. The composition of clause 41, wherein the negatively charged protein is a supernegatively charged protein.
43. The composition of clause 42, wherein the supernegatively charged protein is a supernegatively charged fluorescent protein or a supernegatively charged streptavidin.
44. A method for delivering a protein to be delivered to a cell, comprising contacting the cell with the composition of any of clauses 1-43.
45. The method of clause 44, wherein the contacting is *in vitro.*
46. The method of clause 44, wherein the contacting is *in vivo.*
47. The method of clause 46, wherein the composition is a pharmaceutical composition.
48. A kit comprising the composition of any one of clauses 1-43.
49. A kit for carrying out the method of any one of clauses 45-48.

## Claims

1. A composition for delivering a protein into a cell, the composition comprising:
(a) a protein to be delivered to a cell, wherein the protein is associated with a nucleic acid resulting in a complex that is negatively charged; and
(b) a cationic polymer or a cationic lipid.

2. The composition of claim 1, wherein the protein to be delivered is a transcriptional activator, a transcriptional repressor, a protease, or a therapeutic protein.

3. The composition of claim 1 or 2, wherein the protein to be delivered to a cell is negatively charged and:
(i) is a naturally occurring negatively charged protein or a negatively charged fragment thereof; or
(ii) is a supernegatively charged protein or a negatively charged fragment thereof, optionally wherein the protein to be delivered has been engineered to be supernegatively charged; or
(iii) has been engineered to be negatively charged.

4. The composition of any preceding claim, wherein the protein to be delivered comprises a Cas9 protein.

5. The composition of claim 4, wherein the Cas9 protein comprises a wild-type Cas9 protein, a Cas9 variant that comprises an amino acid sequence that is at least 90% identical to the amino acid sequence of SEQ ID NO: 44, a Cas9 nickase, or a nuclease-inactivated Cas9 (dCas9) that comprises an amino acid sequence that is at least 90% identical to the amino acid sequence of SEQ ID NO: 45, and optionally comprises a D10A and H840A substitution as compared to the amino acid sequence of a wild type Cas9.

6. The composition of claim 4 or 5, wherein the Cas9 protein is fused to a deaminase domain, optionally wherein:
(i) the deaminase domain comprises a cytidine deaminase domain of the APOBEC family of cytosine deaminase enzymes, including APOBEC-1, AID, and APOBEC-3G deaminases; or
(ii) the deaminase domain comprises an adenosine deaminase domain comprising an ADAT family deaminase.

7. The composition of claim 4 or 5, wherein the Cas9 is fused to a transcriptional activator domain, a transcriptional repressor domain, a nuclease domain, a recombinase domain, a deaminase domain, an epigenetic modifier domain, a protease domain or a therapeutic protein domain; optionally wherein the therapeutic protein domain is a tumor suppressor protein domain, a therapeutic enzyme domain, a growth factor domain, a growth factor receptor domain, or a transcription factor domain.

8. The composition of any one of claims 1-3, wherein the protein to be delivered is:
(i) a protein listed in Table 3, Table 4 or Table 5,
(ii) a protein selected from TACC2, TP53BP1, DMBT1, BRCA1, ANP32A, UBR5, EPB41L3, KANK1, PHLPP2, CHD5, ZBTB7C, URI1, FBLN1, DLEC1, LRP12, LGR6, PHLPP1, MCC, BIN1, TSC1, E4F1, ATM, FOCAD, TP53INP1, REB3, SASH1, MN1, ARHGAP35, PDCD4, VHL, CADMl, MTUS2, ZGPAT, GLTSCRl, BCL11B, TP53BP2, DFNA5, FANCG, FLCN, NDRGl, NDRG2, ST13, CHFR, NBL1, SIRT2, DAB2, EAF2, EXTL3, FRMD3, PIDD1, and PTCH1;
(iii) Sirtl, PPAPγ, PRDM16, PGC1α, TP53BP1, Utrophin, Dystrophin, Bik, IκBα, Von Hippel-Lindau disease tumor suppressor, or an E3 ubiquitin ligase;
(iv) a TALE protein; or
(v) implicated in a disease or disorder.

9. The composition of any preceding claim, wherein the composition comprises:
(i) a cationic polymer selected from polyallylamine (PAH), polyethyleneimine (PEI), polyvinylamine homo- or copolymer, a poly(vinylbenzyl-tri-C₁-C₄-alkylammonium salt), a polymer of an aliphatic or araliphatic dihalide and an aliphatic N,N,N',N'-tetra-C₁-C₄-alkyl-alkylenediamine, a poly(vinylpyridin) or poly(vinylpyridinium) salt, a poly(N,N-diallyl-N,N-di-C₁-C₄-alkyl-ammoniumhalide), a homo- or copolymer of a quaternized di-C₁-C₄-alkyl-aminoethyl acrylate or methacrylate, polidronium chloride (POLYQUAD), and a polyaminoamide; or
(ii) a cationic lipid selected from Lipofectamine^{®} 2000, Lipofectamine^{®} 3000, Lipofectamine^{®} RNAiMAX, Lipofectamine^{®} LTX, and combinations thereof.

10. The composition of any preceding claim, wherein the complex of (a) has:
(i) a net charge of less than -5, less than -10, less than -20, less than -30, less than -40, less than -50, less than -60, less than -70, less than -80, less than -90, less than -100, less than - 110, less than -120, less than -130, less than -140, less than -150, less than -160, less than -170, less than -180, less than -190, less than -200, less than -250, less than -300, or less than -400; and/or
(ii) a charge: molecular weight ratio of less than -0.03, less than -0.04, less than -0.05, less than -0.06, less than -0.07, less than -0.08, less than -0.09, less than -0.1, less than -0.2, less than -0.3, less than -0.4, less than -0.5, less than -0.6, less than -0.7, less than -0.8, less than -0.9, less than -1, less than -1.1, less than -1.2, less than -1.3, less than -1.4, less than -1.5, less than -1.6, less than -1.7, less than -1.8, less than -1.9, less than -2, less than -2.1, less than -2.2, less than - 2.3, less than -2.4, less than -2.5, less than -2.6, less than -2.7, less than-2.8, less than -2.9, less than -3, less than -3.1, less than -3.2, less than -3.3, less than -3.4, less than -3.5, less than -3.6, less than -3.7, less than -3.8, less than -3.9, or less than -4; and/or
(iii) the composition exhibits low toxicity when administered to a population of cells, optionally wherein at least 60%, at least 65%, at least 70%, at least 75%, at least 80%, at least 85%, at least 90%, at least 95%, or at least 99% of the cells are viable 24 hours after administration of an amount of the composition effective for delivery of the protein to be delivered into at least 1% of the cells.

11. The composition of any preceding claim, wherein the composition is a pharmaceutical composition.

12. The composition of any one of claims 1-11 for use in medicine.

13. A method for delivering a protein to a cell comprising contacting the cell with the composition of any one of claims 1-11, wherein the method is not a method for the treatment of the human or animal body by surgery or therapy.

14. The method of claim 13, wherein the cell is a mammalian cell, optionally a human cell, and optionally a T cell, a neuron, a stem cell, a progenitor cell, a blood cell, a fibroblast, an epithelial cell, a neoplastic cell, or a tumor cell.

15. The method of claim 13 or 14, wherein the step of contacting is done *in vitro* or *ex vivo.*

16. The method of any one of claims 13-15, wherein the cell is in a non-human subject, and the step of contacting is done *in vivo.*
